# EUROPEAN PATENT SPECIFICATION

(11) **EP 3 619 311 B1**
(45) Date of publication and mention of the grant of the patent: **03.01.2024**
(21) Application number: 18728221.5
(22) Date of filing: 02.05.2018
(51) Int. Cl.: C12N 15/66, C12N 15/10, C12N 15/64

(54) **DNA ASSEMBLY**
DNA-ANORDNUNG
ASSEMBLAGE D'ADN

(30) Priority: 03.05.2017 GB 201707049; 23.08.2017 GB 201713546
(43) Date of publication of application: 11.03.2020
(73) Proprietor: Oxford University Innovation Ltd., Oxford, Oxfordshire OX2 0JB (GB)
(72) Inventor: LIN, Da, Oxford Oxfordshire OX2 0JB (GB); O'CALLAGHAN, Chris, Oxford Oxfordshire OX2 0JB (GB)
(74) Representative: Barker Brettell LLP
(86) International application number: PCT/GB2018/051174
(87) International publication number: WO 2018/203056

(56) References cited:
- US-A1- 2016 002 644
- MARKO STORCH ET AL: "BASIC: A New Biopart Assembly Standard for Idempotent Cloning Provides Accurate, Single-Tier DNA Assembly for Synthetic Biology", ACS SYNTHETIC BIOLOGY, vol. 4, no. 7, 17 July 2015 (2015-07-17), pages 781-787, XP055343133, Washington, DC,USA ISSN: 2161-5063, DOI: 10.1021/sb500356d
- W.-H. CHEN ET AL: "The MASTER (methylation-assisted tailorable ends rational) ligation method for seamless DNA assembly", NUCLEIC ACIDS RESEARCH, vol. 41, no. 8, 1 April 2013 (2013-04-01), pages e93-e93, XP055239850, UK ISSN: 0305-1048, DOI: 10.1093/nar/gkt122
- Arturo Casini ET AL: "Bricks and blueprints: methods and standards for DNA assembly", Nature reviews. Molecular cell biology, 17 June 2015 (2015-06-17), pages 568-576, XP055213815, England DOI: 10.1038/nrm4014 Retrieved from the Internet: URL:http://www.ncbi.nlm.nih.gov/pubmed/260 81612 [retrieved on 2015-09-16]

## Description

This invention relates to DNA assembly methods, and related nucleic acid material.

Advances in biology and biotechnology have led to development of a variety of methods for assembly of large DNA constructs. Existing methods could be largely divided into two groups: homology-based methods that utilize long overlapping sequence between fragments to specify the order of assembly, such as Gibson assembly and yeast- or *B. subtilis*-based *in vivo* homologous recombination approaches, and restriction enzyme/recombinase-based methods that utilize defined enzyme-specific sequence to specify the order of assembly, such as Moclo (Reviewed in Nat Rev Mol Cell Biol. 2015 Sep;16(9):568-76. doi: 10.1038/nrm4014 (PMID 2608161)2 and Crit Rev Biotechnol. 2017 May;37(3):277-286. doi: 10.3109/07388551.2016.1141394 (PMID 26863154). An ongoing struggle is to achieve a balance between reducing sequence design constraints such as forbidden sequence in assembled DNA and unwanted scar sequence introduced during the assembly process, and improving assembly modularity and part reusability.

The initial type IIS restriction enzyme-based DNA assembly system was named Golden Gate assembly. In Golden Gate assembly system, insert DNA fragments to be assembled within insert plasmids are flanked by recognition sites for a type IIS restriction enzyme that cuts outside the recognition sequence and generates arbitrary adhesive ends with sequences independent of the enzyme recognition sequence. The insert plasmids for Golden Gate assembly contain inserts flanked by type IIS restriction site within the insert vector backbone facing the inserts, so that once the inserts are released from the plasmid by the type IIS restriction enzyme, they do not contain the restriction site. The assembly vector for Golden Gate assembly contains a negative selection marker flanked by type IIS sites located in the selection marker facing the vector, so that once the selection marker is released from the vector, the vector backbone does not contain the type IIS restriction site. These specially designed plasmids allows simple one-pot assembly of multiple DNA fragments by mixing insert plasmids and vectors together with a type IIS restriction enzyme and DNA ligase. In the Golden Gate assembly reaction, restriction digest of inserts and vector, and ligation between inserts and vector occurs in the same tube. A mixture of ligation products will be generated during the one-pot reaction when inserts and assembly vector backbone have compatible adhesive ends. This includes favorable ligation products among inserts and assembly vector backbone, as well as unfavorable ligation products such as between the negative selection marker and assembly vector backbone, between the negative selection marker and insert vector backbone, and between insert and insert vector backbone. Because the type IIS restriction sites are located within the negative selection marker and the insert vector backbone, all the unfavorable ligation products are susceptible to digestion by the type IIS restriction enzyme, while the favorable ligation products are refractory to digestion. As a result the reaction favors generation of correctly assembled ligation products among inserts and assembly vector backbone in the long term. Followed by selection using positive antibiotic selection markers within the assembly vector backbone and negative selection marker within the assembly vector insert, plasmids containing correctly assembled inserts could be selected with high efficiency.

Moclo-based systems were developed from the Golden Gate assembly system by adding a second pair type IIS restriction sites different from the enzyme used for assembly to the assembly vector in order to release the assembled DNA for next stage assembly. In Golden Gate assembly, the assembled DNA could not be released by the same restriction enzyme used for assembly, because the inserts and assembly plasmid vector backbone lack recognition sequence of the type IIS restriction enzyme used. By adding a different pair of type IIS restriction sites to release the assembled fragment, the process of Golden Gate assembly could be repeated using this second restriction enzyme and a different vector carrying a different antibiotic selection marker. Alternative sequential uses of two different type IIS restriction enzymes and antibiotic selection markers enable hierarchical assembly of large DNA fragments by one-pot restriction/ligation reaction.

A major drawback of type IIS enzyme-based DNA assembly is the necessity to remove internal type IIS restriction sites within the DNA parts to be used for assembly. A minimum of two enzymes are required for basic hierarchical assembly, and three enzymes are required for other schemes such as multi-step linear addition of DNA parts. Because most of the known type IIS restriction enzymes recognize <=6bp sequence, most Moclo-based systems use 6bp cutters. This requires that the DNA part must be free of two 6bp asymmetric sequences, which occur at a frequency of ~ 1 in 1kb for a random DNA sequence with 50% GC content. A potential improvement of the current Moclo system would be to use type IIS restriction enzymes with 7bp or more specificity. However, this option is limited by the availability of commercial type IIS restriction enzymes. Only two types of 7bp type IIS restriction enzymes are currently commercially available: LguI/SapI which recognizes GCTCTTC and leaves 3bp sticky end, and AarI which recognizes CACCTGC and leaves 4bp sticky end. AarI also requires addition of oligonucleotides for complete digestion, which is undesirable for DNA assembly.

Existing type IIS restriction enzyme-based DNA assembly systems are also designed for modular DNA assembly which leave unwanted scar sequences in the final assembled DNA, and cannot be used to assemble arbitrary DNA sequence without making custom assembly vectors.

US20160002644 describes a system of DNA assembly. It uses two restriction enzymes LguI and Earl, and a strain with inducible expression of M.TaqI to block overlapping restriction sites in the assembly vector. The M.TaqI site overlaps with the 3bp adhesive end sequence generated by LguI/EarI, which leaves severe constraints on adaptor sequence design.

What is required is an improved DNA assembly method which provides one or more of a greater freedom to choose and design adapter sequences, assembly with minimal scarring, less reaction steps or complexity, and the ability to assemble larger sequences of DNA. Therefore, an aim of the present invention is to provide an improved DNA assembly method and associated material.

According to a first aspect of the invention there is provided a nucleic acid for use in DNA assembly in accordance with claim 1 herein.

Described herein, is a nucleic acid for use in DNA assembly, wherein the nucleic acid comprises at least one methylation-protectable restriction element, the methylation-protectable restriction element comprising:
- a restriction enzyme recognition sequence that is recognised by a restriction enzyme that cleaves outside of the recognition sequence; and
- a DNA methylase recognition sequence,

wherein the restriction enzyme recognition sequence and the DNA methylase recognition sequence overlap such that the base modified by the DNA methylase lies within the restriction enzyme recognition sequence,
wherein the DNA methylase recognition sequence is not identical to, or enclosed by, the restriction enzyme recognition sequence, and
wherein the DNA methylase recognition sequence does not overlap with the sequence that would form the overhang end sequence generated by the restriction enzyme.

The invention herein can advantageously allow for the use of a single restriction enzyme throughout different stages of DNA assembly. This reduces the cost and complexity of preparing a starting part of DNA fragment to be free of the restriction enzyme used. As the methylase recognition sequence does not overlap with the sequence that would form the overhang end sequence generated by the restriction enzyme, the invention further provides more freedom to design adapters (i.e. overhangs) for DNA fragment assembly. The invention also provides the first hierarchical scarless DNA assembly system that uses restriction enzyme-based assembly method. Unlike existing modular DNA assembly methods, which leave unwanted scar sequences in the final assembled DNA, the present invention can be used to assemble large and arbitrary DNA sequences without scarring. Existing scarless assembly systems, all of which are based on homology-based assembly method, such as Gibson assembly and yeast homologous recombination, are unable to assemble large DNA constructs with repetitive DNA sequences, in contrast to the present invention. The method of the invention also allows a simple design for hierarchical scarless assembly of DNA using a universal set of 6 plasmids.

The methylation of the cut control element may block/impair the overlapping restriction enzyme recognition site.

In one description, the restriction enzyme that cleaves outside its recognition sequence is a type IIS restriction enzyme.

In one description, the nucleic acid comprises at least two methylation-protectable restriction elements. In another description, the nucleic acid has two methylation-protectable restriction elements. In adescription comprising two methylation-protectable restriction elements, the nucleic acid sequence between the cut sites of the two methylation-protectable restriction elements may be a discard sequence (i.e. an unwanted fragment that will be removed from the nucleic acid during a restriction reaction). In an alternative description, the discard sequence may be previously removed. For example, the nucleic acid may be a previously-cut linearized vector having a methylation-protectable restriction element at each end.

The discard sequence may comprise a selectable marker, reporter gene, or label. The skilled person will be familiar with typical markers and reporter genes used in DNA assembly and cloning. For example, a selectable marker may comprise a gene encoding an antibiotic resistance, an enzymatic activity, a luciferase, or the like. The marker could be a label, such as a radiolabel. In one description, the discard sequence encodes *lacZ* as a reporter. The skilled person will recognise that discard sequence, that may be replaced by the assembled sequence in a vector, may not contain any functional element at all, as the process favours generating assembled DNA.

Providing a selectable marker, reporter gene, or label on the discard sequence advantageously allows clones to be selected that have had the discard sequence successfully removed or replaced by a fragment/sequence of interest.

In one description, an opposing non-protectable restriction enzyme recognition sequence is provided on the opposing side of the cut site of the methylation-protectable restriction element. For example, in a description comprising a discard sequence, the opposing non-protectable restriction enzyme recognition sequence would be located in the discard sequence. The opposing non-protectable restriction enzyme recognition sequence may also be recognized by a restriction enzyme that cleaves outside its recognition sequence, such as a type IIS restriction enzyme. The opposing non-protectable restriction enzyme recognition sequence may not be protected by (or arranged to be protected by) methylation, or at least may not be protected by methylation from a methylase that recognizes the methylase recognition sequence of the methylation-protectable restriction element. Such an opposing non-protectable restriction enzyme recognition sequence in the discard sequence may otherwise be referred to as an "inner restriction enzyme recognition sequence", whereas the restriction enzyme recognition sequence of the methylation-protectable restriction element may be referred to as an "outer restriction enzyme recognition sequence".

The restriction enzyme recognition sequence of the methylation-protectable restriction element may be recognised by the same restriction enzyme species as the opposing non-protectable restriction enzyme recognition sequence. The restriction enzyme recognition sequence of the methylation-protectable restriction element may comprise the same sequence as the opposing non-protectable restriction enzyme recognition sequence.

In one embodiment, the nucleic acid may comprise a "maintaining composite element", wherein the opposing non-protectable restriction enzyme recognition sequence may be arranged to direct the restriction enzyme to cut the nucleic acid at the same site as the restriction enzyme recognition sequence of the methylation-protectable restriction element, such that the same overhang/sticky end would be produced at the same position. This may be achieved by providing the restriction enzyme recognition sequence and opposing non-protectable restriction enzyme recognition sequence at a specific distance apart, depending on the cut site provided by the restriction enzyme(s) selected. In this embodiment, the sequence of the overhang produced by the restriction enzyme cutting the nucleic acid according to the restriction enzyme recognition sequence of the methylation-protectable restriction element is maintained after cutting the nucleic acid with the restriction enzyme that recognizes the opposing non-protectable restriction enzyme recognition sequence of the discard sequence and a subsequent ligation with a DNA fragment insert. As such, this embodiment may be referred to as the "maintaining composite element", i.e. the maintaining composite element may comprise two restriction sequences arranged in head-to-head direction (one of which is part of the methylation protectable element, and the other the opposing non-protectable restriction enzyme recognition sequence). The overhang produced from a maintaining composite element may be pre-determined by the sequence between the opposing restriction recognition sequences.

In an alternative embodiment to the maintaining composite element, there is provided a "truncating composite element", wherein the distance between the methylation-protectable restriction element and the opposing non-protectable restriction enzyme recognition sequence may be reduced such that the opposing non-protectable restriction enzyme recognition sequence may be arranged to direct the restriction enzyme to cut the nucleic acid at a site that does not overlap the cut site of the restriction enzyme that recognizes the restriction enzyme recognition sequence of the methylation-protectable restriction element (i.e. such that the position of the subsequent overhangs created by the opposing cut sites do not overlap, and the subsequent overhangs may be different in sequence). The cut site of the opposing non-protectable restriction enzyme recognition sequence in a truncating composite element may be within the recognition sequence of the restriction enzyme recognition sequence of the methylation-protectable restriction element, and/or within the nucleotides between the cut site and the recognition sequence of the restriction enzyme recognition sequence of the methylation-protectable restriction element. In one embodiment, the cleavage point between base pairs at the end of one cut site and the cleavage point between base pairs at the start of the opposing cut site may be in adjacent/neighbouring base pairs (i.e. the cut sites may be immediately adjacent to each other, but do not overlap). This may be achieved by providing the restriction enzyme recognition sequence of the methylation-protectable restriction element and opposing non-protectable restriction enzyme recognition sequence at a specific distance apart, depending on the cut site provided by the restriction enzyme(s) selected. The distance will be close enough to cause cutting in the opposing non-protectable restriction enzyme recognition sequence and/or nucleotides between the recognition sequence and cut site, or vice versa (i.e. closer than the opposing restriction enzyme recognition sequences in the "maintaining composite element" for an equivalent/same selected restriction enzyme(s)). In this truncating composite element embodiment, the sequence of the overhang produced by the restriction enzyme cutting the nucleic acid according to the restriction enzyme recognition sequence of the methylation-protectable restriction element may or may not be maintained after cutting the nucleic acid with the restriction enzyme that recognizes the opposing non-protectable restriction enzyme recognition sequence of the discard sequence and a subsequent ligation with a DNA fragment insert, whereby the sequence of the DNA fragment insert would dictate the sequence of the overhang (e.g. the overhang produced from a truncating composite element may not be pre-determined by the sequence between the opposing restriction enzyme recognition sites).

In an alternative embodiment to the maintaining composite element and truncating composite element, there is provided an "insertional composite element", wherein a sequence insert is provided between the methylation-protectable restriction element and the opposing non-protectable restriction enzyme recognition sequence. The sequence insert may comprise a functional sequence such that it provides a function. For example, the sequence insert may comprise a promoter sequence or a terminator sequence. In an embodiment comprising an insertional composite element the methylation-protectable restriction element and the opposing non-protectable restriction enzyme recognition sequence may not overlap and may be distanced apart by the sequence insert. The sequence insert may be any suitable length. For example, the distance between the methylation-protectable restriction element and the opposing non-protectable restriction enzyme recognition sequence may be any number of nucleotides as required for the sequence insert, such as a functional sequence insert. The distance between the restriction enzyme recognition sequence within the methylation-protectable restriction element and the opposing non-protectable restriction enzyme recognition sequence may range between 6bp to 300kb.

Advantageously, following DNA assembly, the functional sequence will be added into the 5' or 3' end of the assembled DNA. This design is convenient for adding common elements to an assembled plasmid by using specialized vectors (for example adding promoter and terminators in a multi-part coding region gene assembly reaction).

In one description, for the "maintaining composite element", the opposing non-protectable restriction enzyme recognition sequence (such as a BsaI sequence) may be distanced apart from the restriction enzyme recognition sequence (such as a BsaI sequence) of the methylation-protectable restriction element by 6 base pairs (i.e. 6 bp in the gap between the end of one recognition sequence and the start of the opposing recognition sequence). The skilled person will recognise that such distance depends on the property of the restriction enzyme(s) selected. For example, assuming the enzyme cuts x bases from the recognition sequence and generates y bases of adhesive end/overhang (e.g. for BsaI x = 1 and y = 4), then the distance (d) for the number of bases between opposing restriction enzyme recognition sequences of the maintaining composite element may be provided by the following equation: d = (2 * x) + y (e.g. d=6bp for BsaI). The distance (d) for the maintaining composite element may range from 5bp (e.g. when using EarI) to 24bp (e.g. when using BtgZI). In one description, the difference between the opposing restriction enzyme recognition sequences of the maintaining composite element is d, wherein d = (2 * x) + y, and wherein x is the number of base pairs the cut site of a given restriction enzyme is from its recognition sequence, and y is the length of the overhang that would be produced by the restriction enzyme.

In the alternative truncating composite element description, for example where the overhang produced by the restriction enzyme recognition sequence of the methylation-protectable restriction element may or may not be maintained, the opposing non-protectable restriction enzyme recognition sequence (such as for BsaI) may be distanced apart from the restriction enzyme recognition sequence (such as for BsaI) of the methylation-protectable restriction element by 2 base pairs. As above, the skilled person will recognise that such distance depends on the property of the restriction enzyme(s) selected. For example, assuming the enzyme cuts x bases from the recognition sequence and generates y bases adhesive end (for BsaI x = 1 and y = 4), the distance (d) for the number of bases between opposing restriction enzyme recognition sequences of the truncating composite element may be provided by the following equation: d = 2 * x (e.g. d=2bp for BsaI). The distance (d) for the truncating composite element may range from 2bp (e.g. when using BsaI) to 20bp (e.g. when using BtgZI). In one description, the difference between the opposing restriction enzyme recognition sequences of the truncating composite element is d, wherein d = 2 * x, and wherein x is the number of base pairs the cut site of a given restriction enzyme is from its recognition sequence.

Advantageously, the provision of a maintaining composite element facilitates one-pot DNA assembly, whereby multiple fragments of DNA may be assembled in a single one-pot reaction with a single restriction enzyme species. Advantageously, the provision of a truncating composite element facilitates scarless DNA assembly, whereby the overhang ends of a particular DNA fragment insert can be pre-determined to be adapted for ligation into a destination vector or provided in accordance with the native sequence of the DNA fragment of interest for joining with neighbouring fragments in the sequence.

In one description, the nucleic acid may comprise a circular nucleic acid, such as a vector, comprising two maintaining composite elements with a discard sequence therebetween, or a linearized version thereof with the discard sequence cut out. In another description, the nucleic acid may comprise a circular nucleic acid, such as a vector, comprising a maintaining composite element and a truncating composite element with a discard sequence therebetween, or a linearized version thereof with the discard sequence cut out. In another description, the nucleic acid may comprise a circular nucleic acid, such as a vector, comprising two truncating composite elements with a discard sequence therebetween, or a linearized version thereof with the discard sequence cut out. In another description, the nucleic acid may comprise a circular nucleic acid, such as a vector, comprising an insertional composite element and a truncating composite element with a discard sequence therebetween, or a linearized version thereof with the discard sequence cut out. In another description, the nucleic acid may comprise a circular nucleic acid, such as a vector, comprising an insertional composite element and a maintaining composite element with a discard sequence therebetween, or a linearized version thereof with the discard sequence cut out. In another description, the nucleic acid may comprise a circular nucleic acid, such as a vector, comprising two insertional composite elements with a discard sequence therebetween, or a linearized version thereof with the discard sequence cut out. In linearized nucleic acid embodiments, the discard sequence may have been cut out by restriction with the restriction enzyme that recognises the opposing non-protectable restriction enzyme sequence present on the discard sequence (i.e. the inner restriction enzyme recognition sequence).

In descriptions comprising two maintaining composite elements, the restriction enzyme recognition sequences of the two maintaining composite elements may be the same and/or the methylase recognition sequences of the two maintaining composite elements may be the same. In descriptions comprising two truncating composite elements, the restriction enzyme recognition sequences of the two truncating composite elements may be the same and/or the methylase recognition sequences of the two truncating composite elements may be the same. In descriptions comprising two insertional composite elements, the restriction enzyme recognition sequences of the two insertional composite elements may be the same and/or the methylase recognition sequences of the two insertional composite elements may be the same. In descriptions comprising a maintaining composite element and a truncating composite element, the restriction enzyme recognition sequences of the maintaining and truncating composite elements may be the same and/or the methylase recognition sequences of the maintaining and truncating composite elements may be the same. In descriptions comprising a maintaining composite element and an insertional composite element, the restriction enzyme recognition sequences of the maintaining and insertional composite elements may be the same and/or the methylase recognition sequences of the maintaining and insertional composite elements may be the same. In descriptions comprising a truncating composite element and an insertional composite element, the restriction enzyme recognition sequences of the truncating and insertional composite elements may be the same and/or the methylase recognition sequences of the truncating and insertional composite elements may be the same. For example, digestion of the nucleic acid to remove a discard sequence or DNA fragment of interest may be carried out by the same restriction enzyme. Additionally the methylation of the nucleic acid to protect all the methylase-protectable restriction enzyme recognition sites in the nucleic acid may be carried out by the same methylase.

In one description, the methylase comprises a type II DNA methylase. In another description, the methylase comprises a type I DNA methylase. In one description, the methylase comprises a single domain methylase without restriction enzyme activity, or a modified methylase having a non-functional restriction enzyme activity. For example, the modification may comprise modifying the N6 position of methyladenine. In one description, the DNA methylase recognition sequence comprises at least 4 base pairs. The DNA methylase may be active, such as optimally active, at about 37°C.

In one description, the opposing non-protectable restriction enzyme recognition sequence is not capable of being blocked by methylation with the methylase that recognises the DNA methylase recognition sequence of the methylation-protectable restriction element. In one description, the methylase that recognises the DNA methylase recognition sequence of the methylation-protectable restriction element may not recognise a sequence within or overlapping with the opposing non-protectable restriction enzyme recognition sequence. For example, the non-protectable opposing restriction enzyme recognition sequence may not overlap with or comprise a sequence that is recognised by the methylase that recognises DNA methylase recognition sequence of the methylation-protectable restriction element. The restriction enzyme recognition sequence of the methylation-protectable restriction element may be methylated, for example by the methylase that recognises the DNA methylase recognition sequence of the methylation-protectable restriction element. The methylase that recognises the DNA methylase recognition sequence of the methylation-protectable restriction element may be arranged to methylate a nucleotide within the sequence of the restriction enzyme recognition sequence of the methylation-protectable restriction element. The methylation may be capable of blocking the cutting of the DNA by the restriction enzyme that recognises the restriction enzyme recognition sequence of the methylation-protectable restriction element. In one description, the restriction enzyme recognition sequence of the methylation-protectable restriction element may become non-functional as a result of methylation of at least one of the nucleotides in the sequence. In one description, the methylated nucleotide may be an adenine, or the nucleotide arranged to be methylated may be an adenine. In another description, the methylated nucleotide may be a cytosine, or the nucleotide arranged to be methylated may be a cytosine. The methylation may be any one of methylation types selected from N4-methylcytosine (m4C), C5-methyylcytosine (m5C) or N6-methyladenine (m6A). The skilled person will recognize that the type of methylation provided should block/impair the overlapping restriction site function. Additionally, for *in vivo* methylation, the strain used to express the methylase may be deficient for modification-dependent restriction enzymes that may recognize the methylated bases, such as Mcr/Mrr family restriction enzymes. In one description, the methylation type may comprise N6-methyladenine (m6A).

In one description, the methylase may or may not methylate the outer-most bases of the restriction enzyme recognition sequence. For example in a restriction enzyme recognition sequence of 6bp, the bases 1 or 6 may not be methylated. In one description, the methylase may or may not methylate the 2^{nd} base of the restriction enzyme recognition sequence. In one description, the methylase may methylate position 3, 4, or 5 for 6bp restriction enzyme recognition sites. In one description, the methylase may methylate position 3, 4, 5, or 6 for restriction enzyme recognition sites.

In a description comprising two methylation-protectable restriction elements, the DNA methylase recognition sequences of each methylation-protectable restriction element may be recognised by the same methylase. The DNA methylase recognition sequence of the methylation-protectable restriction elements may comprise or consist of the same sequence.

In a description comprising two methylation-protectable restriction elements, the restriction enzyme recognition sequences of each methylation-protectable restriction element may be recognised by the same restriction enzyme species. Each restriction enzyme recognition sequence of the methylation-protectable restriction elements may comprise or consist of the same sequence. The two methylation-protectable restriction elements may comprise or consist of the same sequence.

The use of the same restriction enzyme recognition sequence advantageously provides that a single restriction enzyme species can be used in a DNA assembly reaction. The use of a single restriction enzyme species opens the possibility of less complex DNA assembly, because the presence of further restriction enzyme species in a reaction significantly increases the probability the more that the sequence will randomly/inadvertently contain a recognition and restriction cut site, causing a failure in the cloning procedure.

In one description, the methylation-protectable restriction element comprises or consists of a sequence according to any one of the overlapping methylation/restriction sites identified in Table 3 herein. In an alternative description, the methylation-protectable restriction element comprises or consists of a sequence according to any one of the overlapping methylation/restriction sites identified in Table 3 herein, excluding those which provide methylation of the first or last base in the restriction sequence. In one description, the methylation-protectable restriction element comprises or consists of the sequence GACNNGGTCTCNNNNN (BsaI/M.Osp807II - SEQ ID NO: 1). In another description, the methylation-protectable restriction element comprises or consists of the sequence GAAGACGCNNNNNN (BpiI/M2.NmeMC58II - SEQ ID NO: 2). In another description, the methylation-protectable restriction element comprises or consists of the sequence GAAGCTCTTCNNNN (LguI/M.XmnI - SEQ ID NO: 3).

In one description, the methylation-protectable and/or non-protectable restriction enzyme recognition sequence comprises or consists of a sequence according to any one of the restriction enzyme recognition sequences identified in Table 3 herein. In an alternative description, the methylation-protectable and/or non-protectable restriction enzyme recognition sequence comprises or consists of a sequence according to any one of the restriction enzyme recognition sequences identified in Table 3 herein, excluding those which provide for methylation of the first or last base in the restriction sequence. In one description, the methylation-protectable and/or non-protectable restriction enzyme recognition sequence comprises or consists of the sequence GGTCTC (BsaI - SEQ ID NO: 4). In another description, the methylation-protectable and/or non-protectable restriction enzyme recognition sequence comprises or consists of a sequence GAAGAC (BpiI - SEQ ID NO: 5). In another description, the methylation-protectable and/or non-protectable restriction enzyme recognition sequence comprises or consists of a sequence GCTCTTC (LguI - SEQ ID NO: 6).

In one description, the DNA methylase recognition sequence comprises or consists of a sequence according to any one of the type II DNA methylase recognition sequences identified in Table 3 herein. In an alternative description, the DNA methylase recognition sequence comprises or consists of a sequence according to any one of the type II DNA methylase recognition sequences identified in Table 3 herein, excluding those which provide methylation of the first or last base in the restriction sequence. In one description, the DNA methylase recognition sequence comprises or consists of the sequence GACNNNGTC (M.Osp807II - SEQ ID NO: 7). In another description, the DNA methylase recognition sequence comprises or consists of the sequence GACGC (M2.NmeMC58II - SEQ ID NO: 8). In another description, the DNA methylase recognition sequence comprises or consists of the sequence GAANNNNTTC (M.XmnI - SEQ ID NO: 9).

The restriction enzyme that recognizes the restriction enzyme recognition sequence of the methylation-protectable restriction element, and/or the opposing restriction enzyme recognition sequence, may be capable of cutting nucleic acid to leave at least a 2bp overhang/sticky end. Alternatively, the restriction enzyme that recognizes the restriction enzyme recognition sequence of the methylation-protectable restriction element, and/or the opposing restriction enzyme recognition sequence, may be capable of cutting nucleic acid to leave at least a 3bp overhang/sticky end. In another description, the restriction enzyme that recognizes the restriction enzyme recognition sequence of the methylation-protectable restriction element, and/or the opposing restriction enzyme recognition sequence, may be capable of cutting nucleic acid to leave a 3bp overhang/sticky end. In another description, the restriction enzyme that recognizes the restriction enzyme recognition sequence of the methylation-protectable restriction element, and/or the opposing restriction enzyme recognition sequence, may be capable of cutting nucleic acid to leave a 4bp overhang/sticky end. In another description, the restriction enzyme that recognizes the restriction enzyme recognition sequence of the methylation-protectable restriction element, and/or the opposing restriction enzyme recognition sequence, may be capable of cutting nucleic acid to leave a 2bp to 6bp overhang/sticky end. In another description, the restriction enzyme that recognizes the restriction enzyme recognition sequence of the methylation-protectable restriction element, and/or the opposing restriction enzyme recognition sequence, may be capable of cutting nucleic acid to leave a 2bp to 5bp overhang/sticky end. In another description, the restriction enzyme that recognizes the restriction enzyme recognition sequence of the methylation-protectable restriction element, and/or the opposing restriction enzyme recognition sequence, may be capable of cutting nucleic acid to leave a 3bp to 5bp overhang/sticky end. In another description, the restriction enzyme that recognizes the restriction enzyme recognition sequence of the methylation-protectable restriction element, and/or the opposing restriction enzyme recognition sequence, may be capable of cutting nucleic acid to leave a 4bp to 5bp overhang/sticky end.

In one description, the restriction enzyme comprises or consists of any one type IIS restriction enzyme identified in Table 3 herein. In another description, the type IIS restriction enzyme comprises or consists of BsaI, BpiI, or LguI.

In one description, the DNA methylase comprises or consists of any one type II DNA methylase identified in Table 3 herein. In an alternative description, the DNA methylase comprises or consists of any one type II DNA methylase identified in Table 3 herein, excluding those which provide methylation of the first or last base in the restriction sequence. In another description, the DNA methylase comprises or consists of M.Osp807II, M2.NmeMC58II, or M.XmnI.

In one description, the restriction enzyme and the DNA methylase comprise or consist of any one pairing of type IIS restriction enzymes and the type II DNA methylases identified in Table 3 herein. In one description, the restriction enzyme and the DNA methylase comprise or consist of the pairings BsaI/M.Osp807II, BpiI/M2.NmeMC58II, or LguI/M.XmnI.

In one description, the restriction enzyme recognition sequence and the DNA methylase recognition sequence comprise or consist of any one pairing of type IIS restriction enzyme recognition sequences and the type II DNA methylase recognition sequences identified in Table 3 herein. In an alternative description, the restriction enzyme recognition sequence and the DNA methylase recognition sequence comprise or consist of any one pairing of type IIS restriction enzyme recognition sequences and the type II DNA methylase recognition sequences identified in Table 3 herein, excluding those which provide methylation of the first or last base in the restriction sequence.

In a description comprising two methylation-protectable restriction elements (for example with a discard sequence therebetween), the overhang/sticky end produced by cutting with the restriction enzyme at the first methylation-protectable restriction element may be different in sequence than the overhang/sticky end produced by cutting with the restriction enzyme at the second methylation-protectable restriction element. The skilled person will be familiar with providing different overhangs for directional DNA assembly/cloning or controlling which end of the nucleic acid is ligated to an insert/fragment of interest.

In one description involving a maintaining composite element, the nucleic acid may comprise or consist of the sequence of GACNNGGTCTCNNNNNNGAGACC (SEQ ID NO: 10). In another description involving a maintaining composite element, the nucleic acid may comprise or consist of the sequence of GAAGACGCNNNNNNGTCTTC (SEQ ID NO: 11). In another description involving a maintaining composite element, the nucleic acid may comprise or consist of the sequence of GAAGCTCTTCNNNNNGAAGAGC (SEQ ID NO: 12). N may mean A, C, T or G. Other sequences may be provided according to the combined/overlapping restriction enzyme and methylase recognition sequences provided in table 3, wherein following the series of N bases, the sequence further comprises the palindromic/reverse-complementary sequence of the restriction enzyme recognition sequence.

In one description involving two maintaining composite elements, the nucleic acid may comprise or consist of the sequence of GACNNGGTCTCNNNNNNGAGACC(N^{x})GGTCTCNNNNNNGAGACCNNGTC (SEQ ID NO: 13). In another description involving two maintaining composite elements, the nucleic acid may comprise or consist of the sequence of GAAGACGCNNNNNNGTCTTC(N^{x})GAAGACNNNNNNGCGTCTTC (SEQ ID NO: 14). In another description involving two maintaining composite elements, the nucleic acid may comprise or consist of the sequence of GAAGCTCTTCNNNNNGAAGAGC(N^{x})GCTCTTCNNNNNGAAGAGCTTC (SEQ ID NO: 15). N may mean A, C, T or G. (N^{x}) may mean any number of A, C, T or G, or between 0bp and 300kbp of A, C, T, or G.

The skilled person will understand that for some restriction enzyme recognition sequences, such as for LguI, the number of nucleotides (N^{x}) between two maintaining composite elements could be negative with the two inner nonprotectable restriction enzyme recognition sites overlapping. Therefore, in another description involving two maintaining composite elements, the nucleic acid may comprise or consist of the sequence of GAAGCTCTTCNNNNNGAAGA**GC**TCTTCNNNNNGAAGAGCTTC (SEQ ID NO: 16) (the overlapping nucleotides are shown in bold and underlined).

In one description involving a truncating composite element, the nucleic acid may comprise or consist of the sequence of GACNNGGTCTCNNGAGACC (SEQ ID NO: 17). In one description involving a truncating composite element, the nucleic acid may comprise or consist of the sequence of GAAGCTCTTCNNGAAGAGC (SEQ ID NO: 18). N may mean A, C, T or G. N may mean A, C, T or G. Other sequences may be provided according to the combined/overlapping restriction enzyme and methylase recognition sequences provided in table 3, wherein following the series of N bases, the sequence further comprises the palindromic/reverse-complementary sequence of the restriction enzyme recognition sequence, and wherein the number of N bases between the restriction recognition sequences is reduced by the number overhang bases produced by the selected restriction enzyme.

In one description involving a maintaining and truncating composite element, the nucleic acid may comprise or consist of the sequence of GACNNGGTCTCNNNNNNGAGACC(N^{x})GGTCTCNNGAGACCNNGTC (SEQ ID NO: 19). In one description involving a maintaining and truncating composite element, the nucleic acid may comprise or consist of the sequence of GAAGCTCTTCNNNNNGAAGAGC(N^{x})GCTCTTCNNGAAGAGCTTC (SEQ ID NO: 20). N may mean A, C, T or G. (N^{x}) may mean any number of A, C, T or G, or between 0bp and 300kbp of A, C, T, or G.

The skilled person will understand that for some restriction enzyme recognition sequences, such as for LguI, the number of nucleotides (N^{x}) between a maintaining and truncating composite element could be negative with the two inner nonprotectable restriction enzyme recognition sites overlapping. Therefore, in another description involving a maintaining and truncating composite element, the nucleic acid may comprise or consist of the sequence of GAAGCTCTTCNNNNNGAAGA**GC**TCTTCNNGAAGAGCTTC (SEQ ID NO: 21) (the overlapping nucleotides are shown in bold and underlined).

In one description involving a maintaining and truncating composite element, the nucleic acid may comprise or consist of the sequence of GACNNGGTCTCNNGAGACC(N^{x})GGTCTCNNNNNNGAGACCNNGTC (SEQ ID NO: 22). In one description involving a maintaining and truncating composite element, the nucleic acid may comprise or consist of the sequence of GAAGCTCTTCNNGAAGAGC(N^{x})GCTCTTCNNNNNGAAGAGCTTC (SEQ ID NO: 23). N may mean A, C, T or G. (N^{x}) may mean any number of A, C, T or G, or between 0bp and 300kbp of A, C, T, or G. In another description involving a maintaining and truncating composite element, the nucleic acid may comprise or consist of the sequence of GAAGCTCTTCNNGAAGA**GC**TCTTCNNNNNGAAGAGCTTC (SEQ ID NO: 24) (the overlapping nucleotides are shown in bold and underlined).

In one description involving two truncating composite elements, the nucleic acid may comprise or consist of the sequence of GACNNGGTCTCNNGAGACC(N^{x})GGTCTCNNGAGACCNNGTC (SEQ ID NO: 25). In one description involving two truncating composite elements, the nucleic acid may comprise or consist of the sequence of GAAGCTCTTCNNGAAGAGC(N^{x})GCTCTTCNNGAAGAGCTTC (SEQ ID NO: 26). N may mean A, C, T or G. (N^{x}) may mean any number of A, C, T or G, or between 0bp and 300kbp of A, C, T, or G.

The skilled person will understand that for some restriction enzyme recognition sequences, such as for LguI, the number of nucleotides (N^{x}) between two truncating composite elements could be negative with the two inner nonprotectable restriction enzyme recognition sites overlapping. Therefore, in another description involving two truncating composite elements, the nucleic acid may comprise or consist of the sequence of GAAGCTCTTCNNGAAGA**GC**TCTTCNNGAAGAGCTTC (SEQ ID NO: 27) (the overlapping nucleotides are shown in bold and underlined).

The nucleic acid may be isolated nucleic acid. In one description, the nucleic acid may be synthetic (i.e. not found in nature). In one description, the nucleic acid may be isolated or derived from a bacterial strain, such as *E.coli,* that expresses a DNA methylase that recognises (and methylates) the methylase recognition sequence of the methylation-protectable restriction element. For example, a bacterial strain, such as *E.coli,* that expresses any one type II DNA methylase identified in Table 3 herein. In one description the nucleic acid may be isolated or derived from a bacterial strain, such as *E.coli,* that expresses M.Osp807II, M2.NmeMC58II, or M.XmnI. The expressed DNA methylase may be functional. The bacterial strain may be genetically modified/engineered to express such DNA methylase.

The nucleic acid may be a vector, such as a cloning vector. The skilled person will be familiar with various cloning vectors, which may include, for example, a replication origin, a selectable marker, a reporter gene, expression elements, or combinations thereof. The vector may comprise nucleic acid that comprises a DNA element that supports the replication of DNA that contains it (e.g. a replication origin) and a selection marker (e.g. an antibiotic selection marker). The vector may be a high or low copy number vector. In one description, the vector is a high copy number vector. The skilled person will be familiar with cloning methods and materials/vectors, for example as provided in Green, M.R., Sambrook, J. Molecular Cloning: A Laboratory Manual. 4th. Cold Spring Harbor Laboratory Press, Cold Spring Harbor, NY; 2012.

In linearized versions of the nucleic acid, the nucleic acid may not comprise more than two copies/sites of the restriction enzyme recognition sequence.

Also described is a method of assembling DNA comprising:
providing a linearised methylated nucleic acid as described herein, wherein the methylation-protectable restriction element is methylated with the DNA methylase;
providing two or more DNA fragments of interest for assembly with the linearised methylated nucleic acid, wherein a first DNA fragment comprises a complementary overhang for ligation with a first end of the linearised methylated nucleic acid, and a second DNA fragment comprises complementary overhangs for ligation with the other/second end of the linearised methylated nucleic acid; and
   (i) the first and second DNA fragments further comprise complementary overhangs for ligation with each other; or
   (ii) the first and second DNA fragments further comprise complementary overhangs for ligation with one or more further DNA fragments having complementary overhangs, such that ligating the DNA fragments and the linearised methylated nucleic acid with a DNA ligase would result in a single assembled DNA molecule;
ligating the DNA fragments and linearised methylated nucleic acid with a ligase to form a single assembled DNA molecule comprising the sequence of the assembled DNA fragments flanked by the restriction enzyme recognition sequences of the methylation-protectable restriction elements.

The single assembled DNA molecule may be circular, such as a circular DNA vector.

Advantageously, further cutting of the single assembled DNA molecule by the restriction enzyme that recognises the restriction enzyme recognition sequences of the methylation-protectable restriction elements is blocked by the methylation of the restriction enzyme recognition sequences.

The linearised nucleic acid may be provided by providing a nucleic acid as described herein in the form of a circular destination vector comprising two methylated methylation-protectable restriction elements and a discard sequence therebetween, wherein the method comprises the step of cutting the circular destination vector with restriction enzymes that recognise the opposing non-protectable restriction enzyme recognition sequences in the discard sequence, thereby leaving a linearised nucleic acid having overhangs defined by the restriction enzymes. The restriction enzymes may be the same species. The restriction enzyme recognition sequences of the methylated methylation-protectable restriction elements may comprise the same sequence. In one description, the restriction enzyme recognition sequences of the methylated methylation-protectable restriction elements and the opposing non-protectable restriction enzyme recognition sequences may comprise the same sequence.

The restriction enzyme and methylase (or recognition sequences thereof) used in the method may be selected in accordance with the restriction enzyme and methylases (or recognition sequences thereof) provided for the nucleic acid description herein.

The circular destination vector may be purified/isolated from a bacterial strain that expresses the DNA methylase that recognises the DNA methylase recognition sequence of the methylation-protectable restriction element. In another description, the circular or linearised destination vector may be methylated by the DNA methylase *in vitro.*

The two or more DNA fragments of interest for assembly with the nucleic acid may be provided in circular donation vectors, wherein the method comprises the step of cutting the circular donation vectors to release the DNA fragments of interest. The cutting of the donor vectors may be with a restriction enzyme that leaves complementary overhangs for ligation with the linearised methylated nucleic acid. The cutting of the donor vectors may be with a restriction enzyme that leaves complementary overhangs for ligation with the linearised methylated nucleic acid and with another DNA fragment of interest for insertion. The cutting of the donor vectors may be with a restriction enzyme that leaves complementary overhangs for ligation with two other DNA fragments of interest for insertion.

The restriction enzyme for cutting the circular donation vectors may comprise a type IIS restriction enzyme. The restriction enzyme for cutting the circular donation vectors may comprise the same restriction enzyme, or a restriction enzyme that recognises the same sequence, as the restriction enzyme used to cut the circular destination vector. The complementary overhangs of the DNA fragments of interest for ligation with the linearised nucleic acid may be defined by the restriction enzyme.

Advantageously, the provision of the same restriction enzyme recognition sequence for the methylation-protectable restriction elements, the opposing restriction enzyme recognition sequence on the discard sequence, and the restriction enzyme recognition sequences on the donor vectors provides that a single type of restriction enzyme can be used. This allows for reduced cost/complexity in removing internal type IIS restriction site from DNA parts, because there are less restriction sites to remove. This makes assembly of longer sequence easier and less costly. Furthermore, the invention allows for a so-called "one-pot" reaction, whereby the restriction and ligation can be carried out in one pot in a single step.

Therefore, the method may combine the steps of restricting and ligating. In particular, the circular destination vector, the donor vectors, the restriction enzyme and the ligase may be provided in the same composition.

Following assembly of the single assembled DNA molecule, the method may further comprise the step of transforming the single assembled DNA molecule into a bacterial strain that does not express the DNA methylase that recognises the DNA methylase recognition sequence of the methylation-protectable restriction element.

Advantageously, transforming the single assembled DNA molecule into a bacterial strain that does not express the DNA methylase that recognises the DNA methylase recognition sequence of the methylation-protectable restriction element results in the cloning and production of a nucleic acid sequence, such as a vector, that comprises a non-methylated (i.e. non-blocked) restriction enzyme recognition sequence to allow subsequent restriction reactions.

The method may further comprise a multistage assembly process in order to form assembled DNA fragments or vectors of greater sequence length. For example the assembled DNA, which is the product of the method, may be used further for a higher order assembly. For example, the method may comprise a second round of DNA assembly according to the description herein wherein the DNA fragments of interest for assembly with the linearised methylated nucleic acid may comprise assembled DNA from a first/earlier DNA assembly round according to the description herein.

Providing multiple rounds of DNA assembly to form larger fragments of DNA advantageously reduces the number of adapter/overhang sequences that are necessary. The success rate of DNA assembly is also increased with fewer DNA fragments to be assembled. Additionally, it can be easier to screen for the successfully assembled DNA with fewer fragments assembled in a single reaction.

### Scarless DNA assembly method

According to another aspect of the present invention there is provided a method of scarless DNA assembly of DNA fragements in accordance with claim 7 herein.

Described herein, there is provided a method of scarless DNA assembly of DNA fragments comprising the steps of:
(A) providing a first linearised methylated nucleic acid as described herein, having a maintaining composite element at one end and a truncating composite element at the other end, wherein the maintaining and truncating composite elements are methylated with the DNA methylase;
   providing a first DNA fragment for assembly having overhang ends that are adapted to ligate to the overhang ends of the first linearised methylated nucleic acid;
   ligating the first DNA fragment for assembly and first linearised methylated nucleic acid with a ligase to form a first methylated intermediate vector;
   transforming the first methylated intermediate vector into a bacterial strain that does not express the DNA methylase(s) that recognises either of the DNA methylase recognition sequence(s) of the maintaining and truncating composite elements, thereby forming a first intermediate vector that is not methylated;
   isolating the first intermediate vector;
(B) providing a second linearised methylated nucleic acid as described herein, having a maintaining composite element at one end and a truncating composite element at the other end, wherein the maintaining and truncating composite elements are methylated with the DNA methylase, and wherein the overhang provided by the maintaining composite element of the first linearised methylated nucleic acid is different in sequence to the overhang provided by the methylation-protectable restriction element of the second linearised methylated nucleic acid;
   providing a second DNA fragment for assembly having overhang ends that are adapted to ligate to the overhang ends of the second linearised methylated nucleic acid;
   ligating the second DNA fragment for assembly and second linearised methylated nucleic acid with a ligase to form a second methylated intermediate vector;
   transforming the second methylated intermediate vector into a bacterial strain that does not express the DNA methylase(s) that recognises either of the DNA methylase recognition sequence(s) of the maintaining and truncating composite elements, thereby forming a second intermediate vector that is not methylated;
   isolating the second intermediate vector;
(C) cutting the first intermediate vector with a restriction enzyme that recognises the restriction enzyme recognition sequence of the maintaining composite element and a restriction enzyme that recognises the restriction enzyme recognition sequence of the truncating composite element, thereby forming a first adapted DNA fragment insert that comprises a maintained-overhang sequence that is determined by the maintaining composite element and an opposing native-overhang sequence that is determined by the native sequence of the first DNA fragment for assembly;
(D) cutting the second intermediate vector with a restriction enzyme that recognises the restriction enzyme recognition sequence of the maintaining composite element and a restriction enzyme that recognises the restriction enzyme recognition sequence of the truncating composite element, thereby forming a second adapted DNA fragment insert that comprises a maintained-overhang sequence that is determined by the maintaining composite element and an opposing native-overhang sequence that is determined by the native sequence of the second DNA fragment for assembly;
   wherein
   (i) the first and second adapted DNA fragments are end fragments wherein their native-overhang sequences are complementary, such that they are arranged to ligate together; or
   (ii) one or more middle DNA fragments for assembly are provided wherein the first and second adapted DNA fragments are respective end fragments in the assembly, and the one or more middle DNA fragments are arranged to be ligated between the first and second adapted DNA fragments via complementary native-overhang sequences;
further comprising the step of ligating together, with a ligase, the first and second adapted DNA fragments, or the first and second adapted DNA fragments and one or more middle DNA fragments, to form an assembled DNA fragment having maintained-overhangs at each end.

The one or more middle DNA fragments may comprise native-overhang sequences at both ends that are determined by their native sequence. In a description comprising a single middle DNA fragment for assembly, the middle DNA fragment may comprise a first native-overhang sequence that is complementary to the native-overhang of the first adapted DNA fragment and a second native-overhang sequence that is complementary to the native-overhang of the second adapted DNA fragment.

In a description comprising a two or more middle DNA fragments for assembly, the middle DNA fragments may each comprise native-overhang sequences that are complementary to the native-overhang of a neighbouring middle DNA fragment, such that they can be ligated together in a pre-determined order. The first and last middle DNA fragments in a sequence will be arranged to ligate to the respective first adapted DNA fragment and second adapted DNA fragment via complementary native-overhang sequences.

In one description, a middle DNA fragment for assembly may be provided by
providing a further linearised methylated nucleic acid as described herein, wherein the linearised methylated nucleic acid comprises a truncating composite element at each end, wherein the truncating composite elements are methylated with the DNA methylase;
providing an adapted middle DNA fragment for assembly having overhang ends that are adapted to ligate to the overhang ends of the linearised methylated nucleic acid;
ligating the adapted middle DNA fragment for assembly and linearised methylated nucleic acid with a ligase to form a methylated intermediate vector;
transforming the methylated intermediate vector into a bacterial strain that does not express the DNA methylase(s) that recognises either of the DNA methylase recognition sequence(s) of the truncating composite elements, thereby forming an intermediate vector that is not methylated;
isolating the intermediate vector;
cutting the intermediate vector with restriction enzymes that recognise the restriction enzyme recognition sequence of the truncating composite elements, thereby forming a middle DNA fragment insert that comprises native-overhang sequences at each end that are determined by the native sequence of the middle DNA fragment for assembly.

The DNA fragments for assembly may comprise double stranded DNA comprising the overhangs/stick ends at each end. A DNA fragment for assembly may be prepared by PCR from a template, by gene synthesis, or by annealing of two oligonucleotides. Such processes, e.g. PCR or gene synthesis, may generate blunt ended double stranded DNA. For assembly starting with PCR product, an appropriate adaptor sequence containing the restriction site may be included in the PCR primer to trim the DNA to generate the overhangs for cloning into the intermediate vectors. For descriptions comprising the use of annealed oligonucleotides there are more choices. The sequence design could be the same as for PCR/gene synthesis with restriction sites at both ends for trimming with a restriction enzyme to generate the overhangs. Alternatively, two annealed oligos may form the overhangs themselves.

In one description, the DNA fragments for assembly, such as the first DNA fragment for assembly, the second DNA fragment for assembly, and the one or more adapted middle DNA fragments for assembly, may be provided by PCR from a template sequence (i.e. the DNA fragments for assembly may be PCR products). The template sequence may provide the full sequence that is desired to be cloned as the assembled DNA fragment. The DNA fragments for assembly may be replicated, for example by PCR, from different sections of the template, and generated neighbouring PCR products may partially overlap in sequence (i.e. the sequence near the end of one PCR product may comprise the same sequence near the start of the next PCR product for assembly). The overlap may be partial, but sufficient in length to provide a complementary sticky end. In one description, the overlap sequence within one PCR product (DNA fragment for assembly) and the overlap in the next neighbouring PCR product (DNA fragment for assembly) may be at least 2 base pairs in length. In another description, the overlap sequence within one PCR product (DNA fragment for assembly) and the overlap in the next neighbouring PCR product (DNA fragment for assembly) may be at least 3 base pairs in length. In another description, the overlap sequence within one PCR product (DNA fragment for assembly) and the overlap in the next neighbouring PCR product (DNA fragment for assembly) may be 3-6 base pairs in length. In another description, the overlap sequence within one PCR product (DNA fragment for assembly) and the overlap in the next neighbouring PCR product (DNA fragment for assembly) may be 3-5 base pairs in length. In another description, the overlap sequence within one PCR product (DNA fragment for assembly) and the overlap in the next neighbouring PCR product (DNA fragment for assembly) may be 4-5 base pairs in length. In another description, the overlap sequence within one PCR product (DNA fragment for assembly) and the overlap in the next neighbouring PCR product (DNA fragment for assembly) may be 3-4 base pairs in length. In another description, the overlap sequence within one PCR product (DNA fragment for assembly) and the overlap in the next neighbouring PCR product (DNA fragment for assembly) may be 4 base pairs in length.

PCR primers may be arranged to amplify the partially overlapping sections of template DNA in order to form the DNA fragments for assembly that would comprise complementary overhangs. The PCR reaction may add adaptor sequences containing the restriction recognition sequence (i.e. the universal adapter sequence, for example the restriction enzyme recognition sequence may comprise the same sequence as the restriction enzyme recognition sequence of the maintaining composite element and/or truncating composite element) plus the sequence for the overhang that would be complementary to the overhang of the neighbouring/consecutive fragment for assembly. The overhang comprising the sequence that overlaps, for example by 4bp, between consecutive/neighbouring fragments may be formed following digestion with the restriction enzyme (i.e. the overlap among PCR product is referring to the sequence, for example of 4bp, that inside the whole adaptor sequence comprising the restriction enzyme recognition sequence).

The PCR primers may further provide the adapter sequences for the DNA fragments, which form the complementary overhang for ligation with the linearised methylated nucleic acids (such as the first linearised methylated nucleic acid, second linearised methylated nucleic acid or further linearised methylated nucleic acid) and formation of the intermediate vectors. The PCR primers may further provide a restriction enzyme recognition sequence that is arranged to direct the restriction enzyme to cut the PCR product to form the adapter sequences. The restriction enzyme recognition sequence provided by the PCR primers may be a restriction enzyme recognition sequence. The restriction enzyme recognition sequence may comprise the same sequence as the restriction enzyme recognition sequence of the maintaining composite element and/or truncating composite element.

In one description, the adapted middle DNA fragment comprises a region of overlapping native sequence that is the same sequence as a native overhang sequence provided in a consecutive/neighbouring DNA fragment intended for ligation. For example, where neighbouring/consecutive DNA fragments for assembly with each other are encoded from a template, they may each comprise an overlapping/same sequence region from the template, which would form the complementary native overhangs between the two neghbouring/consecutive sequences in a subsequent ligation. In one description, the adapted middle DNA fragment comprises two regions of overlapping native sequence, that are arranged to form the native-overhangs of the middle DNA fragment once cut by the recognising restriction enzyme.

The method may further comprise the step of providing a linearised destination vector for insertion of the assembled DNA fragments. The linearised destination vector may comprise respective overhang sequences that are complementary to the maintained overhangs of the assembled DNA fragment. The overhang sequences of the linearised destination vector may comprise a first overhang that is complementary to the maintained-overhang sequence of the first adapted DNA fragment and a second overhang that is complementary to the second the maintained-overhang sequence of the second adapted DNA fragment.

The linearised destination vector may be provided by cutting a circular destination vector with the restriction enzyme(s) that are arranged to provide the same complementary overhangs as the assembled DNA fragments. For example, the linearised destination vector may be provided by cutting a circular destination vector with the restriction enzyme(s) that recognise the restriction enzyme recognition sequences of the maintaining and/or truncating composite element. The linearised destination vector may be a cloning vector.

In one description, the maintaining composite element comprises the same sequence in all intermediate vectors that comprise the maintaining composite element. In one description, the truncating composite element comprises the same sequence in all intermediate vectors. In one description, the maintaining composite element comprises the same restriction enzyme recognition sequence as the truncating composite element. In one description, the restriction enzyme recognition sequences of the maintaining composite elements and/or the truncating composite elements of the intermediate vectors are the same. In particular the same restriction enzyme species may be used for restriction of the intermediate vectors, and optionally the destination vector.

The vectors used in the scarless assembly method as described herein may be high copy number vectors. For example the first and/or second linearised methylated nucleic acid may comprise a linearised methylated high copy number vector.

In one description, the DNA assembly method described herein is carried out in a single composition (i.e. so called "one-pot" process). For example, the DNA assembly process (including digestion with restriction enzyme and ligation) may be carried out in a single composition containing the restriction enzyme and DNA ligase using circular insert plasmids or linearized insert fragments and circular intermediate and/or destination vectors, or linearized versions thereof.

In one description the methylation of the nucleic acid is carried out in a bacterial strain that is modified to expresses the methylase that recognises the methylase recognition sequence. In one description the methylation of the nucleic acid is carried out *in vitro.*

Also described is a modified bacterial strain that is modified to express a DNA methylase described herein.

The modified bacterial strain that is modified to express a DNA methylase may be an *E. coli* strain. The modification may comprise insertion of the methylase gene in the *arsB* locus of *E. coli* genome. The modified bacterial strain that is modified to express a DNA methylase may be formed by assembly of a vector with a methylase expression cassette followed by an antibiotic selection cassette, such as a zeocin antibiotic selection cassette, and flanked by homologous sequence from the *arsb* gene of *E coli,* followed by PCR using this as a template to generate linear DNA containing the above element, and recombineering with this fragment followed by selection with the antibiotic, such as zeocin, to generate bacteria strains with the methylase expression cassette and selection marker stably inserted into the *arsB* locus of *E. coli* genome.

Also described is the use of a modified bacterial strain that is modified to express a DNA methylase described herein, for manufacturing a nucleic acid molecule as described herein.

The modified bacterial strain may be *E. coli.*

Also described is a composition comprising the nucleic acid according to the description herein. The composition may comprise buffer.

Also described is a kit comprising one or more nucleic acids according to the description herein.

The kit may further comprise a restriction enzyme and/or a ligase, such as T4 DNA ligase. Additionally or alternatively, the kit may comprise a modified bacterial strain that is modified to express a DNA methylase according to the description herein and/or a DNA methylase.

Additionally or alternatively, the kit may comprise one or more buffer solutions. Additionally or alternatively, the kit may comprise antibiotics for clone selection.

One or more, or all, of the nucleic acid, buffer, bacterial strain, restriction enzyme, ligase, and methylase may be provided in a container, such as an Eppendorf tube.

Also described is a host cell comprising nucleic acid according to the description herein.

Also described is the use of the nucleic acid according to the description herein for assembling DNA fragments of interest, optionally wherein the use is *in vitro.*

Reference to the term "scarless" or "scarless assembly" herein is intended to refer to a sequence of DNA that has been assembled from multiple sequences, wherein the joint between the sequences does not comprise artefacts, such as unwanted base pairs, of the restriction and ligation of the DNA assembly process. For example, scarless assembly of DNA does not introduce additional sequence into the assembled DNA fragment. In some literatures it is also called seamless DNA assembly. In one example, an adapter sequence designed to provide complementary sticky ends to facilitate the joining of DNA fragments may be considered a "scar" between the joined sequences.

Reference to the term "adapter" herein is intended to refer to a sequence designed to provide complementary sticky ends to facilitate the joining of DNA fragments.

Reference to the term "overlap" or "overlapping" in the context of overlapping recognition sequences is intended to refer to at least part of the recognition sequence of one enzyme (for example any nucleotide other than N in a recognition sequence) being in a position of the nucleotide sequence that specifies the recognition sequence of another enzyme. (i.e. overlapping sequences share at least some common nucleotides).

Reference to the term "native" in the context of an overhang/sticky end sequence is intended to refer to the natural, non-adapted, sequence of the DNA fragment of interest to be inserted or joined to the nucleic acid. For example, the sequence will not be designed or amended relative to the sequence of the template DNA of interest. This is in contrast with overhangs determined by the vector sequence to facilitate DNA assembly, which might be introduced as a scar into the assembled sequence.

Reference to a "single assembled DNA molecule" is intended to refer to a single type of molecule, and is not intended to refer to the number of copies of the same molecule. In particular, in any given reaction or composition, there may be a plurality of copies of the single assembled DNA molecule.

Reference to "high copy number" plasmids/vectors may refer to those with a copy number of 100 or more copies per cell, including but not limiting to plasmids with replication of origins such as 1. mutated pMB1 replication origin from plasmid pUC plasmids (Vieira and Messing 1982. Gene 19:259-268; Vieira and Messing 1987. Methods Enzymol. 153:3-11; Messing 1983. Methods Enzymol. 101:20-78; Lin-Chao et al. 1992. Mol. Microbiol. 6:3385-3393); and 2. Co1E1-derived replication origin from pBluescript plasmids (https://www.chem-agilent.com/pdf/strata/212205.pdf - pBluescript II Phagemid Vectors, Instruction Manual, Catalog #212205, #212206. #212207 and #212208, Revision A.01). The replication origin carried by pbluescript is from pUC with a single nucleotide mutation. Particular guides on low and high copy plasmid/vector regulation can be found in Molecular Cloning A Laboratory Manual 3rd edition (2001), Joseph Sambrook and David W Russell, volume 1 page 1.4 Table 1-1 and volume 1 page 4.2 Table 4-1.

Reference to "low copy number" plasmids/vectors may refer to those with a copy number of less than 100 copies per cell, including but not limiting to plasmids with replication of origin such as 1. p15A replication origin from pACYC plasmids (Chang and Cohen 1978. J. Bacteriol. 134:1141-1156); 2. pSC101 replication origin from pSC101 plasmids (Stoker et al. 1982. Gene 18:335-341); 3. F replication origin from F plasmids (Kim et al. 1996. Genomics 34:213-218; Asakawa et al. 1997. Gene 191: 69-79); 4. pMB1 replication origin from pBR322 plasmids (Bolivar et al. 1977. Gene 2:95-113); and 5. Co1E1 replication origin from Co1E1 plasmid (Kahn et al. 1979. Methods Enzymol. 68:268-280)

The skilled person will understand that optional features of one description may be applicable, where appropriate, to other descriptions.

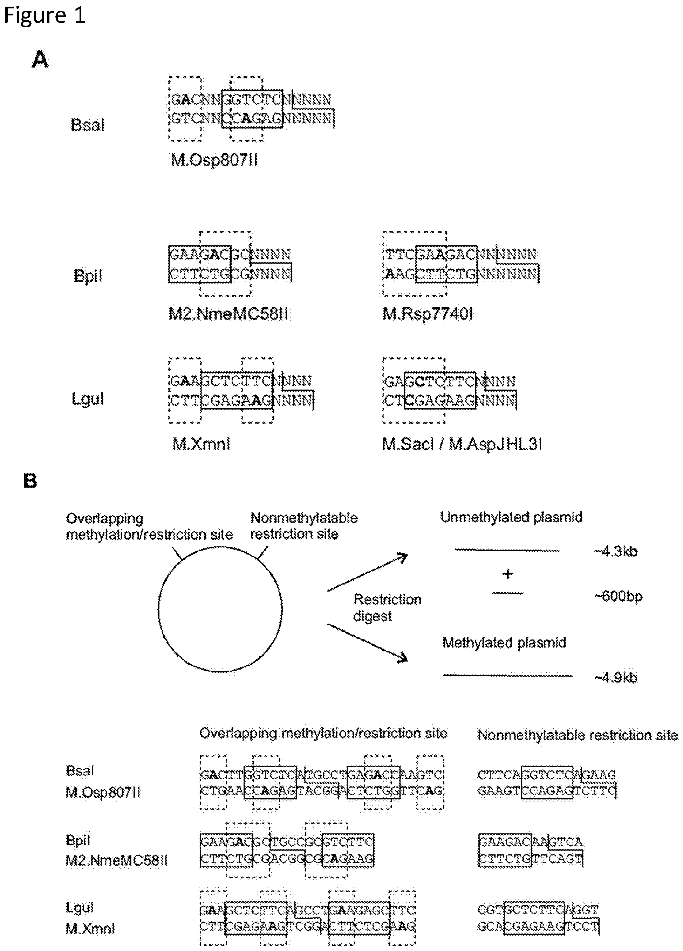

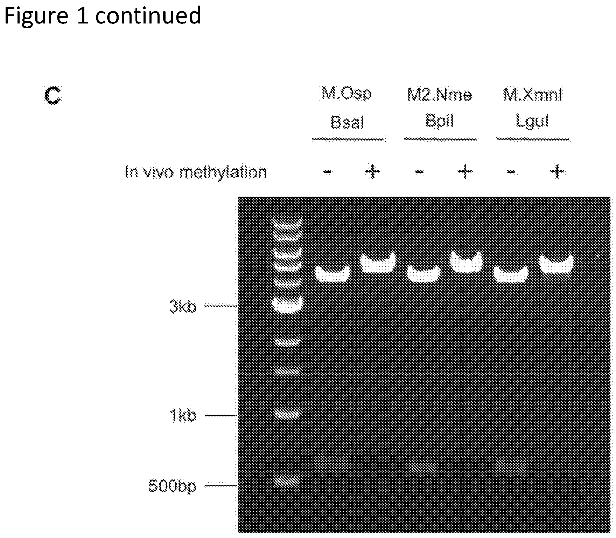

**Figure 1****. Identification of methylases that block overlapping methylation/restriction sites**
   A. Diagram of overlapping methylation/restriction sites for the initial screening. Restriction enzyme recognition sites are boxed in solid lines. The adhesive end generated by the restriction enzyme is shown by a solid line. Methylation enzyme recognition sites are boxed in dashed lines, and methylated bases are in bold font. All the methylases listed modify N6-adenine, except M.SacI and M.AspJHL3I, which modify C5-cytosine and N4-cytosine respectively.
   B. Experimental designs to test blocking of overlapping methylation/restriction sites by *in vivo* methylation. For each methylase/restriction enzyme combination tested, the test plasmid contains a head-to-head overlapping methylation/restriction site and a nonmethylatable restriction site. Restriction digest of test plasmid prepared from normal strain would result in restriction at both sites, and releases a ~600bp fragment from the ~4.3kb vector backbone. Restriction digest of test plasmid prepared from methylase-expressing strain would result in a single ~4.9kb band, due blocking of overlapping methylation/restriction sites by *in vivo* methylation. The restriction sites of test plasmid for each restriction enzyme are shown, with the restriction site boxed in solid line, methylation sequence boxed in dashed line, and methylated bases in bold. The head-to-head arrangement of overlapping methylation/restriction site allows assaying of nickase activity in the same assay. C. Agarose gel electrophoresis analysis of test plasmid for each overlapping methylase/restriction enzyme combination prepared in normal strain or strain expressing DNA methylase and digested with corresponding type IIS restriction enzymes. The combinations tested are BsaI with M.Osp807II (M.Osp) using test plasmid pMOP _BsaINC, BpiI with M2.NmeMC58II (M2.Nme) using test plasmid pMOP_BpiINC, and LguI with M.XmnI using test plasmid pMOP_LguINC. Test conditions are 60fmol test plasmid digested using 5U BsaI or BpiI, or 2.5U LguI in 10µl reaction at 37°C for 1h. Result shows that each of the methylases tested results in successful blocking through *in vivo* methylation of a restriction site for the corresponding type IIS restriction enzyme when the methylase recognition site overlaps the restriction enzyme site. The data shown represents results from three independent experiments.

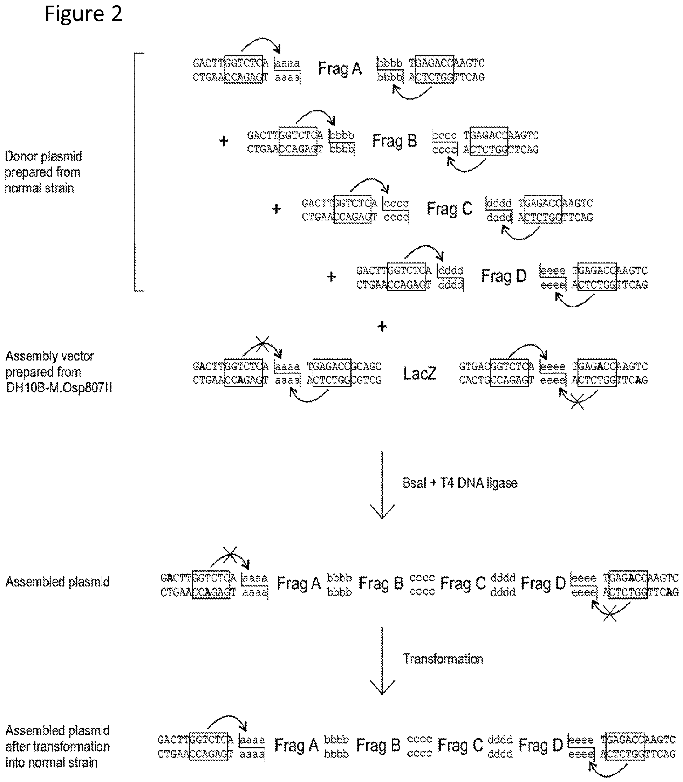

**Figure 2****. BsaI-M.Osp807II based Metclo system**
   The donor plasmids contain DNA fragments to be assembled (Frag A-D) flanked by BsaI sites that generate compatible adhesive ends (labelled aaaa-eeee). The BsaI sites overlap with the M.Osp807II methylase recognition sequence. Donor plasmids were prepared from a normal strain that lacks M.Osp807II methylase activity. As a result the BsaI sites are not methylated and so the insert DNA fragments can be released by BsaI digestion. The assembly vector contains a LacZ selection marker flanked by head-to-head BsaI sites. The outside pair of BsaI sites closer to the vector backbone overlap with M.Osp807II methylation sequence, whereas the inside pair do not. Preparation of the assembly vector in M.Osp807II-expressing DH10B strain results in blocking of the outside pair of BsaI sites specifically. The LacZ fragment could still be released by BsaI through the action on the inside pair of BsaI sites. Following a one-pot reaction using BsaI and T4 DNA ligase, ligation among compatible adhesive ends results in ordered assembly of DNA fragments to the assembly vector backbone. The assembled fragment in the assembled plasmid is flanked by blocked BsaI sites refractory to BsaI digestion. Following transformation into normal strain that lacks M.Osp807II activity, methylation of the flanking restriction sites is lost, and the assembled fragment could be released by BsaI for next stage assembly.

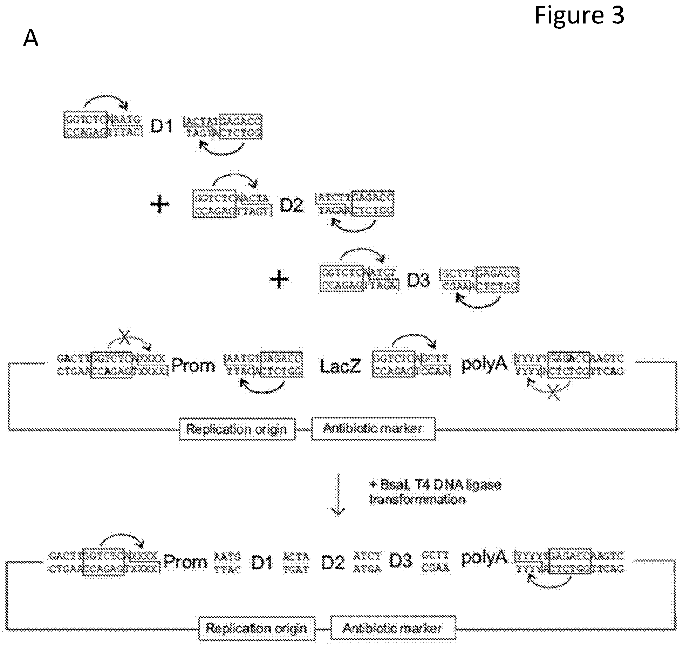

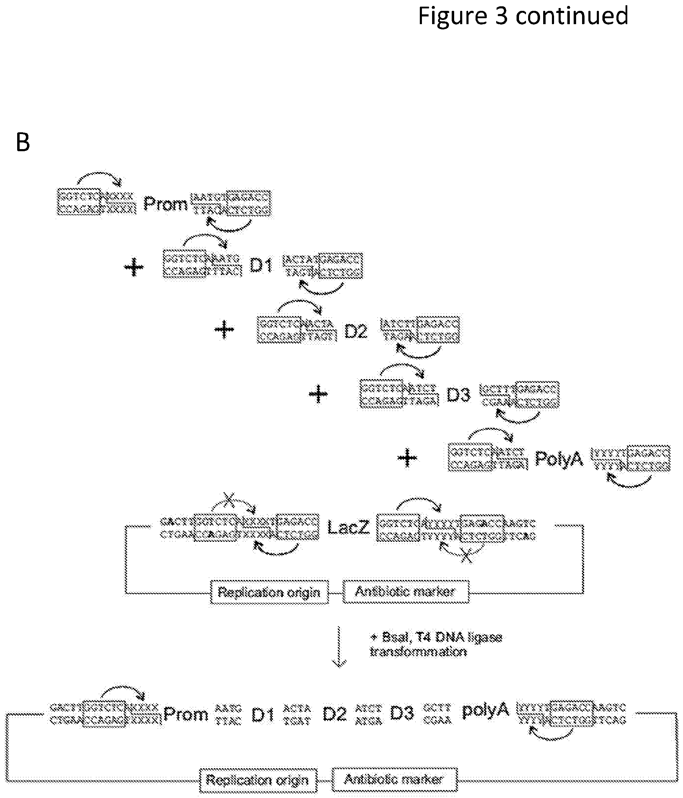

**Figure 3****. DNA assembly using Metclo vector containing functional DNA elements between the methylation-protectable restriction site and non-protectable restriction site**.
   A. Diagram showing assembly of a transcription unit for expression of a protein with three different domains (D1, D2, D3) using Metclo vector with the promoter element (Prom) and polyA signal (polyA) pre-embedded into the vector. Methylation-protected BsaI sites are boxed in solid line, functional BsaI sites in dotted line, and methylated bases in bold. XXXX and YYYY represents the adaptor sequence at 5' and 3'end of the assembled DNA fragment (transcription unit) that could be released for next round of DNA assembly. They may or may not be different from the internal adaptor sequences (AATG, ACTA, ATCT and GCTT), because they are not involved in the assembly reaction.
   B. Diagram showing assembly of the same transcription unit using Metclo with the promoter element and polyA signal provided in separate insert plasmids. XXXX and YYYY represents the adaptor sequence at 5' and 3'end of the assembled DNA fragment (transcription unit) that could be released for next round of DNA assembly. They are different from the internal adaptor sequences.

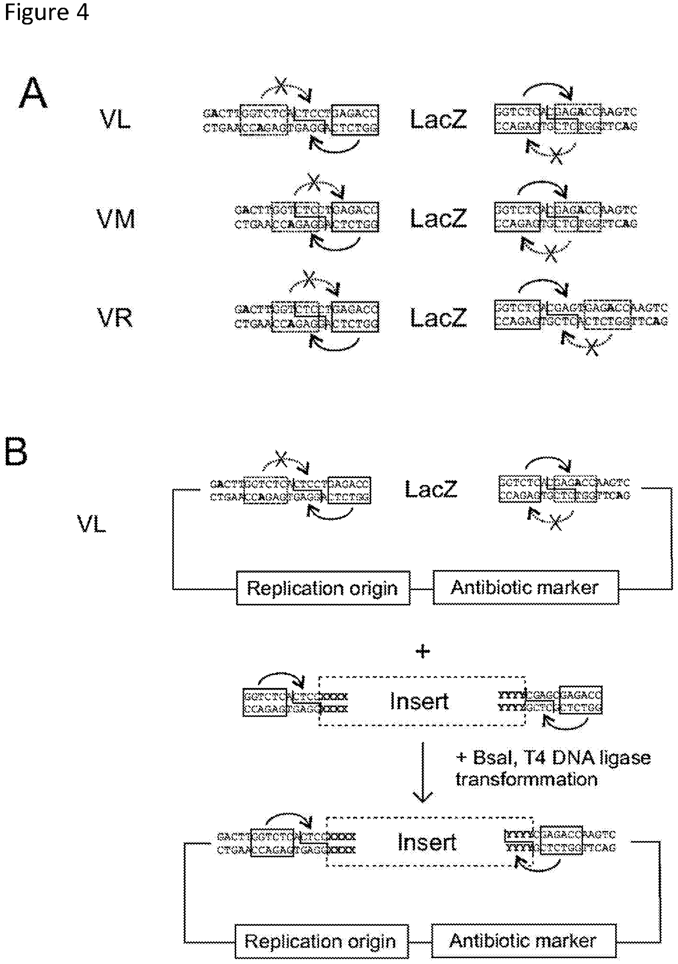

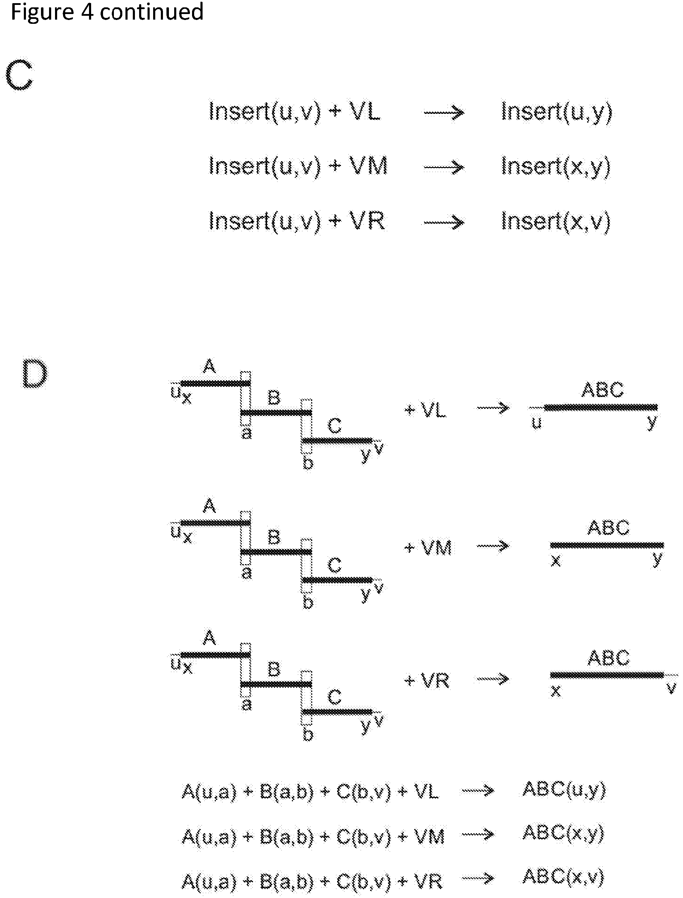

**Figure 4****. Principle of adhesive end switching using universal cloning vectors**
   A. Diagram of three types of universal assembly vectors.
   B. Details of adhesive end switching process of universal cloning vectors through Metclo, using vector VL as an example. Functional type IIS restriction sites are boxed in solid line, blocked restriction sites boxed in dashed line, and methylated bases in bold.
   C. Symbolic system for representation of the adhesive end switching process. u and v represents the universal adhesive ends CTCC and CGAG respectively. x and y represents the 4bp sequence inside the universal adhesive ends defined by the sequence. Assembly using different vector types results in switching of different adhesive ends.
   D. Adhesive end switching process as applied to multiple fragments assembly.

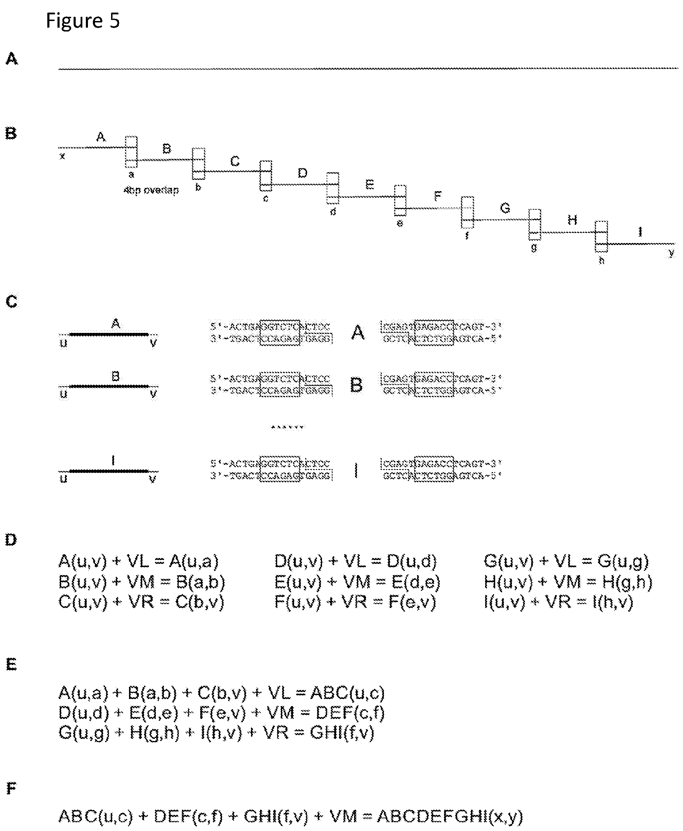

**Figure 5****. Scarless DNA assembly using universal cloning vectors**
   A. The DNA fragment to be assembled carries no internal BsaI restriction sites. The aim of the assembly process is to generate a single assembled fragment that carries adhesive ends x and y defined by the ends of the fragment.
   B. The sequence is first broken up digitally into 9 different fragments (fragments A-I) with 4bp overlaps (a-h).
   C. Adaptor sequences were then added to the fragments digitally, resulting in DNA fragments that could be trimmed by BsaI to generate fragments flanked by the universal adhesive ends u and v.
   D. Synthesized double stranded DNA fragments were first cloned into specific universal assembly vectors to switch the adhesive ends depending on the position of the fragment in the next stage assembly.
   E. Cloned fragments were assembled 3 per group into specific universal assembly vectors, with the vector type depending on the position of the assembled fragment in the next stage assembly.
   F. Final stage of assembly generates the fully assembled fragment with sequence-specified adhesive ends x and y.

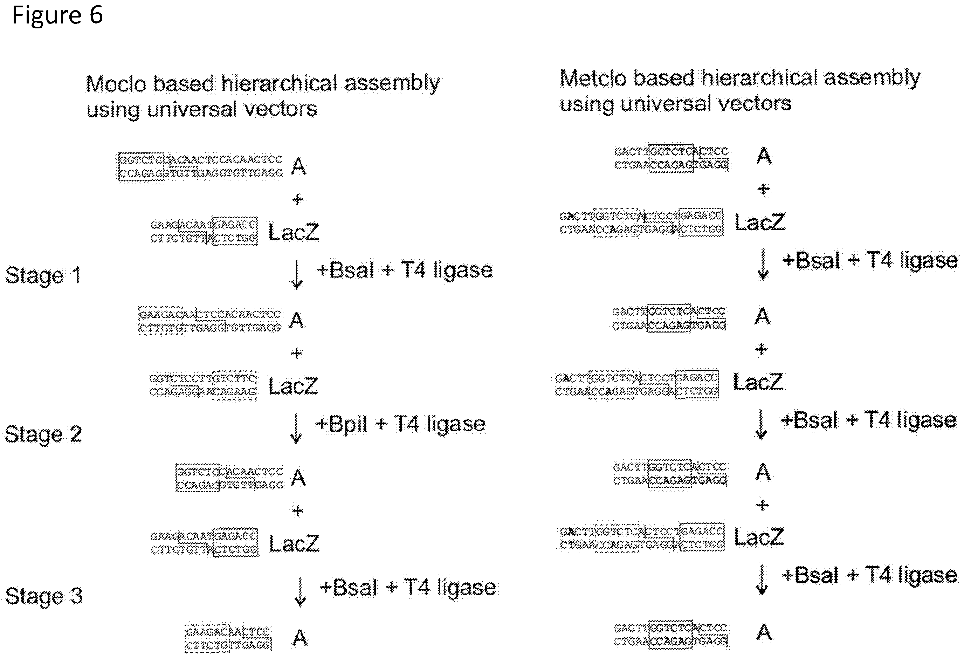

**Figure 6****. Advantage of Metclo based scarless universal assembly compared to Moclo based universal assembly**
   For multistage assembly using universal assembly vectors, the Moclo based system uses two different restriction enzymes (BsaI and Bpil) for consecutive stages, which requires different universal adaptors for a given block (CTCC for BsaI and AGAA for BpiI). In order to clone the fragments from different stages into universal assembly vectors, the 5' end of the first block and the 3' end of the last block in each subassembly needs to switch between different adaptor sequences depending on the enzyme used for the assembly. The illustration shows the change of the 5' adaptor for the first block (block A) during 3 consecutive stages of assembly reaction, with BsaI sites boxed in solid line, and BpiI sites boxed in dashed lines. A very long adaptor needs to be added to the 5' end of block A to cope with the switch of adaptors during the different stages of assembly. The adaptor is "chewed back" 4bp after each stage of assembly, so the length of the adaptor sequence depends on the number of the stages. The design based on Metclo is much simpler, because it uses a single enzyme BsaI for different stages of assembly. Here nonmethylatable BsaI sites are boxed in solid line, and BsaI sites blocked by methylation boxed in dashed line. The universal adaptor sequence CTCC at the 5' end of the first block (block A) can be used for different stages of assembly. There is no need to switch adaptor sequence for the outer blocks. As a result the adaptor design for the initial sequence is much simpler.

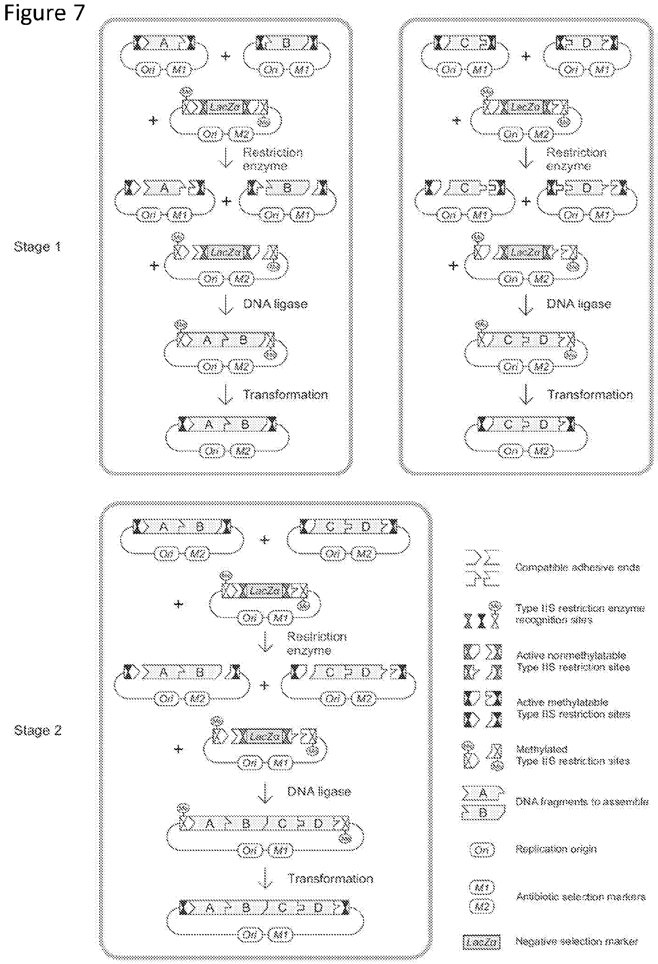

**Figure 7****. DNA assembly using Metclo.** The figure illustrates the assembly of four DNA fragments (Fragments A, B, C and D) into a single DNA fragment (Fragment ABCD) through two cycles of Metclo using a single type IIS restriction enzyme. Stage 1 and Stage 2 are effectively the same process with progressively larger inserts. In Stage 1, the insert donor plasmids contain insert fragments (Fragments A, B, C or D) flanked by methylatable type IIS restriction sites that generate compatible adhesive ends. Preparation of insert plasmids in normal *E. coli* strains, which lack the appropriate site-specific methylase, results in insert fragments releasable by the type IIS restriction enzyme. The assembly vector contains the negative selection marker LacZα flanked by a purposely designed head-to-head configuration of type IIS restriction sites. The inside pair of the restriction sites are designed to be nonmethylatable, but the outside pair are methylatable by a site-specific methylase because the restriction enzyme recognition sequence overlaps with the methylase recognition sequence. Preparation of the assembly vector in *E.coli* strains expressing the corresponding methylase leads to selective methylation of the outside pair of methylatable restriction sites, and digestion with the type IIS restriction enzyme generates a vector backbone that retains the outside pair of methylated type IIS restriction sites. Ligation of the insert fragments with the vector backbone generates correctly assembled plasmid refractory to restriction by the type IIS restriction enzyme. All of the other unwanted fragments including the vector backbone of the insert plasmid and the LacZα fragment from the assembly vector contain active type IIS restriction sites, which are sensitive to restriction by the enzyme. This scheme enables a one-pot assembly reaction that combines the restriction and ligation step because the reaction favors generation of the correctly assembled plasmid. Transformation of the assembly reaction into a normal *E. coli* strain, which lacks the appropriate site-specific methylase, effectively results in demethylation of the methylated type IIS restriction sites flanking the assembled fragment in the assembled plasmid. In Stage 2, the assembled inserts (Fragments AB and CD) can then be used for a subsequent round of Metclo assembly into a larger fragment (Fragment ABCD).

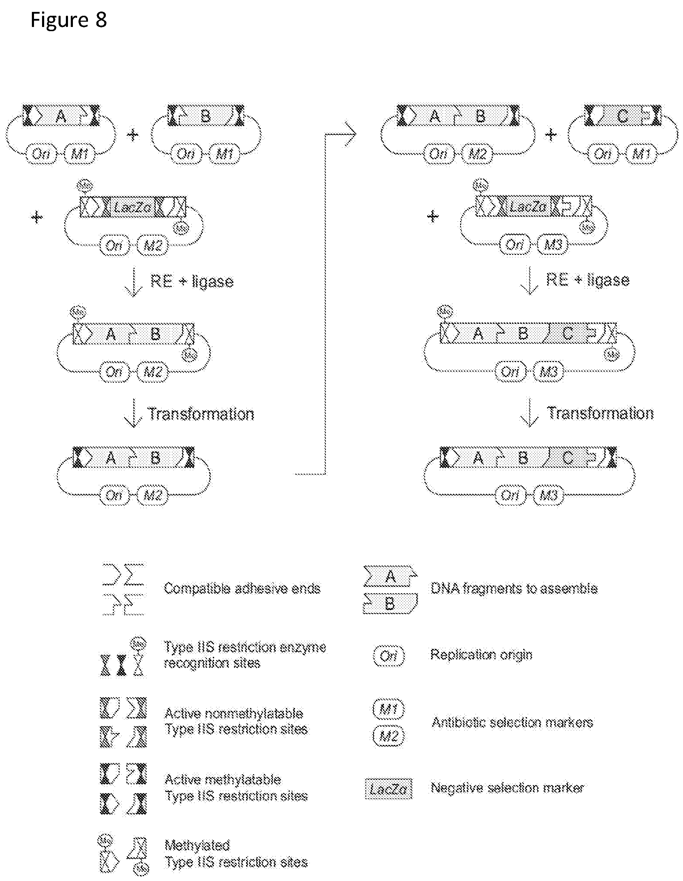

Figure 8. Level jumping: scheme for modification of DNA using components from different levels of assembly. The diagram shows the scheme for assembly of two DNA fragments (Fragments A and B) by the standard Metclo scheme and subsequent modification of the assembled fragment (Fragment AB) using a third fragment (Fragment C). The initial fragments used (Fragments A, B and C) are of the same level in the assembly hierarchy, in the sense that they carry the same antibiotic selection marker. The modification step modifies an existing fragment (Fragment AB) using a fragment (Fragment C) from a different level of assembly, hence "level jumping". The modified fragment (Fragment ABC) carries the same adhesive ends as the starting fragment (Fragment AB), and an antibiotic selection marker (M3) different from both level 1 and level 2 blocks (M1 and M2). Therefore, the new fragment ABC can be used for the next level of DNA assembly using selection marker M1 in place of fragment AB. The maintenance of the adhesive ends during the modification step is achieved through the use of a special arrangement of head-to-head type IIS restriction sites (to the right of the LacZα fragment during the modification step), in which the inside nonmethylatable type IIS restriction site generates an adhesive end that is compatible with the modification fragment to be inserted (Fragment C), and the outside methylatable type IIS restriction site generates an adhesive end identical to the end of the DNA fragment to be modified (the right- hand end of Fragment AB in the diagram). The modification step
   is not possible with the standard Moclo approach using two type IIS restriction enzymes, because DNA fragments from consecutive levels of assembly would then need to be released by two different restriction enzymes, each of which needs to be different from the enzyme used for next level of DNA assembly after the modification step. The level-jumping modification scheme is useful for insertion of functional genetic elements such as transcription units into an existing assembly pipeline without changing the higher level assembly design, to maximize the reusability of existing sequence-verified DNA parts.

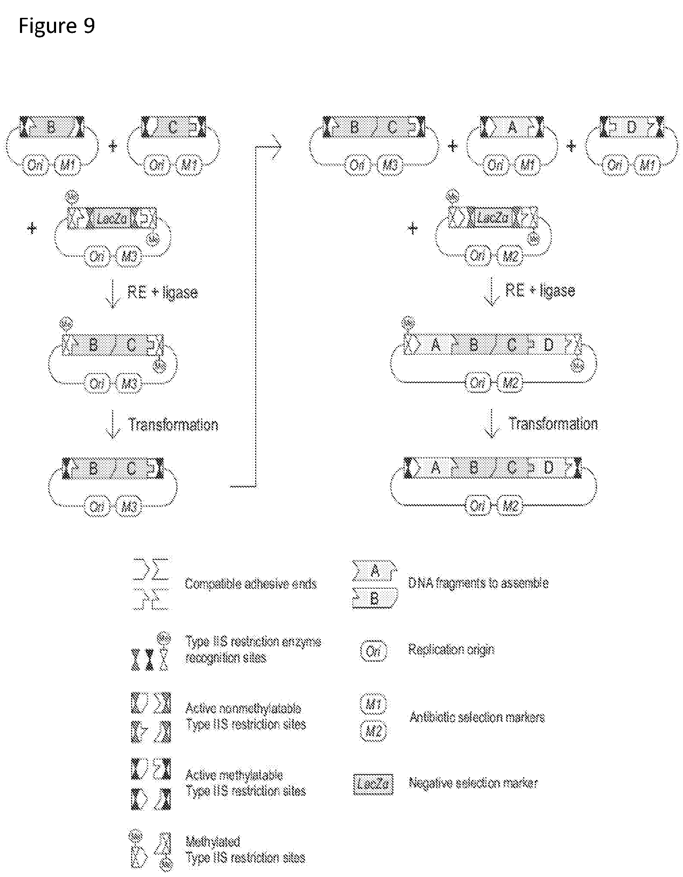

**Figure 9****. Level insertion: scheme for assembly of DNA through an intermediate assembly stage**. The diagram shows the scheme for assembly of four DNA fragments (Fragments A, B, C and D) into a single fragment (Fragment ABCD) through an intermediate stage of DNA assembly (Level insertion). In contrast to the standard Metclo assembly scheme (Figure 3) that assembles the four fragment in a single reaction, this scheme involves an intermediate assembly stage by sub-assembly of fragments B and C into an intermediate assembly vector that carries a selection marker (M3) different from the one for level 1 DNA inserts (M1 carried by fragments A, B, C and D) and for level2 assembled DNA fragment (M2 carried by fragments ABCD). The level-insertion sub-assembly scheme offers more choices in the routes of DNA assembly, and increases the reusablity of intermediate assembled DNA fragments.

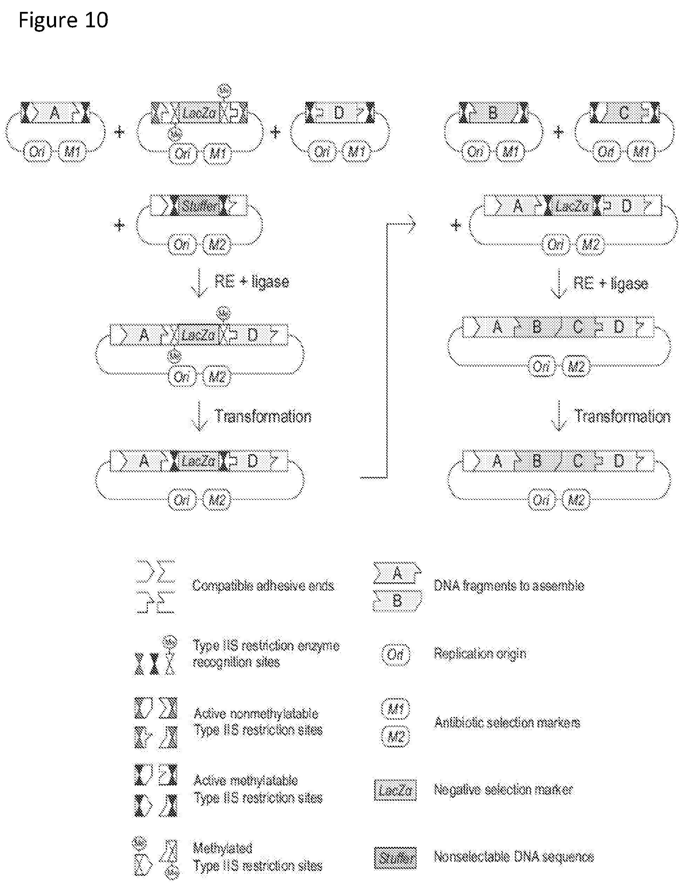

**Figure 10****. Linear addition: scheme for assembly vector construction using Bsal-based Metclo**. The diagram shows the scheme for linear addition of DNA parts for construction of assembly vectors carrying existing functional genetic elements (Fragments A and D). Starting with an assembly vector that carries a nonselectable stuffer DNA fragment flanked by nonmethylatable type IIS restriction sites, two DNA fragments (Fragments A and D) are first assembled with a LacZα selection marker into this vector. The LacZα selection marker is flanked by a specially designed head-to-head arrangement of type IIS restriction sites, such that the inside sites are methylatable and the outside sites are not. Preparation of the LacZα insert plasmid in an *E.coli* strain expressing the corresponding methylase results in selective blocking of the inside pairs of methylatable type IIS restriction sites. One-pot assembly of the LacZα insert with other inserts prepared from the normal *E.coli* strain (Fragments A and D) results in an assembled DNA fragment refractory to restriction by the type IIS restriction enzyme used for DNA assembly. Transformation of the assembled DNA into a normal *E. coli* strain that lacks the corresponding methylase activity results in demethylation of the methylatable type IIS restriction sites flanking the LacZα fragment. As a result, the assembled DNA can then be used as an assembly vector for addition of new DNA fragments (Fragments B and C) in place of the LacZα selection marker in the next round of DNA assembly. The net outcome is linear addition of DNA fragments into the assembly vector (Fragments A and D, then fragments B and C). The scheme is useful for construction of final stage assembly vectors that carry common functional genetic elements, such as mammalian selection markers.

### Example 1 - Methylation assisted modular assembly using a single restriction enzyme (MetClo)

### Introduction

### Overview of Metclo

Here we developed an improved type IIS restriction enzyme-based hierarchical assembly method named Metclo, which reduces the number of restriction enzymes used to one. We achieved this by adding a pair of specific type IIS restriction sites for the same enzyme used for assembly into the assembly vector, which are selectively blocked by DNA methylation. Transformation of assembled DNA into normal strains that lack the specific methylase unblocks the flanking restriction sites, resulting in plasmids carrying the assembled fragment which can be released using the same type IIS restriction enzyme and then used for a subsequent round of DNA assembly. We tested a number of methylases for their abilities to block overlapping methylation/restriction sites for three commonly used commercially available type IIS enzymes BsaI, BpiI and LguI, and identified suitable methylases for each of them, namely M.Osp807II (for BsaI with overlapping sequence **GAC**NN*GGTCTC*N^NNNN), M2.NmeMC58II (for BpiI with overlapping sequence *GAAGAC***GC**^NNNN)*,* and M.XmnI (for LguI with overlapping sequence **GAA***GCTCTTC*N^NNN, methylated base or opposite base is underlined, additional bases required to specify methylase activity in bold, restriction enzyme recognition sequence in italic; all three modifications are m6A N6-methyladenine). We developed *E. coli* strains carrying chromosomal copy of expression cassettes for constitutive expression of these methylases, and showed that DNA propagated in these strains is refractory to digestion by the restriction enzyme when the restriction site overlaps with the corresponding methylase recognition sequence. We then built proof-of-principle Metclo systems for each pair of methylase/restriction enzyme for assembly of four fragments into a single fragment, which could be released by the same restriction enzyme. Finally, we demonstrated the utility of the M.Osp807II/BsaI-based Metclo system for hierarchical assembly a 218kb DNA from 28 x 7.7kb DNA in 2 stages using BsaI.

### Materials and methods

### Plasmid construction

Plasmids were constructed by standard restriction enzyme- and ligation-based cloning techniques. DNA fragments for cloning were generated by PCR or by gene synthesis (IDT or Invitrogen). For proof of principle modular assembly of ~1kb DNA from 4 fragments, the modular assembly acceptor vector backbone is based on ampicillin-resistant Moclo vector pICH47732 (available from Addgene, and described in A modular cloning system for standardized assembly of multigene constructs. Weber E, Engler C, Gruetzner R, Werner S, Marillonnet S. PLoS One. 2011 Feb 18;6(2):e16765. doi: 10.1371/journal.pone.0016765) and the donor plasmid backbone based on a kanamycin-resistant vector built by replacing the ampicillin-resistant gene in pICH47732 with a kanamycin-resistant gene. For hierarchical assembly of ~200kb DNA from 28 fragments, the modular assembly acceptor vectors are based on gentamicin or kanamycin-resistant low copy BAC vector backbone with F replication origin, and the donor plasmid backbone is based on kanamycin-resistant low copy vector backbones with p15A or F replication origin. Plasmids for methylase overexpression were built based on a kanamycin-resistant low copy BAC vector backbone with F replication origin. The methylase genes were under the control of J23100 synthetic promoter, and flanked by a zeocin selection cassette as well as homologous sequences of the arsB locus to facilitate stable integration into the E coli genome by recombineering. Template plasmids for methylase assay are based on amplicillin-resistant pICH47732.

### Generation of methylase expressing strains

*E. coli* strains with stable expression of specific methylases were generated by recombineering. Briefly, DNA fragments containing the methylase gene, zeocin selection marker and flanking arsB homologous sequences were generated by PCR using the corresponding methylase expressing BAC plasmid as template. The fragments were used for lambda red recombineering in DH10B cells followed by zeocin selection to insert the methylase expression cassette into the arsB locus. Clones with correct chromosomal insertions were verified by PCR and sequencing. The methylase expressing strains can be stably maintained without antibiotic selection.

### Modular assembly

For proof of principle modular assembly of ~1kb DNA from 4 fragments, assembly reactions were set up using 60fmol each of donor plasmids prepared from DH5alpha cells, 60fmol assembly vector prepared from DH10B strains expressing compatible methylase, 1,000U T4 DNA ligase (NEB), and 5U BsaI (NEB) or BpiI (Fermentas) or 2.5U LguI (Fermentas) in 20µl 1x T4 ligase buffer (NEB). The reaction condition was: 37°C 15min, followed by 45 cycles of 37°C 2min plus 16°C 5min, then 37°C 20min, and 80°C 5min. Assembled samples were transformed into DH5alpha chemical competent cells, and plated on LB plates with AIX selection (ampicillin 100ug/ml, IPTG 100µM, X-Gal 50µg/ml) at 37°C overnight. White colonies were expanded and screened by DNA sequencing.

For hierarchical assembly of ~200kb DNA from 28 fragments, the assembly was carried out in two stages. At stage 1, the fragments were assembled 7 per group using 15fmol each of donor plasmids prepared from DH10B cells, 15fmol assembly vector prepared from DH10B-M.Osp807II cells, 1,000U T4 ligase (NEB), and 5U BsaI (NEB) in 20µl 1x T4 ligase buffer (NEB). The reaction condition was the same as described above. The assembled samples were then drop-dialyzed against 50ml dH2O using 0.05um cellulose filter (Millipore) at room temperature for 1h. 5µl of dialyzed sample was transformed into 25 µl NEB-10beta eletrocompetent cells by electroporation at 0.9kV 100Ω 25µF, using Gene Pulser and 1mm electroporation cuvettes (Biorad). Following electroporation, cells were recovered by adding 1ml LB medium and incubated at 37°C 220rpm for 1h. Cells were plated on LB plates with GIX selection (gentamicin 2.5µg/ml, IPTG 100µM, X-gal 50µg/ml) at 37°C overnight. White colonies were expanded and screened by restriction digestion using XhoI.

At stage 2, the ~200kb DNA was assembled using protocols similar to stage 1 with the following modifications: 30fmol each of donor plasmids and 15fmol assembly vector was used in the assembly reaction; following modular assembly and before the drop dialysis step, 10U BsaI was added to the assembly reaction for incubation at 37°C for 45min followed by heat inactivation at 80°C for 5min. Transformed cells were plated on LB plates with KIX selection (kanamycin 30µg/ml, IPTG 100µM, X-gal 50µg/ml). White colonies were screened by PCR using primers detecting the junction between the second and the third 54kb fragments (TACCCACGTGATTCACGCTG and ATCCTACAGACTCGCTGTGG). Selected PCR positive clones were screened by restriction digest using XhoI or BsaI with pulsed-field electrophoresis.

### Result

The Metclo system is based on the standard type IIS restriction enzyme-based one-pot DNA assembly system, and added an additional pair of type IIS restriction sites in the assembly vector backbone for release of the assembled DNA for next stage assembly. Unlike the Moclo system which uses restriction sites different from the type IIS enzyme used in the assembly process, Metclo uses the restriction sites for the same enzyme. To prevent the assembled plasmid from being cut by this enzyme in the one-pot assembly reaction, Metclo uses specific methylase to methylate the type IIS restriction sites overlapping with the methylase recognition sequences, in order to block these type IIS restriction sites in the vector backbone. The methylation is lost when the assembled plasmid is transformed into *E. coli* strains lacking the specific methylase, thus enabling the release of assembled fragment from the assembled plasmid by the same type IIS restriction enzyme used in the assembly reaction. The resulting Metclo system therefore allows hierarchical assembly of DNA fragments using one-pot reaction with a single type IIS restriction enzyme, among various applications.

The key to the Metclo system is the identification of suitable methylase to block type IIS restriction sites that overlap with the methylase recognition sequence in the vector backbone. There are several criteria for selection of suitable methylase. First of all, methylation of the overlapping methylation/restriction site must result in blocking the action of the type IIS restriction enzyme. The methylation process could occur by *in vitro* methylation of purified vector plasmid, or by propagating vector plasmid in strains that express the methylase *in vivo. In vivo* methylation is preferred due to the reduced cost and time in the vector preparation process. Secondly, the methylase recognition sequence must not be identical to or entirely enclosed by the type IIS restriction site; additional base pairs must be required to specify the action of the methylase on the overlapping methylation/restriction site. The reason for this requirement is because there are two pairs of type IIS restriction sites for the same enzyme in the assembly vector. One pair within the vector backbone need to be blocked by the methylase, whereas the other pair within the negative selection marker insert must not be blocked. If the methylase recognition sequence is identical to, or enclosed by the type IIS restriction site, all the restriction sites in the assembly vector for this particular type II restriction enzyme will be blocked by methylation, including the pair within the negative selection marker insert; the resulting methylated assembly vector won't be cut by the type IIS enzyme at all, therefore cannot be used for DNA assembly. Thirdly, additional base pairs to specify methylase activity should not overlap with the adhesive end sequence generated by the type IIS restriction enzyme. This will maintain maximum flexibility in design of adaptor sequence for DNA assembly.

We searched for methylases with predicted ability to block restriction sites for type IIS restriction enzymes BsaI, BpiI and LguI. These are common type IIS restriction enzymes used in type IIS-based assembly systems. We used a few extra criteria in addition to the ones listed above to choose candidate methylases for testing. We aimed to generate methylated plasmids by *in vivo* methylation, so the methylase should ideally be active at the growth temperature of E coli. We also preferred methylases that recognize longer sequences (5-6bp), because with longer recognition sequences, fewer bases in the E coli host genome and assembly vector backbone would serve as methylation substrate by chance. This potentially increases the efficiency of methylation, and reduces the chance of adverse effect of heterologous DNA methylation on host gene function. In addition, we preferred single subunit methylases from type II restriction/modification systems that lack restriction activity towards unmethylated sequence to avoid potential problem of restriction during the vector construction step. Lastly, to increase the chance of methylation blocking the overlapping methylation/restriction sites, we preferred methylases that modify bases close to the middle of the restriction site.

Based on these criteria, we selected the following methylases for testing: M.Osp807II (GACNNGGTCTC m6A) for BsaI, M.Rsp7740I (TTCGAAGAC m6A) and M2.NmeMC58II (GAAGACGC m6A) for BpiI, and M.SacI (GAGCTCTTC m5C), M.AspJHL3I (GAGCTCTTC m4C) and M.XmnI (GAAGCTCTTC m6A) for LguI (methylated base or complementary base underlined). We screened methylase activity by preparing ColE-based (i.e. ColE-derived) high copy template plasmid carrying overlapping methylation/restriction sites in DH10B cells expressing the corresponding methylase under the J23100 constitutive synthetic promoter from a low copy BAC vector (low copy BAC vector backbone with F replication origin). This identified M.Osp807II (for BsaI), M2.NmeMC58II (for BpiI) and M.XmnI (for LguI) as suitable candidates for further analysis (Figure 1A). We then integrated the methylase expression cassette with a zeocin selection marker into the arsB locus using lambda-red recombineering to generate stable strains overexpressing the methylase from a chromosomal copy (Microb Cell Fact. 2013 Jun 24;12:60. doi: 10.1186/1475-2859-12-60 (PMID 23799955)), and tested the ability of these methylases to block overlapping methylation/restriction sites *in vivo.* The test vector carries one restriction site that does not overlap with methylation sequence, thus permissible for restriction in both wild-type and methylase-expressing strain, and one site with two head-to-head type IIS restriction sites, both of which overlap with the corresponding methylation sequence and would, therefore, be blocked in the strain expressing the methylase. The head-to-head arrangement allows for assessment of nickase activity as well as double strand restriction activity in the same assay (Figure 1B). Following 1h digestion of 60fmol test plasmid (pMOP _BsaINC, pMOP_BpiINC and pMOP _LguINC) in 10µl reaction using 5U (BsaI, BpiI) or 2.5U (LguI) enzyme at 37°C, DNA were analyzed by agarose gel electrophoresis with EtBr staining. The result shows that under test condition, the three methylases block restriction of the overlapping sites, as demonstrated by the lack of generation of -600bp fragment (Figure 1C).

We then developed proof-of-principle Metclo DNA assembly systems using these methylases for one-pot DNA assembly (Figure 2 shows BsaI-MOsp807II as an example). In these systems, the donor DNA inserts to be assembled are flanked by overlapping methylation/restriction site. Because the donor plasmids containing the DNA inserts to be assembled are prepared in a normal E coli strain that lacks the corresponding methylase, the restriction site is not blocked and the donor insert could be released by the enzyme in the assembly reaction. The assembly vector carries a different selection marker from the donor plasmid, and a LacZ selection marker flanked by head-to-head typeIIS restriction sites. For each head-to-head site, the two type IIS restriction sites generate the same adhesive end. The outside site close to the vector backbone overlaps with the methylation sequence, and the inside site close to the LacZ insert does not. The destination vectors are prepared from an E coli strain that expresses the corresponding methylase. As a result, only the inside typeIIS sites are functional in the assembly reaction. During the reaction, the LacZ insert is released from the vector backbone due to action of enzyme on the inside typeIIS site; ligation of the donor insert with the destination vector results in typeIIS restriction sites within from the destination vector backbone, which is blocked by methylation; religation of the freed LacZ insert with the destination vector backbone results in regeneration of the head-to-head typeIIS site, which is sensitive to restriction due to the inside site not blocked. As a result, the reaction favors ligation of donor fragments to destination vector backbone. The moclo reaction is then transformed into normal E coli strain that lacks the specific methylase activity for screening for LacZ expression using LB plates containing IPTG and X-Gal. The white colonies that lack LacZ activity should contain successfully assembled plasmids. The assembled DNA fragment have flanking type IIS restriction sites that overlap with methylase recognition sequences, which are identical to the donor vector. As a result, this allows a further round of modular assembly at the next level using the same restriction enzyme and so on for multiple rounds as required.

We tested the systems for the three enzymes for assembly from four fragments (Table 1). The donor vectors are kanamycin-resistant, and destination vectors ampicillin-resistant. Destination vectors were prepared from corresponding methylase-expressing strains. Following selection, 8 white colonies were picked for miniprep and analyzed by Sanger sequencing. Sequencing result shows that 100% (8/8) of clones are correct for each of the three Metclo systems (Table 1). This confirms the utility of Metclo for modular assembly using a single restriction enzyme such as BsaI, BpiI or LguI.

To further explore the utility of Metclo for hierarchical assembly of large DNA constructs, we assembled a 218kb DNA fragment from 28 x 7.7kb fragments using BsaI restriction enzyme in two stages. In stage one, 28 DNA fragments of 7.7kb which were free of BsaI sites were assembled 7 per group into 4 pieces of 54kb fragments using BsaI-based Metclo system. In stage two, the 4 pieces of 54kb fragments were assembled into a single 218kb fragment. The final 218kb fragment is composed of non-repetitive sequence with -48% GC content. Restriction digest shows a -50% success rate for stage 1 assembly (2/4 to 3/4 clones correct), and -20% for stage 2 assembly (14 out of 27 PCR verified, of which 3 out of 8 verified by restriction digest) (Table 2). This confirms that the Metclo system can be used for hierarchical assembly of large DNA construct using a single type IIS restriction enzyme.

### Discussion

Here we present Metclo, a modified type IIS restriction enzyme based DNA assembly system for one-pot assembly of DNA using common type IIS restriction enzymes such as BsaI, BpiI or LguI, that generates assembled DNA releasable by the same restriction enzyme. This enables hierarchical assembly of large DNA constructs using a single type IIS restriction enzyme. The system uses methylase-overexpressing strains during the vector preparation step for *in vivo* methylation of overlapping methylation/restriction sites to block selective restriction sites in the vector. The assembly process is the same as the type IIS DNA assembly systems through the use of one-pot reaction, thus preserves the simplicity of the existing systems. The part preparation and transformation steps utilize normal strains similar to the standard Moclo system, allowing the use of routine cloning strains for DNA assembly, similar to the current type IIS assembly systems.

The Metclo system has a number of advantages over the existing type IIS restriction enzyme-based hierarchical DNA assembly systems. Firstly, the Metclo approach reduces the burden to remove internal restriction sites during component part construction. Compared with the standard Moclo system that uses two or more enzymes, the BsaI- and BpiI-based single enzyme Metclo system uses a 6bp cutter that reduces the frequency of forbidden restriction sites from 1 per ~1kb to 1 per ~2kb for random DNA with 50% GC content, and the LguI-based Metclo system uses a 7bp cutter that reduces the frequency further to 1 per ~8kb.

Metclo improves the flexibility of the hierarchical type IIS assembly system. Because assembled DNA fragments from different stages of assembly can be released by the same restriction enzyme, with appropriate design of adaptors and choice of antibiotic resistant vector, the system potentially allows design of several assembly schemes not achievable using the existing system. These include level jumping-the assembly of DNA using parts from different stages of assembly-and level insertion-the assembly of DNA fragments through an intermediate assembly stage. These schemes are useful for modification of large DNA constructs using existing parts, and for subassembly of complex parts such as multi-part open reading frames for added reusability.

Additional schemes could also be devised for linear addition of DNA parts during the assembly process. For example, DNA parts containing selection markers flanked by internal methylation-blocked type IIS restriction sites could be used during DNA assembly. The assembled plasmid could then be used as an assembly vector for insertion of additional DNA parts in place of the selection marker in the next stage assembly. This is particularly useful for construction of common assembly vectors containing functional modules.

Metclo also increases the exchangeability of parts from different assembly standards. Independent development of part library and assembly standards lead to assembly systems that use different type IIS restriction enzymes. For example, for plant biology, Moclo uses BsaI/BpiI (and Esp3I), Goldenbraid uses BsaI and BsmBI (and BtgZI); for E.coli, Ecoflex uses Bsal/BsmBI, CIDAR uses BsaI/BpiI. This place constraints on exchangeability of parts among different collections. The Metclo system that uses a single type IIS restriction enzyme is compatible with any system that uses that restriction enzyme. In particular, the BsaI-based system is compatible with all the existing systems that use BsaI. Given that BsaI has been used in most of the type IIS restriction enzyme-based hierarchical modular assembly systems to date (ACS Synth Biol. 2016 Oct 21;5(10):1059-1069 (PMID 27096716); ACS Synth Biol. 2016 Jan 15;5(1):99-103. doi: 10.1021/acssynbio.5b00124 (PMID 26479688; ACS Synth Biol. 2015 Sep 18;4(9):975-86. doi: 10.1021/sb500366v (PMID 25871405); and PLoS One. 2011 Feb 18;6(2):e16765. doi: 10.1371/journal.pone.0016765 (PMID 21364738), the BsaI-based Metclo system has potential to be used as a standard for modular assembly that is compatible with most of the existing part libraries.

There are DNA assembly systems that use DNA methylation to block internal type IIS restriction sites during the assembly process. For example, the TNT assembly system (Sci Rep. 2016 Jan 13;6: 19278. doi: 10.1038/srep19278 (PMID 26758940)) uses M.TaqI to methylate overlapping EarI recognition site, which results in an assembly system using 7bp cutter LguI (GCTCTTCN^NNN) and 6bp cutter EarI (CTCTTCN^NNN) both giving 3bp sticky ends, thus requiring removal of a single 6bp sequence from DNA parts (CTCTTC). However, because the M.TaqI recognition sequence (TCGA) that overlaps with the EarI restriction site intrudes the adhesive end sequence (CTCTTCG^ANN), the system places limitations on the design of adaptors, thus could only assembly fragments three at a time, and could not be expanded to an LguI-only assembly system. As another example, Greengate used methylated linker DNA as parts during the DNA assembly process to introduce new BsaI sites for subsequent rounds of assembly. The resulting system however could only be used for linear addition of DNA parts, thus limiting the use of the system to construction of DNA constructs with a small number of transcription units. Compared to these systems, the Metclo system, with the use of *in vivo* methylation of overlapping methylation/restriction site outside the adaptor sequence, preserves the advantage of the hierarchical multiplicative assembly scheme of the Moclo system, and maintains the freedom of choice of adaptor sequences for DNA assembly. In practice, similar result could be achieved by adding methylated linker DNA as the first and last parts during the assembly process. However, this would increase the number of parts in the assembly by two, which reduces success rate of assembly compared to the *in vivo* vector methylation approach in the Metclo system.

In summary, the Metclo system, through the use of *in vivo* methylation to block specific type IIS restriction sites within the assembly vector, enables hierarchical assembly of large DNA fragments using a single type IIS restriction enzyme. The system simplifies the existing type IIS restriction enzyme-based assembly method and increases the flexibility of design of assembly schemes. The BsaI-based Metclo system in particular is compatible with all the existing part libraries that uses BsaI, and could be used as a standard type IIS-based assembly system.

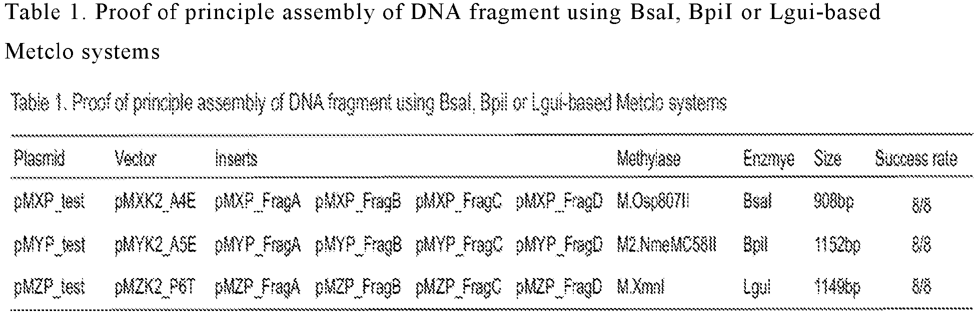

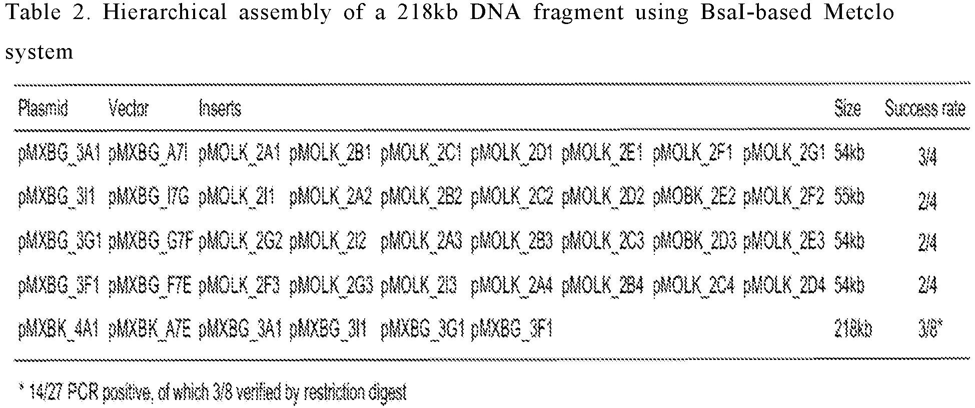

### Example of an insertional composite element

In another scenario, longer DNA sequences could be included between the methylation-protectable restriction site and the opposing non-protectable restriction site in the assembly vector, such that following DNA assembly, these sequences will be added into the 5' or 3' end of the assembled DNA plasmid. This design is convenient for adding common elements to the assembled plasmid by using specialized vectors.

An example is shown in figure 3 for assembly of a transcription unit for expression of a protein with three different domains, starting from insert plasmids containing the three individual domains D1, D2 and D3 using BsaI-based Metclo. Figure A shows the Metclo assembly using a special Metclo vector containing a promoter between the methylation-protectable restriction site and the non-protectable restriction site at 5' of the LacZ (discard sequence), and a polyA element at 3' ("pre-embedded vector". Metclo using this special pre-embedded vector allows the assembly of the three domains into a transcription unit in a one-pot reaction from three insert plasmids, and the assembled transcription unit consisting a promoter, a coding region (D1 D2 and D3), and a polyA signal could be released from the assembled plasmid for next round of Metclo assembly. This is in contrast with the standard Metclo using "maintaining composite element", whereas the transcription unit has to be assembled from 5 different insert plasmids (Figure B). In the figures XXXX and YYYY represents the adaptor sequence at 5' and 3'end of the assembled DNA fragment (transcription unit) that could be released for next round of DNA assembly. For DNA assembly using "pre-embedded vectors", these two adaptor sequences can be the same as internal adaptor sequences because they are not involved in the one pot three fragment assembly. For DNA assembly using standard Metclo vector though, XXXX and YYYY adaptor sequences must be different from the internal adaptor sequences (AATG, ACTA, ATCT and GCTT). In addition, the method using pre-embedded vectors (Figure A) has advantage that less number of DNA parts need to be assembled in a single reaction, which increases the rate of success. The disadvantage is that the specialized vector is more difficult to prepare.

### Example 2 - Scarless DNA assembly (Scarless Metclo)

A unique application of Metclo is a system for scarless hierarchical assembly of large DNA construct using a limited set of universal cloning vectors. Type IIS based DNA assembly using universal vectors relies on a specific arrangement of head-to-head type IIS restriction sites in the assembly vector that changes the adhesive end of the cloned fragment. Scarless assembly is achieved by using these universal vectors to generate assembled DNA fragments with adhesive ends (scars) defined by the DNA fragment for subsequent round of assembly, which buried the assembly scar into the assembled sequence. The use of the Metclo system allows maintenance of the same universal adhesive ends for the outer fragments during different stages of hierarchical assembly. A combination of these three features results in a universal scarless assembly system.

Here we describe the principles of scarless Metclo assembly system using the Bsal restriction enzyme. The system relies on a set of three types of universal cloning vectors that change the adhesive ends of the cloned fragments (Figure 4A). The three vector types are named VL, VM and VR. The vectors are designed to clone DNA fragments with adhesive end CTCC at 5' end and CGAG at 3' end could be cloned into the above vectors. The cloned fragment, once released by Bsal from the vector, will have different adhesive ends depending on the vector used. Vector VL is designed to keep the 5' adhesive end, and change the 3' adhesive end to the four bases inside the 3' CGAG sequence; Vector VR to keep the 3' adhesive end, and change the 5' adhesive end to the four bases inside the 5' CTCC sequence; Vector VM to change both ends to the four bases inside. In this way a set of universal vectors could be used to generate DNA fragments with adhesive ends defined by the insert sequence.

The detail of adhesive end switching process is described in Figure 4B using vector VL as an example. When the vector is prepared in methylase-expressing competent cells, methylation of adenine (bold) due to overlapping methylase recognition sequence results in blocking of the outside Bsal sites (boxed in dotted line), where the inside BsaI sites (boxed in solid line) are available to remove the LacZ insert. Note that the left arm of vector VL is similar to standard Metclo, that both the outside and inside BsaI sites give rise to the same adhesive end CTCC. The right arm of VL however is different that the adhesive end CGAG generated by the inside BsaI site overlaps with the outside BsaI site. The donor DNA carries an insert that could be released by flanking BsaI sites with compatible universal adhesive ends CTCC and CGAG. The double strand insert carries 4bp sequence XXXX on the 5' end and YYYY on the 3' end inside the universal adhesive end. Following Metclo assembly of the donor DNA into vector VL, the resulting plasmid carries the corresponding insert that could be released by the flanking BsaI sites (boxed in solid line) in the vector. The released fragment would carry a 5' adhesive end CTCC identical to the one used for cloning, but the 3' adhesive end would be YYYY as defined by the insert sequence, which may be different from the universal 3' adhesive end CGAG. In this way, vector VL switches the right hand adhesive end of the cloned fragment from universal adhesive end CGAG to the sequence-defined adhesive end YYYY.

The conversion process using the three types of universal cloning vectors could be described using a simple symbolic representation system (Figure 41C). Here we use letter u to represent the 5' universal adhesive end CTCC, and letter v to represent the 3' universal adhesive end CGAG. x and y represents the 4bp sequence at the 5' and 3'end of the insert fragment inside the universal adhesive end. Insert (u,v) represents a plasmid carrying insert fragments flanked by BsaI sites that generate adhesive ends u and v. Assembly reaction with different vectors results in different adhesive ends being converted.

The same set of universal vectors could be used to assemble multiple fragments with compatible adhesive ends, as long as the 5' end of the first fragment and the 3' end of the last fragment have universal adhesive ends u and v (Figure 4D). The assembled fragment carries different adhesive ends depending on the type of assembly vector used. Assembly of fragments into vector VL keeps the 5' universal adhesive end, and converts the 3' end into the internal 4bp. Assembly into vector VM converts both ends, and assembly into vector VR converts the 5' end and keeps the 3' universal adhesive end. Note the fragment assembled into vector VL resembles fragment A that it carries only 5' universal adhesive end u; the fragment assembled in vector VM resembles fragment B; and the fragment assembled in vector VR resembles fragment C. Therefore the process is self-similar, such that the assembled fragments could be used for next round DNA assembly using the same principle depending on the position of the fragment in the assembly order. This enables hierarchical scarless assembly of large DNA constructs using universal cloning vectors.

The whole process of hierarchical scarless assembly of DNA is described in Figure 2, using DNA assembly from 9 fragments in 2 stages as an example. Starting with a DNA sequence free of internal BsaI sites, with the 4bp at 5' end named x, and 3'end named y, the aim is to assemble the DNA from 9 fragments into a single fragment that once released from the assembled plasmid carries adhesive ends x and y (Figure 5A). The DNA sequence is first broken up into 9 fragments named A-I with 4bp overlapping sequences, with the overlapping sequences named a-h (Figure 5B). The positions of 4bp overlaps could be chosen freely, as long as they are different to universal adhesive ends u and v, and to each other within each subassembly reaction. For the current set up, the following pairs of 4bp overlaps should be different within each pair: (a,b), (d,e), (g,h) and (c,f). Appropriate adaptor sequences were added to the ends of sequences of fragments A-I, and double DNA synthesized by gene synthesis or PCR, so that following restriction digest with BsaI, the double stranded DNA could be trimmed into fragments A-I carrying universal adhesive ends u and v (Figure 5C). These double stranded synthetic DNA fragments could then be cloned into appropriate types of universal cloning vectors to change the adhesive end of the fragment depending on the position of the fragment in the next stage assembly (Figure 5D). Fragments A, D and G, which will locate at the 5' end position in the next round of assembly, are cloned into vector type VL; B, E, H in the middle position in the next round are cloned into vector type VM; C, F, I at 3' end position into vector type VR. Cloned fragments could be sequenced at this stage to check for errors in gene synthesis or PCR. These fragments could then be used for a new round of assembly, and again the type of assembly vector used depends on the position of the assembled fragment in the next round DNA assembly (Figure 5E). Fragments A, B, C were assembled into vector VL, because the assembled fragment ABC will be used as the 5' fragment in the next round assembly; fragments D, E, F into vector VM, and fragments G, H, I into vector VR. The final step is similar so that the three assembled fragments from step 2 is further assembled into vector VM, which generates the fully assembled fragment that carries adhesive ends x and y defined by the ends of the initial sequence (Figure 5F).

A set of at least 6 universal cloning vectors representing 3 types (VL, VM, VR) and 2 antibiotic selection markers are required for hierarchical assembly. Ideally 12 vectors representing 2 different copy numbers are optimal in order to cope with different sizes of DNA fragments at different stages of assembly. Similar system could be developed for LguI based Metclo, but not BpiI, because the scarless system requires universal adaptor u and v to be different. For BsaI and SapI, the bases between the enzyme recognition sequence and adhesive end generated by the enzyme is flexible, therefore it is possible to design different adaptors u and v. For Bpil however, these bases are fixed because the two bases are both required to specify the methylase recognition sequence in BpiI based Metclo. In addition, the methylated base of BpiI based Metclo is located on the top strand of the restriction site. Head-to-head arrangement of Bpil sites for adhesive end switching inevitably results in the methylated base being carried by the LacZ selection marker, not the assembly vector backbone following BpiI digestion, which makes one pot Golden Gate assembly impossible.

### The relationships between scarless Metclo and Metclo

Metclo was invented to solve the problem of using a single restriction enzyme for type IIS restriction enzyme-based hierarchical assembly of large DNA construct. Scarless Metclo is a further development of Metclo that utilizes key components of the Metclo system (methylase-expressing strains, overlapping methylation/restriction site) to achieve scarless hierarchical assembly of large DNA constructs using a set of universal assembly vectors.

Scarless Metclo is based on a combination of several different concepts of type IIS-based assembly system:
1. A special design of head-to-head type IIS restriction site for adhesive end switching, which has been used in the Moclo system for "level -1" universal cloning vector design. The Moclo "level -1" vector design uses a BpiI-BsaI head-to-head restriction site, which is different from the BsaI-only head-to-head design in the Metclo, but the concept of adhesive end switching is based on the Moclo "level -1" vector design.
2. The removal of the assembly scar by designing the scar sequence around the assembled DNA sequence. This hasn't been demonstrated in type IIS-based hierarchical assembly before.
3. The use of Metclo system to maintain the same adhesive end sequences for the outer blocks of DNA fragments for each stage of assembly.

The use of Metclo system is key to achieve multi-stage hierarchical assembly using the same universal adhesive end sequence. The first two concepts alone are sufficient for scarless single stage assembly of DNA using a universal assembly vector. The single stage scarless assembly system however could not be adapted easily for multi-stage hierarchical assembly using the traditional Moclo system, because the universal adaptor sequence of the adhesive end switching vector design for different enzymes are different (BsaI would be CTCN and NGAG, and Bpil ACNN and NNGT). This means that in order to achieve hierarchical scarless assembly using the two enzyme-based Moclo, very long repetitive adaptor sequences need to be added to the ends of the outer blocks depending on the number of stages of assembly, which will be "chewed back" following each stage of assembly (Figure 6). Because Metclo system uses a single enzyme for different stages of assembly, the same universal adaptor sequence could be used for the outer blocks during different stages of assembly, which greatly simplifies the design of the assembly process.

### Example 3 - Identified methylase and Type IIS restriction enzyme combinations

Table 3 contains a list of methylase/restriction enzyme combinations that may result in blocking of overlapping methylation/restriction site for all known type IIS restriction enzymes (78 enzymes representing 19 different specificity).

The list was generated using a custom Perl script based on a list of known type IIS restriction enzymes and a list of known type II DNA methylases, both from Rebase (PMID 25378308. (Nucleic Acids Res. 2015 43: D298-9).

The listed methylases are limited to methylases from type II restriction/modification systems with recognition sequences of at least 4bp and for which the position of the methylated base is known. Methylases from the type IIG restriction/modification system that contain both methylase and restriction enzyme activity in the same protein are excluded. In addition to the restriction enzyme recognition sequence, further bases must be required to specify the methylase activity towards the overlapping methylation/restriction site. Any methylase whose recognition sequence is identical to or enclosed by the restriction site are removed. In addition, the methylation sequence must not intrude into the adhesive end sequence generated by the type IIS restriction enzyme.

The DNA sequences are listed using the IUPAC Ambiguity Codes. M represents A or C; R represents A or G; W represents A or T; S represents C or G; Y represents C or T; K represents G or T; V represents A or C or G; H represents A or C or T; D represents A or G or T; B represents C or G or T; N represents A or T or C or G.

`Restriction enzyme' lists the name of type IIS restriction enzyme. Restriction enzymes that recognize the same sequence are grouped together.

`Restriction site' lists the recognition sequence of the restriction enzyme.

'Methylase' listed the name of the methylase that could block the overlapping methylation/restriction site. Methylases that recognize the same sequence and generate the same pattern of methylation are grouped together.

'Methylation sequence' lists the recognition sequence of the methylase.

`Position of methylated base' listed the position of the methylated base or opposite base in the methylase recognition sequence.

'Type of methylation' lists the types of methylation for the methylated bases in the order of the positions of methylated bases listed in the previous column. m6A represents N6-methyladenine; m5C represents C5-methylcytosine; m4C represents N4-methylcytosine; 'Unknown' represents unknown type of methylation.

'Overlapping methylation/restriction site' lists the sequence of the overlapping methylation/restriction site that may result in blocking of the action of listed restriction enzyme through methylation by the listed methylase. For some pairs of restriction enzyme/methylase combination, there are several different overlapping methylation/restriction sites to choose from. The bases bracketed in '[]' represent the adhesive ends generated by the restriction enzyme.

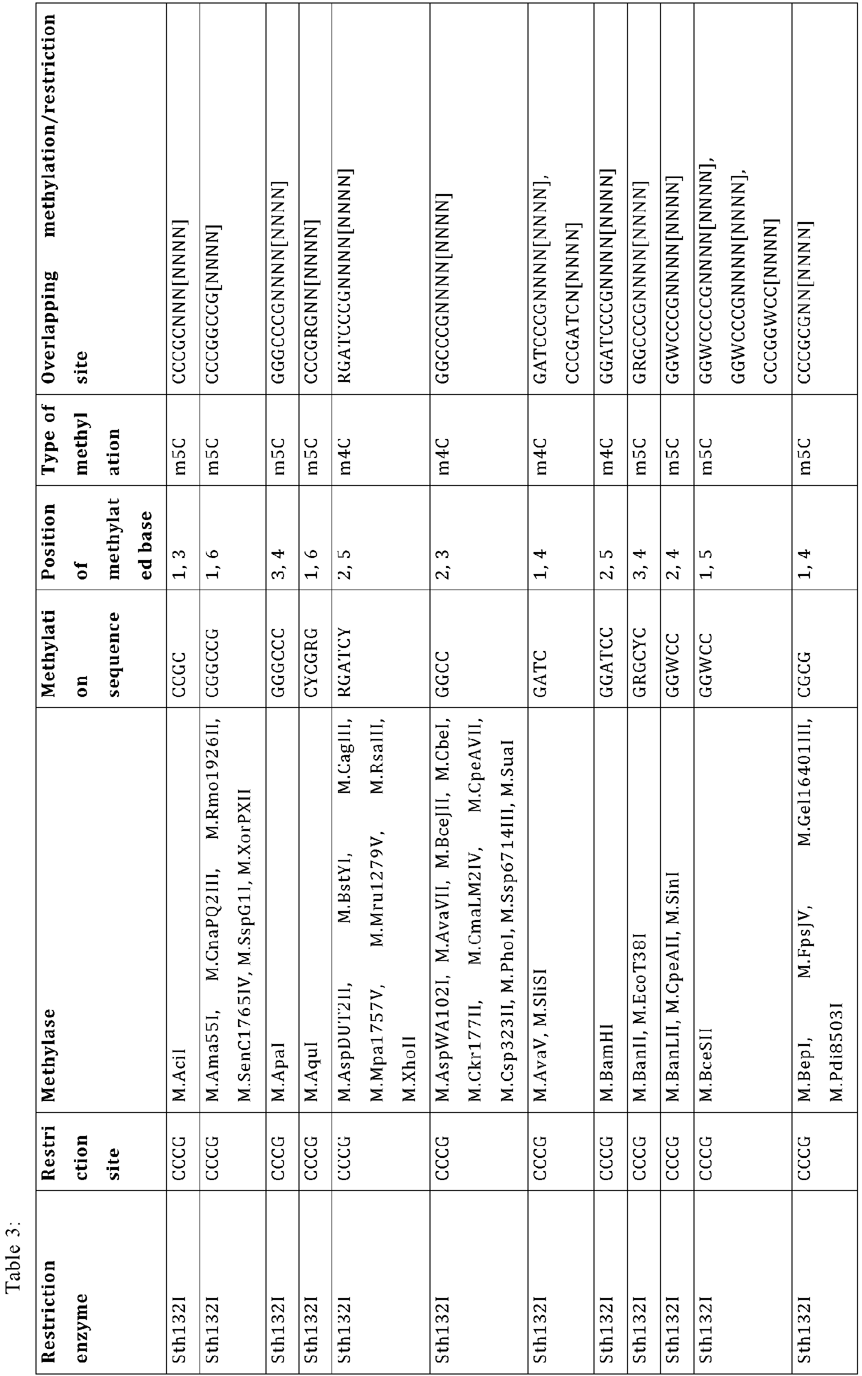

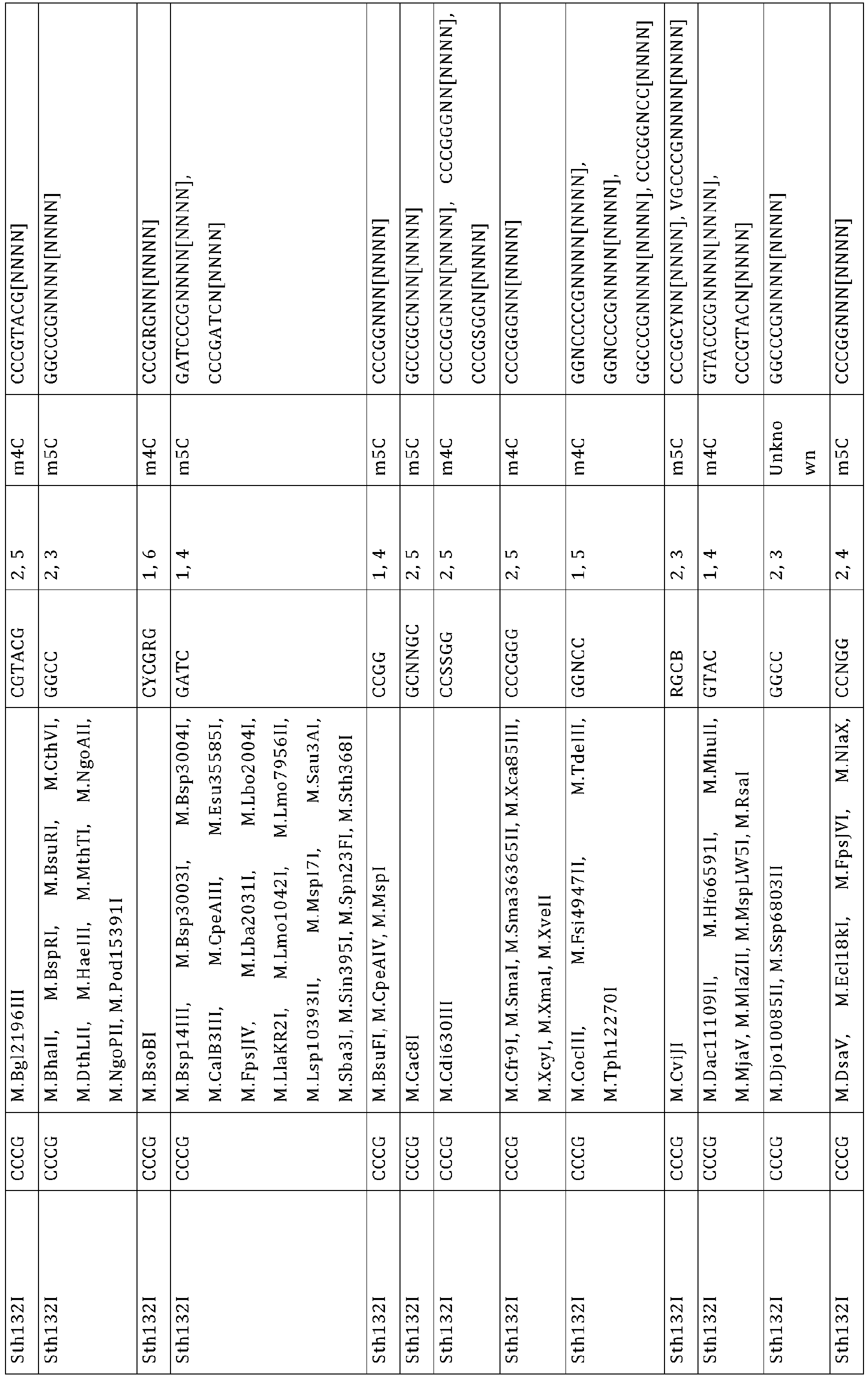

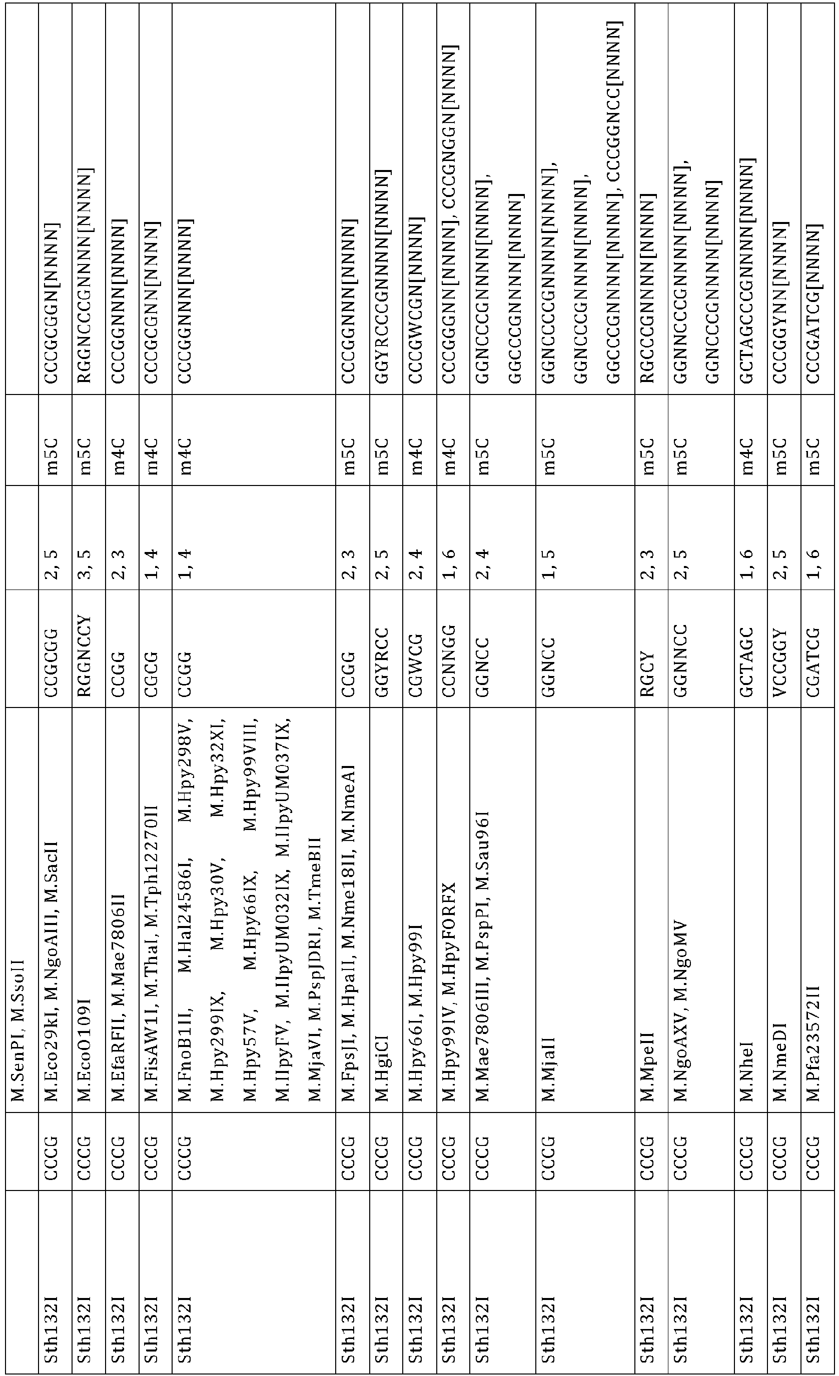

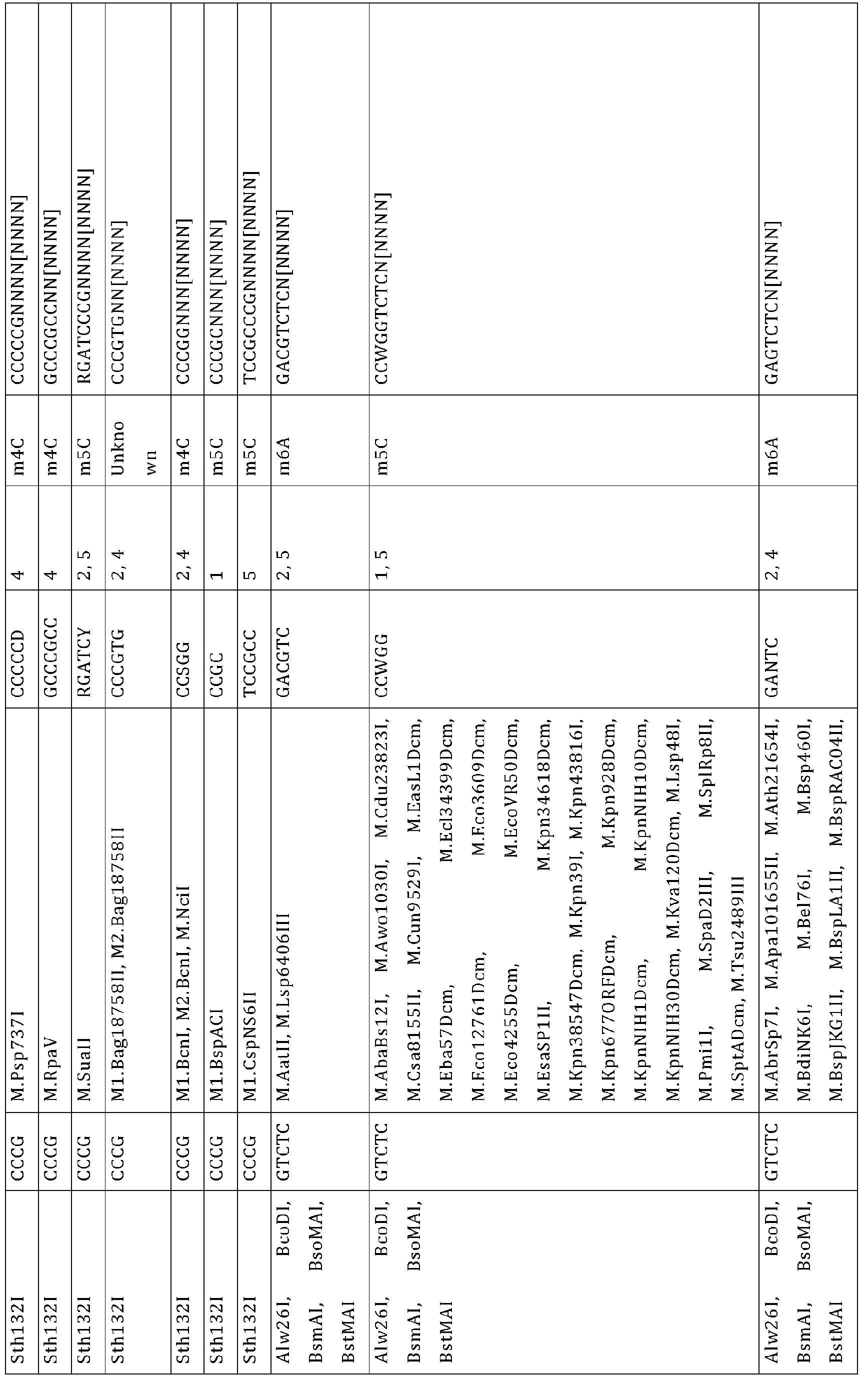

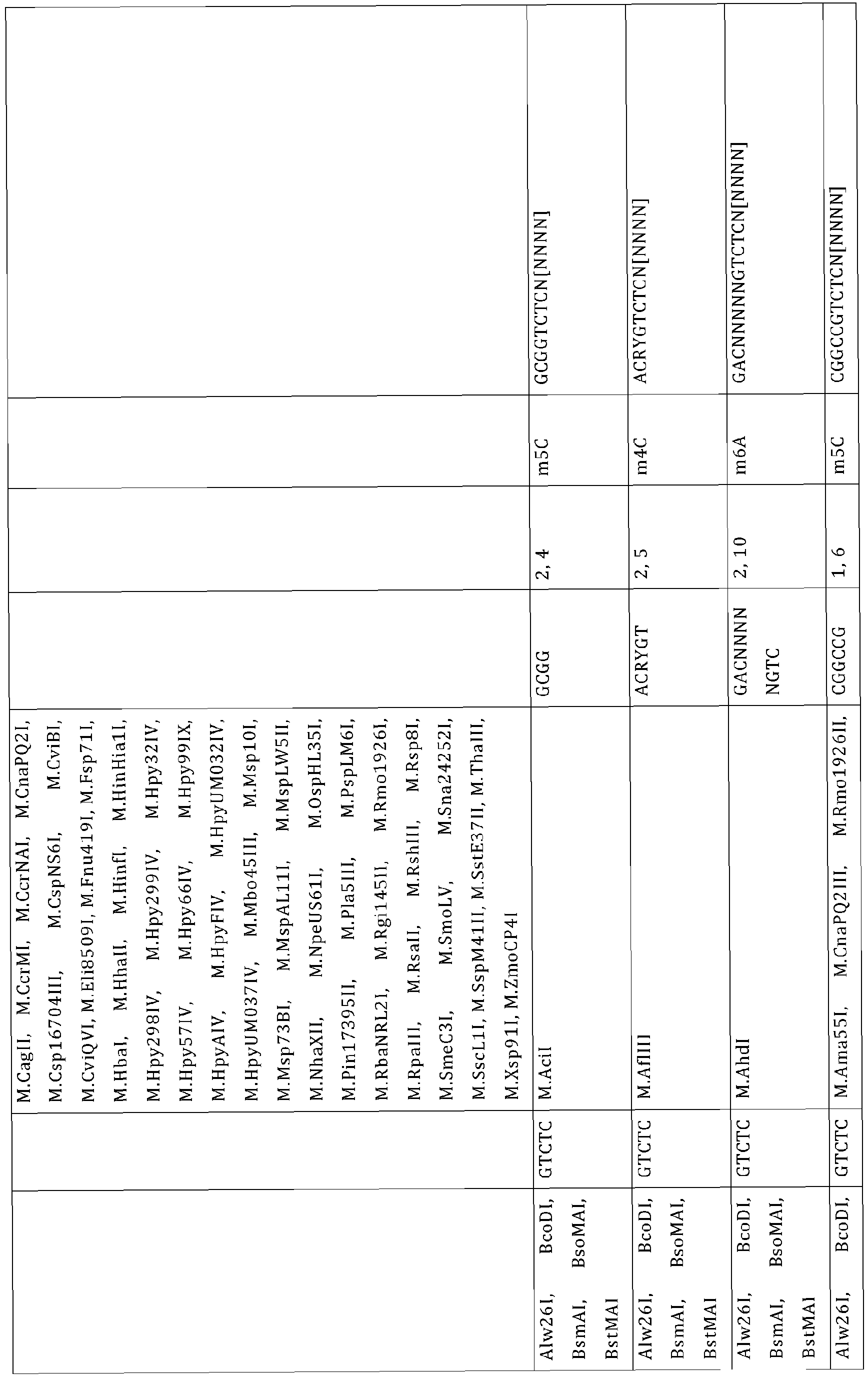

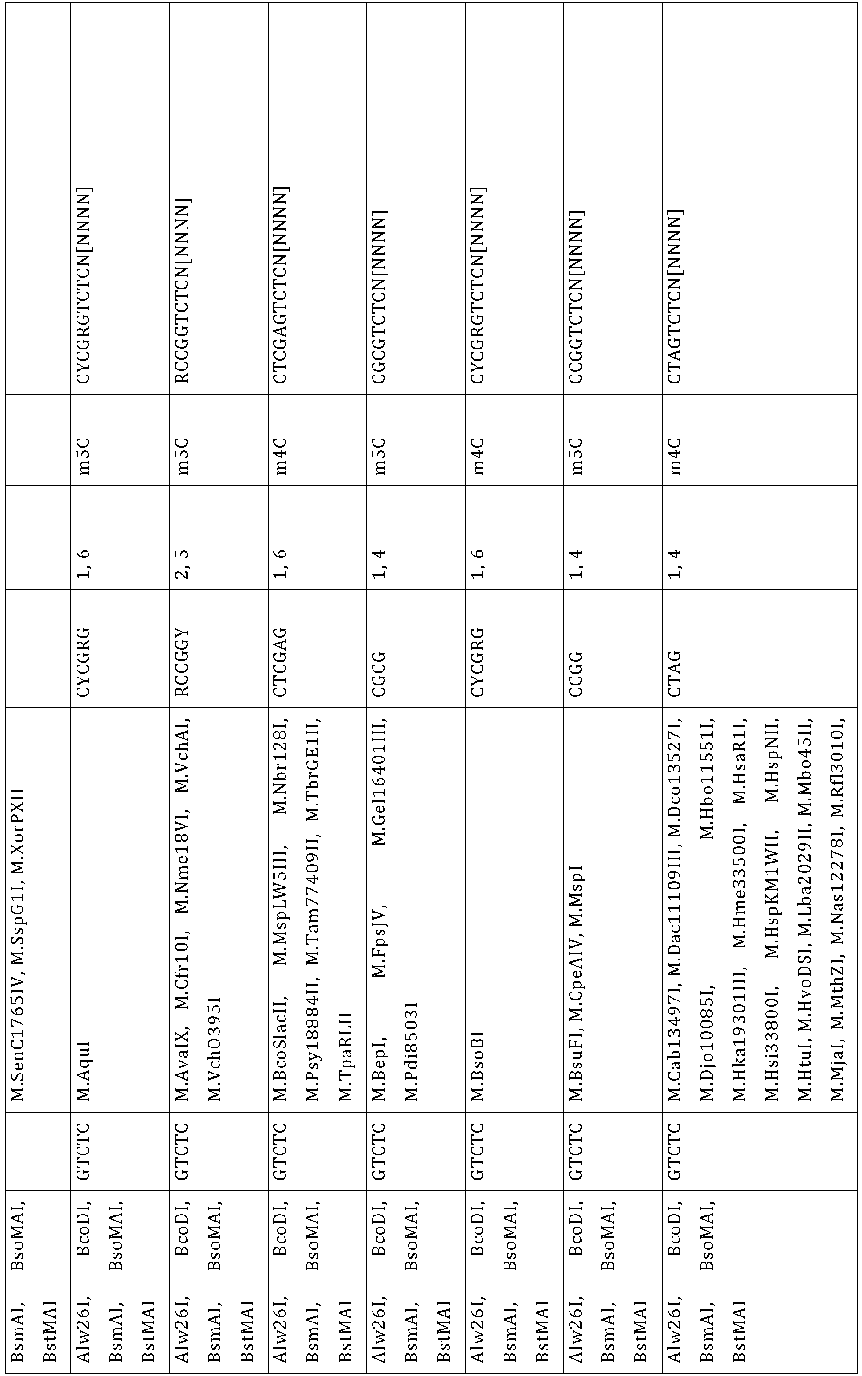

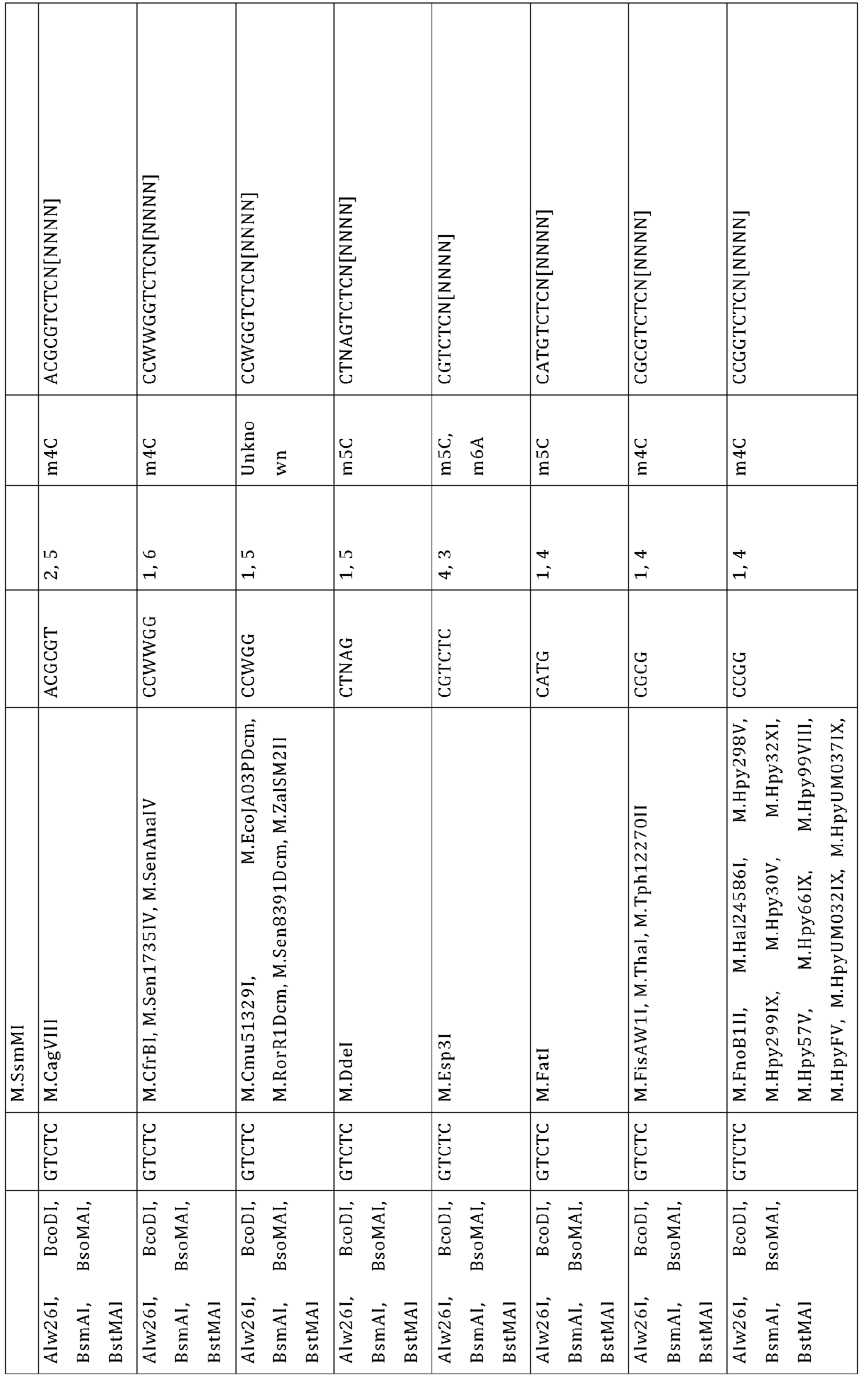

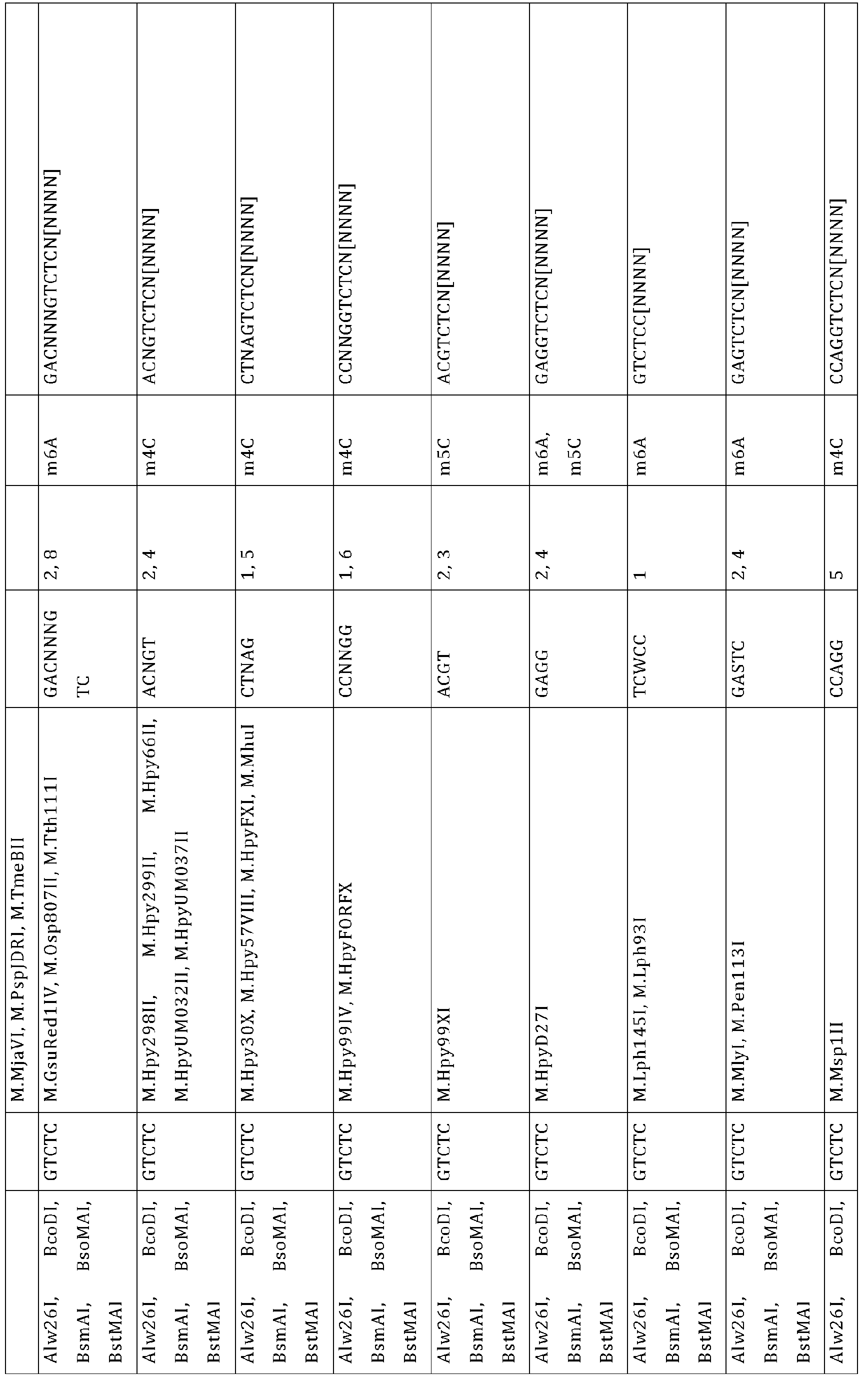

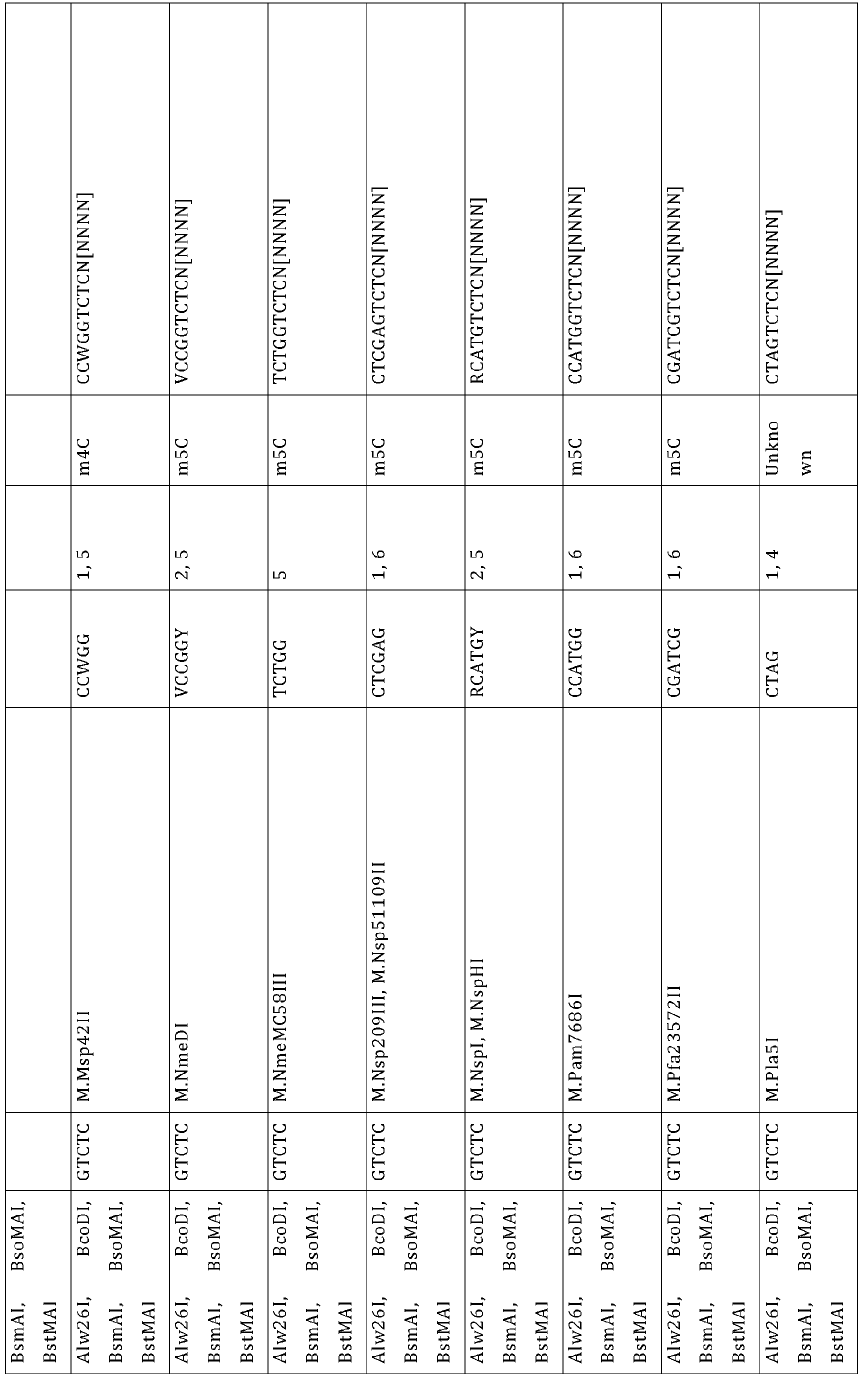

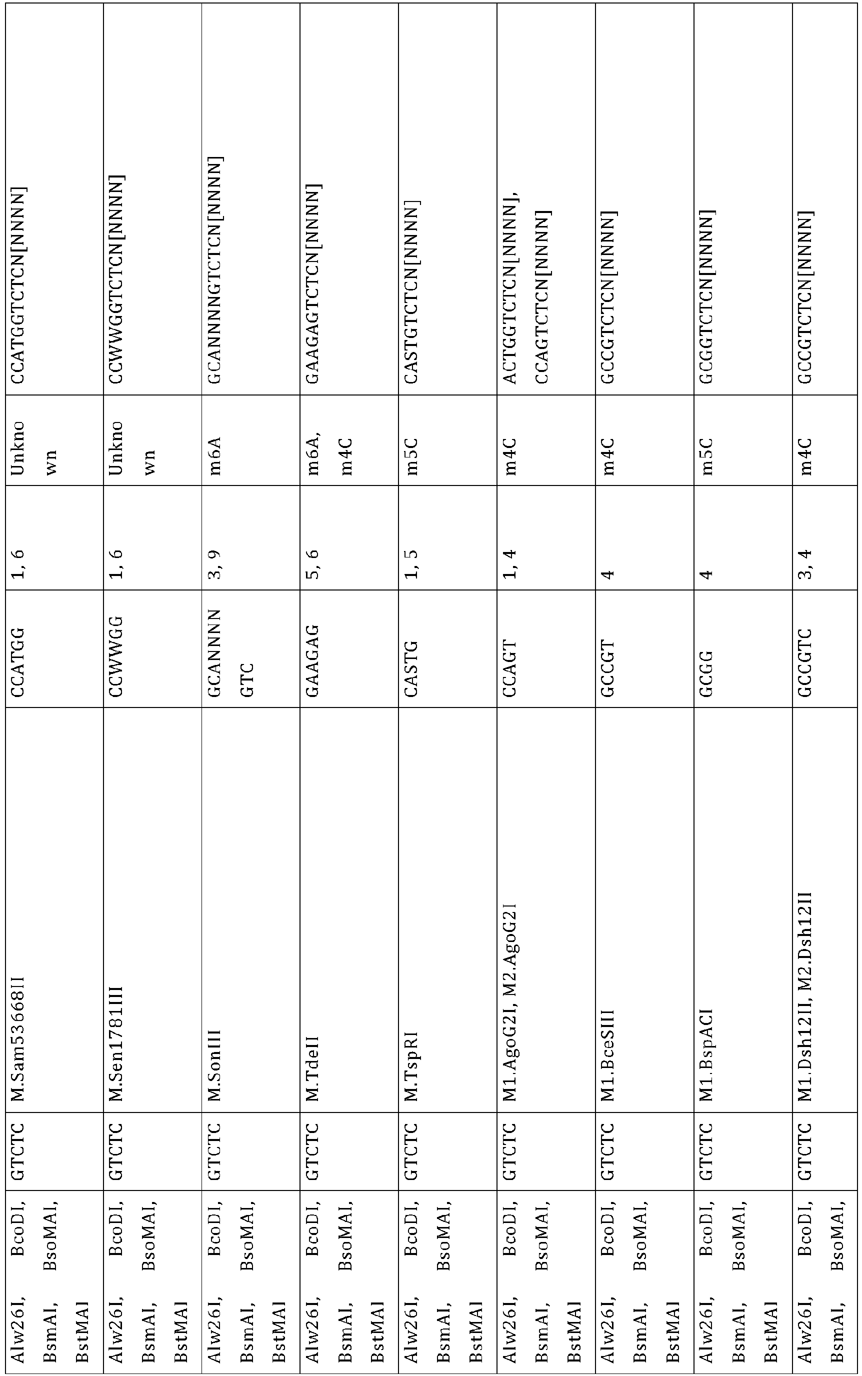

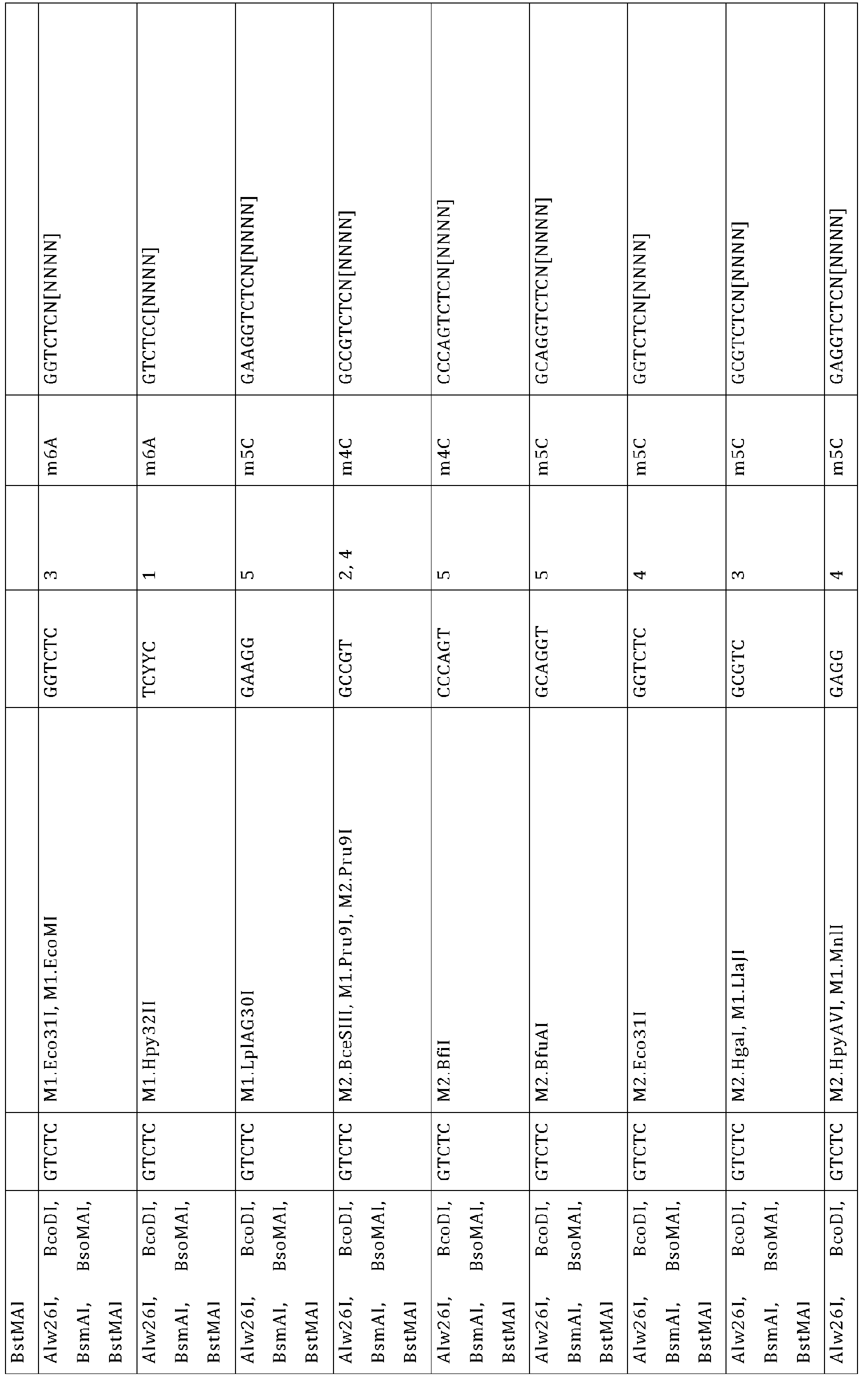

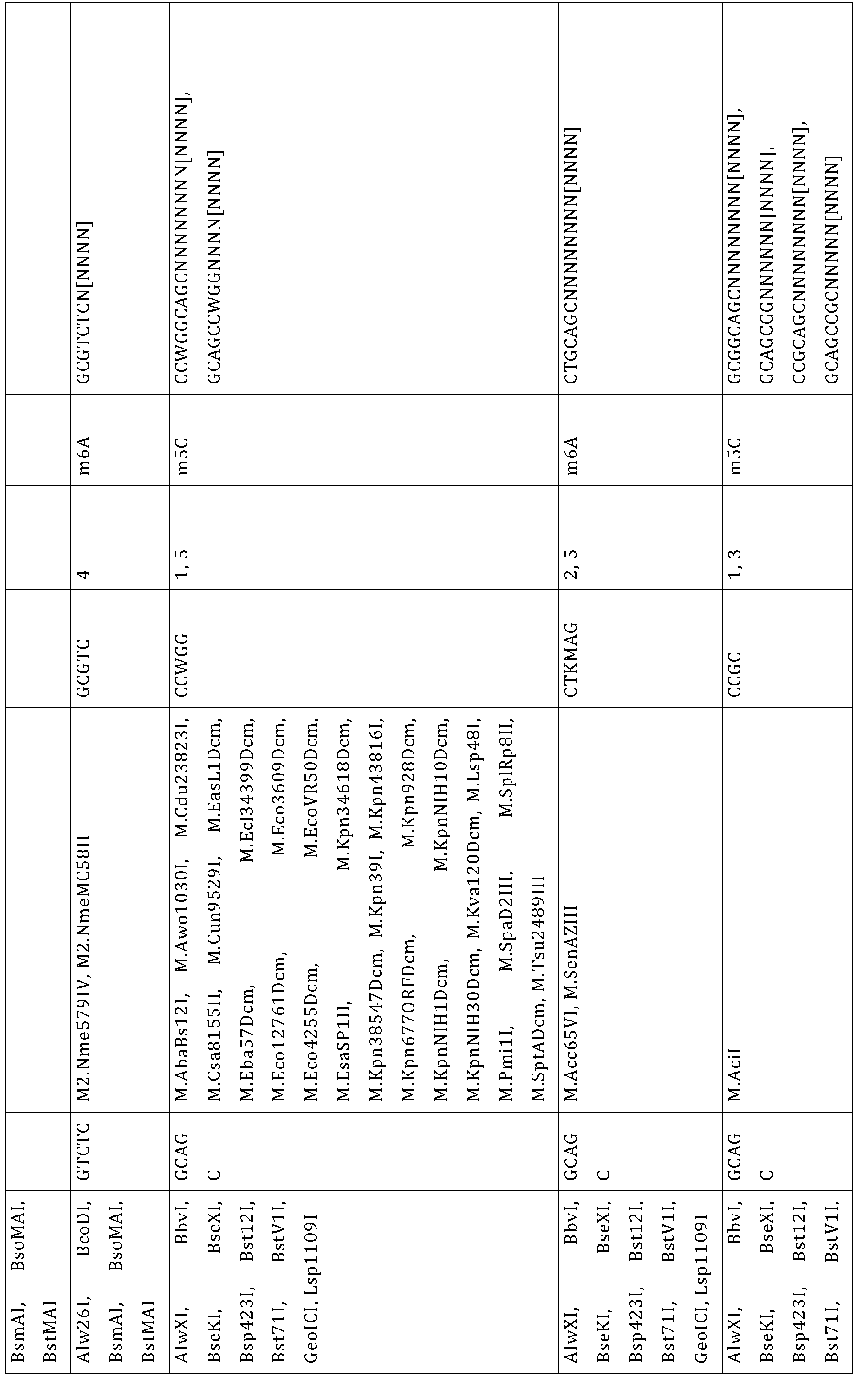

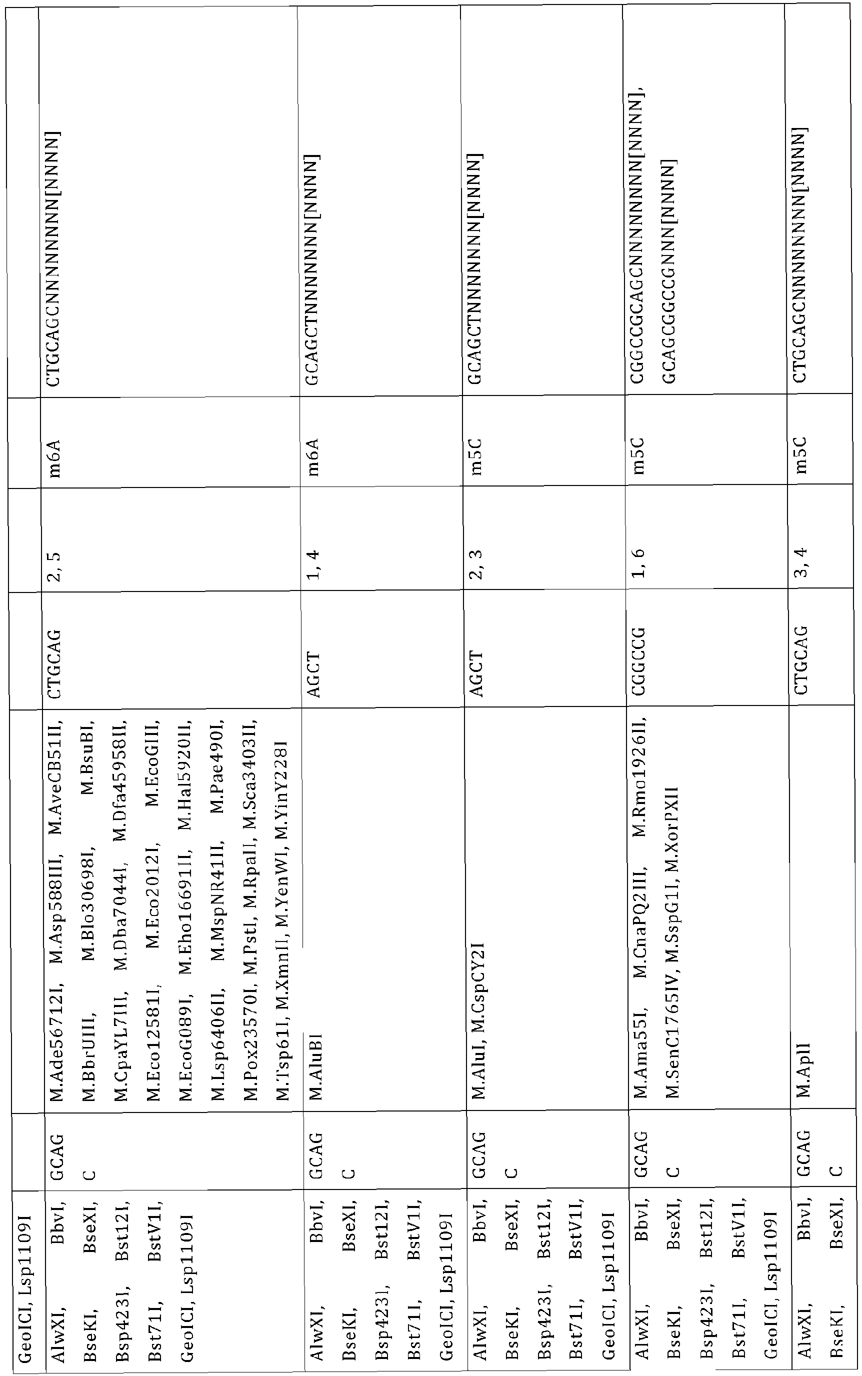

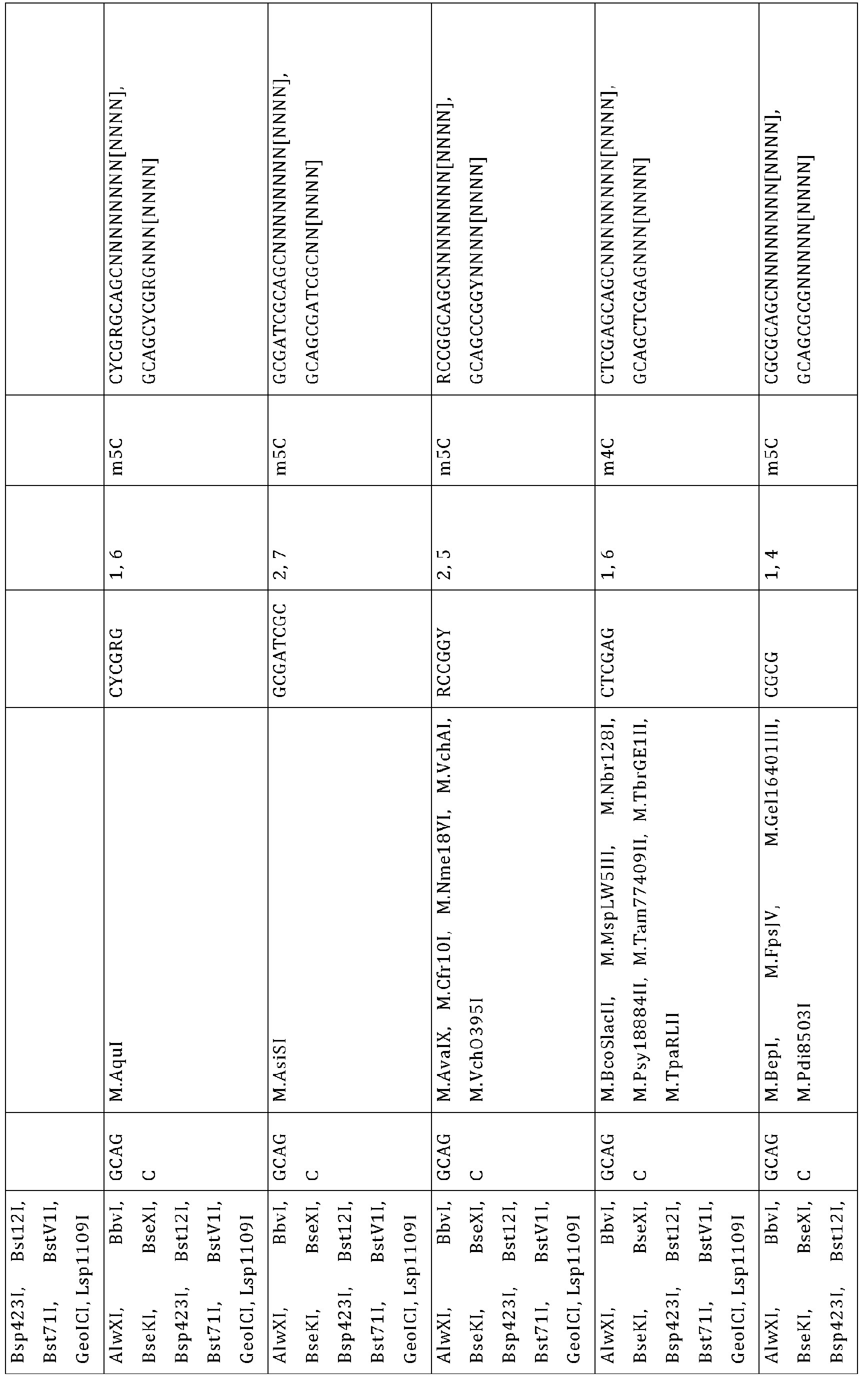

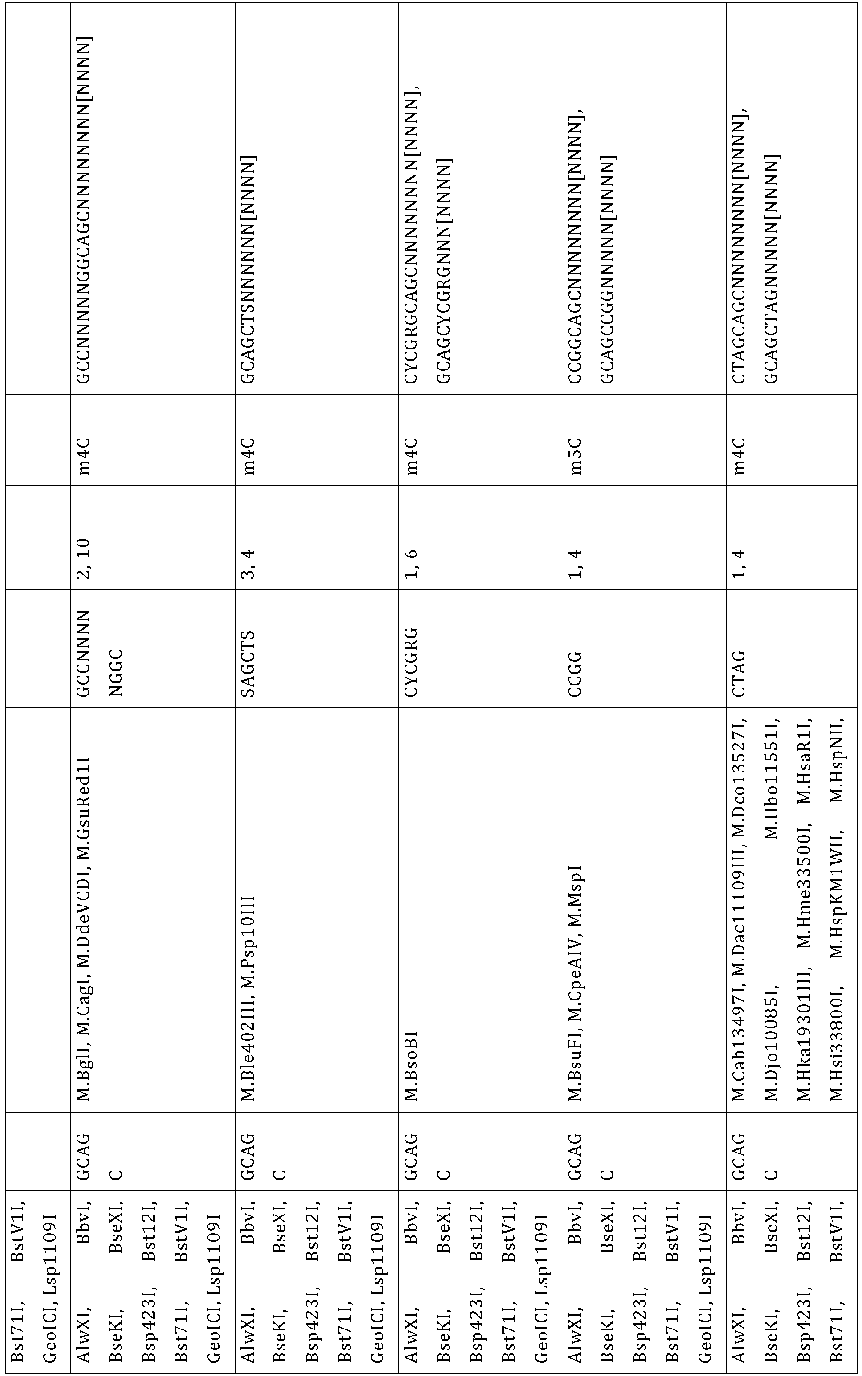

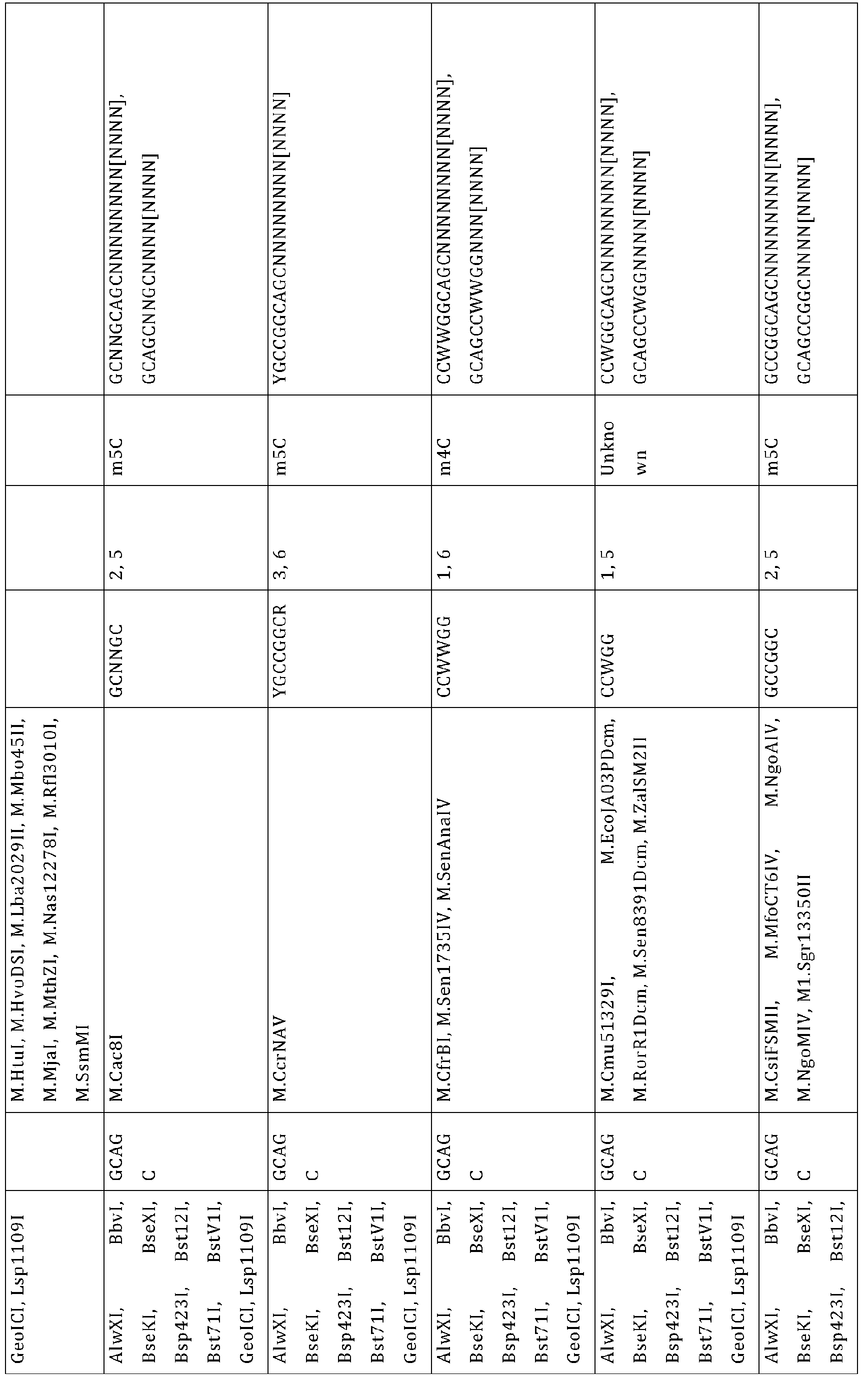

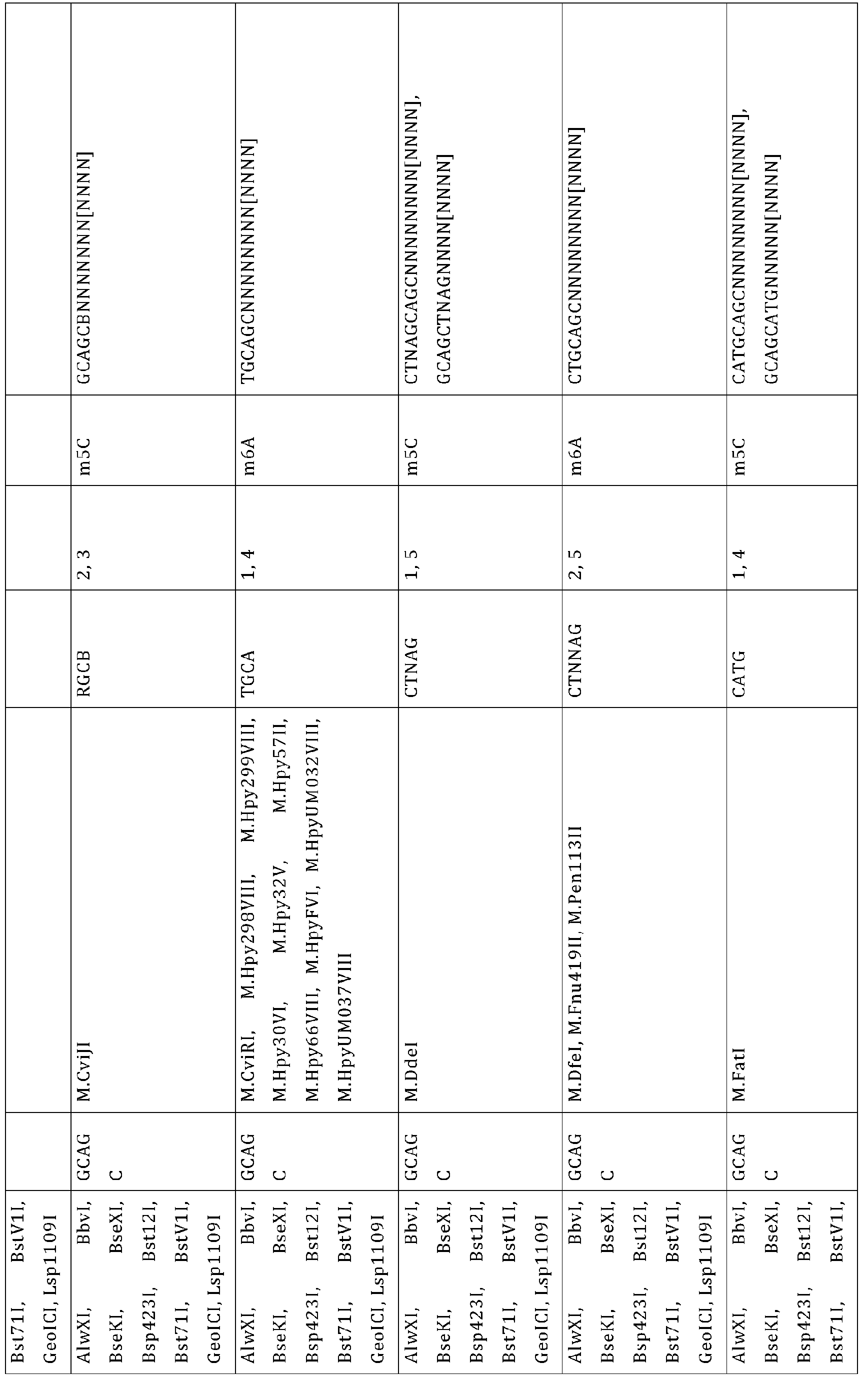

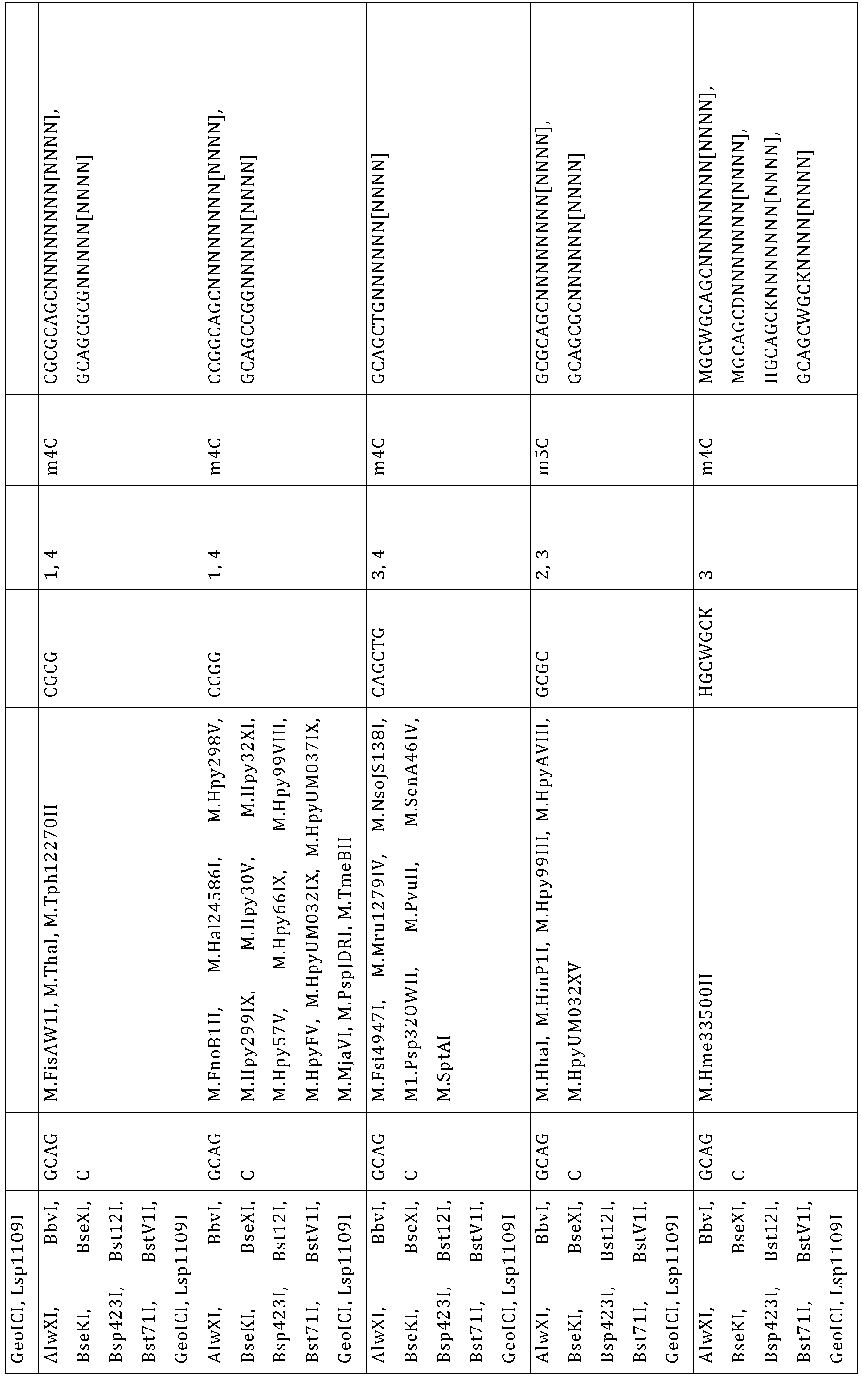

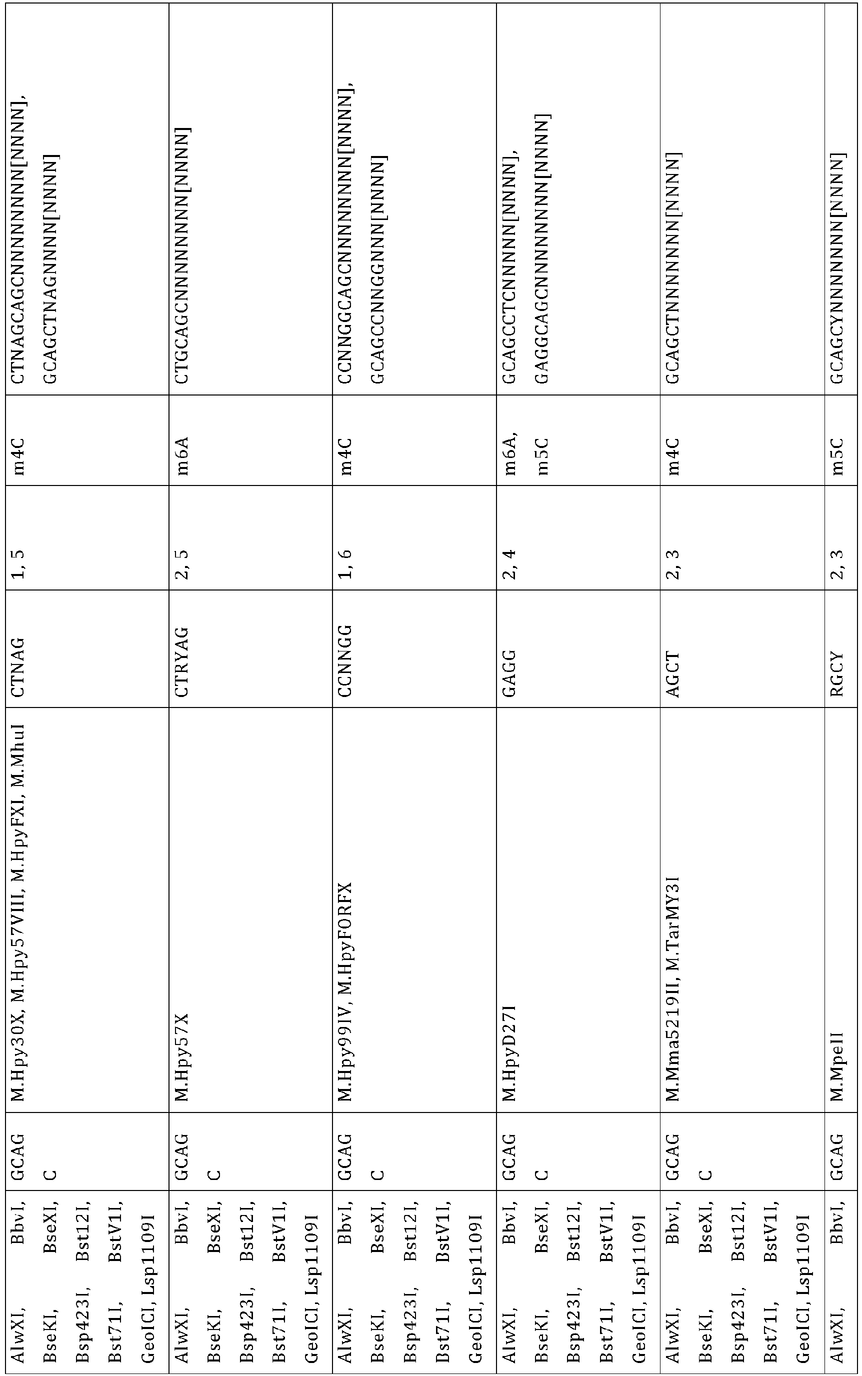

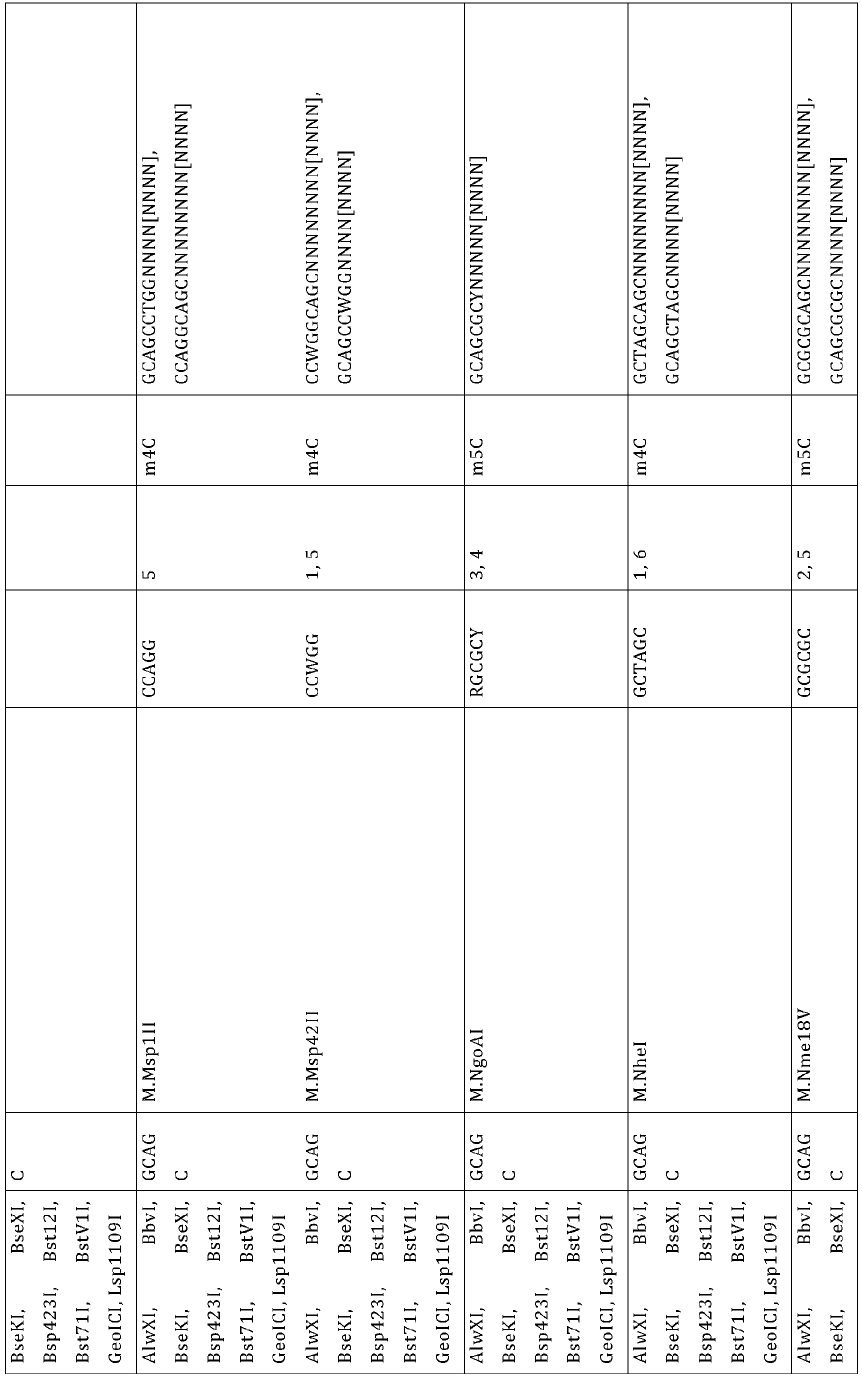

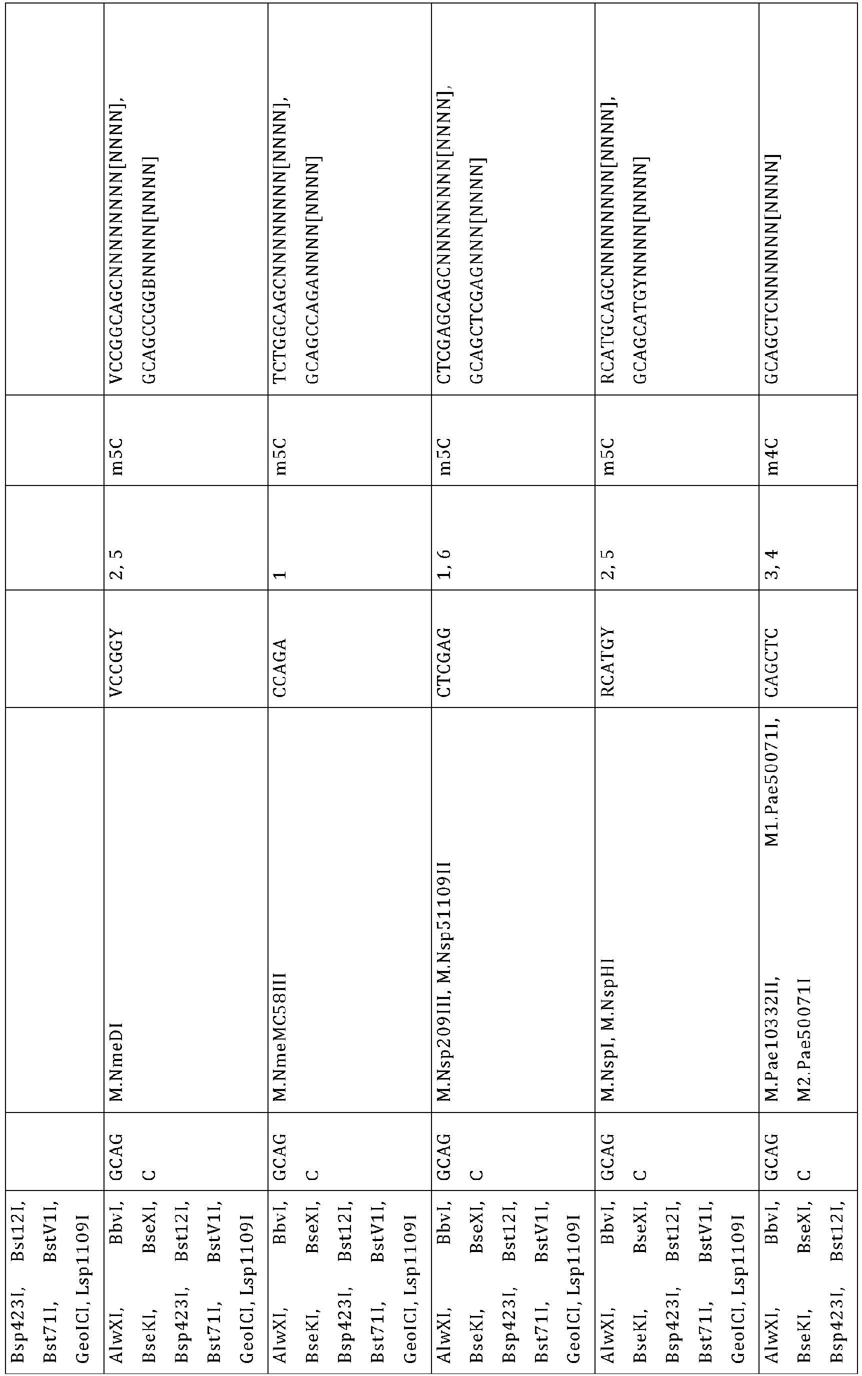

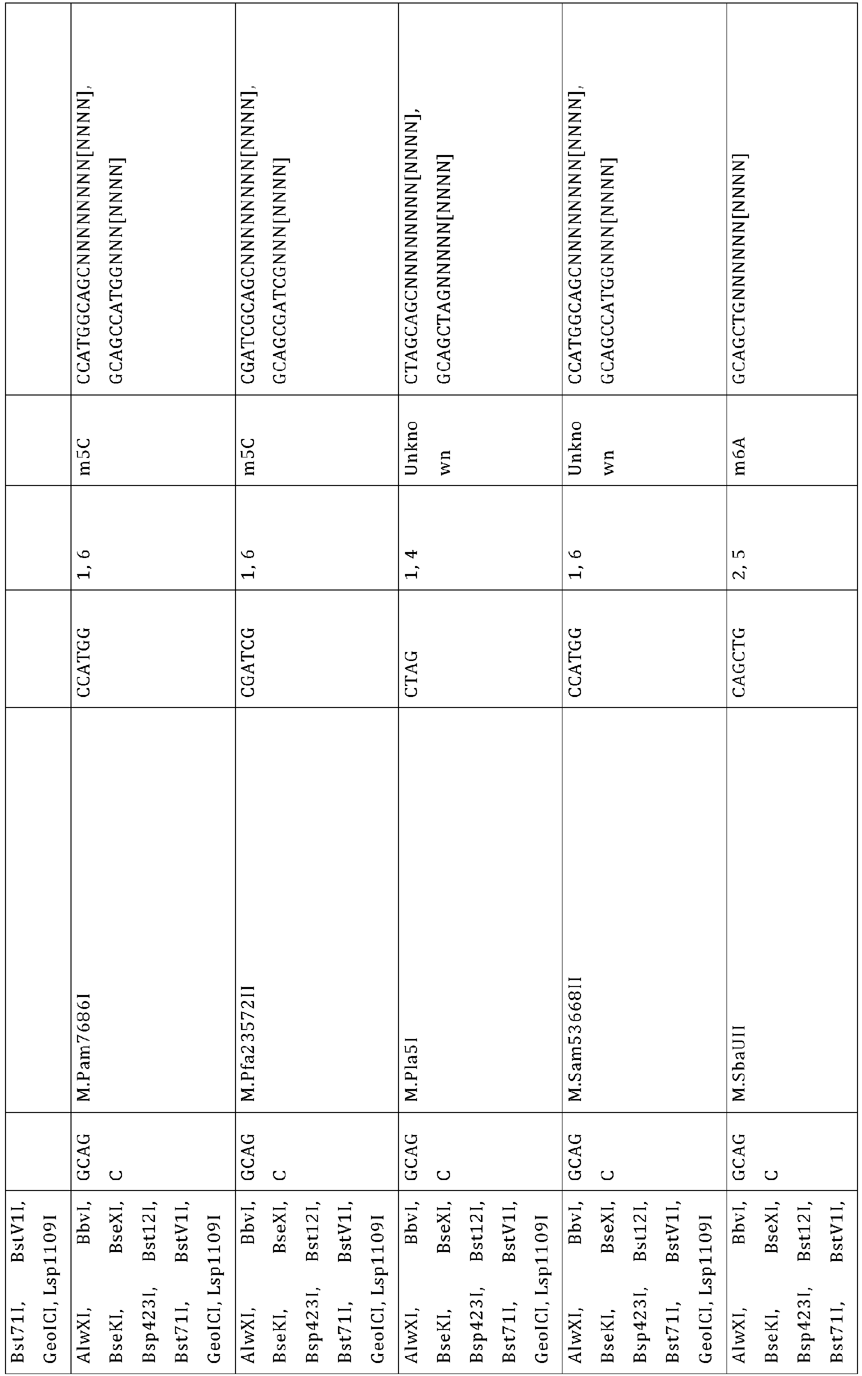

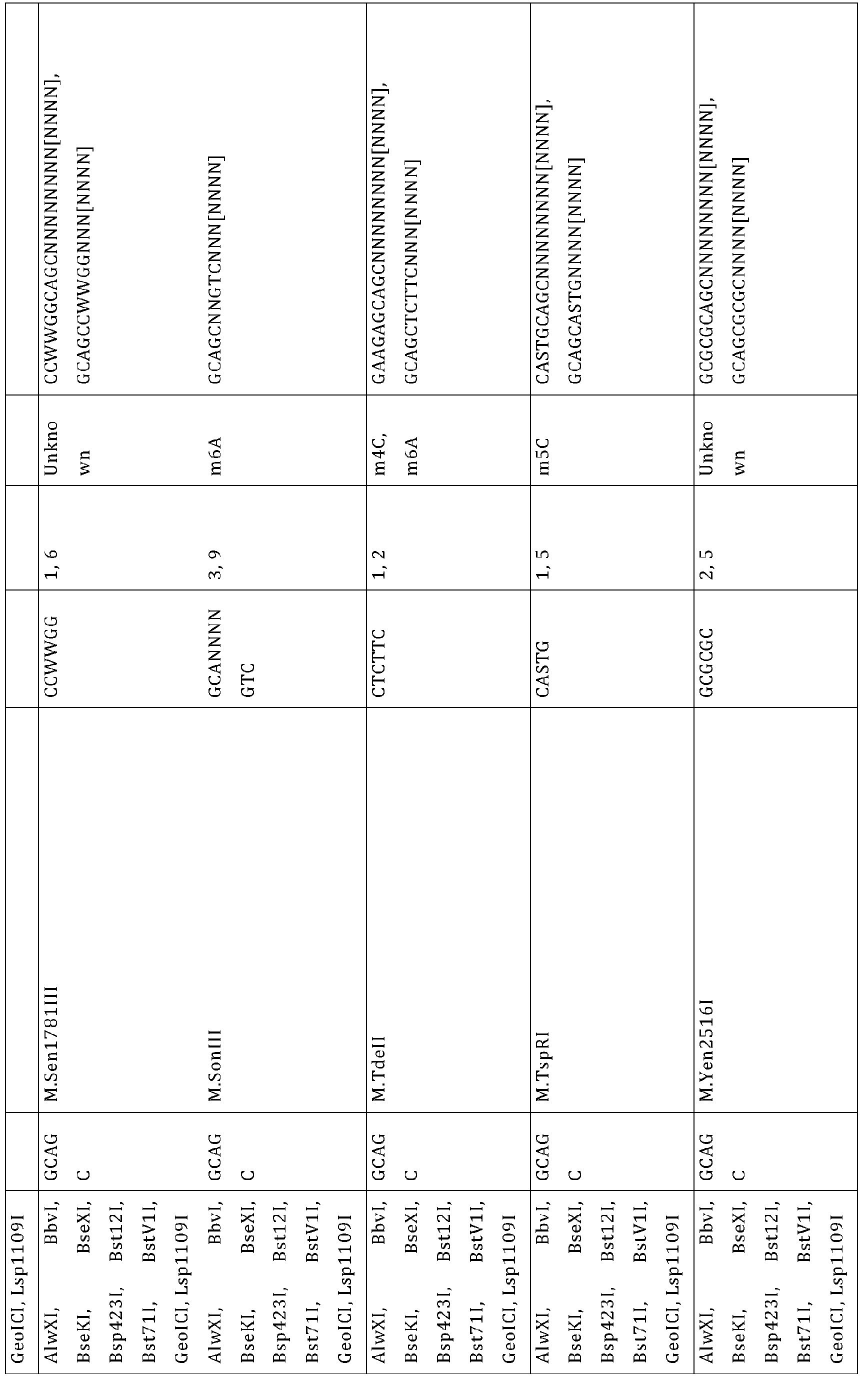

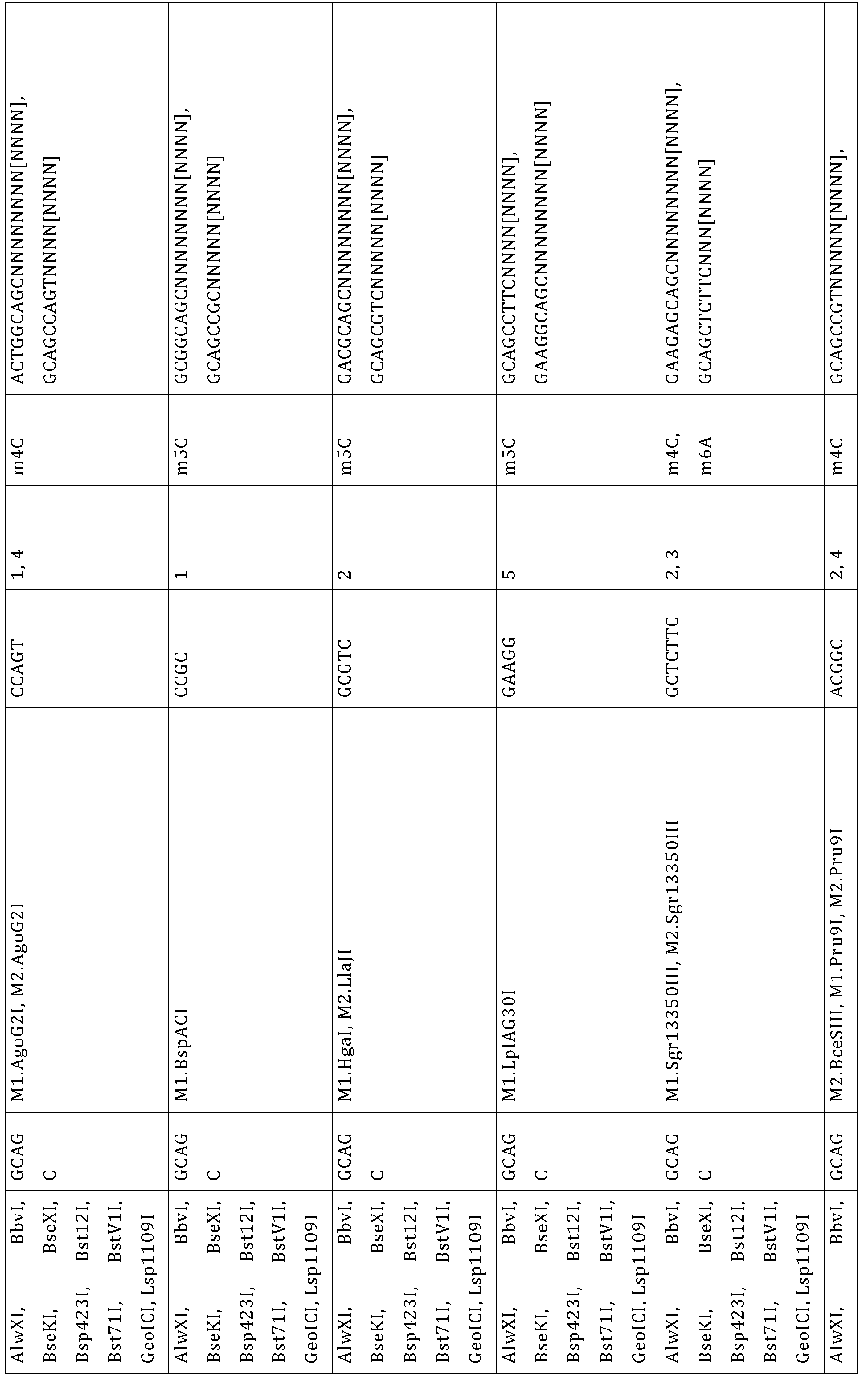

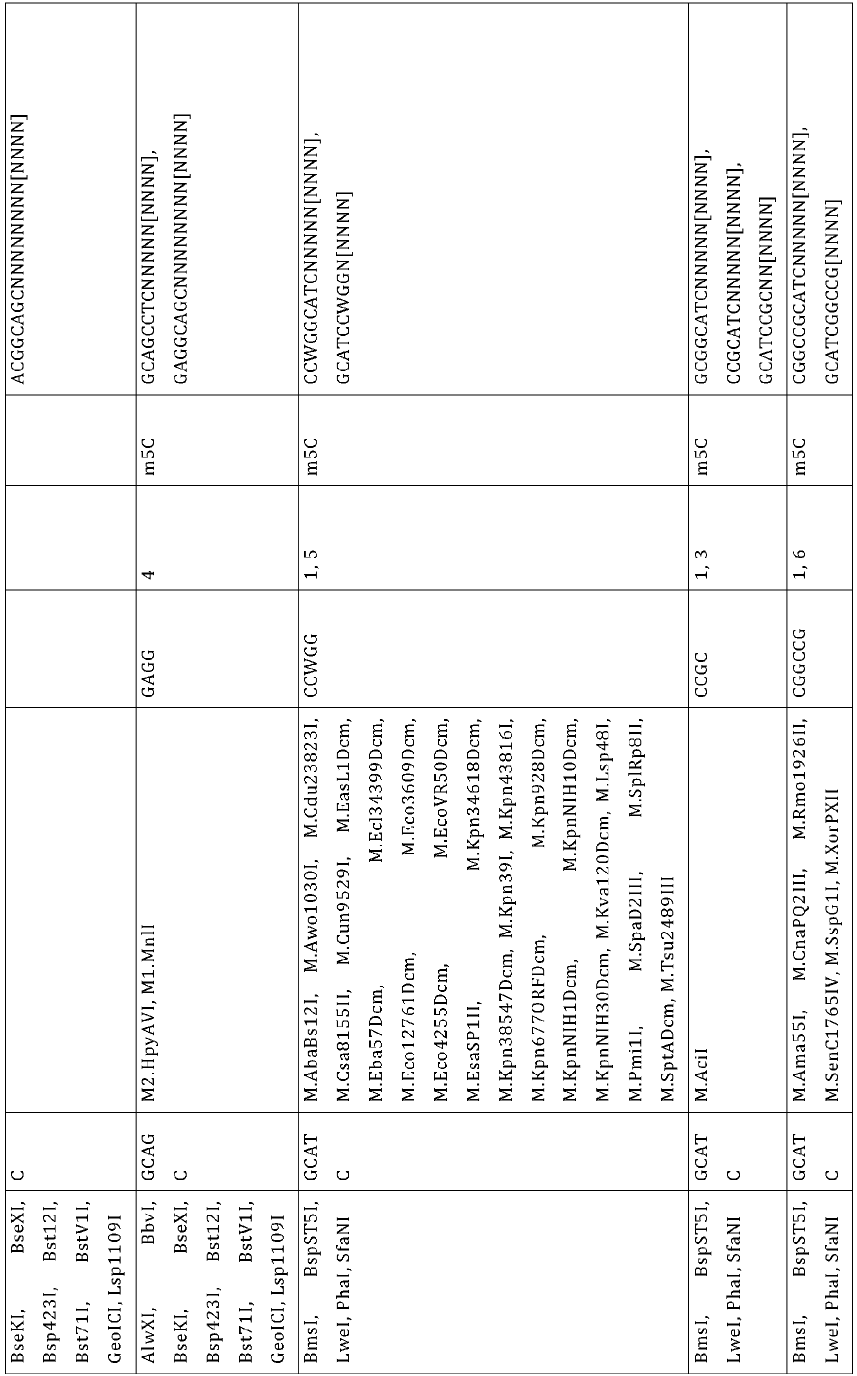

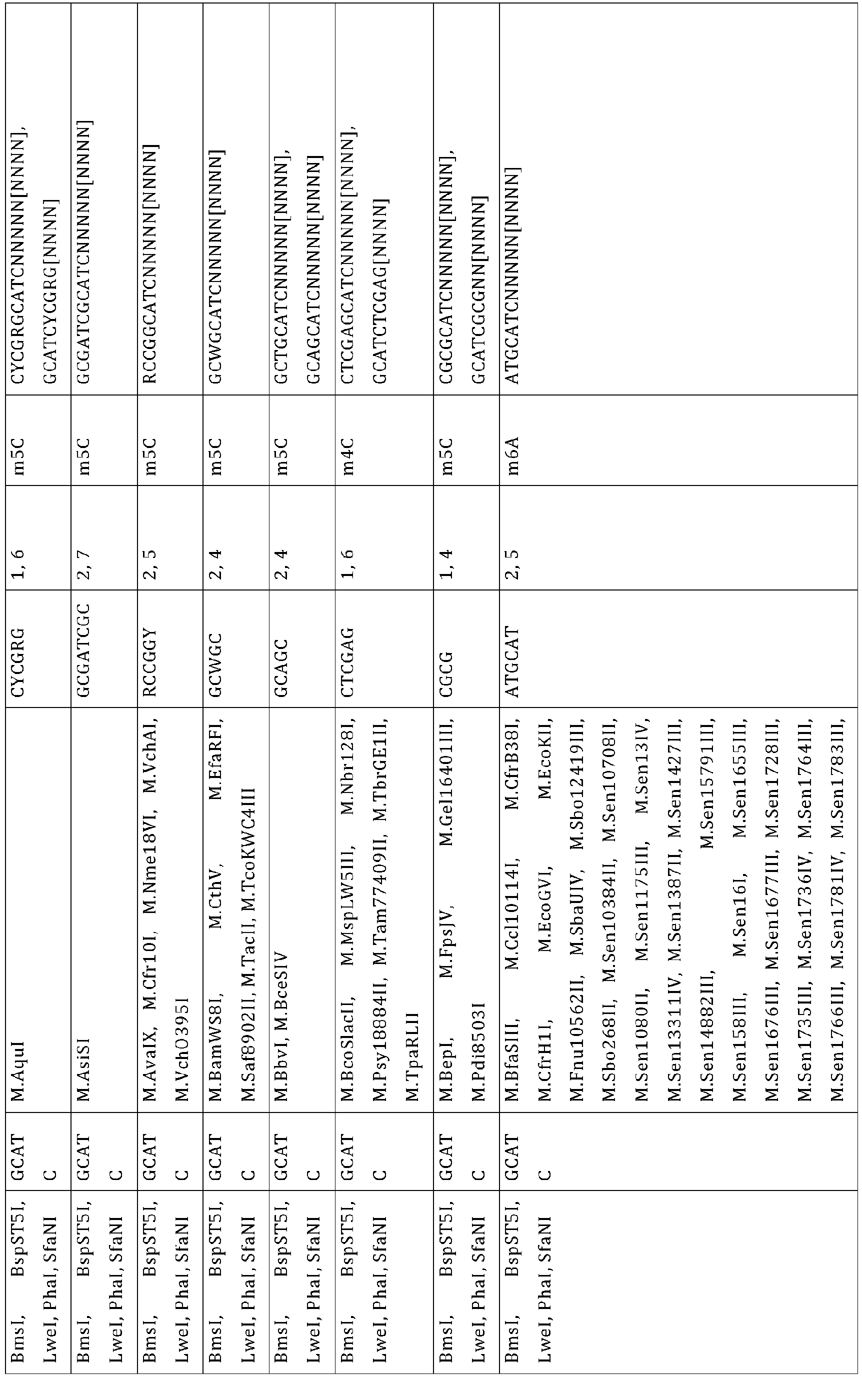

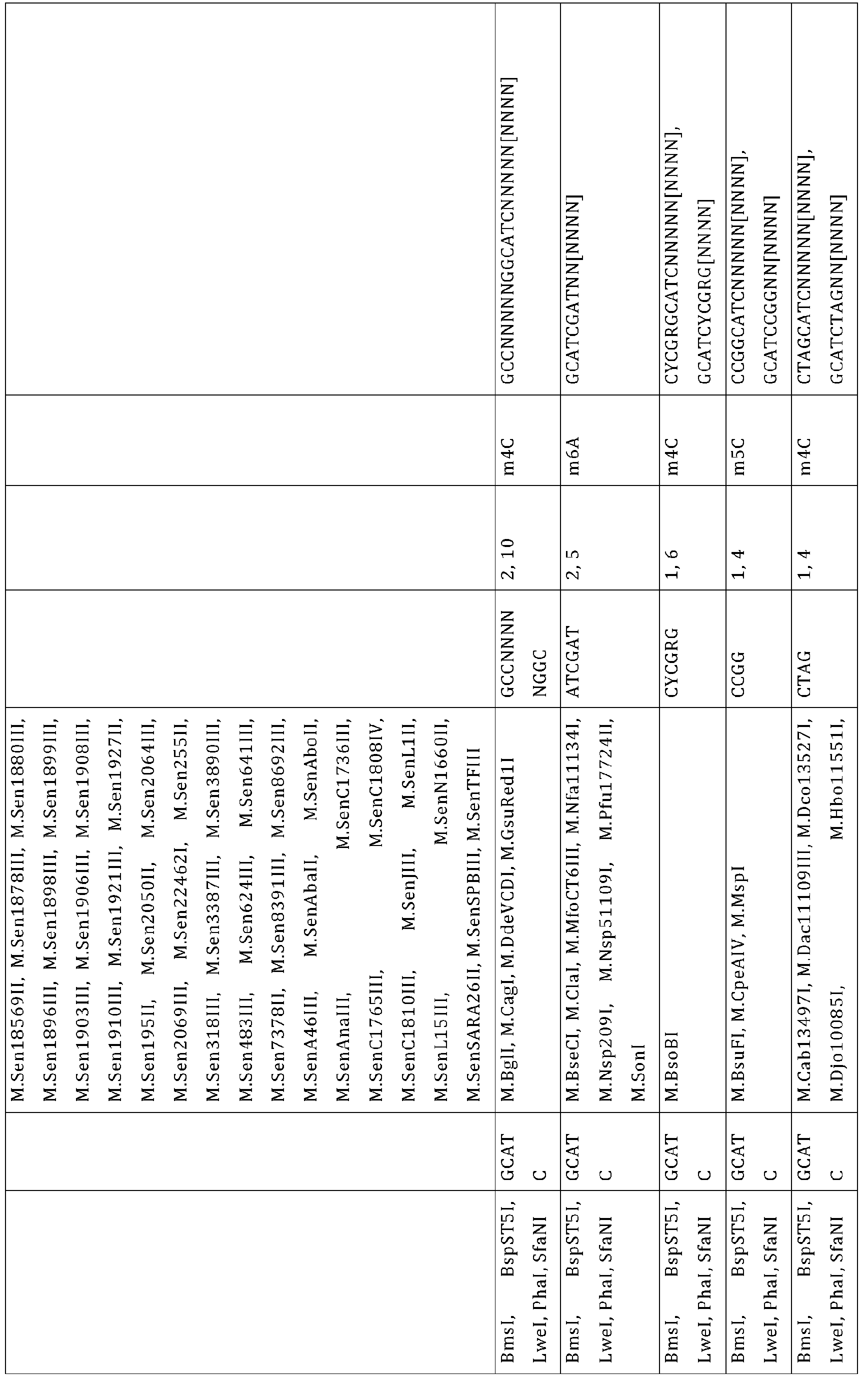

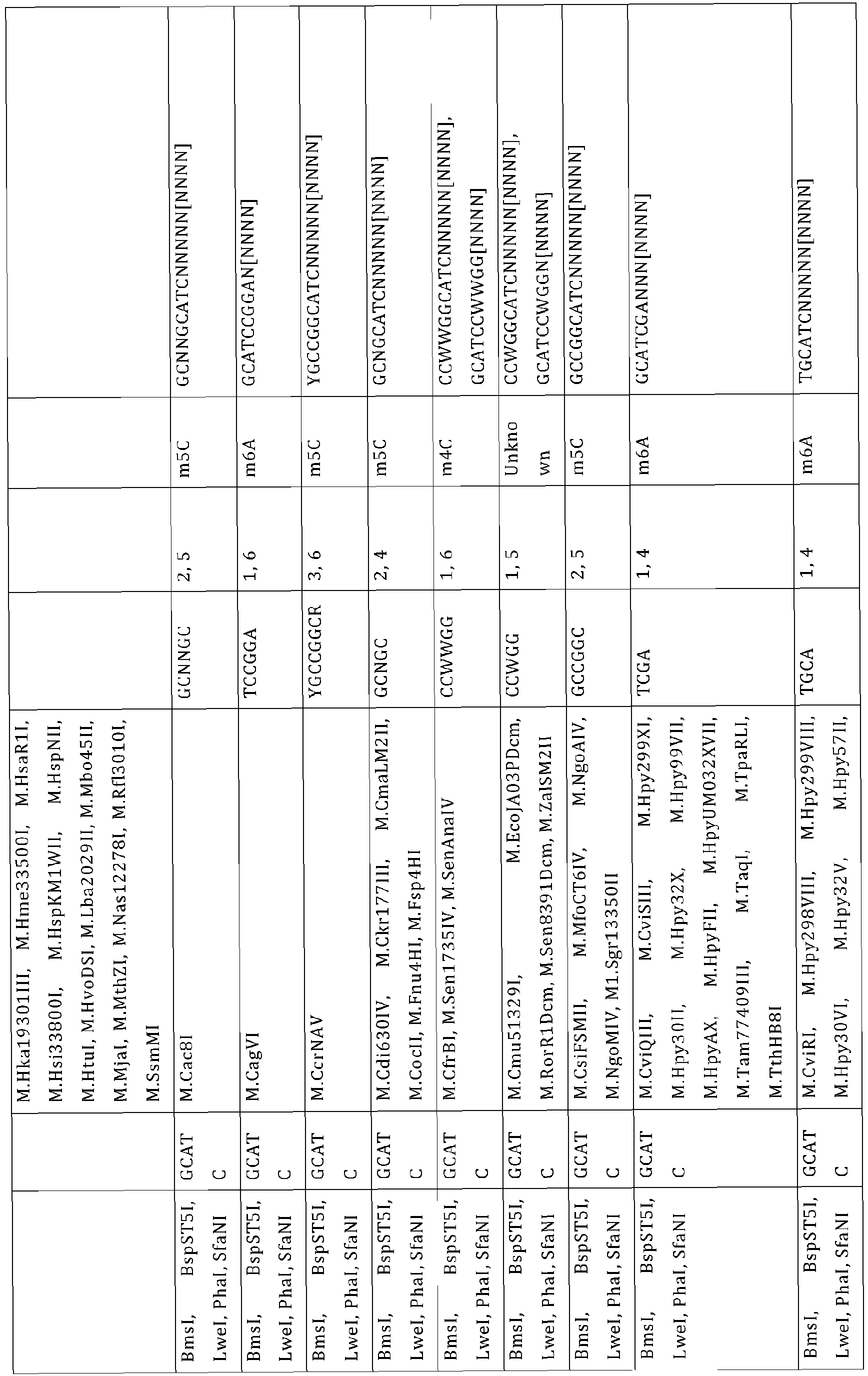

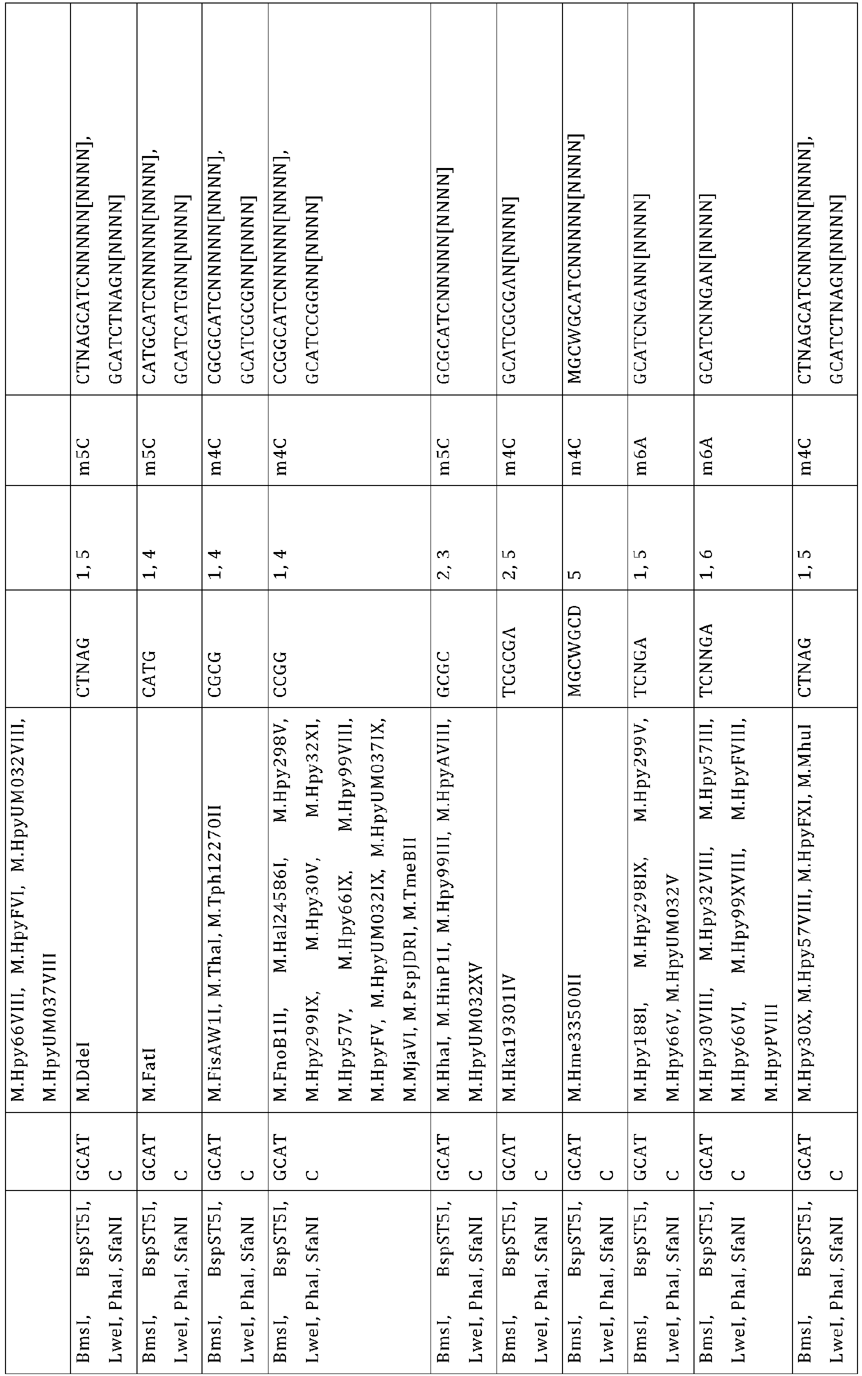

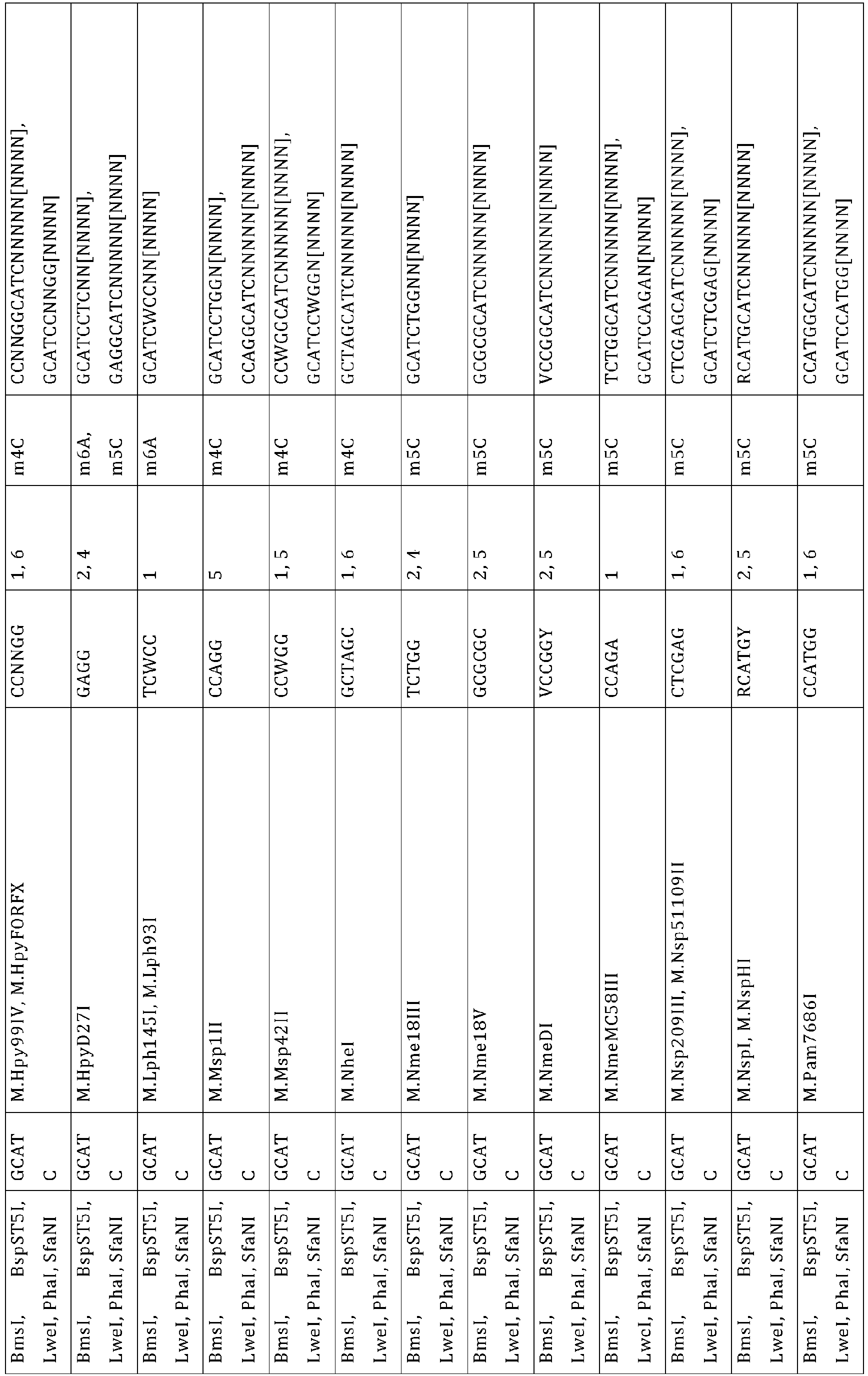

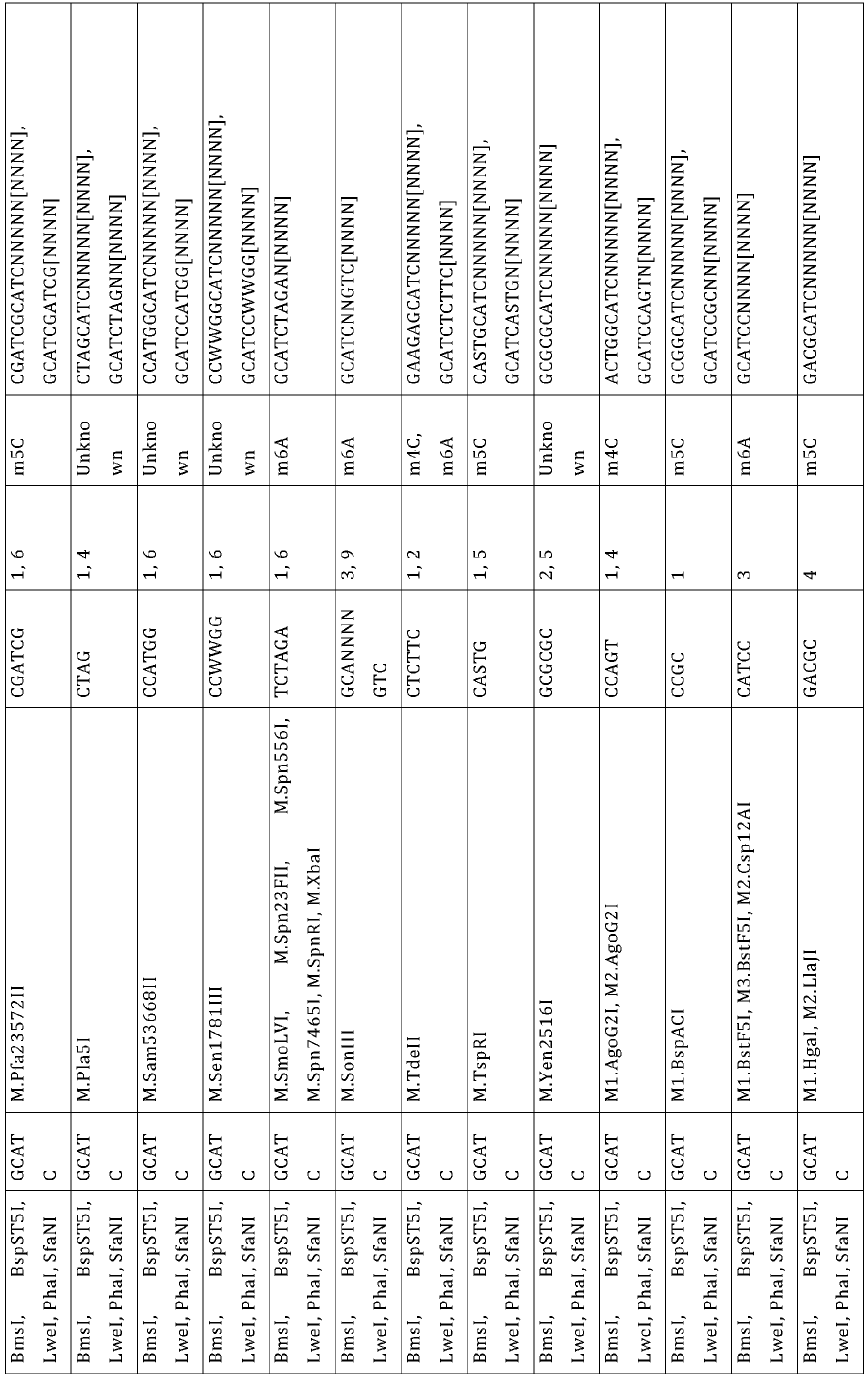

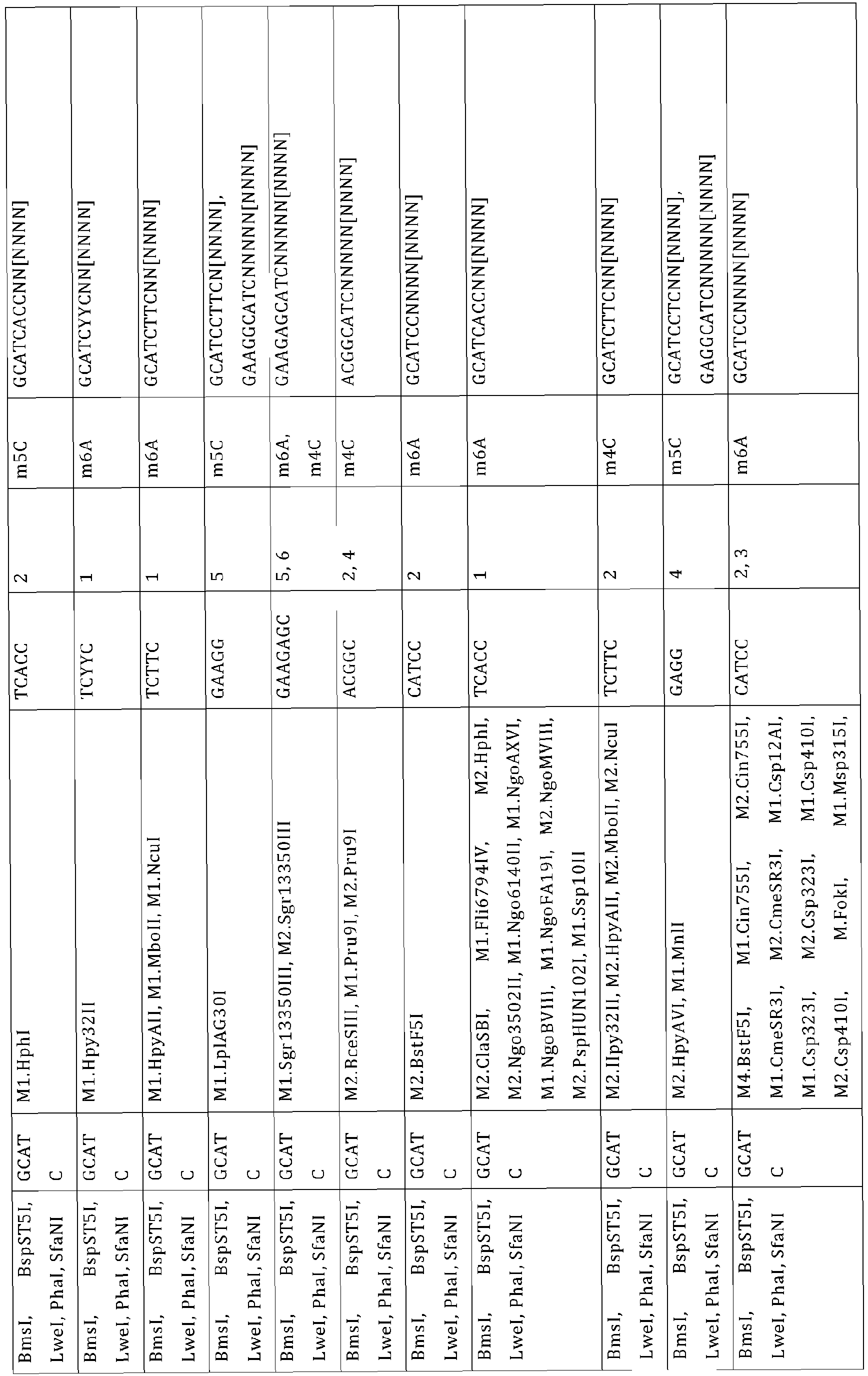

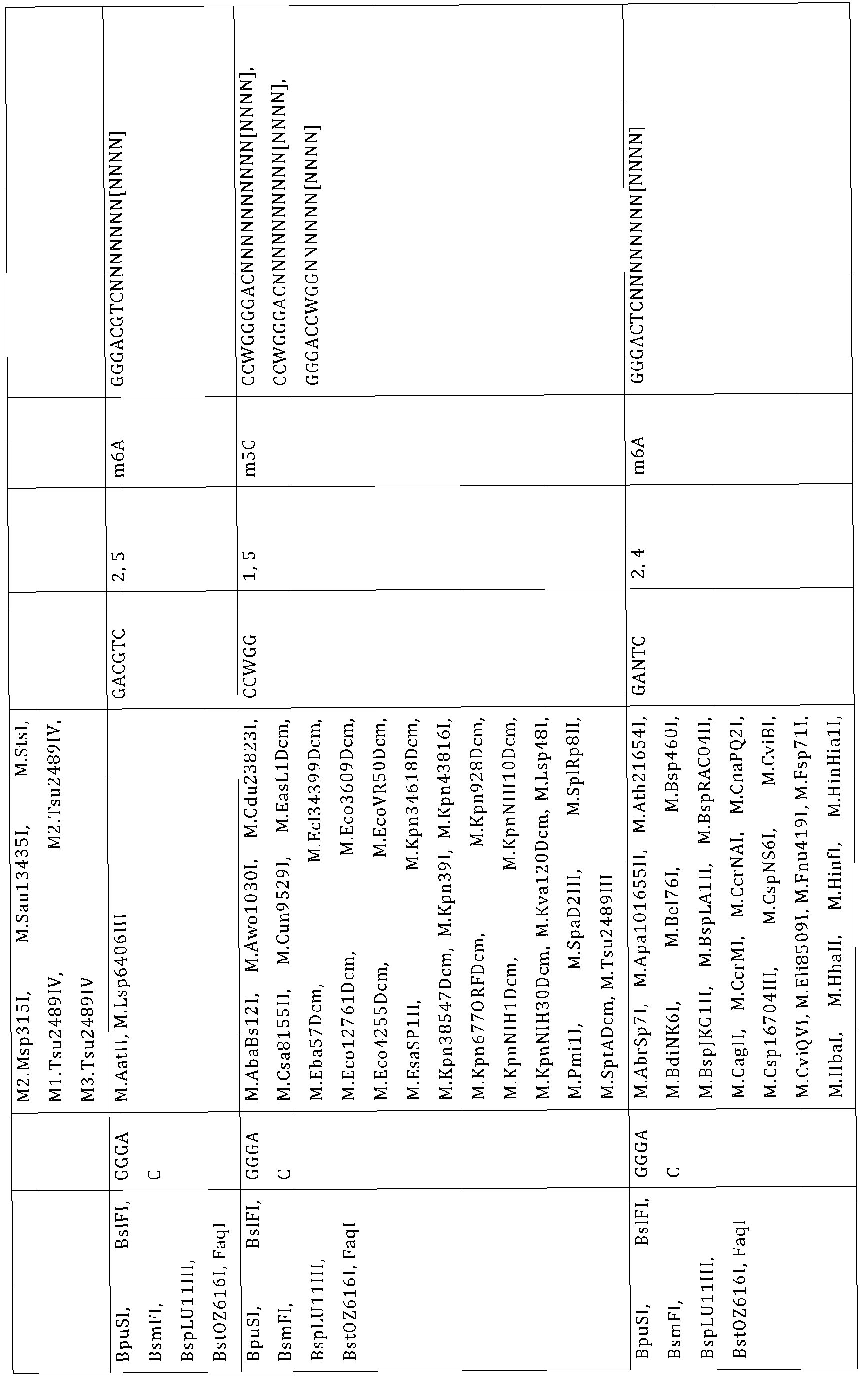

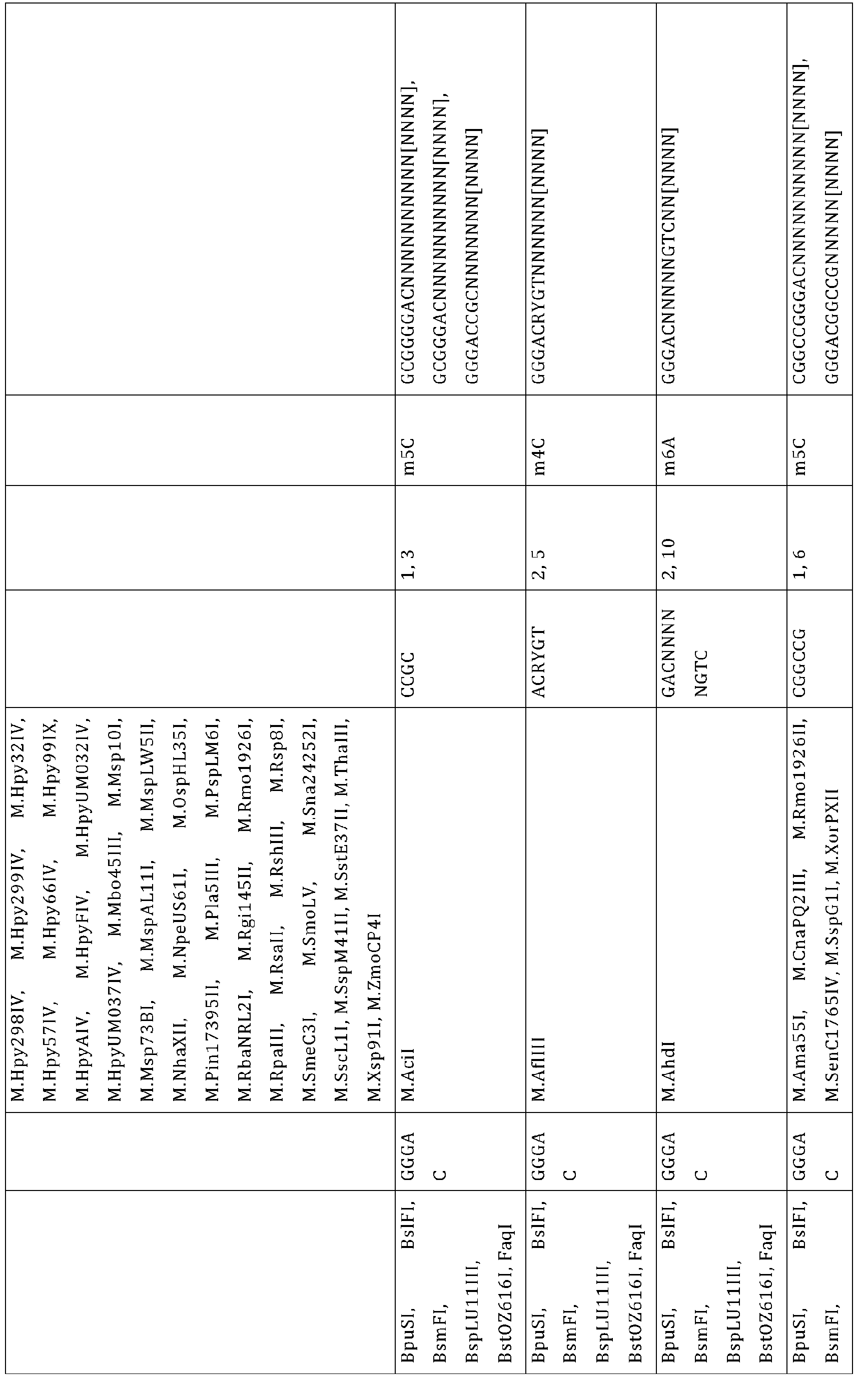

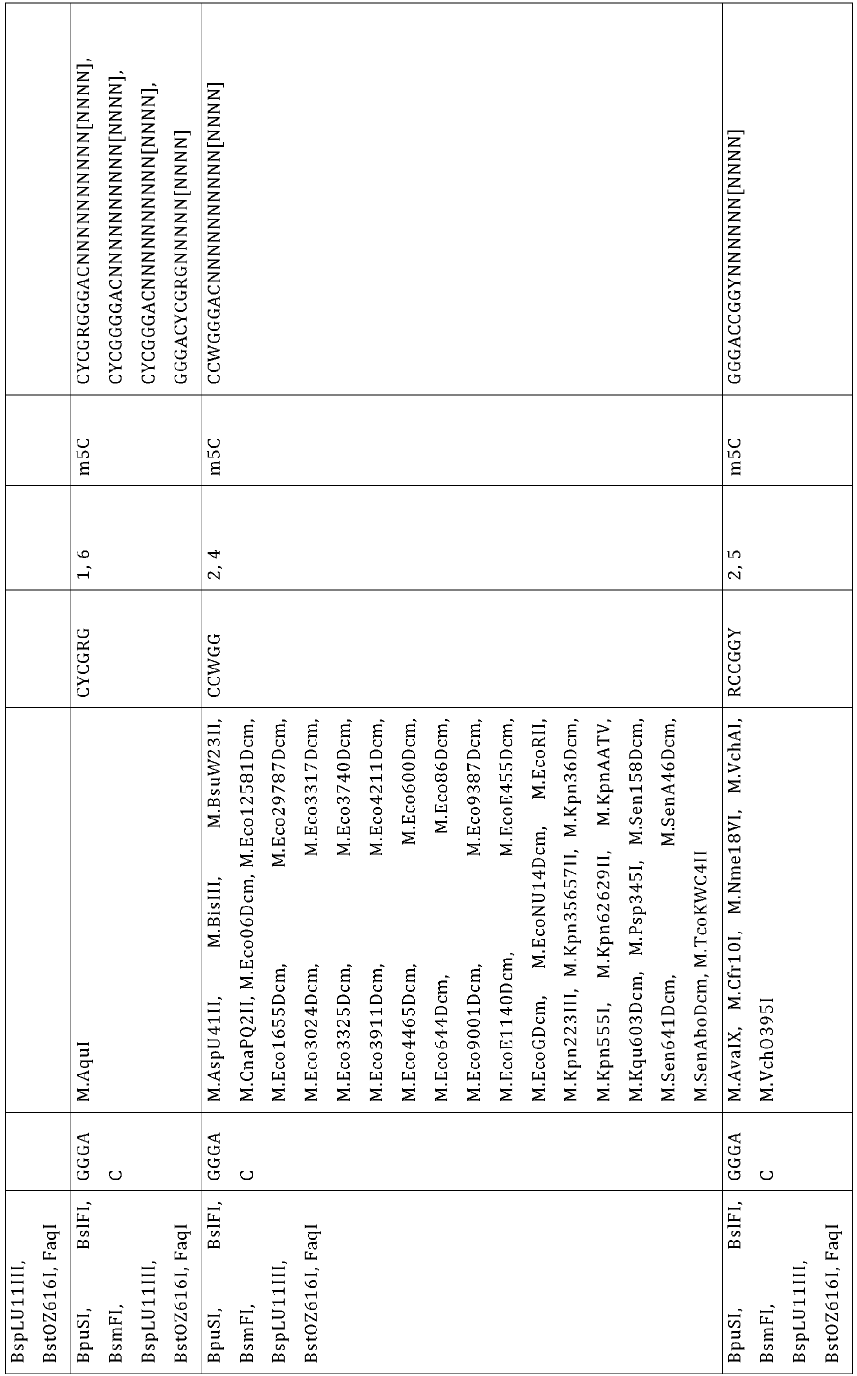

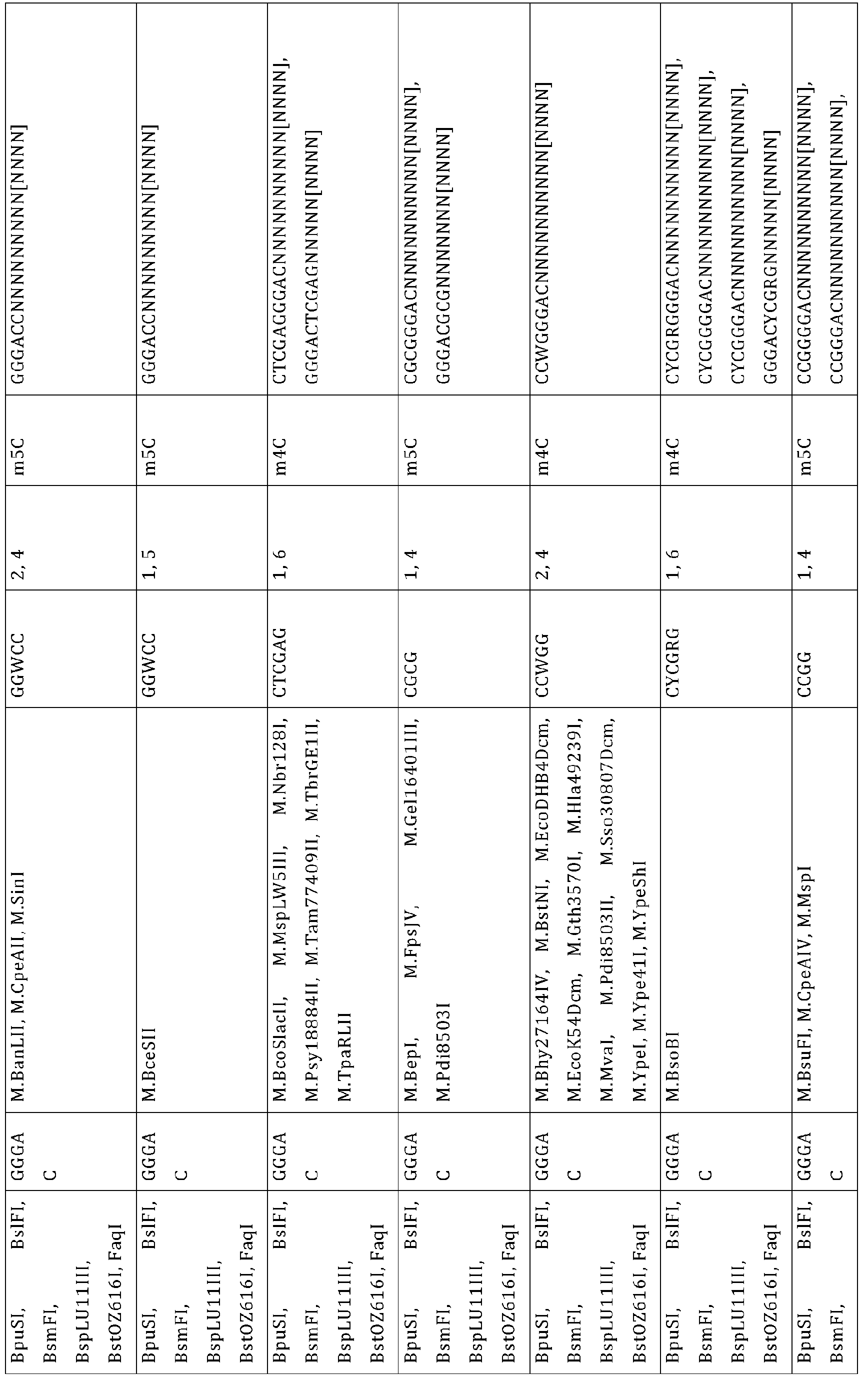

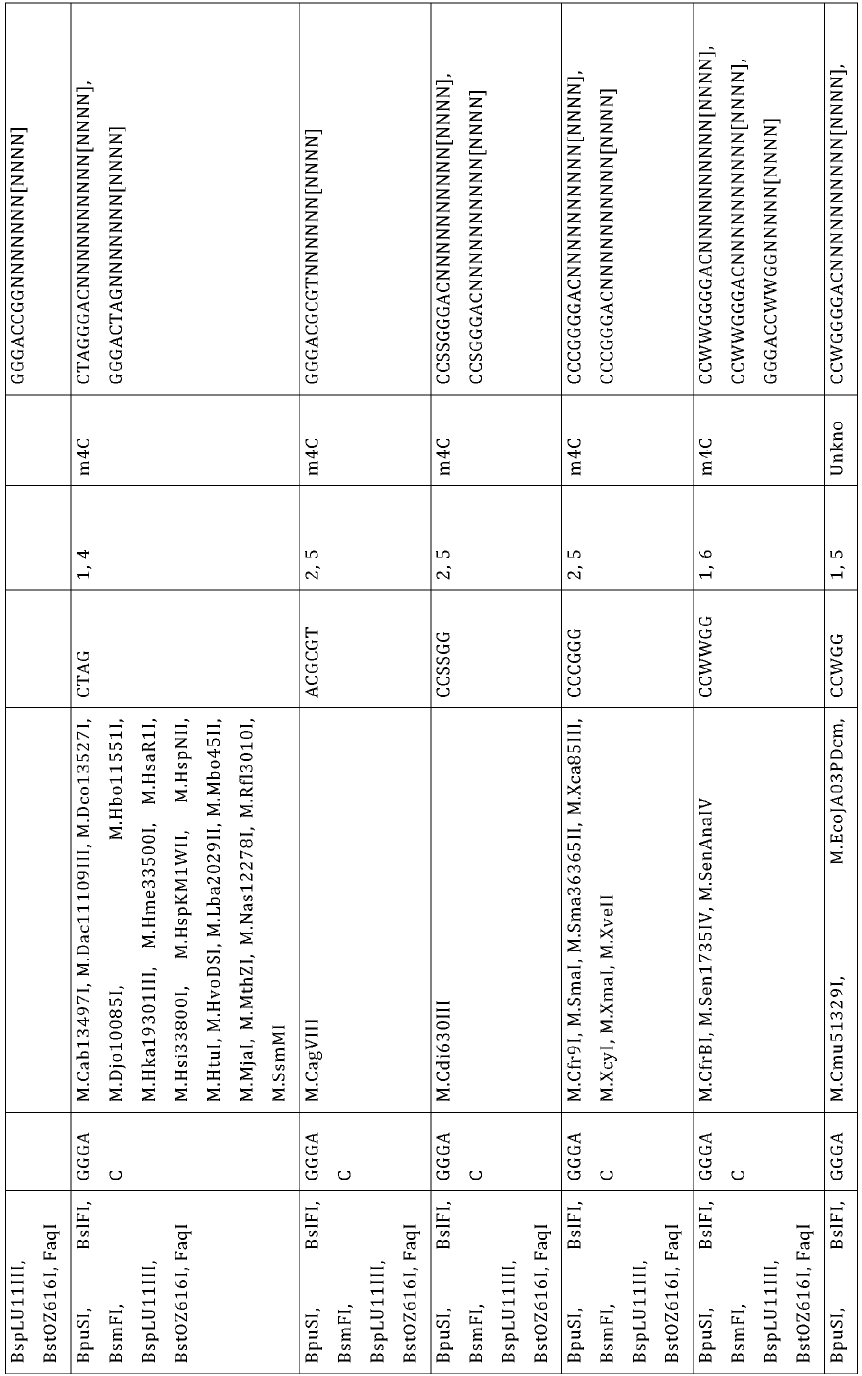

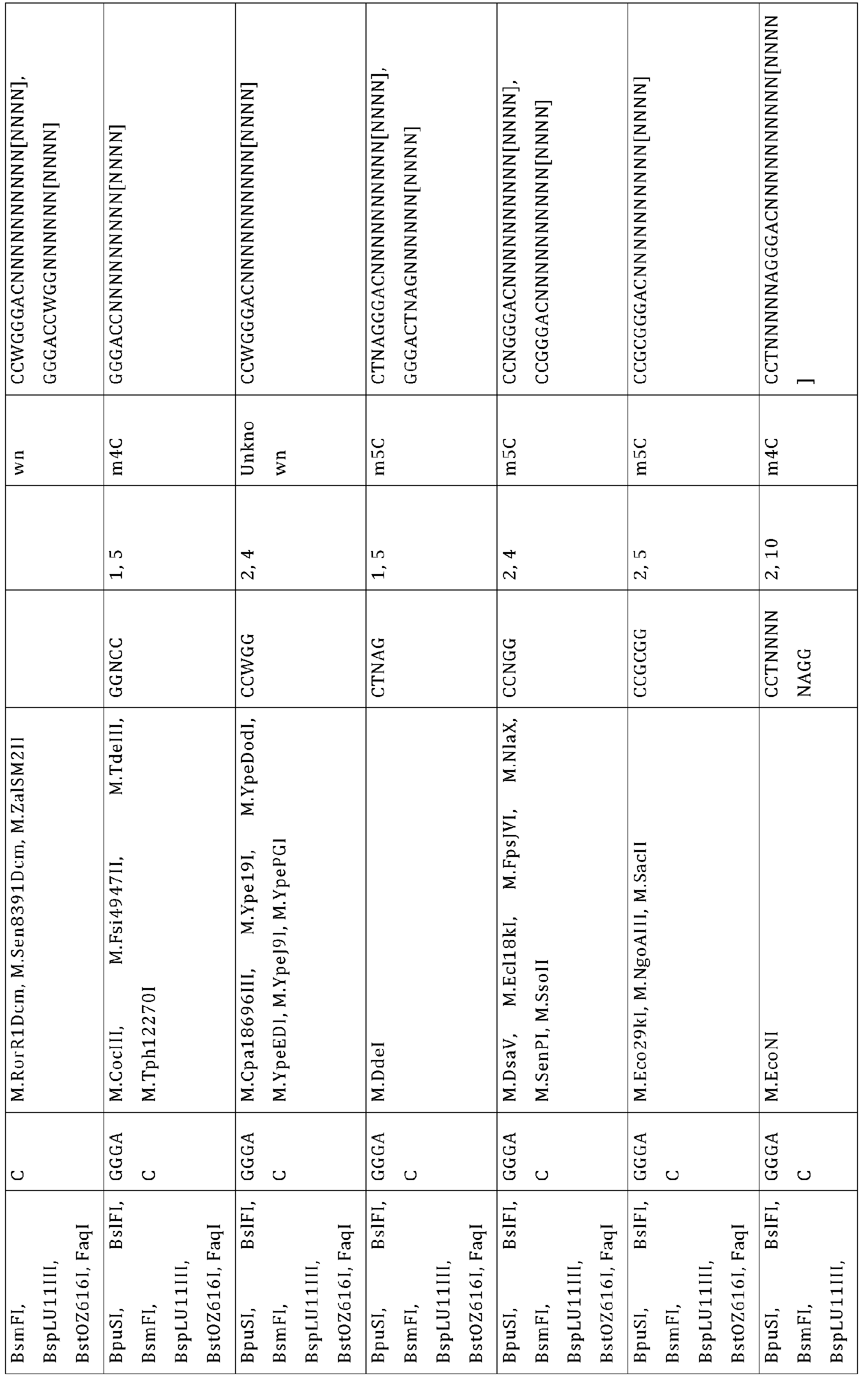

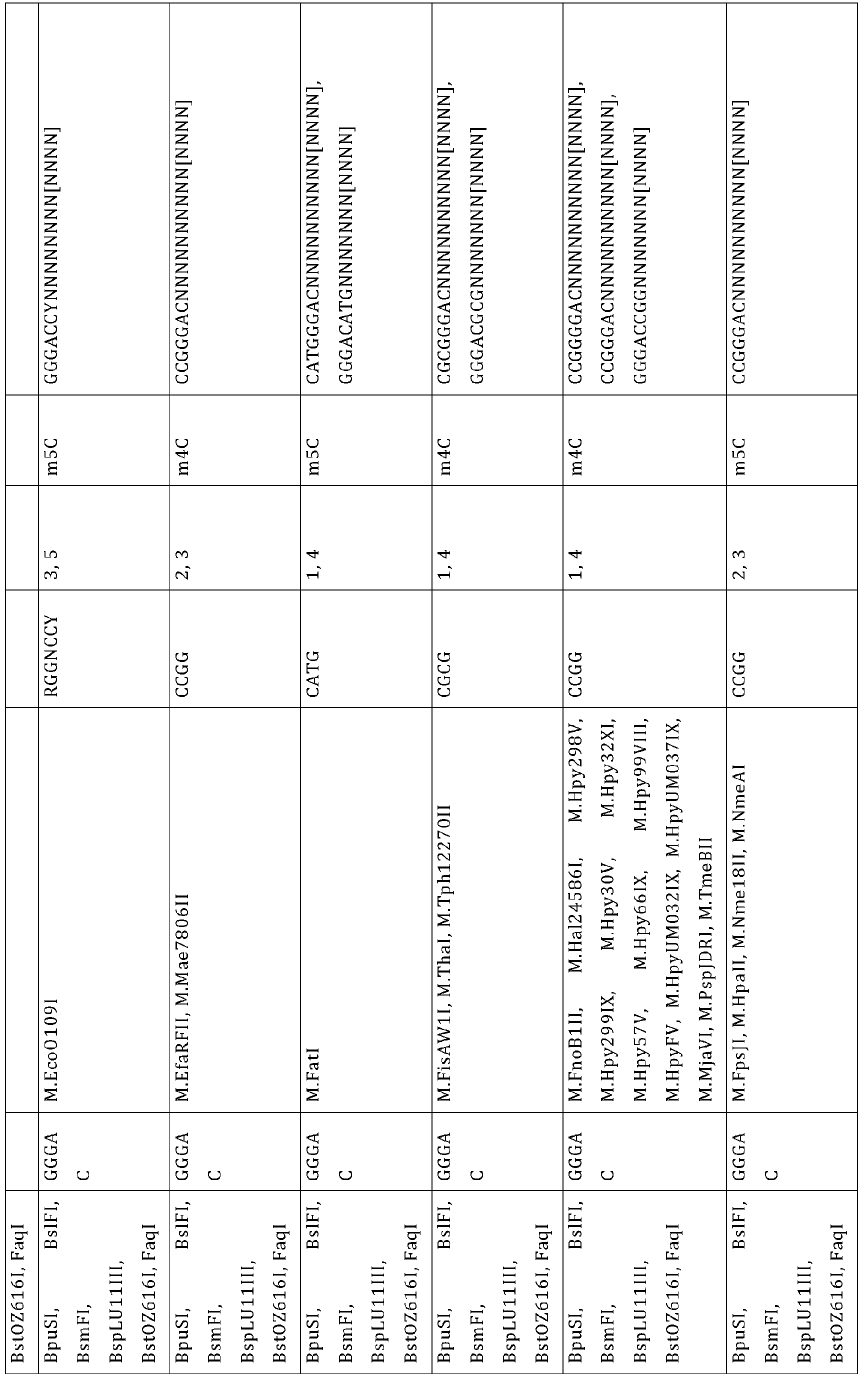

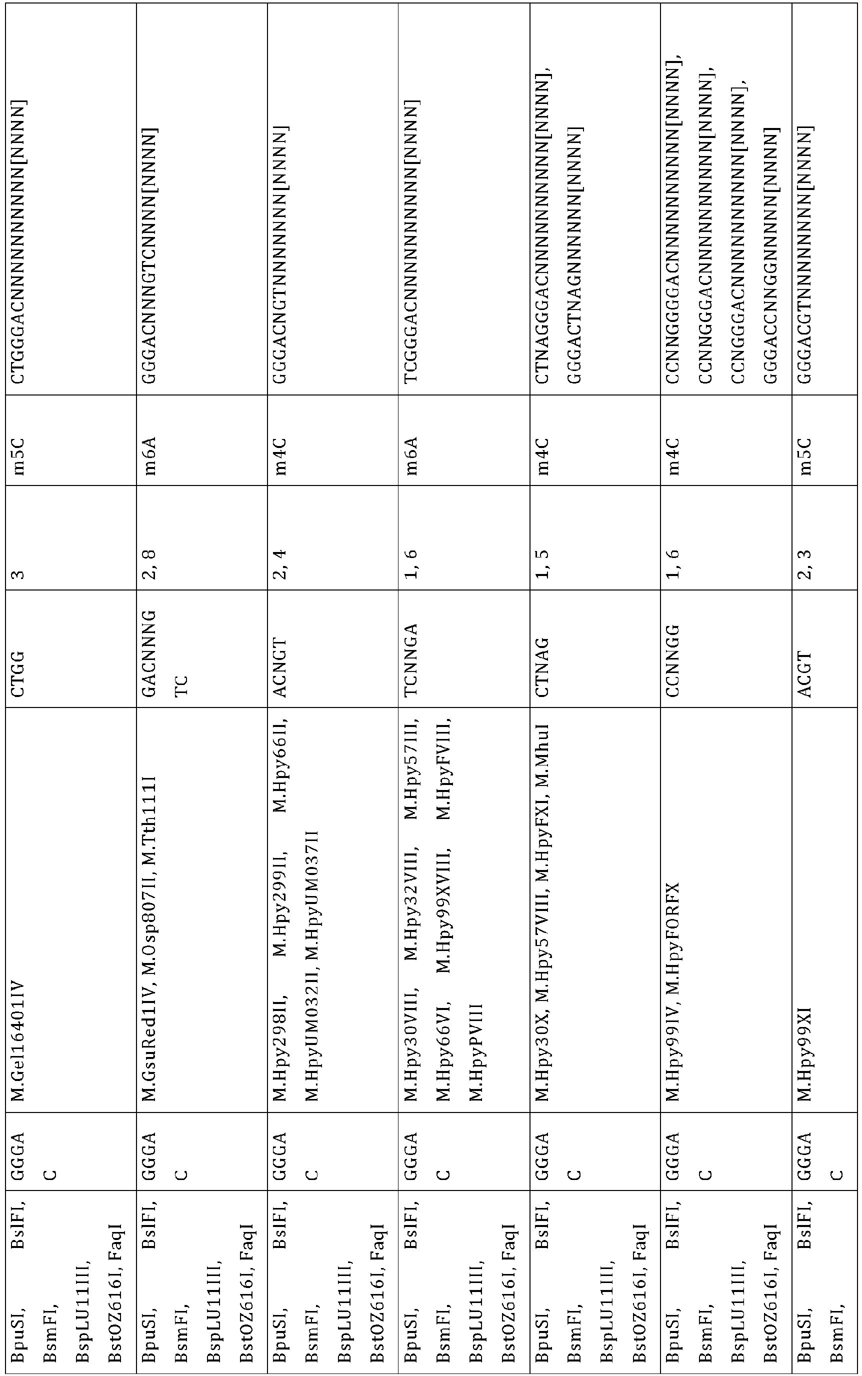

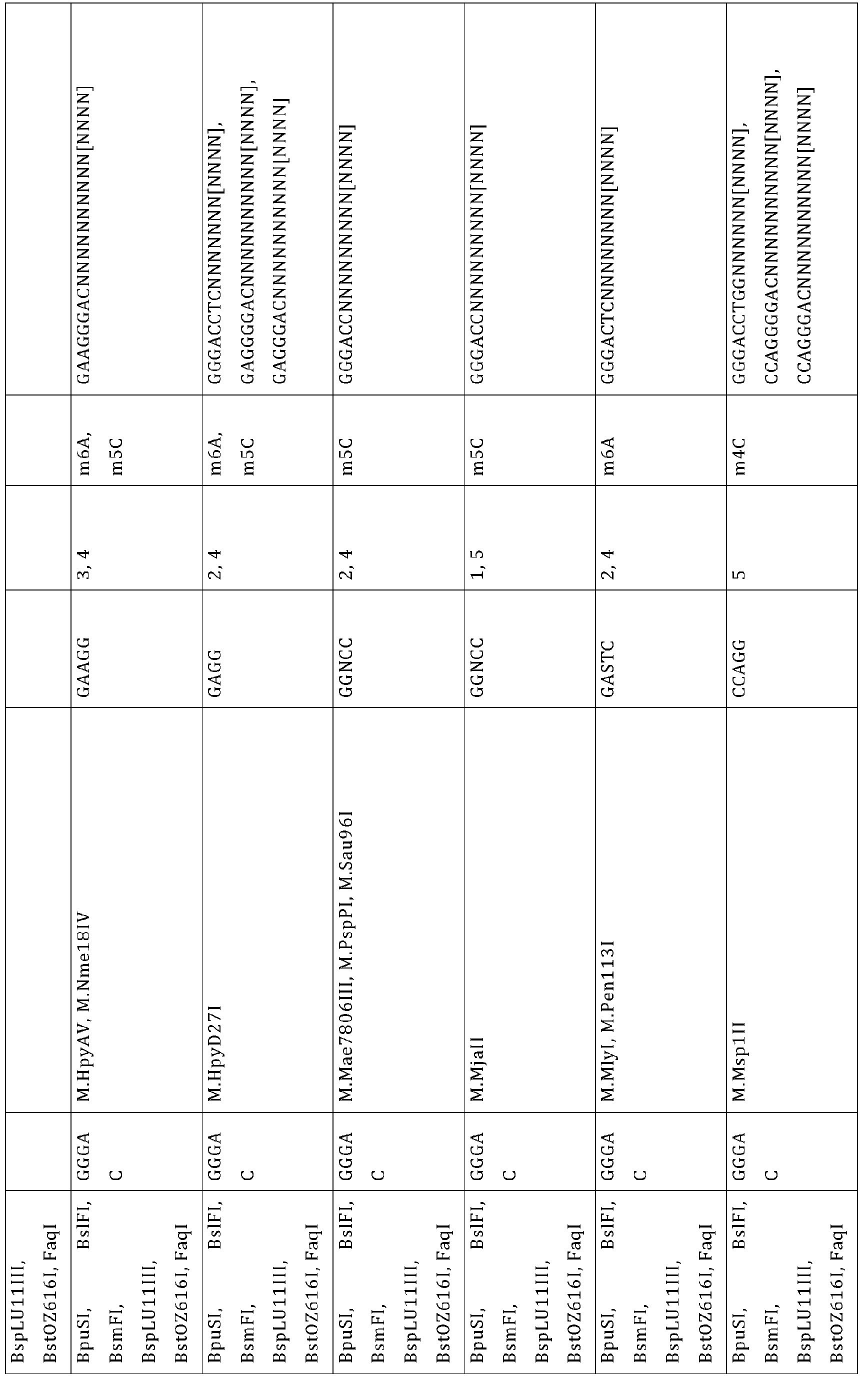

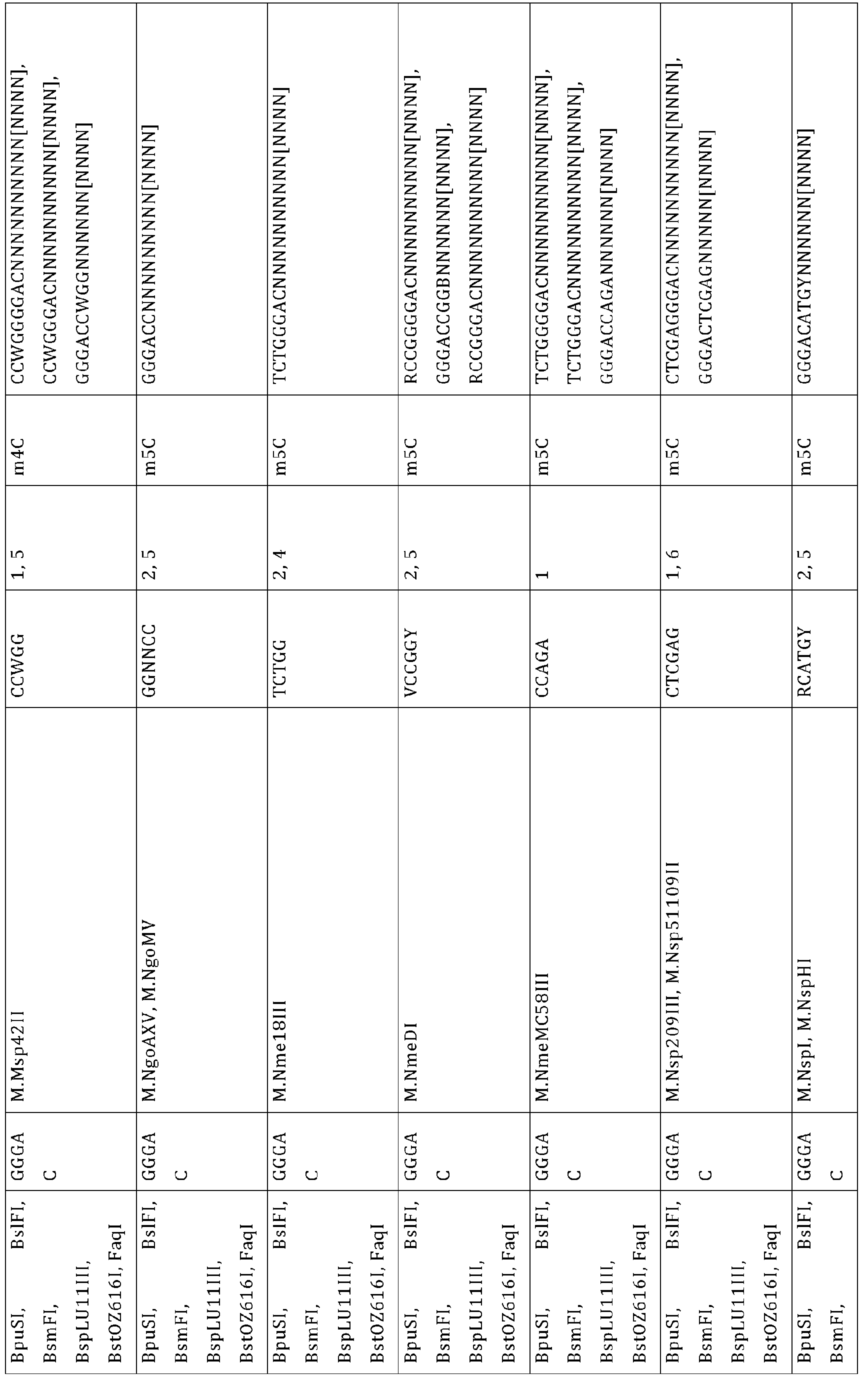

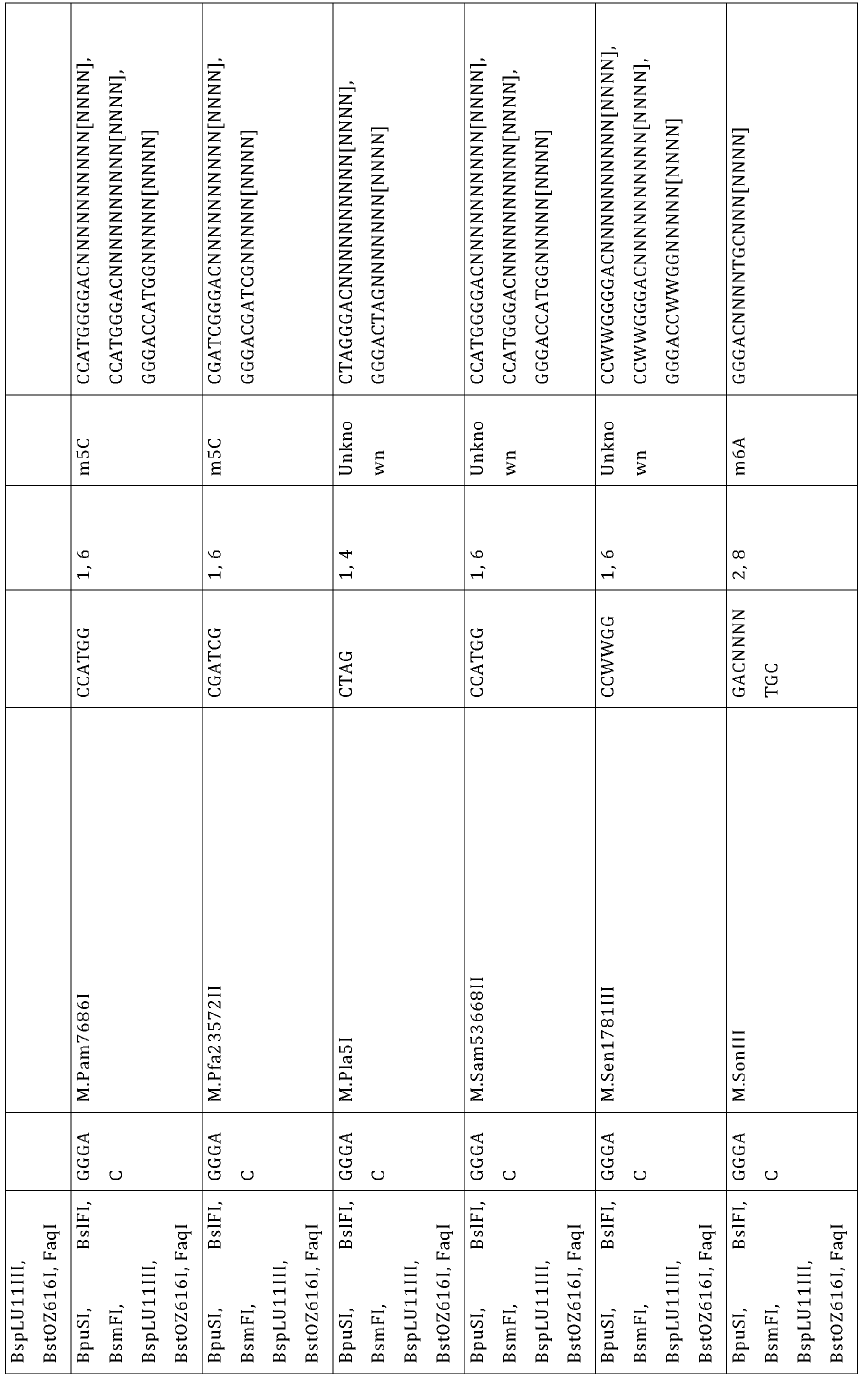

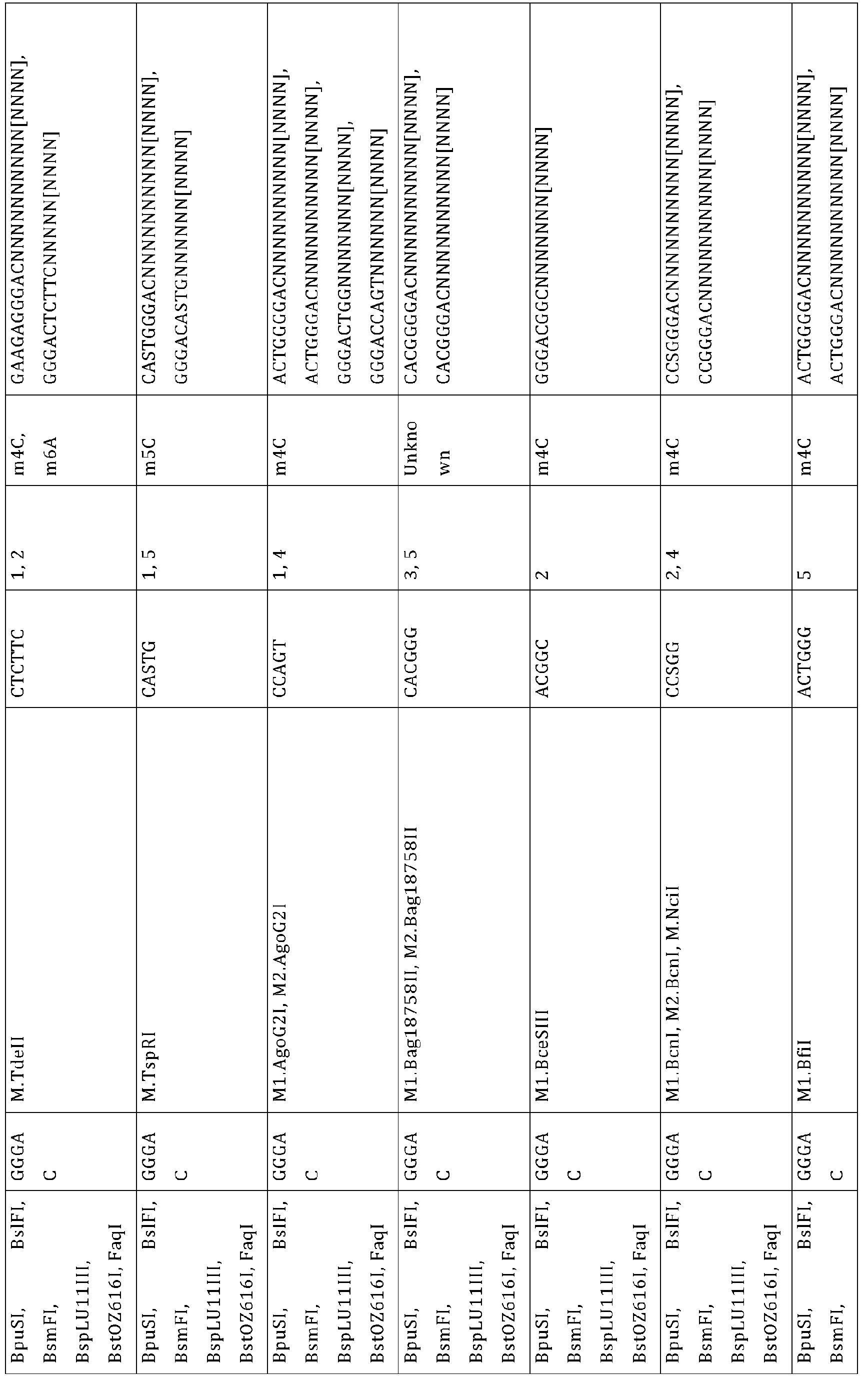

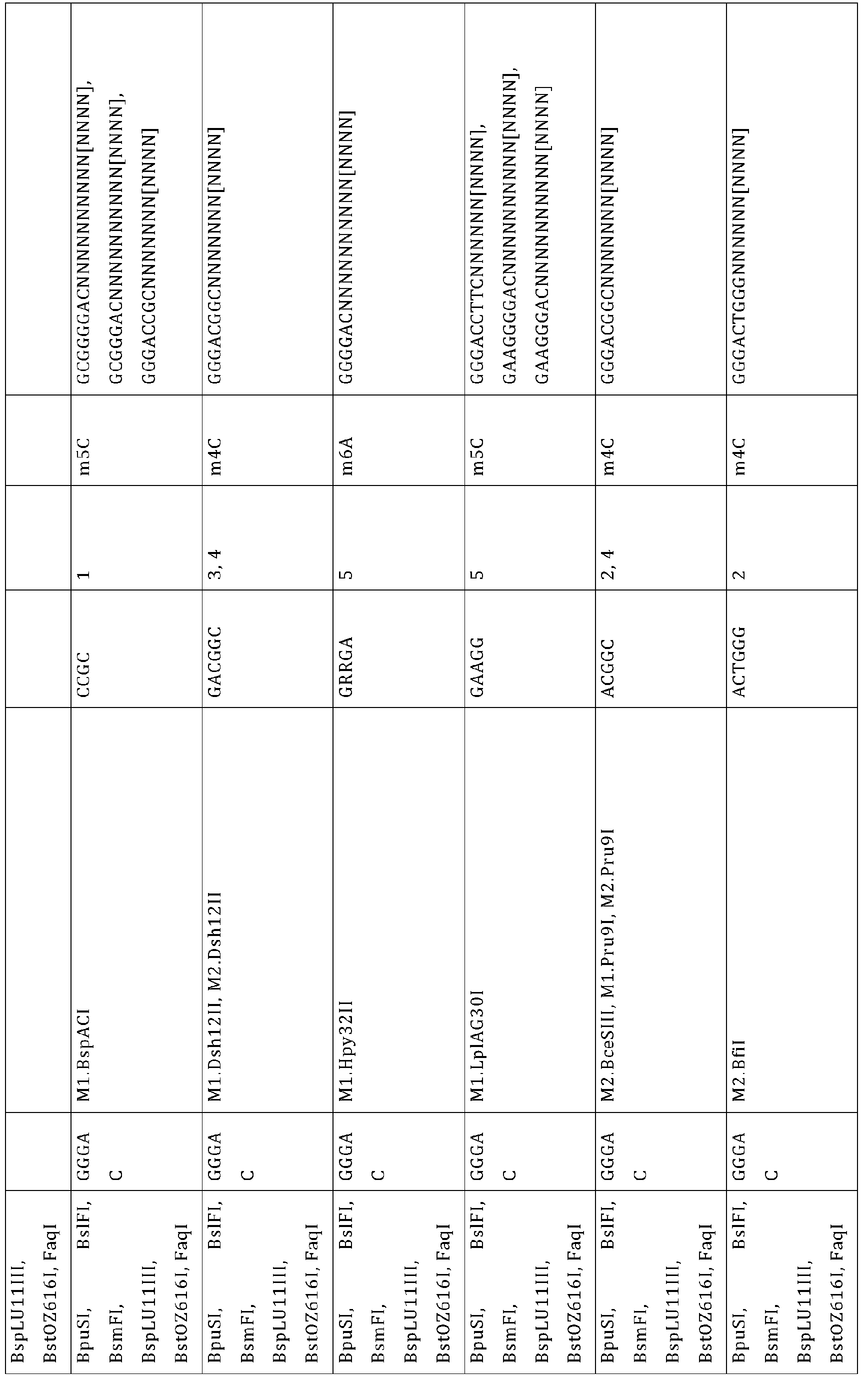

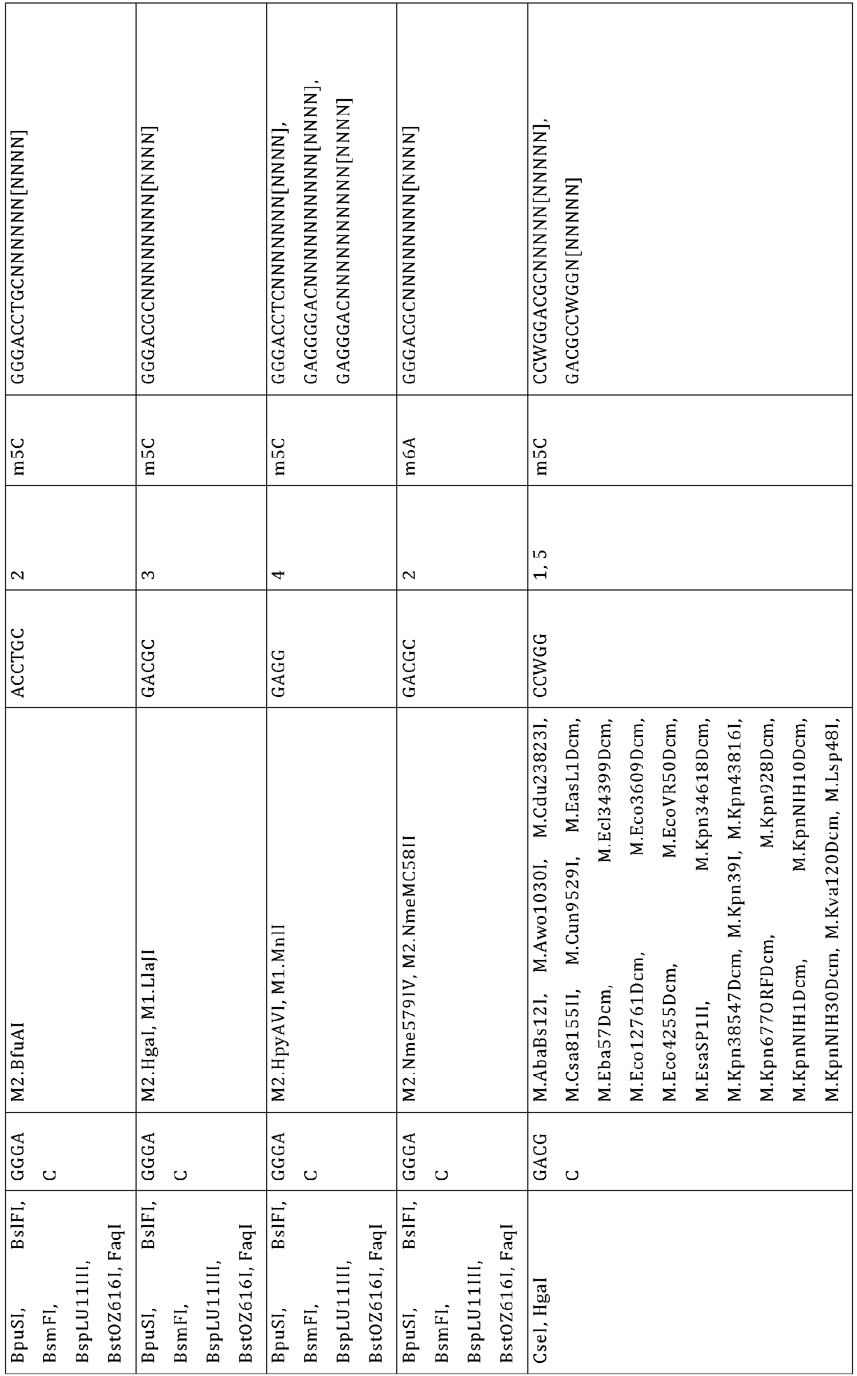

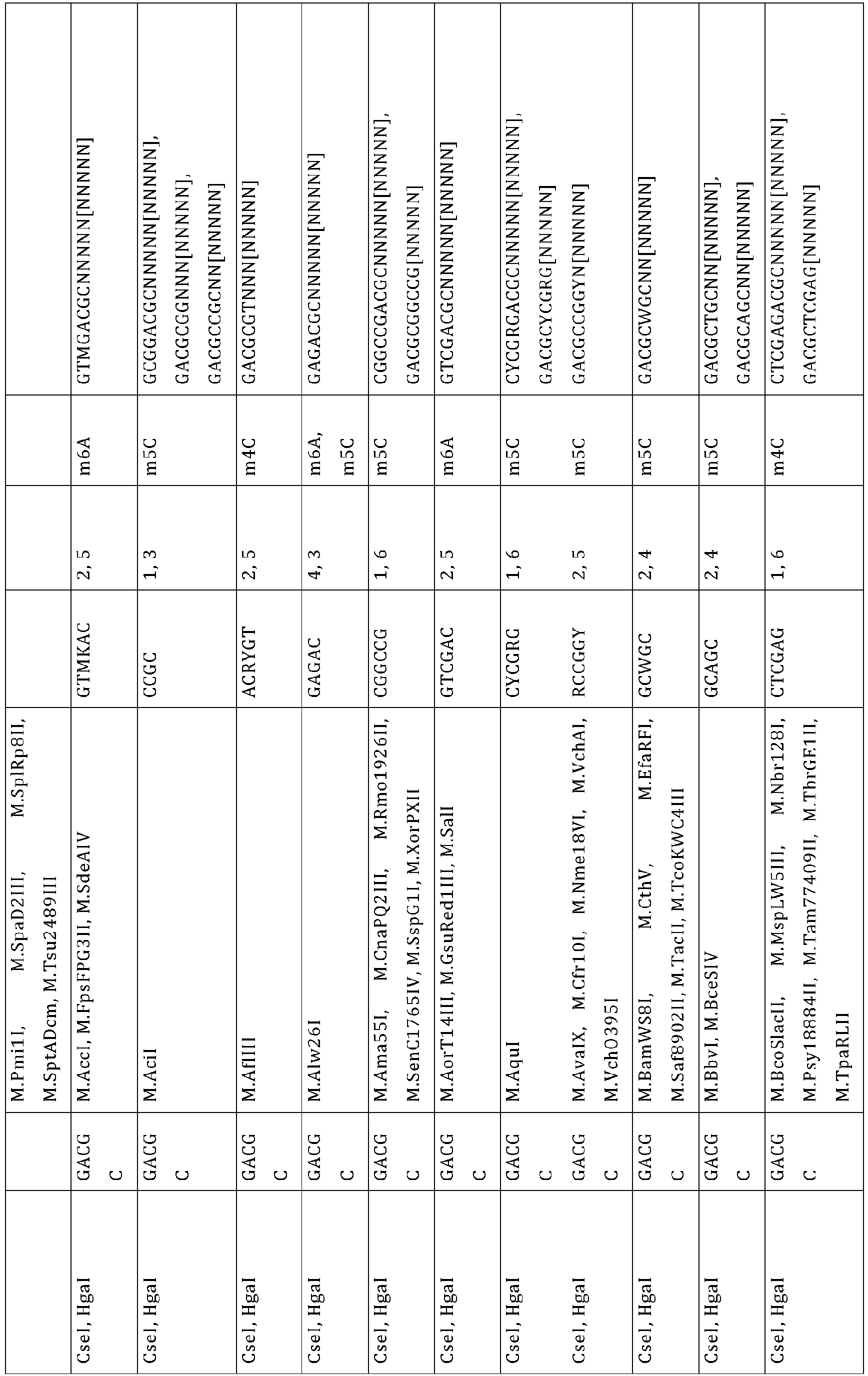

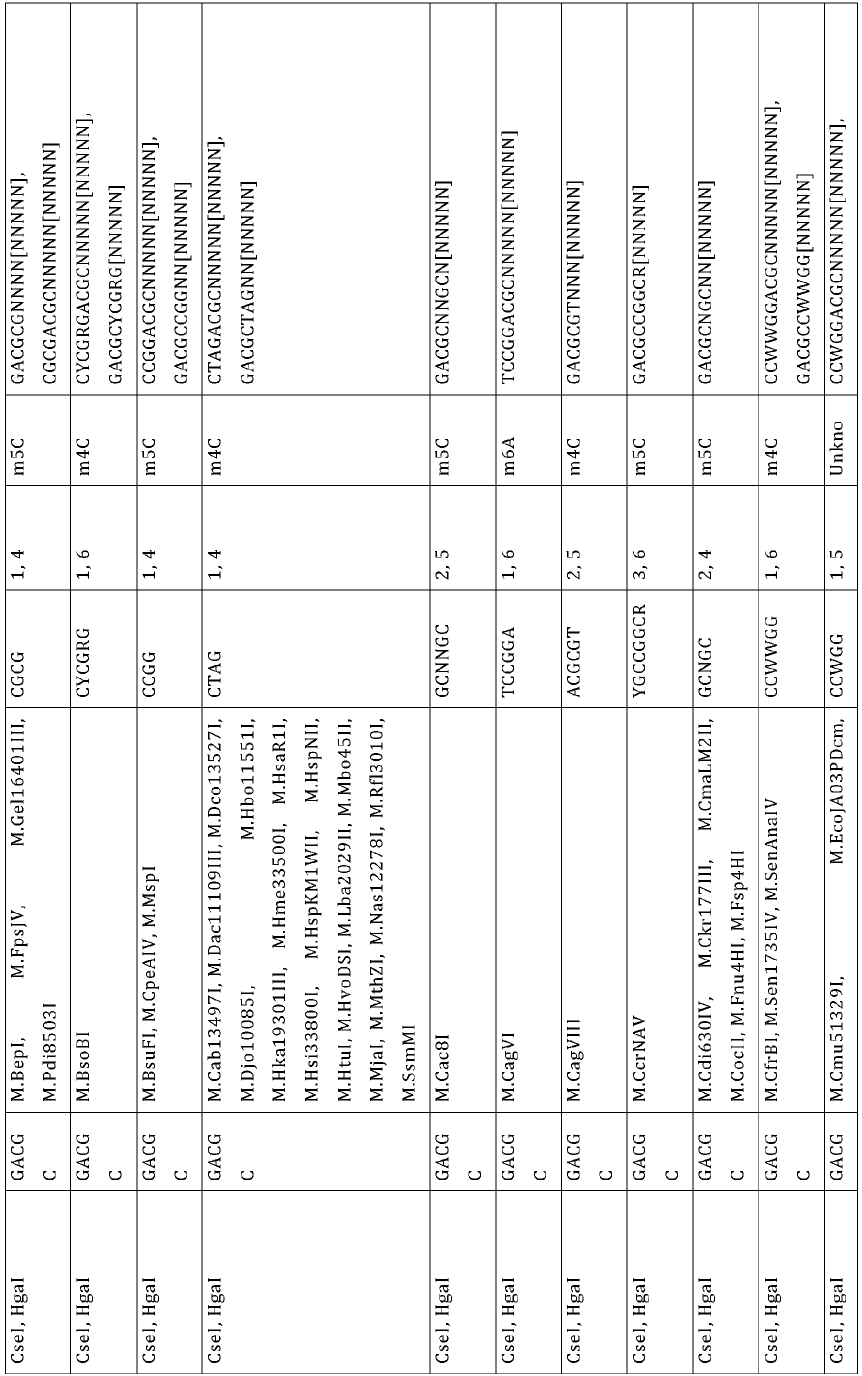

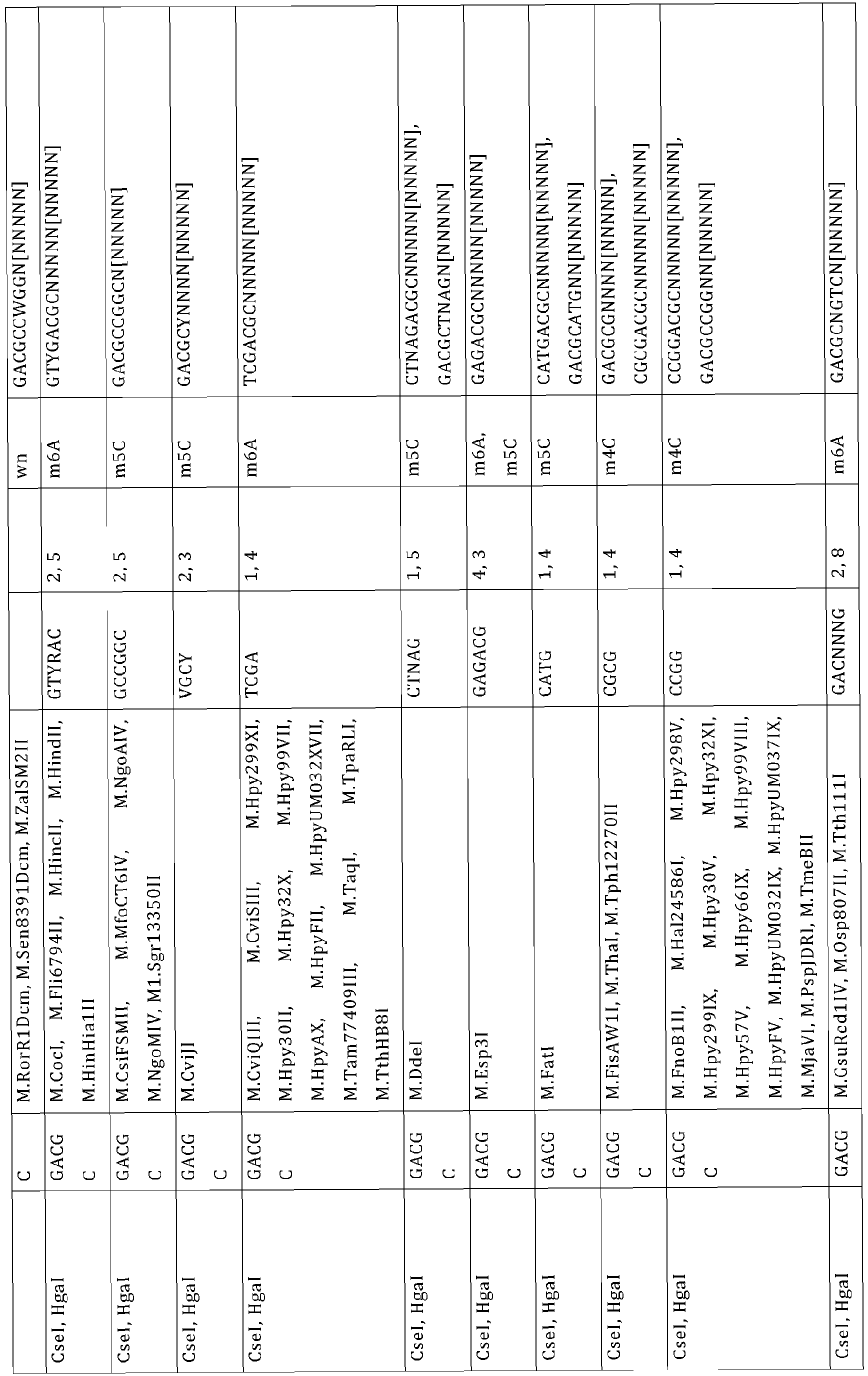

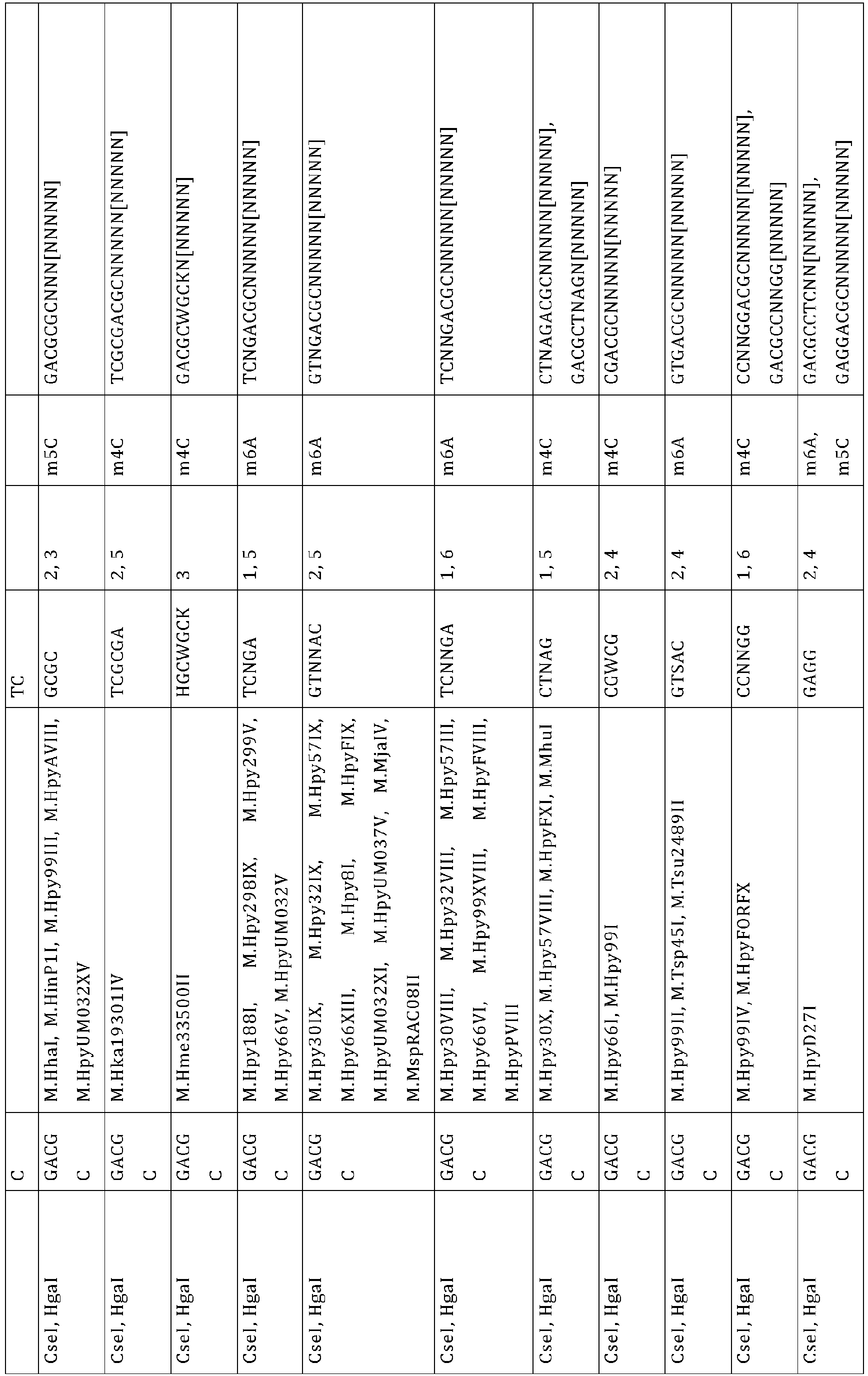

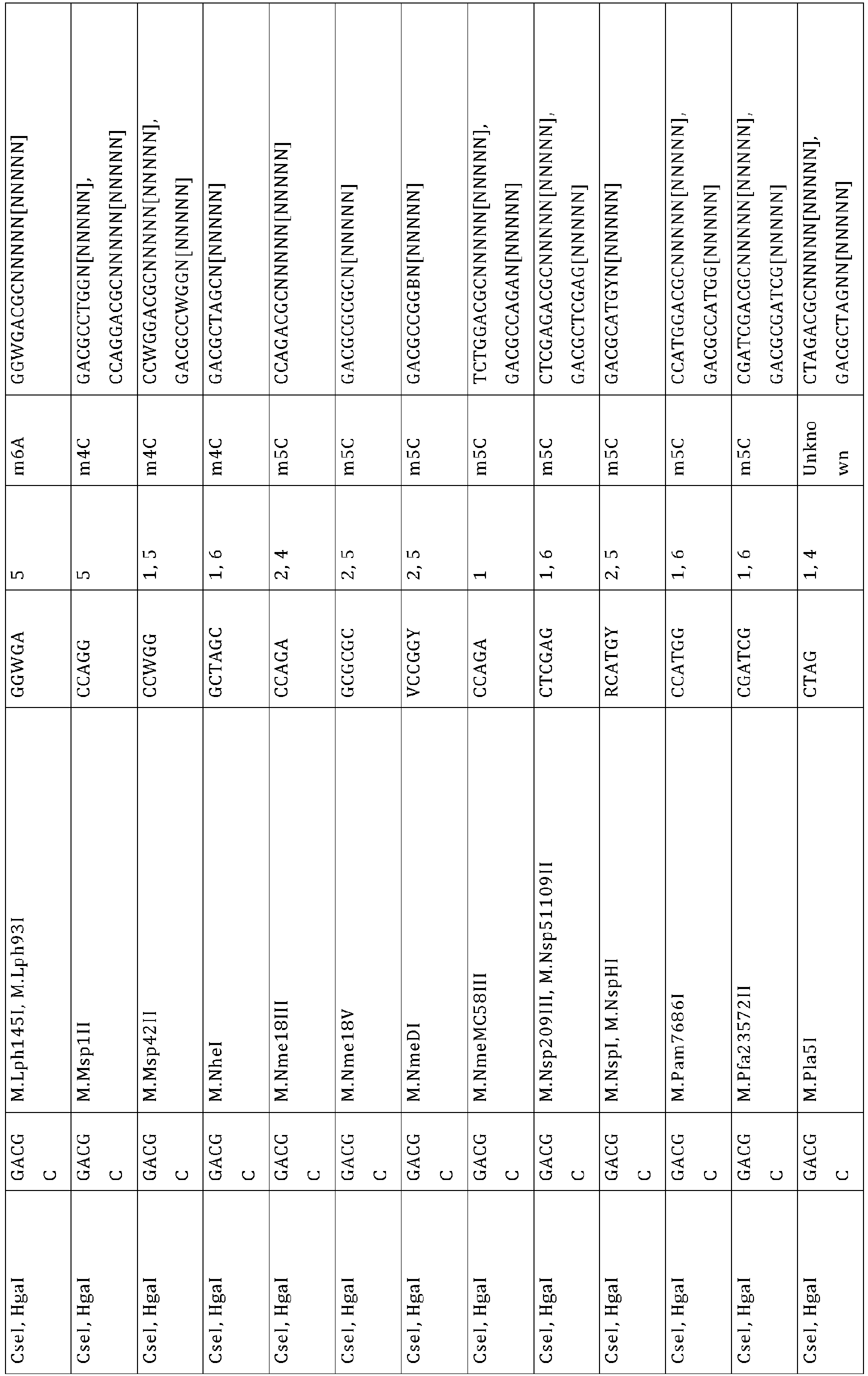

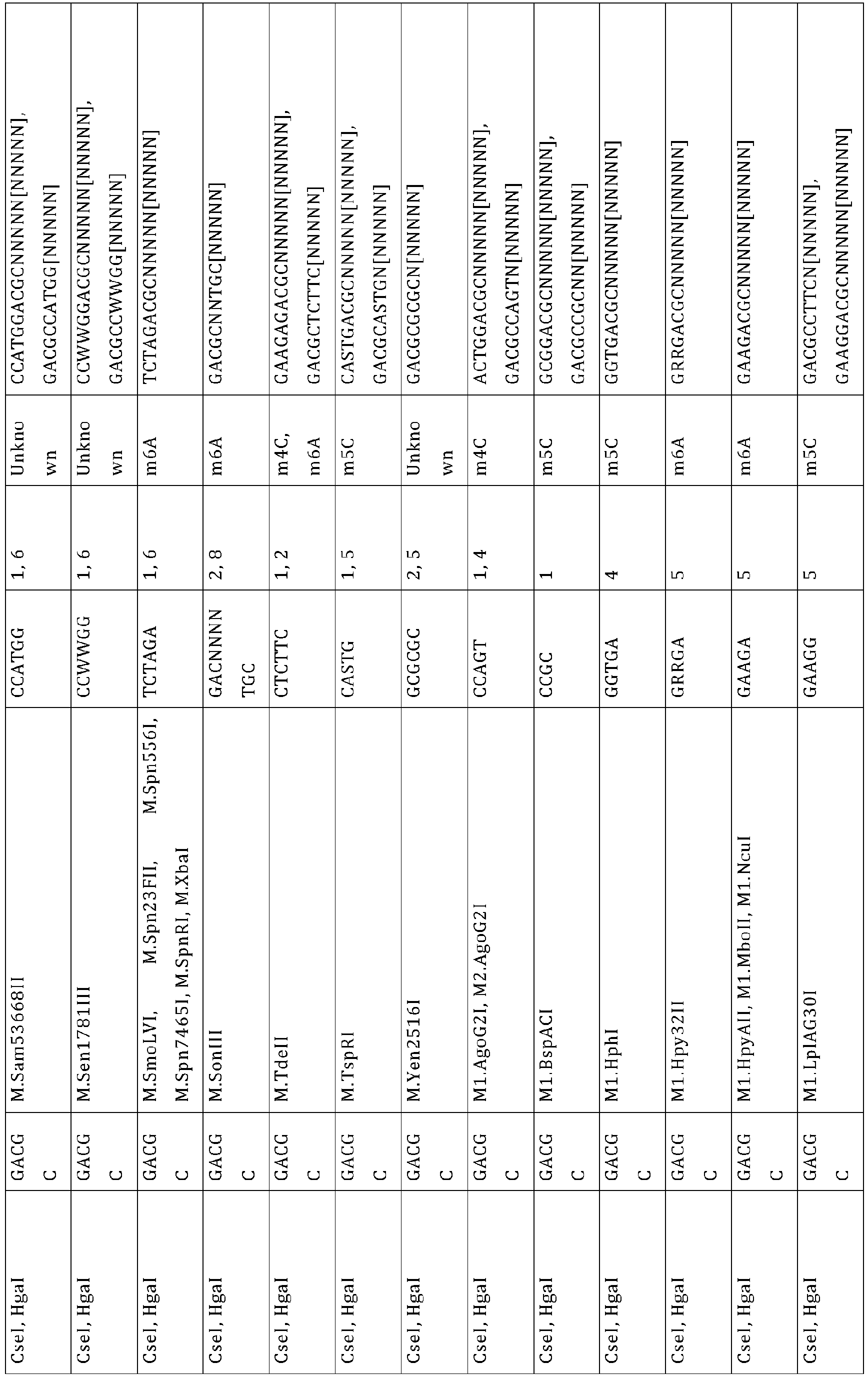

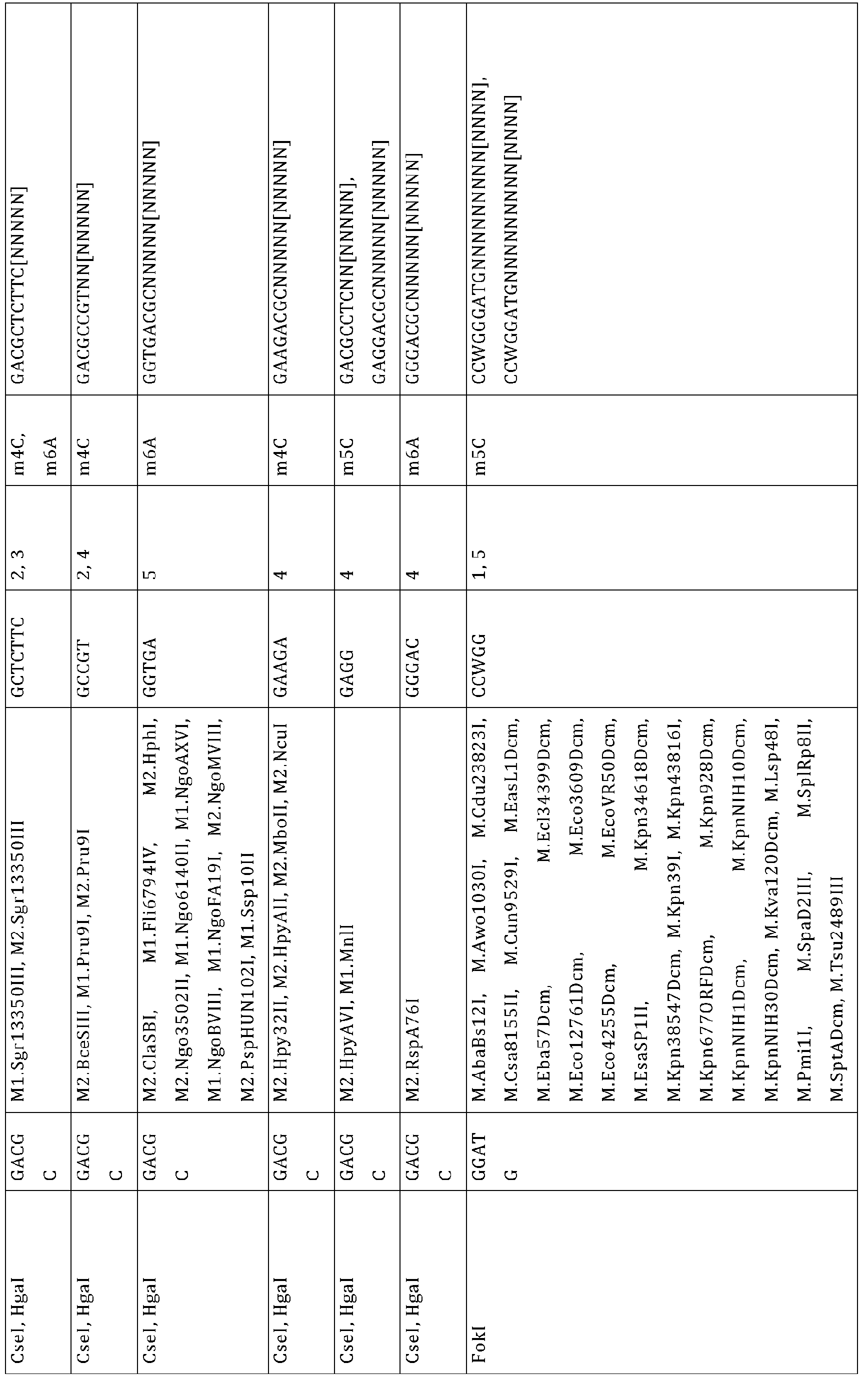

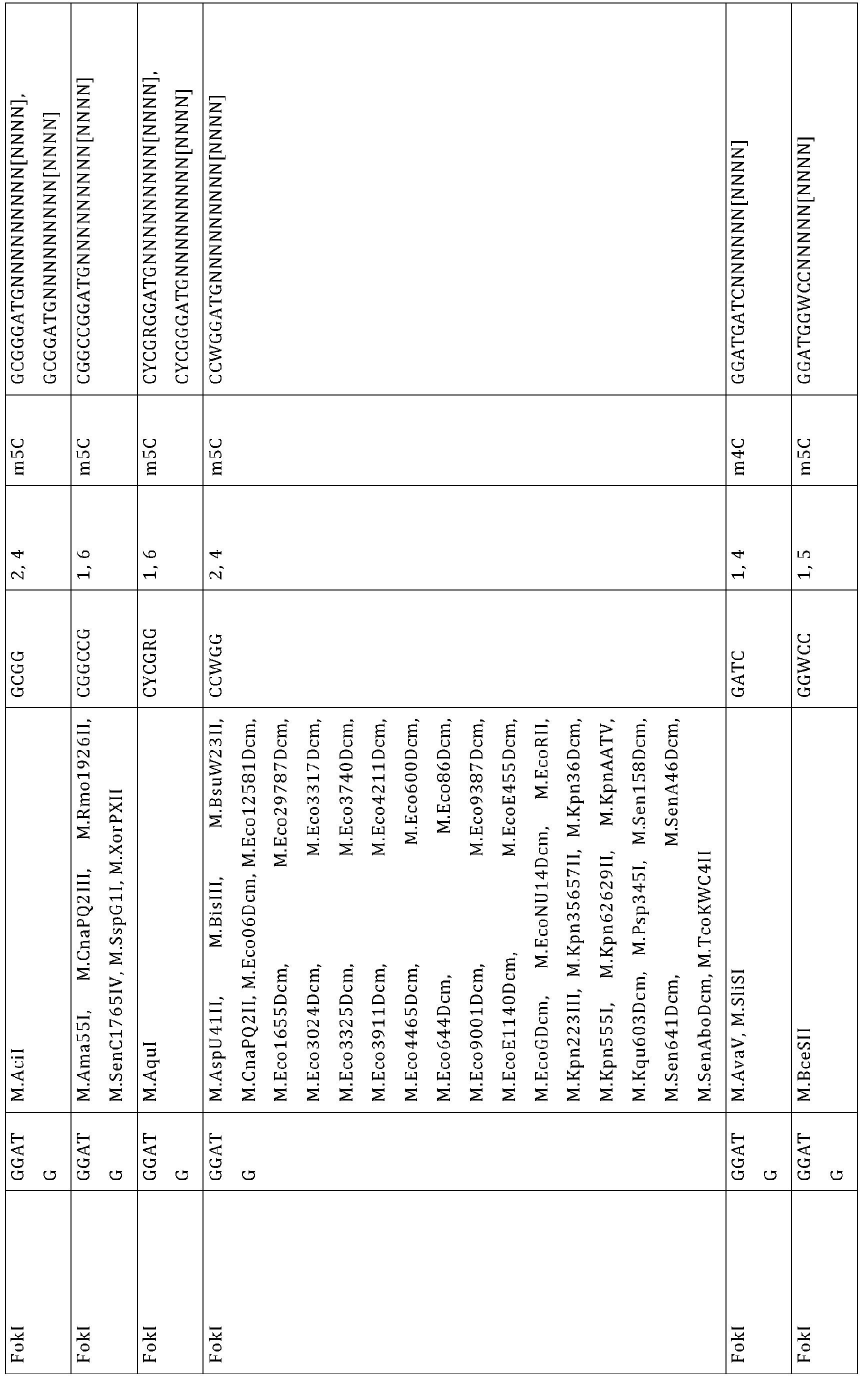

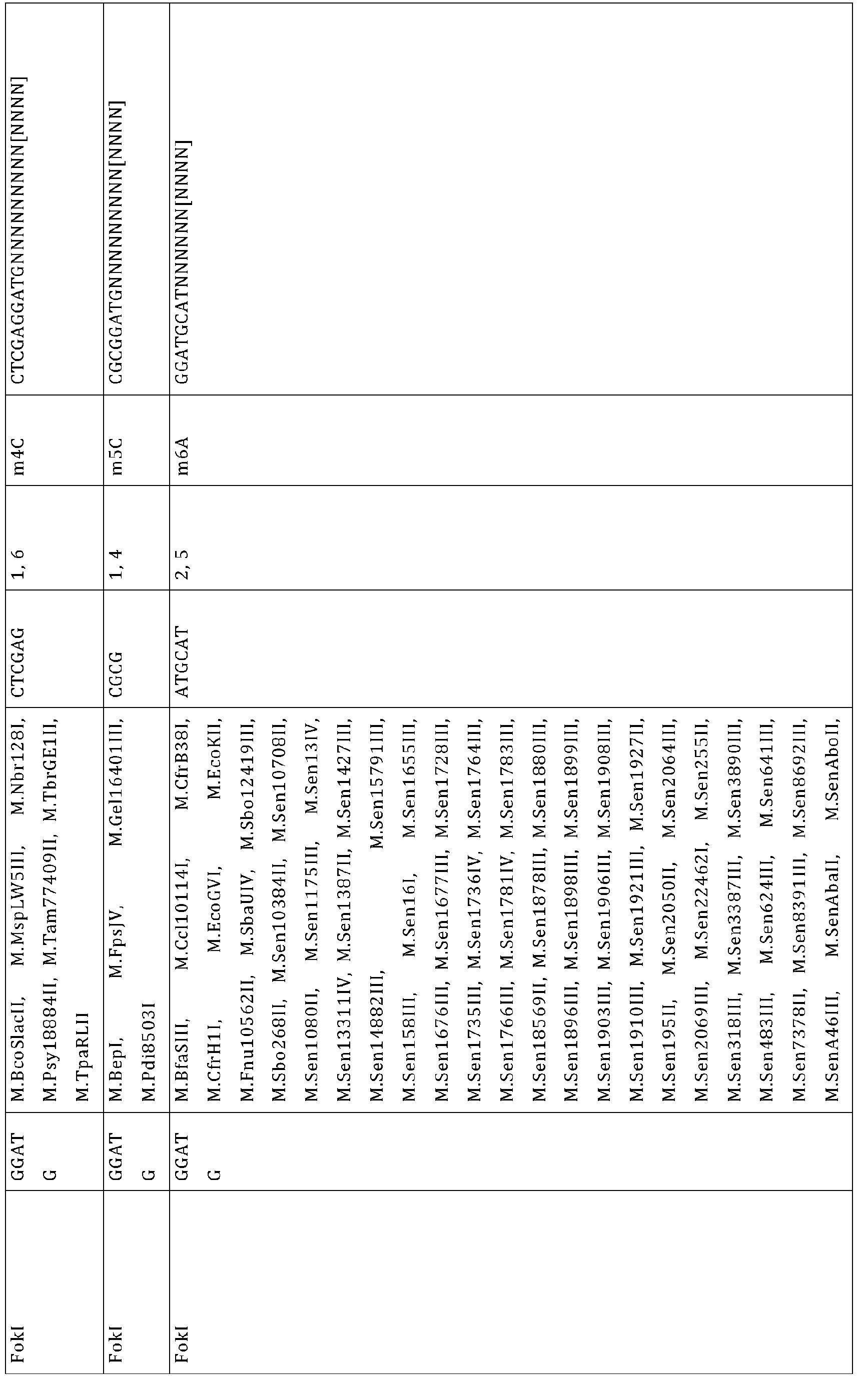

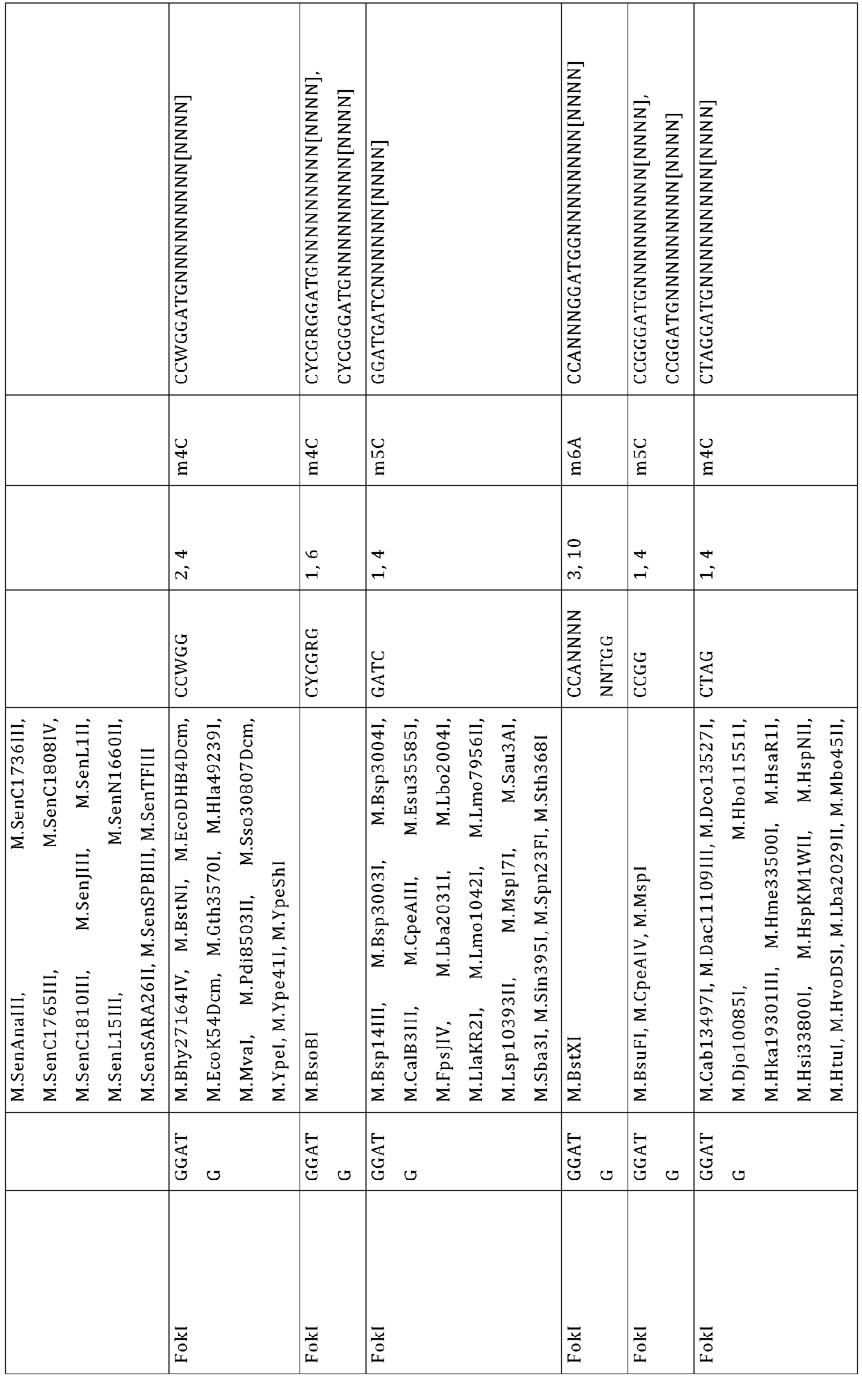

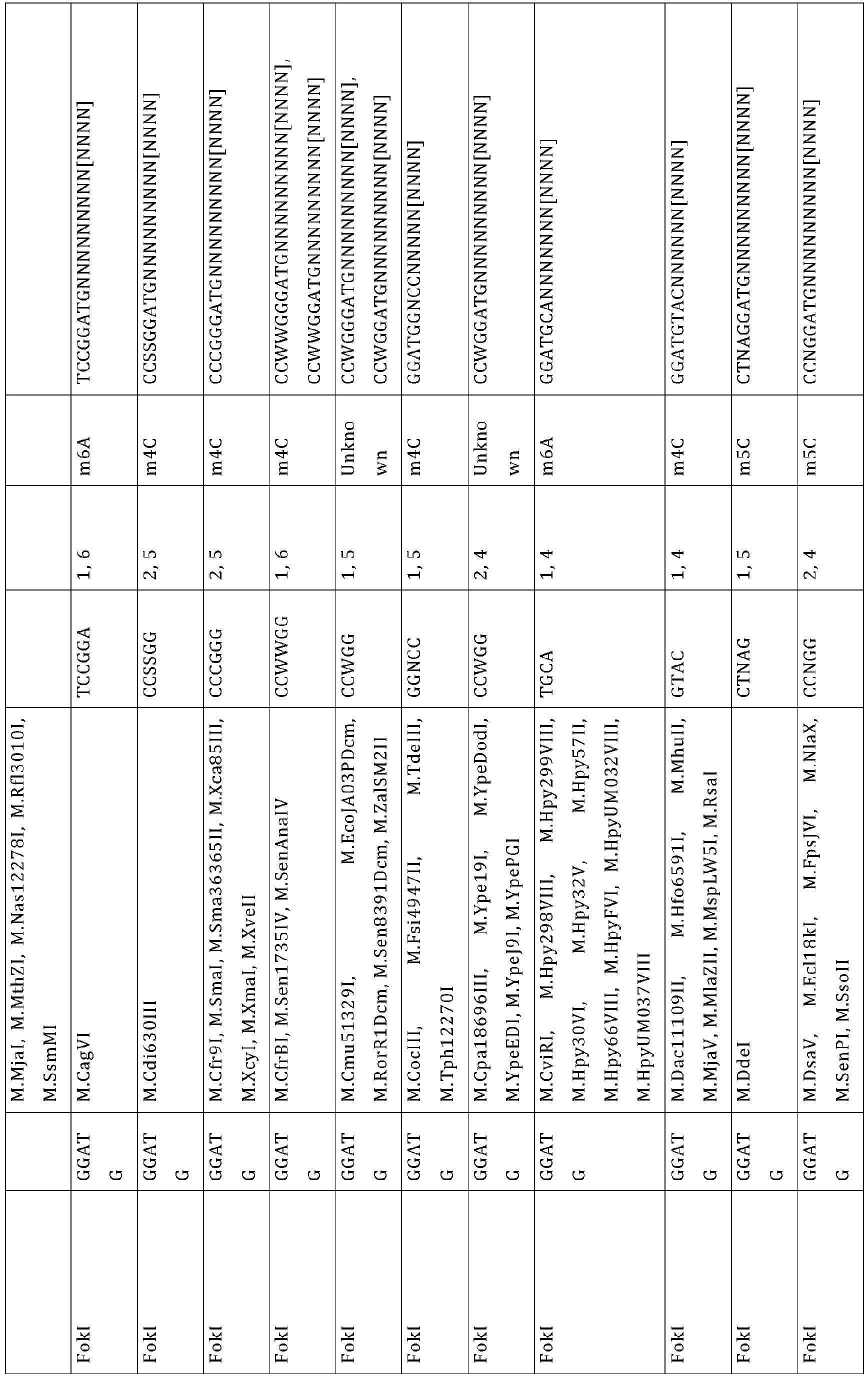

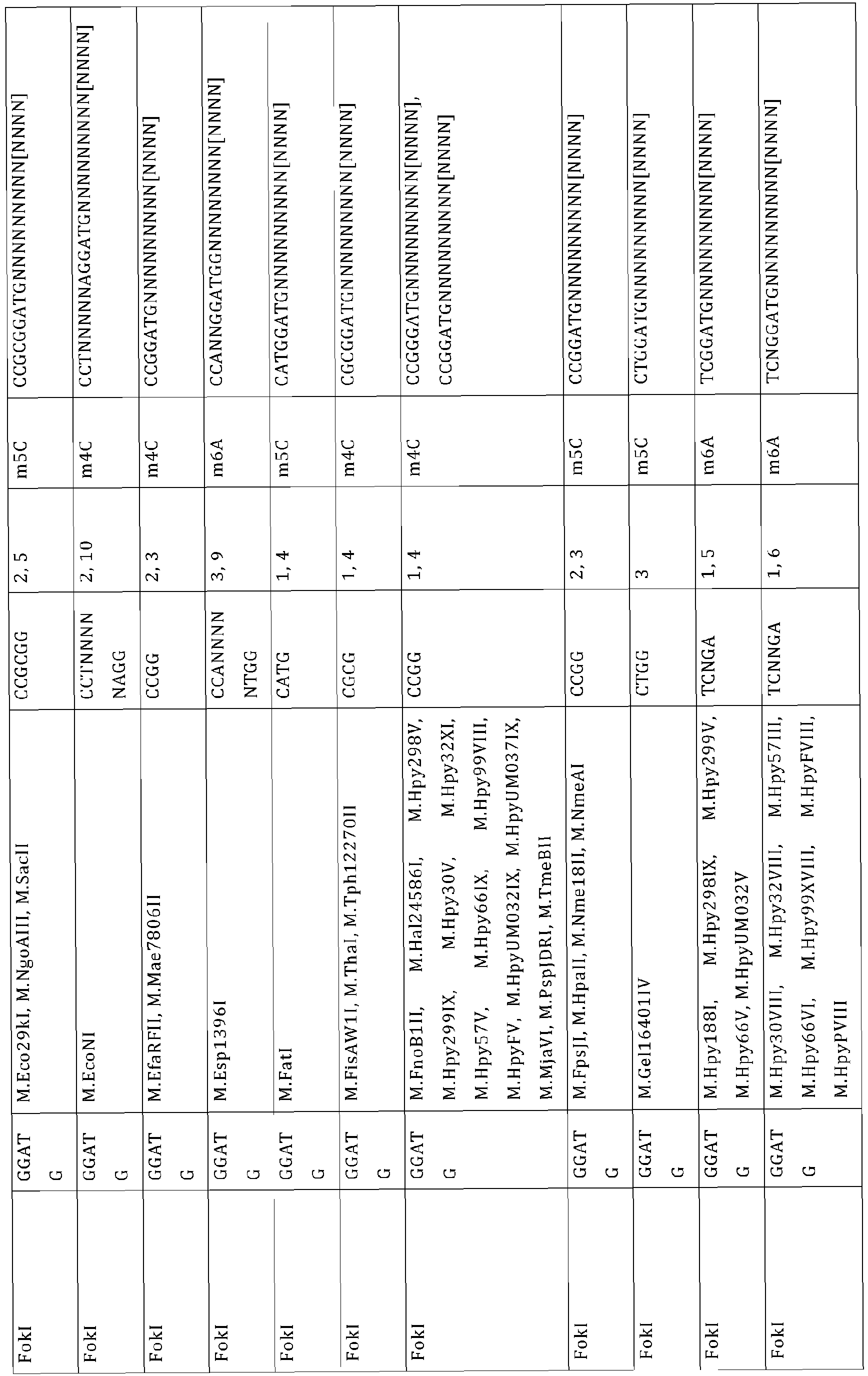

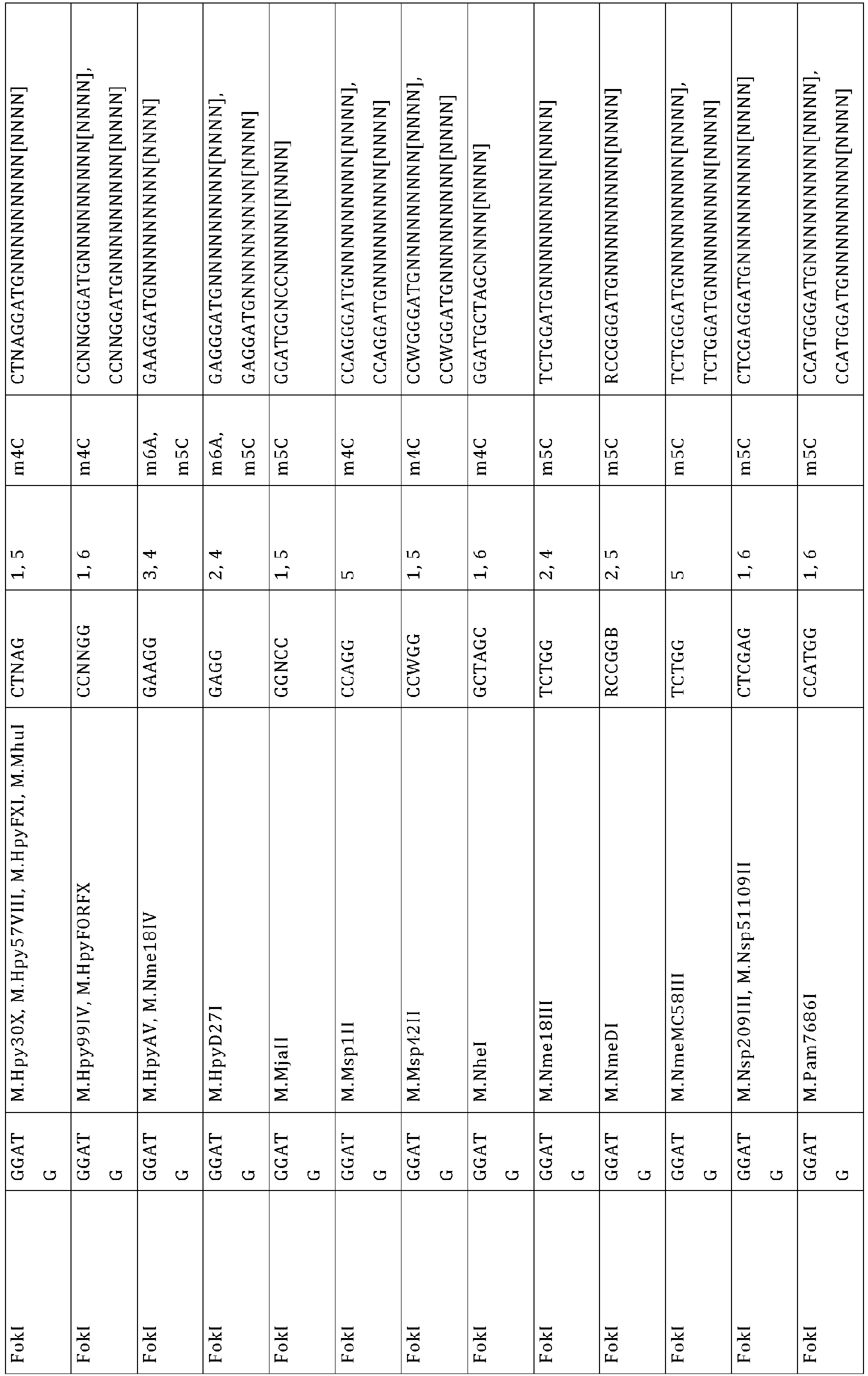

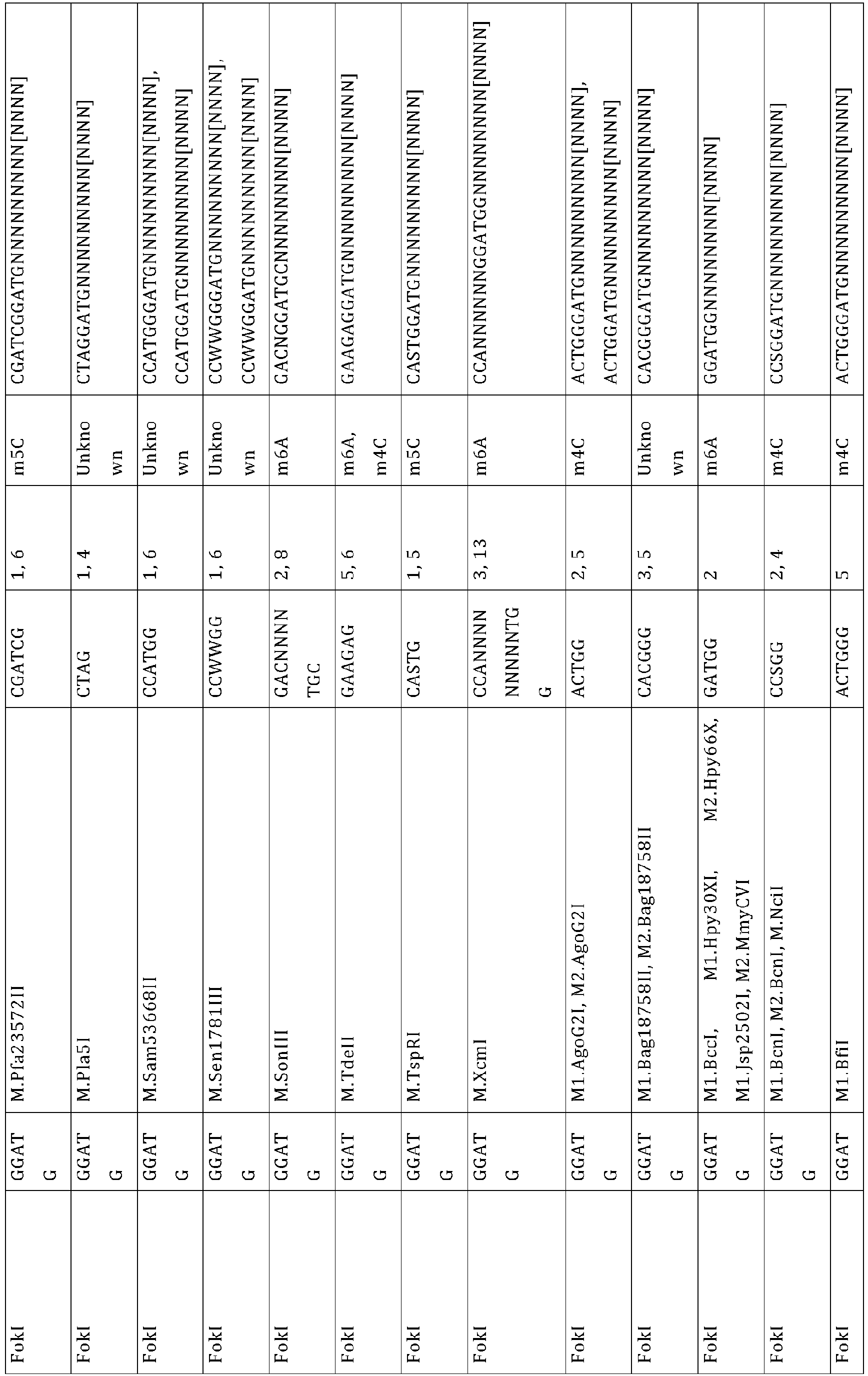

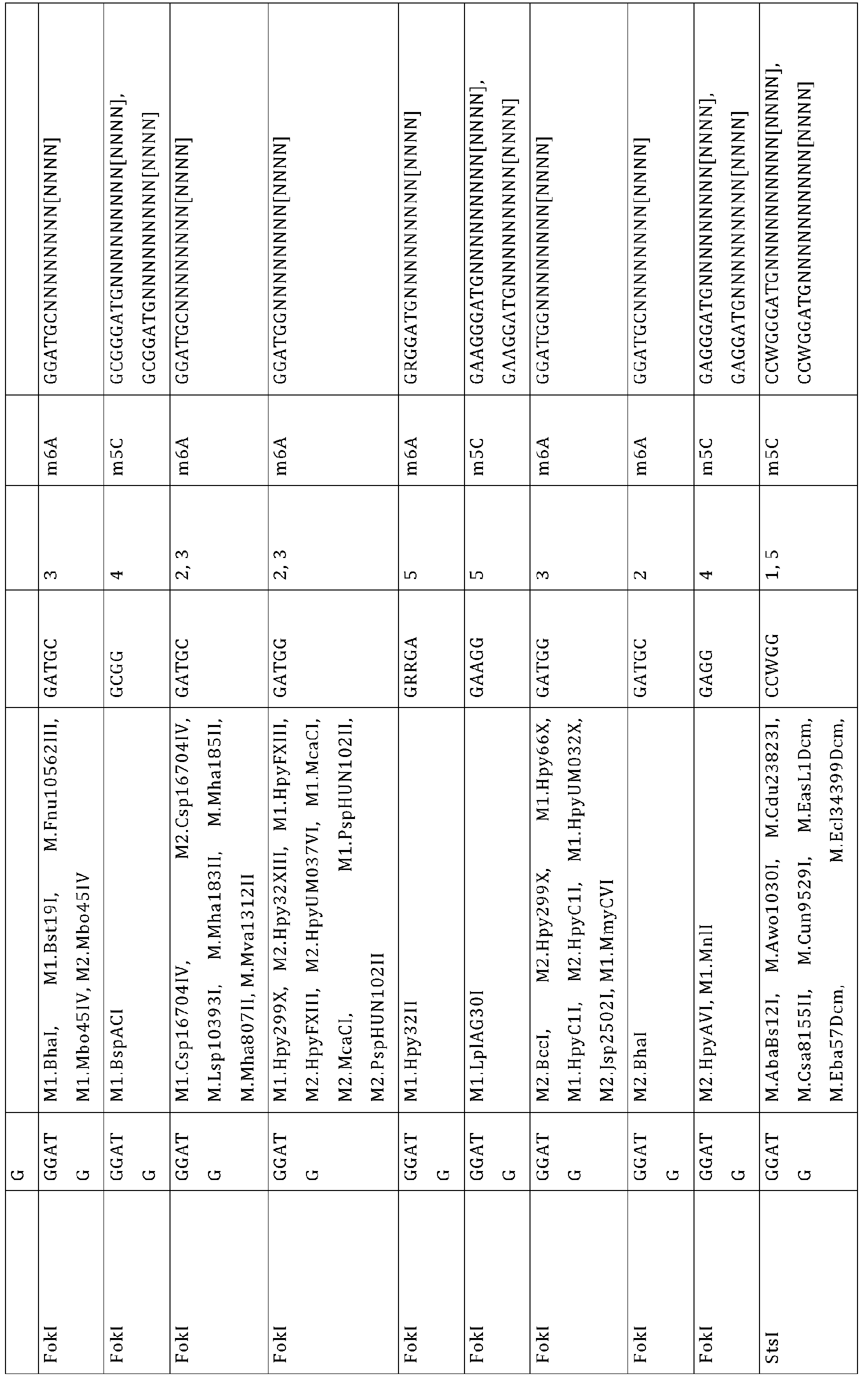

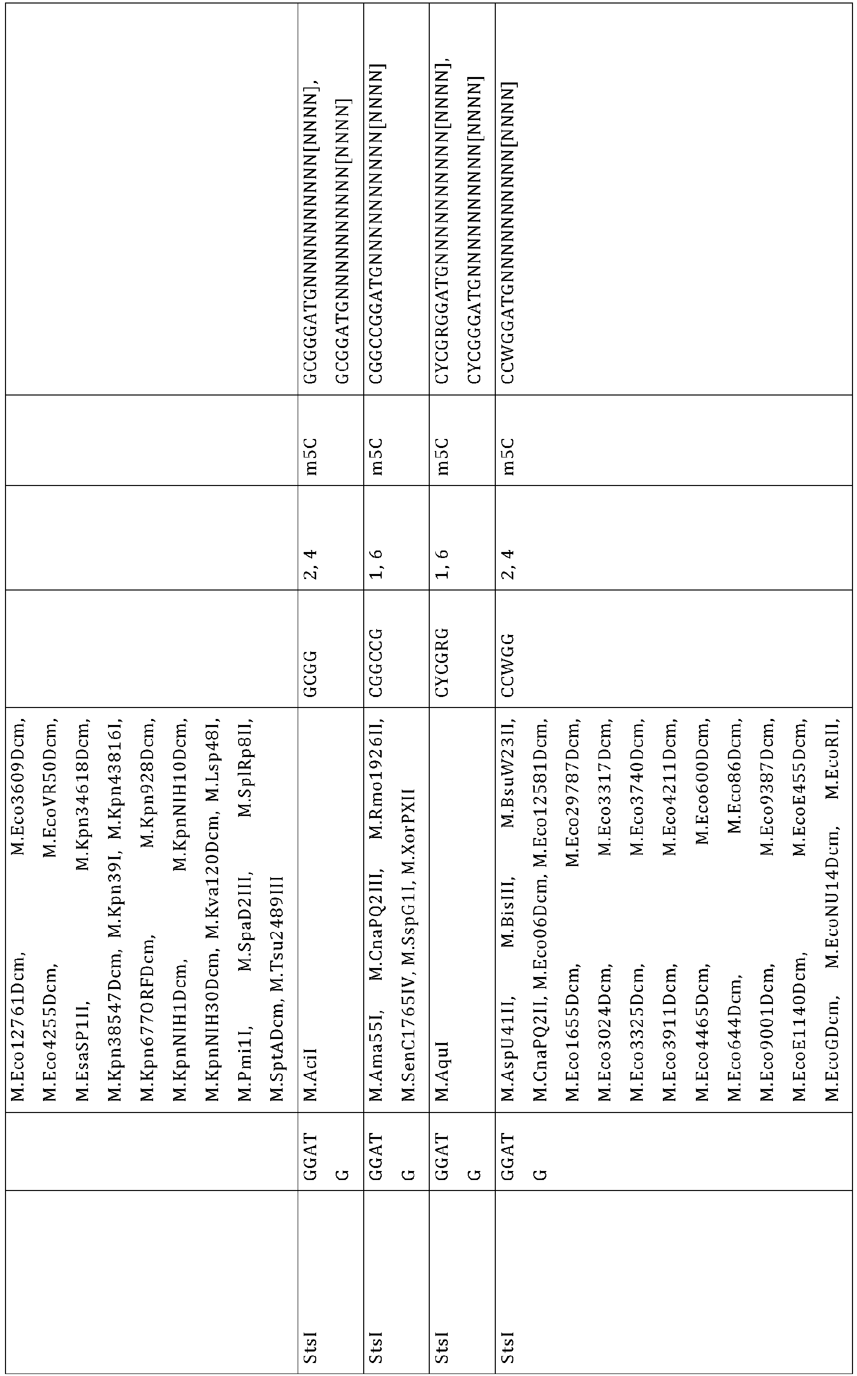

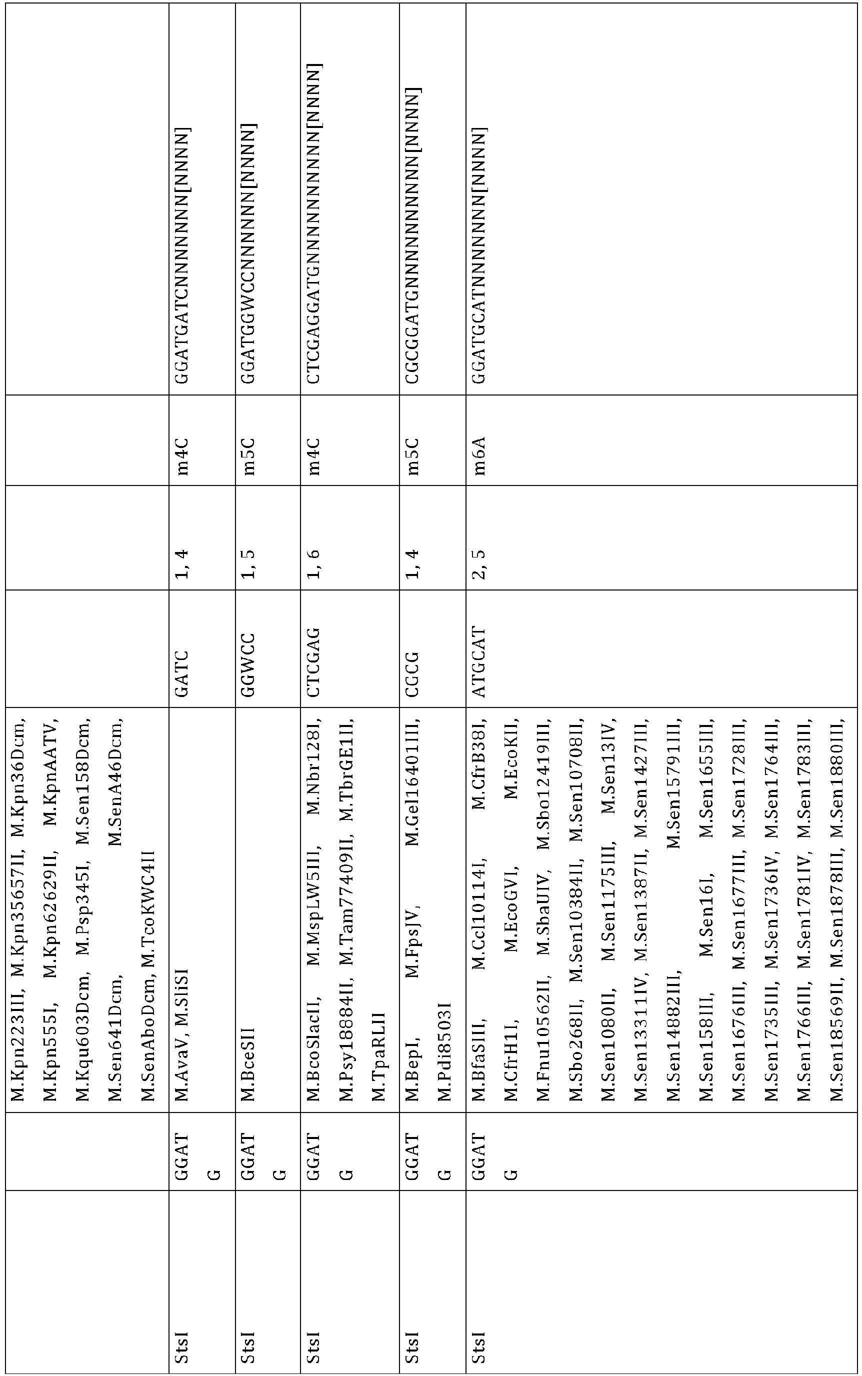

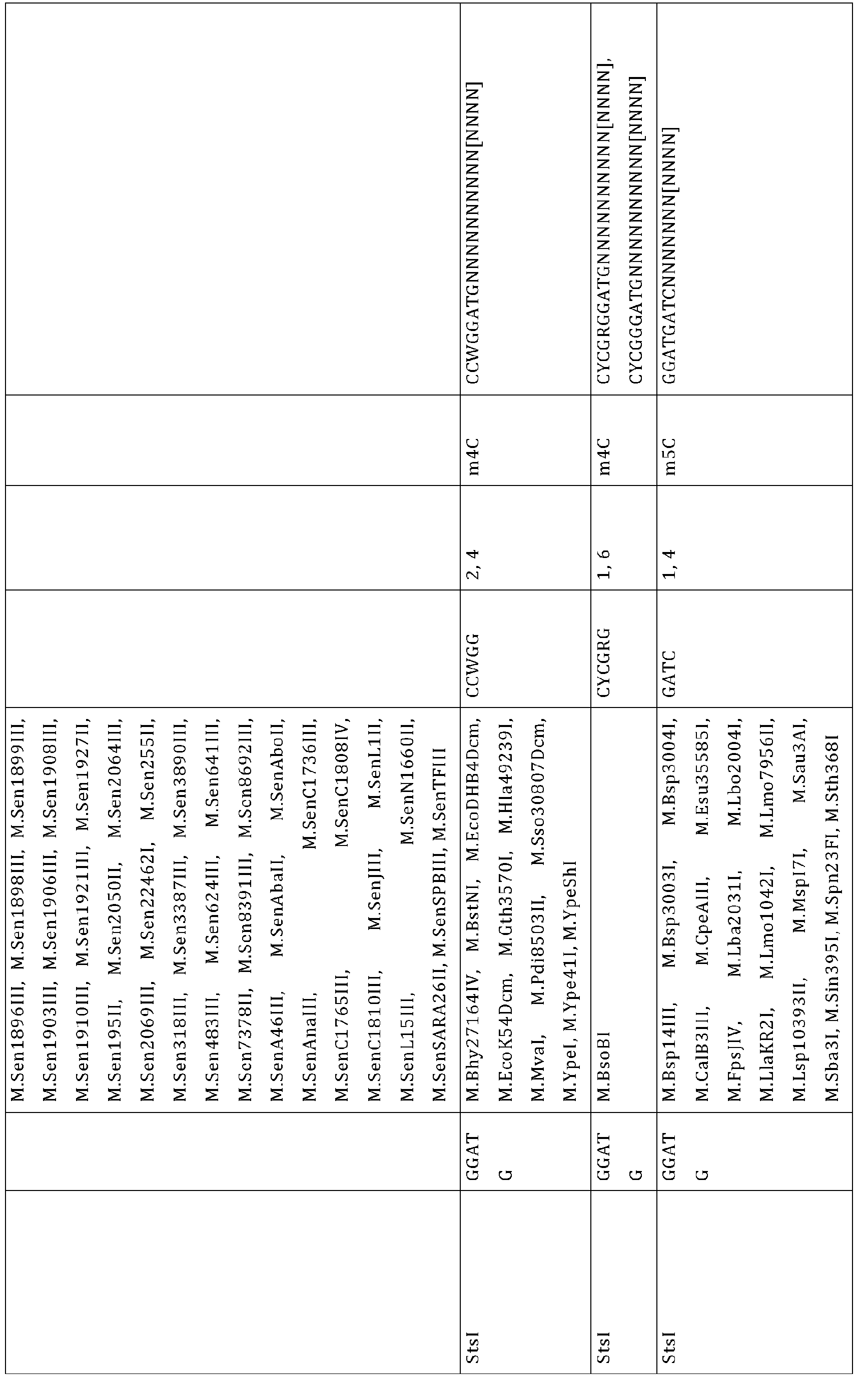

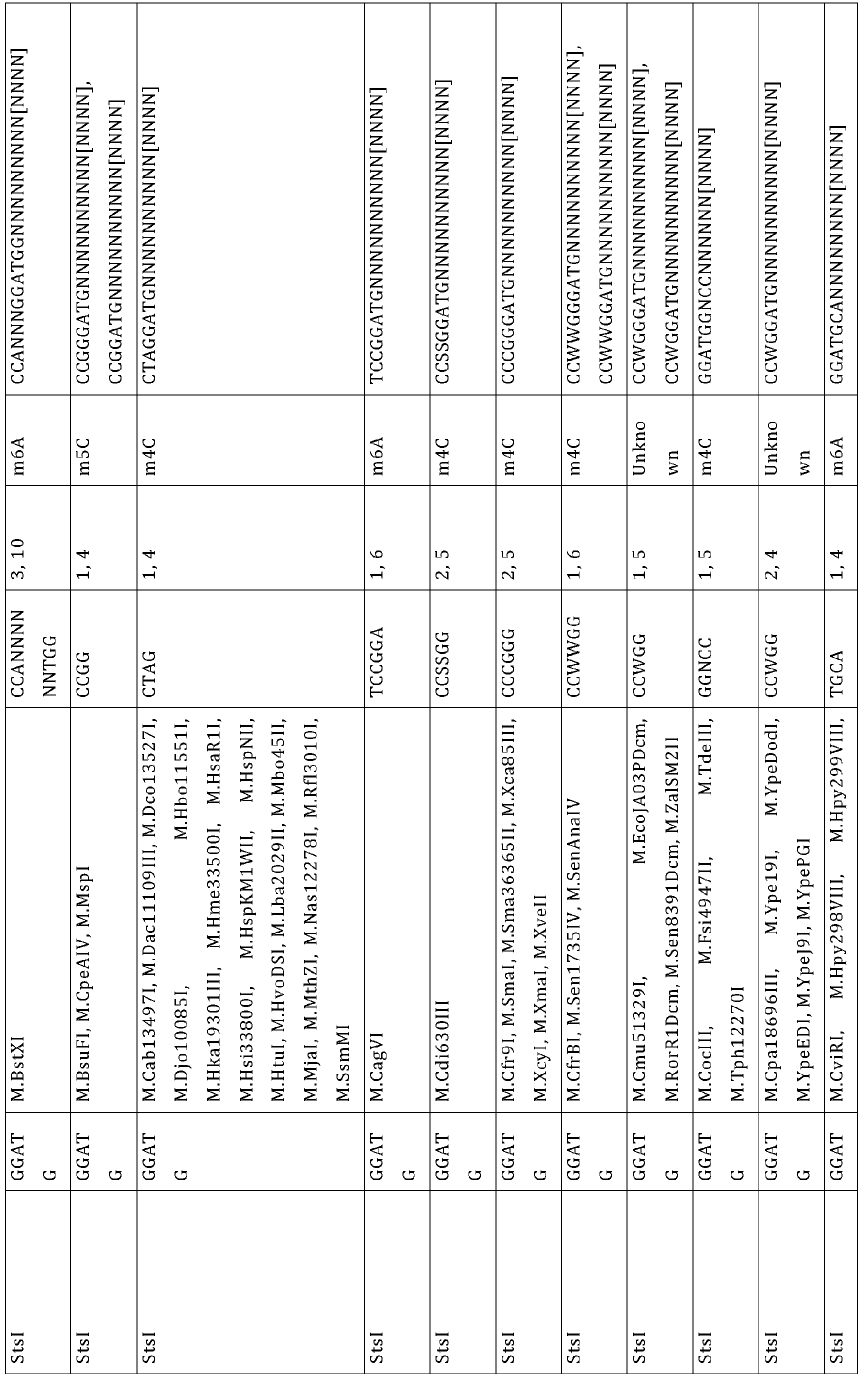

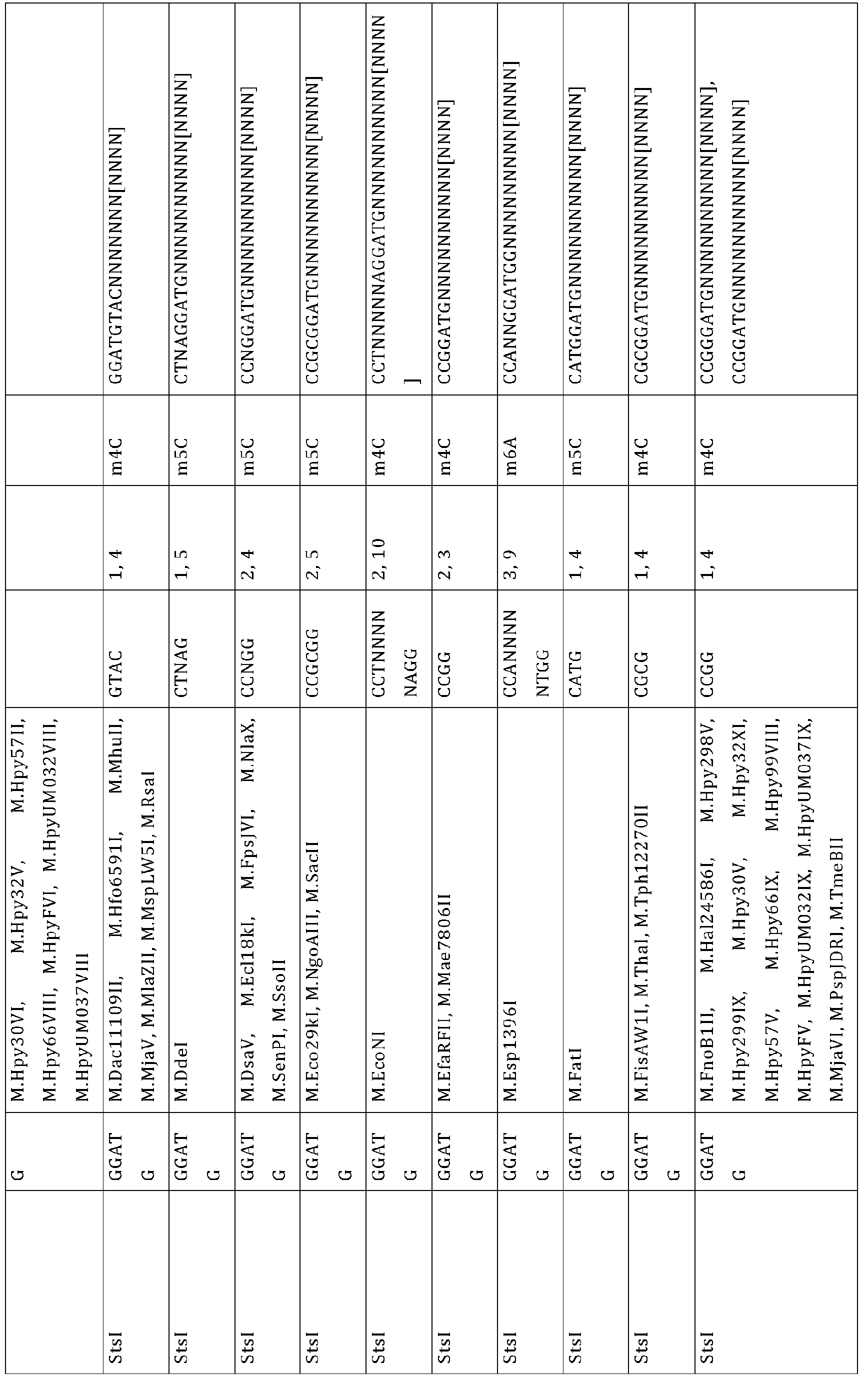

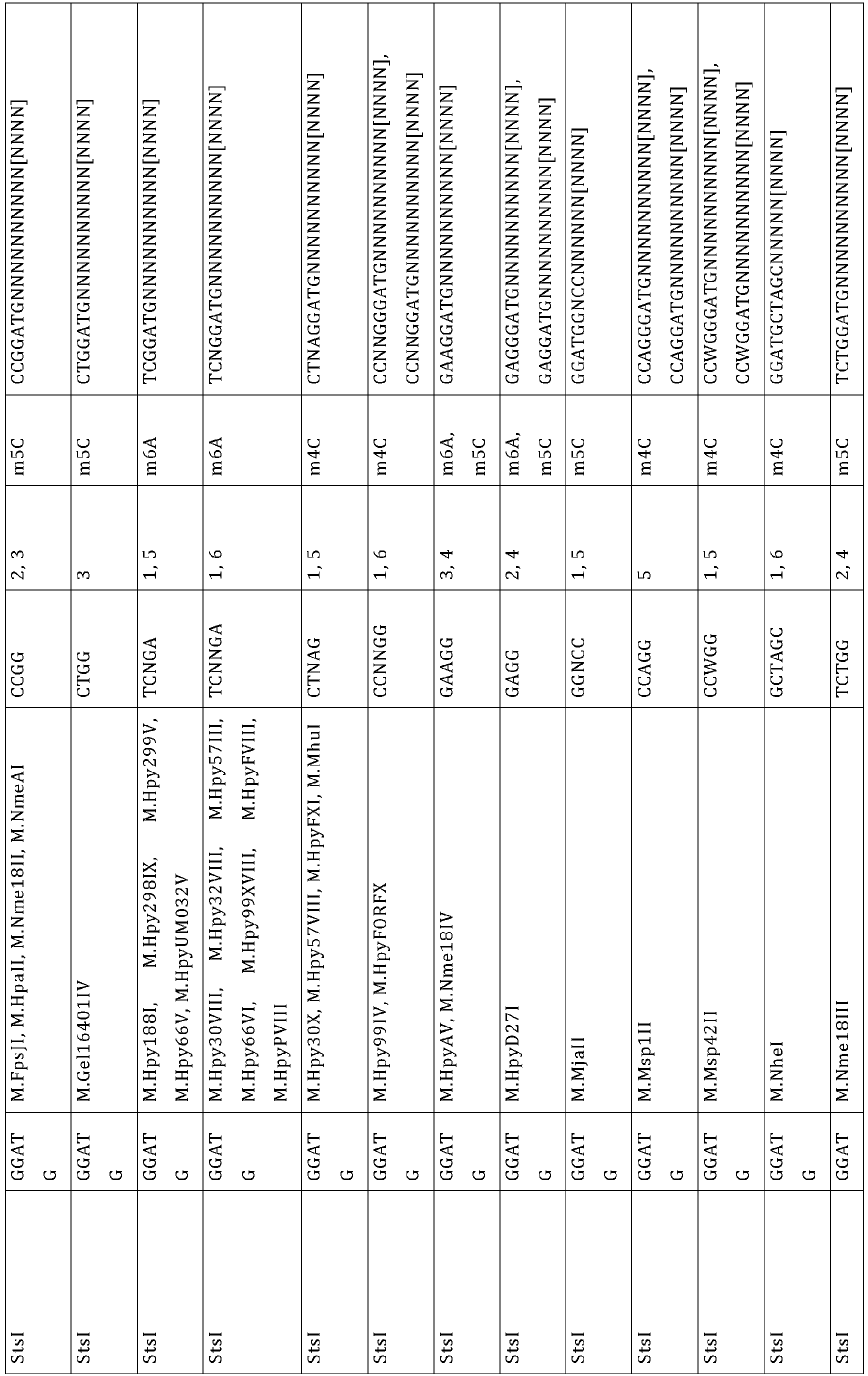

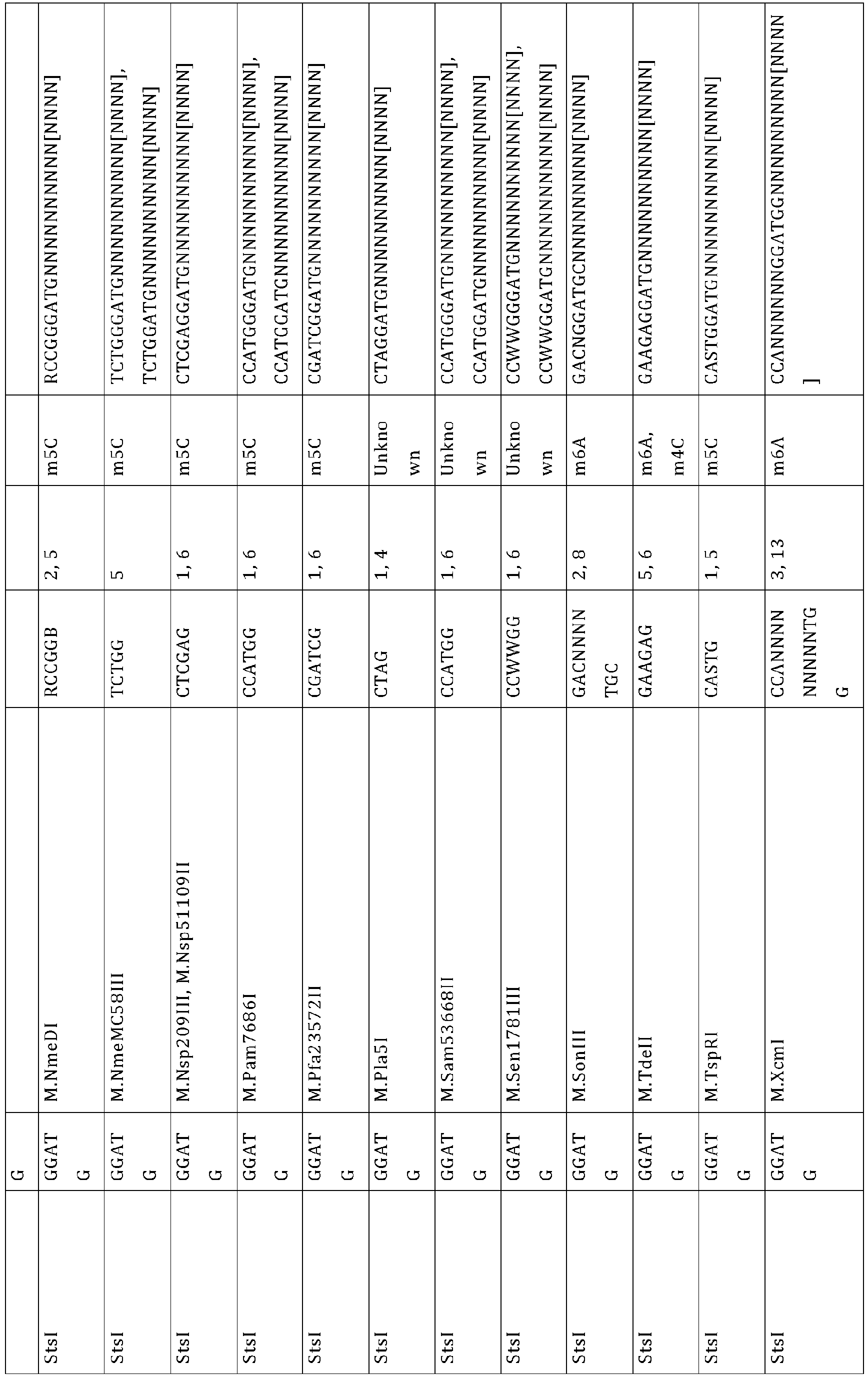

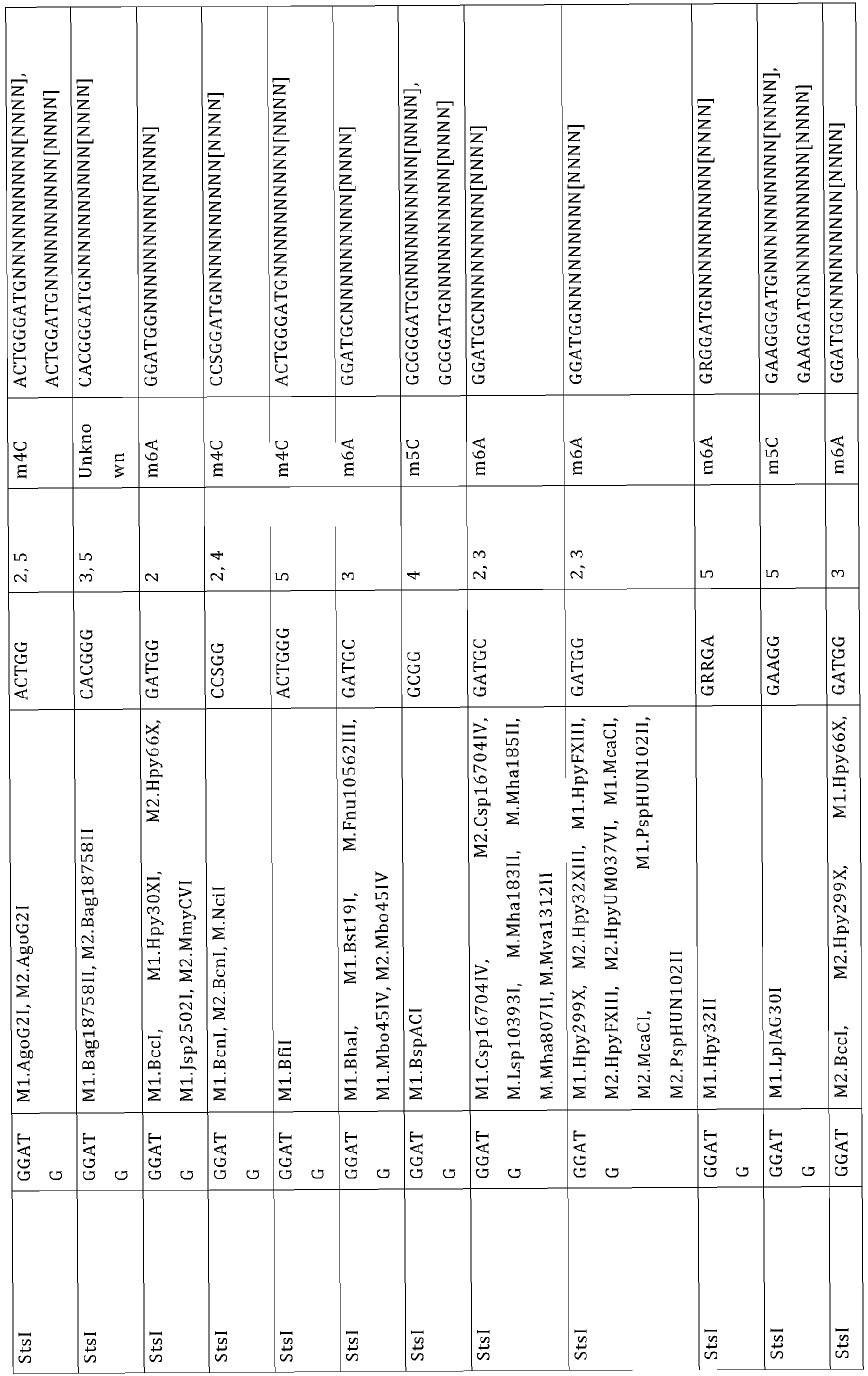

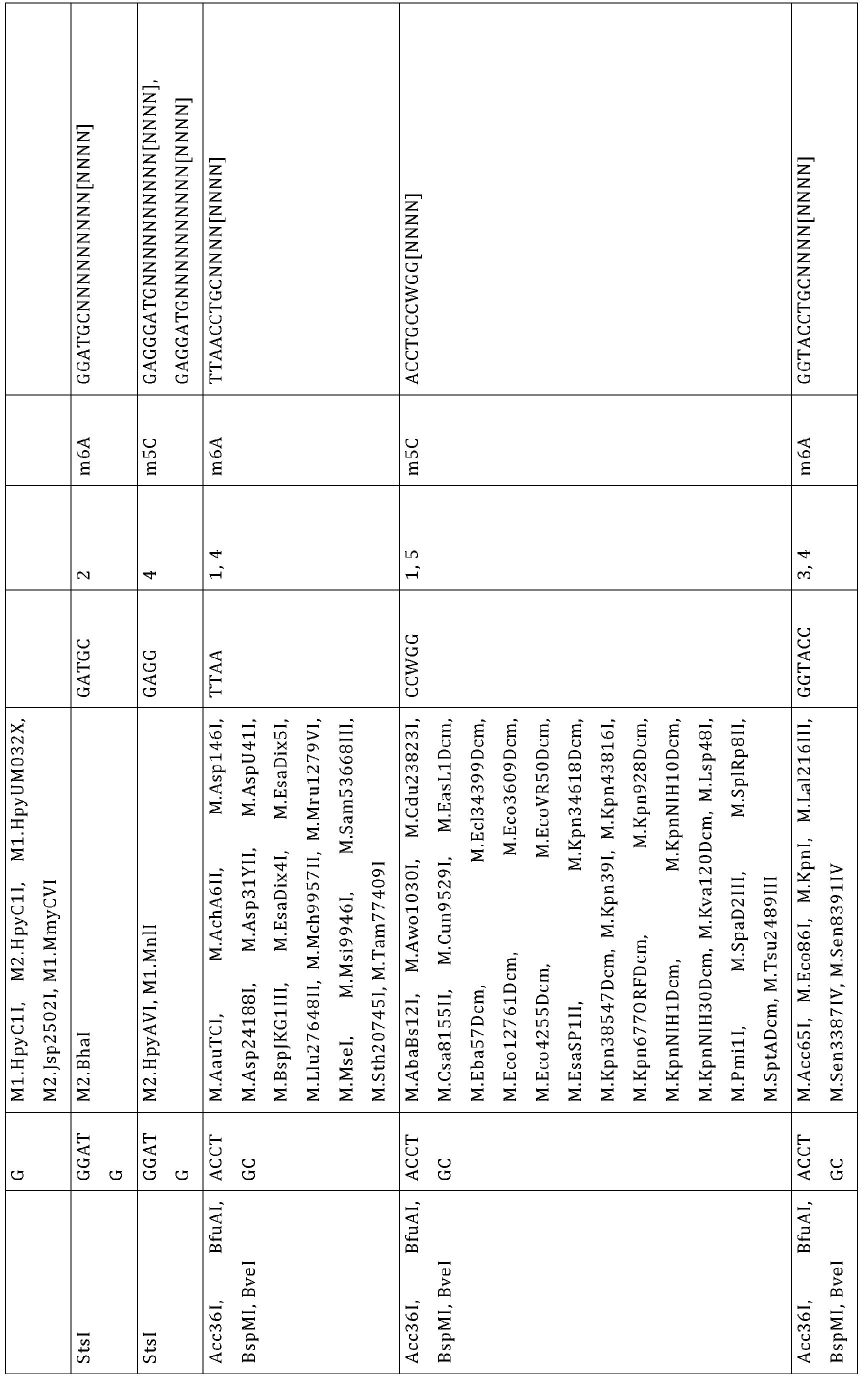

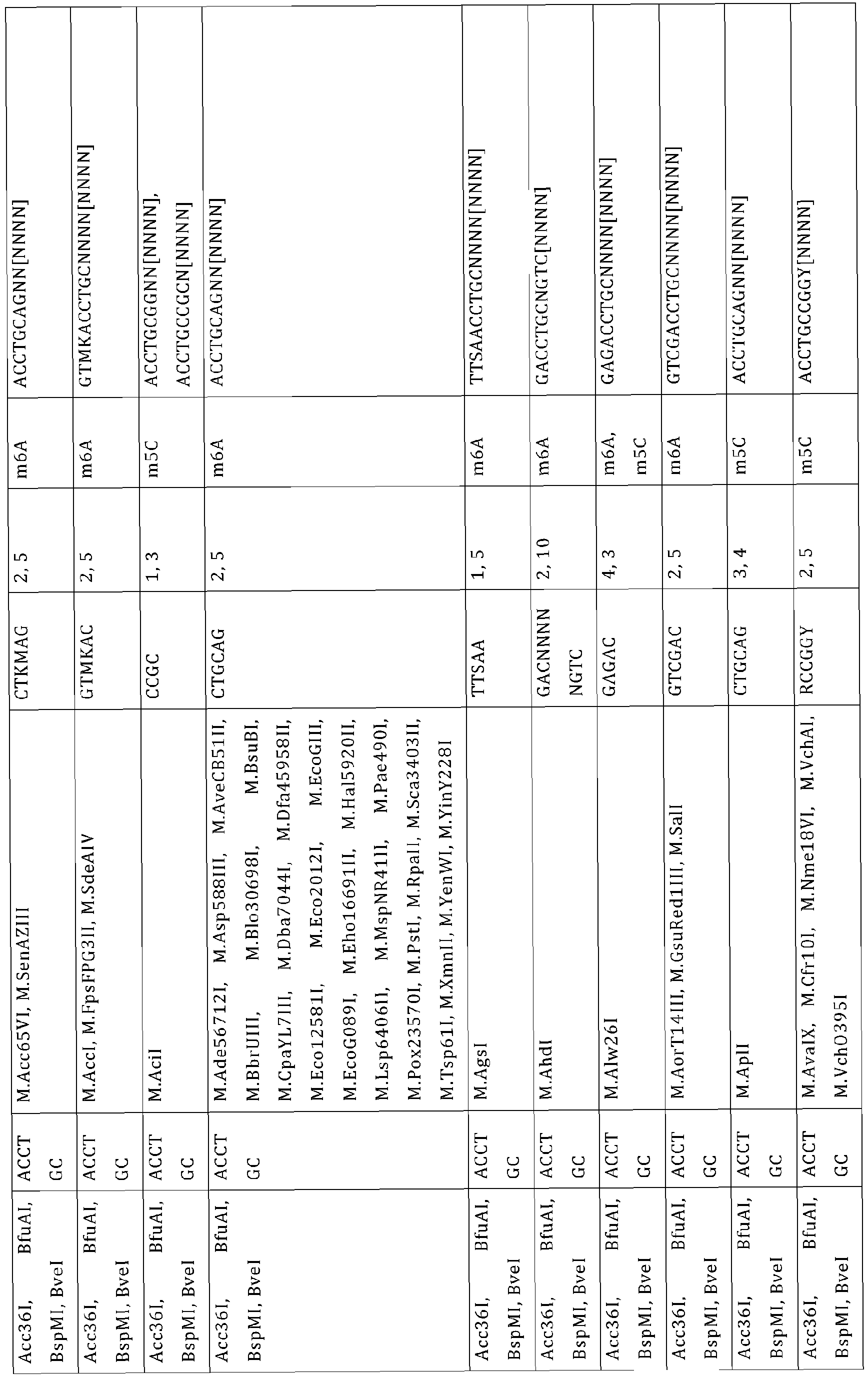

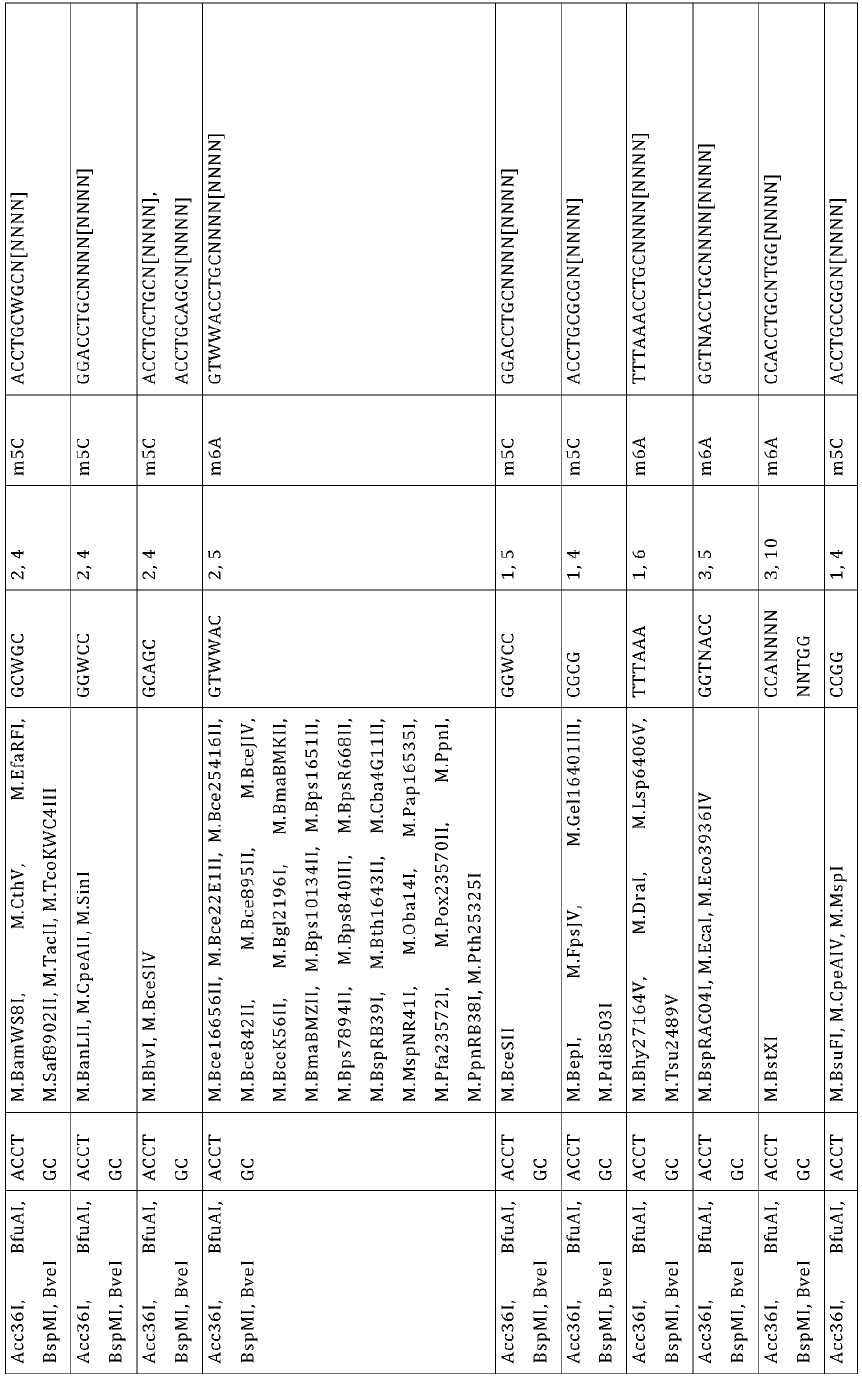

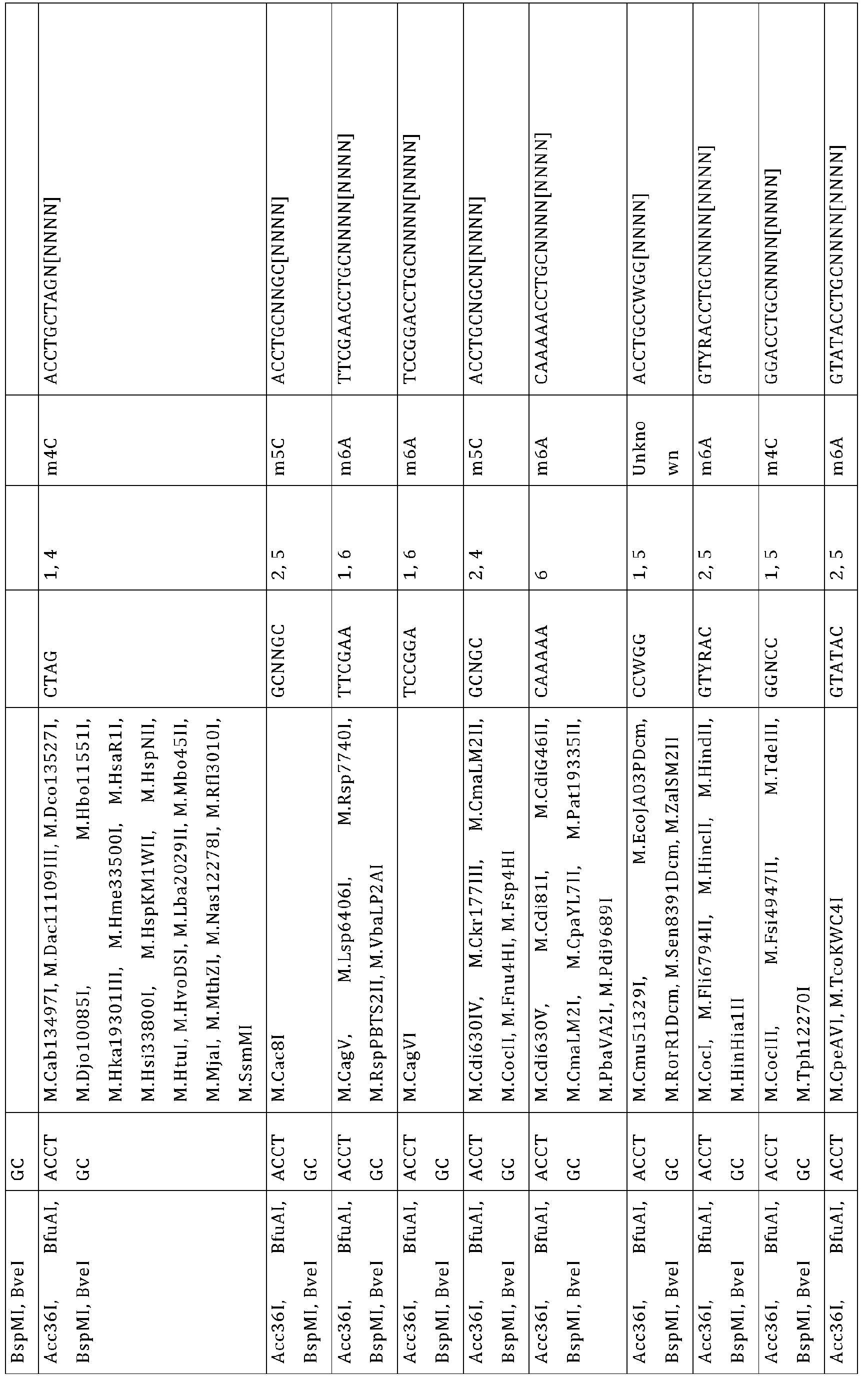

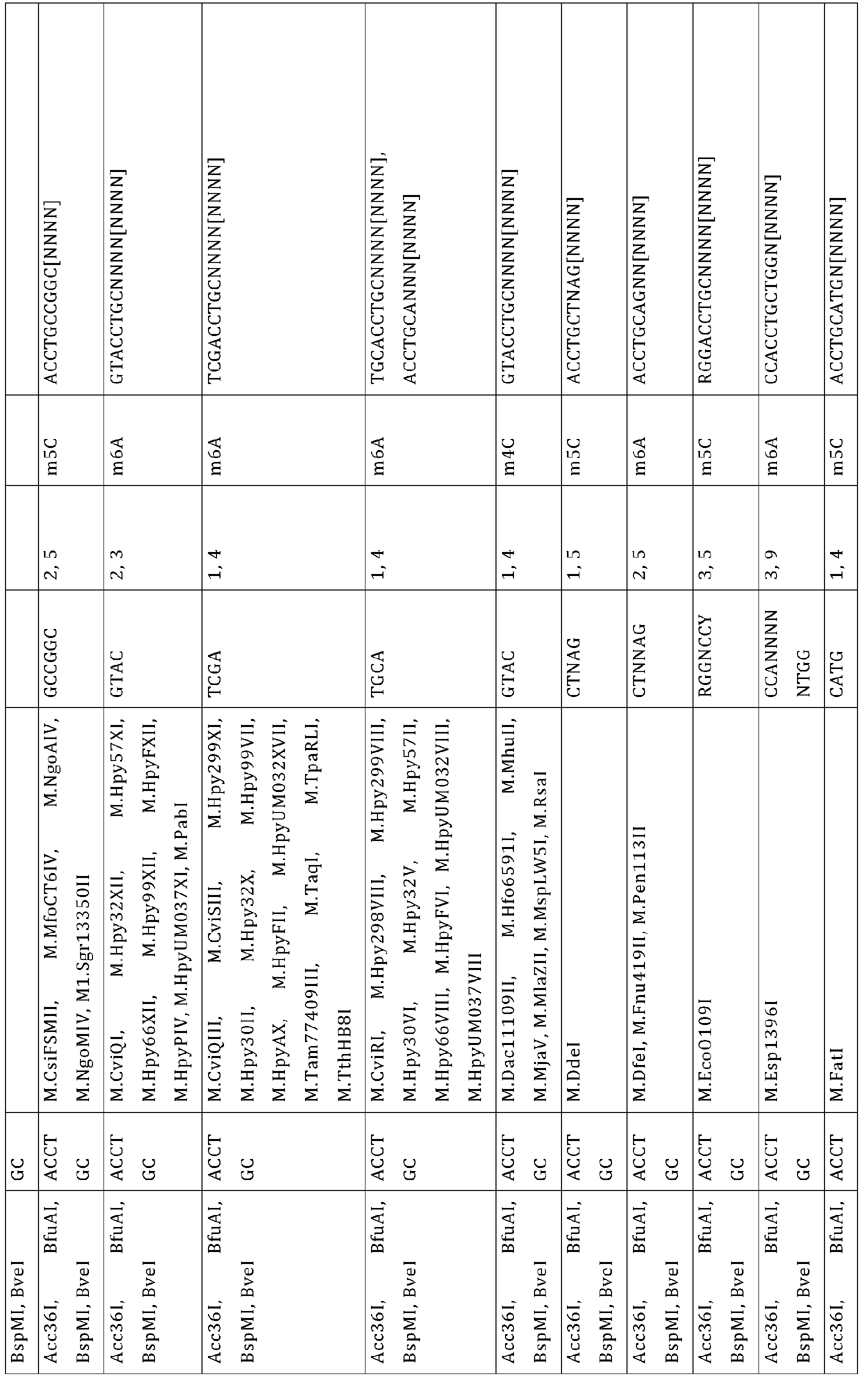

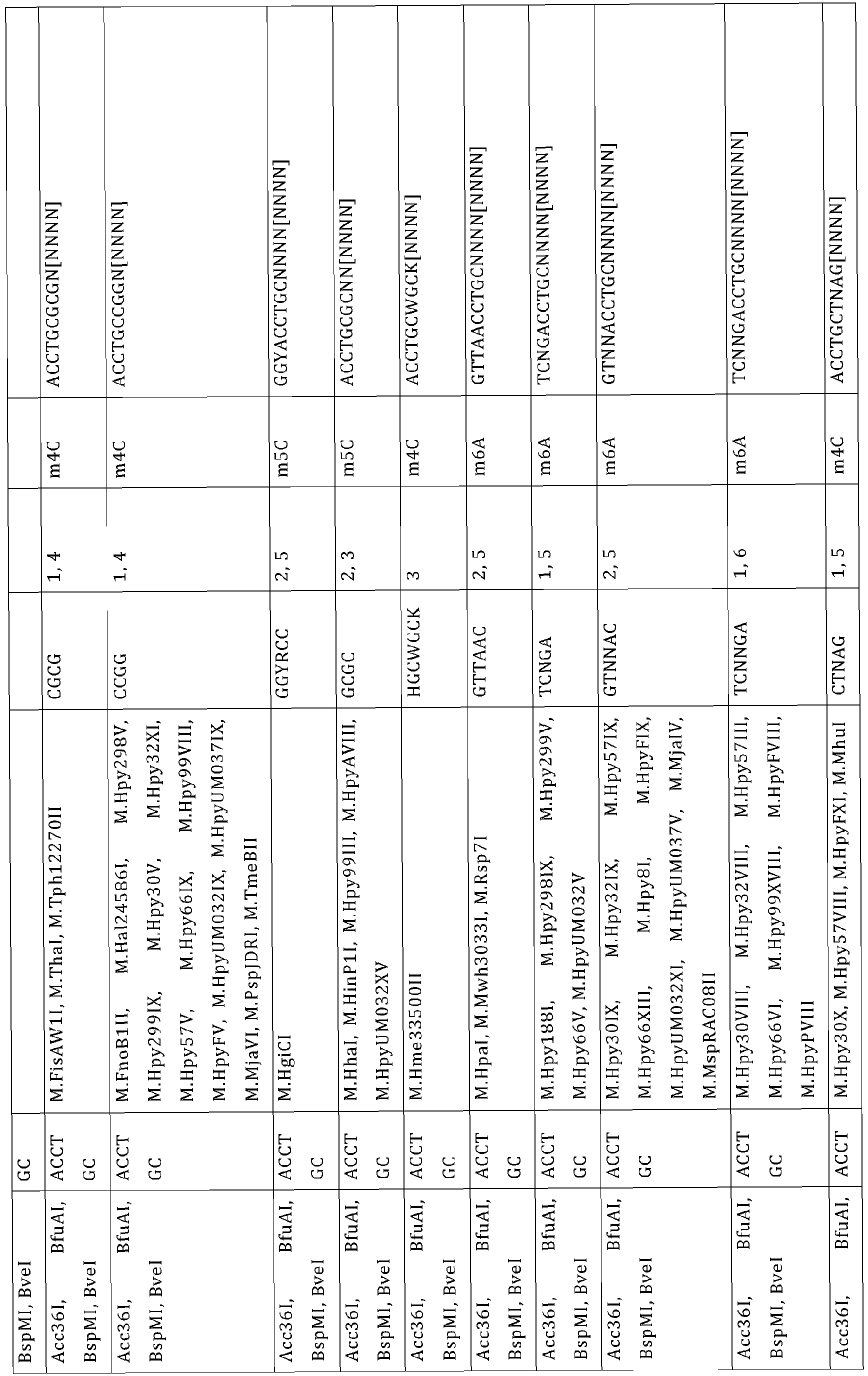

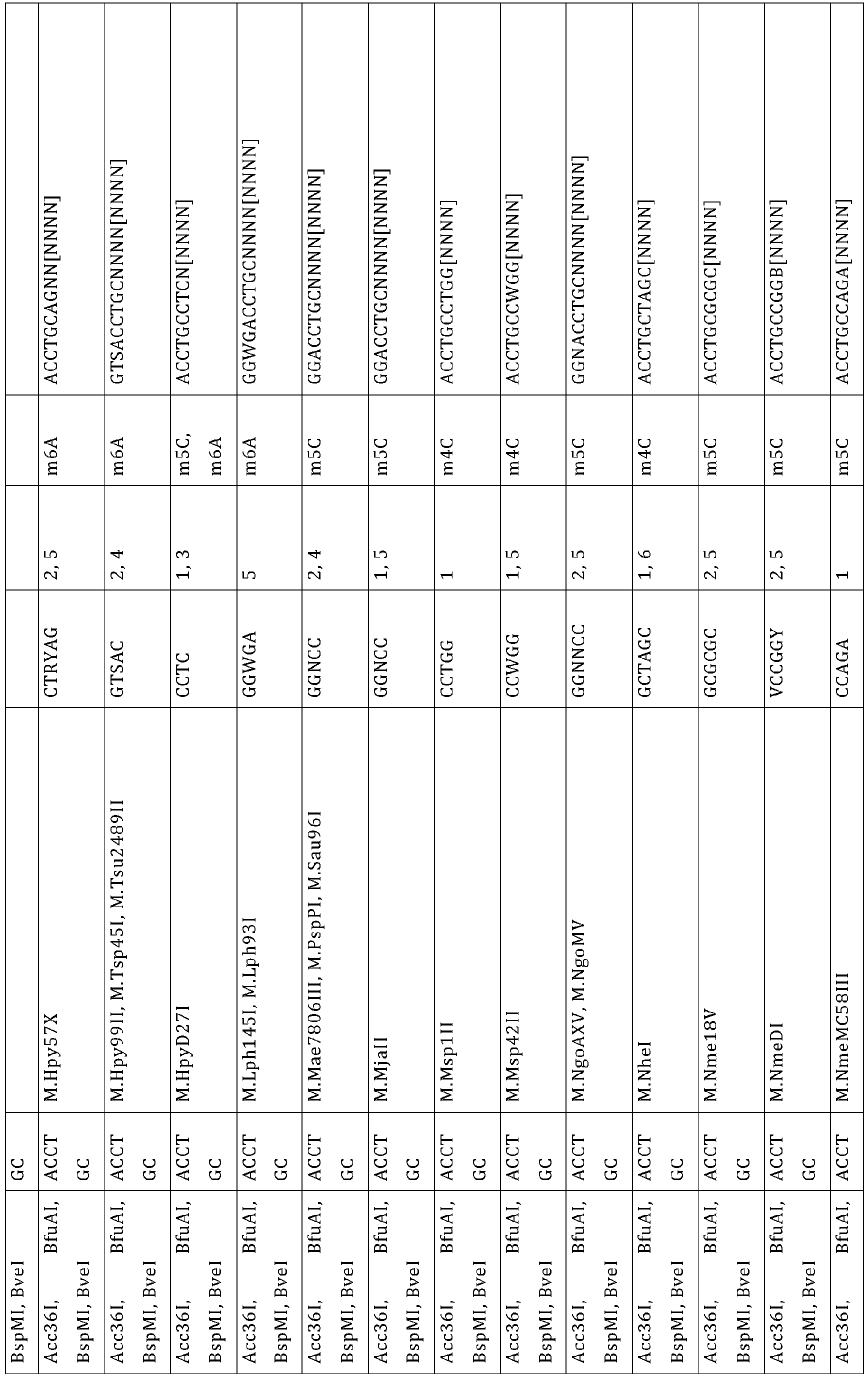

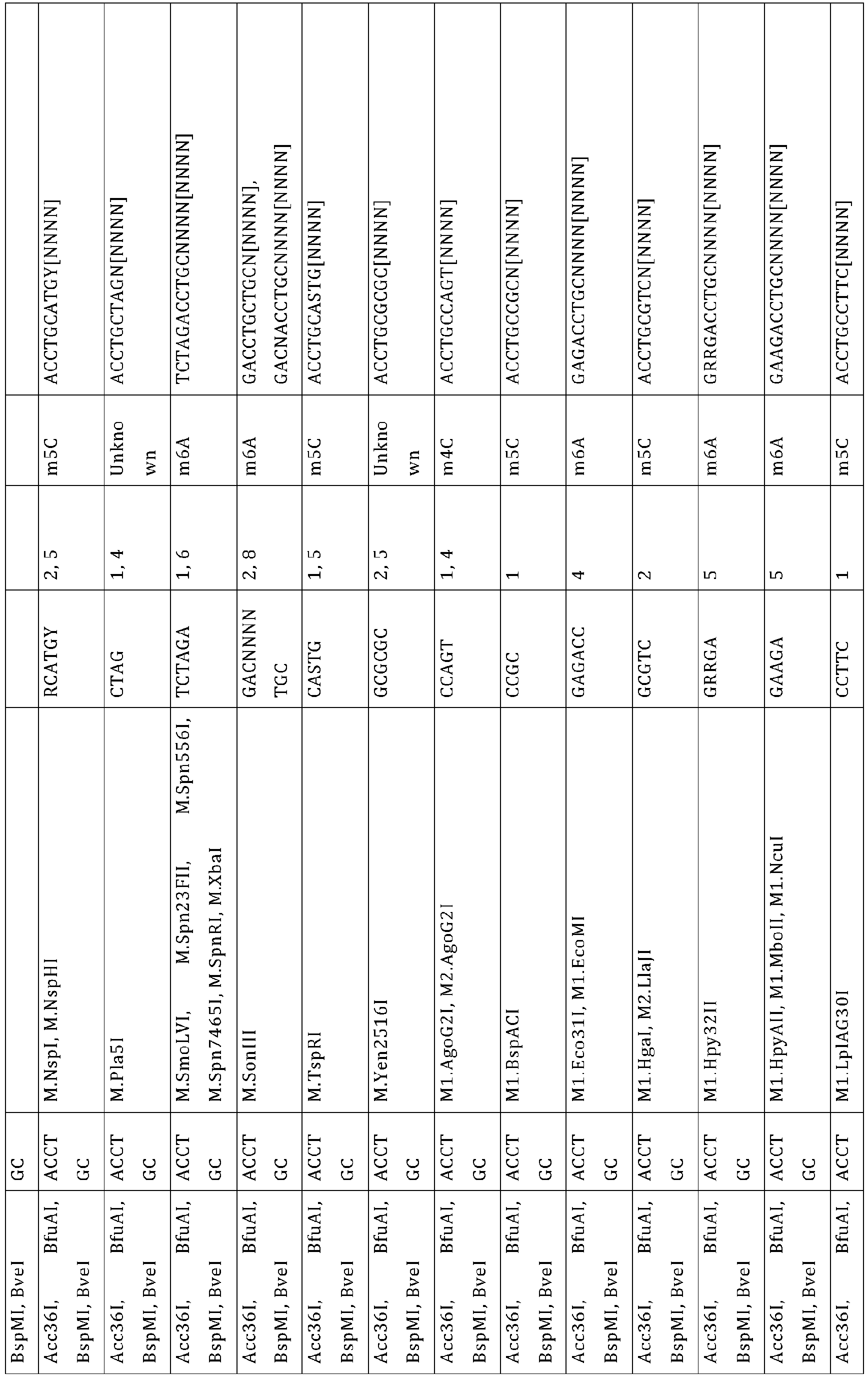

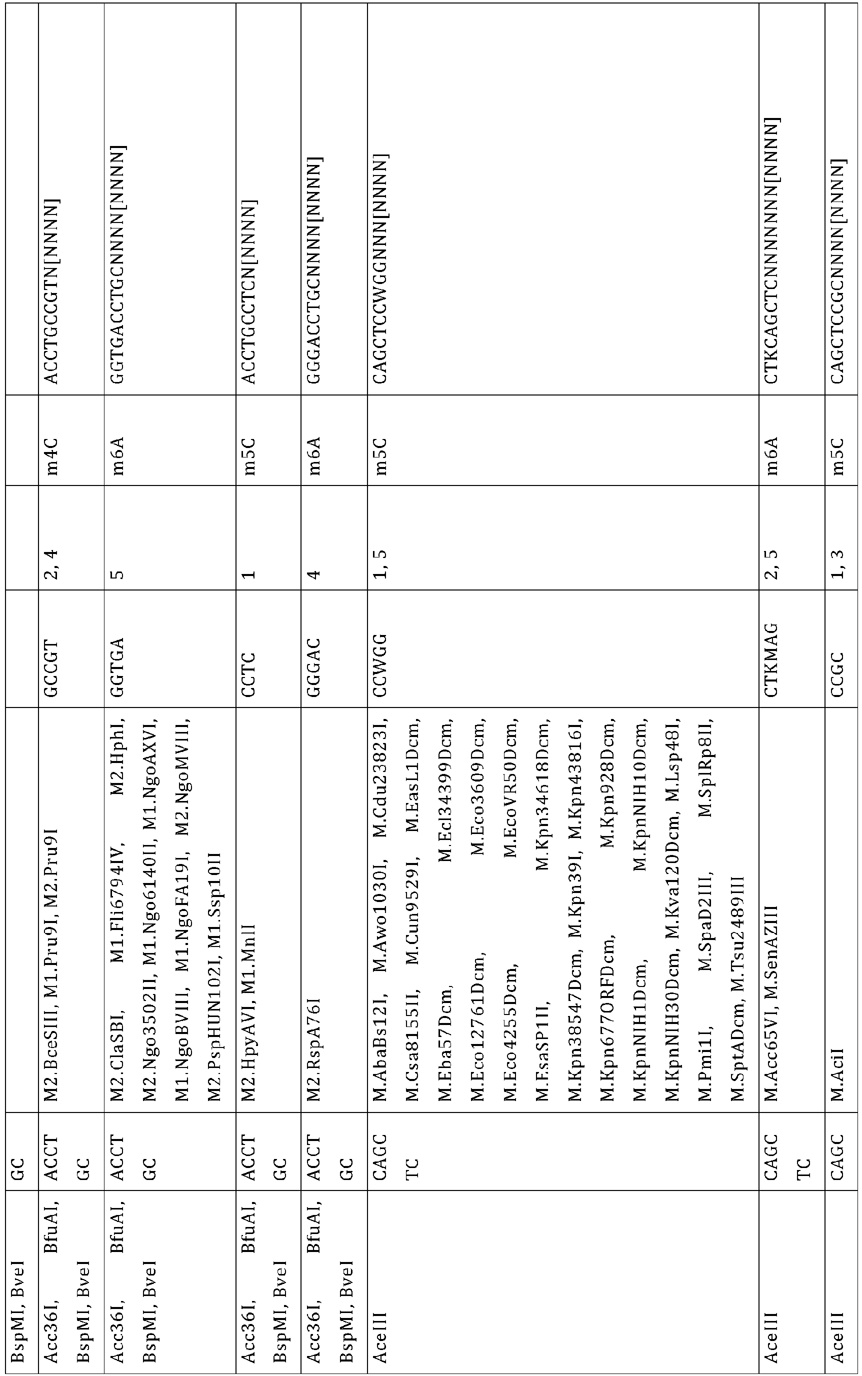

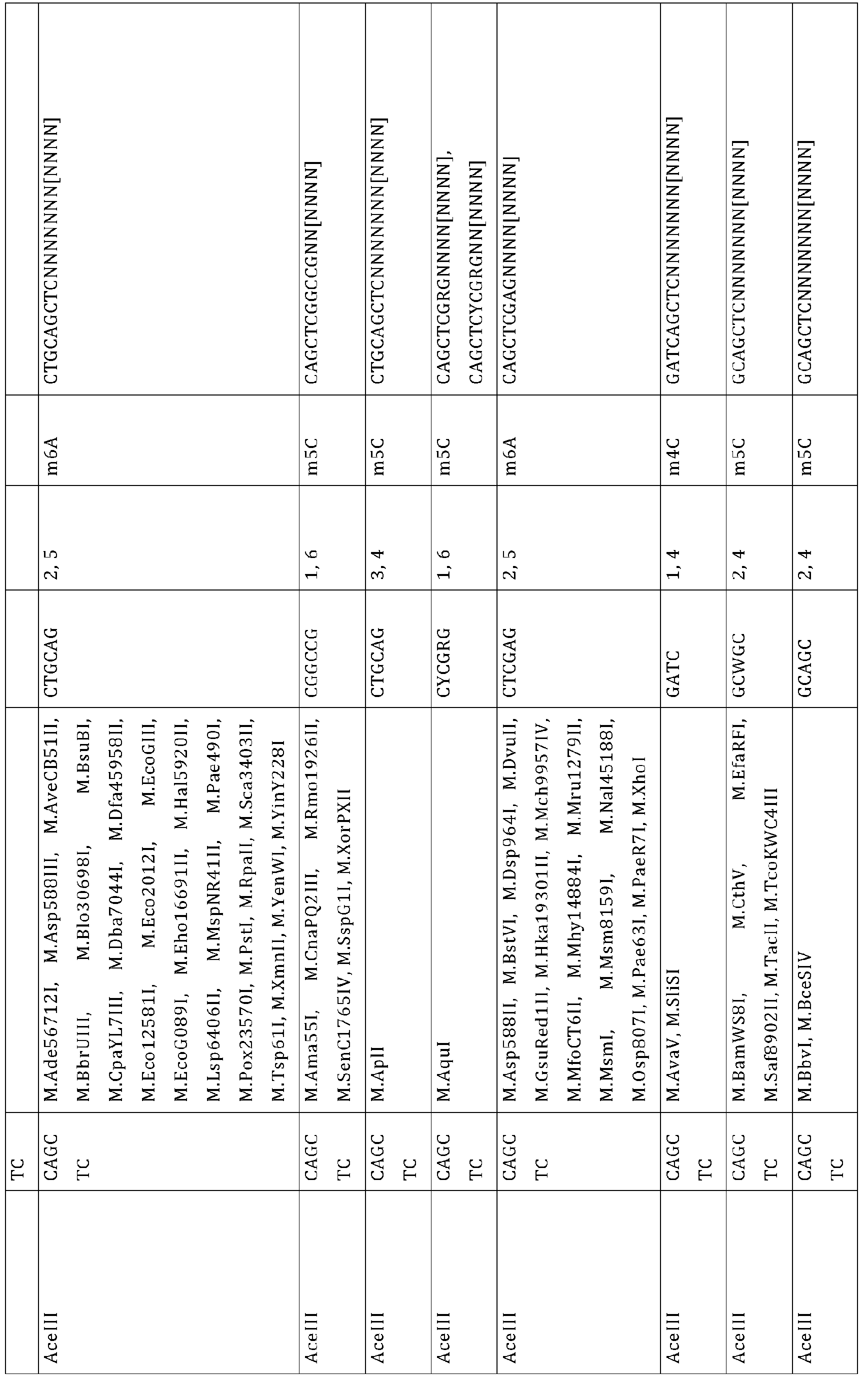

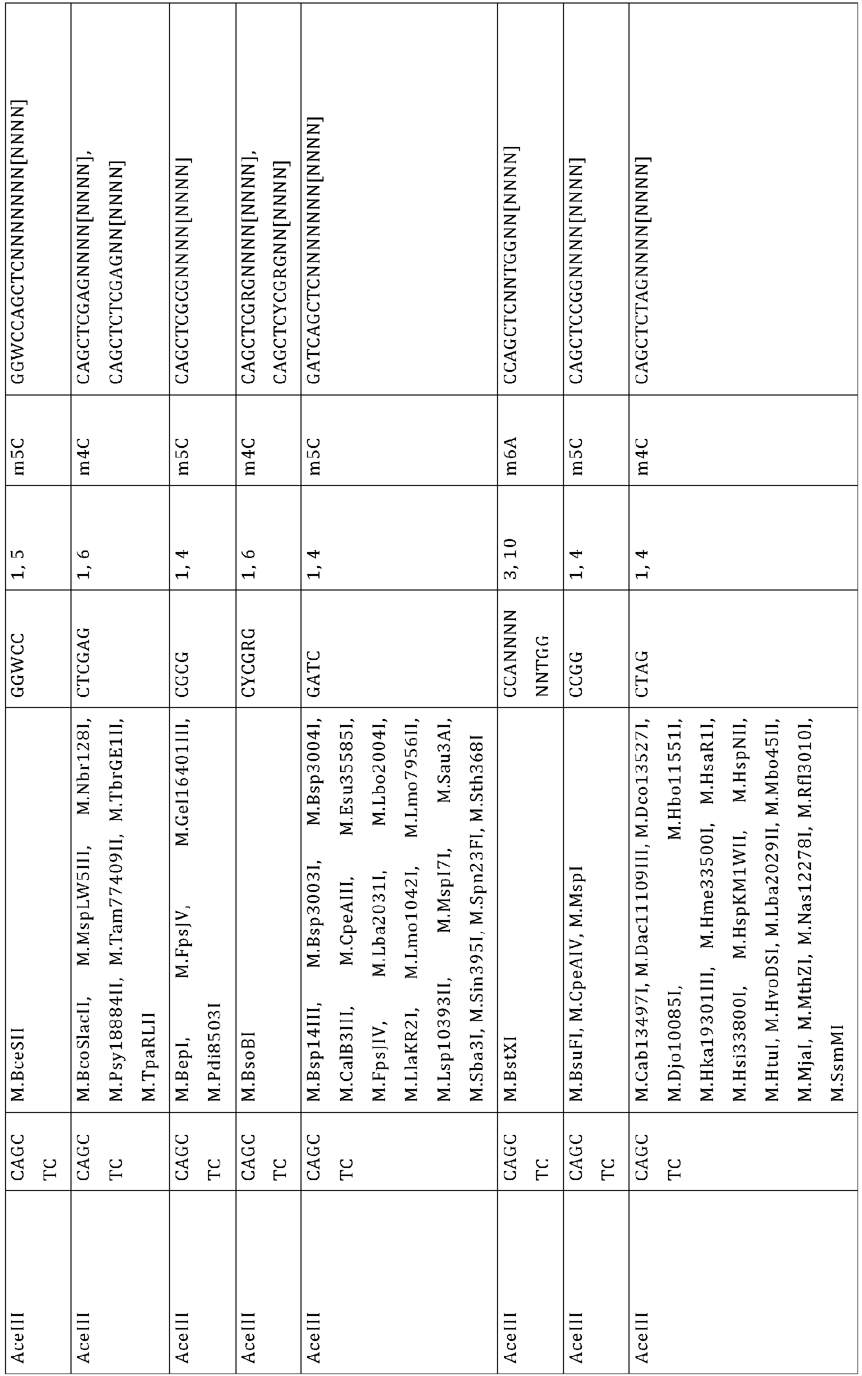

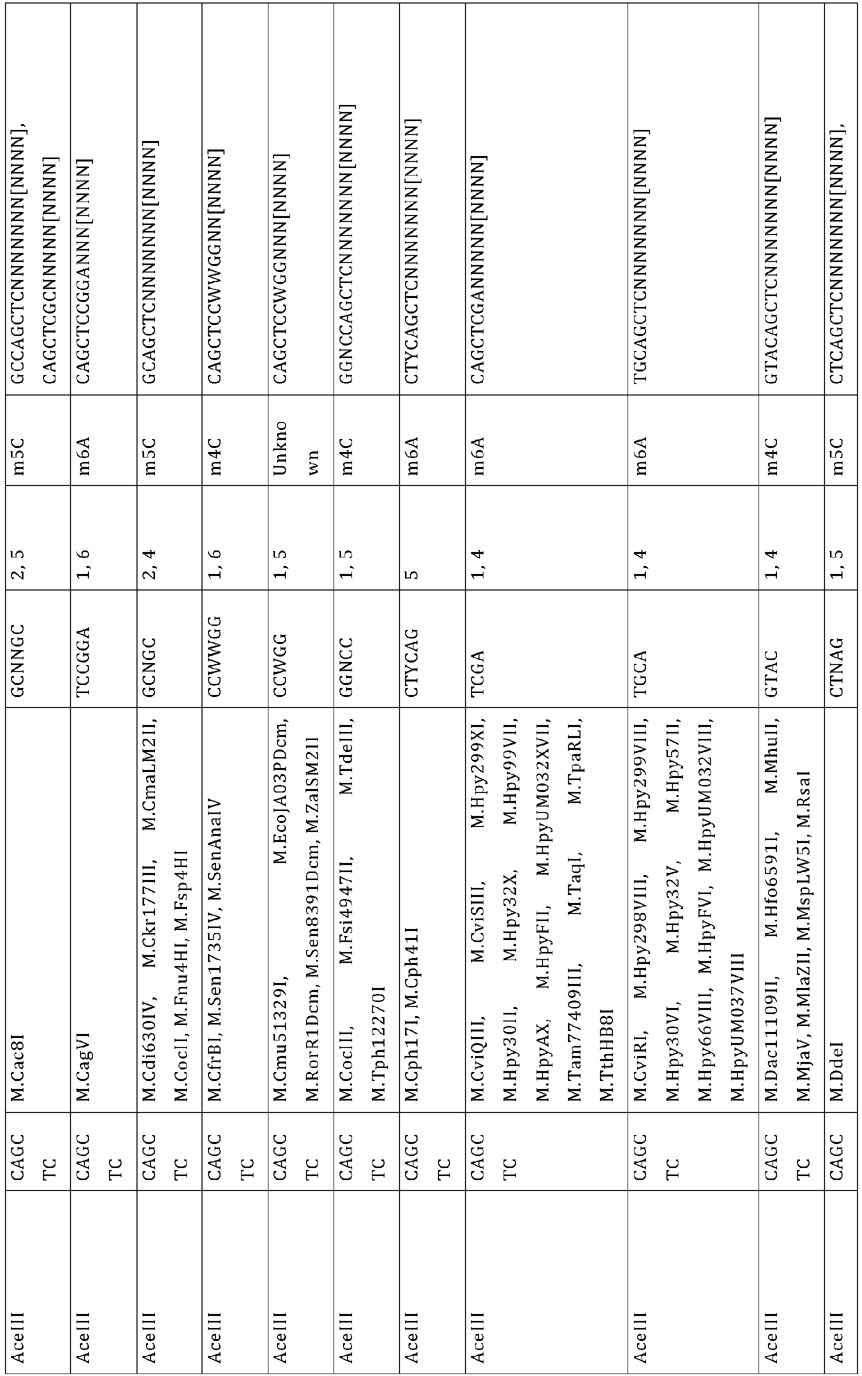

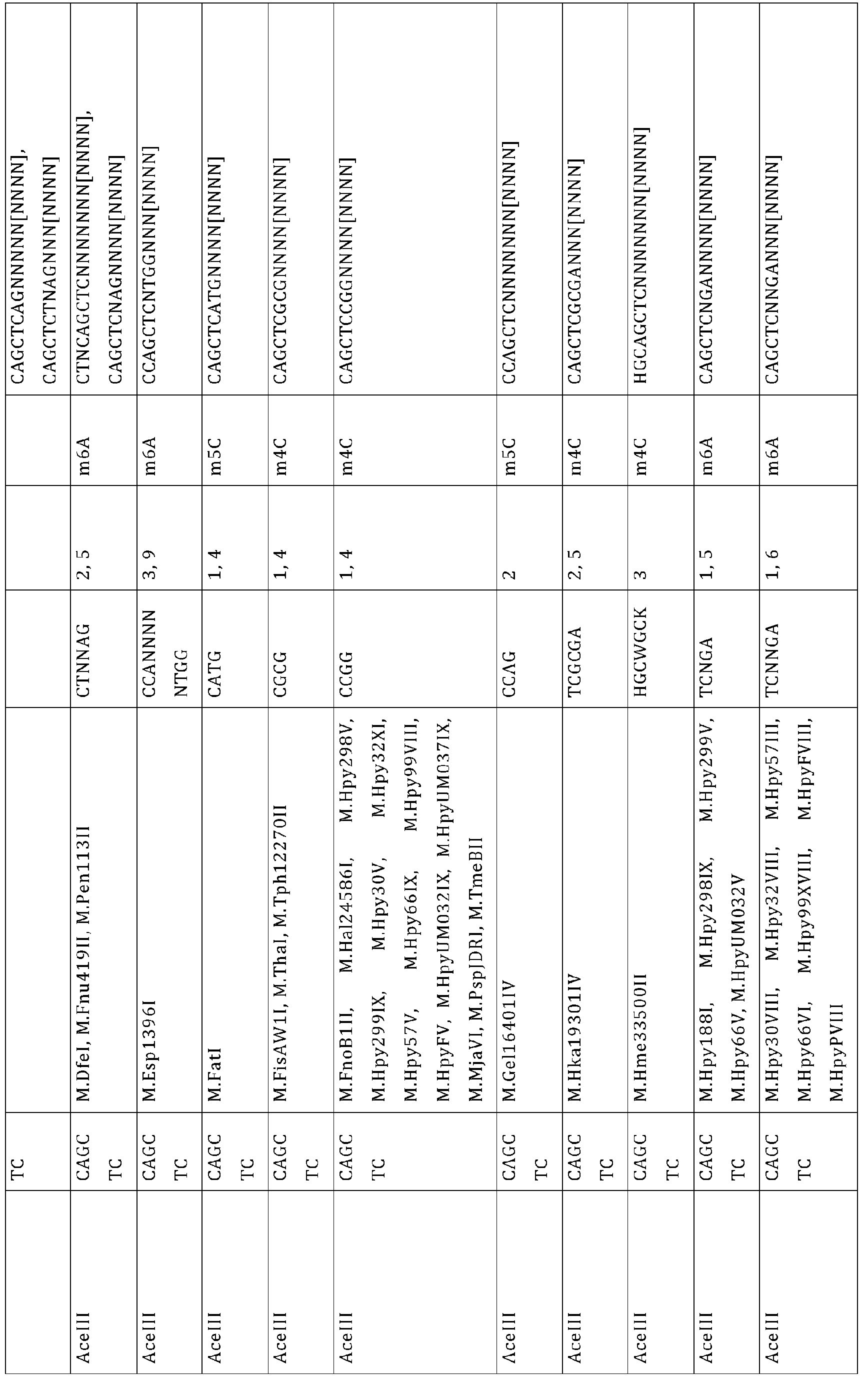

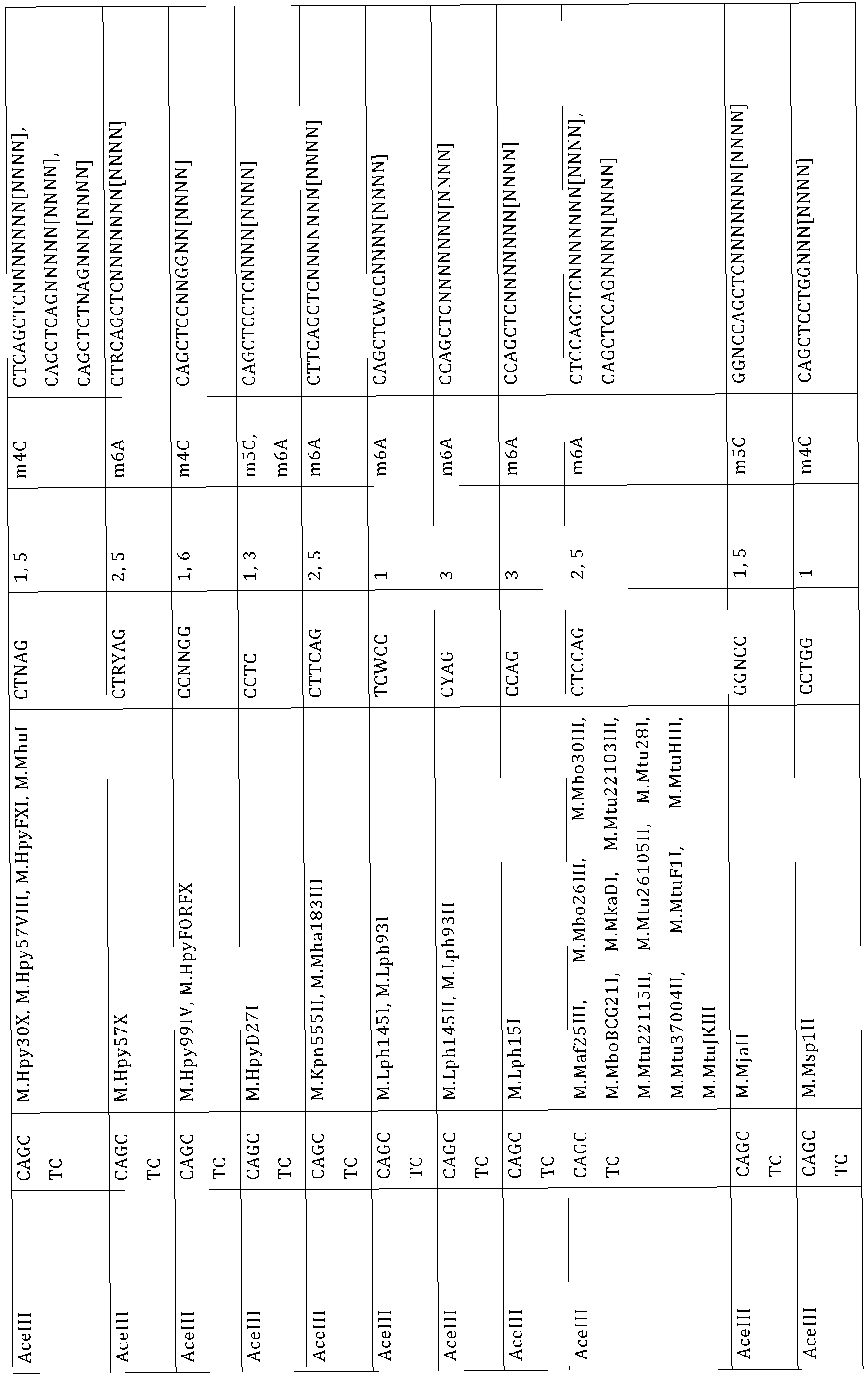

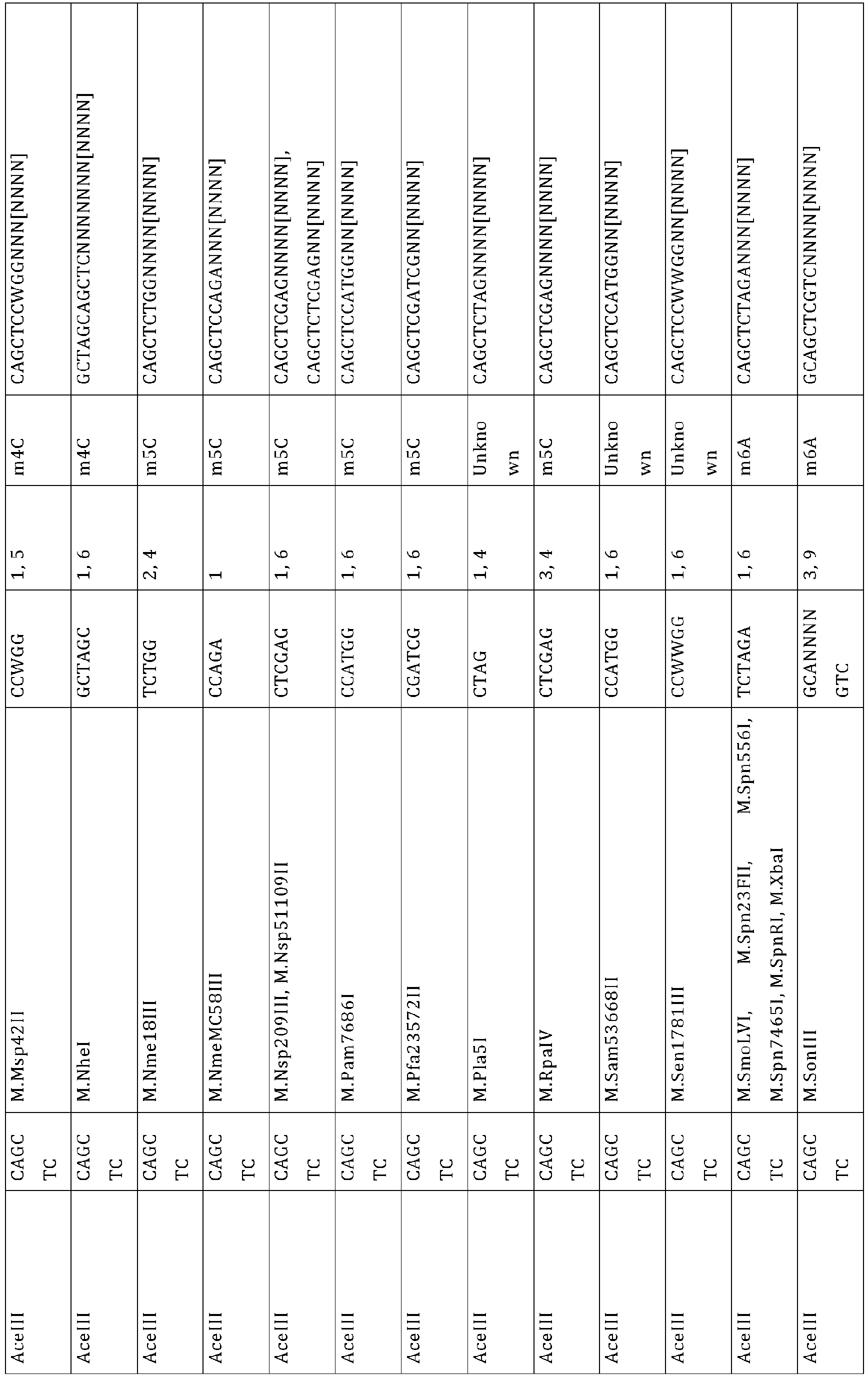

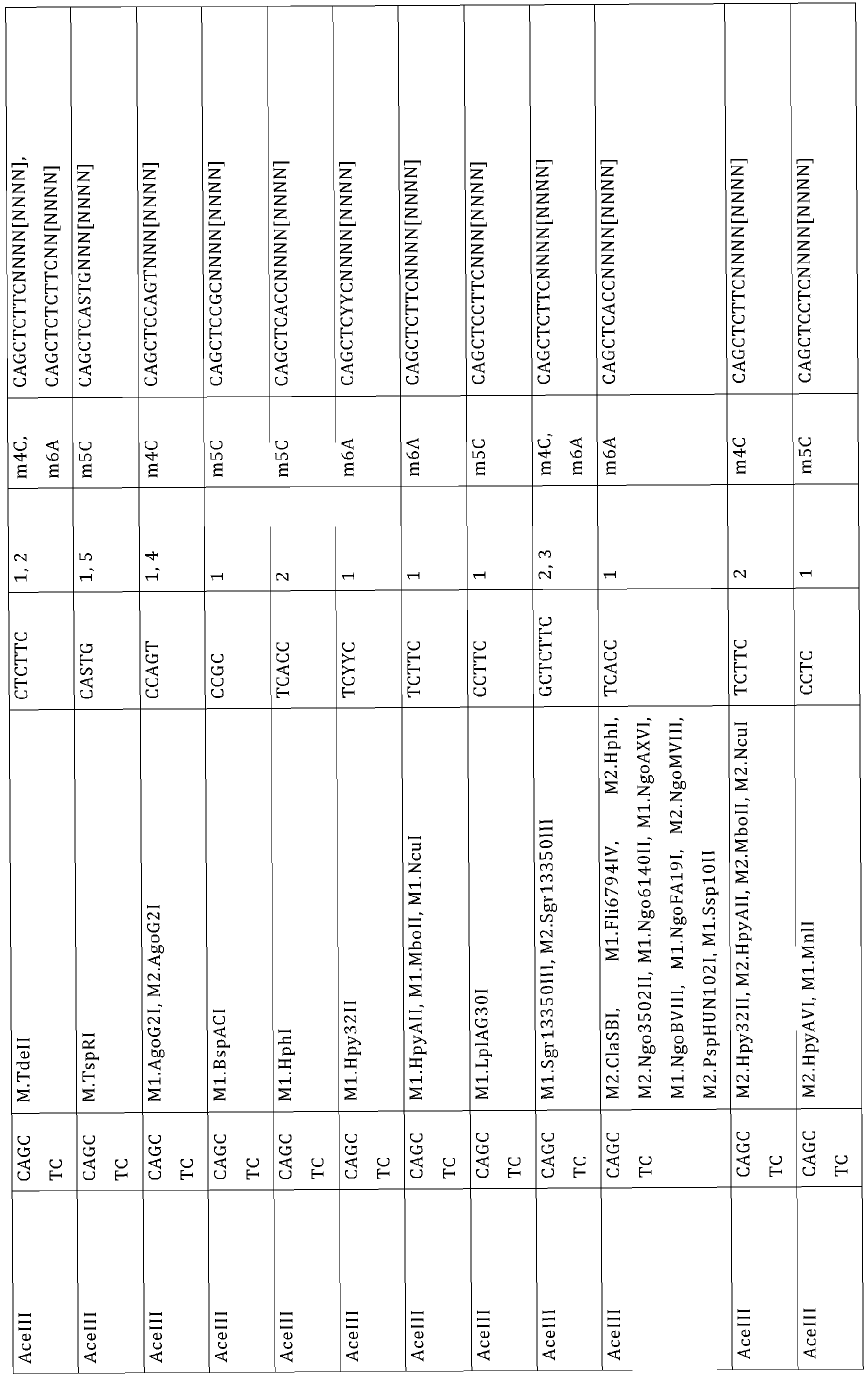

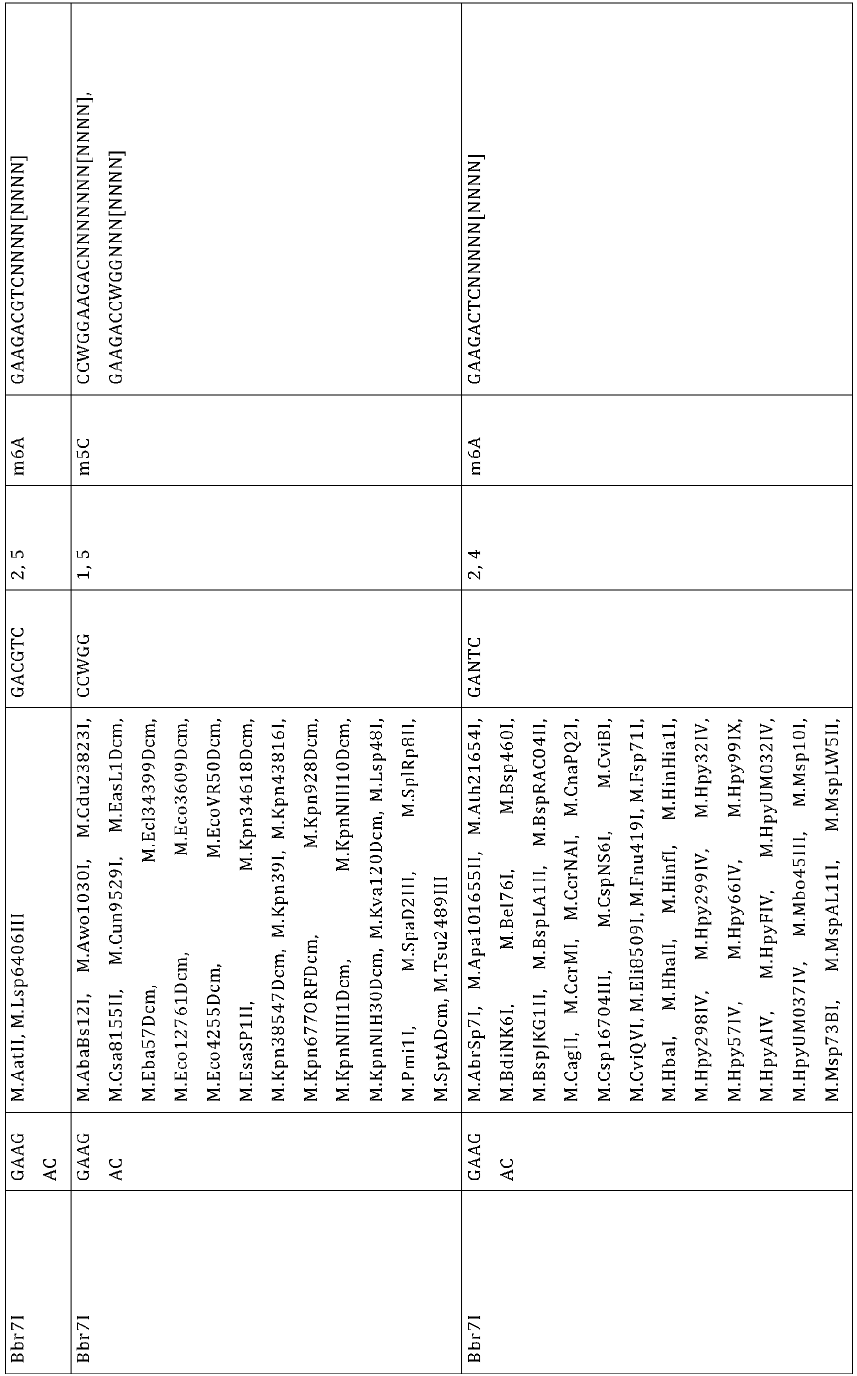

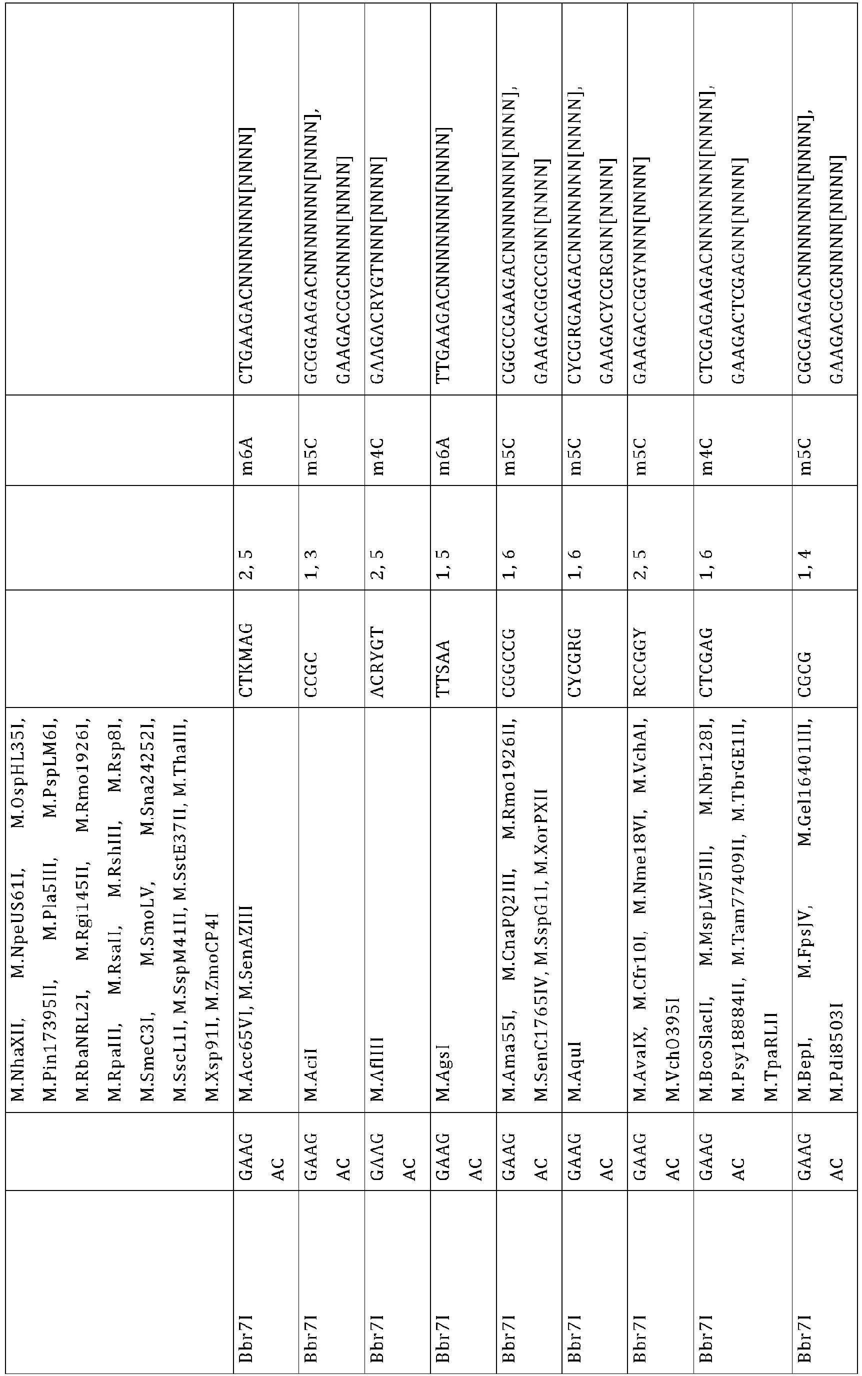

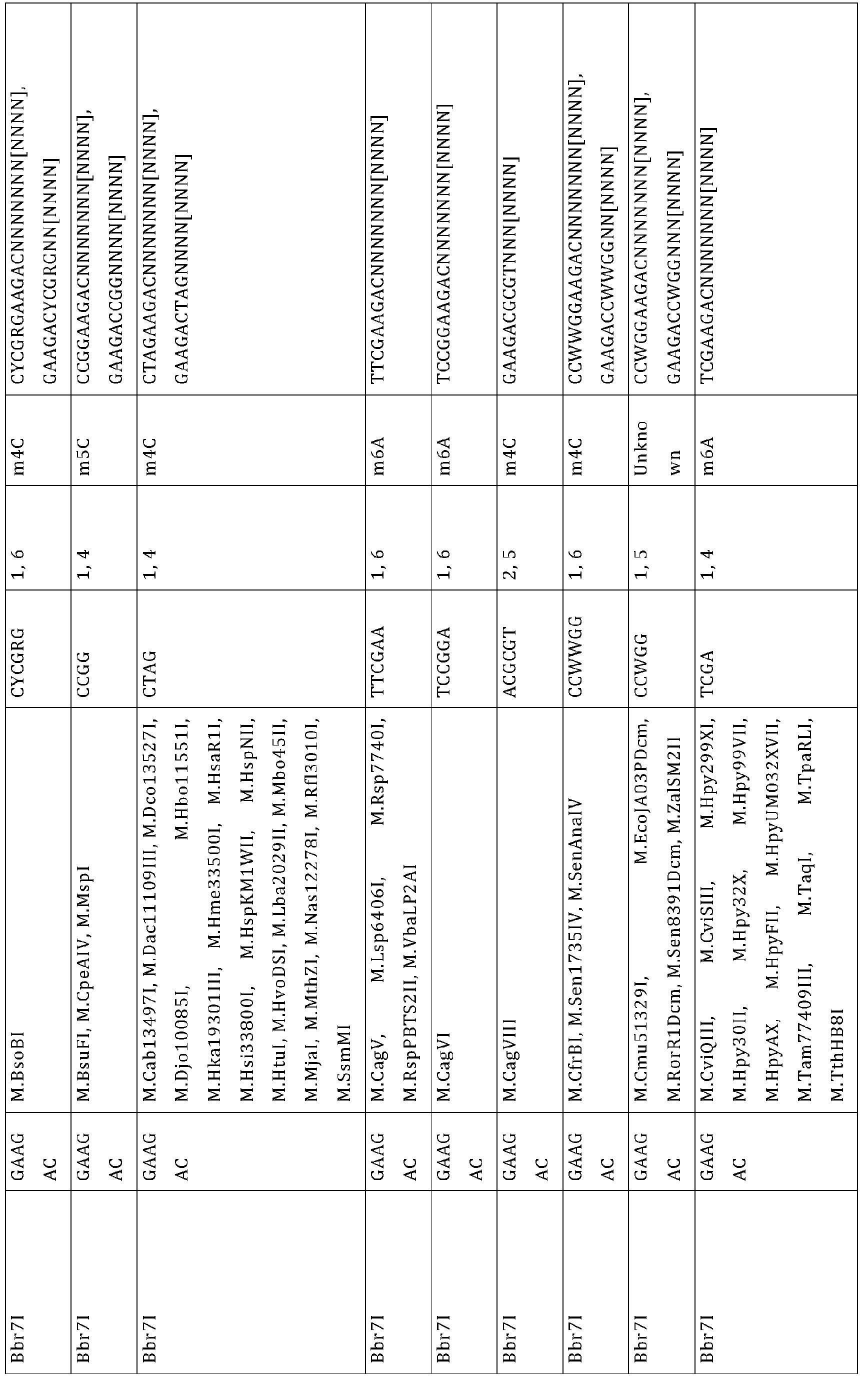

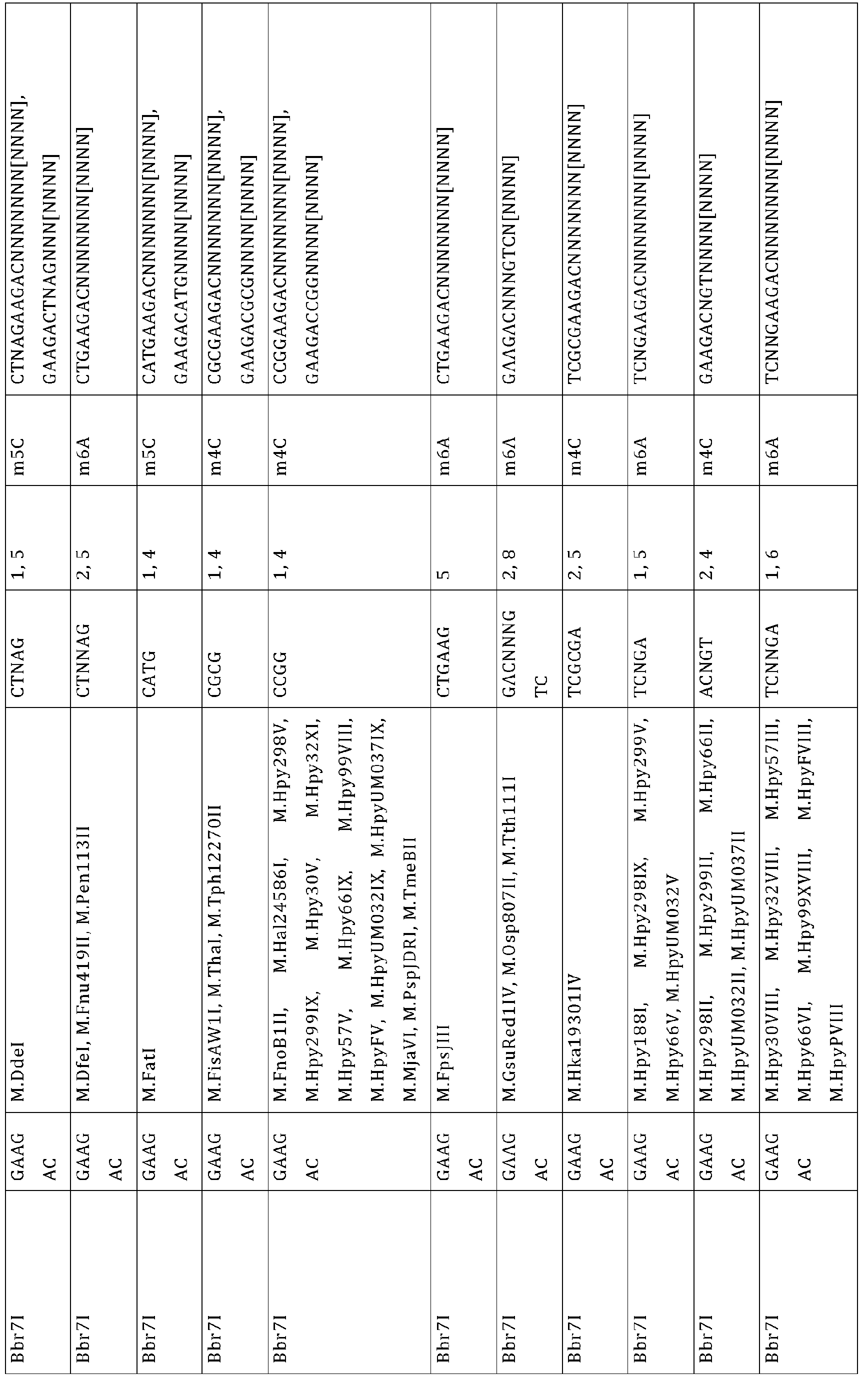

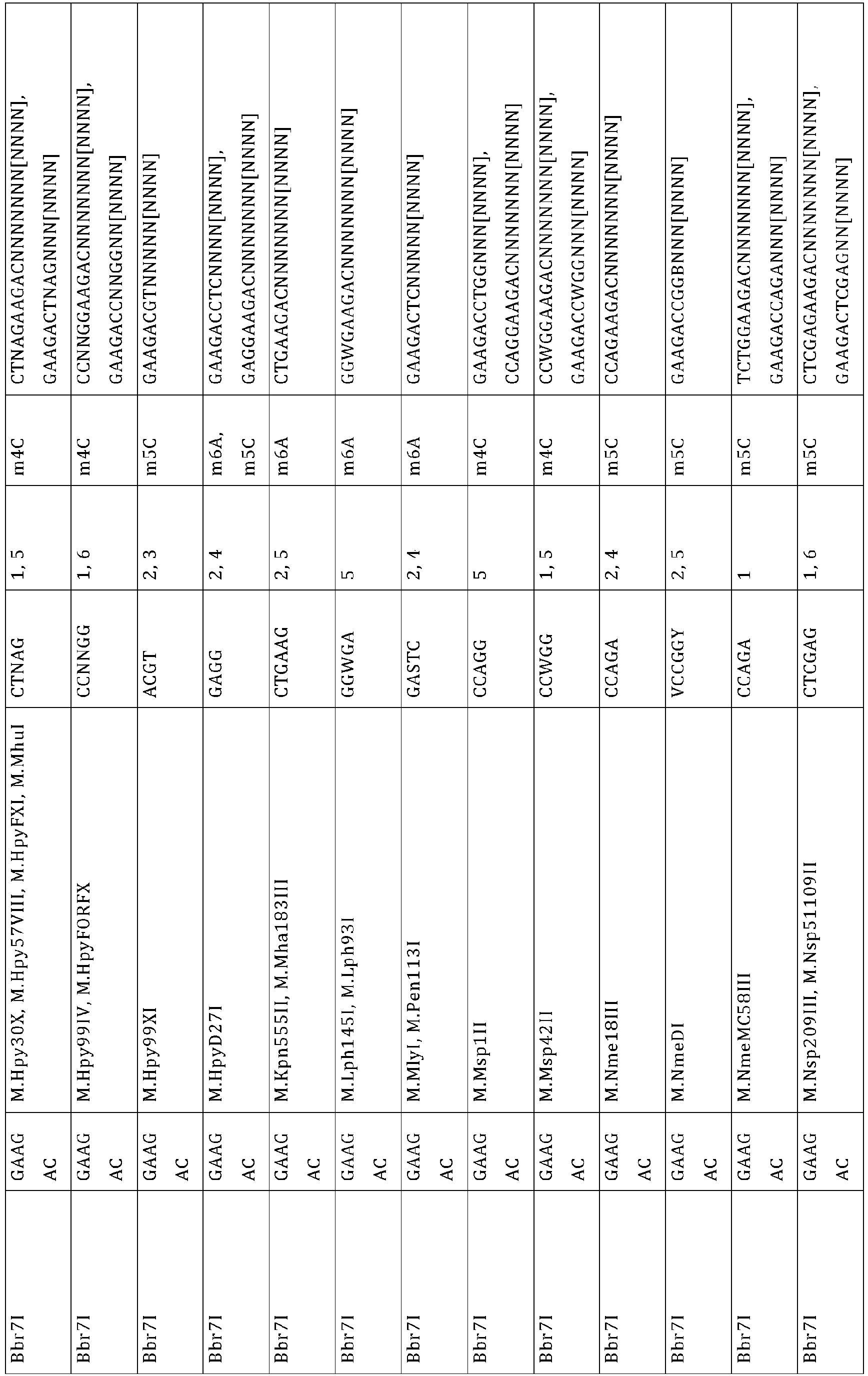

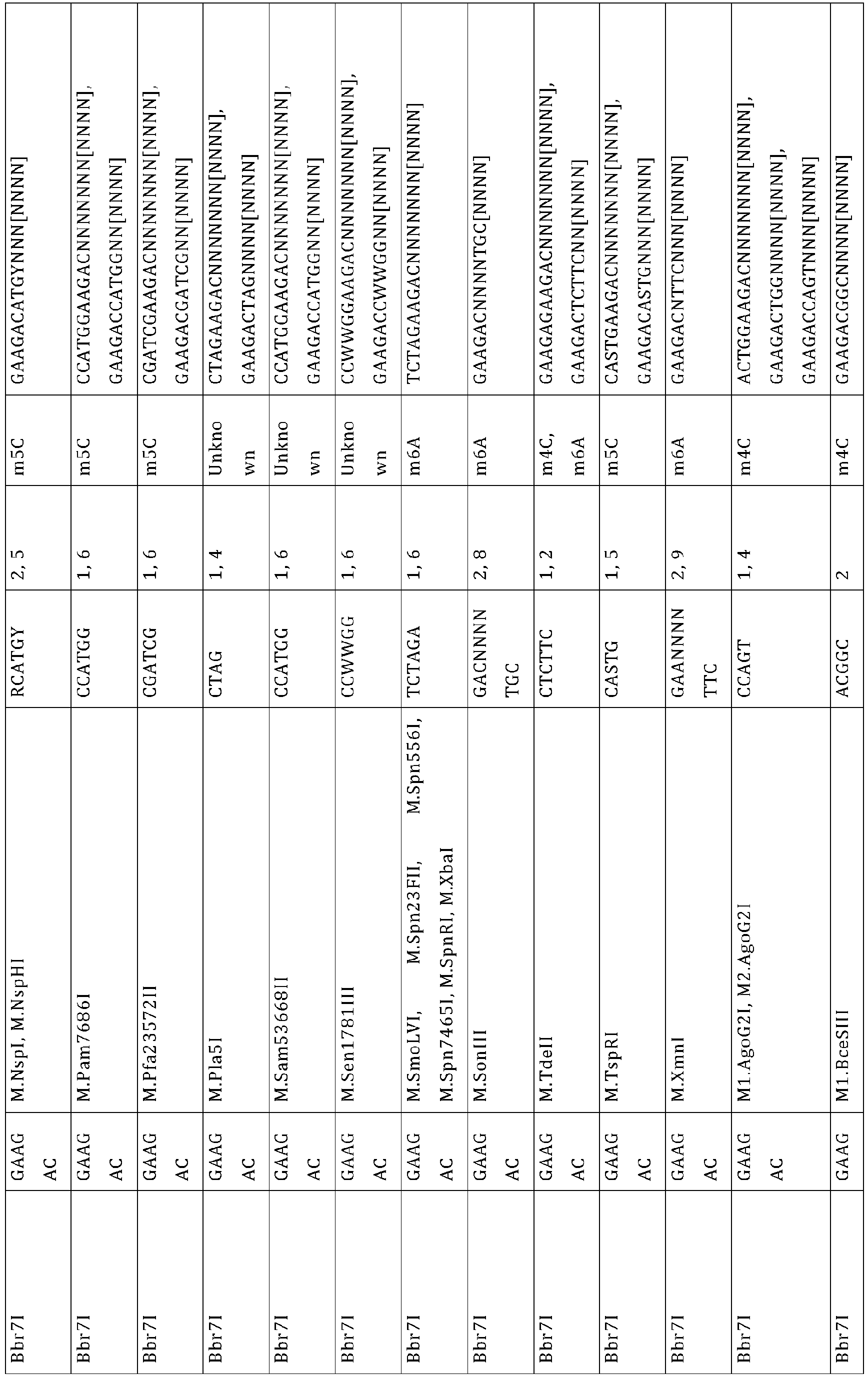

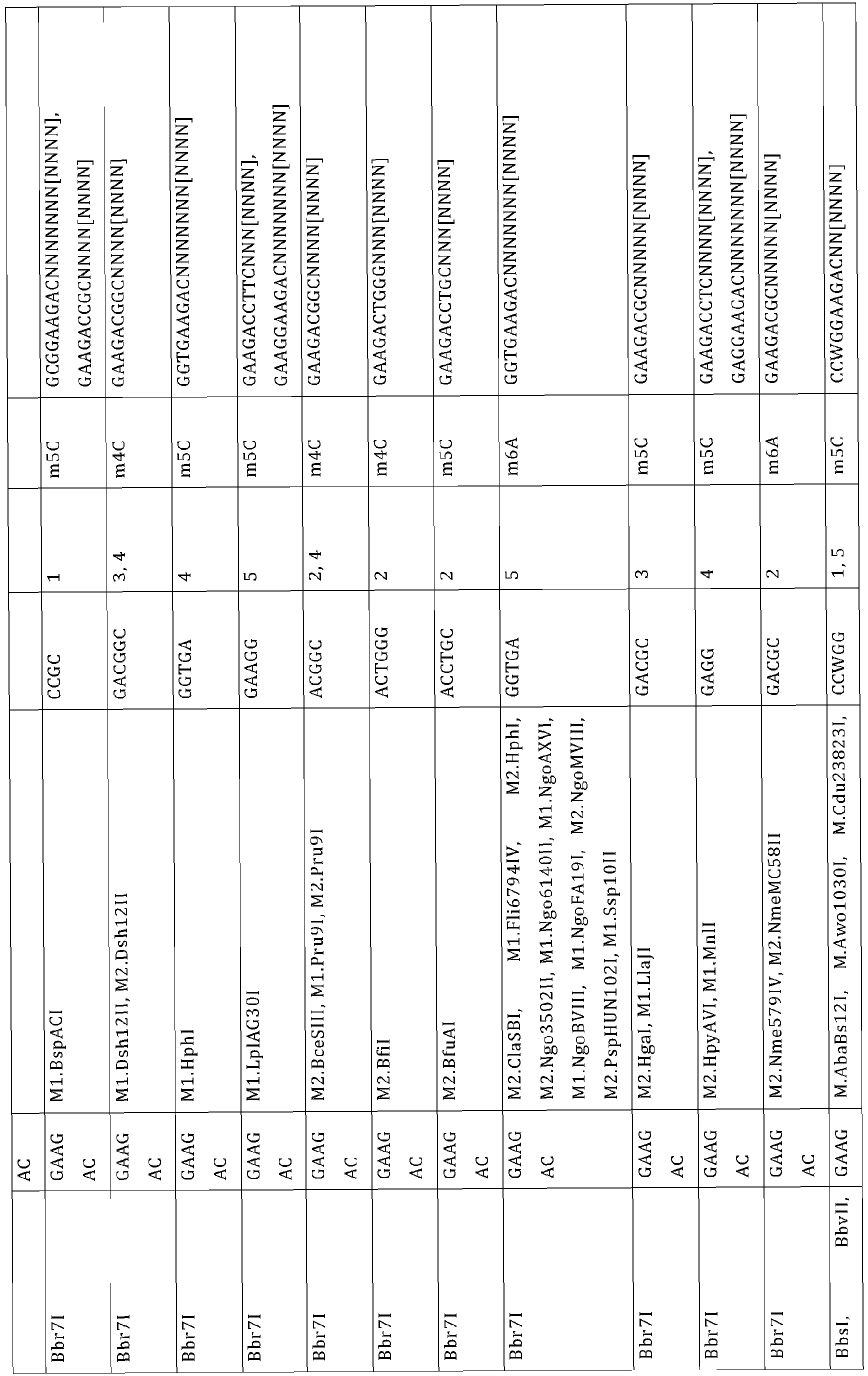

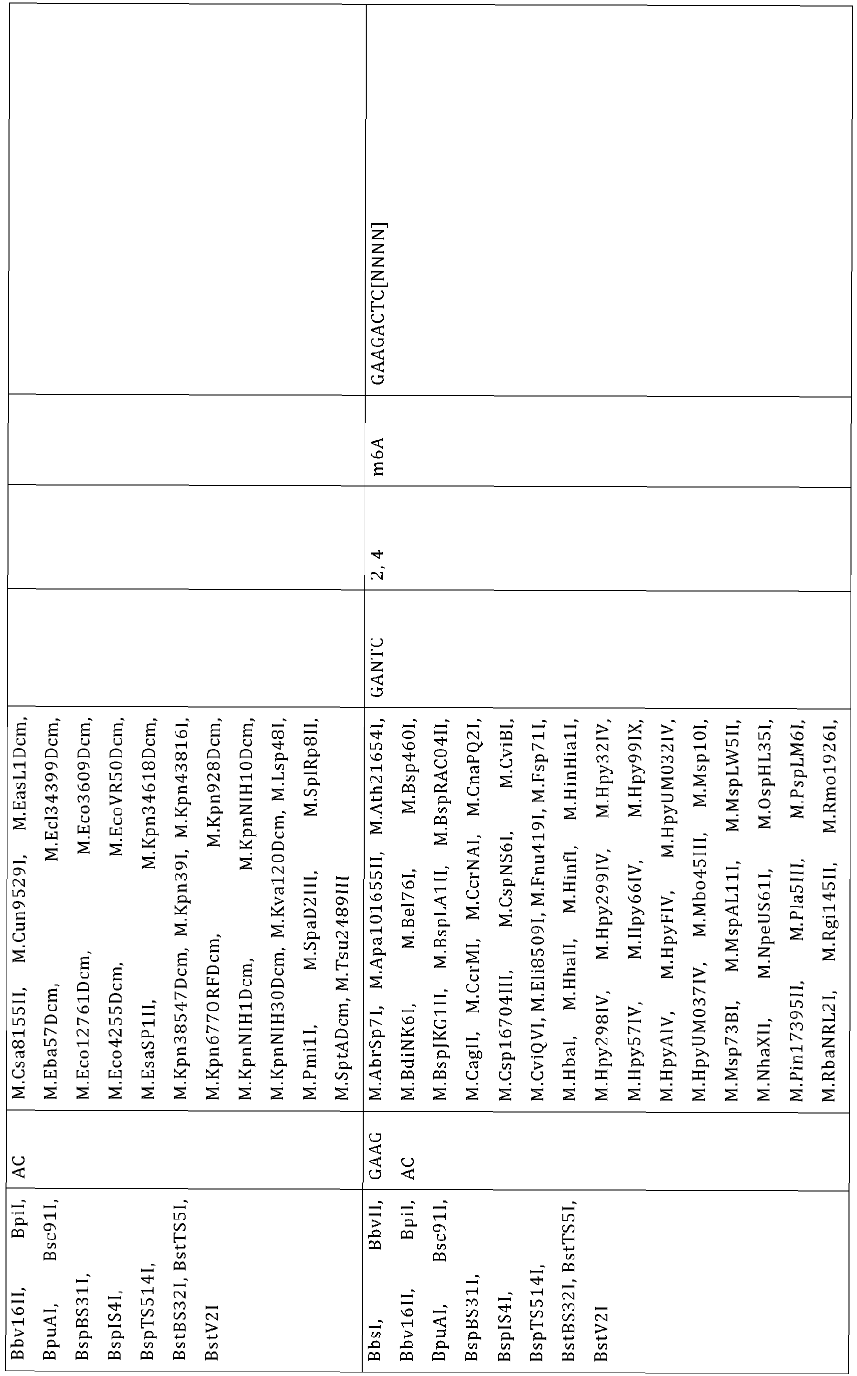

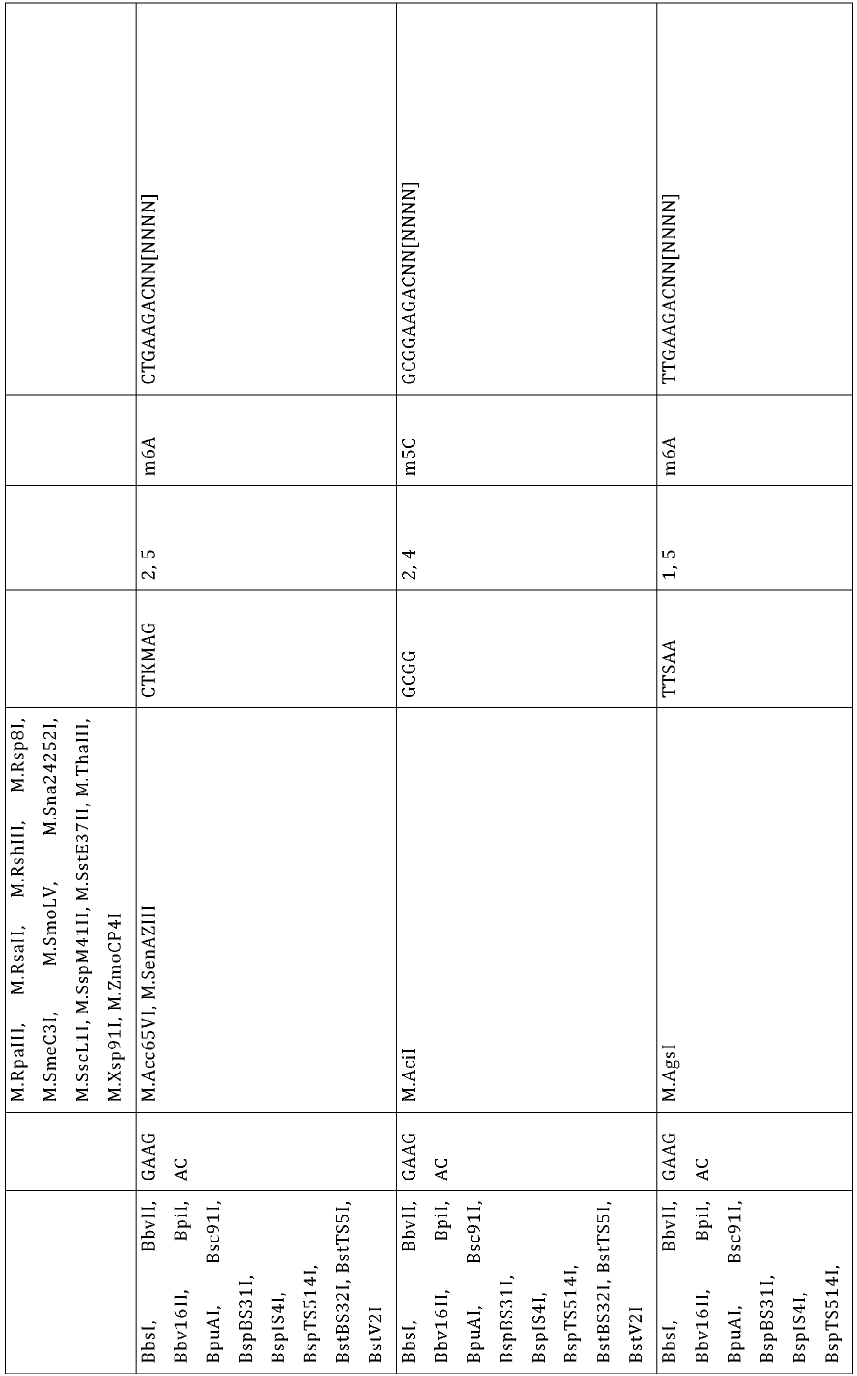

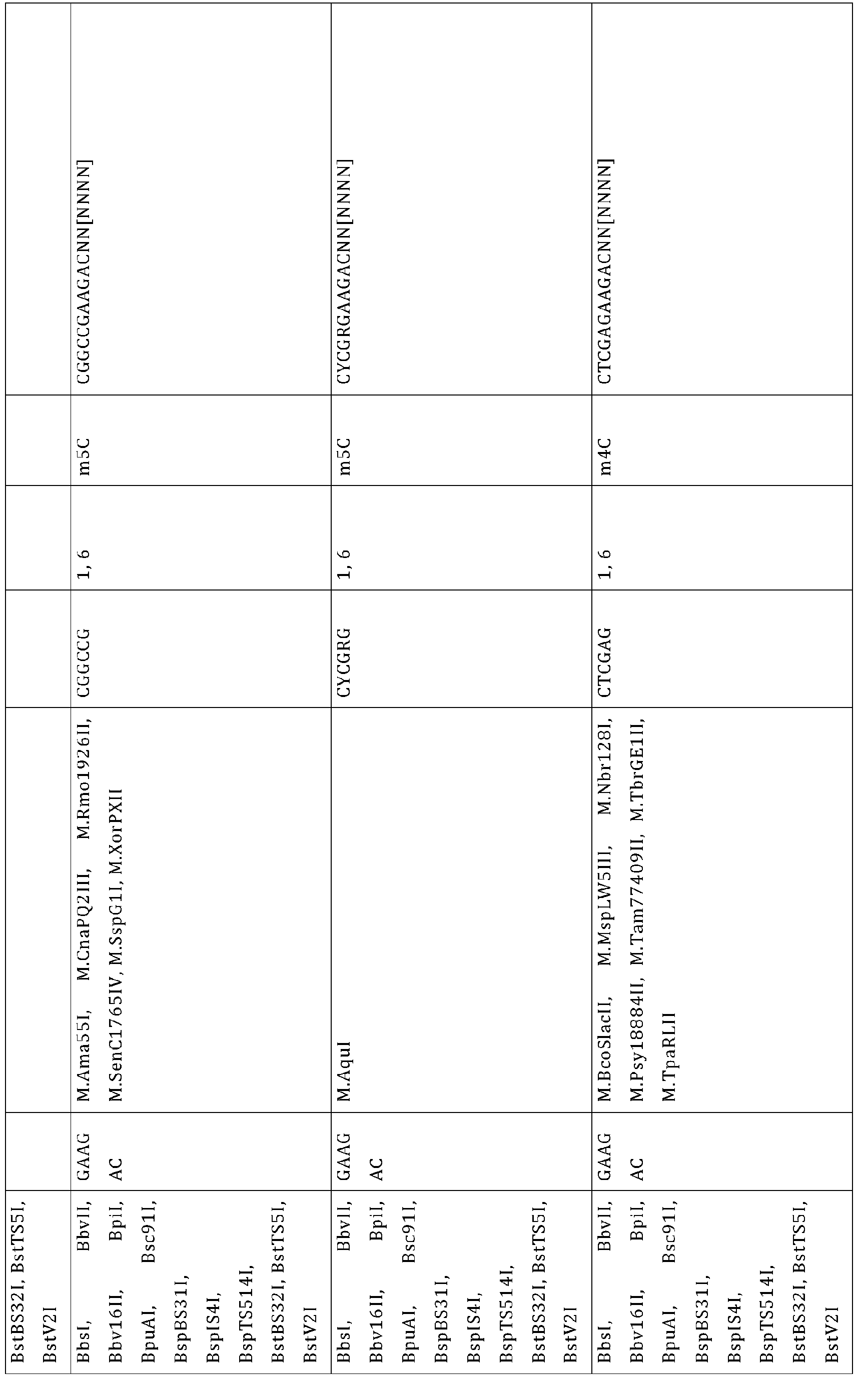

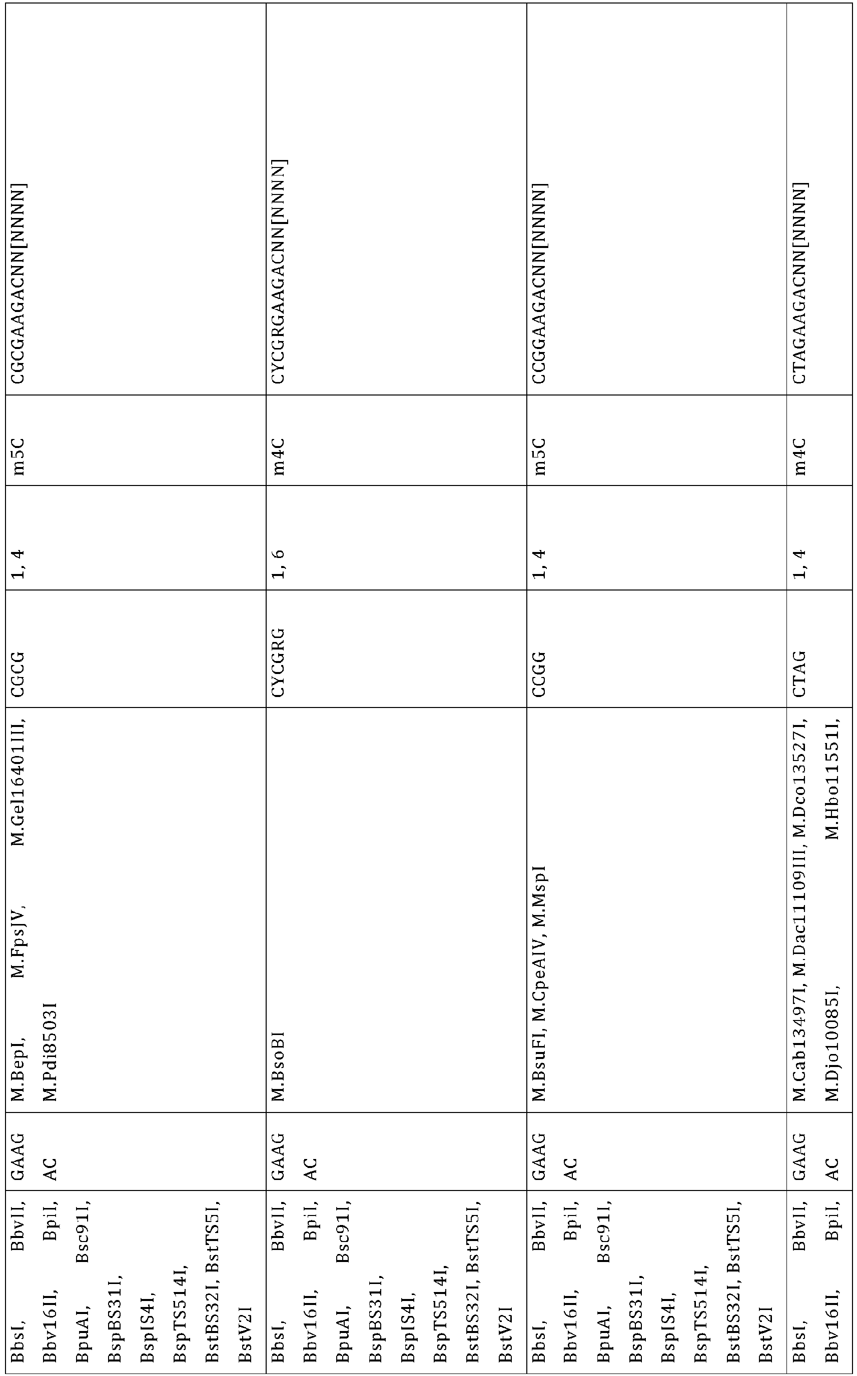

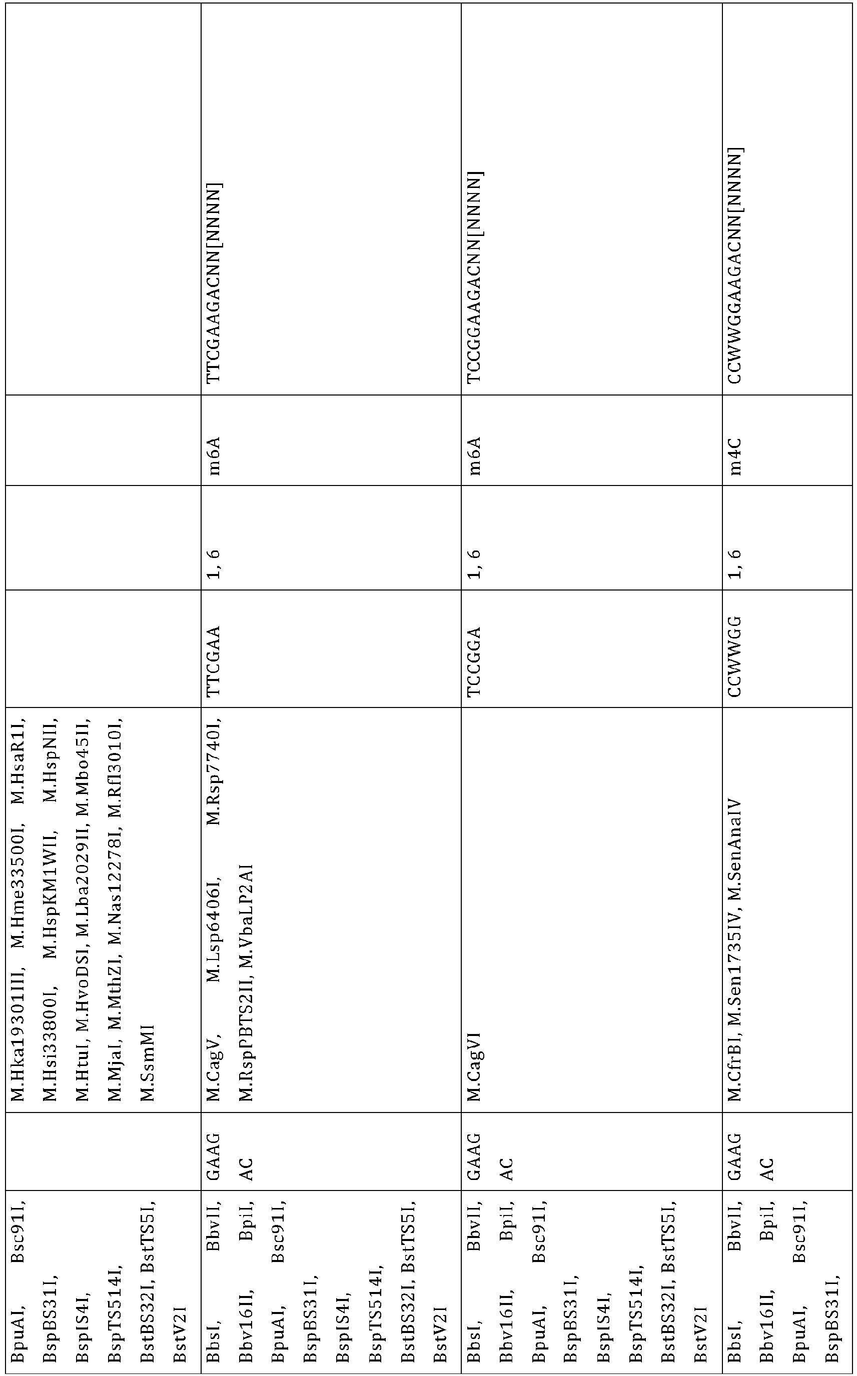

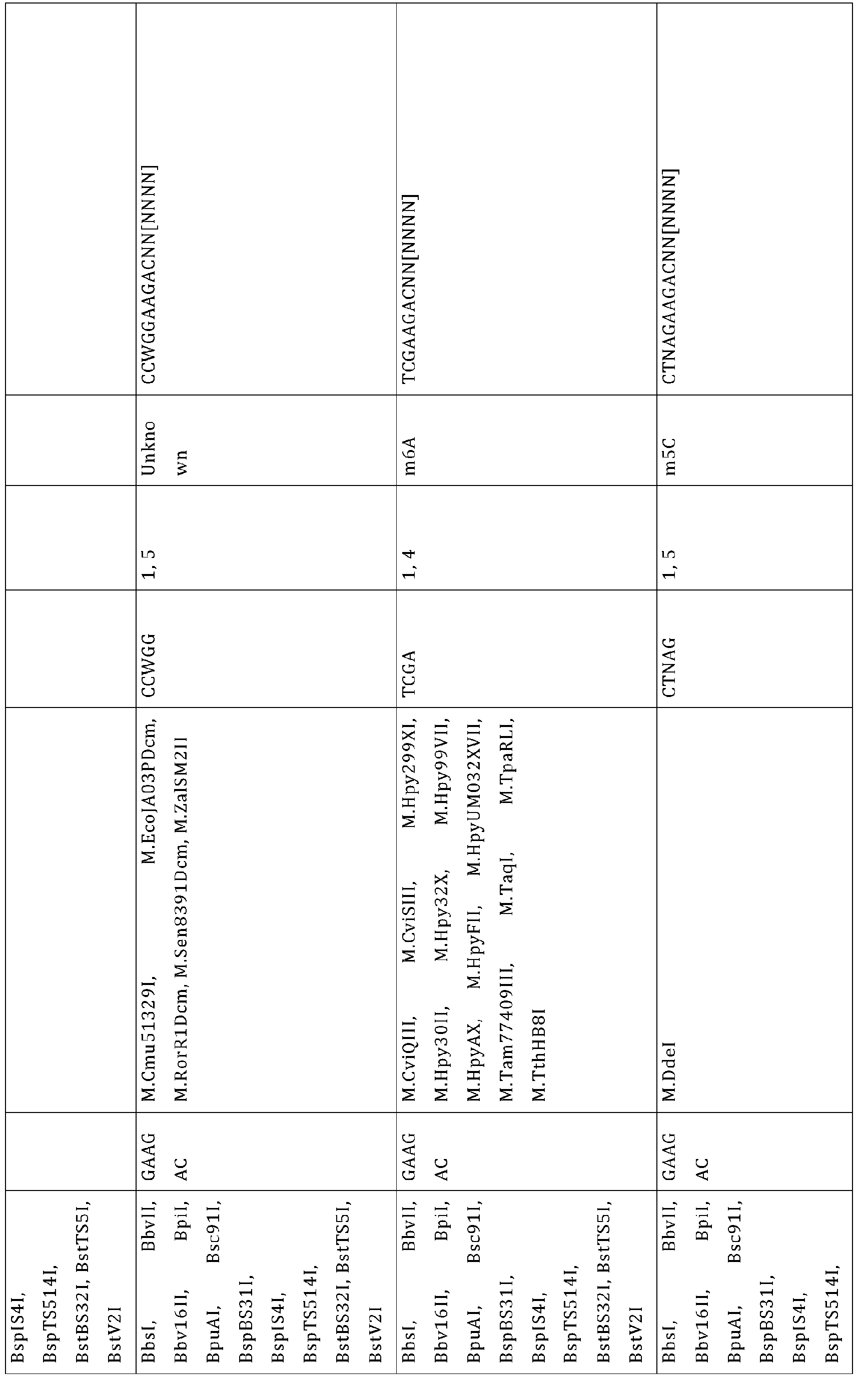

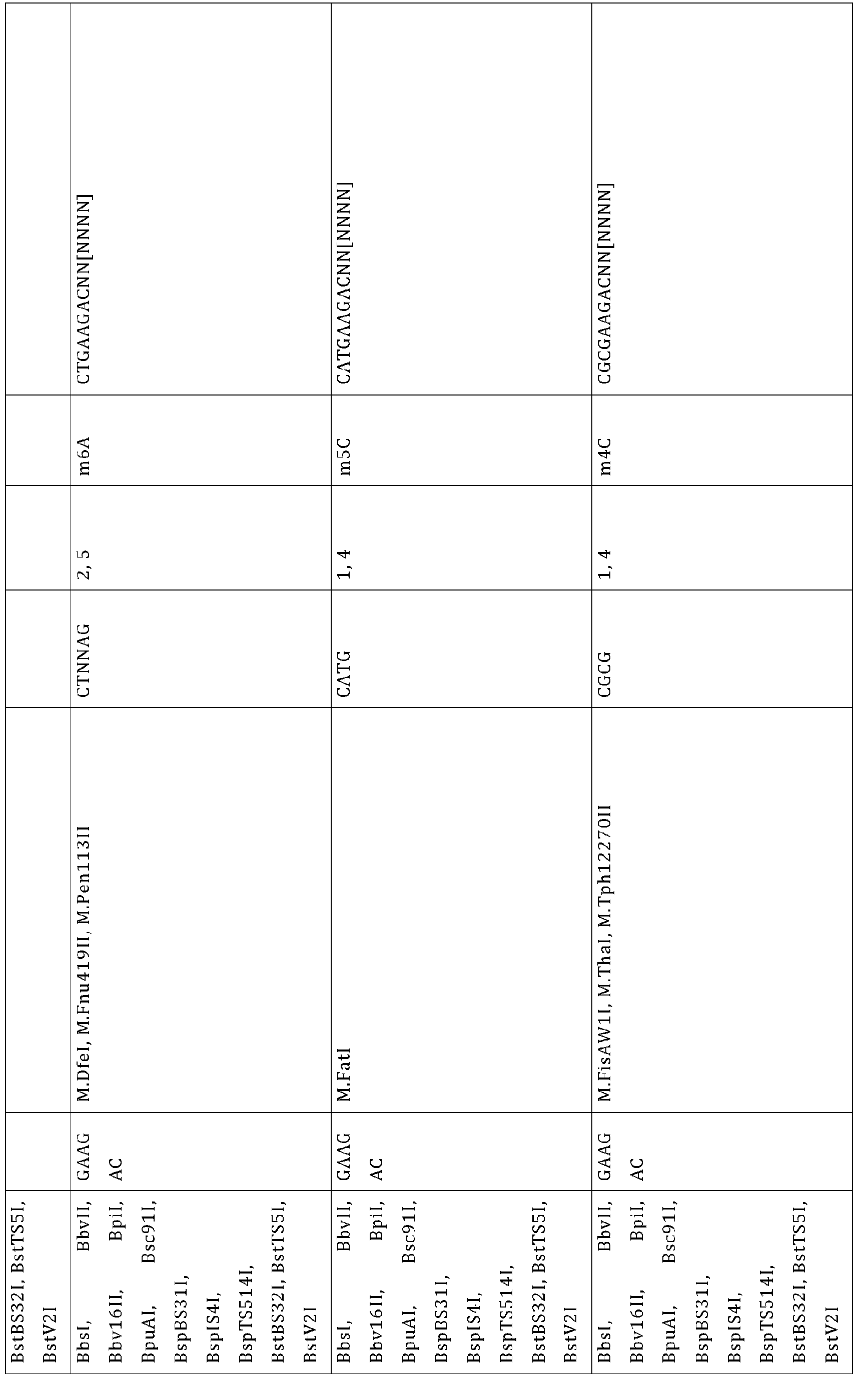

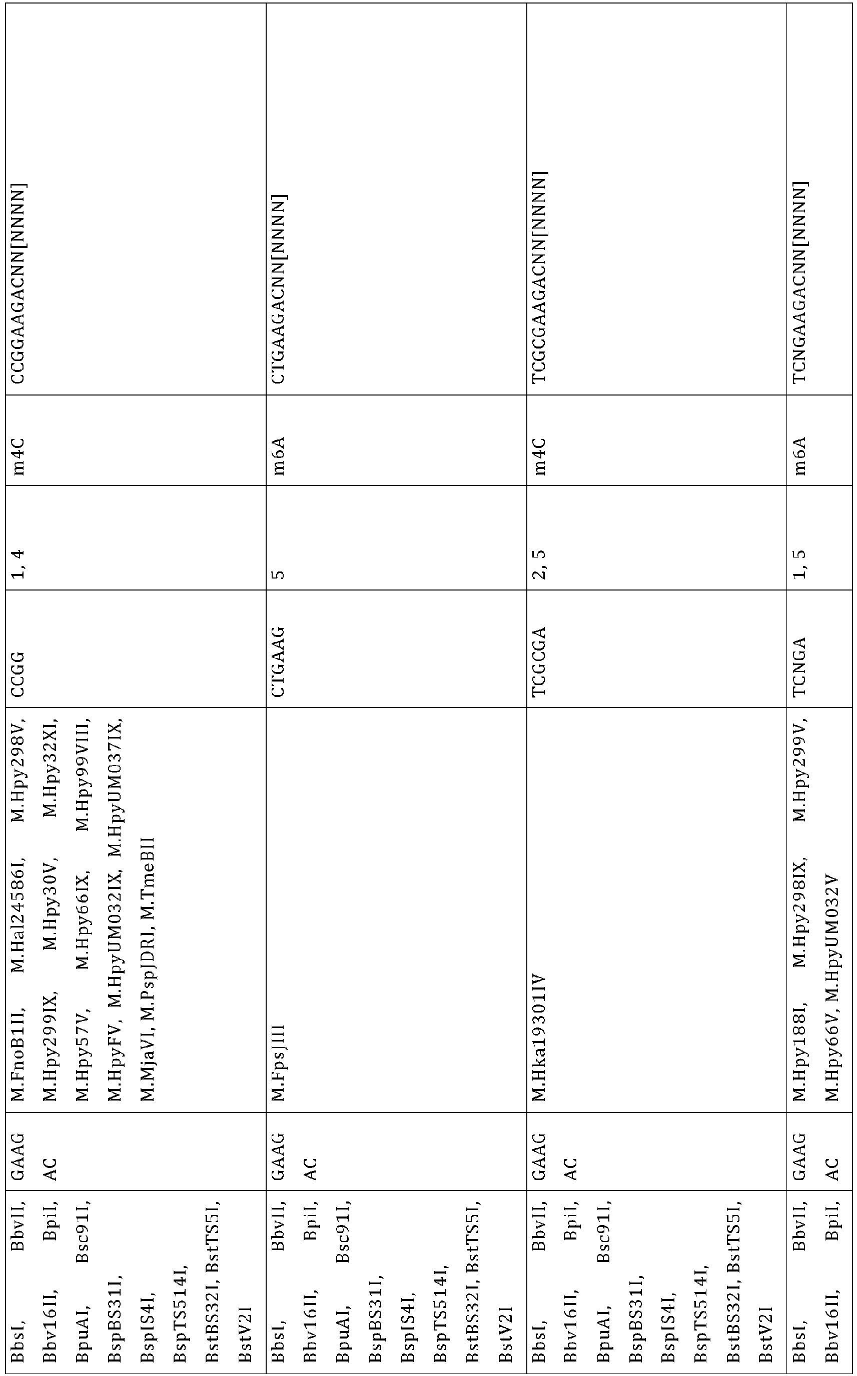

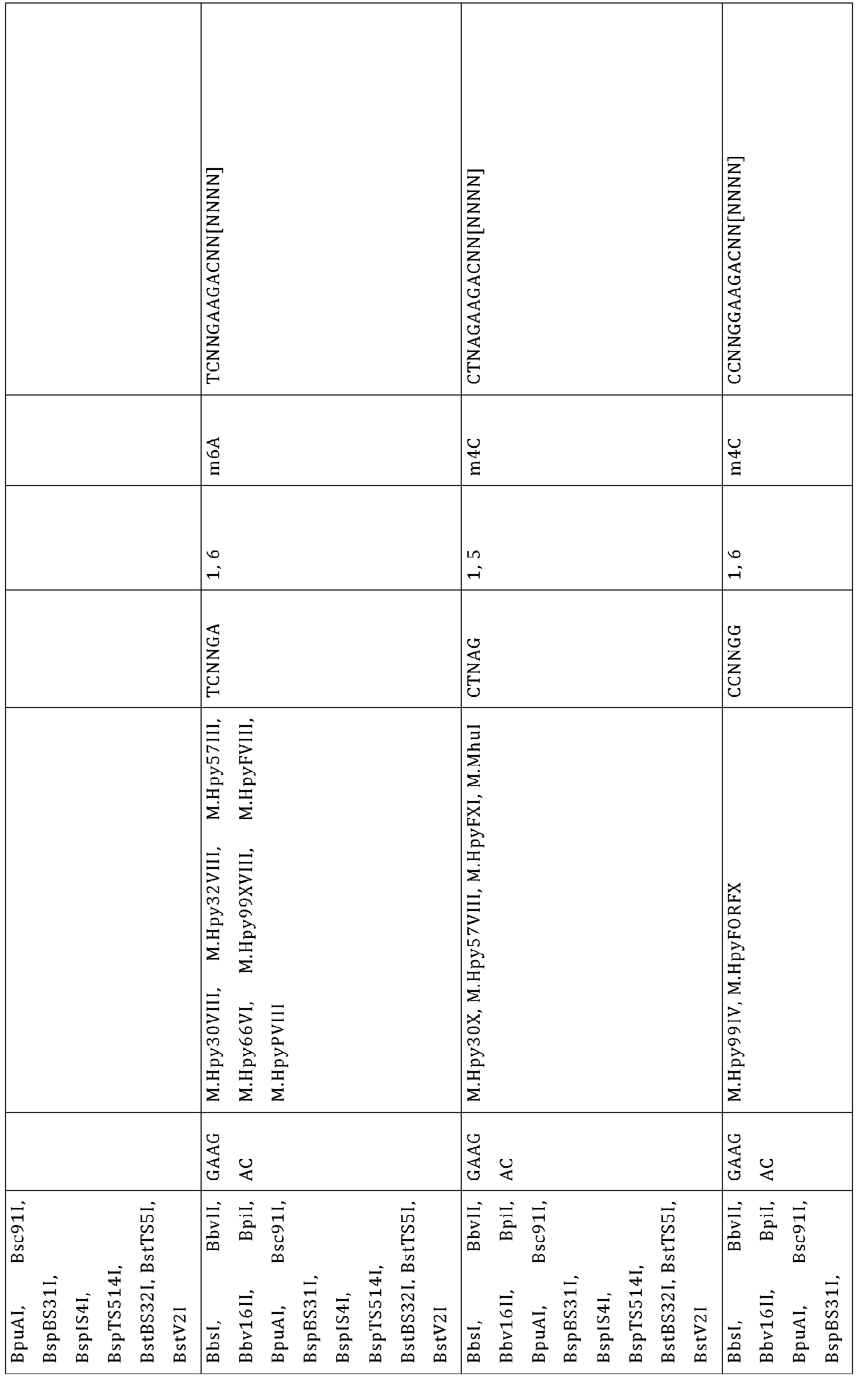

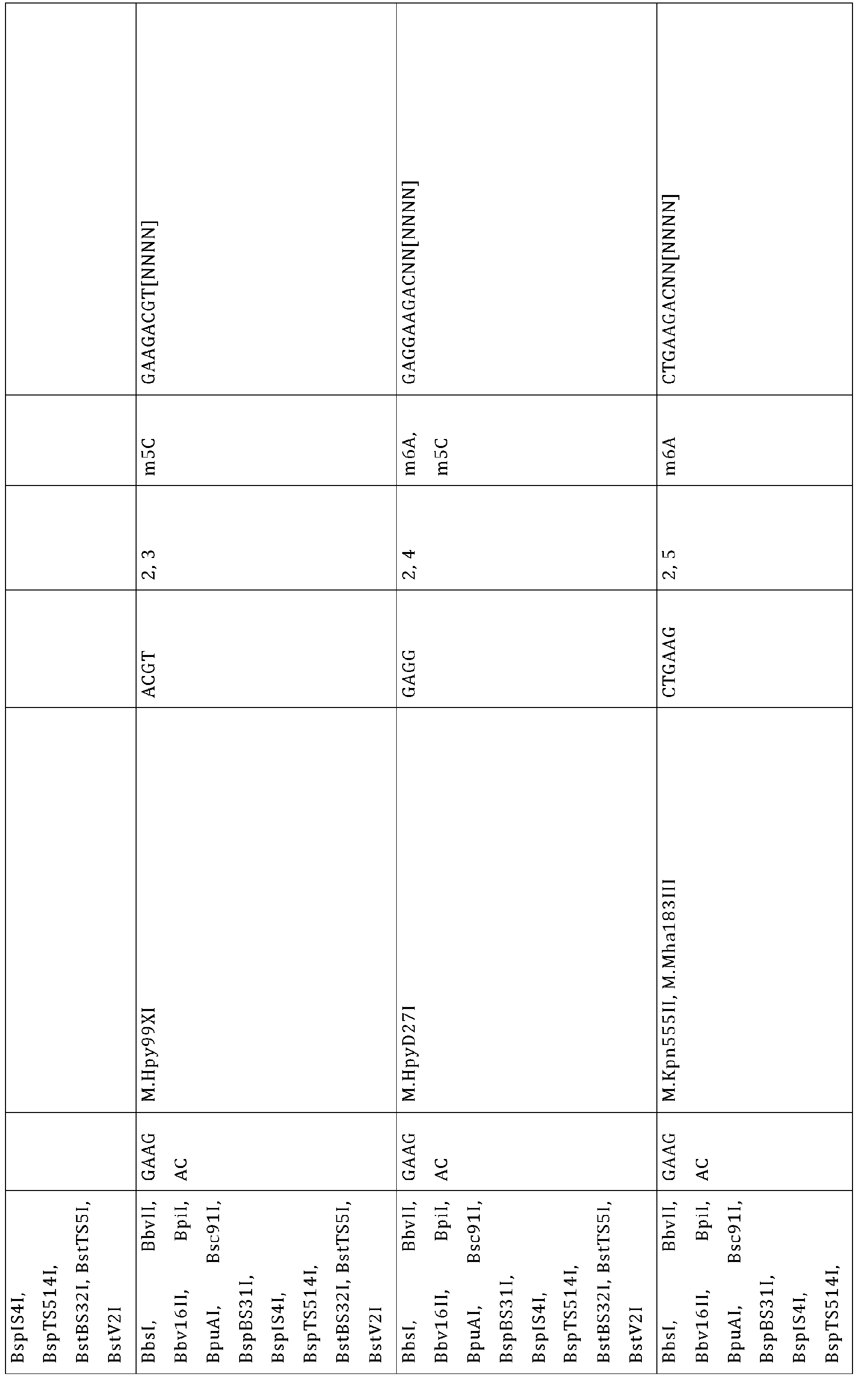

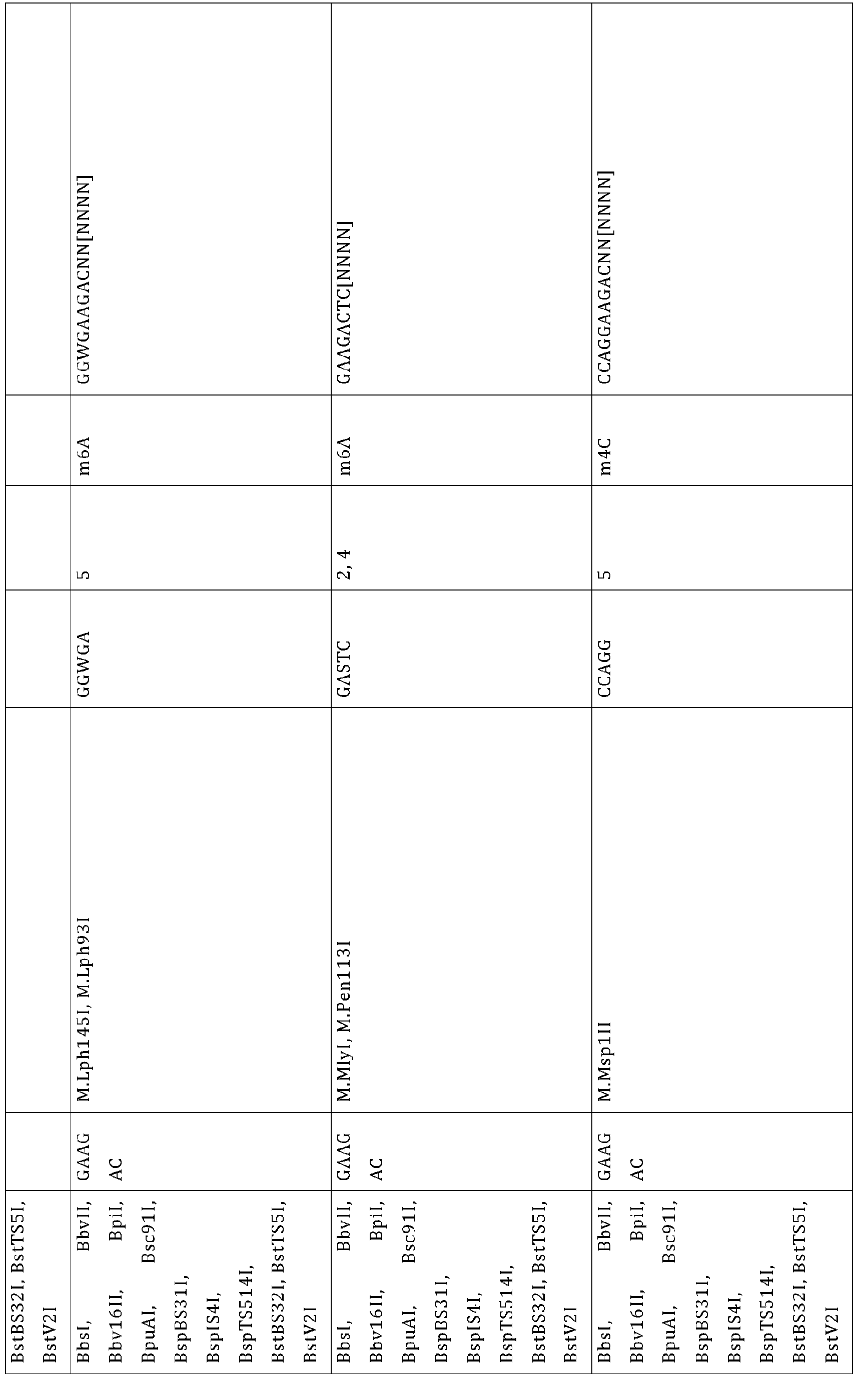

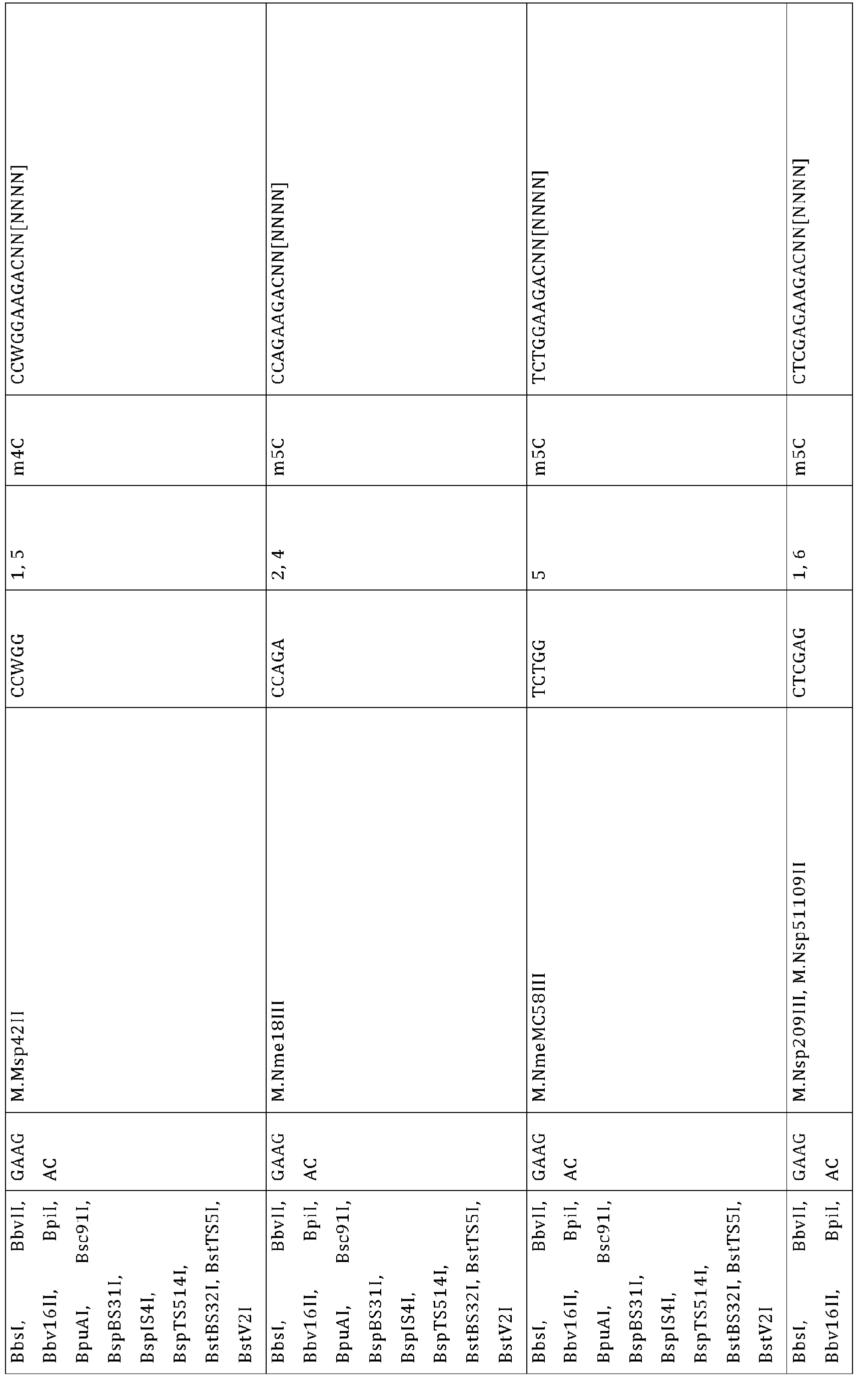

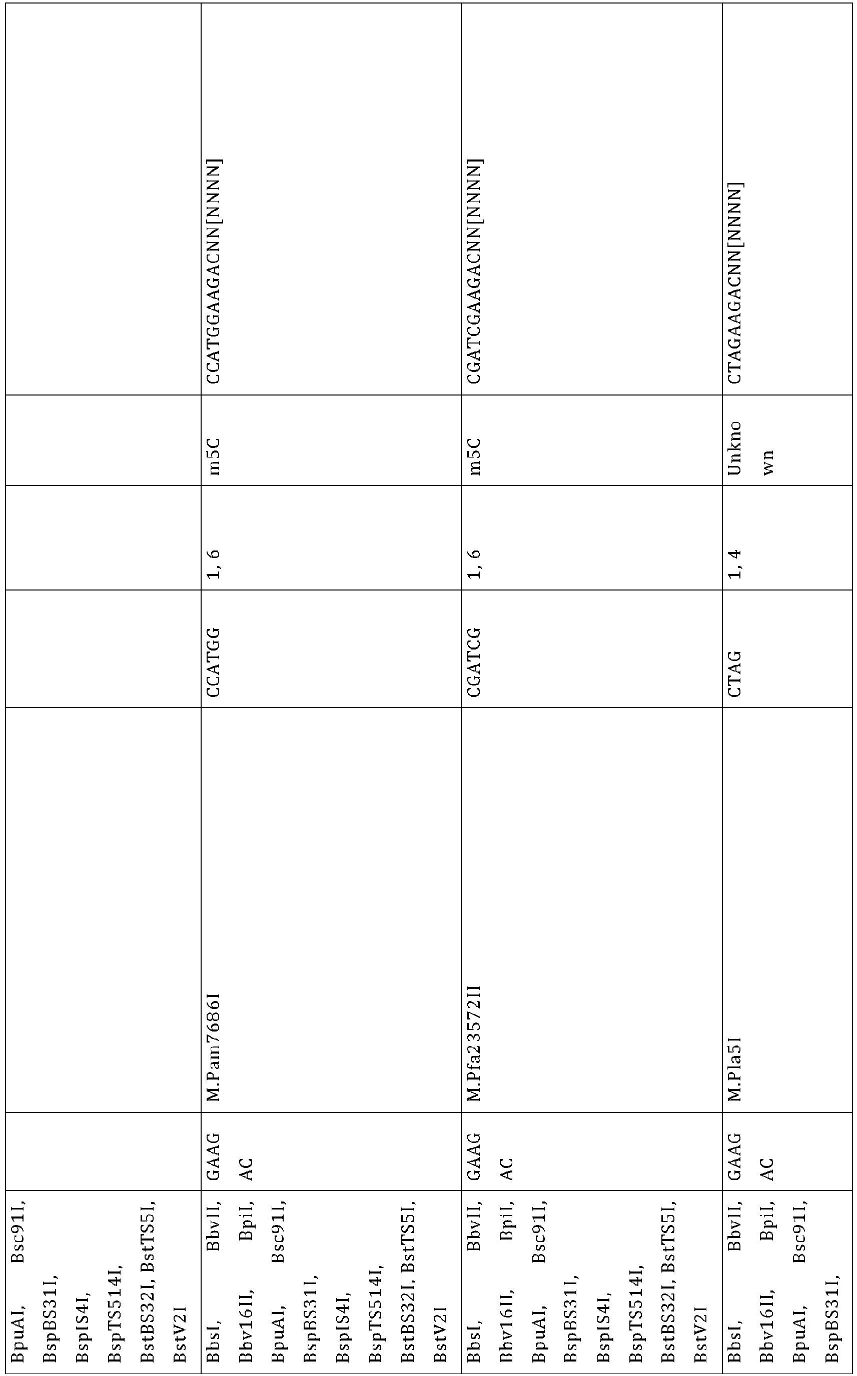

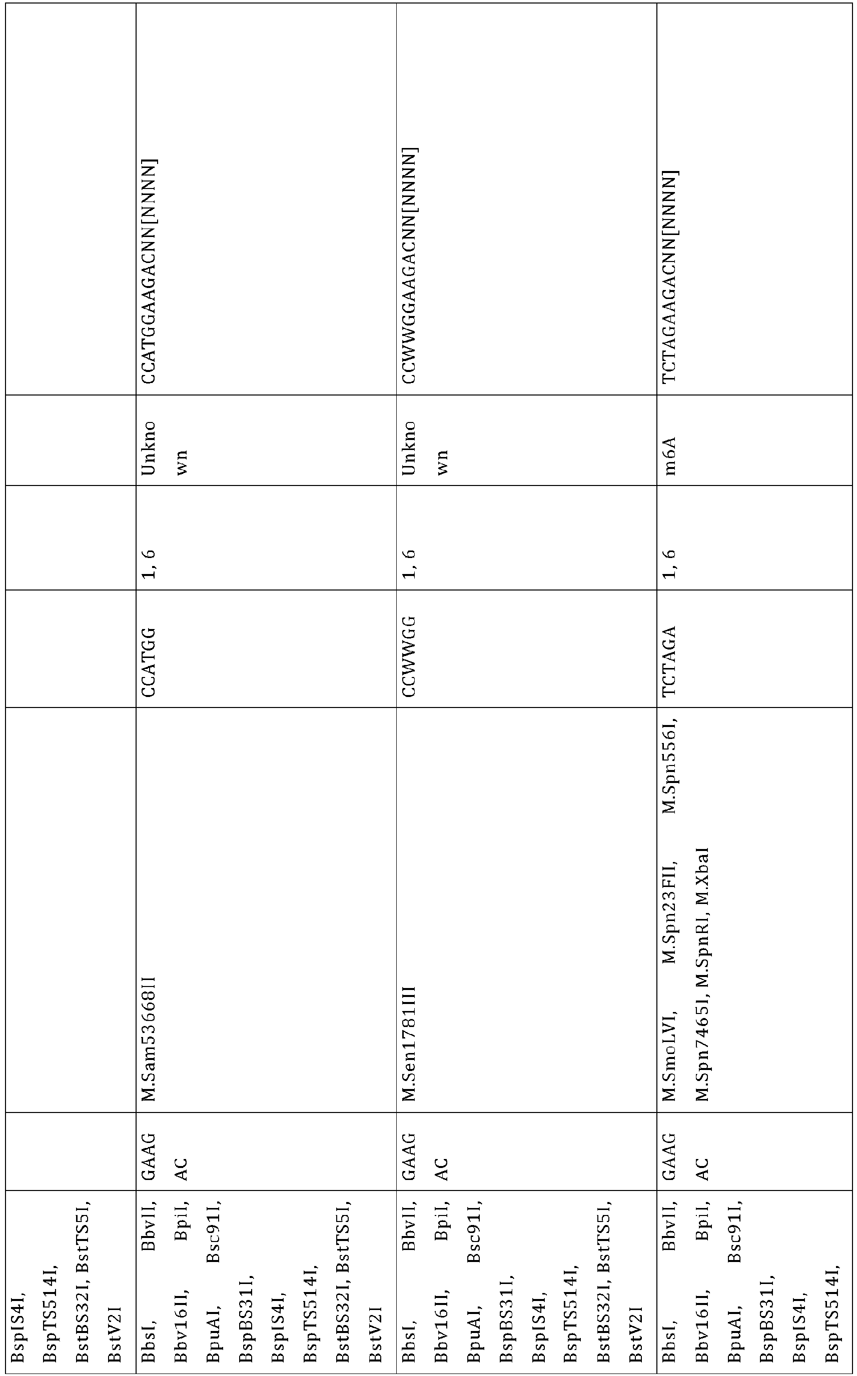

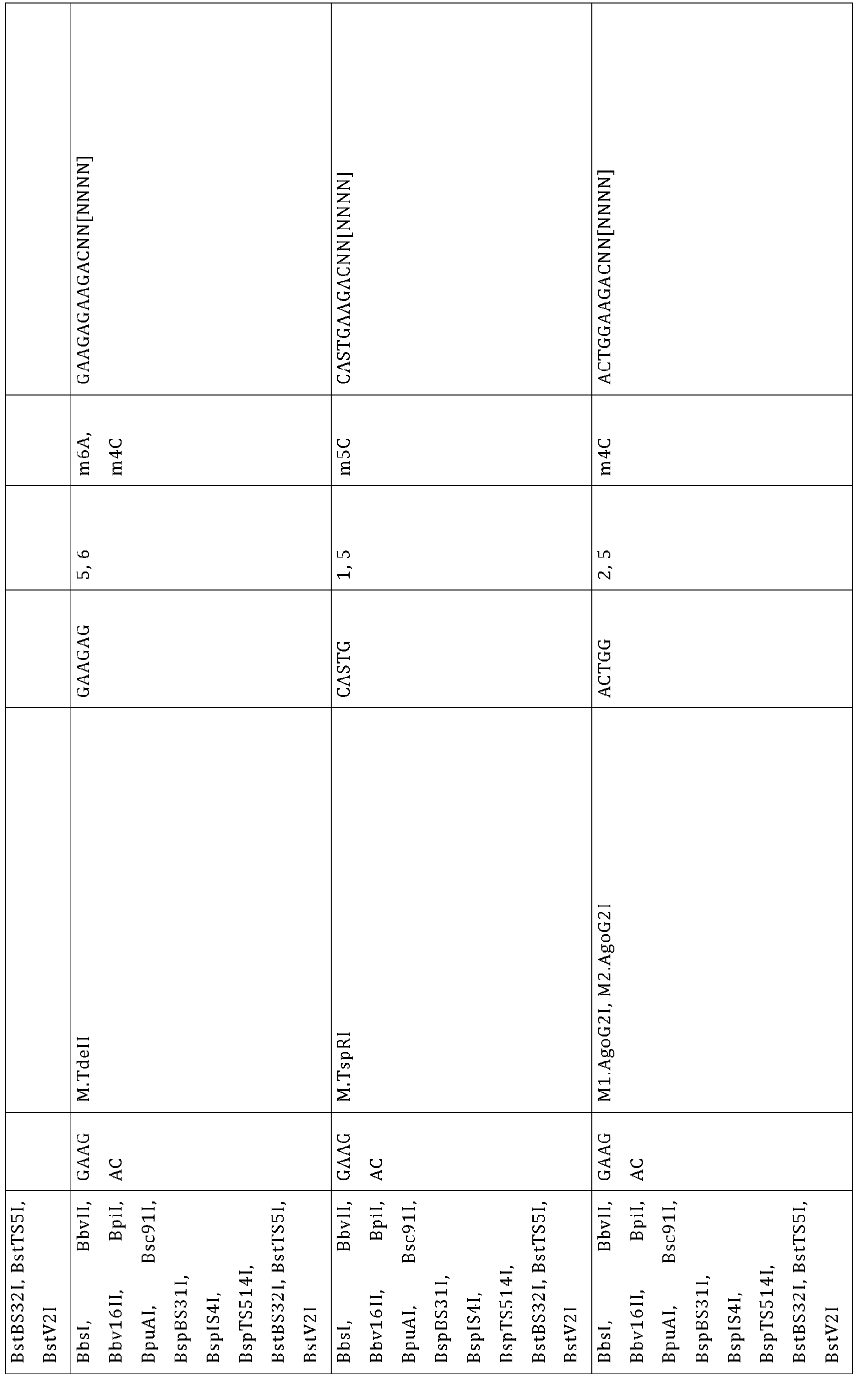

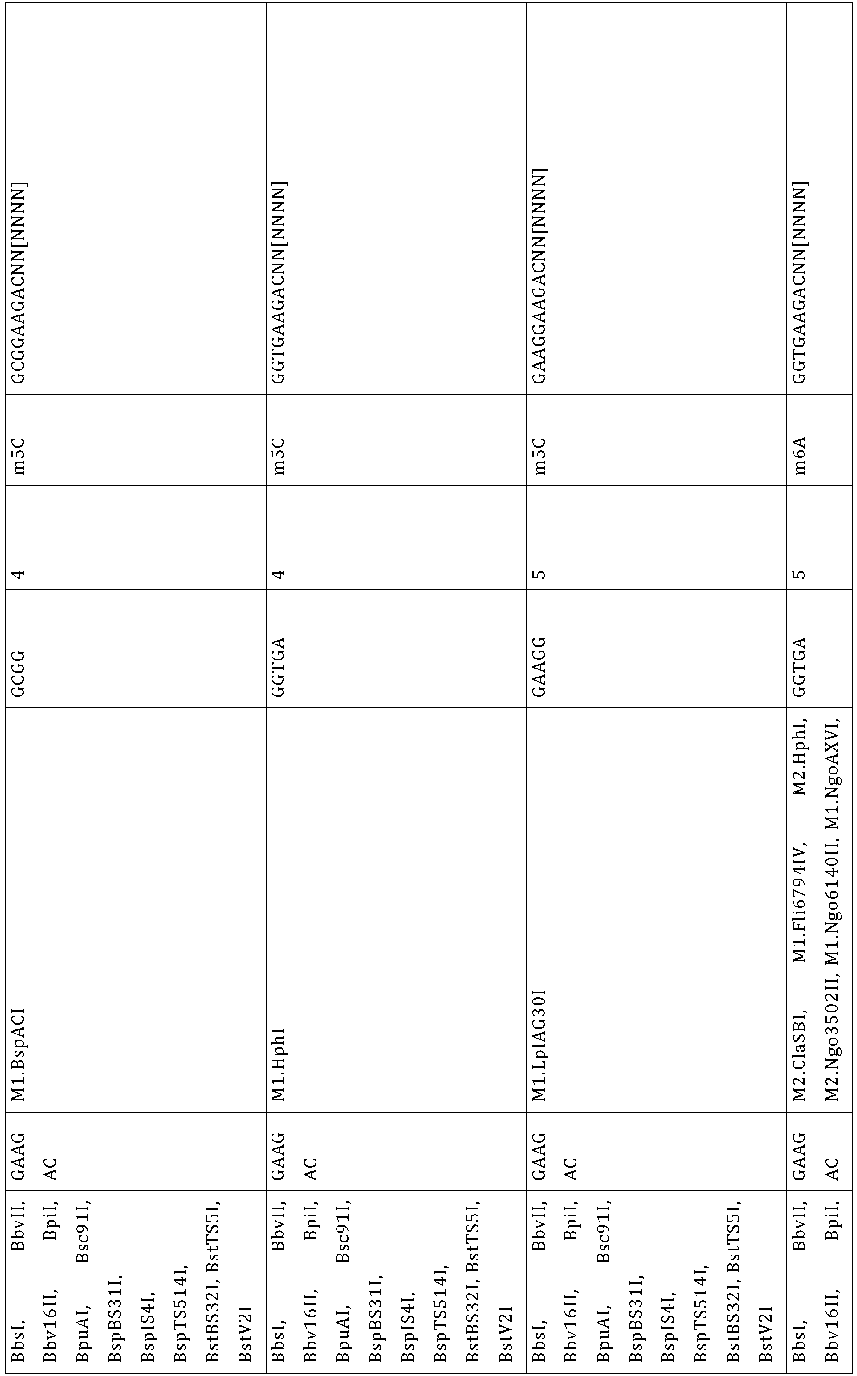

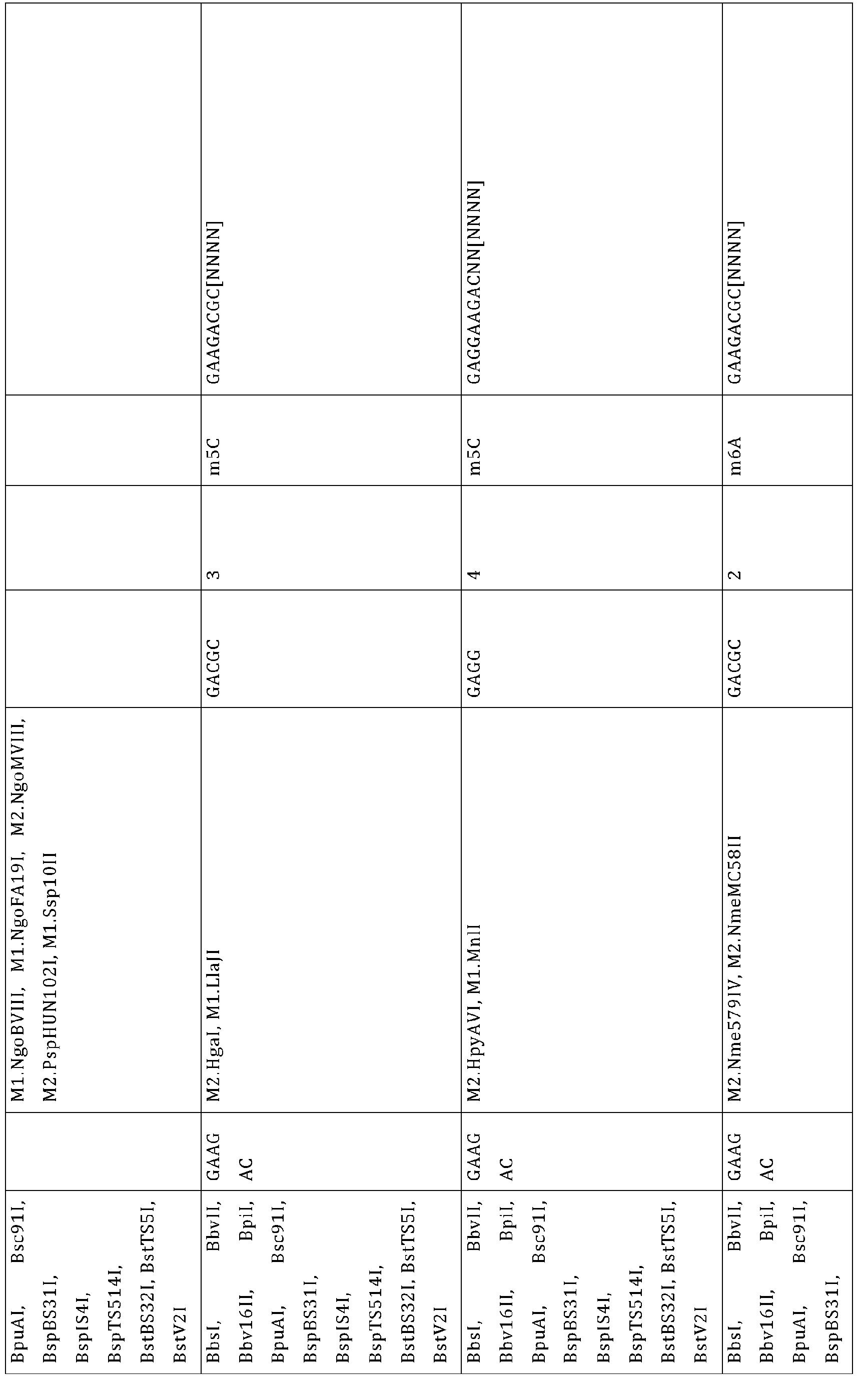

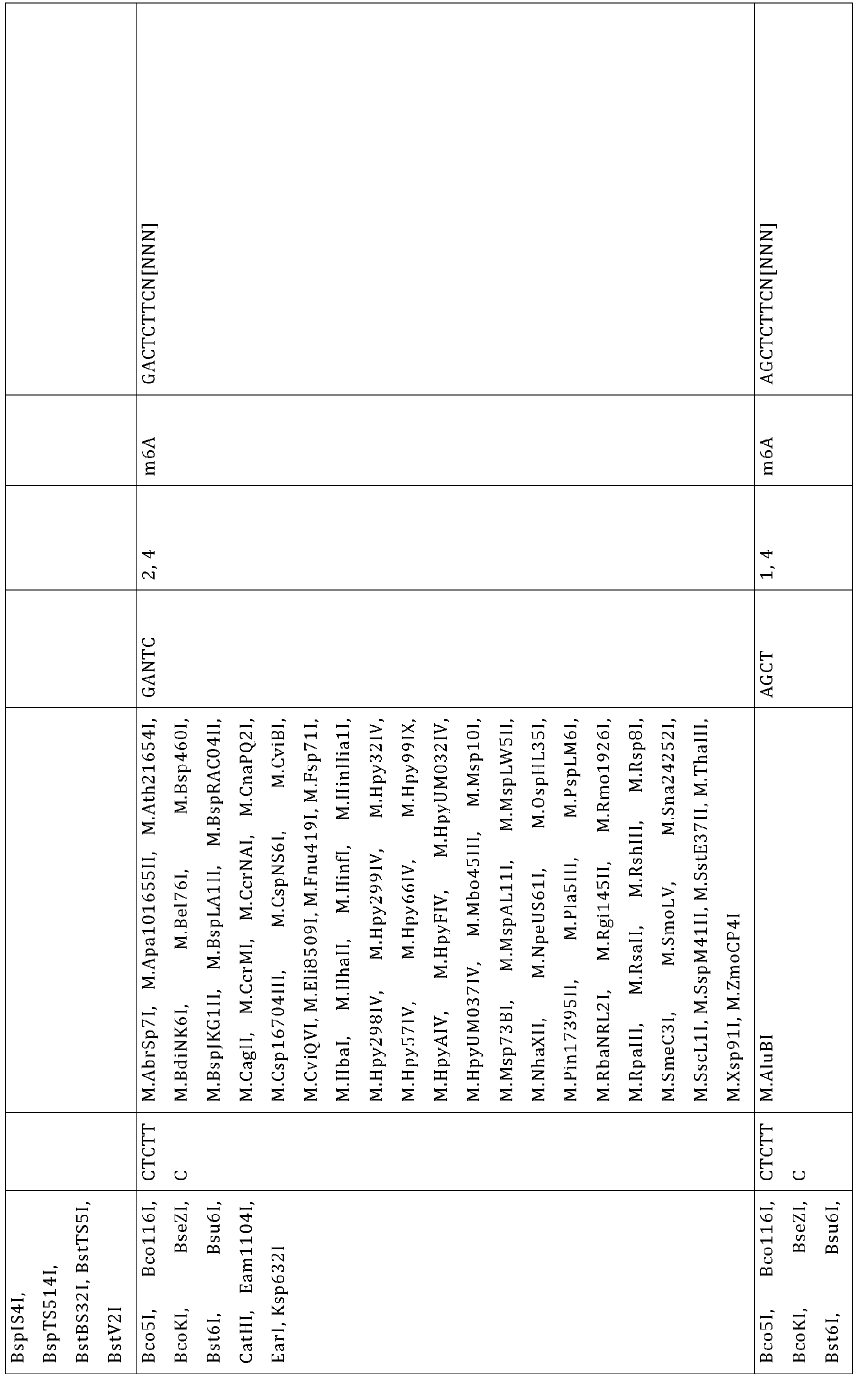

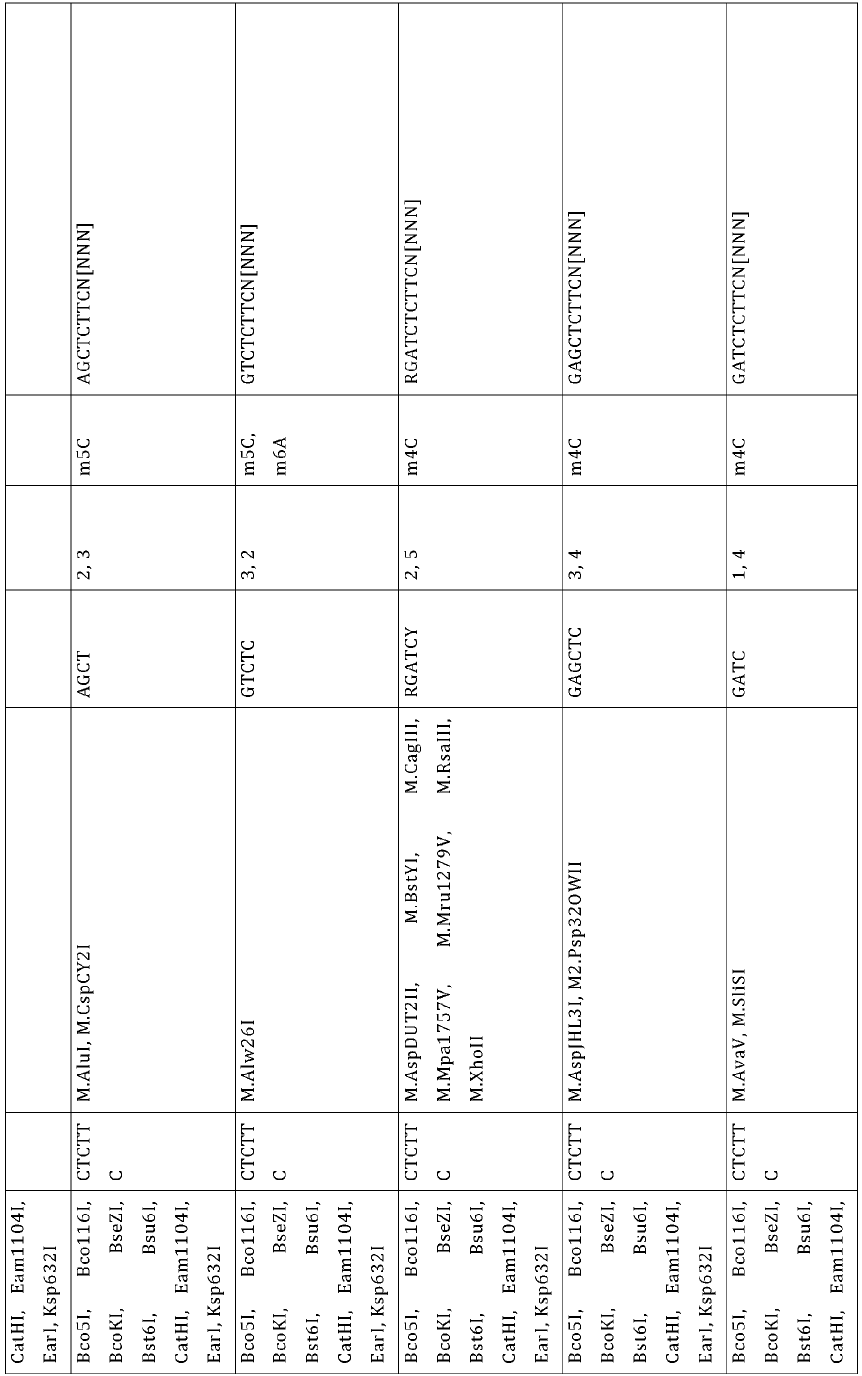

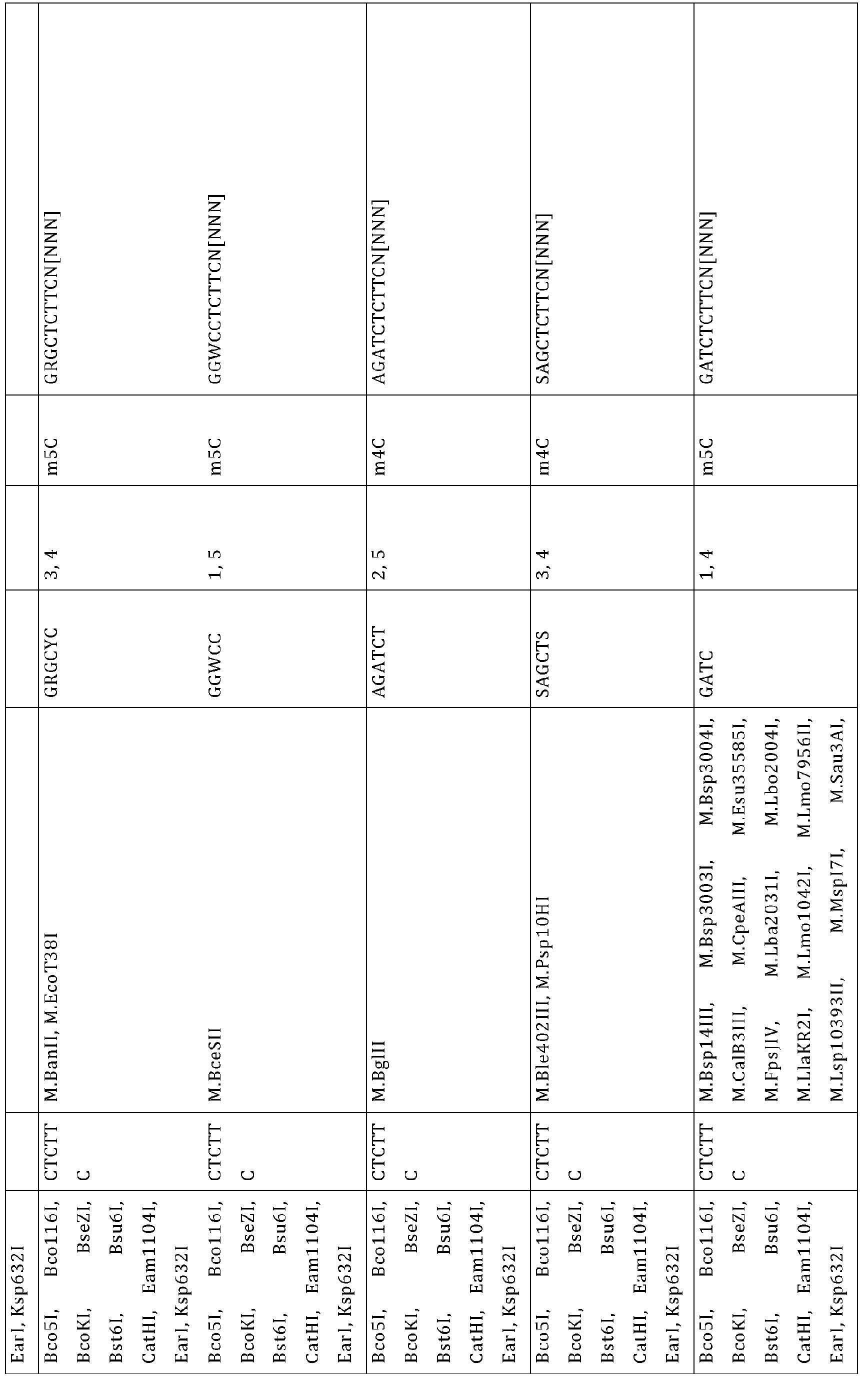

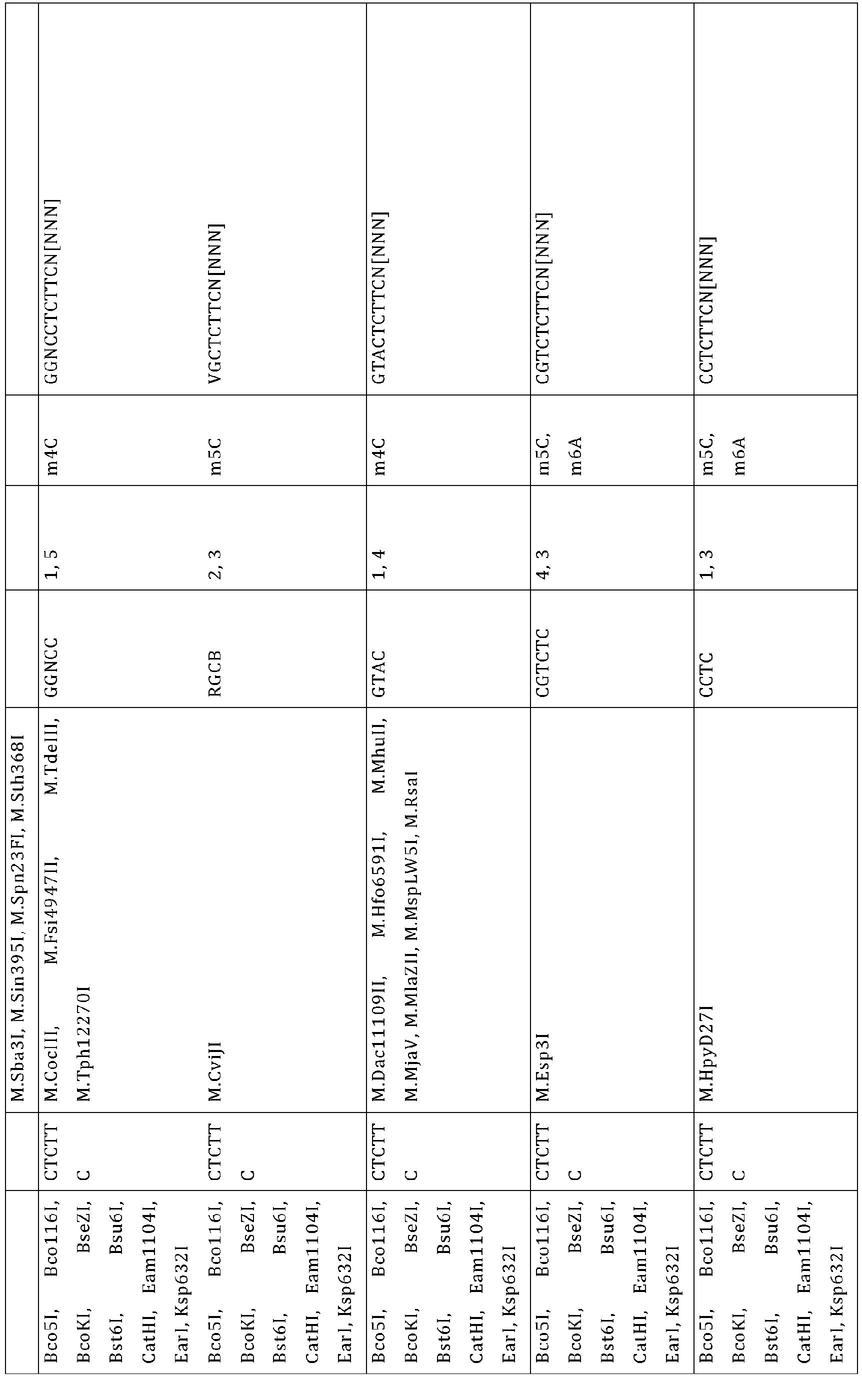

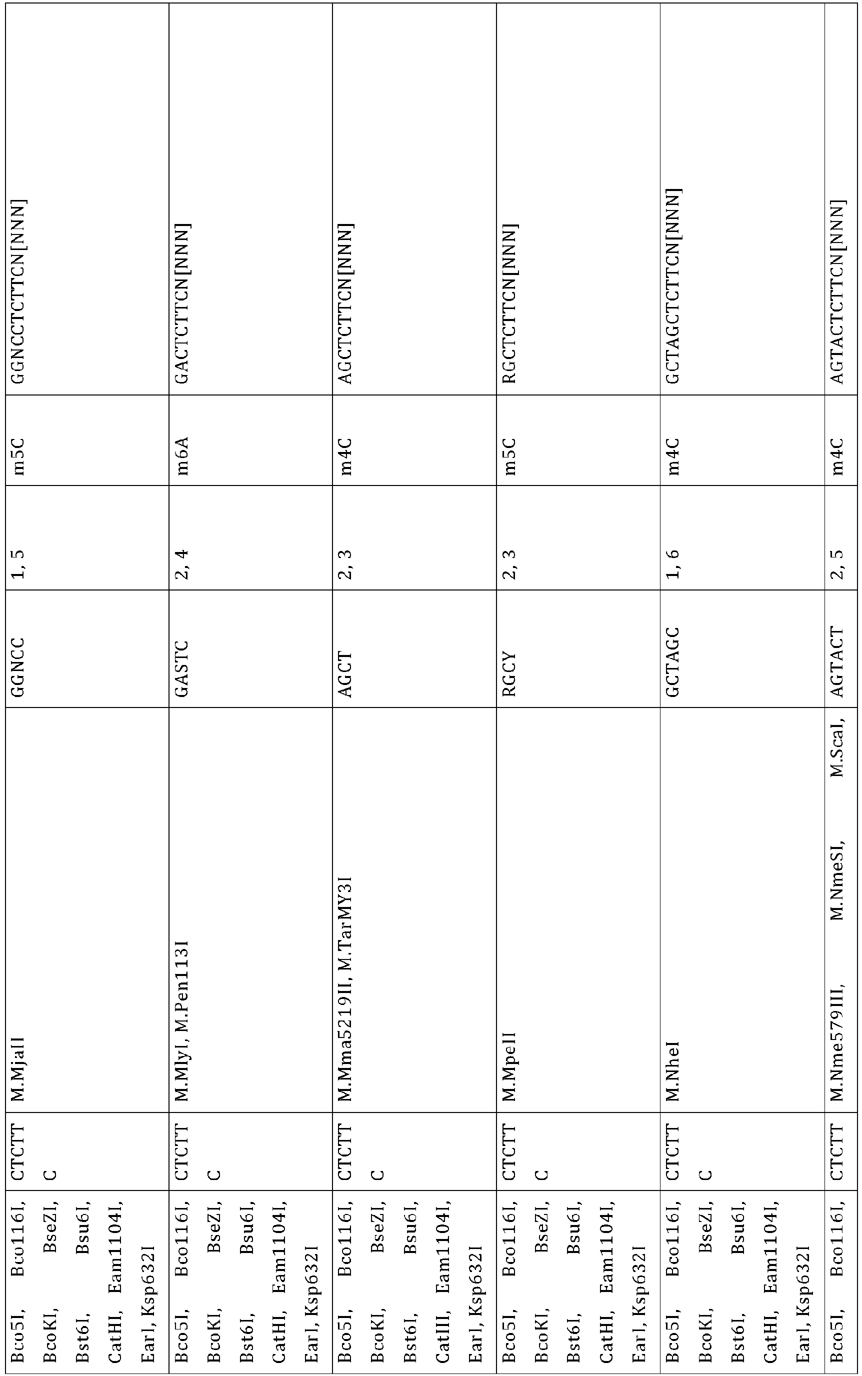

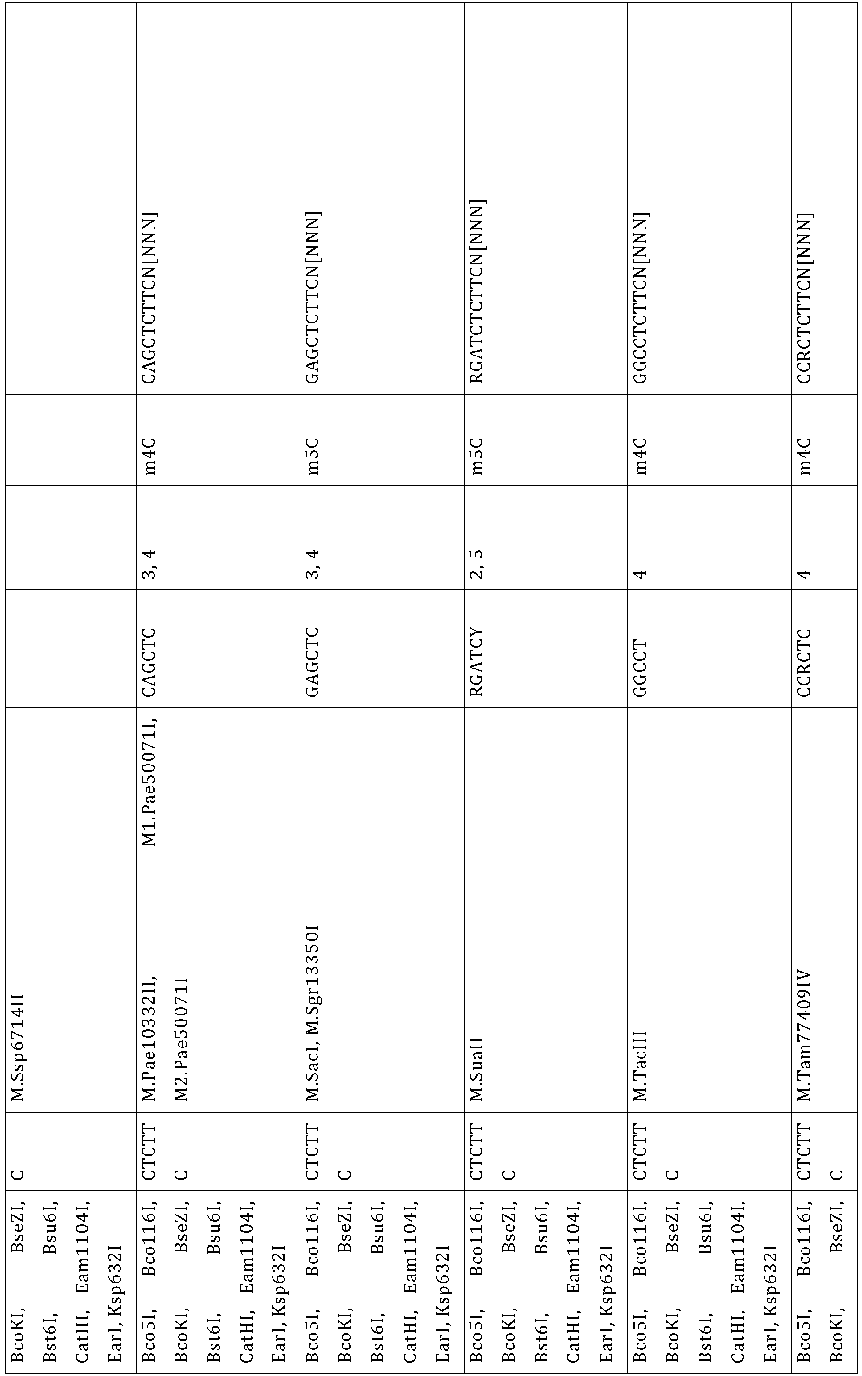

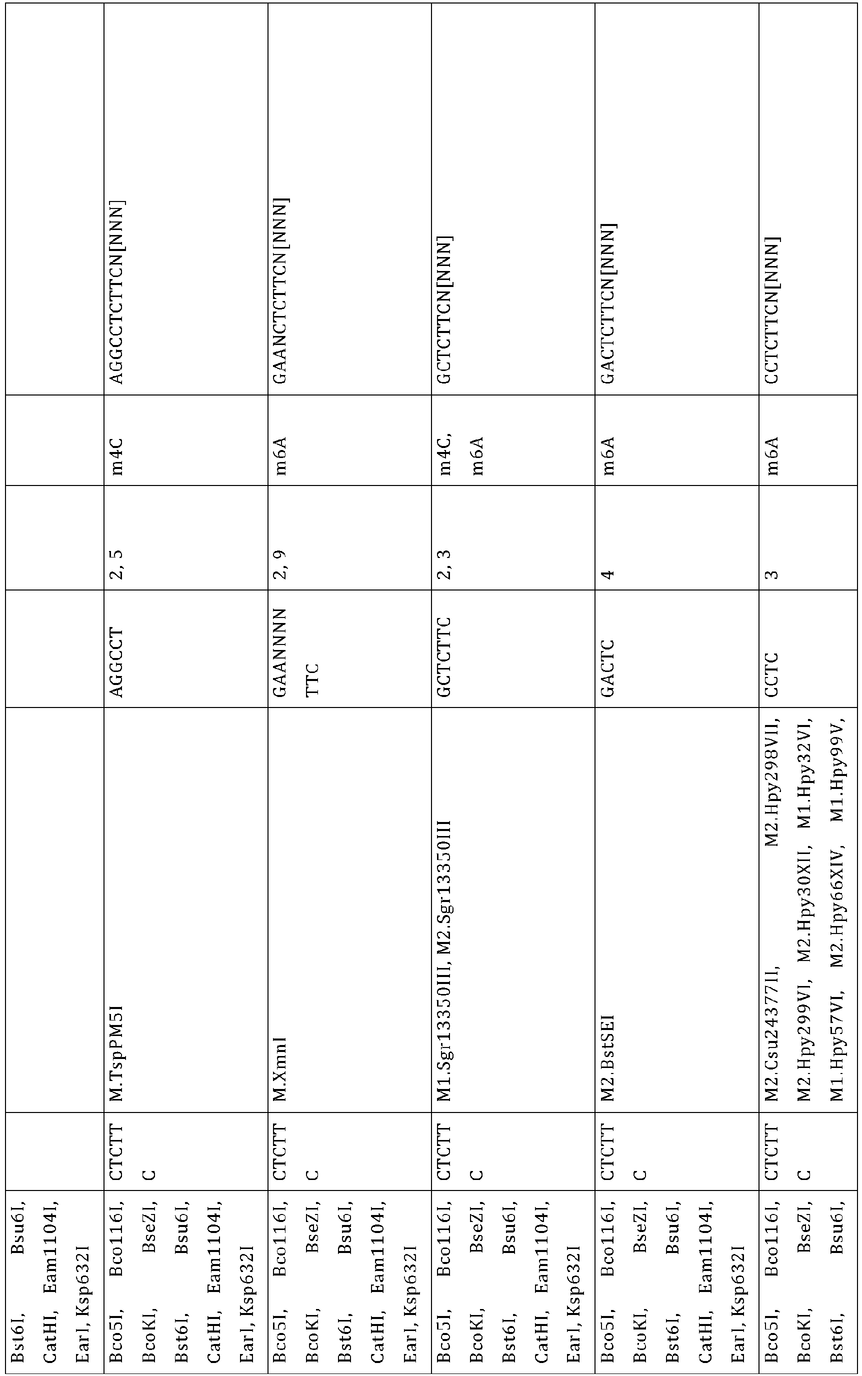

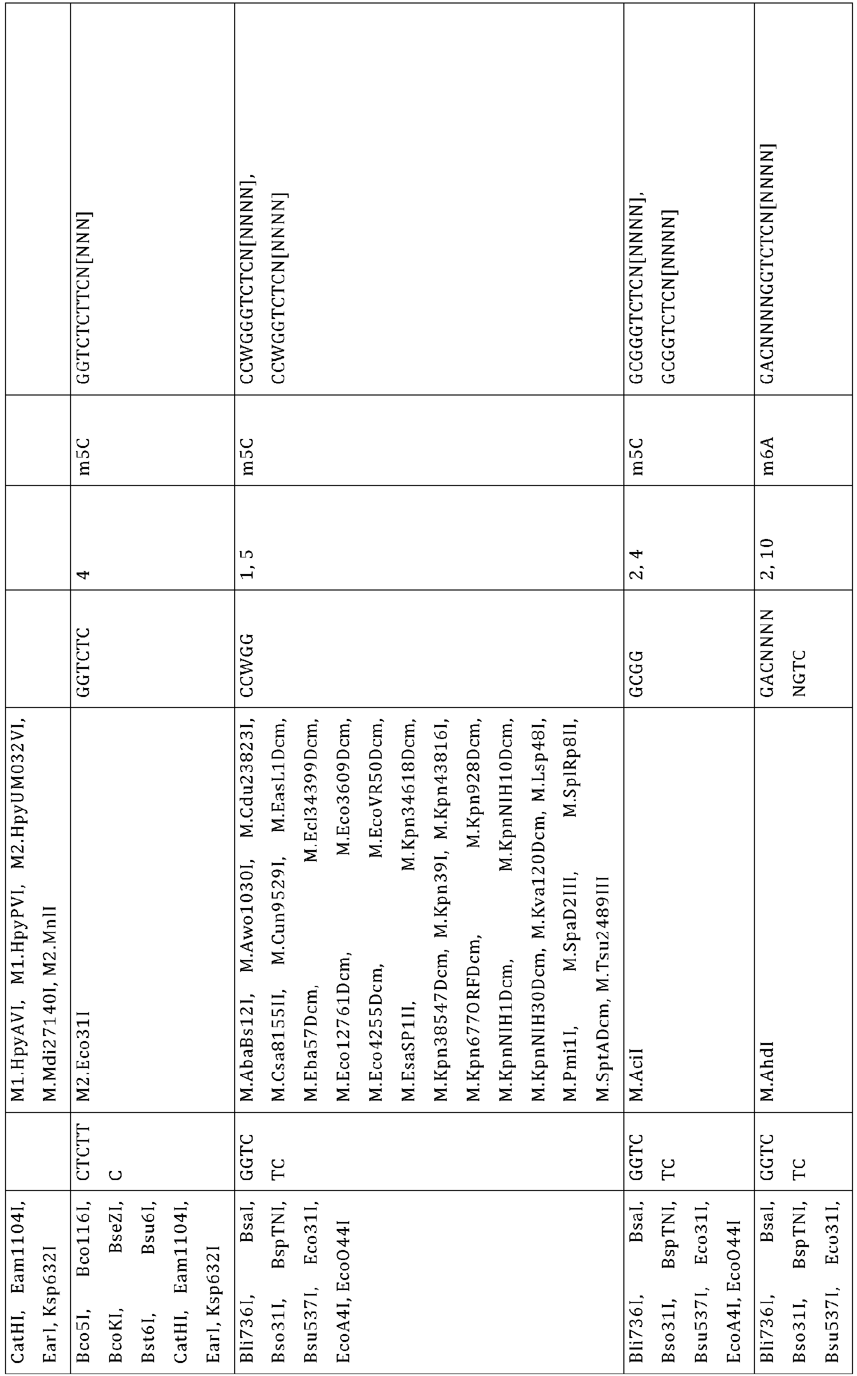

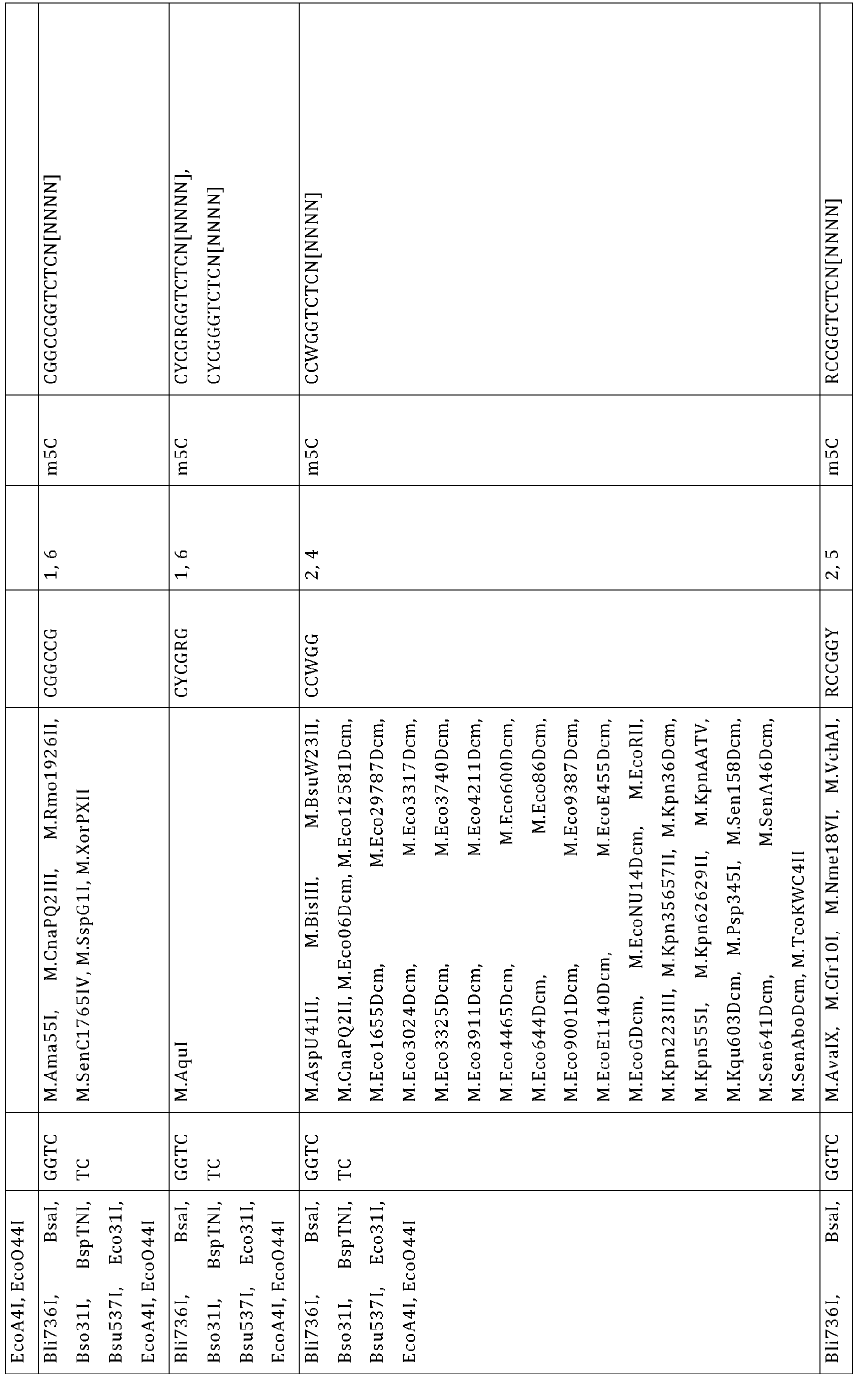

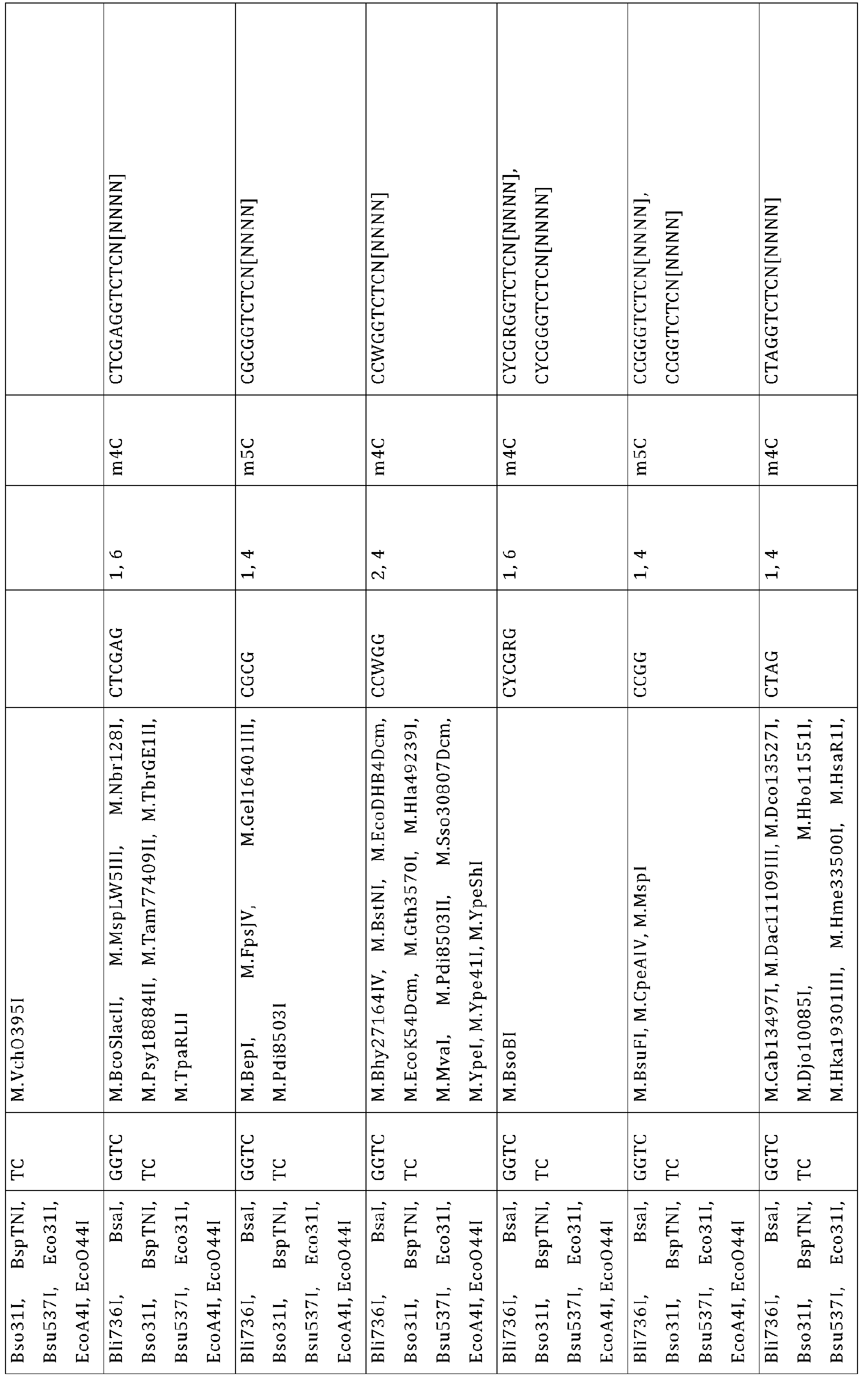

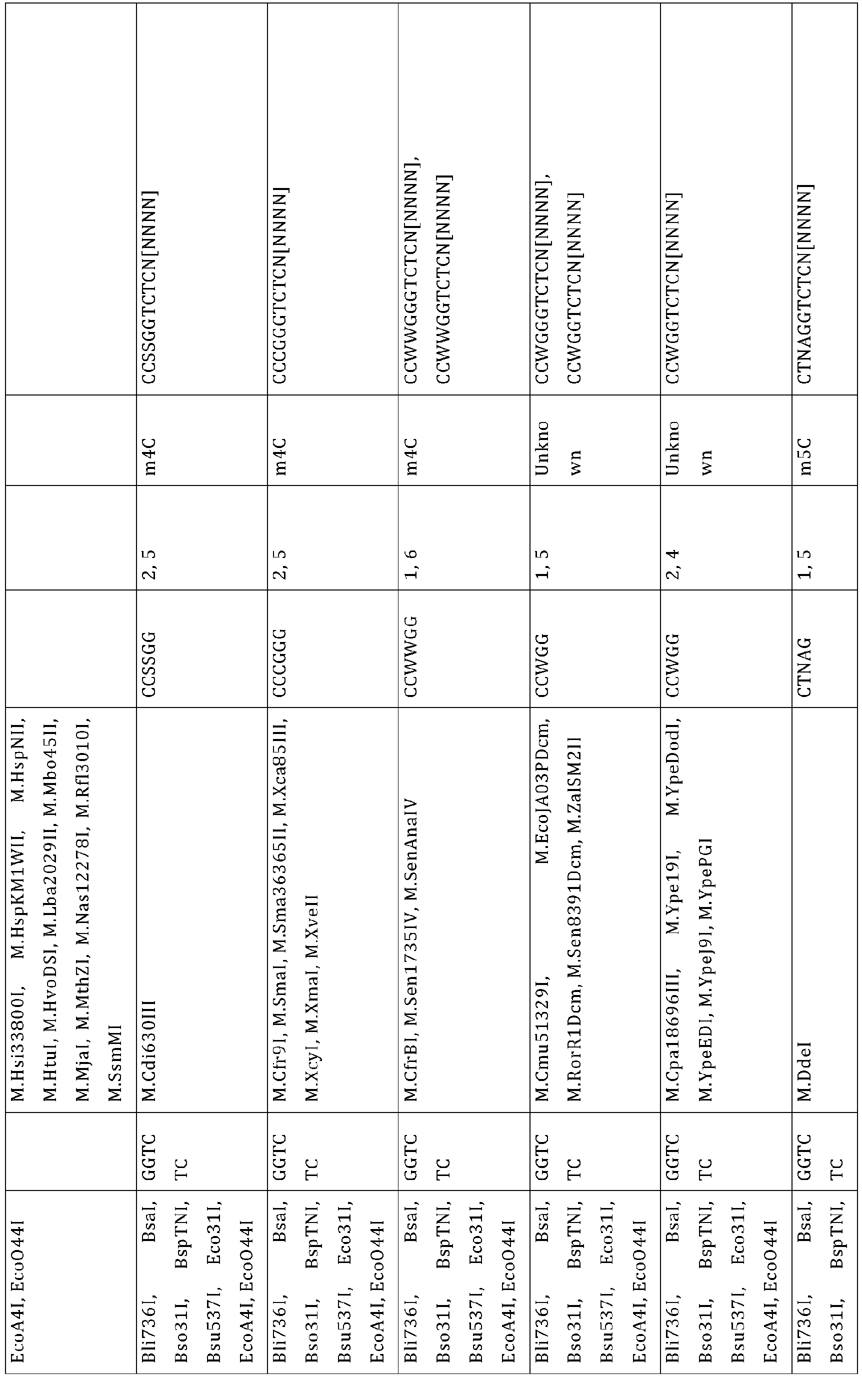

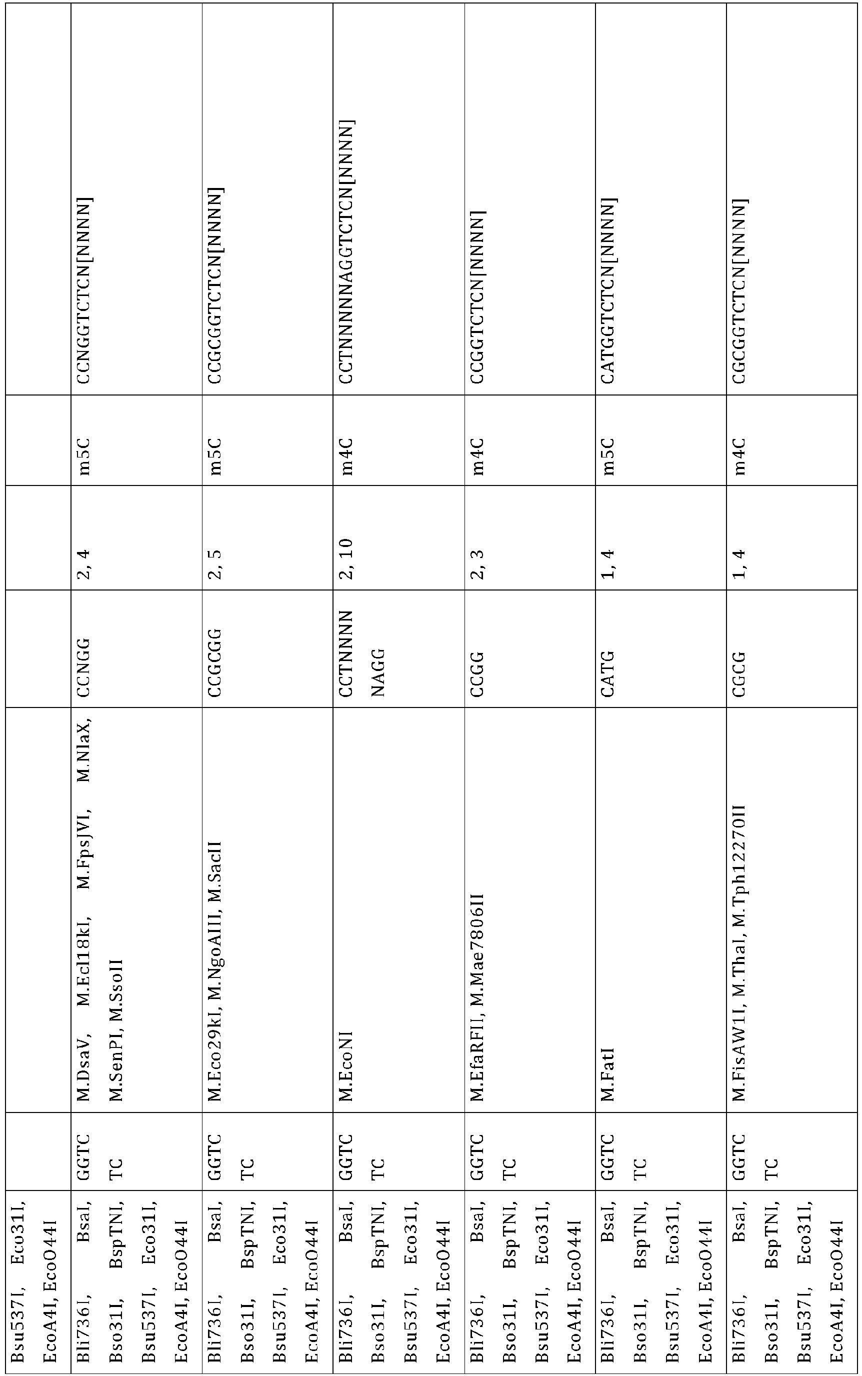

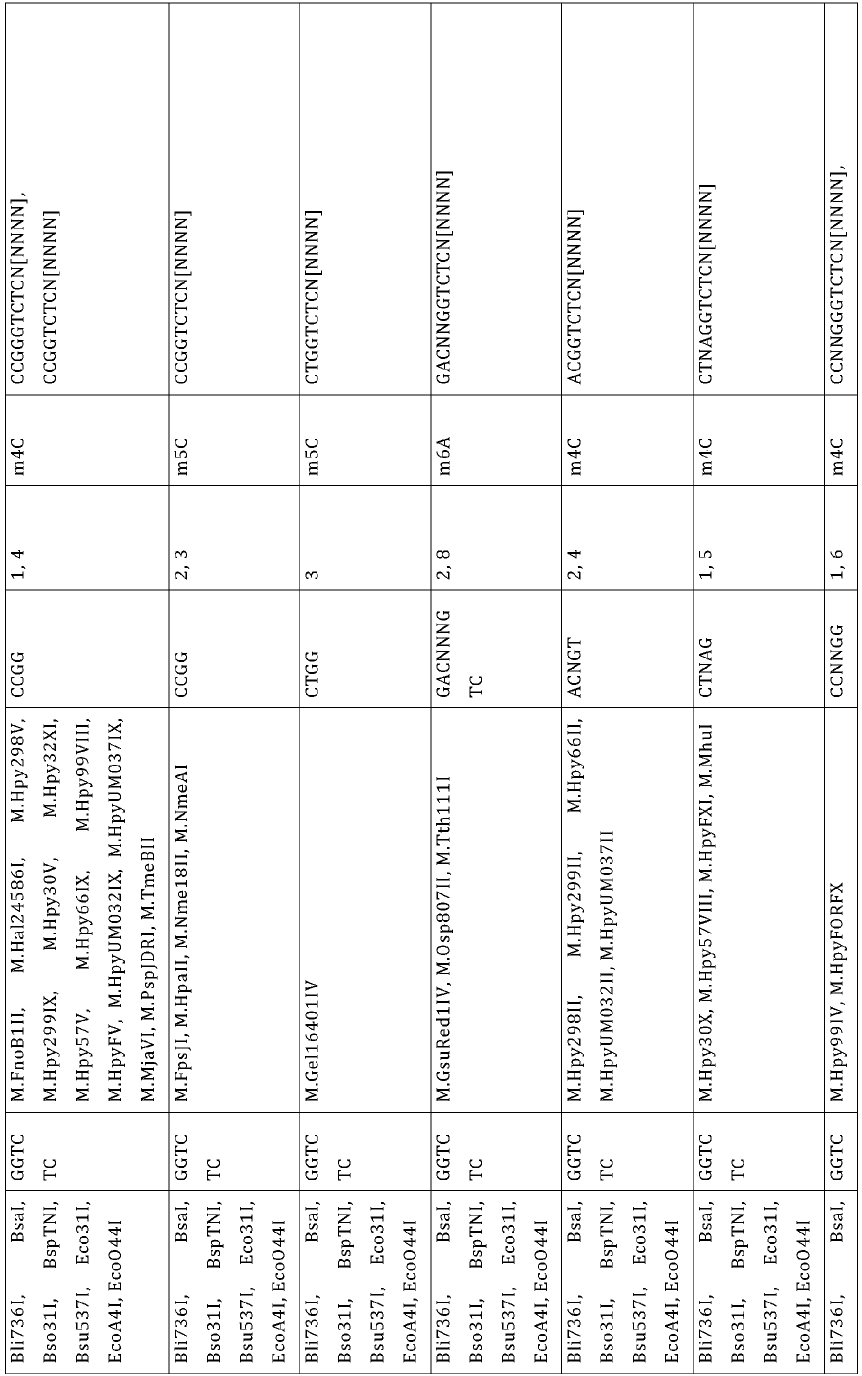

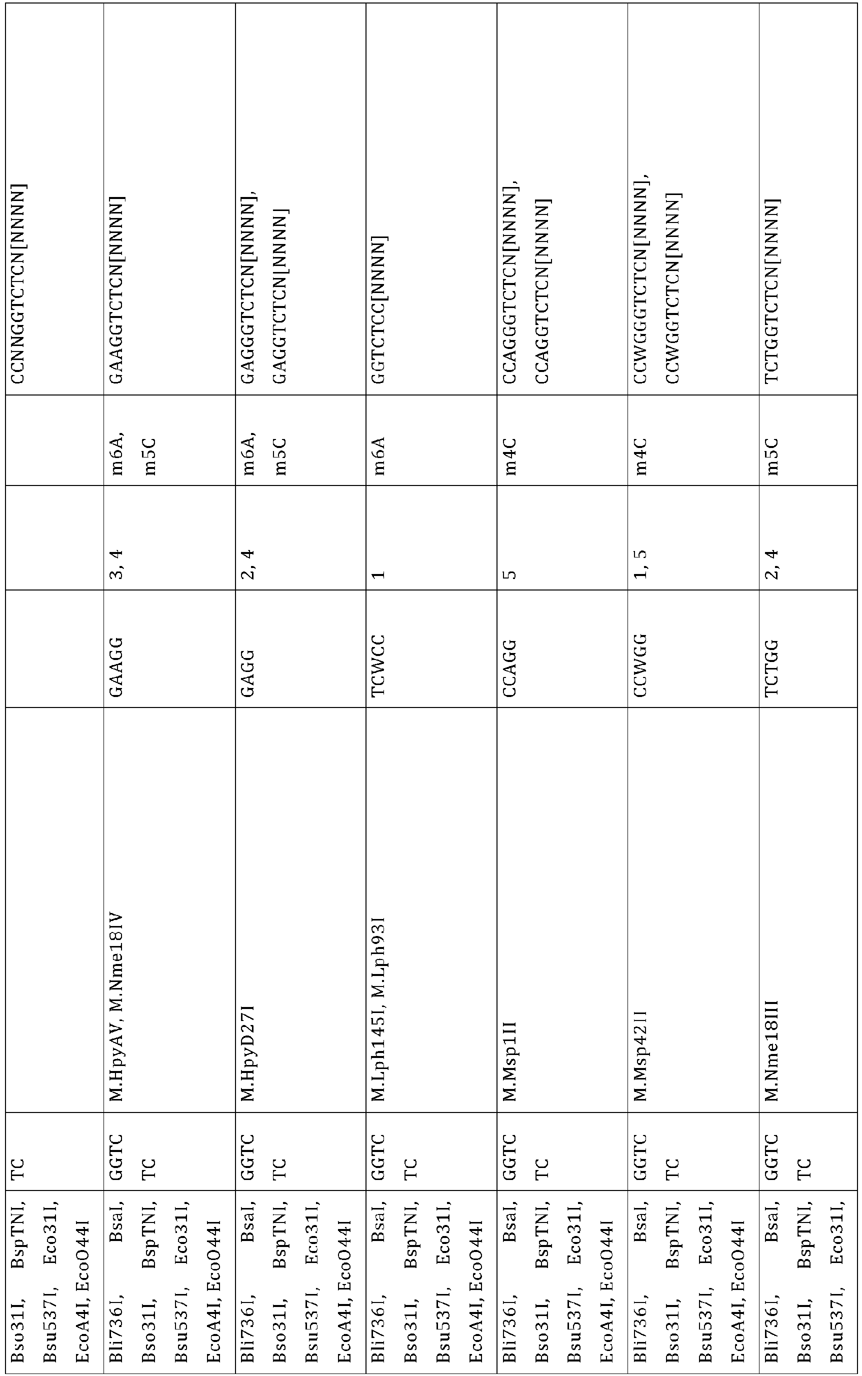

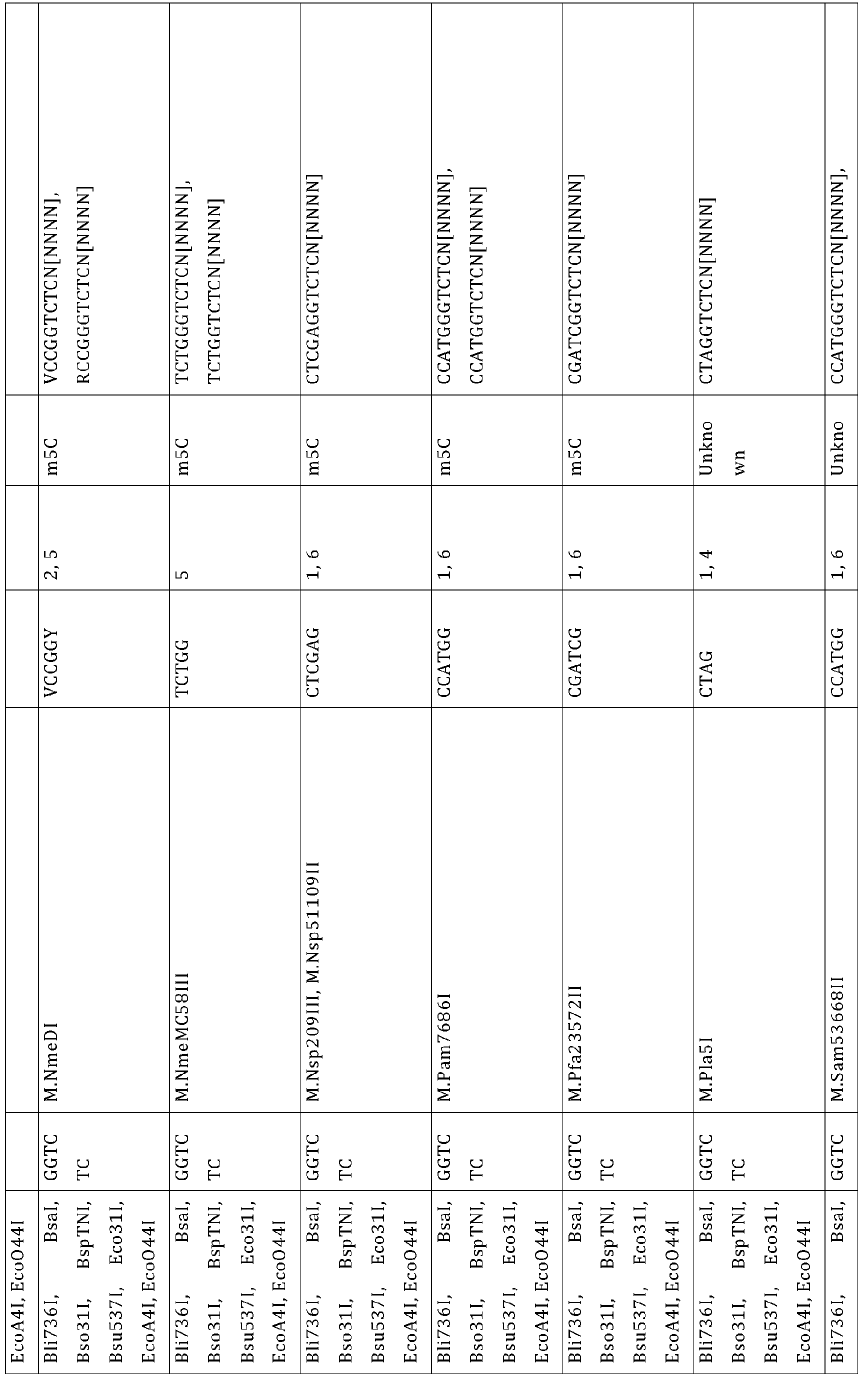

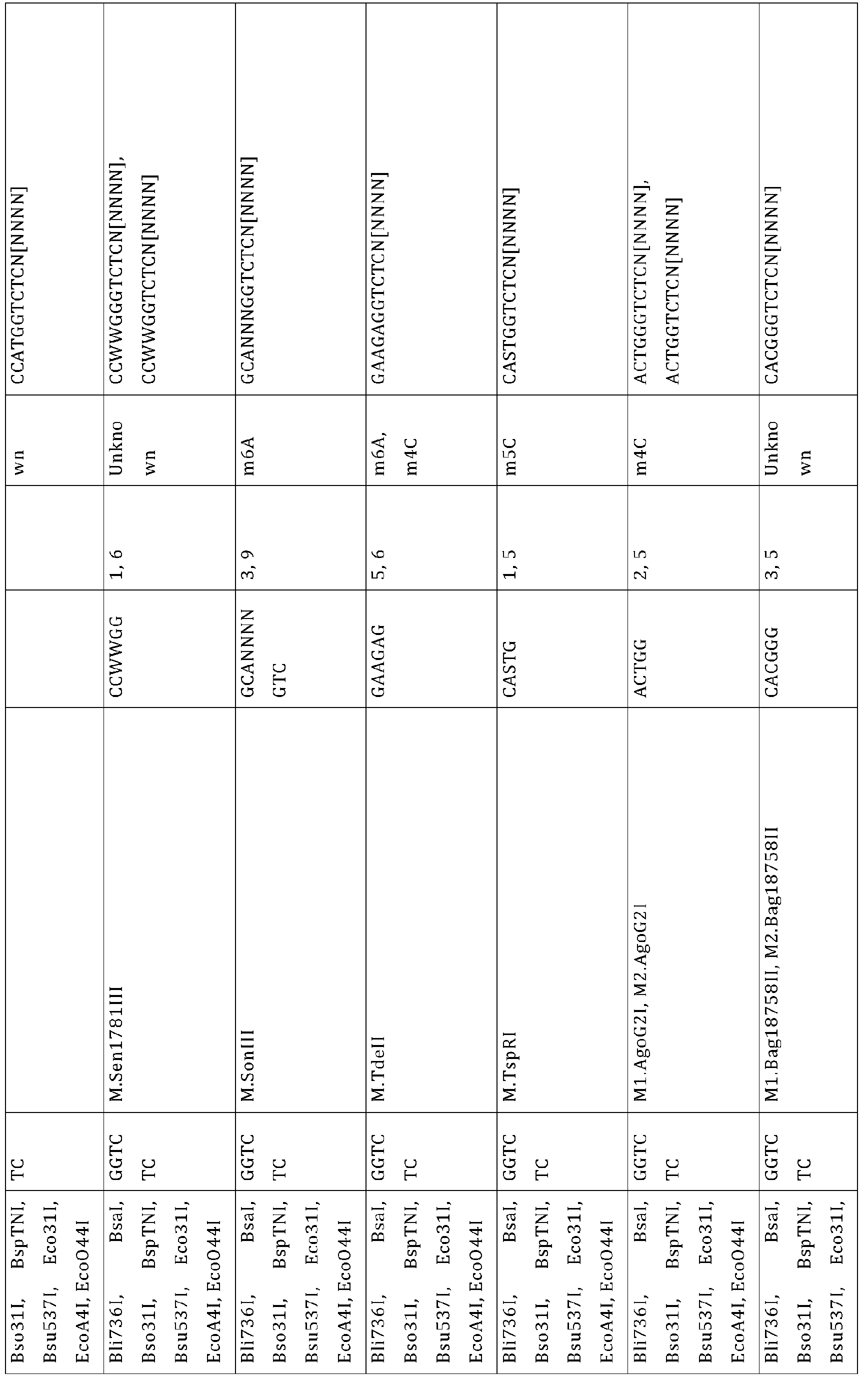

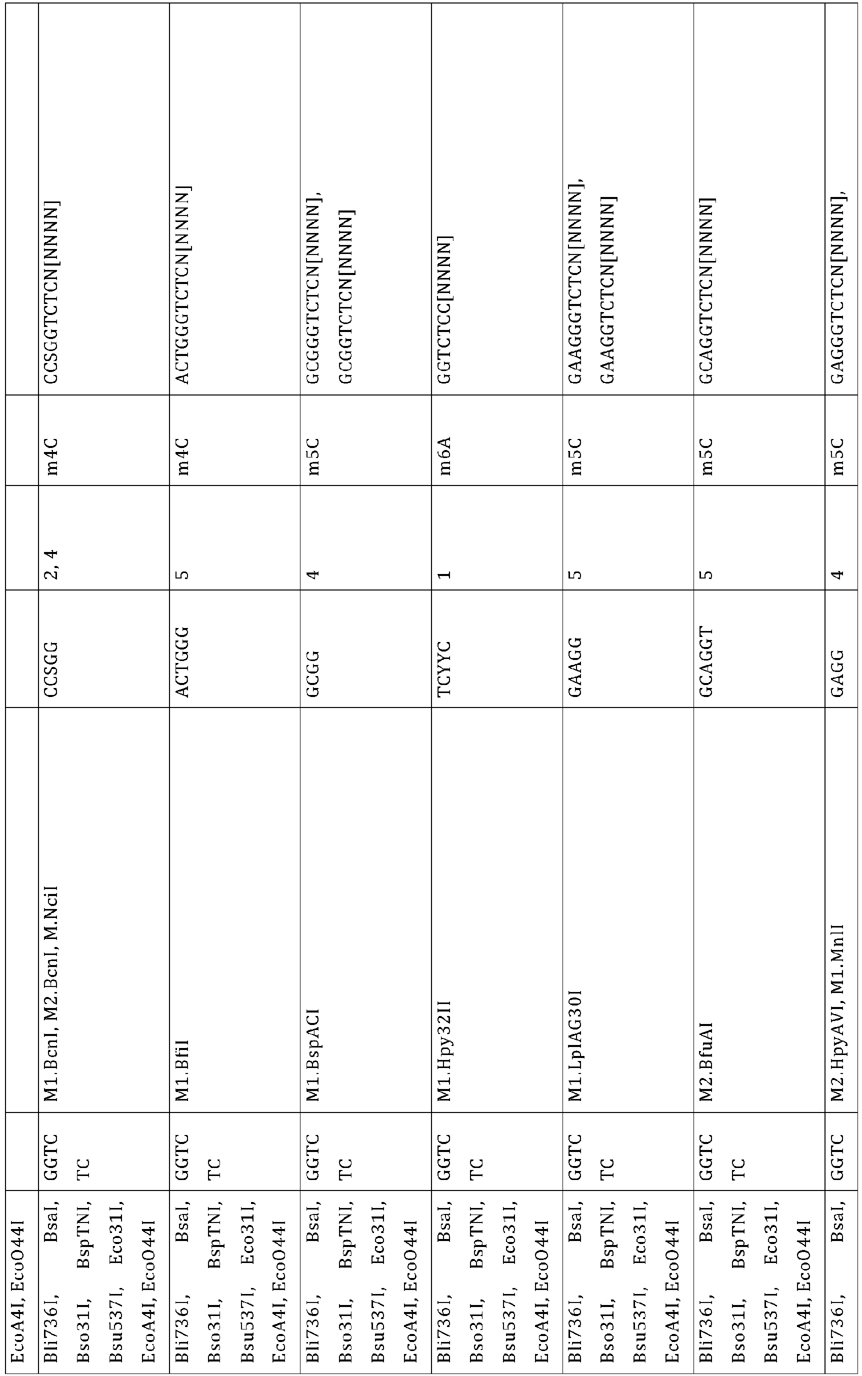

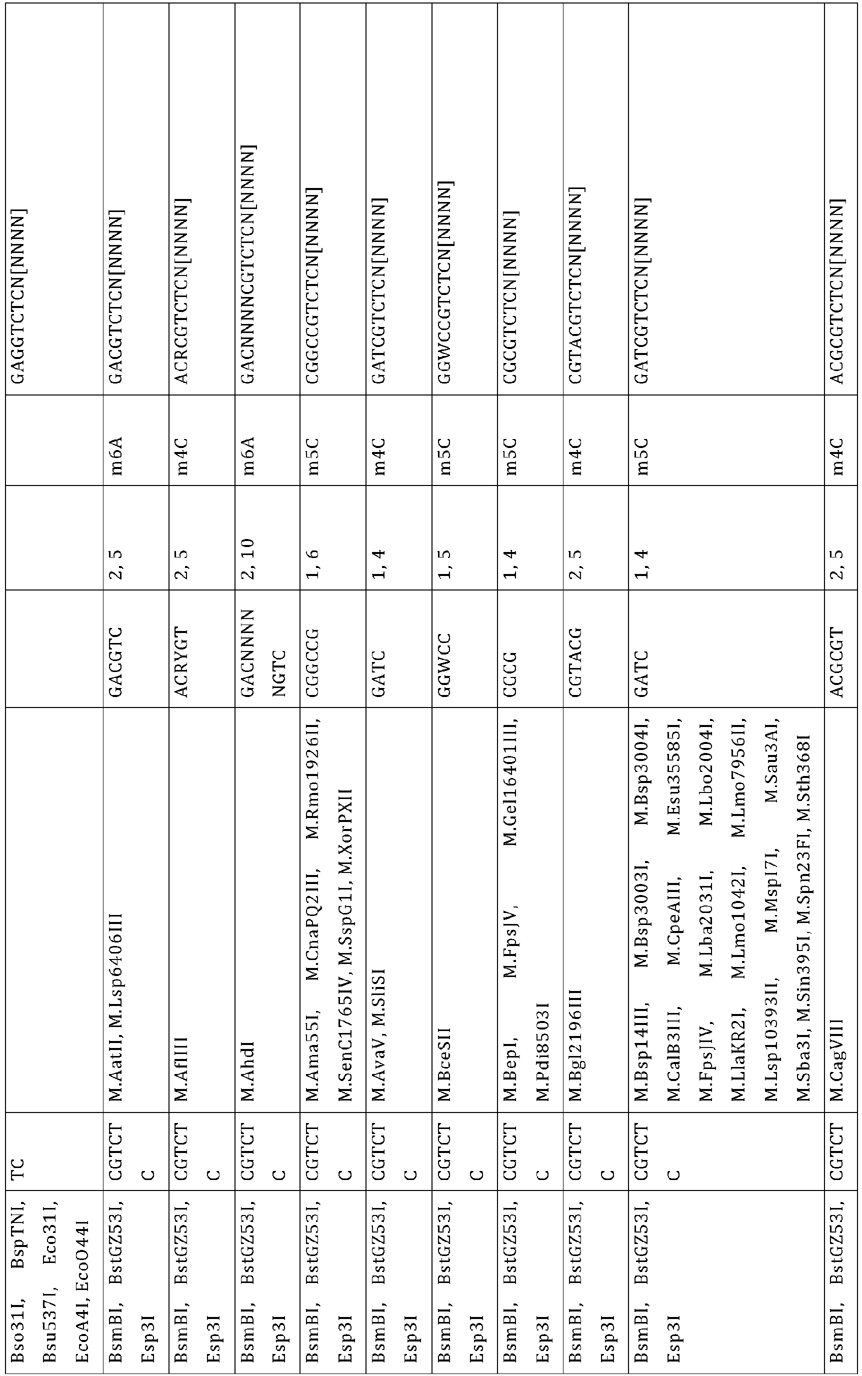

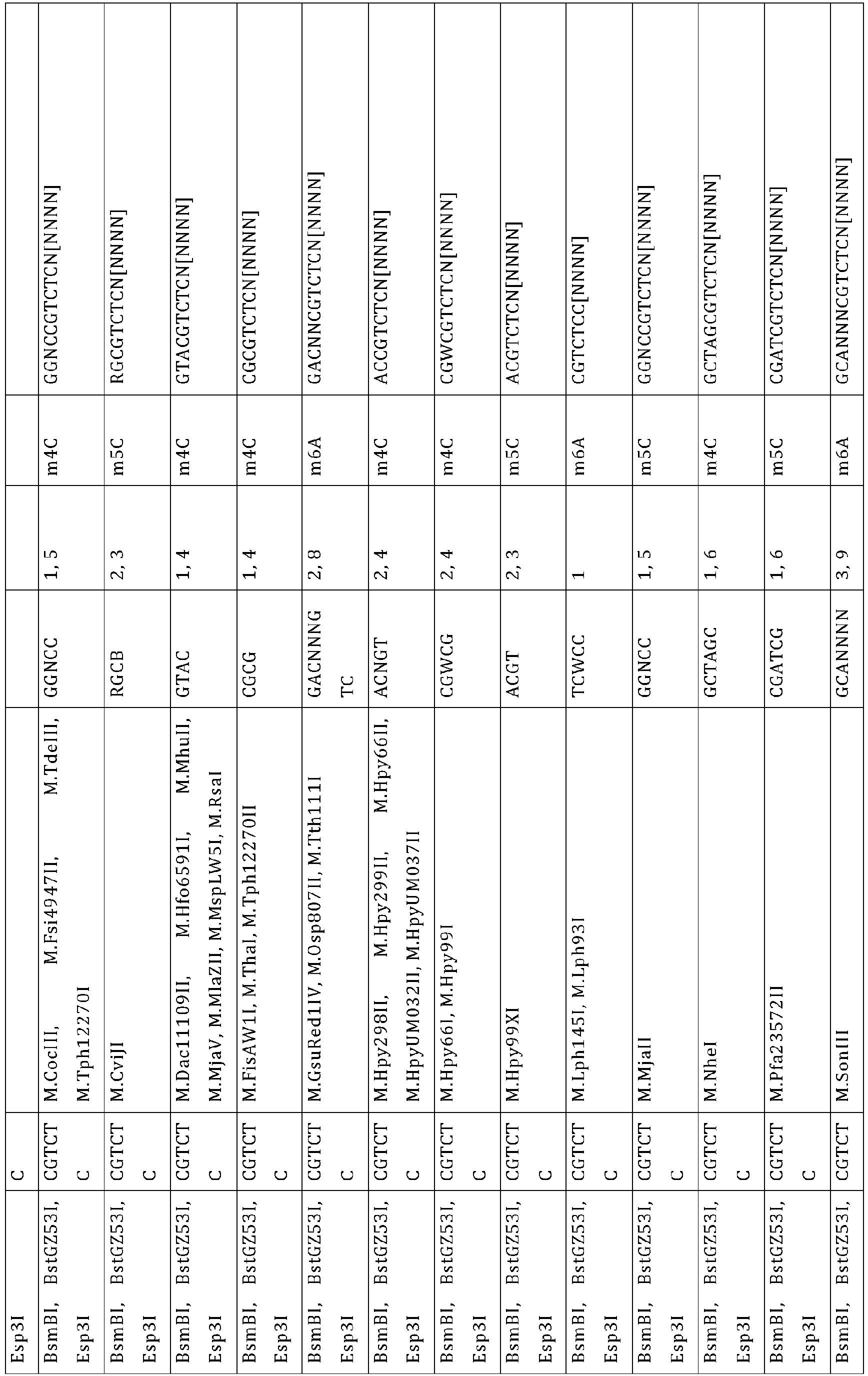

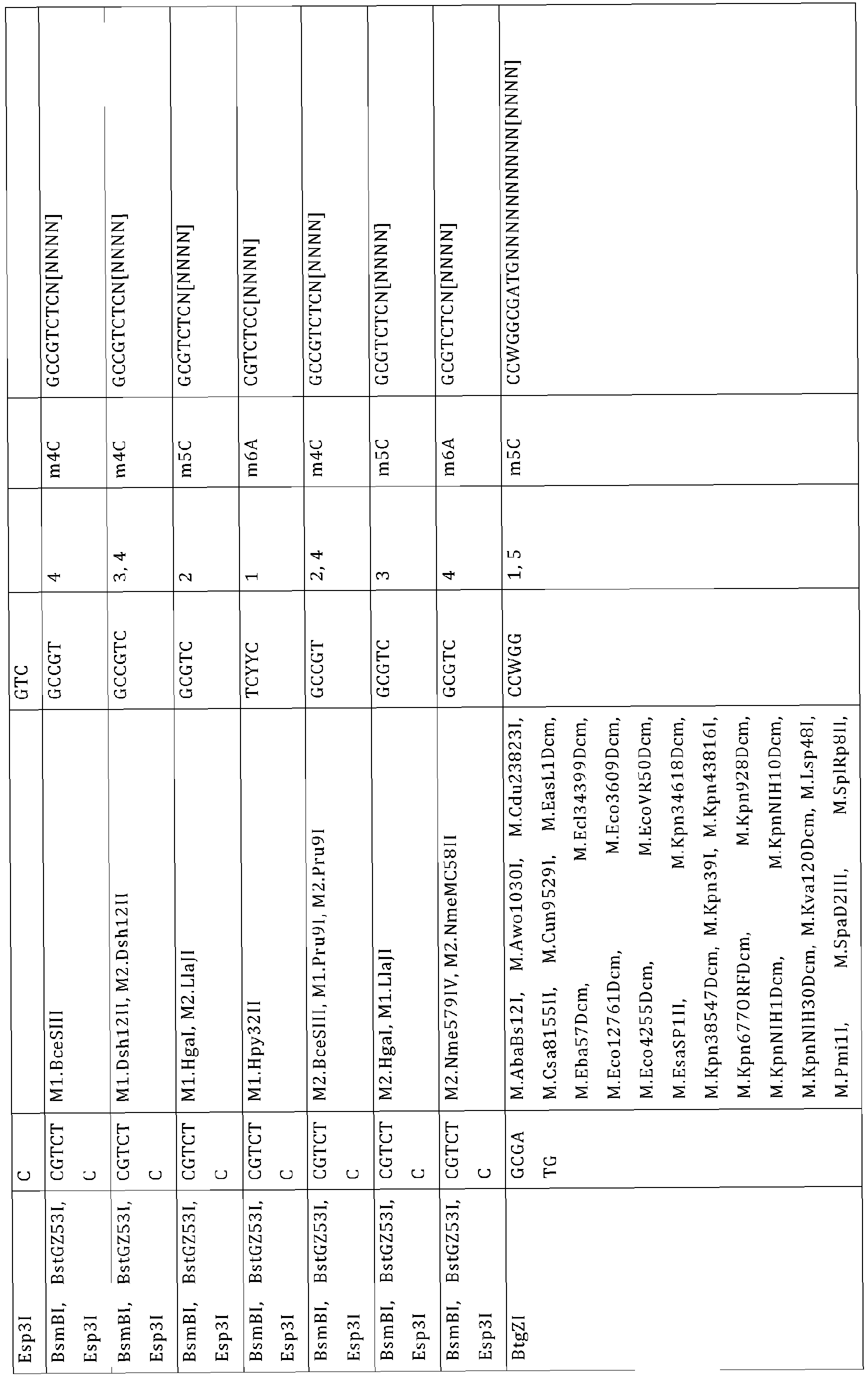

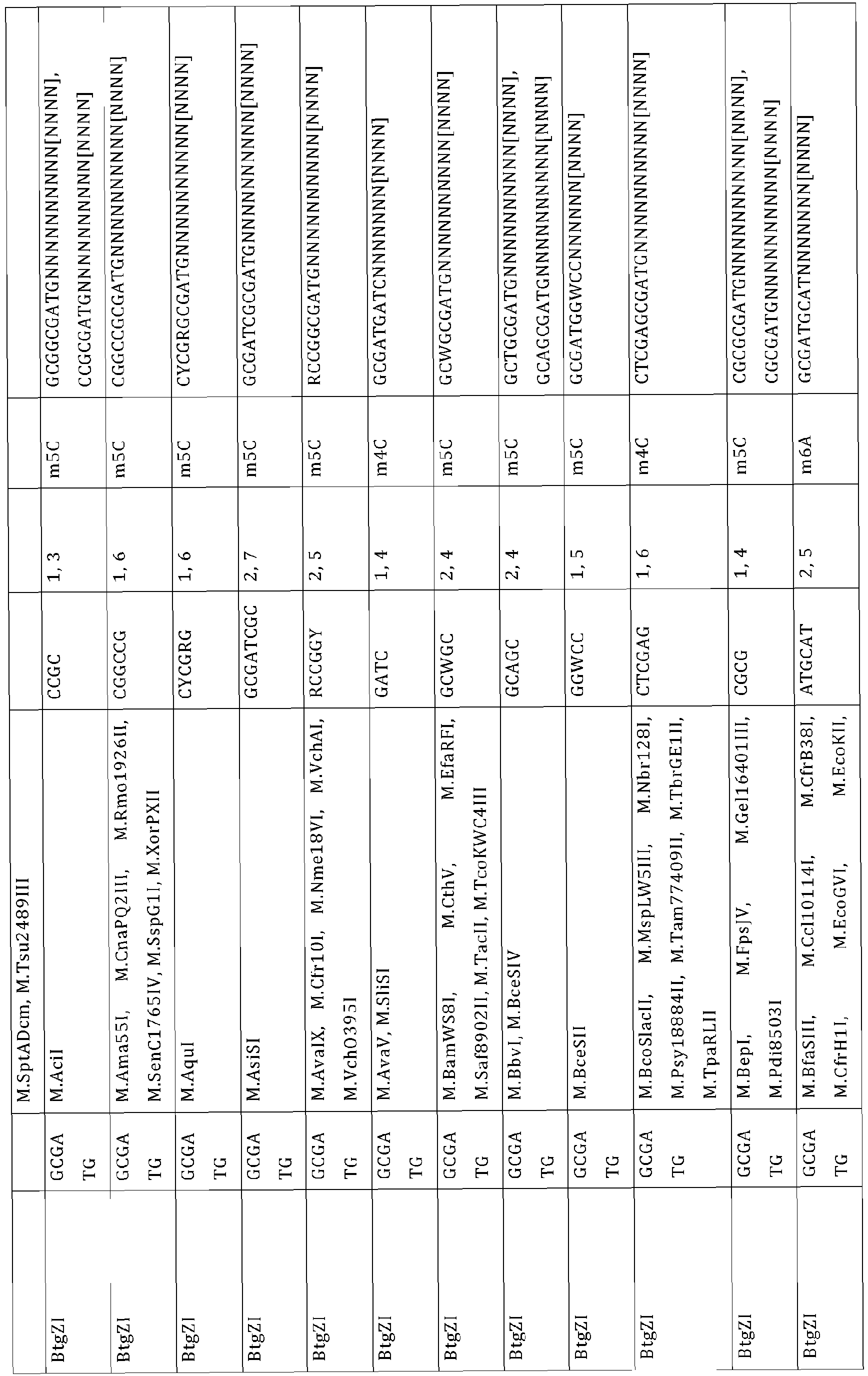

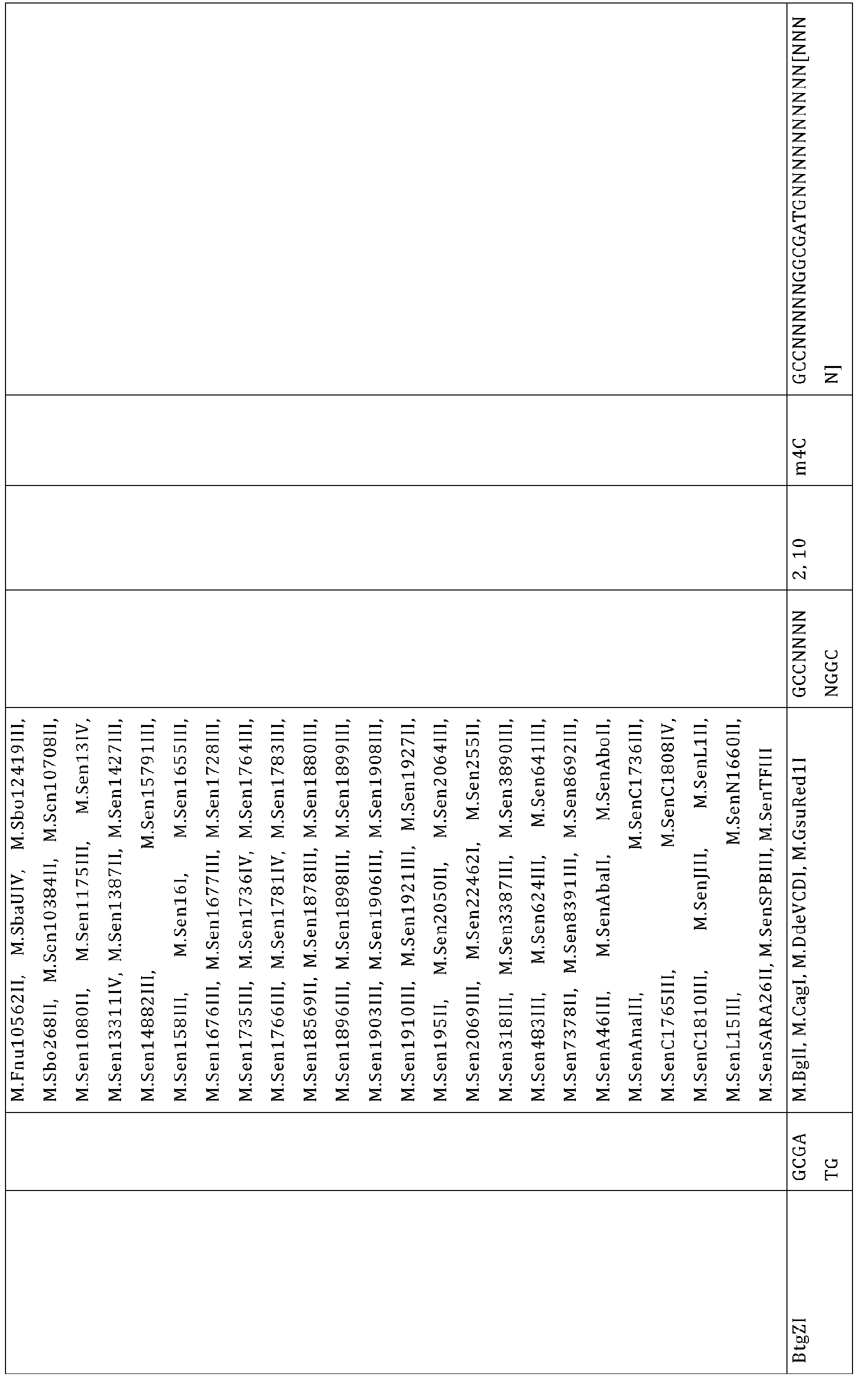

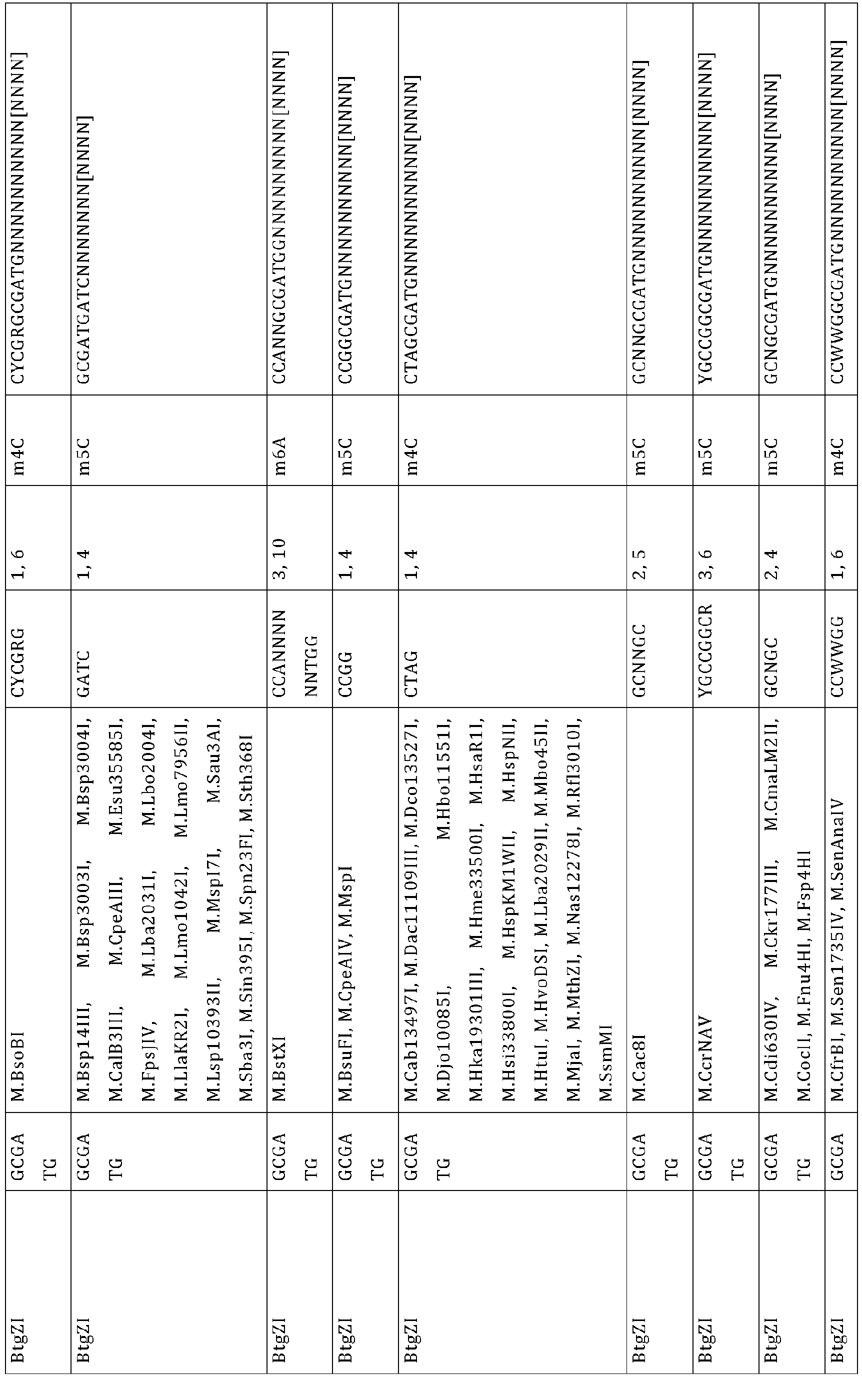

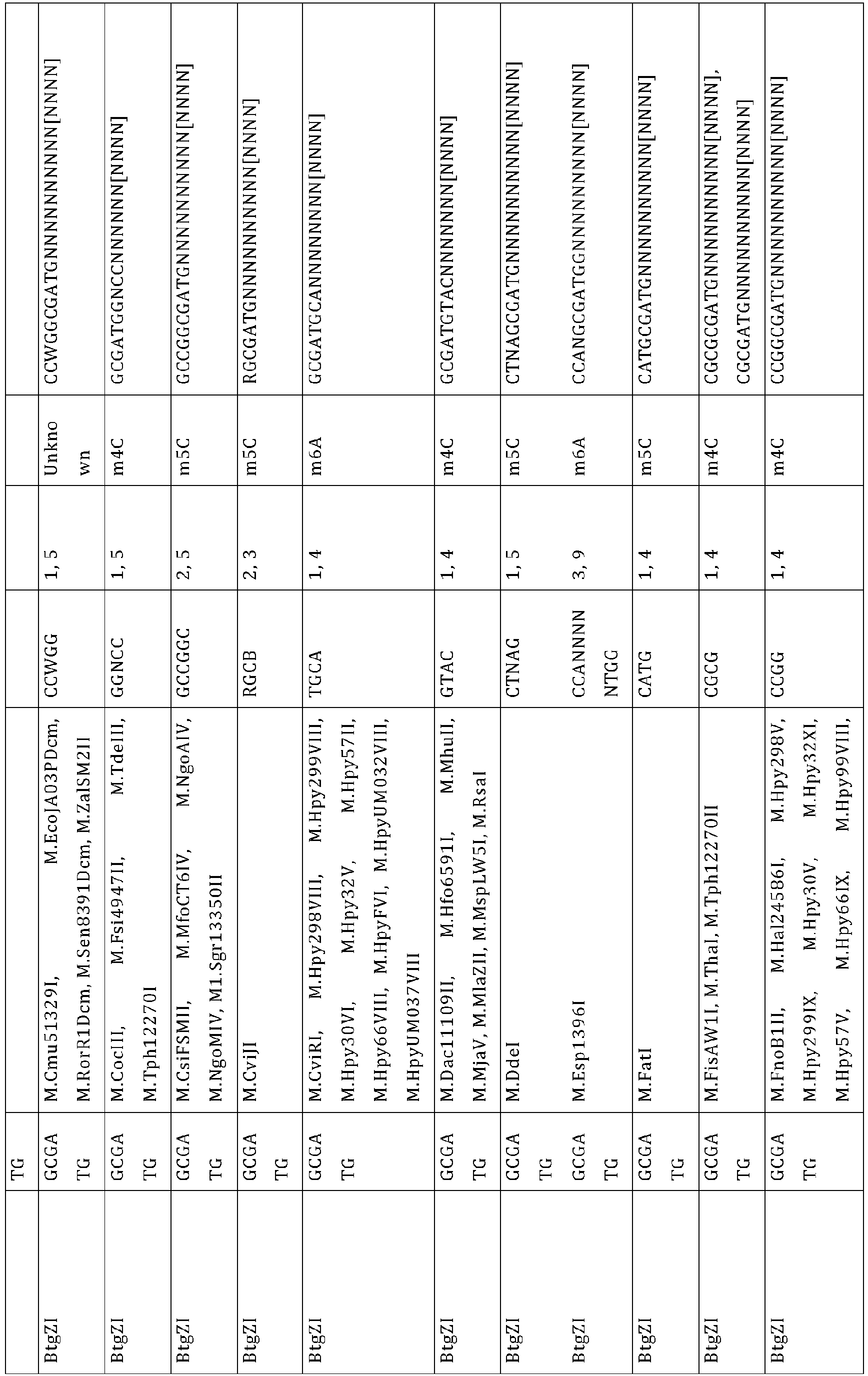

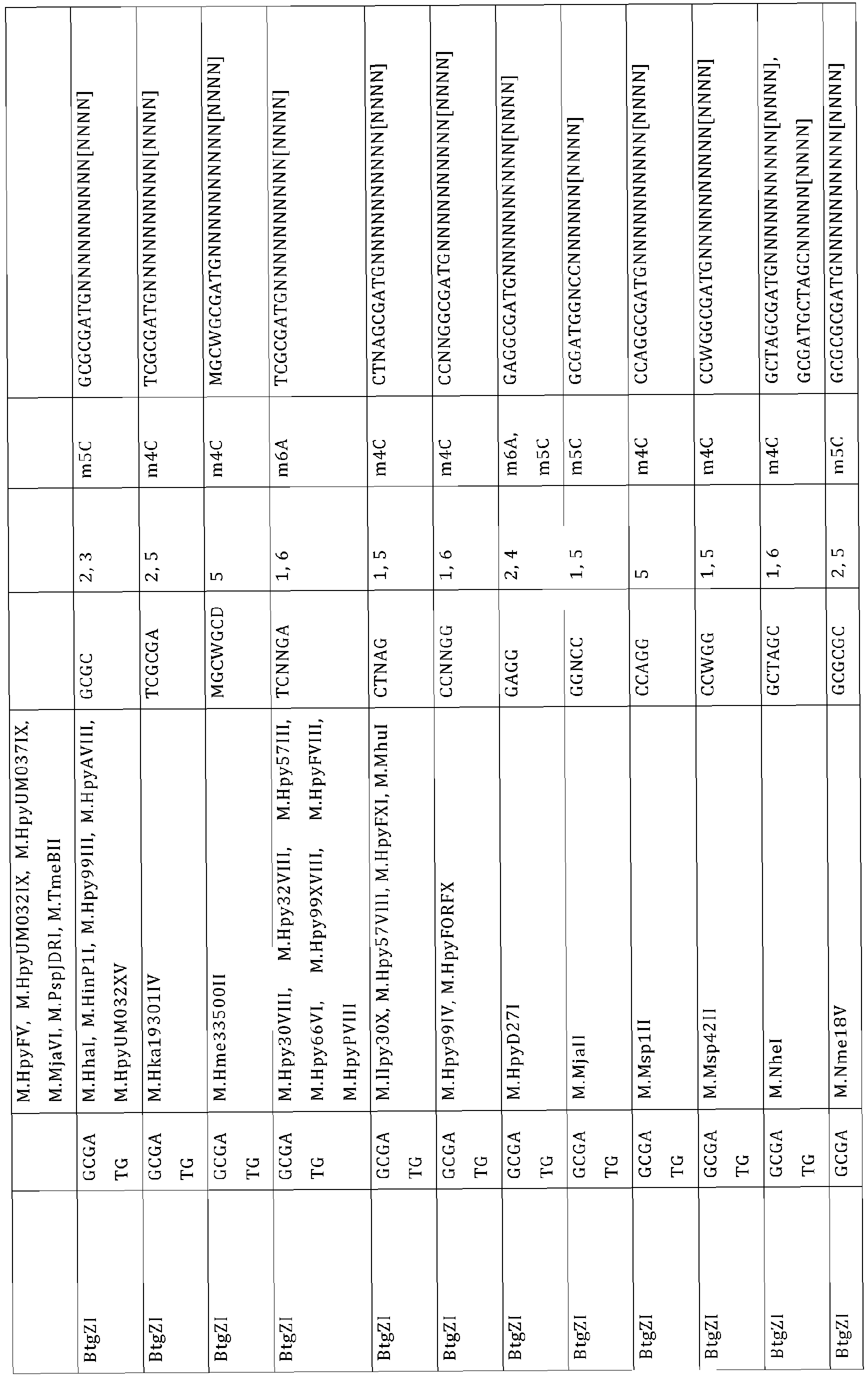

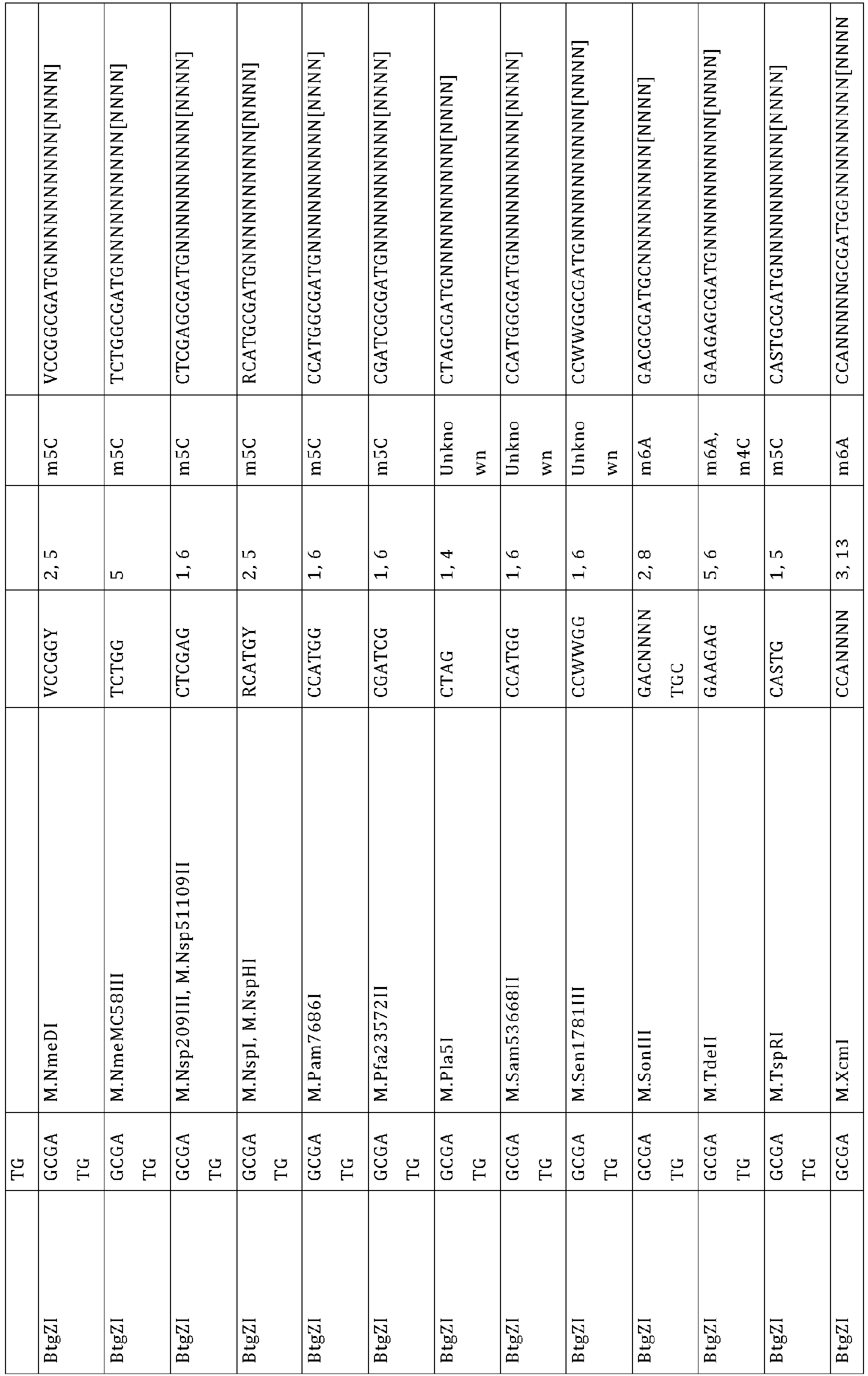

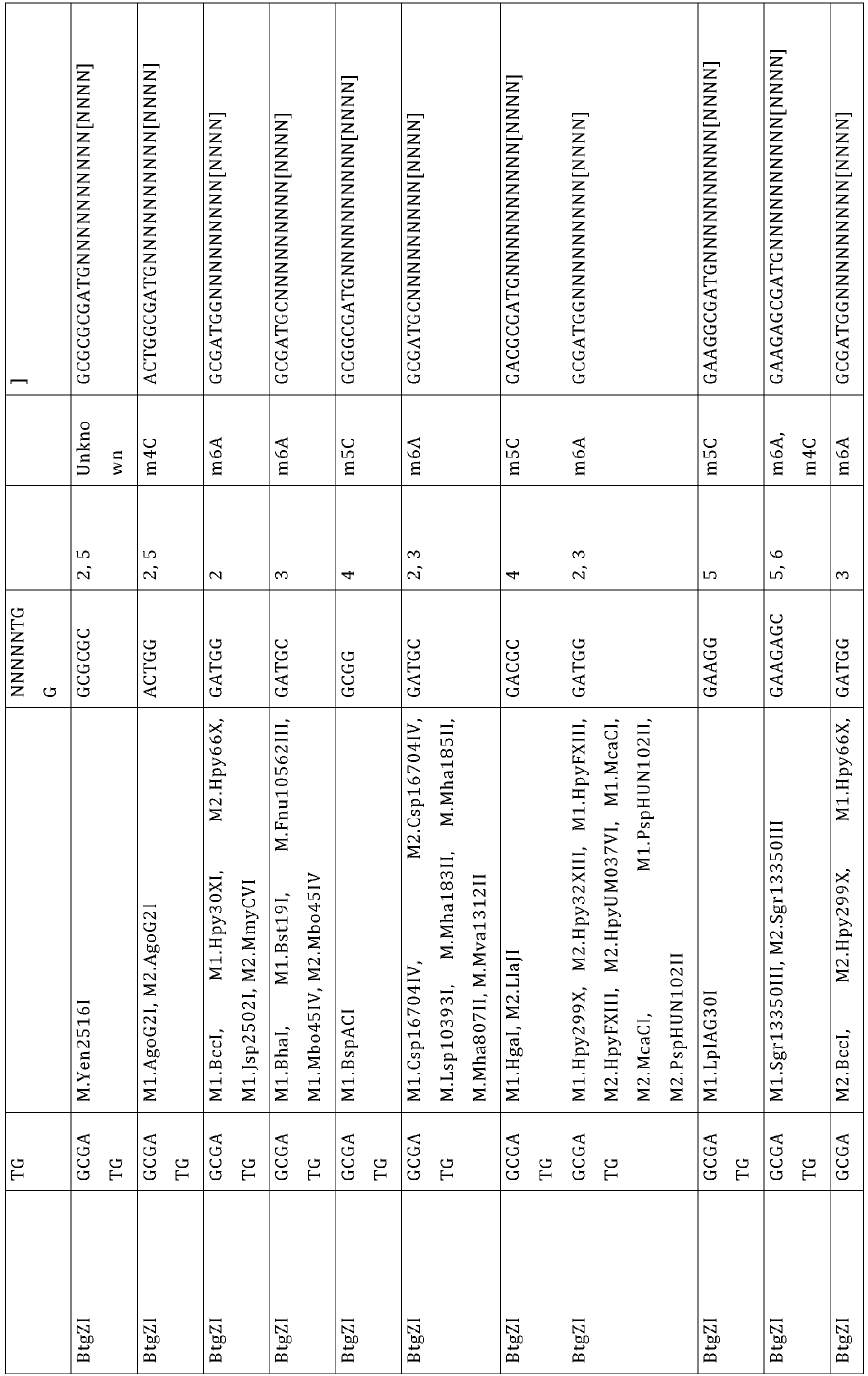

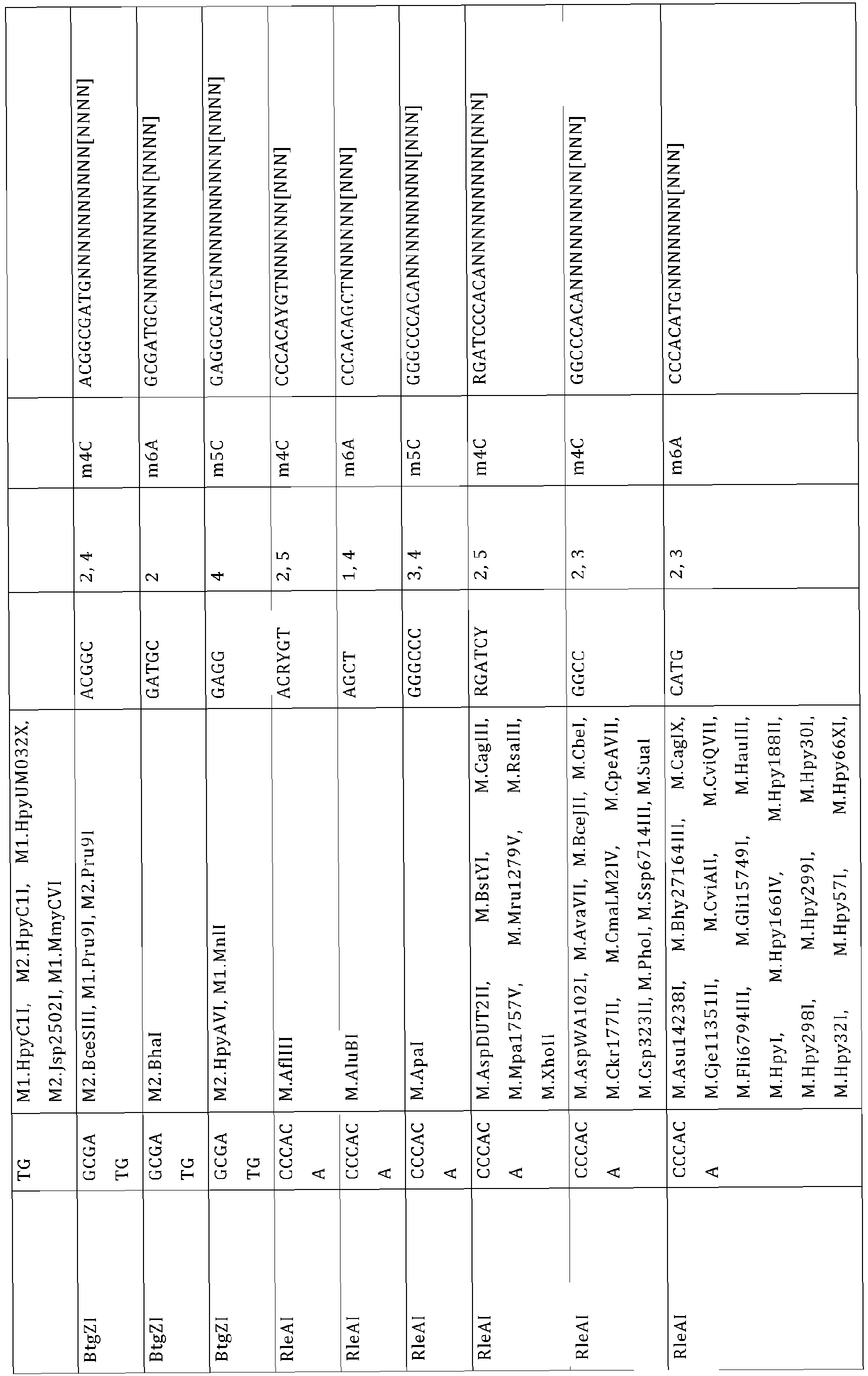

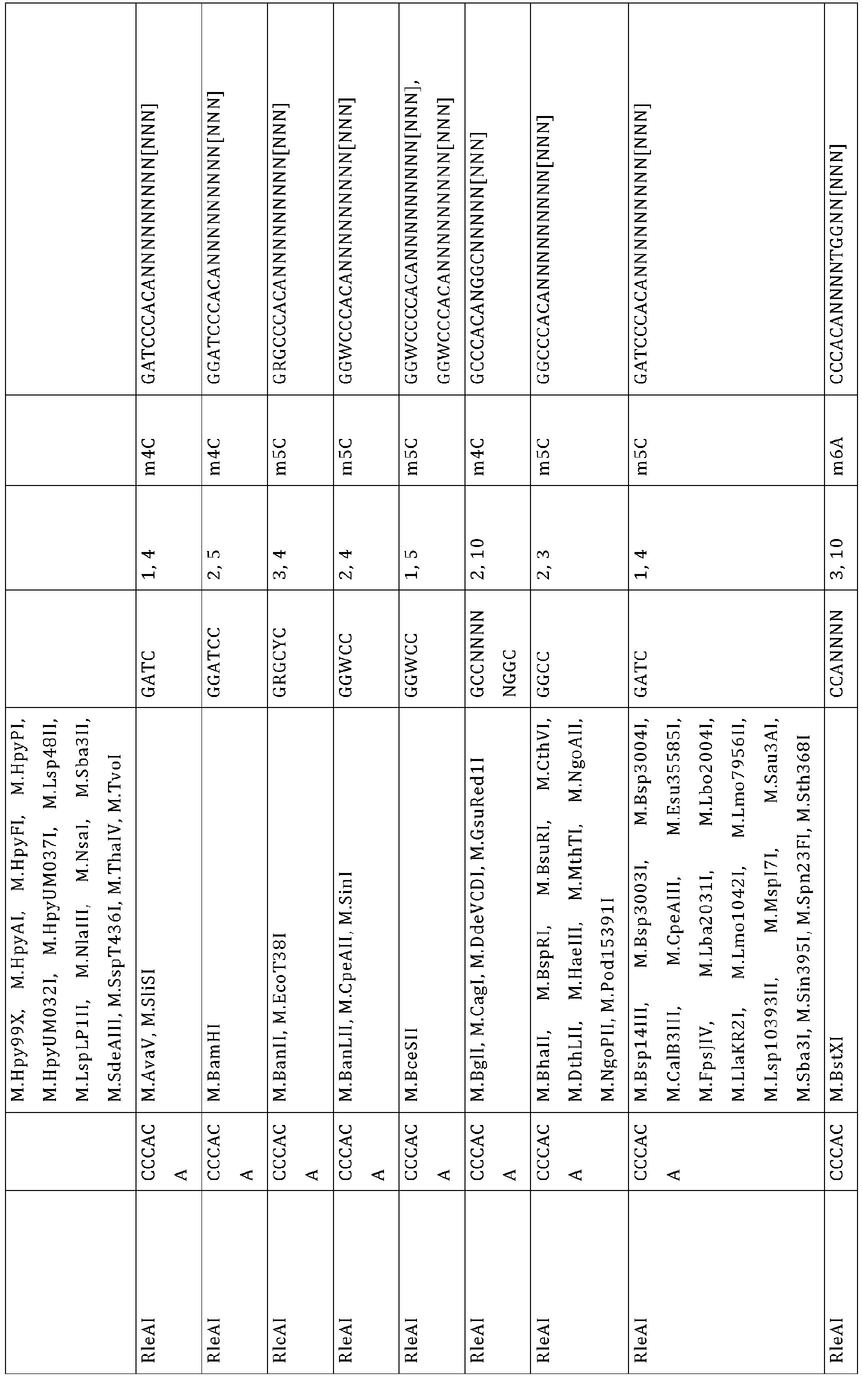

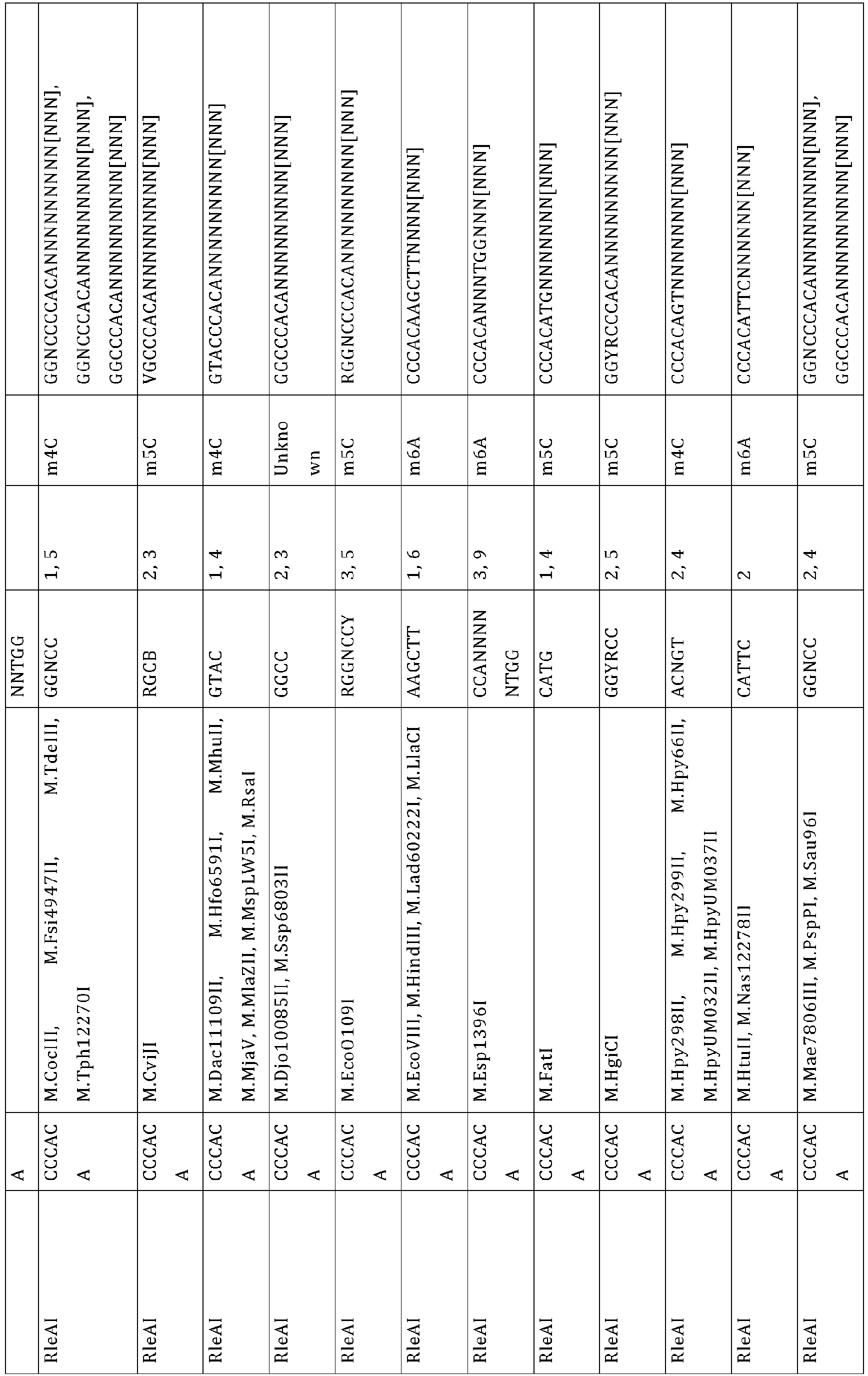

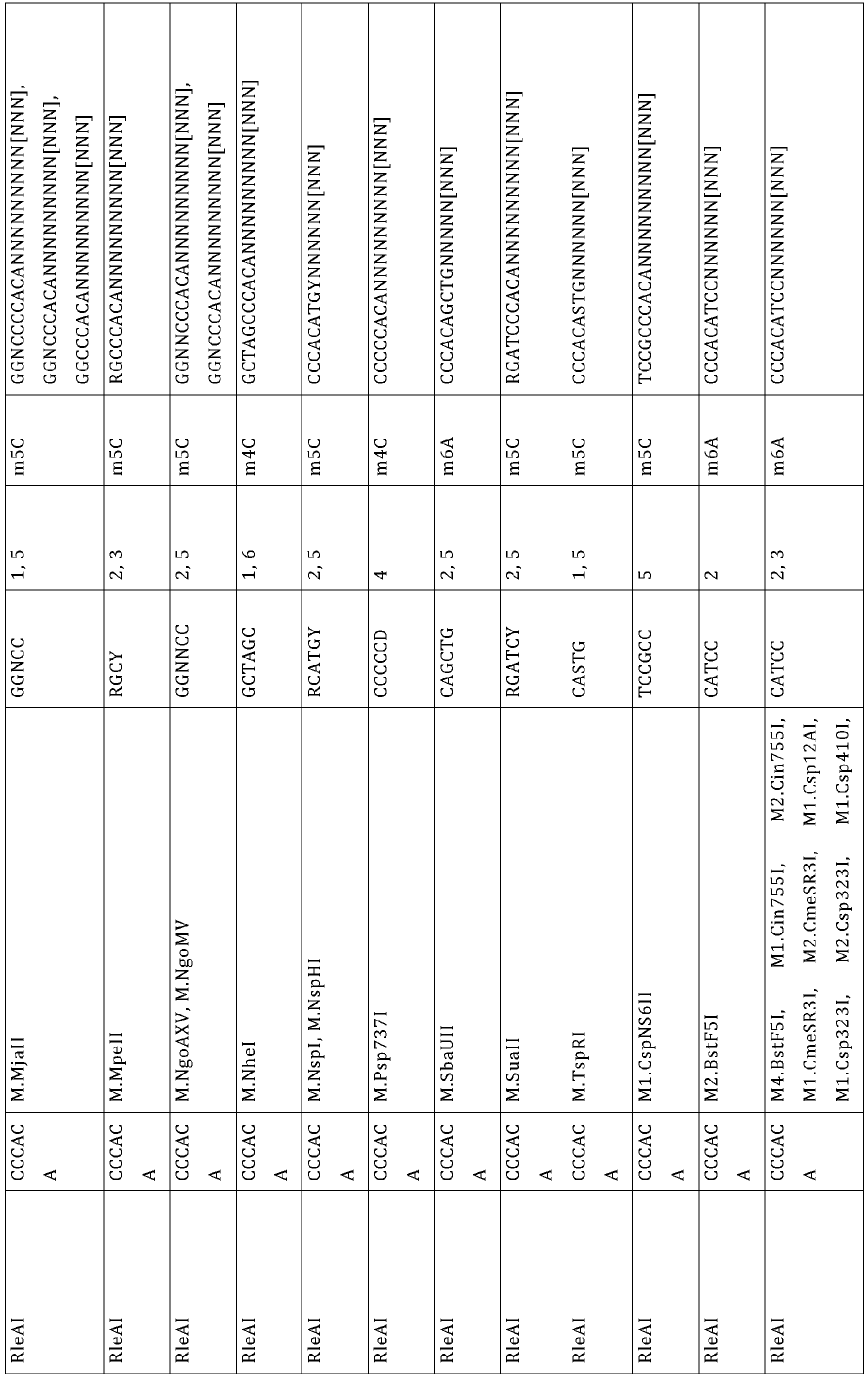

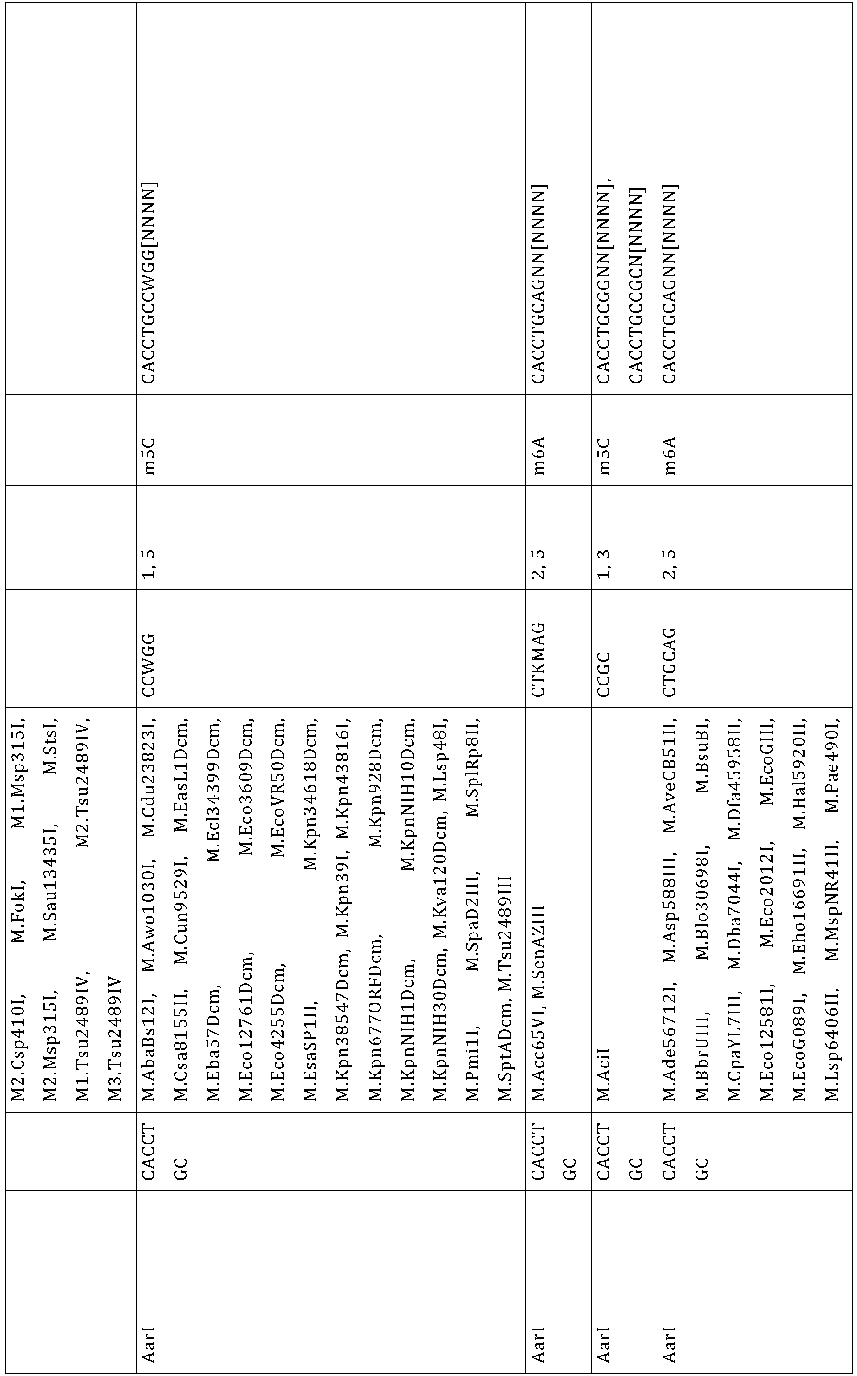

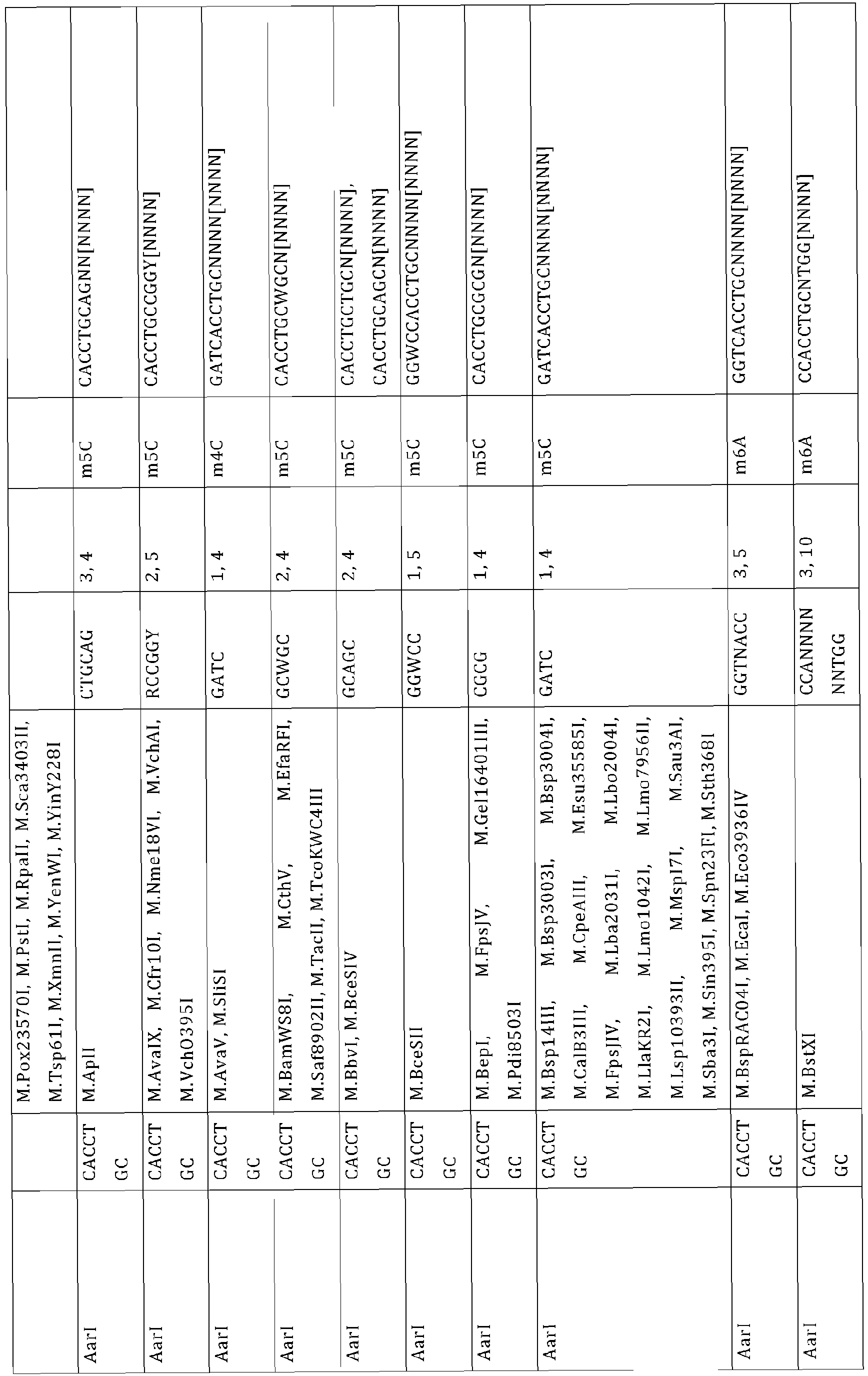

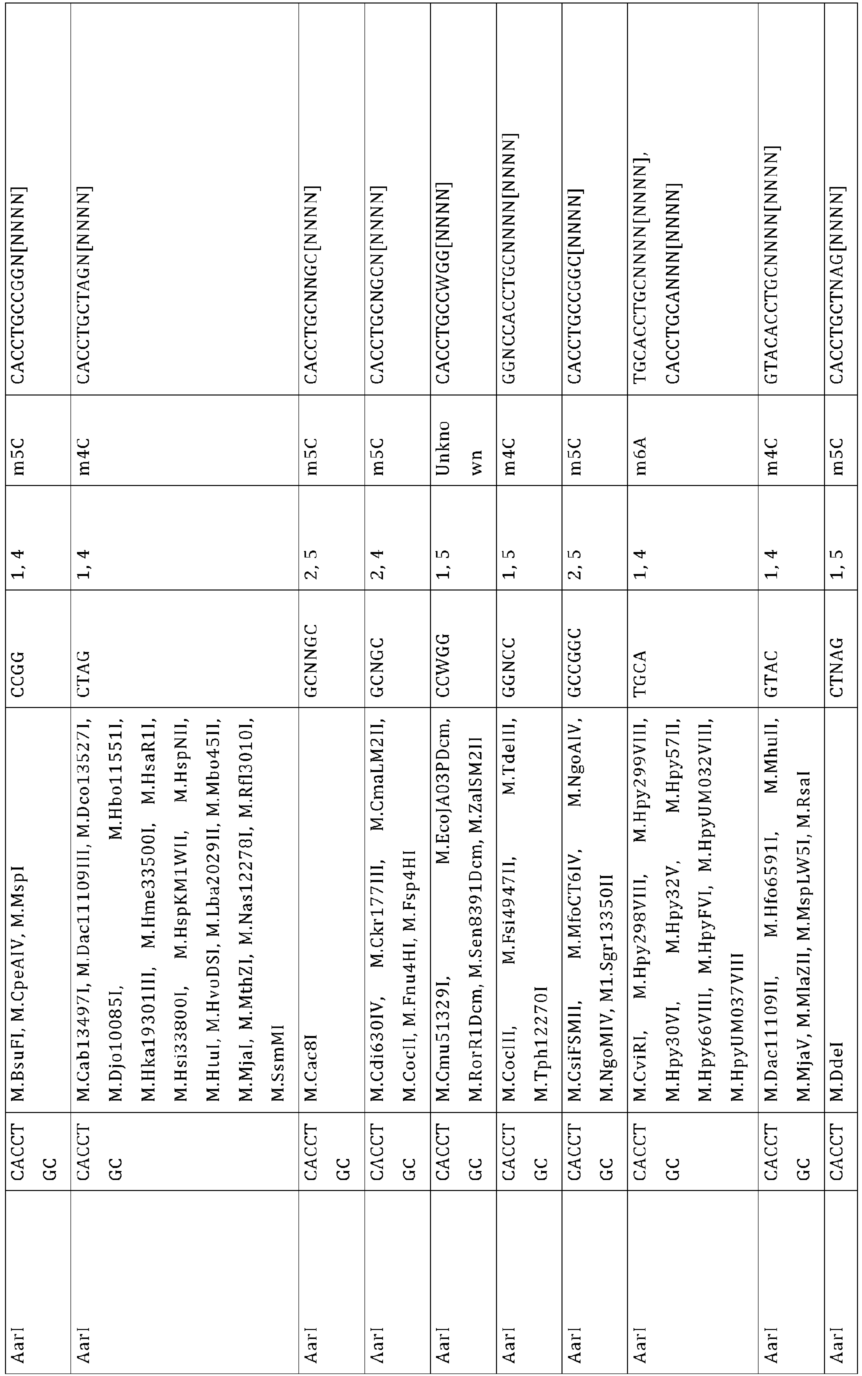

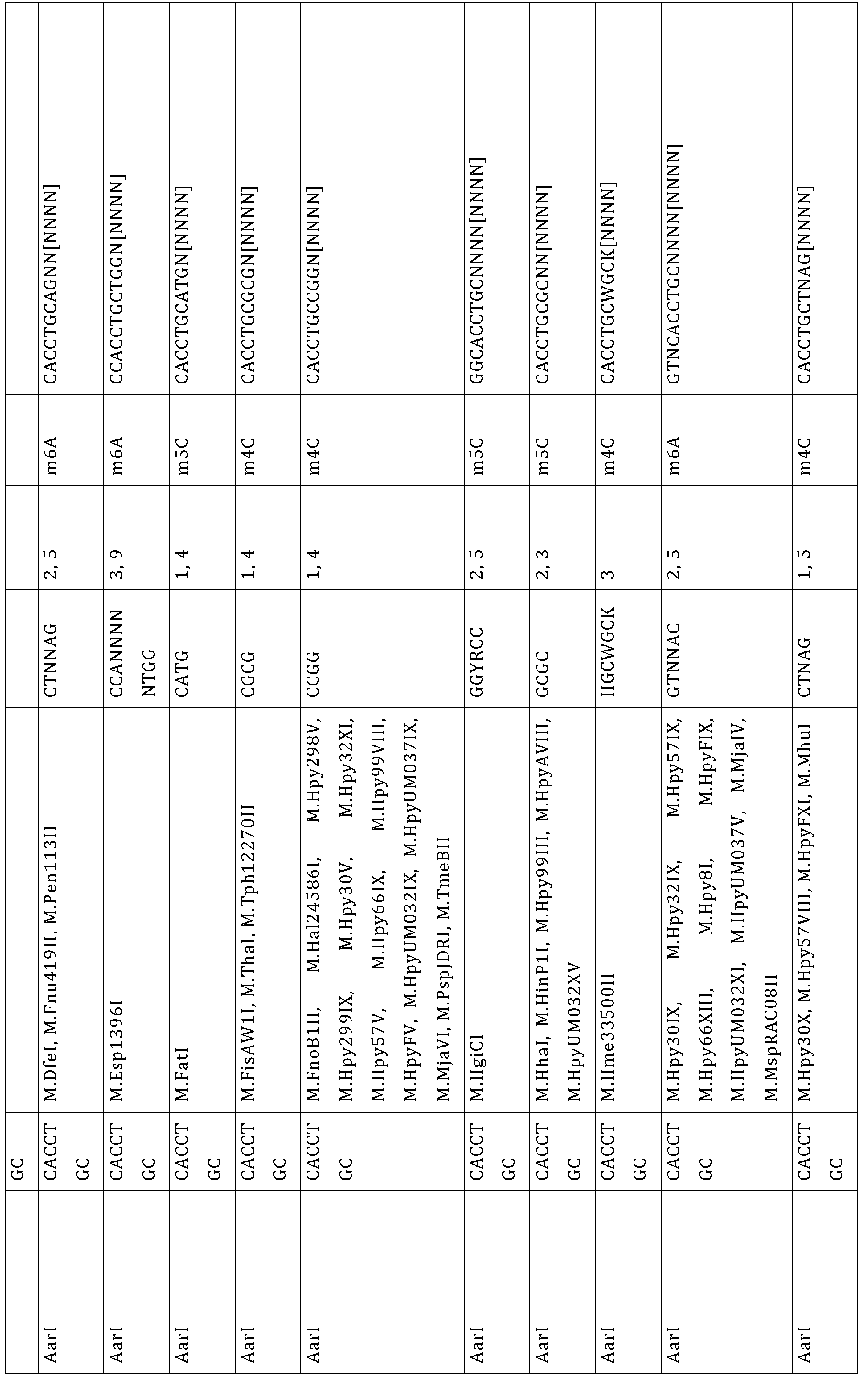

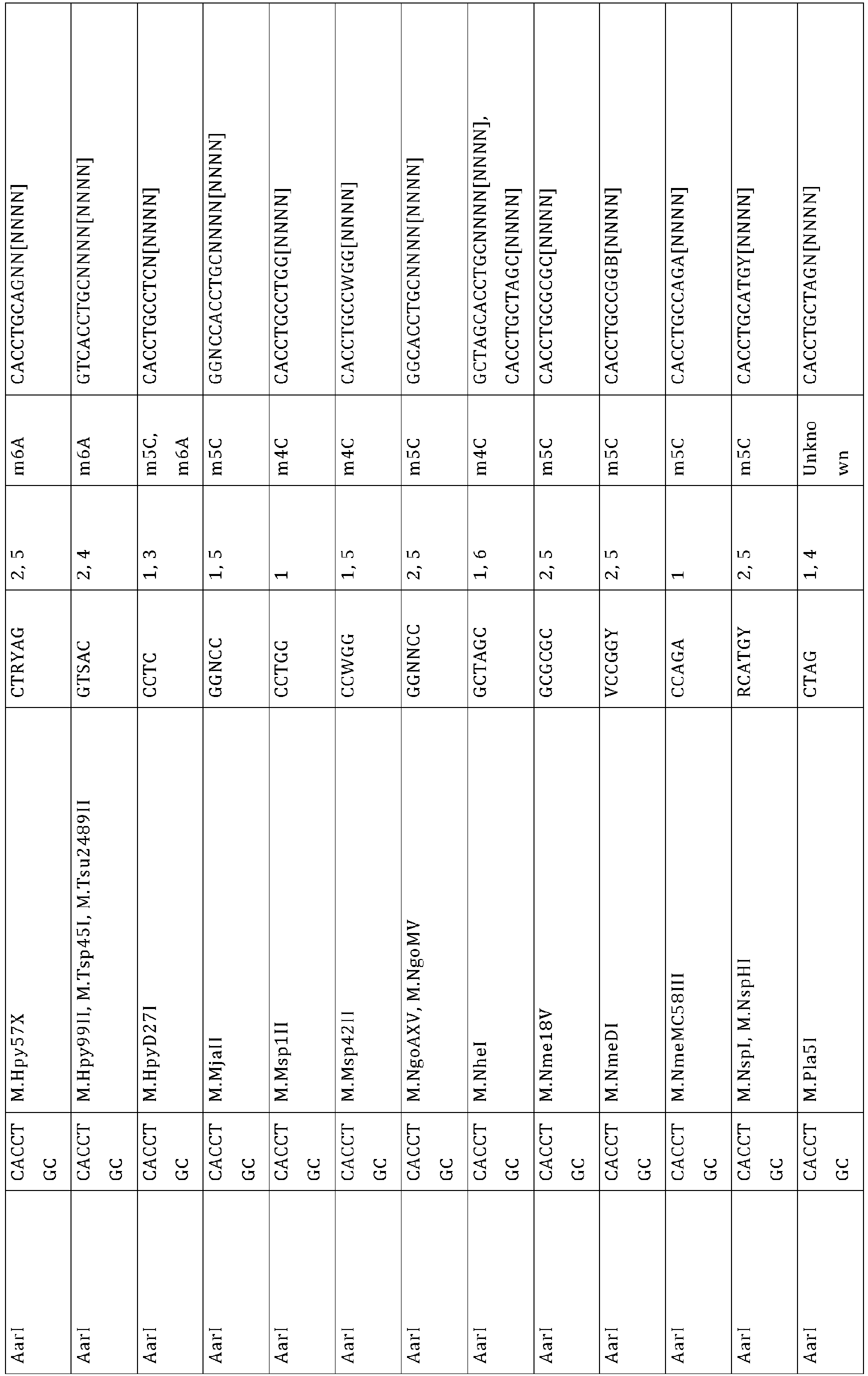

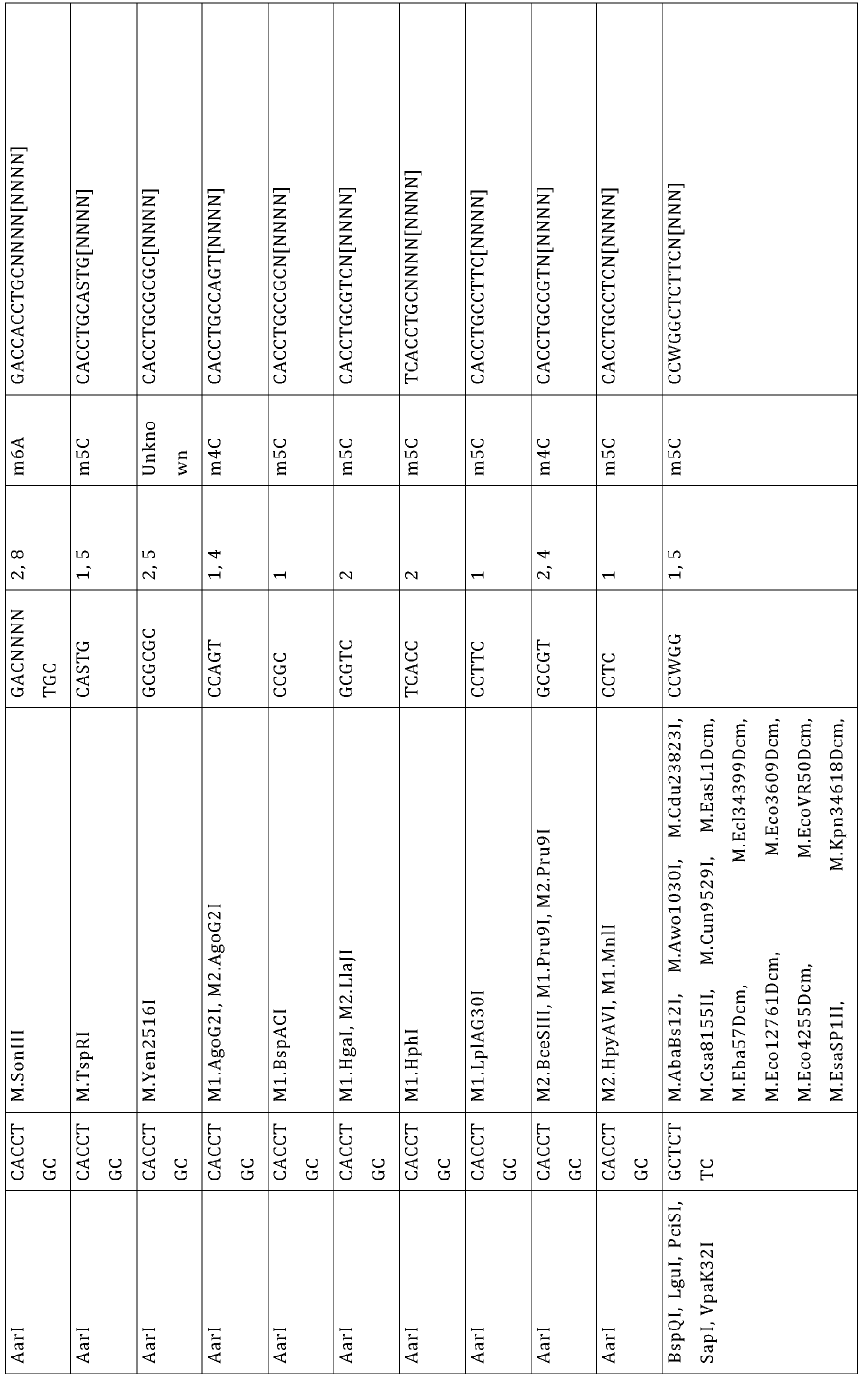

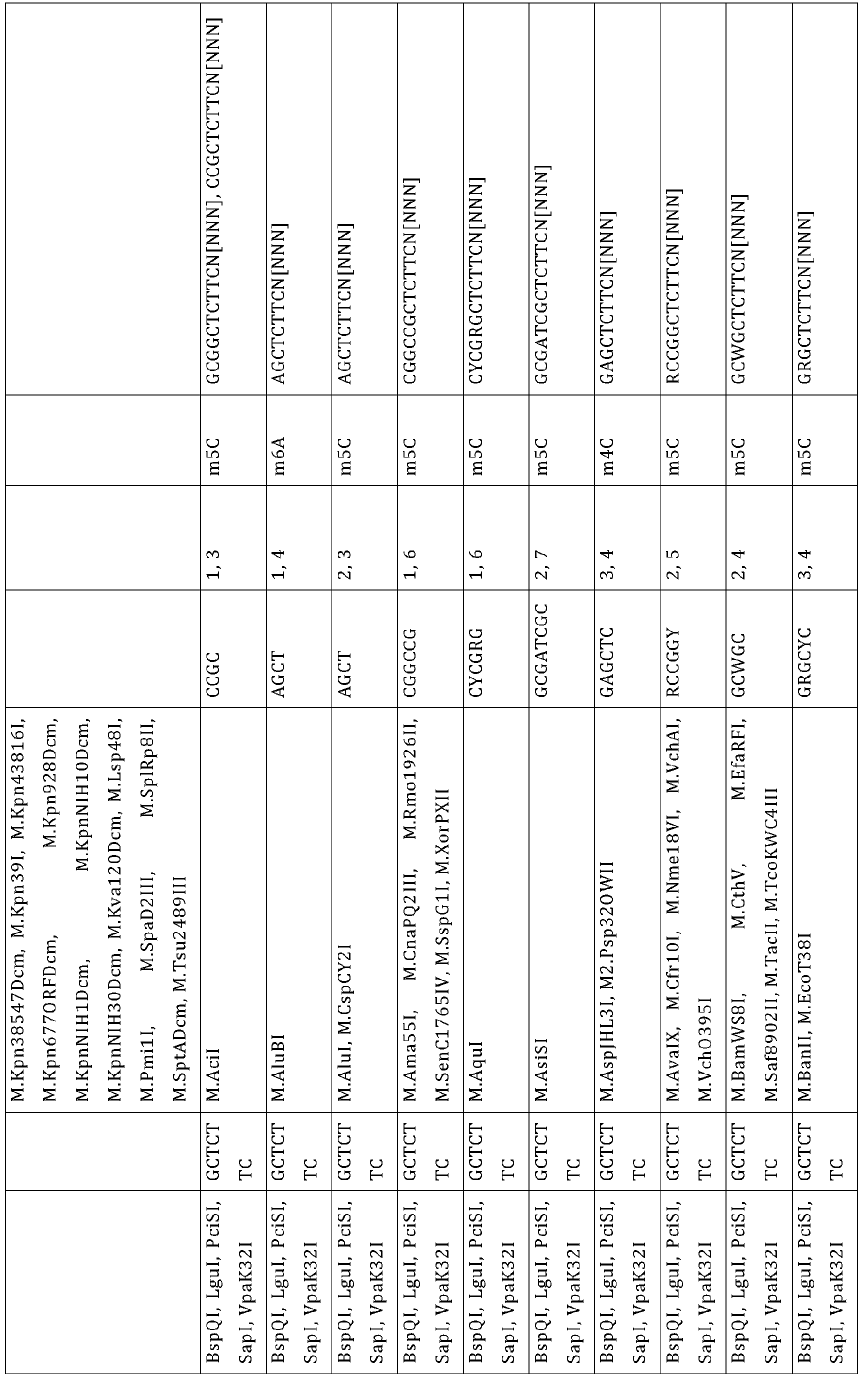

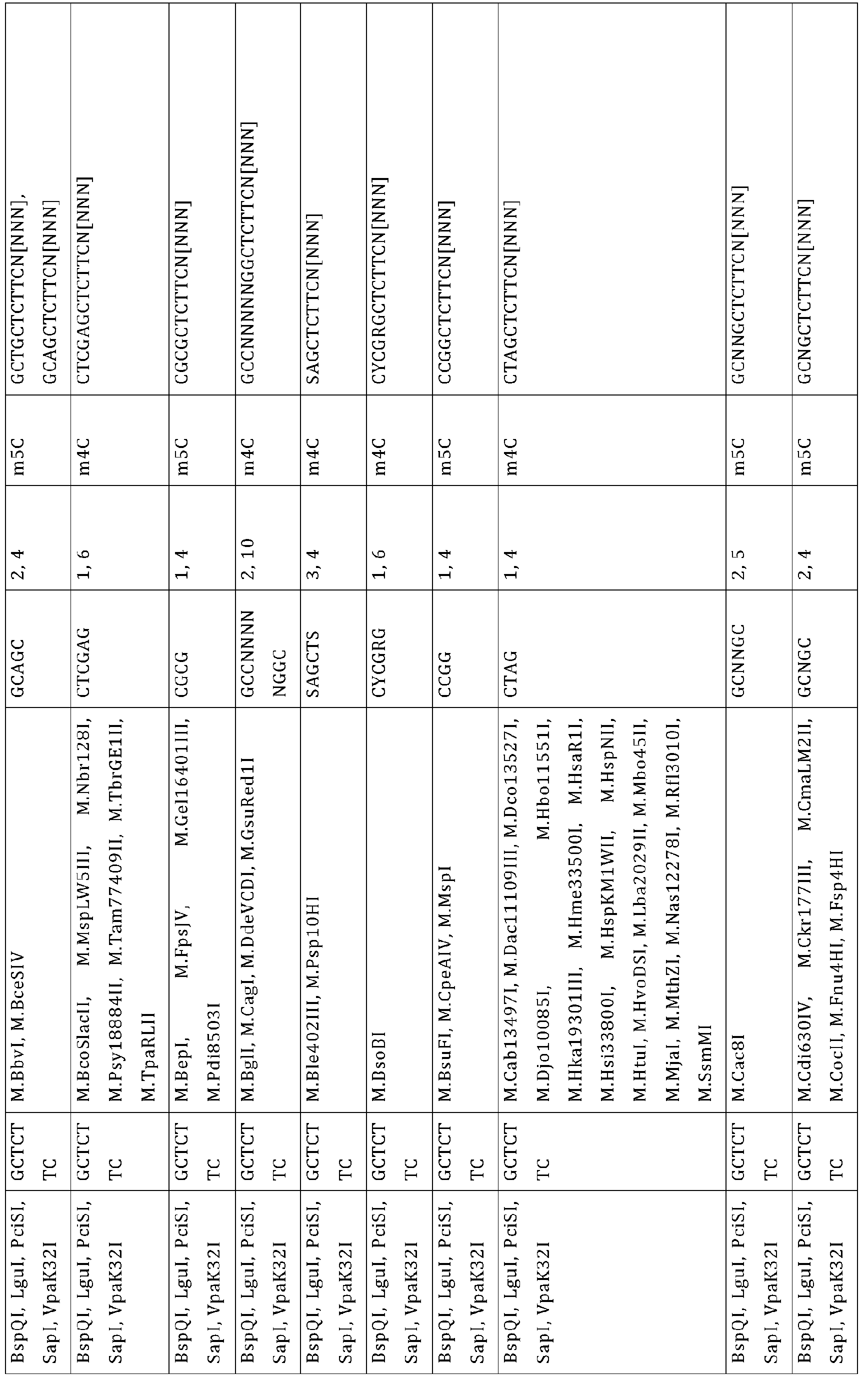

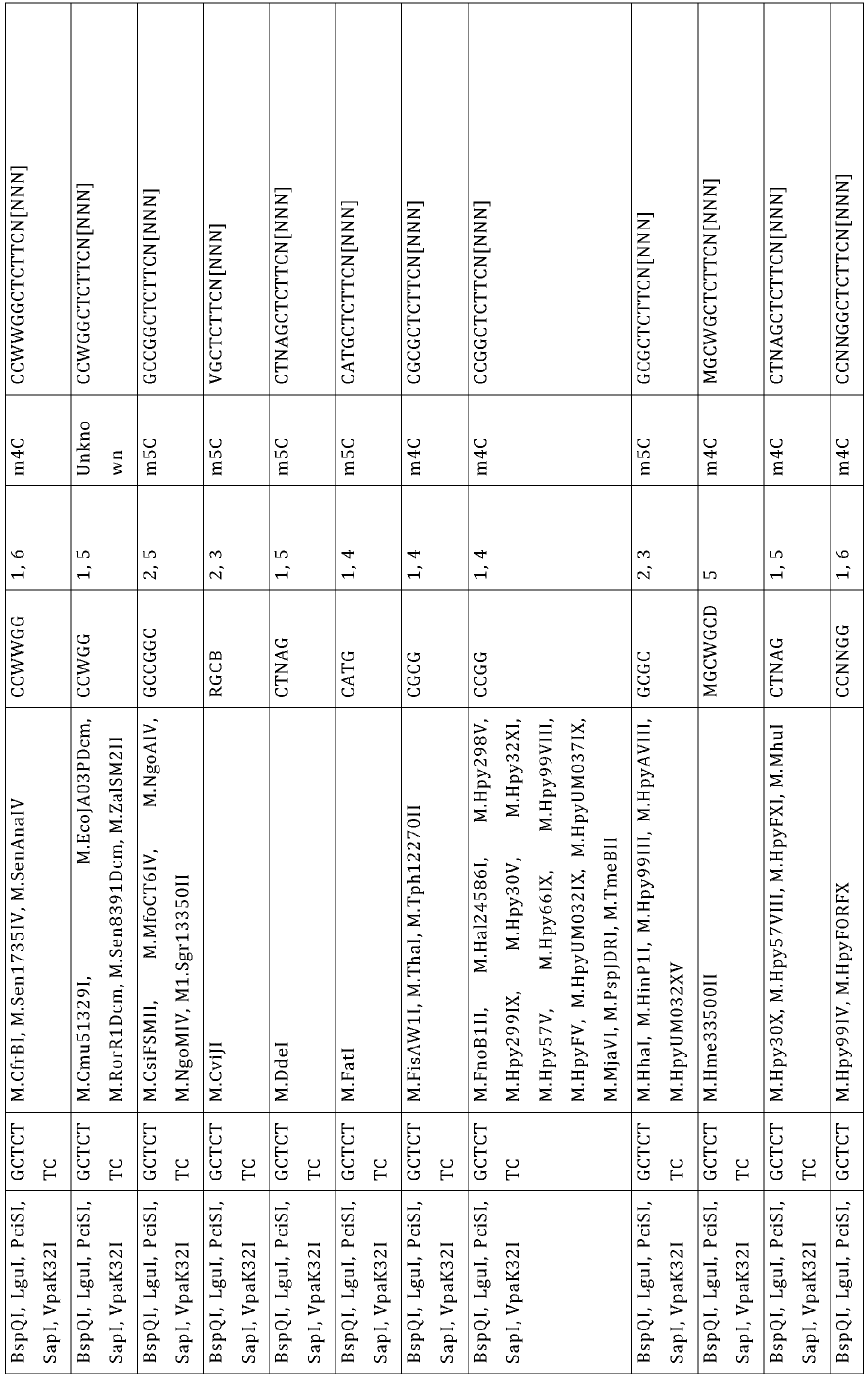

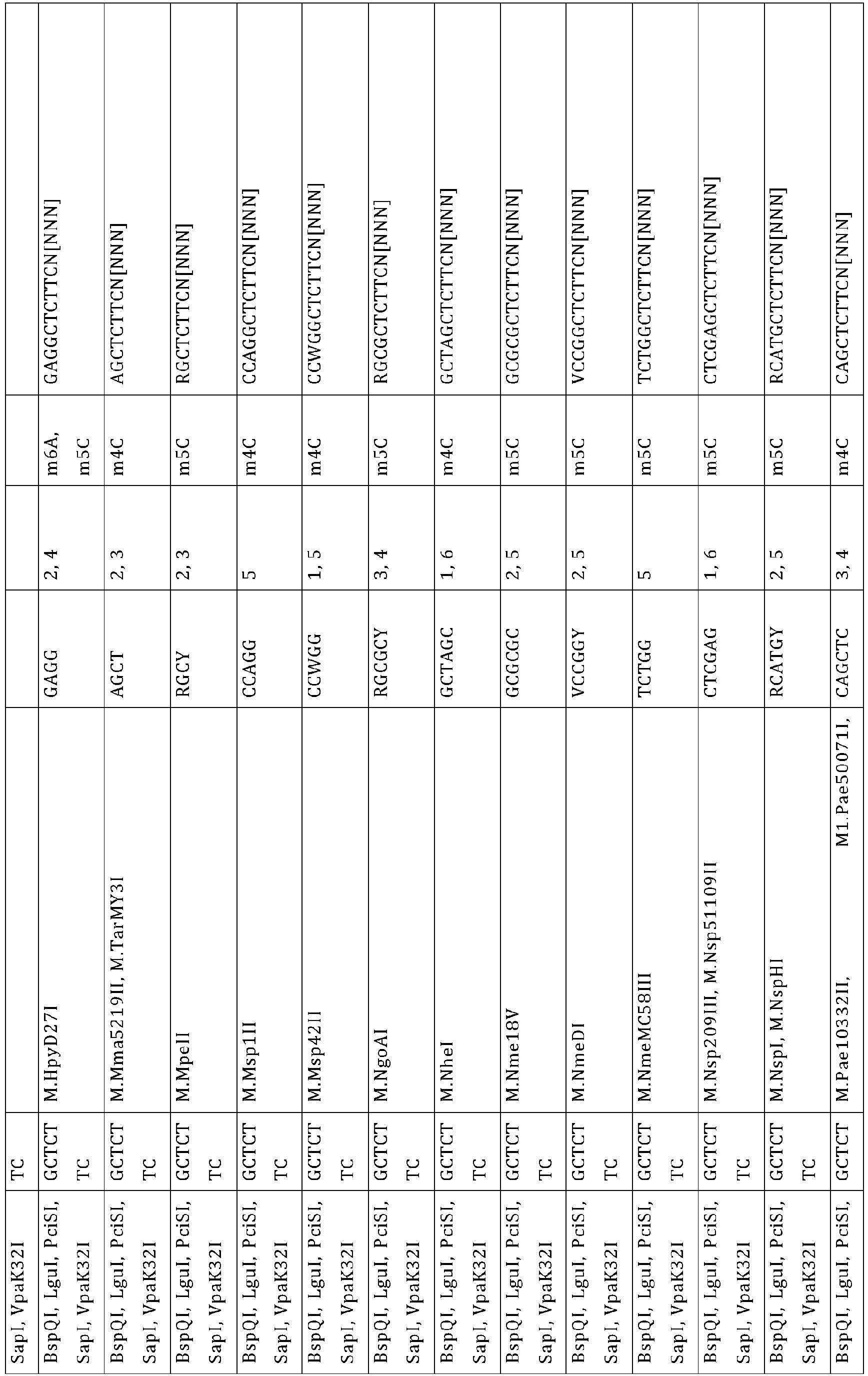

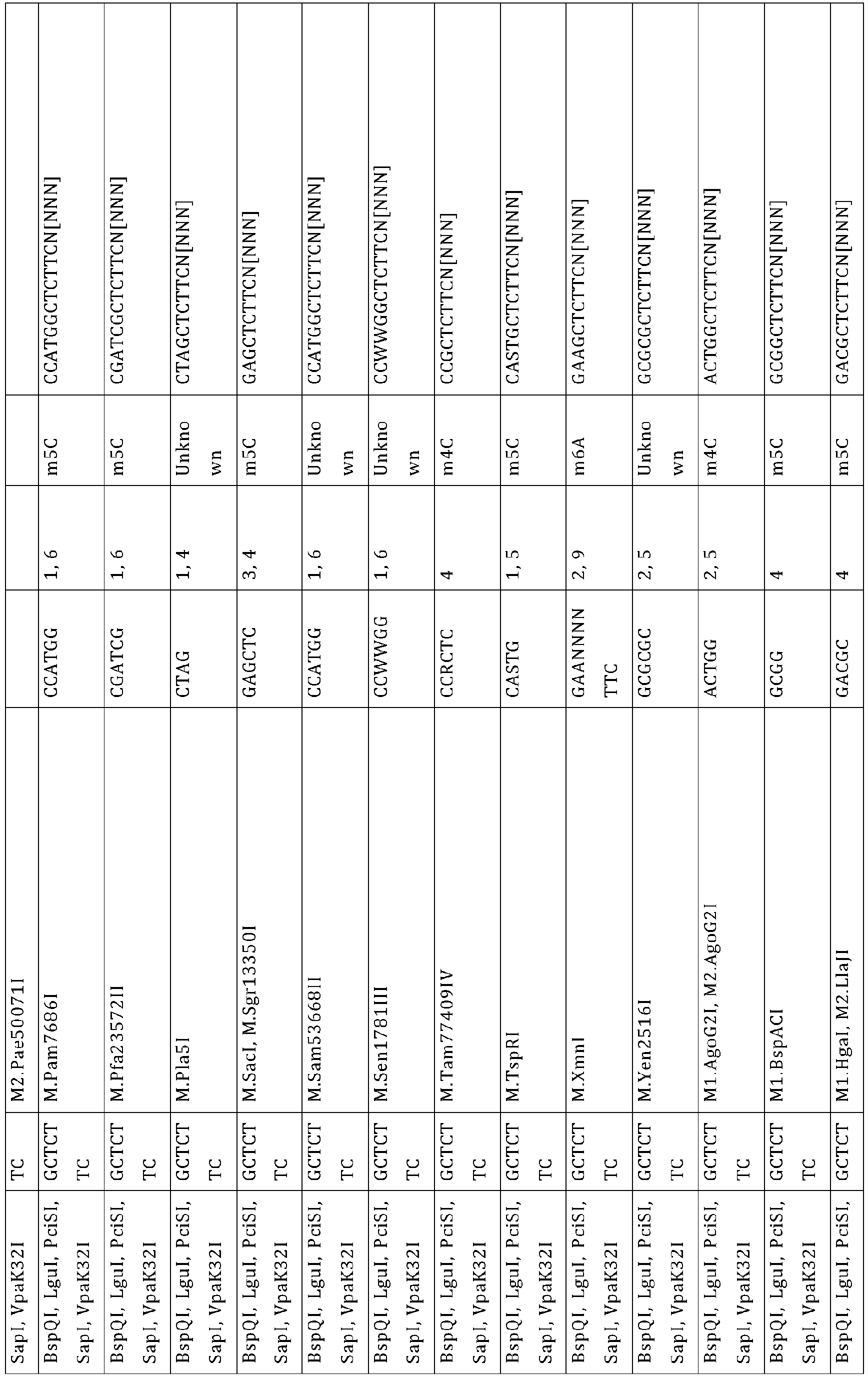

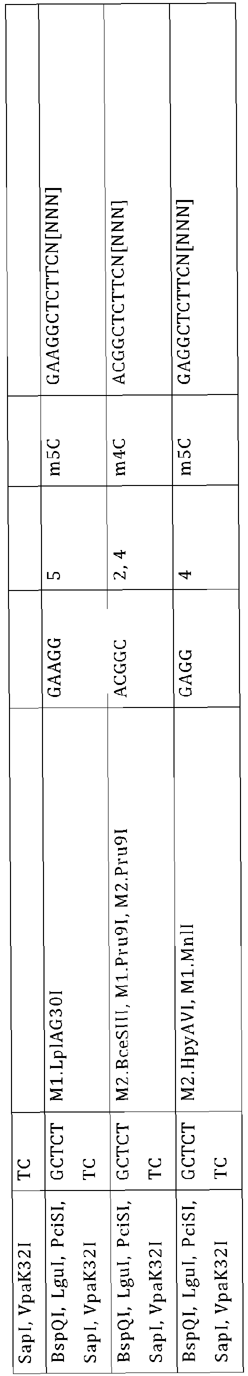

### Use of other types of methyltransferases

Despite using type II DNA methlyase enzymes in the examples, type I restriction-modification enzymes may also be considered. Type I restriction-modification enzymes are multi-subunit enzymes that have both methyltransferase and restriction endonuclease activity. They are usually consists of three types of subunits encoded by three different genes: methylation subunit, restriction subunit and DNA sequence-recognition subunit (specificity subunit). Both the methylation subunit and specificity subunit are required for methyltransferase activity.

For example, M.EcoCI5I is known to recognize G**A**CNNNNNNR**T**AAY (methylated base/opposite base in bold, reverse complementary RTT**A**YNNNNNNG**T**C). A methylation-protectable restriction site according to the description herein could be provided using this sequence to protect an overlapping BsaI site: *RTT**A**T*NNNNNG*G**T**C*TC (methylated base/opposite base in bold, BsaI recognition sequence underlined, M.EcoCI5I recognition sequence in italic). An *E. coli* strain expressing both the methylation subunit M.EcoCI5I and the specificity subunit S.EcoCI5I may be able to methylate the corresponding bases in the cut control element, which would block the methylation-protected BsaI site.

Table 4 provides some further example restriction enzyme and type I restriction-modification enzyme combinations, or recognition sequences thereof, that may be provided in accordance with the description herein. The skilled person will understand that these are examples and not an exhaustive list of potential combinations that woulc be encompassed by the claims.

**Table 4**

| **Restric tion enzym e** | **Restric tion site** | **Type I restricti on-modific ation enzyme** | **Methylation sequence** | **Positio n of methyl ated base** | **Type of methyl ation** | **Overlapping methylation/restriction site** |
|---|---|---|---|---|---|---|
| AlwXI, Bbvl, BseKI, BseXI, Bsp42 3I, Bst12I, Bst71I, BstV1I, GeolCI, Lsp110 9I | GCAGC | M.AspU 41III | GGCANNNNN NTGA | 4, 11 | m6A | GGCAGCNNNNTGAN[NNNN ] |
| AlwXI, Bbvl, BseKI, BseXI, Bsp42 3I, Bst121, Bst711, BstV1I, GeolCl , Lsp110 9I | GCAGC | M.Sth3 01I | GCANNNNNN NTCG | 3, 11 | m6A | GCAGCNNNNNTCG[NNNN] |
| StsI | GGATG | M.Bme D34I | GAGNNNNNN NTGG | 2, 11 | m6A | GAGNNNNGGATGGNNNNN NNNN[NNNN] |
| BpmI, GsuI | CTGGA G | M.Cbal 3I | GAGNNNNNR TGT | 2, 10 | m6A | CTGGAGNNNNNRTGTNNNN N[NN] |
| BsmBI, BstGZS 3I, Esp3I | CGTCT C | M.SmaF 1I | CAYNNNNNNN TCA | 2, 10 | m6A | CAYNNCGTCTCA[NNNN] |
| RdeGB II | ACCCA G | M.Lal21 6I | CCAANNNNN NNNTGC | 4, 13 | m6A | GCACCCAGNNNTTGGNNNN NNNNNNN[NN], CCAACCCAGNNNTGCNNNNN NNNNNNNNN[NN] |

### Example Vector Sequences

The sequences of plasmids for scarless Metclo are listed below in fasta format. The underlined sequence is the "discard sequence" containing the LacZ selection fragment and a replication origin for high copy vector DNA preparation. The boxed sequence is the "recessive/truncating composite element" that contains a "methylation-protectable element" and a "nonprotectable element" arranged head-to-head at appropriate distance for adapter switching. The rest of the sequence is the vector backbone containing a low copy replication origin (pl5A based) and a selection marker (either kanamycin or spectinomycin).

For BsaI/M.Osp807II based scarless Metclo, the plasmids are:
pMXLK_VL, pMXLK_VM, pMXLK_VR (kanamycin)
pMXLS_VL, pMXLS_VM, pMXLS_VR (spectinomycin)

For LguI/M.XmnI based scarless Metclo, the plasmids are:
pMZLK_VL, pMZLK_VM, pMZLK_VR (kanamycin)
pMZLS_VL, pMZLS_VM, pMZLS_VR (spectinomycin)

The sequences of plasmids for standard 4-fragment assembly of 1kb DNA and 2-stage assembly of ∼200kb DNA from 28 fragments are listed in fasta format as well. The underlined sequence for 1kb assembly vectors contain LacZ selection marker. The underlined sequence for 200kb assembly vectors contain LacZ selection marker and a replication origin for high copy vector DNA preparation. The rest of the sequences are vector backbones.

For 1kb assembly, the plasmids are:
pMXP_A4E, pMYP_A5E, pMZP_P6T

For 200kb assembly, the plasmids are:
pMXBG_A7I, pMXBG_I7G, pMXBG_G7F, pMXBG_F7E, pMXBK_A7E

Such vector sequences are applicable for the vectors of the examples herein, with the specific truncating, maintaining or insertional composite elements exchanged as appropriate. A skilled person familiar with molecular cloning can re-construct the vectors herein by PCR from the commercially available Moclo kit.
>pMXLK_VL (SEQ ID NO: 28) 
CACTCTGTGACTTGGTCTCA CTCC TGAGACC
GTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
 
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGAC
GGTCTCA CGAG ACCAAGTCACGAAGTGTGCGGCCGCCTTCGTGCGCGGAAC
CCCTATTTGTTTATTTTTCTAAATACATTCAAATATGTATCCGCTCATGA
GACAATAACCCTGATAAATGCTTCAATAATATTGAAAAAGGAAGAGTATG
GCTAAAATGAGAATATCACCGGAATTGAAAAAACTGATCGAAAAATACCG
CTGCGTAAAAGATACGGAAGGAATGTCTCCTGCTAAGGTATATAAGCTGG
TGGGAGAAAATGAAAACCTATATTTAAAAATGACGGACAGCCGGTATAAA
GGGACCACCTATGATGTGGAACGGGAAAAGGACATGATGCTATGGCTGGA
AGGAAAGCTGCCTGTTCCAAAGGTCCTGCACTTTGAACGGCATGATGGCT
GGAGCAATCTGCTCATGAGTGAGGCCGATGGCGTCCTTTGCTCGGAAGAG
TATGAAGATGAACAAAGCCCTGAAAAGATTATCGAGCTGTATGCGGAGTG
CATCAGGCTCTTTCACTCCATCGACATATCGGATTGTCCCTATACGAATA
GCTTAGACAGCCGCTTAGCCGAATTGGATTACTTACTGAATAACGATCTG
GCCGATGTGGATTGCGAAAACTGGGAAGAGGACACTCCATTTAAAGATCC
GCGCGAGCTGTATGATTTTTTAAAGACGGAAAAGCCCGAAGAGGAACTTG
TCTTTTCCCACGGCGACCTGGGAGACAGCAACATCTTTGTGAAAGATGGC
AAAGTAAGTGGCTTTATTGATCTTGGGAGAAGCGGCAGGGCGGACAAGTG
GTATGACATTGCCTTCTGCGTCCGGTCGCTCAGGGAGGATATCGGGGAAG
AACAGTATGTCGAGCTATTTTTTGACTTACTGGGGATCAAGCCTGATTGG
GAGAAAATAAAATATTATATTTTACTGGATGAATTGTTTTAGGAGCCTGT
TTAATAAGATGATCTTCTTGAGATCGTTTTGGTCTGCGCGTAATCTCTTG
CTCTGAAAACGAAAAAACCGCCTTGCAGGGCGGTTTTTCGAAGGTTCTCT
GAGCTACCAACTCTTTGAACCGAGGTAACTGGCTTGGAGGAGCGCAGTCA
CCAAAACTTGTCCTTTCAGTTTAGCCTTAACCGGCGCATGACTTCAAGAC
TAACTCCTCTAAATCAATTACCAGTGGCTGCTGCCAGTGGTGCTTTTGCA
TGTCTTTCCGGGTTGGACTCAAGACGATAGTTACCGGATAAGGCGCAGCG
GTCGGACTGAACGGGGGGTTCGTGCATACAGTCCAGCTTGGAGCGAACTG
CCTACCCGGAACTGAGTGTCAGGCGTGGAATGAGACAAACGCGGCCATAA
CAGCGGAATGACACCGGTAAACCGAAAGGCAGGAACAGGAGAGCGCACGA
GGGAGCCGCCAGGGGGAAACGCCTGGTATCTTTATAGTCCTGTCGGGTTT
CGCCACCACTGATTTGAGCGTCAGATTTCGTGATGCTTGTCAGGGGGGCG
GAGCCTATGGAAAAACGGCTTTGCCGCGGCCCTCTCACTTCCCTGTTAAG
TATCTTCCTGGCATCTTCCAGGAAATCTCCGCCCCGTTCGTAAGCCATTT
CCGCTCGCCGCAGTCGAACGACCGAGCGTAGCGAGTCAGTGAGCGAGGAA
GCGGAATATATCCTGTATCACATATTCTGCTGACGCACCGGTGCAGCCTT
TTTTCTCCTGCCACATGAAGCACTTCACTGACACCCTCATCAGTGCCAAC
ATAGTAAGCCAGTATACACTCCGCTAGCGCGCAAGCGGCCGCA
>pMXLK_VM (SEQ ID NO: 29) 
CACTCTGTGACTTGGT CTCC TGAGACC
GTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGAC
GGTCTCA CGAG ACCAAGTCACGAAGTGTGCGGCCGCCTTCGTGCGCGGAAC
CCCTATTTGTTTATTTTTCTAAATACATTCAAATATGTATCCGCTCATGA
GACAATAACCCTGATAAATGCTTCAATAATATTGAAAAAGGAAGAGTATG
GCTAAAATGAGAATATCACCGGAATTGAAAAAACTGATCGAAAAATACCG
CTGCGTAAAAGATACGGAAGGAATGTCTCCTGCTAAGGTATATAAGCTGG
TGGGAGAAAATGAAAACCTATATTTAAAAATGACGGACAGCCGGTATAAA
GGGACCACCTATGATGTGGAACGGGAAAAGGACATGATGCTATGGCTGGA
AGGAAAGCTGCCTGTTCCAAAGGTCCTGCACTTTGAACGGCATGATGGCT
GGAGCAATCTGCTCATGAGTGAGGCCGATGGCGTCCTTTGCTCGGAAGAG
TATGAAGATGAACAAAGCCCTGAAAAGATTATCGAGCTGTATGCGGAGTG
CATCAGGCTCTTTCACTCCATCGACATATCGGATTGTCCCTATACGAATA
GCTTAGACAGCCGCTTAGCCGAATTGGATTACTTACTGAATAACGATCTG
GCCGATGTGGATTGCGAAAACTGGGAAGAGGACACTCCATTTAAAGATCC
GCGCGAGCTGTATGATTTTTTAAAGACGGAAAAGCCCGAAGAGGAACTTG
TCTTTTCCCACGGCGACCTGGGAGACAGCAACATCTTTGTGAAAGATGGC
AAAGTAAGTGGCTTTATTGATCTTGGGAGAAGCGGCAGGGCGGACAAGTG
GTATGACATTGCCTTCTGCGTCCGGTCGCTCAGGGAGGATATCGGGGAAG
AACAGTATGTCGAGCTATTTTTTGACTTACTGGGGATCAAGCCTGATTGG
 
GAGAAAATAAAATATTATATTTTACTGGATGAATTGTTTTAGGAGCCTGT
TTAATAAGATGATCTTCTTGAGATCGTTTTGGTCTGCGCGTAATCTCTTG
CTCTGAAAACGAAAAAACCGCCTTGCAGGGCGGTTTTTCGAAGGTTCTCT
GAGCTACCAACTCTTTGAACCGAGGTAACTGGCTTGGAGGAGCGCAGTCA
CCAAAACTTGTCCTTTCAGTTTAGCCTTAACCGGCGCATGACTTCAAGAC
TAACTCCTCTAAATCAATTACCAGTGGCTGCTGCCAGTGGTGCTTTTGCA
TGTCTTTCCGGGTTGGACTCAAGACGATAGTTACCGGATAAGGCGCAGCG
GTCGGACTGAACGGGGGGTTCGTGCATACAGTCCAGCTTGGAGCGAACTG
CCTACCCGGAACTGAGTGTCAGGCGTGGAATGAGACAAACGCGGCCATAA
CAGCGGAATGACACCGGTAAACCGAAAGGCAGGAACAGGAGAGCGCACGA
GGGAGCCGCCAGGGGGAAACGCCTGGTATCTTTATAGTCCTGTCGGGTTT
CGCCACCACTGATTTGAGCGTCAGATTTCGTGATGCTTGTCAGGGGGGCG
GAGCCTATGGAAAAACGGCTTTGCCGCGGCCCTCTCACTTCCCTGTTAAG
TATCTTCCTGGCATCTTCCAGGAAATCTCCGCCCCGTTCGTAAGCCATTT
CCGCTCGCCGCAGTCGAACGACCGAGCGTAGCGAGTCAGTGAGCGAGGAA
GCGGAATATATCCTGTATCACATATTCTGCTGACGCACCGGTGCAGCCTT
TTTTCTCCTGCCACATGAAGCACTTCACTGACACCCTCATCAGTGCCAAC
ATAGTAAGCCAGTATACACTCCGCTAGCGCGCAAGCGGCCGCA
>pMXLK_VR (SEQ ID NO: 30) 
CACTCTGTGACTTGGT CTCC TGAGACC
GTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
 
TGACGGGCTTGTCTGCTCCCGGCATCCGCTTACAGACAAGCTGTGAC
GGTCTCA CGAG TGAGACCAAGTCACGAAGTGTGCGGCCGCCTTCGTGCGCGGAAC
CCCTATTTGTTTATTTTTCTAAATACATTCAAATATGTATCCGCTCATGA
GACAATAACCCTGATAAATGCTTCAATAATATTGAAAAAGGAAGAGTATG
GCTAAAATGAGAATATCACCGGAATTGAAAAAACTGATCGAAAAATACCG
CTGCGTAAAAGATACGGAAGGAATGTCTCCTGCTAAGGTATATAAGCTGG
TGGGAGAAAATGAAAACCTATATTTAAAAATGACGGACAGCCGGTATAAA
GGGACCACCTATGATGTGGAACGGGAAAAGGACATGATGCTATGGCTGGA
AGGAAAGCTGCCTGTTCCAAAGGTCCTGCACTTTGAACGGCATGATGGCT
GGAGCAATCTGCTCATGAGTGAGGCCGATGGCGTCCTTTGCTCGGAAGAG
TATGAAGATGAACAAAGCCCTGAAAAGATTATCGAGCTGTATGCGGAGTG
CATCAGGCTCTTTCACTCCATCGACATATCGGATTGTCCCTATACGAATA
GCTTAGACAGCCGCTTAGCCGAATTGGATTACTTACTGAATAACGATCTG
GCCGATGTGGATTGCGAAAACTGGGAAGAGGACACTCCATTTAAAGATCC
GCGCGAGCTGTATGATTTTTTAAAGACGGAAAAGCCCGAAGAGGAACTTG
TCTTTTCCCACGGCGACCTGGGAGACAGCAACATCTTTGTGAAAGATGGC
AAAGTAAGTGGCTTTATTGATCTTGGGAGAAGCGGCAGGGCGGACAAGTG
GTATGACATTGCCTTCTGCGTCCGGTCGCTCAGGGAGGATATCGGGGAAG
AACAGTATGTCGAGCTATTTTTTGACTTACTGGGGATCAAGCCTGATTGG
GAGAAAATAAAATATTATATTTTACTGGATGAATTGTTTTAGGAGCCTGT
TTAATAAGATGATCTTCTTGAGATCGTTTTGGTCTGCGCGTAATCTCTTG
CTCTGAAAACGAAAAAACCGCCTTGCAGGGCGGTTTTTCGAAGGTTCTCT
GAGCTACCAACTCTTTGAACCGAGGTAACTGGCTTGGAGGAGCGCAGTCA
CCAAAACTTGTCCTTTCAGTTTAGCCTTAACCGGCGCATGACTTCAAGAC
TAACTCCTCTAAATCAATTACCAGTGGCTGCTGCCAGTGGTGCTTTTGCA
TGTCTTTCCGGGTTGGACTCAAGACGATAGTTACCGGATAAGGCGCAGCG
GTCGGACTGAACGGGGGGTTCGTGCATACAGTCCAGCTTGGAGCGAACTG
CCTACCCGGAACTGAGTGTCAGGCGTGGAATGAGACAAACGCGGCCATAA
CAGCGGAATGACACCGGTAAACCGAAAGGCAGGAACAGGAGAGCGCACGA
GGGAGCCGCCAGGGGGAAACGCCTGGTATCTTTATAGTCCTGTCGGGTTT
CGCCACCACTGATTTGAGCGTCAGATTTCGTGATGCTTGTCAGGGGGGCG
GAGCCTATGGAAAAACGGCTTTGCCGCGGCCCTCTCACTTCCCTGTTAAG
TATCTTCCTGGCATCTTCCAGGAAATCTCCGCCCCGTTCGTAAGCCATTT
CCGCTCGCCGCAGTCGAACGACCGAGCGTAGCGAGTCAGTGAGCGAGGAA
GCGGAATATATCCTGTATCACATATTCTGCTGACGCACCGGTGCAGCCTT
TTTTCTCCTGCCACATGAAGCACTTCACTGACACCCTCATCAGTGCCAAC
ATAGTAAGCCAGTATACACTCCGCTAGCGCGCAAGCGGCCGCA
>pMZLK_VL (SEQ ID NO: 31) 
CACTCTGT GAAGCTCTTCA TCC TGAAGAGC
GGTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
 
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGACC
GCTCTTCA CGA AGAGCTTC ACGAAGTGTGCGGCCGCCTTCGTGCGGAAC
CCCTATTTGTTTATTTTTCTAAATACATTCAAATATGTATCCGCTCATGA
GACAATAACCCTGATAAATGCTTCAATAATATTGAAAAAGGAAGAGTATG
GCTAAAATGAGAATATCACCGGAATTGAAAAAACTGATCGAAAAATACCG
CTGCGTAAAAGATACGGAAGGAATGTCTCCTGCTAAGGTATATAAGCTGG
TGGGAGAAAATGAAAACCTATATTTAAAAATGACGGACAGCCGGTATAAA
GGGACCACCTATGATGTGGAACGGGAAAAGGACATGATGCTATGGCTGGA
AGGAAAGCTGCCTGTTCCAAAGGTCCTGCACTTTGAACGGCATGATGGCT
GGAGCAATCTGCTCATGAGTGAGGCCGATGGCGTCCTTTGCTCGGAAGAG
TATGAAGATGAACAAAGCCCTGAAAAGATTATCGAGCTGTATGCGGAGTG
CATCAGGCTCTTTCACTCCATCGACATATCGGATTGTCCCTATACGAATA
GCTTAGACAGCCGCTTAGCCGAATTGGATTACTTACTGAATAACGATCTG
GCCGATGTGGATTGCGAAAACTGGGAAGAGGACACTCCATTTAAAGATCC
GCGCGAGCTGTATGATTTTTTAAAGACGGAAAAGCCCGAAGAGGAACTTG
TCTTTTCCCACGGCGACCTGGGAGACAGCAACATCTTTGTGAAAGATGGC
AAAGTAAGTGGCTTTATTGATCTTGGGAGAAGCGGCAGGGCGGACAAGTG
GTATGACATTGCCTTCTGCGTCCGGTCGCTCAGGGAGGATATCGGGGAAG
AACAGTATGTCGAGCTATTTTTTGACTTACTGGGGATCAAGCCTGATTGG
GAGAAAATAAAATATTATATTTTACTGGATGAATTGTTTTAGGAGCCTGT
TTAATAAGATGATCTTCTTGAGATCGTTTTGGTCTGCGCGTAATCTCTTG
CTCTGAAAACGAAAAAACCGCCTTGCAGGGCGGTTTTTCGAAGGTTCTCT
GAGCTACCAACTCTTTGAACCGAGGTAACTGGCTTGGAGGAGCGCAGTCA
CCAAAACTTGTCCTTTCAGTTTAGCCTTAACCGGCGCATGACTTCAAGAC
TAACTCCTCTAAATCAATTACCAGTGGCTGCTGCCAGTGGTGCTTTTGCA
TGTCTTTCCGGGTTGGACTCAAGACGATAGTTACCGGATAAGGCGCAGCG
GTCGGACTGAACGGGGGGTTCGTGCATACAGTCCAGCTTGGAGCGAACTG
CCTACCCGGAACTGAGTGTCAGGCGTGGAATGAGACAAACGCGGCCATAA
CAGCGGAATGACACCGGTAAACCGAAAGGCAGGAACAGGAGAGCGCACGA
GGGAGCCGCCAGGGGGAAACGCCTGGTATCTTTATAGTCCTGTCGGGTTT
CGCCACCACTGATTTGAGCGTCAGATTTCGTGATGCTTGTCAGGGGGGCG
GAGCCTATGGAAAAACGGCTTTGCCGCGGCCCTCTCACTTCCCTGTTAAG
TATCTTCCTGGCATCTTCCAGGAAATCTCCGCCCCGTTCGTAAGCCATTT
CCGCTCGCCGCAGTCGAACGACCGAGCGTAGCGAGTCAGTGAGCGAGGAA
GCGGAATATATCCTGTATCACATATTCTGCTGACGCACCGGTGCAGCCTT
 
TTTTCTCCTGCCACATGAAGCACTTCACTGACACCCTCATCAGTGCCAAC
ATAGTAAGCCAGTATACACTCCGCTAGCGCGCAAGCGGCCGCA
>pMZLK_VM (SEQ ID NO: 32) 
CACTCTGT GAAGCTCT TCC TGAAGAGC
GGTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGACC
GCTCTTCA CGA AGAGCTTC ACGAAGTGTGCGGCCGCCTTCGTGCGCGGAAC
CCCTATTTGTTTATTTTTCTAAATACATTCAAATATGTATCCGCTCATGA
GACAATAACCCTGATAAATGCTTCAATAATATTGAAAAAGGAAGAGTATG
GCTAAAATGAGAATATCACCGGAATTGAAAAAACTGATCGAAAAATACCG
CTGCGTAAAAGATACGGAAGGAATGTCTCCTGCTAAGGTATATAAGCTGG
TGGGAGAAAATGAAAACCTATATTTAAAAATGACGGACAGCCGGTATAAA
GGGACCACCTATGATGTGGAACGGGAAAAGGACATGATGCTATGGCTGGA
AGGAAAGCTGCCTGTTCCAAAGGTCCTGCACTTTGAACGGCATGATGGCT
GGAGCAATCTGCTCATGAGTGAGGCCGATGGCGTCCTTTGCTCGGAAGAG
TATGAAGATGAACAAAGCCCTGAAAAGATTATCGAGCTGTATGCGGAGTG
CATCAGGCTCTTTCACTCCATCGACATATCGGATTGTCCCTATACGAATA
GCTTAGACAGCCGCTTAGCCGAATTGGATTACTTACTGAATAACGATCTG
GCCGATGTGGATTGCGAAAACTGGGAAGAGGACACTCCATTTAAAGATCC
GCGCGAGCTGTATGATTTTTTAAAGACGGAAAAGCCCGAAGAGGAACTTG
TCTTTTCCCACGGCGACCTGGGAGACAGCAACATCTTTGTGAAAGATGGC
AAAGTAAGTGGCTTTATTGATCTTGGGAGAAGCGGCAGGGCGGACAAGTG
 
GTATGACATTGCCTTCTGCGTCCGGTCGCTCAGGGAGGATATCGGGGAAG
AACAGTATGTCGAGCTATTTTTTGACTTACTGGGGATCAAGCCTGATTGG
GAGAAAATAAAATATTATATTTTACTGGATGAATTGTTTTAGGAGCCTGT
TTAATAAGATGATCTTCTTGAGATCGTTTTGGTCTGCGCGTAATCTCTTG
CTCTGAAAACGAAAAAACCGCCTTGCAGGGCGGTTTTTCGAAGGTTCTCT
GAGCTACCAACTCTTTGAACCGAGGTAACTGGCTTGGAGGAGCGCAGTCA
CCAAAACTTGTCCTTTCAGTTTAGCCTTAACCGGCGCATGACTTCAAGAC
TAACTCCTCTAAATCAATTACCAGTGGCTGCTGCCAGTGGTGCTTTTGCA
TGTCTTTCCGGGTTGGACTCAAGACGATAGTTACCGGATAAGGCGCAGCG
GTCGGACTGAACGGGGGGTTCGTGCATACAGTCCAGCTTGGAGCGAACTG
CCTACCCGGAACTGAGTGTCAGGCGTGGAATGAGACAAACGCGGCCATAA
CAGCGGAATGACACCGGTAAACCGAAAGGCAGGAACAGGAGAGCGCACGA
GGGAGCCGCCAGGGGGAAACGCCTGGTATCTTTATAGTCCTGTCGGGTTT
CGCCACCACTGATTTGAGCGTCAGATTTCGTGATGCTTGTCAGGGGGGCG
GAGCCTATGGAAAAACGGCTTTGCCGCGGCCCTCTCACTTCCCTGTTAAG
TATCTTCCTGGCATCTTCCAGGAAATCTCCGCCCCGTTCGTAAGCCATTT
CCGCTCGCCGCAGTCGAACGACCGAGCGTAGCGAGTCAGTGAGCGAGGAA
GCGGAATATATCCTGTATCACATATTCTGCTGACGCACCGGTGCAGCCTT
TTTTCTCCTGCCACATGAAGCACTTCACTGACACCCTCATCAGTGCCAAC
ATAGTAAGCCAGTATACACTCCGCTAGCGCGCAAGCGGCCGCA
>pMZLK_VR (SEQ ID NO: 33) 
CACTCTGT GAAGCTCT TCC TGAAGAGC
GGTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
 
CCGCATAGTTAAGCCAGCCCCGACACCCGCCAACACCCGCTGACGCGCCC
TGACGGGCTTGTCTGCTCCCGGCATCCGCTTACAGACAAGCTGTGACC
GCTCTTCA CGA TGAAGAGCTTC ACGAAGTGTGCGGCCGCCTTCGTGCGCGGAAC
CCCTATTTGTTTATTTTTCTAAATACATTCAAATATGTATCCGCTCATGA
GACAATAACCCTGATAAATGCTTCAATAATATTGAAAAAGGAAGAGTATG
GCTAAAATGAGAATATCACCGGAATTGAAAAAACTGATCGAAAAATACCG
CTGCGTAAAAGATACGGAAGGAATGTCTCCTGCTAAGGTATATAAGCTGG
TGGGAGAAAATGAAAACCTATATTTAAAAATGACGGACAGCCGGTATAAA
GGGACCACCTATGATGTGGAACGGGAAAAGGACATGATGCTATGGCTGGA
AGGAAAGCTGCCTGTTCCAAAGGTCCTGCACTTTGAACGGCATGATGGCT
GGAGCAATCTGCTCATGAGTGAGGCCGATGGCGTCCTTTGCTCGGAAGAG
TATGAAGATGAACAAAGCCCTGAAAAGATTATCGAGCTGTATGCGGAGTG
CATCAGGCTCTTTCACTCCATCGACATATCGGATTGTCCCTATACGAATA
GCTTAGACAGCCGCTTAGCCGAATTGGATTACTTACTGAATAACGATCTG
GCCGATGTGGATTGCGAAAACTGGGAAGAGGACACTCCATTTAAAGATCC
GCGCGAGCTGTATGATTTTTTAAAGACGGAAAAGCCCGAAGAGGAACTTG
TCTTTTCCCACGGCGACCTGGGAGACAGCAACATCTTTGTGAAAGATGGC
AAAGTAAGTGGCTTTATTGATCTTGGGAGAAGCGGCAGGGCGGACAAGTG
GTATGACATTGCCTTCTGCGTCCGGTCGCTCAGGGAGGATATCGGGGAAG
AACAGTATGTCGAGCTATTTTTTGACTTACTGGGGATCAAGCCTGATTGG
GAGAAAATAAAATATTATATTTTACTGGATGAATTGTTTTAGGAGCCTGT
TTAATAAGATGATCTTCTTGAGATCGTTTTGGTCTGCGCGTAATCTCTTG
CTCTGAAAACGAAAAAACCGCCTTGCAGGGCGGTTTTTCGAAGGTTCTCT
GAGCTACCAACTCTTTGAACCGAGGTAACTGGCTTGGAGGAGCGCAGTCA
CCAAAACTTGTCCTTTCAGTTTAGCCTTAACCGGCGCATGACTTCAAGAC
TAACTCCTCTAAATCAATTACCAGTGGCTGCTGCCAGTGGTGCTTTTGCA
TGTCTTTCCGGGTTGGACTCAAGACGATAGTTACCGGATAAGGCGCAGCG
GTCGGACTGAACGGGGGGTTCGTGCATACAGTCCAGCTTGGAGCGAACTG
CCTACCCGGAACTGAGTGTCAGGCGTGGAATGAGACAAACGCGGCCATAA
CAGCGGAATGACACCGGTAAACCGAAAGGCAGGAACAGGAGAGCGCACGA
GGGAGCCGCCAGGGGGAAACGCCTGGTATCTTTATAGTCCTGTCGGGTTT
CGCCACCACTGATTTGAGCGTCAGATTTCGTGATGCTTGTCAGGGGGGCG
GAGCCTATGGAAAAACGGCTTTGCCGCGGCCCTCTCACTTCCCTGTTAAG
TATCTTCCTGGCATCTTCCAGGAAATCTCCGCCCCGTTCGTAAGCCATTT
CCGCTCGCCGCAGTCGAACGACCGAGCGTAGCGAGTCAGTGAGCGAGGAA
GCGGAATATATCCTGTATCACATATTCTGCTGACGCACCGGTGCAGCCTT
TTTTCTCCTGCCACATGAAGCACTTCACTGACACCCTCATCAGTGCCAAC
ATAGTAAGCCAGTATACACTCCGCTAGCGCGCAAGCGGCCGCA
>pMXLS_VL (SEQ ID NO: 34) 
CACTCTGTGACTTGGTCTCA CTCC TGAGACC

GTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
 
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGAC

GGTCTCA CGAG ACCAAGTCACGAAGTG TGCGGCCGCCTTCG
TGCGCGGAACCCCTATTTGTTTATTTTTCTAAATACATTCAAATATGTAT
CCGCTCATGAGACAATAACCCTGATAAATGCTTCAATAATATTGAAAAAG
GAAGAGTATGCGCTCACGCAACTGGTCCAGAACCTTGACCGAACGCAGCG
GTGGTAACGGCGCAGTGGCGGTTTTCATGGCTTGTTATGACTGTTTTTTT
GGGGTACAGTCTATGCCTCGGGCATCCAAGCAGCAAGCGCGTTACGCCGT
GGGTCGATGTTTGATGTTATGGAGCAGCAACGATGTTACGCAGCAGGGCA
GTCGCCCTAAAACAAAGTTAAACATCATGAGGGAAGCGGTGATCGCCGAA
GTATCGACTCAACTATCAGAGGTAGTTGGCGTCATCGAGCGCCATCTCGA
ACCGACGTTGCTGGCCGTACATTTGTACGGCTCCGCAGTGGATGGCGGCC
TGAAGCCACACAGCGATATTGATTTGCTGGTTACGGTGACCGTAAGGCTT
GATGAAACAACGCGGCGAGCTTTGATCAACGACCTTTTGGAAACTTCGGC
TTCCCCTGGAGAGAGCGAGATTCTCCGCGCTGTAGAAGTCACCATTGTTG
TGCACGACGACATCATTCCGTGGCGTTATCCAGCTAAGCGCGAACTGCAA
TTTGGAGAATGGCAGCGCAATGACATTCTTGCAGGTATCTTCGAGCCAGC
CACGATCGACATTGATCTGGCTATCTTGCTGACAAAAGCAAGAGAACATA
GCGTTGCCTTGGTAGGTCCAGCGGCGGAGGAACTCTTTGATCCGGTTCCT
GAACAGGATCTATTTGAGGCGCTAAATGAAACCTTAACGCTATGGAACTC
GCCGCCCGACTGGGCTGGCGATGAGCGAAATGTAGTGCTTACGTTGTCCC
GCATTTGGTACAGCGCAGTAACCGGCAAAATCGCGCCGAAGGATGTCGCT
GCCGACTGGGCAATGGAGCGCCTGCCGGCCCAGTATCAGCCCGTCATACT
TGAAGCTAGACAGGCTTATCTTGGACAAGAAGATCGCTTGGCCTCGC
GCGCAGATCAGTTGGAAGAATTTGTCCATTACGTAAAAGGCGAGATCACC
AAGGTAGTCGGCAAATAAGAGCCTGTTTAATAAGATGATCTTCTTGAGAT
CGTTTTGGTCTGCGCGTAATCTCTTGCTCTGAAAACGAAAAAACCGCCTT
GCAGGGCGGTTTTTCGAAGGTTCTCTGAGCTACCAACTCTTTGAACCGAG
GTAACTGGCTTGGAGGAGCGCAGTCACCAAAACTTGTCCTTTCAGTTTAG
CCTTAACCGGCGCATGACTTCAAGACTAACTCCTCTAAATCAATTACCAG
TGGCTGCTGCCAGTGGTGCTTTTGCATGTCTTTCCGGGTTGGACTCAAGA
CGATAGTTACCGGATAAGGCGCAGCGGTCGGACTGAACGGGGGGTTCGTG
CATACAGTCCAGCTTGGAGCGAACTGCCTACCCGGAACTGAGTGTCAGGC
 GTGGAATGAGACAAACGCGGCCATAACAGCGGAATGACACCGGTAAACCG AAAGGCAGGAACAGGAGAGCGCACGAGGGAGCCGCCAGGGGGAAACGCCT GGTATCTTTATAGTCCTGTCGGGTTTCGCCACCACTGATTTGAGCGTCAG ATTTCGTGATGCTTGTCAGGGGGGCGGAGCCTATGGAAAAACGGCTTTGC CGCGGCCCTCTCACTTCCCTGTTAAGTATCTTCCTGGCATCTTCCAGGAA ATCTCCGCCCCGTTCGTAAGCCATTTCCGCTCGCCGCAGTCGAACGACCG AGCGTAGCGAGTCAGTGAGCGAGGAAGCGGAATATATCCTGTATCACATA TTCTGCTGACGCACCGGTGCAGCCTTTTTTCTCCTGCCACATGAAGCACT TCACTGACACCCTCATCAGTGCCAACATAGTAAGCCAGTATACACTCCGC TAGCGCGCAAGCGGCCGCA
>pMXLS VM (SEQ ID NO: 35) 
CACTCTGTGACTTGGT CTCC TGAGACC
GTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGAC
GGTCTCA CGAG ACCAAGTCACGAAGTG TGCGGCCGCCTTCG
TGCGCGGAACCCCTATTTGTTTATTTTTCTAAATACATTCAAATATGTAT
CCGCTCATGAGACAATAACCCTGATAAATGCTTCAATAATATTGAAAAAG
GAAGAGTATGCGCTCACGCAACTGGTCCAGAACCTTGACCGAACGCAGCG
GTGGTAACGGCGCAGTGGCGGTTTTCATGGCTTGTTATGACTGTTTTTTT
GGGGTACAGTCTATGCCTCGGGCATCCAAGCAGCAAGCGCGTTACGCCGT
GGGTCGATGTTTGATGTTATGGAGCAGCAACGATGTTACGCAGCAGGGCA
GTCGCCCTAAAACAAAGTTAAACATCATGAGGGAAGCGGTGATCGCCGAA
 
GTATCGACTCAACTATCAGAGGTAGTTGGCGTCATCGAGCGCCATCTCGA
ACCGACGTTGCTGGCCGTACATTTGTACGGCTCCGCAGTGGATGGCGGCC
TGAAGCCACACAGCGATATTGATTTGCTGGTTACGGTGACCGTAAGGCTT
GATGAAACAACGCGGCGAGCTTTGATCAACGACCTTTTGGAAACTTCGGC
TTCCCCTGGAGAGAGCGAGATTCTCCGCGCTGTAGAAGTCACCATTGTTG
TGCACGACGACATCATTCCGTGGCGTTATCCAGCTAAGCGCGAACTGCAA
TTTGGAGAATGGCAGCGCAATGACATTCTTGCAGGTATCTTCGAGCCAGC
CACGATCGACATTGATCTGGCTATCTTGCTGACAAAAGCAAGAGAACATA
GCGTTGCCTTGGTAGGTCCAGCGGCGGAGGAACTCTTTGATCCGGTTCCT
GAACAGGATCTATTTGAGGCGCTAAATGAAACCTTAACGCTATGGAACTC
GCCGCCCGACTGGGCTGGCGATGAGCGAAATGTAGTGCTTACGTTGTCCC
GCATTTGGTACAGCGCAGTAACCGGCAAAATCGCGCCGAAGGATGTCGCT
GCCGACTGGGCAATGGAGCGCCTGCCGGCCCAGTATCAGCCCGTCATACT
TGAAGCTAGACAGGCTTATCTTGGACAAGAAGATCGCTTGGCCTCGC
GCGCAGATCAGTTGGAAGAATTTGTCCATTACGTAAAGGCGAGATCACC
AAGGTAGTCGGCAAATAAGAGCCTGTTTAATAAGATGATCTTCTTGAGAT
CGTTTTGGTCTGCGCGTAATCTCTTGCTCTGAAAACGAAAAAACCGCCTT
GCAGGGCGGTTTTTCGAAGGTTCTCTGAGCTACCAACTCTTTGAACCGAG
GTAACTGGCTTGGAGGAGCGCAGTCACCAAAACTTGTCCTTTCAGTTTAG
CCTTAACCGGCGCATGACTTCAAGACTAACTCCTCTAAATCAATTACCAG
TGGCTGCTGCCAGTGGTGCTTTTGCATGTCTTTCCGGGTTGGACTCAAGA
CGATAGTTACCGGATAAGGCGCAGCGGTCGGACTGAACGGGGGGTTCGTG
CATACAGTCCAGCTTGGAGCGAACTGCCTACCCGGAACTGAGTGTCAGGC
GTGGAATGAGACAAACGCGGCCATAACAGCGGAATGACACCGGTAAACCG
AAAGGCAGGAACAGGAGAGCGCACGAGGGAGCCGCCAGGGGGAAACGCCT
GGTATCTTTATAGTCCTGTCGGGTTTCGCCACCACTGATTTGAGCGTCAG
ATTTCGTGATGCTTGTCAGGGGGGCGGAGCCTATGGAAAAACGGCTTTGC
CGCGGCCCTCTCACTTCCCTGTTAAGTATCTTCCTGGCATCTTCCAGGAA
ATCTCCGCCCCGTTCGTAAGCCATTTCCGCTCGCCGCAGTCGAACGACCG
AGCGTAGCGAGTCAGTGAGCGAGGAAGCGGAATATATCCTGTATCACATA
TTCTGCTGACGCACCGGTGCAGCCTTTTTTCTCCTGCCACATGAAGCACT
TCACTGACACCCTCATCAGTGCCAACATAGTAAGCCAGTATACACTCCGC
TAGCGCGCAAGCGGCCGCA
>pMXLS VR (SEQ ID NO: 36) 
CACTCTGTGACTTGGT CTCC TGAGACC
GTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGAC
GGTCTCA CGAG TGAGACCAAGTCACGAAGTG TGCGGCCGCCTTCG
TGCGCGGAACCCCTATTTGTTTATTTTTCTAAATACATTCAAATATGTAT
CCGCTCATGAGACAATAACCCTGATAAATGCTTCAATAATATTGAAAAAG
GAAGAGTATGCGCTCACGCAACTGGTCCAGAACCTTGACCGAACGCAGCG
GTGGTAACGGCGCAGTGGCGGTTTTCATGGCTTGTTATGACTGTTTTTTT
GGGGTACAGTCTATGCCTCGGGCATCCAAGCAGCAAGCGCGTTACGCCGT
GGGTCGATGTTTGATGTTATGGAGCAGCAACGATGTTACGCAGCAGGGCA
GTCGCCCTAAAACAAAGTTAAACATCATGAGGGAAGCGGTGATCGCCGAA
GTATCGACTCAACTATCAGAGGTAGTTGGCGTCATCGAGCGCCATCTCGA
ACCGACGTTGCTGGCCGTACATTTGTACGGCTCCGCAGTGGATGGCGGCC
TGAAGCCACACAGCGATATTGATTTGCTGGTTACGGTGACCGTAAGGCTT
GATGAAACAACGCGGCGAGCTTTGATCAACGACCTTTTGGAAACTTCGGC
TTCCCCTGGAGAGAGCGAGATTCTCCGCGCTGTAGAAGTCACCATTGTTG
TGCACGACGACATCATTCCGTGGCGTTATCCAGCTAAGCGCGAACTGCAA
TTTGGAGAATGGCAGCGCAATGACATTCTTGCAGGTATCTTCGAGCCAGC
CACGATCGACATTGATCTGGCTATCTTGCTGACAAAAGCAAGAGAACATA
GCGTTGCCTTGGTAGGTCCAGCGGCGGAGGAACTCTTTGATCCGGTTCCT
GAACAGGATCTATTTGAGGCGCTAAATGAAACCTTAACGCTATGGAACTC
GCCGCCCGACTGGGCTGGCGATGAGCGAAATGTAGTGCTTACGTTGTCCC
 
GCATTTGGTACAGCGCAGTAACCGGCAAAATCGCGCCGAAGGATGTCGCT
GCCGACTGGGCAATGGAGCGCCTGCCGGCCCAGTATCAGCCCGTCATACT
TGAAGCTAGACAGGCTTATCTTGGACAAGAAGAAGATCGCTTGGCCTCGC
GCGCAGATCAGTTGGAAGAATTTGTCCATTACGTAAAGGCGAGATCACC
AAGGTAGTCGGCAAATAAGAGCCTGTTTAATAAGATGATCTTCTTGAGAT
CGTTTTGGTCTGCGCGTAATCTCTTGCTCTGAAAACGAAAAAACCGCCTT
GCAGGGCGGTTTTTCGAAGGTTCTCTGAGCTACCAACTCTTTGAACCGAG
GTAACTGGCTTGGAGGAGCGCAGTCACCAAAACTTGTCCTTTCAGTTTAG
CCTTAACCGGCGCATGACTTCAAGACTAACTCCTCTAAATCAATTACCAG
TGGCTGCTGCCAGTGGTGCTTTTGCATGTCTTTCCGGGTTGGACTCAAGA
CGATAGTTACCGGATAAGGCGCAGCGGTCGGACTGAACGGGGGGTTCGTG
CATACAGTCCAGCTTGGAGCGAACTGCCTACCCGGAACTGAGTGTCAGGC
GTGGAATGAGACAAACGCGGCCATAACAGCGGAATGACACCGGTAAACCG
AAAGGCAGGAACAGGAGAGCGCACGAGGGAGCCGCCAGGGGGAAACGCCT
GGTATCTTTATAGTCCTGTCGGGTTTCGCCACCACTGATTTGAGCGTCAG
ATTTCGTGATGCTTGTCAGGGGGGCGGAGCCTATGGAAAAACGGCTTTGC
CGCGGCCCTCTCACTTCCCTGTTAAGTATCTTCCTGGCATCTTCCAGGAA
ATCTCCGCCCCGTTCGTAAGCCATTTCCGCTCGCCGCAGTCGAACGACCG
AGCGTAGCGAGTCAGTGAGCGAGGAAGCGGAATATATCCTGTATCACATA
TTCTGCTGACGCACCGGTGCAGCCTTTTTTCTCCTGCCACATGAAGCACT
TCACTGACACCCTCATCAGTGCCAACATAGTAAGCCAGTATACACTCCGC
TAGCGCGCAAGCGGCCGCA
>pMZLS_VL (SEQ ID NO: 37) 
CACTCTGT GAAGCTCTTCA TCC TGAAGAGC
GGTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
 
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGACC
GCTCTTCA CGA AGAGCTTC ACGAAGTG TGCGGCCGCCTTCG
TGCGCGGAACCCCTATTTGTTTATTTTTCTAAATACATTCAAATATGTAT
CCGCTCATGAGACAATAACCCTGATAAATGCTTCAATAATATTGAAAAAG
GAAGAGTATGCGCTCACGCAACTGGTCCAGAACCTTGACCGAACGCAGCG
GTGGTAACGGCGCAGTGGCGGTTTTCATGGCTTGTTATGACTGTTTTTTT
GGGGTACAGTCTATGCCTCGGGCATCCAAGCAGCAAGCGCGTTACGCCGT
GGGTCGATGTTTGATGTTATGGAGCAGCAACGATGTTACGCAGCAGGGCA
GTCGCCCTAAAACAAAGTTAAACATCATGAGGGAAGCGGTGATCGCCGAA
GTATCGACTCAACTATCAGAGGTAGTTGGCGTCATCGAGCGCCATCTCGA
ACCGACGTTGCTGGCCGTACATTTGTACGGCTCCGCAGTGGATGGCGGCC
TGAAGCCACACAGCGATATTGATTTGCTGGTTACGGTGACCGTAAGGCTT
GATGAAACAACGCGGCGAGCTTTGATCAACGACCTTTTGGAAACTTCGGC
TTCCCCTGGAGAGAGCGAGATTCTCCGCGCTGTAGAAGTCACCATTGTTG
TGCACGACGACATCATTCCGTGGCGTTATCCAGCTAAGCGCGAACTGCAA
TTTGGAGAATGGCAGCGCAATGACATTCTTGCAGGTATCTTCGAGCCAGC
CACGATCGACATTGATCTGGCTATCTTGCTGACAAAAGCAAGAGAACATA
GCGTTGCCTTGGTAGGTCCAGCGGCGGAGGAACTCTTTGATCCGGTTCCT
GAACAGGATCTATTTGAGGCGCTAAATGAAACCTTAACGCTATGGAACTC
GCCGCCCGACTGGGCTGGCGATGAGCGAAATGTAGTGCTTACGTTGTCCC
GCATTTGGTACAGCGCAGTAACCGGCAAAATCGCGCCGAAGGATGTCGCT
GCCGACTGGGCAATGGAGCGCCTGCCGGCCCAGTATCAGCCCGTCATACT
TGAAGCTAGACAGGCTTATCTTGGACAAGAAGATCGCTTGGCCTCGC
GCGCAGATCAGTTGGAAGAATTTGTCCATTACGTAAAGGCGAGATCACC
AAGGTAGTCGGCAAATAAGAGCCTGTTTAATAAGATGATCTTCTTGAGAT
CGTTTTGGTCTGCGCGTAATCTCTTGCTCTGAAAACGAAAAAACCGCCTT
GCAGGGCGGTTTTTCGAAGGTTCTCTGAGCTACCAACTCTTTGAACCGAG
GTAACTGGCTTGGAGGAGCGCAGTCACCAAAACTTGTCCTTTCAGTTTAG
CCTTAACCGGCGCATGACTTCAAGACTAACTCCTCTAAATCAATTACCAG
TGGCTGCTGCCAGTGGTGCTTTTGCATGTCTTTCCGGGTTGGACTCAAGA
CGATAGTTACCGGATAAGGCGCAGCGGTCGGACTGAACGGGGGGTTCGTG
CATACAGTCCAGCTTGGAGCGAACTGCCTACCCGGAACTGAGTGTCAGGC
GTGGAATGAGACAAACGCGGCCATAACAGCGGAATGACACCGGTAAACCG
AAAGGCAGGAACAGGAGAGCGCACGAGGGAGCCGCCAGGGGGAAACGCCT
GGTATCTTTATAGTCCTGTCGGGTTTCGCCACCACTGATTTGAGCGTCAG
ATTTCGTGATGCTTGTCAGGGGGGCGGAGCCTATGGAAAAACGGCTTTGC
CGCGGCCCTCTCACTTCCCTGTTAAGTATCTTCCTGGCATCTTCCAGGAA
ATCTCCGCCCCGTTCGTAAGCCATTTCCGCTCGCCGCAGTCGAACGACCG
AGCGTAGCGAGTCAGTGAGCGAGGAAGCGGAATATATCCTGTATCACATA
TTCTGCTGACGCACCGGTGCAGCCTTTTTTCTCCTGCCACATGAAGCACT
TCACTGACACCCTCATCAGTGCCAACATAGTAAGCCAGTATACACTCCGC
TAGCGCGCAAGCGGCCGCA
>pMZLS_VM (SEQ ID NO: 38) 
CACTCTGT GAAGCTCT TCC TGAAGAGC
GGTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGACC
GCTCTTCA CGA AGAGCTTC ACGAAGTG TGCGGCCGCCTTCG
TGCGCGGAACCCCTATTTGTTTATTTTTCTAAATACATTCAAATATGTAT
CCGCTCATGAGACAATAACCCTGATAAATGCTTCAATAATATTGAAAAAG
GAAGAGTATGCGCTCACGCAACTGGTCCAGAACCTTGACCGAACGCAGCG
GTGGTAACGGCGCAGTGGCGGTTTTCATGGCTTGTTATGACTGTTTTTTT
GGGGTACAGTCTATGCCTCGGGCATCCAAGCAGCAAGCGCGTTACGCCGT
GGGTCGATGTTTGATGTTATGGAGCAGCAACGATGTTACGCAGCAGGGCA
GTCGCCCTAAAACAAAGTTAAACATCATGAGGGAAGCGGTGATCGCCGAA
GTATCGACTCAACTATCAGAGGTAGTTGGCGTCATCGAGCGCCATCTCGA
ACCGACGTTGCTGGCCGTACATTTGTACGGCTCCGCAGTGGATGGCGGCC
TGAAGCCACACAGCGATATTGATTTGCTGGTTACGGTGACCGTAAGGCTT
GATGAAACAACGCGGCGAGCTTTGATCAACGACCTTTTGGAAACTTCGGC
TTCCCCTGGAGAGAGCGAGATTCTCCGCGCTGTAGAAGTCACCATTGTTG
TGCACGACGACATCATTCCGTGGCGTTATCCAGCTAAGCGCGAACTGCAA
TTTGGAGAATGGCAGCGCAATGACATTCTTGCAGGTATCTTCGAGCCAGC
CACGATCGACATTGATCTGGCTATCTTGCTGACAAAAGCAAGAGAACATA
GCGTTGCCTTGGTAGGTCCAGCGGCGGAGGAACTCTTTGATCCGGTTCCT
GAACAGGATCTATTTGAGGCGCTAAATGAAACCTTAACGCTATGGAACTC
GCCGCCCGACTGGGCTGGCGATGAGCGAAATGTAGTGCTTACGTTGTCCC
 
GCATTTGGTACAGCGCAGTAACCGGCAAAATCGCGCCGAAGGATGTCGCT
GCCGACTGGGCAATGGAGCGCCTGCCGGCCCAGTATCAGCCCGTCATACT
TGAAGCTAGACAGGCTTATCTTGGACAAGAAGAAGATCGCTTGGCCTCGC
GCGCAGATCAGTTGGAAGAATTTGTCCATTACGTAAAGGCGAGATCACC
AAGGTAGTCGGCAAATAAGAGCCTGTTTAATAAGATGATCTTCTTGAGAT
CGTTTTGGTCTGCGCGTAATCTCTTGCTCTGAAAACGAAAAAACCGCCTT
GCAGGGCGGTTTTTCGAAGGTTCTCTGAGCTACCAACTCTTTGAACCGAG
GTAACTGGCTTGGAGGAGCGCAGTCACCAAAACTTGTCCTTTCAGTTTAG
CCTTAACCGGCGCATGACTTCAAGACTAACTCCTCTAAATCAATTACCAG
TGGCTGCTGCCAGTGGTGCTTTTGCATGTCTTTCCGGGTTGGACTCAAGA
CGATAGTTACCGGATAAGGCGCAGCGGTCGGACTGAACGGGGGGTTCGTG
CATACAGTCCAGCTTGGAGCGAACTGCCTACCCGGAACTGAGTGTCAGGC
GTGGAATGAGACAAACGCGGCCATAACAGCGGAATGACACCGGTAAACCG
AAAGGCAGGAACAGGAGAGCGCACGAGGGAGCCGCCAGGGGGAAACGCCT
GGTATCTTTATAGTCCTGTCGGGTTTCGCCACCACTGATTTGAGCGTCAG
ATTTCGTGATGCTTGTCAGGGGGGCGGAGCCTATGGAAAAACGGCTTTGC
CGCGGCCCTCTCACTTCCCTGTTAAGTATCTTCCTGGCATCTTCCAGGAA
ATCTCCGCCCCGTTCGTAAGCCATTTCCGCTCGCCGCAGTCGAACGACCG
AGCGTAGCGAGTCAGTGAGCGAGGAAGCGGAATATATCCTGTATCACATA
TTCTGCTGACGCACCGGTGCAGCCTTTTTTCTCCTGCCACATGAAGCACT
TCACTGACACCCTCATCAGTGCCAACATAGTAAGCCAGTATACACTCCGC
TAGCGCGCAAGCGGCCGCA
>pMZLS_VR (SEQ ID NO: 39) 
CACTCTGT GAAGCTCT TCC TGAAGAGC
GGTCAGACCAAGTTTACTCAT
ATATACTTTAGATTGATTTAAAACTTCATTTTTAATTTAAAAGGATCTAG
GTGAAGATCCTTTTTGATAATCTCATGACCAAAATCCCTTAACGTGAGTT
TTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTC
TAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGA
TAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAG
CGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGA
GTGAGCTGATACCGCTCGCCGCAGCCGAACGACCGAGCGCAGCGAGTCAG
TGAGCGAGGAAGCGGATGAGCGCCCAATACGCAAACCGCCTCTCCCCGCG
CGTTGGCCGATTCATTAATGCAGCTGGCACGACAGGTTTCCCGACTGGAA
AGCGGGCAGTGAGCGCAACGCAATTAATGTGAGTTAGCTCACTCATTAGG
CACCCCAGGCTTTACACTTTATGCTTCCGGCTCGTATGTTGTGTGGAATT
GTGAGCGGATAACAATTTCACACAGGAAACAGCTATGACCATGATTACGC
CAAGCTTGCATGCCTGCAGGTCGACTCTAGAGGATCCCCGGGTACCGAGC
TCGAATTCACTGGCCGTCGTTTTACAACGTCGTGACTGGGAAAACCCTGG
CGTTACCCAACTTAATCGCCTTGCAGCACATCCCCCTTTCGCCAGCTGGC
GTAATAGCGAAGAGGCCCGCACCGATCGCCCTTCCCAACAGTTGCGCAGC
 
CTGAATGGCGAATGGCGCCTGATGCGGTATTTTCTCCTTACGCATCTGTG
CGGTATTTCACACCGCATATGGTGCACTCTCAGTACAATCTGCTCTGATG
CCGCATAGTTAAGCCAGCCCCGACACCCGCCAACACCCGCTGACGCGCCC
TGACGGGCTTGTCTGCTCCCGGCATCCGCTTACAGACAAGCTGTGACC
GCTCTTCA CGA TGAAGAGCTTC ACGAAGTG TGCGGCCGCCTTCG
TGCGCGGAACCCCTATTTGTTTATTTTTCTAAATACATTCAAATATGTAT
CCGCTCATGAGACAATAACCCTGATAAATGCTTCAATAATATTGAAAAAG
GAAGAGTATGCGCTCACGCAACTGGTCCAGAACCTTGACCGAACGCAGCG
GTGGTAACGGCGCAGTGGCGGTTTTCATGGCTTGTTATGACTGTTTTTTT
GGGGTACAGTCTATGCCTCGGGCATCCAAGCAGCAAGCGCGTTACGCCGT
GGGTCGATGTTTGATGTTATGGAGCAGCAACGATGTTACGCAGCAGGGCA
GTCGCCCTAAAACAAAGTTAAACATCATGAGGGAAGCGGTGATCGCCGAA
GTATCGACTCAACTATCAGAGGTAGTTGGCGTCATCGAGCGCCATCTCGA
ACCGACGTTGCTGGCCGTACATTTGTACGGCTCCGCAGTGGATGGCGGCC
TGAAGCCACACAGCGATATTGATTTGCTGGTTACGGTGACCGTAAGGCTT
GATGAAACAACGCGGCGAGCTTTGATCAACGACCTTTTGGAAACTTCGGC
TTCCCCTGGAGAGAGCGAGATTCTCCGCGCTGTAGAAGTCACCATTGTTG
TGCACGACGACATCATTCCGTGGCGTTATCCAGCTAAGCGCGAACTGCAA
TTTGGAGAATGGCAGCGCAATGACATTCTTGCAGGTATCTTCGAGCCAGC
CACGATCGACATTGATCTGGCTATCTTGCTGACAAAAGCAAGAGAACATA
GCGTTGCCTTGGTAGGTCCAGCGGCGGAGGAACTCTTTGATCCGGTTCCT
GAACAGGATCTATTTGAGGCGCTAAATGAAACCTTAACGCTATGGAACTC
GCCGCCCGACTGGGCTGGCGATGAGCGAAATGTAGTGCTTACGTTGTCCC
GCATTTGGTACAGCGCAGTAACCGGCAAAATCGCGCCGAAGGATGTCGCT
GCCGACTGGGCAATGGAGCGCCTGCCGGCCCAGTATCAGCCCGTCATACT
TGAAGCTAGACAGGCTTATCTTGGACAAGAAGAAGATCGCTTGGCCTCGC
GCGCAGATCAGTTGGAAGAATTTGTCCATTACGTAAAGGCGAGATCACC
AAGGTAGTCGGCAAATAAGAGCCTGTTTAATAAGATGATCTTCTTGAGAT
CGTTTTGGTCTGCGCGTAATCTCTTGCTCTGAAAACGAAAAAACCGCCTT
GCAGGGCGGTTTTTCGAAGGTTCTCTGAGCTACCAACTCTTTGAACCGAG
GTAACTGGCTTGGAGGAGCGCAGTCACCAAAACTTGTCCTTTCAGTTTAG
CCTTAACCGGCGCATGACTTCAAGACTAACTCCTCTAAATCAATTACCAG
TGGCTGCTGCCAGTGGTGCTTTTGCATGTCTTTCCGGGTTGGACTCAAGA
CGATAGTTACCGGATAAGGCGCAGCGGTCGGACTGAACGGGGGGTTCGTG
CATACAGTCCAGCTTGGAGCGAACTGCCTACCCGGAACTGAGTGTCAGGC
GTGGAATGAGACAAACGCGGCCATAACAGCGGAATGACACCGGTAAACCG
AAAGGCAGGAACAGGAGAGCGCACGAGGGAGCCGCCAGGGGGAAACGCCT
GGTATCTTTATAGTCCTGTCGGGTTTCGCCACCACTGATTTGAGCGTCAG
ATTTCGTGATGCTTGTCAGGGGGGCGGAGCCTATGGAAAAACGGCTTTGC
CGCGGCCCTCTCACTTCCCTGTTAAGTATCTTCCTGGCATCTTCCAGGAA
ATCTCCGCCCCGTTCGTAAGCCATTTCCGCTCGCCGCAGTCGAACGACCG
AGCGTAGCGAGTCAGTGAGCGAGGAAGCGGAATATATCCTGTATCACATA
TTCTGCTGACGCACCGGTGCAGCCTTTTTTCTCCTGCCACATGAAGCACT
TCACTGACACCCTCATCAGTGCCAACATAGTAAGCCAGTATACACTCCGC
TAGCGCGCAAGCGGCCGCA
>pMXP A4E (SEQ ID NO: 40) 
CACTCTGT GACTTGGTCTCA TGCC TGAGACC GCAGCTGGCACGACAGG
TTTCCCGACTGGAAAGCGGGCAGTGAGCGCAACGCAATTAATGTGAGTTA
GCTCACTCATTAGGCACCCCAGGCTTTACACTTTATGCTTCCGGCTCGTA
TGTTGTGTGGAATTGTGAGCGGATAACAATTTCACACAGGAAACAGCTAT
GACCATGATTACGCCAAGCTTGCATGCCTGCAGGTCGACTCTAGAGGATC
CCCGGGTACCGAGCTCGAATTCACTGGCCGTCGTTTTACAACGTCGTGAC
TGGGAAAACCCTGGCGTTACCCAACTTAATCGCCTTGCAGCACATCCCCC
TTTCGCCAGCTGGCGTAATAGCGAAGAGGCCCGCACCGATCGCCCTTCCC
AACAGTTGCGCAGCCTGAATGGCGAATGGCGCCTGATGCGGTATTTTCTC
CTTACGCATCTGTGCGGTATTTCACACCGCATATGGTGCACTCTCAGTAC
AATCTGCTCTGATGCCGCATAGTTAAGCCAGCCCCGACACCCGCCAACAC
CCGCTGACGCGCCCTGACGGGCTTGTCTGCTCCCGGCATCCGCTTACAGA
CAAGCTGTGAC GGTCTCA CAGA TGAGACCAAGTC ACGAAGTGGTTTAA
ACTATCAGTGTTTGACAGGATATATTGGCGGGTAAACCTAAGAGAAAAGA
GCGTTTATTAGAATAATCGGATATTTAAAAGGGCGTGAAAAGGTTTATCC
GTTCGTCCATTTGTATGTGCATGCCAACCACAGGGTTCCCCAGATCAGGC
GCTGGCTGCTGAACCCCCAGCCGGAACTGACCCCACAAGGCCCTAGCGTT
TGCAATGCACCAGGTCATCATTGACCCAGGCGTGTTCCACCAGGCCGCTG
CCTCGCAACTCTTCGCAGGCTTCGCCGACCTGCTCGCGCCACTTCTTCAC
GCGGGTGGAATCCGATCCGCACATGAGGCGGAAGGTTTCCAGCTTGAGCG
GGTACGGCTCCCGGTGCGAGCTGAAATAGTCGAACATCCGTCGGGCCGTC
GGCGACAGCTTGCGGTACTTCTCCCATATGAATTTCGTGTAGTGGTCGCC
AGCAAACAGCACGACGATTTCCTCGTCGATCAGGACCTGGCAACGGGACG
TTTTCTTGCCACGGTCCAGGACGCGGAAGCGGTGCAGCAGCGACACCGAT
TCCAGGTGCCCAACGCGGTCGGACGTGAAGCCCATCGCCGTCGCCTGTAG
GCGCGACAGGCATTCCTCGGCCTTCGTGTAATACCGGCCATTGATCGACC
AGCCCAGGTCCTGGCAAAGCTCGTAGAACGTGAAGGTGATCGGCTCGCCG
ATAGGGGTGCGCTTCGCGTACTCCAACACCTGCTGCCACACCAGTTCGTC
ATCGTCGGCCCGCAGCTCGACGCCGGTGTAGGTGATCTTCACGTCCTTGT
TGACGTGGAAAATGACCTTGTTTTGCAGCGCCTCGCGCGGGATTTTCTTG
TTGCGCGTGGTGAACAGGGCAGAGCGGGCCGTGTCGTTTGGCATCGCTCG
CATCGTGTCCGGCCACGGCGCAATATCGAACAAGGAAAGCTGCATTTCCT
TGATCTGCTGCTTCGTGTGTTTCAGCAACGCGGCCTGCTTGGCCTCGCTG
ACCTGTTTTGCCAGGTCCTCGCCGGCGGTTTTTCGCTTCTTGGTCGTCAT
AGTTCCTCGCGTGTCGATGGTCATCGACTTCGCCAAACCTGCCGCCTCCT
GTTCAAGACGACGCGAACGCTCCACGGCGGCCGATGGCGCGGGCAGGGCA
GGGGGAGCCAGTTGCACGCTGTCGCGCTCGATCTTGGCCGTAGCTTGCTG
GACCATCGAGCCGACGGACTGGAAGGTTTCGCGGGGCGCACGCATGACGG
TGCGGCTTGCGATGGTTTCGGCATCCTCGGCGGAAAACCCCGCGTCGATC
AGTTCTTGCCTGTATGCCTTCCGGTCAAACGTCCGATTCATTCACCCTCC
TTGCGGGATTGCCCCGACTCACGCCGGGGCAATGTGCCCTTATTCCTGAT
TTGACCCGCCTGGTGCCTTGGTGTCCAGATAATCCACCTTATCGGCAATG
AAGTCGGTCCCGTAGACCGTCTGGCCGTCCTTCTCGTACTTGGTATTCCG
AATCTTGCCCTGCACGAATACCAGCGACCCCTTGCCCAAATACTTGCCGT
GGGCCTCGGCCTGAGAGCCAAAACACTTGATGCGGAAGAAGTCGGTGCGC
TCCTGCTTGTCGCCGGCATCGTTGCGCCACATCTAGGATCTGCCAGGAAC
CGTAAAAAGGCCGCGTTGCTGGCGTTTTTCCATAGGCTCCGCCCCCCTGA
CGAGCATCACAAAAATCGACGCTCAAGTCAGAGGTGGCGAAACCCGACAG
GACTATAAAGATACCAGGCGTTTCCCCCTGGAAGCTCCCTCGTGCGCTCT
CCTGTTCCGACCCTGCCGCTTACCGGATACCTGTCCGCCTTTCTCCCTTC
GGGAAGCGTGGCGCTTTCTCATAGCTCACGCTGTAGGTATCTCAGTTCGG
TGTAGGTCGTTCGCTCCAAGCTGGGCTGTGTGCACGAACCCCCCGTTCAG
 
CCCGACCGCTGCGCCTTATCCGGTAACTATCGTCTTGAGTCCAACCCGGT
AAGACACGACTTATCGCCACTGGCAGCAGCCACTGGTAACAGGATTAGCA
GAGCGAGGTATGTAGGCGGTGCTACAGAGTTCTTGAAGTGGTGGCCTAAC
TACGGCTACACTAGAAGGACAGTATTTGGTATCTGCGCTCTGCTGAAGCC
AGTTACCTTCGGAAAAAGAGTTGGTAGCTCTTGATCCGGCAAACAAACCA
CCGCTGGTAGCGGTGGTTTTTTTGTTTGCAAGCAGCAGATTACGCGCAGA
AAAAAAGGATCTCAAGAAGATCCTTTGATCTTTTCTACGGGGTCTGACGC
TCAGTGGAACGAAAACTCACGTTAAGGGATTTTGGTCATGAGATTATCAA
AAAGGATCTTCACCTAGATCCTTTTAAATTAAAAATGAAGTTTTAAATCA
ATCTAAAGTATATATGAGTAAACTTGGTCTGACAGTTACCAATGCTTAAT
CAGTGAGGCACCTATCTCAGCGATCTGTCTATTTCGTTCATCCATAGTTG
CCTGACTCCCCGTCGTGTAGATAACTACGATACGGGAGGGCTTACCATCT
GGCCCCAGTGCTGCAATGATACCGCGAGAACCACGCTCACCGGCTCCAGA
TTTATCAGCAATAAACCAGCCAGCCGGAAGGGCCGAGCGCAGAAGTGGTC
CTGCAACTTTATCCGCCTCCATCCAGTCTATTAATTGTTGCCGGGAAGCT
AGAGTAAGTAGTTCGCCAGTTAATAGTTTGCGCAACGTTGTTGCCATTGC
TACAGGCATCGTGGTGTCACGCTCGTCGTTTGGTATGGCTTCATTCAGCT
CCGGTTCCCAACGATCAAGGCGAGTTACATGATCCCCCATGTTGTGCAAA
AAAGCGGTTAGCTCCTTCGGTCCTCCGATCGTTGTCAGAAGTAAGTTGGC
CGCAGTGTTATCACTCATGGTTATGGCAGCACTGCATAATTCTCTTACTG
TCATGCCATCCGTAAGATGCTTTTCTGTGACTGGTGAGTACTCAACCAAG
TCATTCTGAGAATAGTGTATGCGGCGACCGAGTTGCTCTTGCCCGGCGTC
AATACGGGATAATACCGCGCCACATAGCAGAACTTTAAAAGTGCTCATCA
TTGGAAAACGTTCTTCGGGGCGAAAACTCTCAAGGATCTTACCGCTGTTG
AGATCCAGTTCGATGTAACCCACTCGTGCACCCAACTGATCTTCAGCATC
TTTTACTTTCACCAGCGTTTCTGGGTGAGCAAAAACAGGAAGGCAAAATG
CCGCAAAAAAGGGAATAAGGGCGACACGGAAATGTTGAATACTCATACTC
TTCCTTTTTCAATATTATTGAAGCATTTATCAGGGTTATTGTCTCATGAG
CGGATACATATTTGAATGTATTTAGAAAAATAAACAAATAGGGGTTCCGC
GCACGAATTGGCCAGCGCTGCCATTTTTGGGGTGAGGCCGTTCGCGGCCG
AGGGGCGCAGCCCCTGGGGGGATGGAGGCCCGCGTTAGCGGGCCGGGAG
GGTTCGAGAAGGGGGGGCACCCCCCTTCGGCGTGCGCGGTCACGCGCACA
GGGCGCAGCCCTGGTTAAAAACAAGGTTTATAAATATTGGTTTAAAAGCA
GGTTAAAAGACAGGTTAGCGGTGGCCGAAAAACGGGCGGAAACCCTTGCA
AATGCTGGATTTTCTGCCTGTGGACAGCCCCTCAAATGTCAATAGGTGCG
CCCCTCATCTGTCAGCACTCTGCCCCTCAAGTGTCAAGGATCGCGCCCCT
CATCTGTCAGTAGTCGCGCCCCTCAAGTGTCAATACCGCAGGGCACTTAT
CCCCAGGCTTGTCCACATCATCTGTGGGAAACTCGCGTAAAATCAGGCGT
TTTCGCCGATTTGCGAGGCTGGCCAGCTCCACGTCGCCGGCCGAAATCGA
GCCTGCCCCTCATCTGTCAACGCCGCGCCGGGTGAGTCGGCCCCTCAAGT
GTCAACGTCCGCCCCTCATCTGTCAGTGAGGGCCAAGTTTTCCGCGAGGT
ATCCACAACGCCGGCGGCCGCGGTGTCTCGCACACGGCTTCGACGGCGTT
TCTGGCGCGTTTGCAGGGCCATAGACGGCCGCCAGCCCAGCGGCGAGGGC
AACCAGCCCGGTGAGCGTCGCAAAGGAGATCCTGATCTGACTGATGGGCT
GCCTGTATCGAGTGGTGATTTTGTGCCGAGCTGCCGGTCGGGGAGCTGTT
GGCTGGCTGGTGGCAGGATATATTGTGGTGTAAACAAATTGACGCTTAGA
CAACTTAATAACACATTGCGGACGTTTTTAATGTACTGGGGTGGATGCAG
TGGGCCC
>pMYP_A5E (SEQ ID NO: 41) 
CACTCTGT GAAGACGC TGCC TTGTCTTC GCAGCTGGCACGACAGGTTT
CCCGACTGGAAAGCGGGCAGTGAGCGCAACGCAATTAATGTGAGTTAGCT
CACTCATTAGGCACCCCAGGCTTTACACTTTATGCTTCCGGCTCGTATGT
TGTGTGGAATTGTGAGCGGATAACAATTTCACACAGGAAACAGCTATGAC
CATGATTACGCCAAGCTTGCATGCCTGCAGGTCGACTCTAGAGGATCCCC
GGGTACCGAGCTCGAATTCACTGGCCGTCGTTTTACAACGTCGTGACTGG
GAAAACCCTGGCGTTACCCAACTTAATCGCCTTGCAGCACATCCCCCTTT
CGCCAGCTGGCGTAATAGCGAAGAGGCCCGCACCGATCGCCCTTCCCAAC
AGTTGCGCAGCCTGAATGGCGAATGGCGCCTGATGCGGTATTTTCTCCTT
ACGCATCTGTGCGGTATTTCACACCGCATATGGTGCACTCTCAGTACAAT
CTGCTCTGATGCCGCATAGTTAAGCCAGCCCCGACACCCGCCAACACCCG
CTGACGCGCCCTGACGGGCTTGTCTGCTCCCGGCATCCGCTTACAGACAA
GCTGTGAC GAAGACAA CAGA GCGTCTTC ACGAAGTGGTTTAAACTATC
AGTGTTTGACAGGATATATTGGCGGGTAAACCTAAGAGAAAAGAGCGTTT
ATTAGAATAATCGGATATTTAAAAGGGCGTGAAAAGGTTTATCCGTTCGT
CCATTTGTATGTGCATGCCAACCACAGGGTTCCCCAGATCAGGCGCTGGC
TGCTGAACCCCCAGCCGGAACTGACCCCACAAGGCCCTAGCGTTTGCAAT
GCACCAGGTCATCATTGACCCAGGCGTGTTCCACCAGGCCGCTGCCTCGC
AACTCTTCGCAGGCTTCGCCGACCTGCTCGCGCCACTTCTTCACGCGGGT
GGAATCCGATCCGCACATGAGGCGGAAGGTTTCCAGCTTGAGCGGGTACG
GCTCCCGGTGCGAGCTGAAATAGTCGAACATCCGTCGGGCCGTCGGCGAC
AGCTTGCGGTACTTCTCCCATATGAATTTCGTGTAGTGGTCGCCAGCAAA
CAGCACGACGATTTCCTCGTCGATCAGGACCTGGCAACGGGACGTTTTCT
TGCCACGGTCCAGGACGCGGAAGCGGTGCAGCAGCGACACCGATTCCAGG
TGCCCAACGCGGTCGGACGTGAAGCCCATCGCCGTCGCCTGTAGGCGCGA
CAGGCATTCCTCGGCCTTCGTGTAATACCGGCCATTGATCGACCAGCCCA
GGTCCTGGCAAAGCTCGTAGAACGTGAAGGTGATCGGCTCGCCGATAGGG
GTGCGCTTCGCGTACTCCAACACCTGCTGCCACACCAGTTCGTCATCGTC
GGCCCGCAGCTCGACGCCGGTGTAGGTGATCTTCACGTCCTTGTTGACGT
GGAAAATGACCTTGTTTTGCAGCGCCTCGCGCGGGATTTTCTTGTTGCGC
GTGGTGAACAGGGCAGAGCGGGCCGTGTCGTTTGGCATCGCTCGCATCGT
GTCCGGCCACGGCGCAATATCGAACAAGGAAAGCTGCATTTCCTTGATCT
GCTGCTTCGTGTGTTTCAGCAACGCGGCCTGCTTGGCCTCGCTGACCTGT
TTTGCCAGGTCCTCGCCGGCGGTTTTTCGCTTCTTGGTCGTCATAGTTCC
TCGCGTGTCGATGGTCATCGACTTCGCCAAACCTGCCGCCTCCTGTTCAA
GACGACGCGAACGCTCCACGGCGGCCGATGGCGCGGGCAGGGCAGGGGGA
GCCAGTTGCACGCTGTCGCGCTCGATCTTGGCCGTAGCTTGCTGGACCAT
CGAGCCGACGGACTGGAAGGTTTCGCGGGGCGCACGCATGACGGTGCGGC
TTGCGATGGTTTCGGCATCCTCGGCGGAAAACCCCGCGTCGATCAGTTCT
TGCCTGTATGCCTTCCGGTCAAACGTCCGATTCATTCACCCTCCTTGCGG
GATTGCCCCGACTCACGCCGGGGCAATGTGCCCTTATTCCTGATTTGACC
CGCCTGGTGCCTTGGTGTCCAGATAATCCACCTTATCGGCAATGAAGTCG
GTCCCGTAGACCGTCTGGCCGTCCTTCTCGTACTTGGTATTCCGAATCTT
GCCCTGCACGAATACCAGCGACCCCTTGCCCAAATACTTGCCGTGGGCCT
CGGCCTGAGAGCCAAAACACTTGATGCGGAAGAAGTCGGTGCGCTCCTGC
TTGTCGCCGGCATCGTTGCGCCACATCTAGGATCTGCCAGGAACCGTAAA
AAGGCCGCGTTGCTGGCGTTTTTCCATAGGCTCCGCCCCCCTGACGAGCA
TCACAAAAATCGACGCTCAAGTCAGAGGTGGCGAAACCCGACAGGACTAT
AAAGATACCAGGCGTTTCCCCCTGGAAGCTCCCTCGTGCGCTCTCCTGTT
CCGACCCTGCCGCTTACCGGATACCTGTCCGCCTTTCTCCCTTCGGGAAG
CGTGGCGCTTTCTCATAGCTCACGCTGTAGGTATCTCAGTTCGGTGTAGG
TCGTTCGCTCCAAGCTGGGCTGTGTGCACGAACCCCCCGTTCAGCCCGAC
 
CGCTGCGCCTTATCCGGTAACTATCGTCTTGAGTCCAACCCGGTAAGACA
CGACTTATCGCCACTGGCAGCAGCCACTGGTAACAGGATTAGCAGAGCGA
GGTATGTAGGCGGTGCTACAGAGTTCTTGAAGTGGTGGCCTAACTACGGC
TACACTAGAAGGACAGTATTTGGTATCTGCGCTCTGCTGAAGCCAGTTAC
CTTCGGAAAAAGAGTTGGTAGCTCTTGATCCGGCAAACAAACCACCGCTG
GTAGCGGTGGTTTTTTTGTTTGCAAGCAGCAGATTACGCGCAGAAAAAAA
GGATCTCAAGAAGATCCTTTGATCTTTTCTACGGGGTCTGACGCTCAGTG
GAACGAAAACTCACGTTAAGGGATTTTGGTCATGAGATTATCAAAAAGGA
TCTTCACCTAGATCCTTTTAAATTAAAAATGAAGTTTTAAATCAATCTAA
AGTATATATGAGTAAACTTGGTCTGACAGTTACCAATGCTTAATCAGTGA
GGCACCTATCTCAGCGATCTGTCTATTTCGTTCATCCATAGTTGCCTGAC
TCCCCGTCGTGTAGATAACTACGATACGGGAGGGCTTACCATCTGGCCCC
AGTGCTGCAATGATACCGCGAGAACCACGCTCACCGGCTCCAGATTTATC
AGCAATAAACCAGCCAGCCGGAAGGGCCGAGCGCAGAAGTGGTCCTGCAA
CTTTATCCGCCTCCATCCAGTCTATTAATTGTTGCCGGGAAGCTAGAGTA
AGTAGTTCGCCAGTTAATAGTTTGCGCAACGTTGTTGCCATTGCTACAGG
CATCGTGGTGTCACGCTCGTCGTTTGGTATGGCTTCATTCAGCTCCGGTT
CCCAACGATCAAGGCGAGTTACATGATCCCCCATGTTGTGCAAAAAAGCG
GTTAGCTCCTTCGGTCCTCCGATCGTTGTCAGAAGTAAGTTGGCCGCAGT
GTTATCACTCATGGTTATGGCAGCACTGCATAATTCTCTTACTGTCATGC
CATCCGTAAGATGCTTTTCTGTGACTGGTGAGTACTCAACCAAGTCATTC
TGAGAATAGTGTATGCGGCGACCGAGTTGCTCTTGCCCGGCGTCAATACG
GGATAATACCGCGCCACATAGCAGAACTTTAAAAGTGCTCATCATTGGAA
AACGTTCTTCGGGGCGAAAACTCTCAAGGATCTTACCGCTGTTGAGATCC
AGTTCGATGTAACCCACTCGTGCACCCAACTGATCTTCAGCATCTTTTAC
TTTCACCAGCGTTTCTGGGTGAGCAAAAACAGGAAGGCAAAATGCCGCAA
AAAAGGGAATAAGGGCGACACGGAAATGTTGAATACTCATACTCTTCCTT
TTTCAATATTATTGAAGCATTTATCAGGGTTATTGTCTCATGAGCGGATA
CATATTTGAATGTATTTAGAAAAATAAACAAATAGGGGTTCCGCGCACGA
ATTGGCCAGCGCTGCCATTTTTGGGGTGAGGCCGTTCGCGGCCGAGGGGC
GCAGCCCCTGGGGGGATGGGAGGCCCGCGTTAGCGGGCCGGGAGGGTTCG
AGAAGGGGGGGCACCCCCCTTCGGCGTGCGCGGTCACGCGCACAGGGCGC
AGCCCTGGTTAAAAACAAGGTTTATAAATATTGGTTTAAAAGCAGGTTAA
AAGACAGGTTAGCGGTGGCCGAAAAACGGGCGGAAACCCTTGCAAATGCT
GGATTTTCTGCCTGTGGACAGCCCCTCAAATGTCAATAGGTGCGCCCCTC
ATCTGTCAGCACTCTGCCCCTCAAGTGTCAAGGATCGCGCCCCTCATCTG
TCAGTAGTCGCGCCCCTCAAGTGTCAATACCGCAGGGCACTTATCCCCAG
GCTTGTCCACATCATCTGTGGGAAACTCGCGTAAAATCAGGCGTTTTCGC
CGATTTGCGAGGCTGGCCAGCTCCACGTCGCCGGCCGAAATCGAGCCTGC
CCCTCATCTGTCAACGCCGCGCCGGGTGAGTCGGCCCCTCAAGTGTCAAC
GTCCGCCCCTCATCTGTCAGTGAGGGCCAAGTTTTCCGCGAGGTATCCAC
AACGCCGGCGGCCGCGGTGTCTCGCACACGGCTTCGACGGCGTTTCTGGC
GCGTTTGCAGGGCCATAGACGGCCGCCAGCCCAGCGGCGAGGGCAACCAG
CCCGGTGAGCGTCGCAAAGGAGATCCTGATCTGACTGATGGGCTGCCTGT
ATCGAGTGGTGATTTTGTGCCGAGCTGCCGGTCGGGGAGCTGTTGGCTGG
CTGGTGGCAGGATATATTGTGGTGTAAACAAATTGACGCTTAGACAACTT
AATAACACATTGCGGACGTTTTTAATGTACTGGGGTGGATGCAGTGGGCC
C
>pMZP_P6T (SEQ ID NO: 42) 
CACTCTGT GAAGCTCTTCA GCG TGAAGAGC GCAGCTGGCACGACAGGT
TTCCCGACTGGAAAGCGGGCAGTGAGCGCAACGCAATTAATGTGAGTTAG
CTCACTCATTAGGCACCCCAGGCTTTACACTTTATGCTTCCGGCTCGTAT
GTTGTGTGGAATTGTGAGCGGATAACAATTTCACACAGGAAACAGCTATG
ACCATGATTACGCCAAGCTTGCATGCCTGCAGGTCGACTCTAGAGGATCC
CCGGGTACCGAGCTCGAATTCACTGGCCGTCGTTTTACAACGTCGTGACT
GGGAAAACCCTGGCGTTACCCAACTTAATCGCCTTGCAGCACATCCCCCT
TTCGCCAGCTGGCGTAATAGCGAAGAGGCCCGCACCGATCGCCCTTCCCA
ACAGTTGCGCAGCCTGAATGGCGAATGGCGCCTGATGCGGTATTTTCTCC
TTACGCATCTGTGCGGTATTTCACACCGCATATGGTGCACTCTCAGTACA
ATCTGCTCTGATGCCGCATAGTTAAGCCAGCCCCGACACCCGCCAACACC
CGCTGACGCGCCCTGACGGGCTTGTCTGCTCCCGGCATCCGCTTACAGAC
AAGCTGTGAC GCTCTTCA TAG TGAAGAGCTTC ACGAAGTGGTTTAAAC
TATCAGTGTTTGACAGGATATATTGGCGGGTAAACCTAAGAGAAAAGAGC
GTTTATTAGAATAATCGGATATTTAAAAGGGCGTGAAAAGGTTTATCCGT
TCGTCCATTTGTATGTGCATGCCAACCACAGGGTTCCCCAGATCAGGCGC
TGGCTGCTGAACCCCCAGCCGGAACTGACCCCACAAGGCCCTAGCGTTTG
CAATGCACCAGGTCATCATTGACCCAGGCGTGTTCCACCAGGCCGCTGCC
TCGCAACTCTTCGCAGGCTTCGCCGACCTGCTCGCGCCACTTCTTCACGC
GGGTGGAATCCGATCCGCACATGAGGCGGAAGGTTTCCAGCTTGAGCGGG
TACGGCTCCCGGTGCGAGCTGAAATAGTCGAACATCCGTCGGGCCGTCGG
CGACAGCTTGCGGTACTTCTCCCATATGAATTTCGTGTAGTGGTCGCCAG
CAAACAGCACGACGATTTCCTCGTCGATCAGGACCTGGCAACGGGACGTT
TTCTTGCCACGGTCCAGGACGCGGAAGCGGTGCAGCAGCGACACCGATTC
CAGGTGCCCAACGCGGTCGGACGTGAAGCCCATCGCCGTCGCCTGTAGGC
GCGACAGGCATTCCTCGGCCTTCGTGTAATACCGGCCATTGATCGACCAG
CCCAGGTCCTGGCAAAGCTCGTAGAACGTGAAGGTGATCGGCTCGCCGAT
AGGGGTGCGCTTCGCGTACTCCAACACCTGCTGCCACACCAGTTCGTCAT
CGTCGGCCCGCAGCTCGACGCCGGTGTAGGTGATCTTCACGTCCTTGTTG
ACGTGGAAAATGACCTTGTTTTGCAGCGCCTCGCGCGGGATTTTCTTGTT
GCGCGTGGTGAACAGGGCAGAGCGGGCCGTGTCGTTTGGCATCGCTCGCA
TCGTGTCCGGCCACGGCGCAATATCGAACAAGGAAAGCTGCATTTCCTTG
ATCTGCTGCTTCGTGTGTTTCAGCAACGCGGCCTGCTTGGCCTCGCTGAC
CTGTTTTGCCAGGTCCTCGCCGGCGGTTTTTCGCTTCTTGGTCGTCATAG
TTCCTCGCGTGTCGATGGTCATCGACTTCGCCAAACCTGCCGCCTCCTGT
TCAAGACGACGCGAACGCTCCACGGCGGCCGATGGCGCGGGCAGGGCAGG
GGGAGCCAGTTGCACGCTGTCGCGCTCGATCTTGGCCGTAGCTTGCTGGA
CCATCGAGCCGACGGACTGGAAGGTTTCGCGGGGCGCACGCATGACGGTG
CGGCTTGCGATGGTTTCGGCATCCTCGGCGGAAAACCCCGCGTCGATCAG
TTCTTGCCTGTATGCCTTCCGGTCAAACGTCCGATTCATTCACCCTCCTT
GCGGGATTGCCCCGACTCACGCCGGGGCAATGTGCCCTTATTCCTGATTT
GACCCGCCTGGTGCCTTGGTGTCCAGATAATCCACCTTATCGGCAATGAA
GTCGGTCCCGTAGACCGTCTGGCCGTCCTTCTCGTACTTGGTATTCCGAA
TCTTGCCCTGCACGAATACCAGCGACCCCTTGCCCAAATACTTGCCGTGG
GCCTCGGCCTGAGAGCCAAAACACTTGATGCGGAAGAAGTCGGTGCGCTC
CTGCTTGTCGCCGGCATCGTTGCGCCACATCTAGGATCTGCCAGGAACCG
TAAAAAGGCCGCGTTGCTGGCGTTTTTCCATAGGCTCCGCCCCCCTGACG
AGCATCACAAAAATCGACGCTCAAGTCAGAGGTGGCGAAACCCGACAGGA
CTATAAAGATACCAGGCGTTTCCCCCTGGAAGCTCCCTCGTGCGCTCTCC
TGTTCCGACCCTGCCGCTTACCGGATACCTGTCCGCCTTTCTCCCTTCGG
GAAGCGTGGCGCTTTCTCATAGCTCACGCTGTAGGTATCTCAGTTCGGTG
TAGGTCGTTCGCTCCAAGCTGGGCTGTGTGCACGAACCCCCCGTTCAGCC
 
CGACCGCTGCGCCTTATCCGGTAACTATCGTCTTGAGTCCAACCCGGTAA
GACACGACTTATCGCCACTGGCAGCAGCCACTGGTAACAGGATTAGCAGA
GCGAGGTATGTAGGCGGTGCTACAGAGTTCTTGAAGTGGTGGCCTAACTA
CGGCTACACTAGAAGGACAGTATTTGGTATCTGCGCTCTGCTGAAGCCAG
TTACCTTCGGAAAAAGAGTTGGTAGCTCTTGATCCGGCAAACAAACCACC
GCTGGTAGCGGTGGTTTTTTTGTTTGCAAGCAGCAGATTACGCGCAGAAA
AAAAGGATCTCAAGAAGATCCTTTGATCTTTTCTACGGGGTCTGACGCTC
AGTGGAACGAAAACTCACGTTAAGGGATTTTGGTCATGAGATTATCAAAA
AGGATCTTCACCTAGATCCTTTTAAATTAAAAATGAAGTTTTAAATCAAT
CTAAAGTATATATGAGTAAACTTGGTCTGACAGTTACCAATGCTTAATCA
GTGAGGCACCTATCTCAGCGATCTGTCTATTTCGTTCATCCATAGTTGCC
TGACTCCCCGTCGTGTAGATAACTACGATACGGGAGGGCTTACCATCTGG
CCCCAGTGCTGCAATGATACCGCGAGAACCACGCTCACCGGCTCCAGATT
TATCAGCAATAAACCAGCCAGCCGGAAGGGCCGAGCGCAGAAGTGGTCCT
GCAACTTTATCCGCCTCCATCCAGTCTATTAATTGTTGCCGGGAAGCTAG
AGTAAGTAGTTCGCCAGTTAATAGTTTGCGCAACGTTGTTGCCATTGCTA
CAGGCATCGTGGTGTCACGCTCGTCGTTTGGTATGGCTTCATTCAGCTCC
GGTTCCCAACGATCAAGGCGAGTTACATGATCCCCCATGTTGTGCAAAAA
AGCGGTTAGCTCCTTCGGTCCTCCGATCGTTGTCAGAAGTAAGTTGGCCG
CAGTGTTATCACTCATGGTTATGGCAGCACTGCATAATTCTCTTACTGTC
ATGCCATCCGTAAGATGCTTTTCTGTGACTGGTGAGTACTCAACCAAGTC
ATTCTGAGAATAGTGTATGCGGCGACCGAGTTGCTCTTGCCCGGCGTCAA
TACGGGATAATACCGCGCCACATAGCAGAACTTTAAAAGTGCTCATCATT
GGAAAACGTTCTTCGGGGCGAAAACTCTCAAGGATCTTACCGCTGTTGAG
ATCCAGTTCGATGTAACCCACTCGTGCACCCAACTGATCTTCAGCATCTT
TTACTTTCACCAGCGTTTCTGGGTGAGCAAAAACAGGAAGGCAAAATGCC
GCAAAAAAGGGAATAAGGGCGACACGGAAATGTTGAATACTCATACTCTT
CCTTTTTCAATATTATTGAAGCATTTATCAGGGTTATTGTCTCATGAGCG
GATACATATTTGAATGTATTTAGAAAAATAAACAAATAGGGGTTCCGCGC
ACGAATTGGCCAGCGCTGCCATTTTTGGGGTGAGGCCGTTCGCGGCCGAG
GGGCGCAGCCCCTGGGGGGATGGGAGGCCCGCGTTAGCGGGCCGGGAGGG
TTCGAGAAGGGGGGGCACCCCCCTTCGGCGTGCGCGGTCACGCGCACAGG
GCGCAGCCCTGGTTAAAAACAAGGTTTATAAATATTGGTTTAAAAGCAGG
TTAAAAGACAGGTTAGCGGTGGCCGAAAAACGGGCGGAAACCCTTGCAAA
TGCTGGATTTTCTGCCTGTGGACAGCCCCTCAAATGTCAATAGGTGCGCC
CCTCATCTGTCAGCACTCTGCCCCTCAAGTGTCAAGGATCGCGCCCCTCA
TCTGTCAGTAGTCGCGCCCCTCAAGTGTCAATACCGCAGGGCACTTATCC
CCAGGCTTGTCCACATCATCTGTGGGAAACTCGCGTAAAATCAGGCGTTT
TCGCCGATTTGCGAGGCTGGCCAGCTCCACGTCGCCGGCCGAAATCGAGC
CTGCCCCTCATCTGTCAACGCCGCGCCGGGTGAGTCGGCCCCTCAAGTGT
CAACGTCCGCCCCTCATCTGTCAGTGAGGGCCAAGTTTTCCGCGAGGTAT
CCACAACGCCGGCGGCCGCGGTGTCTCGCACACGGCTTCGACGGCGTTTC
TGGCGCGTTTGCAGGGCCATAGACGGCCGCCAGCCCAGCGGCGAGGGCAA
CCAGCCCGGTGAGCGTCGCAAAGGAGATCCTGATCTGACTGATGGGCTGC
CTGTATCGAGTGGTGATTTTGTGCCGAGCTGCCGGTCGGGGAGCTGTTGG
CTGGCTGGTGGCAGGATATATTGTGGTGTAAACAAATTGACGCTTAGACA
ACTTAATAACACATTGCGGACGTTTTTAATGTACTGGGGTGGATGCAGTG
GGCCC
>pMXBG_A7I (SEQ ID NO: 43) 
CACTCTGT GACTTGGTCTCA TGCC TGAGACC GTCAGACCAAGTTTACT
CATATATACTTTAGATTGATTTAAAACTTCATTTTTAATTTAAAAGGATC
TAGGTGAAGATCCTTTTTGATAATCTCATGACCAAAATCCCTTAACGTGA
GTTTTCGTTCCACTGAGCGTCAGACCCCGTAGAAAAGATCAAAGGATCTT
CTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCT
TTTTCCGAAGGTAACTGGCTTCAGCAGAGCGCAGATACCAAATACTGTCC
TTCTAGTGTAGCCGTAGTTAGGCCACCACTTCAAGAACTCTGTAGCACCG
CCTACATACCTCGCTCTGCTAATCCTGTTACCAGTGGCTGCTGCCAGTGG
CGATAAGTCGTGTCTTACCGGGTTGGACTCAAGACGATAGTTACCGGATA
AGGCGCAGCGGTCGGGCTGAACGGGGGGTTCGTGCACACAGCCCAGCTTG
GAGCGAACGACCTACACCGAACTGAGATACCTACAGCGTGAGCTATGAGA
AAGCGCCACGCTTCCCGAAGGGAGAAAGGCGGACAGGTATCCGGTAAGCG
GCAGGGTCGGAACAGGAGAGCGCACGAGGGAGCTTCCAGGGGGAAACGCC
TGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAGCA
ACGCGGCCTTTTTACGGTTCCTGGCCTTTTGCTGGCCTTTTGCTCACATG
TTCTTTCCTGCGTTATCCCCTGATTCTGTGGATAACCGTATTACCGCCTT
TGAGTGAGCTGATACCGCTCGCCGCAGCCGAACGACCGAGCGCAGCGAGT
CAGTGAGCGAGGAAGCGGATGAGCGCCCAATACGCAAACCGCCTCTCCCC
GCGCGTTGGCCGATTCATTAATGCAGCTGGCACGACAGGTTTCCCGACTG
GAAAGCGGGCAGTGAGCGCAACGCAATTAATGTGAGTTAGCTCACTCATT
AGGCACCCCAGGCTTTACACTTTATGCTTCCGGCTCGTATGTTGTGTGGA
ATTGTGAGCGGATAACAATTTCACACAGGAAACAGCTATGACCATGATTA
CGCCAAGCTTGCATGCCTGCAGGTCGACTCTAGAGGATCCCCGGGTACCG
AGCTCGAATTCACTGGCCGTCGTTTTACAACGTCGTGACTGGGAAAACCC
TGGCGTTACCCAACTTAATCGCCTTGCAGCACATCCCCCTTTCGCCAGCT
GGCGTAATAGCGAAGAGGCCCGCACCGATCGCCCTTCCCAACAGTTGCGC
AGCCTGAATGGCGAATGGCGCCTGATGCGGTATTTTCTCCTTACGCATCT
GTGCGGTATTTCACACCGCATATGGTGCACTCTCAGTACAATCTGCTCTG
ATGCCGCATAGTTAAGCCAGCCCCGACACCCGCCAACACCCGCTGACGCG
CCCTGACGGGCTTGTCTGCTCCCGGCATCCGCTTACAGACAAGCTGTGAC
GGTCTCA CTTC TGAGACCAAGTC ACGAAGTGTGCGGCCGCCTTCAGCG
GCCGCACGAATCCATGTGGGAGTTTATTCTTGACACAGATATTTATGATA
TAATAACTGAGTAAGCTTAACATAAGGAGGAAAAACATATGTTACGCAGC
AGCAACGATGTTACGCAGCAGGGCAGTCGCCCTAAAACAAAGTTAGGTGG
CTCAAGTATGGGCATCATTCGCACATGTAGGCTCGGCCCTGACCAAGTCA
AATCCATGCGGGCTGCTCTTGATCTTTTCGGTCGTGAGTTCGGAGACGTA
GCCACCTACTCCCAACATCAGCCGGACTCCGATTACCTCGGGAACTTGCT
CCGTAGTAAGACATTCATCGCGCTTGCTGCCTTCGACCAAGAAGCGGTTG
TTGGCGCTCTCGCGGCTTACGTTCTGCCCAAGTTTGAGCAGCCGCGTAGT
GAGATCTATATCTATGATCTCGCAGTCTCCGGCGAGCACCGGAGGCAGGG
CATTGCCACCGCGCTCATCAATCTCCTCAAGCATGAGGCCAACGCGCTTG
GTGCTTATGTGATCTACGTGCAAGCAGATTACGGTGACGATCCCGCAGTG
GCTCTCTATACAAAGTTGGGCATACGGGAAGAAGTGATGCACTTTGATAT
CGACCCAAGTACCGCCACCTAAGAGCTGGAGCGGCGTAACCGTCGCACAG
GAAGGACAGAGAAAGCGCGGATCTGGGAAGTGACGGACAGAACGGTCAGG
ACCTGGATTGGGGAGGCGGTTGCCGCCGCTGCTGCTGACGGTGTGACGTT
CTCTGTTCCGGTCACACCACATACGTTCCGCCATTCCTATGCGATGCACA
TGCTGTATGCCGGTATACCGCTGAAAGTTCTGCAAAGCCTGATGGGACAT
AAGTCCATCAGTTCAACGGAAGTCTACACGAAGGTTTTTGCGCTGGATGT
GGCTGCCCGGCTCCGGGTGCAGTTTGCGATGCCGGAGTCTGATGCGGTTG
 
CGATGCTGAAACAATTATCCTGAGAATAAATGCCTTGGCCTTTATATGGA
AATGTGGAACTGAGTGGATATGCTGTTTTTGTCTGTTAAACAGAGAAGCT
GGCTGTTATCCACTGAGAAGCGAACGAAACAGTCGGGAAAATCTCCCATT
ATCGTAGAGATCCGCATTATTAATCTCAGGAGCCTGTGTAGCGTTTATAG
GAAGTAGTGTTCTGTCATGATGCCTGCAAGCGGTAACGAAAACGATTTGA
ATATGCCTTCAGGAACAATAGAAATCTTCGTGCGGTGTTACGTTGAAGTG
GAGCGGATTATGTCAGCAATGGACAGAACAACCTAATGAACACAGAACCA
TGATGTGGTCTGTCCTTTTACAGCCAGTAGTGCTCGCCGCAGTCGAGCGA
CAGGGCGAAGCCCTCGAGTGAGCGAGGAAGCACCAGGGAACAGCACTTAT
ATATTCTGCTTACACACGATGCCTGAAAAAACTTCCCTTGGGGTTATCCA
CTTATCCACGGGGATATTTTTATAATTATTTTTTTTATAGTTTTTAGATC
TTCTTTTTTAGAGCGCCTTGTAGGCCTTTATCCATGCTGGTTCTAGAGAA
GGTGTTGTGACAAATTGCCCTTTCAGTGTGACAAATCACCCTCAAATGAC
AGTCCTGTCTGTGACAAATTGCCCTTAACCCTGTGACAAATTGCCCTCAG
AAGAAGCTGTTTTTTCACAAAGTTATCCCTGCTTATTGACTCTTTTTTAT
TTAGTGTGACAATCTAAAAACTTGTCACACTTCACATGGATCTGTCATGG
CGGAAACAGCGGTTATCAATCACAAGAAACGTAAAAATAGCCCGCGAATC
GTCCAGTCAAACGACCTCACTGAGGCGGCATATAGTCTCTCCCGGGATCA
AAAACGTATGCTGTATCTGTTCGTTGACCAGATCAGAAAATCTGATGGCA
CCCTACAGGAACATGACGGTATCTGCGAGATCCATGTTGCTAAATATGCT
GAAATATTCGGATTGACCTCTGCGGAAGCCAGTAAGGATATACGGCAGGC
ATTGAAGAGTTTCGCGGGGAAGGAAGTGGTTTTTTATCGCCCTGAAGAGG
ATGCCGGCGATGAAAAAGGCTATGAATCTTTTCCTTGGTTTATCAAACGT
GCGCACAGTCCATCCAGAGGGCTTTACAGTGTACATATCAACCCATATCT
CATTCCCTTCTTTATCGGGTTACAGAACCGGTTTACGCAGTTTCGGCTTA
GTGAAACAAAAGAAATCACCAATCCGTATGCCATGCGTTTATACGAATCC
CTGTGTCAGTATCGTAAGCCGGATGGCTCAGGCATCGTGTCTCTGAAAAT
CGACTGGATCATAGAGCGTTACCAGCTGCCTCAAAGTTACCAGCGTATGC
CTGACTTCCGCCGCCGCTTCCTGCAGGTCTGTGTTAATGAGATCAACAGC
AGAACTCCAATGCGCCTCTCATACATTGAGAAAAAGAAAGGCCGCCAGAC
GACTCATATCGTATTTTCCTTCCGCGATATCACTTCCATGACGACAGGAT
AGTCTGAGGGTTATCTGTCACAGATTTGAGGGTGGTTCGTCACATTTGTT
CTGACCTACTGAGGGTAATTTGTCACAGTTTTGCTGTTTCCTTCAGCCTG
CATGGATTTTCTCATACTTTTTGAACTGTAATTTTTAAGGAAGCCAAATT
TGAGGGCAGTTTGTCACAGTTGATTTCCTTCTCTTTCCCTTCGTCATGTG
ACCTGATATCGGGGGTTAGTTCGTCATCATTGATGAGGGTTGATTATCAC
AGTTTATTACTCTGAATTGGCTATCCGCGTGTGTACCTCTACCTGGAGTT
TTTCCCACGGTGGATATTTCTTCTTGCGCTGAGCGTAAGAGCTATCTGAC
AGAACAGTTCTTCTTTGCTTCCTCGCCAGTTCGCTCGCTATGCTCGGTTA
CACGGCTGCGGCGAGCGCTAGTGATAATAAGTGACTGAGGTATGTGGTGT
TCTTATCTCCTTTTGTAGTGTTGCTCTTATTTTAAACAACTTTGCGGTTT
TTTGATGACTTTGCGATTTTGTTGTTGCTTTGCAGTAAATTGCAAGATTT
AATAAAAAAACGCAAAGCAATGATTAAAGGATGTTCAGAATGAAACTCAT
GGAAACACTTAACCAGTGCATAAACGCTGGTCATGAAATGACGAAGGCTA
TCGCCATTGCACAGTTTAATGATGACAGCCCGGAAGCGAGGAAAATAACC
CGGCGCTGGAGAATAGGTGAAGCAGCGGATTTAGTTGGGGTTTCTTCTCA
GGCTATCAGAGATGCCGAGAAAGCAGGGCGACTACCGCACCCGGATATGG
AAATTCGAGGACGGGTTGAGCAACGTGTTGGTTATACAATTGAACAAATT
AATCATATGCGTGATGTGTTTGGTACGCGATTGCGACGTGCTGAGGACGT
ATTTCCACCGGTGATCGGGGTTGCTGCCCATAAAGGTGGCGTTTACAAAA
CCTCAGTTTCTGTTCATCTTGCTCAGGATCTGGCTCTGAAGGGGCTACGT
GTTTTGCTCGTGGAAGGTAACGACCCCCAGGGAACAGCCTCAATGTATCA
CGGATGGGTACCAGATCTTCATATTCATGCAGAAGATACTCTCCTGCCTT
 
TCTATCTTGGGGAAAGGACGATGTCACTTATGCAATAAAGCCCACTTGC
TGGCCGGGGCTTGACATTATTCCTTCCTGTCTGGCTCTGCACCGTATTGA
AACTGAGTTAATGGGCAAATTTGATGAAGGTAAACTGCCCACCGATCCAC
ACCTGATGCTCCGACTGGCCATTGAAACTGTTGCTCATGACTATGATGTC
ATAGTTATTGACAGCGCGCCTAACCTGGGTATCGGCACGATTAATGTCGT
ATGTGCTGCTGATGTGCTGATTGTTCCCACGCCTGCTGAGTTGTTTGACT
ACACCTCCGCACTGCAGTTTTCGATATGCTTCGTGATCTGCTCAAGAAC
GTTGATCTTAAAGGGTTCGAGCCTGATGTACGTATTTTGCTTACCAAATA
CAGCAATAGTAATGGCTCTCAGTCCCCGTGGATGGAGGAGCAAATTCGGG
ATGCCTGGGGAAGCATGGTTCTAAAAAATGTTGTACGTGAAACGGATGAA
GTTGGTAAAGGTCAGATCCGGATGAGAACTGTTTTTGAACAGGCCATTGA
TCAACGCAGTTCAACTGGTGCCTGGAGAAATGCTCTTTCTATTTGGGAAC
CTGTCTGCAATGAAATTTTCGATCGTCTGATTAAACCACGCTGGAGATT
AGATAATGAAGCGTGCGCCTGTTATTCCAAAACATACGCTCAATACTCAA
CCGGTTGAAGATACTTCGTTATCGACACCAGCTGCCCCGATGGTGGATTC
GTTAATTGCGCGCGTAGGAGTAATGGCTCGCGGTAATGCCATTACTTTGC
CTGTATGTGGTCGGGATGTGAAGTTTACTCTTGAAGTGCTCCGGGGTGAT
AGTGTTGAGAAAACCTCTCGGGTATGGTCAGGTAATGAACGTGACCAGGA
GCTGCTTACTGAGGACGCACTGGATGATCTCATCCCTTCTTTTCTACTGA
CTGGTCAACAGACACCGGCGTTCGGTCGAAGAGTATCTGGTGTCATAGAA
ATTGCCGATGGGAGTCGCCGTCGTAAAGCTGCTGCACTTACCGAAAGTGA
TTATCGTGTTCTGGTTGGCGAGCTGGATGATGAGCAGATGGCTGCATTAT
CCAGATTGGGTAACGATTATCGCCCAACAAGTGCTTATGAACGTGGTCAG
CGTTATGCAAGCCGATTGCAGAATGAATTTGCTGGAAATATTTCTGCGCT
GGCTGATGCGGAAAATATTTCACGTAAGATTATTACCCGCTGTATCAACA
CCGCCAAATTGCCTAAATCAGTTGTTGCTCTTTTTTCTCACCCCGGTGAA
CTATCTGCCCGGTCAGGTGATGCACTTCAAAAAGCCTTTACAGATAAAGA
GGAATTACTTAAGCAGCAGGCATCTAACCTTCATGAGCAGAAAAAAGCTG
GGGTGATATTTGAAGCTGAAGAAGTTATCACTCTTTTAACTTCTGTGCTT
AAAACGTCATCTGCATCAAGAACTAGTTTAAGCTCACGACATCAGTTTGC
TCCTGGAGCGACAGTATTGTATAAGGGCGATAAAATGGTGCTTAACCTGG
ACAGGTCACGTGTTCCAACTGAGTGTATAGAGAAAATTGAGGCCATTCTT
AAGGAACTTGAAAAGCCAGCACCCTGATGCGACCACGTTTTAGTCTACGT
TTATCTGTCTTTACTTAATGTCCTTTGTTACAGGCCAGAAAGCATAACTG
GCCTGAATATTCTCTCTGGGCCCACTGTTCCACTTGTATCGTCGGTCTGA
TAATCAGACTGGGACCACGGTCCCACTCGTATCGTCGGTCTGATTATTAG
TCTGGGACCACGGTCCCACTCGTATCGTCGGTCTGATTATTAGTCTGGGA
CCACGGTCCCACTCGTATCGTCGGTCTGATAATCAGACTGGGACCACGGT
CCCACTCGTATCGTCGGTCTGATTATTAGTCTGGGACCATGGTCCCACTC
GTATCGTCGGTCTGATTATTAGTCTGGGACCACGGTCCCACTCGTATCGT
CGGTCTGATTATTAGTCTGGAACCACGGTCCCACTCGTATCGTCGGTCTG
ATTATTAGTCTGGGACCACGGTCCCACTCGTATCGTCGGTCTGATTATTA
GTCTGGGACCACGATCCCACTCGTGTTGTCGGTCTGATTATCGGTCTGGG
ACCACGGTCCCACTTGTATTGTCGATCAGACTATCAGCGTGAGACTACGA
TTCCATCAATGCCTGTCAAGGGCAAGTATTGACATGTCGTCGTAACCTGT
AGAACGGAGTAACCTCGGTGTGCGGTTGTATGCCTGCTGTGGATTGCTGC
TGTGTCCTGCTTATCCACAACATTTTGCGCACGGTTATGTGGACAAAATA
CCTGGTTACCCAGGCCGTGCCGGCACGTTAACCGGGCTGCATCCGATGCA
AGTGTGTCGCTGTCGACGCAAGCGGCCGCA
>pMXBG_I7G (SEQ ID NO: 44) 
CACTCTGT GACTTGGTCTCA CTTC TGAGACC GTCAGACCAAGTTTACT
CATATATACTTTAGATTGATTTAAAACTTCATTTTTAATTTAAAAGGATC
TAGGTGAAGATCCTTTTTGATAATCTCATGACCAAAATCCCTTAACGTGA
GTTTTCGTTCCACTGAGCGTCAGACCCCGTAGAAAAGATCAAAGGATCTT
CTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCT
TTTTCCGAAGGTAACTGGCTTCAGCAGAGCGCAGATACCAAATACTGTCC
TTCTAGTGTAGCCGTAGTTAGGCCACCACTTCAAGAACTCTGTAGCACCG
CCTACATACCTCGCTCTGCTAATCCTGTTACCAGTGGCTGCTGCCAGTGG
CGATAAGTCGTGTCTTACCGGGTTGGACTCAAGACGATAGTTACCGGATA
AGGCGCAGCGGTCGGGCTGAACGGGGGGTTCGTGCACACAGCCCAGCTTG
GAGCGAACGACCTACACCGAACTGAGATACCTACAGCGTGAGCTATGAGA
AAGCGCCACGCTTCCCGAAGGGAGAAAGGCGGACAGGTATCCGGTAAGCG
GCAGGGTCGGAACAGGAGAGCGCACGAGGGAGCTTCCAGGGGGAAACGCC
TGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAGCA
ACGCGGCCTTTTTACGGTTCCTGGCCTTTTGCTGGCCTTTTGCTCACATG
TTCTTTCCTGCGTTATCCCCTGATTCTGTGGATAACCGTATTACCGCCTT
TGAGTGAGCTGATACCGCTCGCCGCAGCCGAACGACCGAGCGCAGCGAGT
CAGTGAGCGAGGAAGCGGATGAGCGCCCAATACGCAAACCGCCTCTCCCC
GCGCGTTGGCCGATTCATTAATGCAGCTGGCACGACAGGTTTCCCGACTG
GAAAGCGGGCAGTGAGCGCAACGCAATTAATGTGAGTTAGCTCACTCATT
AGGCACCCCAGGCTTTACACTTTATGCTTCCGGCTCGTATGTTGTGTGGA
ATTGTGAGCGGATAACAATTTCACACAGGAAACAGCTATGACCATGATTA
CGCCAAGCTTGCATGCCTGCAGGTCGACTCTAGAGGATCCCCGGGTACCG
AGCTCGAATTCACTGGCCGTCGTTTTACAACGTCGTGACTGGGAAAACCC
TGGCGTTACCCAACTTAATCGCCTTGCAGCACATCCCCCTTTCGCCAGCT
GGCGTAATAGCGAAGAGGCCCGCACCGATCGCCCTTCCCAACAGTTGCGC
AGCCTGAATGGCGAATGGCGCCTGATGCGGTATTTTCTCCTTACGCATCT
GTGCGGTATTTCACACCGCATATGGTGCACTCTCAGTACAATCTGCTCTG
ATGCCGCATAGTTAAGCCAGCCCCGACACCCGCCAACACCCGCTGACGCG
CCCTGACGGGCTTGTCTGCTCCCGGCATCCGCTTACAGACAAGCTGTGAC
GGTCTCA GAGC TGAGACCAAGTC ACGAAGTGTGCGGCCGCCTTCAGCG
GCCGCACGAATCCATGTGGAGTTTATTCTTGACACAGATATTTATGATA
TAATAACTGAGTAAGCTTAACATAAGGAGGAAAAACATATGTTACGCAGC
AGCAACGATGTTACGCAGCAGGGCAGTCGCCCTAAAACAAAGTTAGGTGG
CTCAAGTATGGGCATCATTCGCACATGTAGGCTCGGCCCTGACCAAGTCA
AATCCATGCGGGCTGCTCTTGATCTTTTCGGTCGTGAGTTCGGAGACGTA
GCCACCTACTCCCAACATCAGCCGGACTCCGATTACCTCGGGAACTTGCT
CCGTAGTAAGACATTCATCGCGCTTGCTGCCTTCGACCAAGAAGCGGTTG
TTGGCGCTCTCGCGGCTTACGTTCTGCCCAAGTTTGAGCAGCCGCGTAGT
GAGATCTATATCTATGATCTCGCAGTCTCCGGCGAGCACCGGAGGCAGGG
CATTGCCACCGCGCTCATCAATCTCCTCAAGCATGAGGCCAACGCGCTTG
GTGCTTATGTGATCTACGTGCAAGCAGATTACGGTGACGATCCCGCAGTG
GCTCTCTATACAAAGTTGGGCATACGGGAAGAAGTGATGCACTTTGATAT
CGACCCAAGTACCGCCACCTAAGAGCTGGAGCGGCGTAACCGTCGCACAG
GAAGGACAGAGAAAGCGCGGATCTGGGAAGTGACGGACAGAACGGTCAGG
ACCTGGATTGGGGAGGCGGTTGCCGCCGCTGCTGCTGACGGTGTGACGTT
CTCTGTTCCGGTCACACCACATACGTTCCGCCATTCCTATGCGATGCACA
TGCTGTATGCCGGTATACCGCTGAAAGTTCTGCAAAGCCTGATGGGACAT
AAGTCCATCAGTTCAACGGAAGTCTACACGAAGGTTTTTGCGCTGGATGT
GGCTGCCCGGCTCCGGGTGCAGTTTGCGATGCCGGAGTCTGATGCGGTTG
 
CGATGCTGAAACAATTATCCTGAGAATAAATGCCTTGGCCTTTATATGGA
AATGTGGAACTGAGTGGATATGCTGTTTTTGTCTGTTAAACAGAGAAGCT
GGCTGTTATCCACTGAGAAGCGAACGAAACAGTCGGGAAAATCTCCCATT
ATCGTAGAGATCCGCATTATTAATCTCAGGAGCCTGTGTAGCGTTTATAG
GAAGTAGTGTTCTGTCATGATGCCTGCAAGCGGTAACGAAAACGATTTGA
ATATGCCTTCAGGAACAATAGAAATCTTCGTGCGGTGTTACGTTGAAGTG
GAGCGGATTATGTCAGCAATGGACAGAACAACCTAATGAACACAGAACCA
TGATGTGGTCTGTCCTTTTACAGCCAGTAGTGCTCGCCGCAGTCGAGCGA
CAGGGCGAAGCCCTCGAGTGAGCGAGGAAGCACCAGGGAACAGCACTTAT
ATATTCTGCTTACACACGATGCCTGAAAAAACTTCCCTTGGGGTTATCCA
CTTATCCACGGGGATATTTTTATAATTATTTTTTTTATAGTTTTTAGATC
TTCTTTTTTAGAGCGCCTTGTAGGCCTTTATCCATGCTGGTTCTAGAGAA
GGTGTTGTGACAAATTGCCCTTTCAGTGTGACAAATCACCCTCAAATGAC
AGTCCTGTCTGTGACAAATTGCCCTTAACCCTGTGACAAATTGCCCTCAG
AAGAAGCTGTTTTTTCACAAAGTTATCCCTGCTTATTGACTCTTTTTTAT
TTAGTGTGACAATCTAAAAACTTGTCACACTTCACATGGATCTGTCATGG
CGGAAACAGCGGTTATCAATCACAAGAAACGTAAAAATAGCCCGCGAATC
GTCCAGTCAAACGACCTCACTGAGGCGGCATATAGTCTCTCCCGGGATCA
AAAACGTATGCTGTATCTGTTCGTTGACCAGATCAGAAAATCTGATGGCA
CCCTACAGGAACATGACGGTATCTGCGAGATCCATGTTGCTAAATATGCT
GAAATATTCGGATTGACCTCTGCGGAAGCCAGTAAGGATATACGGCAGGC
ATTGAAGAGTTTCGCGGGGAAGGAAGTGGTTTTTTATCGCCCTGAAGAGG
ATGCCGGCGATGAAAAAGGCTATGAATCTTTTCCTTGGTTTATCAAACGT
GCGCACAGTCCATCCAGAGGGCTTTACAGTGTACATATCAACCCATATCT
CATTCCCTTCTTTATCGGGTTACAGAACCGGTTTACGCAGTTTCGGCTTA
GTGAAACAAAAGAAATCACCAATCCGTATGCCATGCGTTTATACGAATCC
CTGTGTCAGTATCGTAAGCCGGATGGCTCAGGCATCGTGTCTCTGAAAAT
CGACTGGATCATAGAGCGTTACCAGCTGCCTCAAAGTTACCAGCGTATGC
CTGACTTCCGCCGCCGCTTCCTGCAGGTCTGTGTTAATGAGATCAACAGC
AGAACTCCAATGCGCCTCTCATACATTGAGAAAAAGAAAGGCCGCCAGAC
GACTCATATCGTATTTTCCTTCCGCGATATCACTTCCATGACGACAGGAT
AGTCTGAGGGTTATCTGTCACAGATTTGAGGGTGGTTCGTCACATTTGTT
CTGACCTACTGAGGGTAATTTGTCACAGTTTTGCTGTTTCCTTCAGCCTG
CATGGATTTTCTCATACTTTTTGAACTGTAATTTTTAAGGAAGCCAAATT
TGAGGGCAGTTTGTCACAGTTGATTTCCTTCTCTTTCCCTTCGTCATGTG
ACCTGATATCGGGGGTTAGTTCGTCATCATTGATGAGGGTTGATTATCAC
AGTTTATTACTCTGAATTGGCTATCCGCGTGTGTACCTCTACCTGGAGTT
TTTCCCACGGTGGATATTTCTTCTTGCGCTGAGCGTAAGAGCTATCTGAC
AGAACAGTTCTTCTTTGCTTCCTCGCCAGTTCGCTCGCTATGCTCGGTTA
CACGGCTGCGGCGAGCGCTAGTGATAATAAGTGACTGAGGTATGTGGTGT
TCTTATCTCCTTTTGTAGTGTTGCTCTTATTTTAAACAACTTTGCGGTTT
TTTGATGACTTTGCGATTTTGTTGTTGCTTTGCAGTAAATTGCAAGATTT
AATAAAAAAACGCAAAGCAATGATTAAAGGATGTTCAGAATGAAACTCAT
GGAAACACTTAACCAGTGCATAAACGCTGGTCATGAAATGACGAAGGCTA
TCGCCATTGCACAGTTTAATGATGACAGCCCGGAAGCGAGGAAAATAACC
CGGCGCTGGAGAATAGGTGAAGCAGCGGATTTAGTTGGGGTTTCTTCTCA
GGCTATCAGAGATGCCGAGAAAGCAGGGCGACTACCGCACCCGGATATGG
AAATTCGAGGACGGGTTGAGCAACGTGTTGGTTATACAATTGAACAAATT
AATCATATGCGTGATGTGTTTGGTACGCGATTGCGACGTGCTGAGGACGT
ATTTCCACCGGTGATCGGGGTTGCTGCCCATAAAGGTGGCGTTTACAAAA
CCTCAGTTTCTGTTCATCTTGCTCAGGATCTGGCTCTGAAGGGGCTACGT
GTTTTGCTCGTGGAAGGTAACGACCCCCAGGGAACAGCCTCAATGTATCA
CGGATGGGTACCAGATCTTCATATTCATGCAGAAGATACTCTCCTGCCTT
 
TCTATCTTGGGGAAAGGACGATGTCACTTATGCAATAAAGCCCACTTGC
TGGCCGGGGCTTGACATTATTCCTTCCTGTCTGGCTCTGCACCGTATTGA
AACTGAGTTAATGGGCAAATTTGATGAAGGTAAACTGCCCACCGATCCAC
ACCTGATGCTCCGACTGGCCATTGAAACTGTTGCTCATGACTATGATGTC
ATAGTTATTGACAGCGCGCCTAACCTGGGTATCGGCACGATTAATGTCGT
ATGTGCTGCTGATGTGCTGATTGTTCCCACGCCTGCTGAGTTGTTTGACT
ACACCTCCGCACTGCAGTTTTTCGATATGCTTCGTGATCTGCTCAAGAAC
GTTGATCTTAAAGGGTTCGAGCCTGATGTACGTATTTTGCTTACCAAATA
CAGCAATAGTAATGGCTCTCAGTCCCCGTGGATGGAGGAGCAAATTCGGG
ATGCCTGGGGAAGCATGGTTCTAAAAAATGTTGTACGTGAAACGGATGAA
GTTGGTAAAGGTCAGATCCGGATGAGAACTGTTTTTGAACAGGCCATTGA
TCAACGCAGTTCAACTGGTGCCTGGAGAAATGCTCTTTCTATTTGGGAAC
CTGTCTGCAATGAAATTTTCGATCGTCTGATTAAACCACGCTGGGAGATT
AGATAATGAAGCGTGCGCCTGTTATTCCAAAACATACGCTCAATACTCAA
CCGGTTGAAGATACTTCGTTATCGACACCAGCTGCCCCGATGGTGGATTC
GTTAATTGCGCGCGTAGGAGTAATGGCTCGCGGTAATGCCATTACTTTGC
CTGTATGTGGTCGGGATGTGAAGTTTACTCTTGAAGTGCTCCGGGGTGAT
AGTGTTGAGAAAACCTCTCGGGTATGGTCAGGTAATGAACGTGACCAGGA
GCTGCTTACTGAGGACGCACTGGATGATCTCATCCCTTCTTTTCTACTGA
CTGGTCAACAGACACCGGCGTTCGGTCGAAGAGTATCTGGTGTCATAGAA
ATTGCCGATGGGAGTCGCCGTCGTAAAGCTGCTGCACTTACCGAAAGTGA
TTATCGTGTTCTGGTTGGCGAGCTGGATGATGAGCAGATGGCTGCATTAT
CCAGATTGGGTAACGATTATCGCCCAACAAGTGCTTATGAACGTGGTCAG
CGTTATGCAAGCCGATTGCAGAATGAATTTGCTGGAAATATTTCTGCGCT
GGCTGATGCGGAAAATATTTCACGTAAGATTATTACCCGCTGTATCAACA
CCGCCAAATTGCCTAAATCAGTTGTTGCTCTTTTTTCTCACCCCGGTGAA
CTATCTGCCCGGTCAGGTGATGCACTTCAAAAAGCCTTTACAGATAAAGA
GGAATTACTTAAGCAGCAGGCATCTAACCTTCATGAGCAGAAAAAAGCTG
GGGTGATATTTGAAGCTGAAGAAGTTATCACTCTTTTAACTTCTGTGCTT
AAAACGTCATCTGCATCAAGAACTAGTTTAAGCTCACGACATCAGTTTGC
TCCTGGAGCGACAGTATTGTATAAGGGCGATAAAATGGTGCTTAACCTGG
ACAGGTCACGTGTTCCAACTGAGTGTATAGAGAAAATTGAGGCCATTCTT
AAGGAACTTGAAAAGCCAGCACCCTGATGCGACCACGTTTTAGTCTACGT
TTATCTGTCTTTACTTAATGTCCTTTGTTACAGGCCAGAAAGCATAACTG
GCCTGAATATTCTCTCTGGGCCCACTGTTCCACTTGTATCGTCGGTCTGA
TAATCAGACTGGGACCACGGTCCCACTCGTATCGTCGGTCTGATTATTAG
TCTGGGACCACGGTCCCACTCGTATCGTCGGTCTGATTATTAGTCTGGGA
CCACGGTCCCACTCGTATCGTCGGTCTGATAATCAGACTGGGACCACGGT
CCCACTCGTATCGTCGGTCTGATTATTAGTCTGGGACCATGGTCCCACTC
GTATCGTCGGTCTGATTATTAGTCTGGGACCACGGTCCCACTCGTATCGT
CGGTCTGATTATTAGTCTGGAACCACGGTCCCACTCGTATCGTCGGTCTG
ATTATTAGTCTGGGACCACGGTCCCACTCGTATCGTCGGTCTGATTATTA
GTCTGGGACCACGATCCCACTCGTGTTGTCGGTCTGATTATCGGTCTGGG
ACCACGGTCCCACTTGTATTGTCGATCAGACTATCAGCGTGAGACTACGA
TTCCATCAATGCCTGTCAAGGGCAAGTATTGACATGTCGTCGTAACCTGT
AGAACGGAGTAACCTCGGTGTGCGGTTGTATGCCTGCTGTGGATTGCTGC
TGTGTCCTGCTTATCCACAACATTTTGCGCACGGTTATGTGGACAAAATA
CCTGGTTACCCAGGCCGTGCCGGCACGTTAACCGGGCTGCATCCGATGCA
AGTGTGTCGCTGTCGACGCAAGCGGCCGCA
>pMXBG_G7F (SEQ ID NO: 45) 
CACTCTGT GACTTGGTCTCA GAGC TGAGACC GTCAGACCAAGTTTACT
CATATATACTTTAGATTGATTTAAAACTTCATTTTTAATTTAAAAGGATC
TAGGTGAAGATCCTTTTTGATAATCTCATGACCAAAATCCCTTAACGTGA
GTTTTCGTTCCACTGAGCGTCAGACCCCGTAGAAAAGATCAAAGGATCTT
CTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCT
TTTTCCGAAGGTAACTGGCTTCAGCAGAGCGCAGATACCAAATACTGTCC
TTCTAGTGTAGCCGTAGTTAGGCCACCACTTCAAGAACTCTGTAGCACCG
CCTACATACCTCGCTCTGCTAATCCTGTTACCAGTGGCTGCTGCCAGTGG
CGATAAGTCGTGTCTTACCGGGTTGGACTCAAGACGATAGTTACCGGATA
AGGCGCAGCGGTCGGGCTGAACGGGGGGTTCGTGCACACAGCCCAGCTTG
GAGCGAACGACCTACACCGAACTGAGATACCTACAGCGTGAGCTATGAGA
AAGCGCCACGCTTCCCGAAGGGAGAAAGGCGGACAGGTATCCGGTAAGCG
GCAGGGTCGGAACAGGAGAGCGCACGAGGGAGCTTCCAGGGGGAAACGCC
TGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAGCA
ACGCGGCCTTTTTACGGTTCCTGGCCTTTTGCTGGCCTTTTGCTCACATG
TTCTTTCCTGCGTTATCCCCTGATTCTGTGGATAACCGTATTACCGCCTT
TGAGTGAGCTGATACCGCTCGCCGCAGCCGAACGACCGAGCGCAGCGAGT
CAGTGAGCGAGGAAGCGGATGAGCGCCCAATACGCAAACCGCCTCTCCCC
GCGCGTTGGCCGATTCATTAATGCAGCTGGCACGACAGGTTTCCCGACTG
GAAAGCGGGCAGTGAGCGCAACGCAATTAATGTGAGTTAGCTCACTCATT
AGGCACCCCAGGCTTTACACTTTATGCTTCCGGCTCGTATGTTGTGTGGA
ATTGTGAGCGGATAACAATTTCACACAGGAAACAGCTATGACCATGATTA
CGCCAAGCTTGCATGCCTGCAGGTCGACTCTAGAGGATCCCCGGGTACCG
AGCTCGAATTCACTGGCCGTCGTTTTACAACGTCGTGACTGGGAAAACCC
TGGCGTTACCCAACTTAATCGCCTTGCAGCACATCCCCCTTTCGCCAGCT
GGCGTAATAGCGAAGAGGCCCGCACCGATCGCCCTTCCCAACAGTTGCGC
AGCCTGAATGGCGAATGGCGCCTGATGCGGTATTTTCTCCTTACGCATCT
GTGCGGTATTTCACACCGCATATGGTGCACTCTCAGTACAATCTGCTCTG
ATGCCGCATAGTTAAGCCAGCCCCGACACCCGCCAACACCCGCTGACGCG
CCCTGACGGGCTTGTCTGCTCCCGGCATCCGCTTACAGACAAGCTGTGAC
GGTCTCA TGTG TGAGACCAAGTC ACGAAGTGTGCGGCCGCCTTCAGCG
GCCGCACGAATCCATGTGGGAGTTTATTCTTGACACAGATATTTATGATA
TAATAACTGAGTAAGCTTAACATAAGGAGGAAAAACATATGTTACGCAGC
AGCAACGATGTTACGCAGCAGGGCAGTCGCCCTAAAACAAAGTTAGGTGG
CTCAAGTATGGGCATCATTCGCACATGTAGGCTCGGCCCTGACCAAGTCA
AATCCATGCGGGCTGCTCTTGATCTTTTCGGTCGTGAGTTCGGAGACGTA
GCCACCTACTCCCAACATCAGCCGGACTCCGATTACCTCGGGAACTTGCT
CCGTAGTAAGACATTCATCGCGCTTGCTGCCTTCGACCAAGAAGCGGTTG
TTGGCGCTCTCGCGGCTTACGTTCTGCCCAAGTTTGAGCAGCCGCGTAGT
GAGATCTATATCTATGATCTCGCAGTCTCCGGCGAGCACCGGAGGCAGGG
CATTGCCACCGCGCTCATCAATCTCCTCAAGCATGAGGCCAACGCGCTTG
GTGCTTATGTGATCTACGTGCAAGCAGATTACGGTGACGATCCCGCAGTG
GCTCTCTATACAAAGTTGGGCATACGGGAAGAAGTGATGCACTTTGATAT
CGACCCAAGTACCGCCACCTAAGAGCTGGAGCGGCGTAACCGTCGCACAG
GAAGGACAGAGAAAGCGCGGATCTGGGAAGTGACGGACAGAACGGTCAGG
ACCTGGATTGGGGAGGCGGTTGCCGCCGCTGCTGCTGACGGTGTGACGTT
CTCTGTTCCGGTCACACCACATACGTTCCGCCATTCCTATGCGATGCACA
TGCTGTATGCCGGTATACCGCTGAAAGTTCTGCAAAGCCTGATGGGACAT
AAGTCCATCAGTTCAACGGAAGTCTACACGAAGGTTTTTGCGCTGGATGT
GGCTGCCCGGCTCCGGGTGCAGTTTGCGATGCCGGAGTCTGATGCGGTTG
 
CGATGCTGAAACAATTATCCTGAGAATAAATGCCTTGGCCTTTATATGGA
AATGTGGAACTGAGTGGATATGCTGTTTTTGTCTGTTAAACAGAGAAGCT
GGCTGTTATCCACTGAGAAGCGAACGAAACAGTCGGGAAAATCTCCCATT
ATCGTAGAGATCCGCATTATTAATCTCAGGAGCCTGTGTAGCGTTTATAG
GAAGTAGTGTTCTGTCATGATGCCTGCAAGCGGTAACGAAAACGATTTGA
ATATGCCTTCAGGAACAATAGAAATCTTCGTGCGGTGTTACGTTGAAGTG
GAGCGGATTATGTCAGCAATGGACAGAACAACCTAATGAACACAGAACCA
TGATGTGGTCTGTCCTTTTACAGCCAGTAGTGCTCGCCGCAGTCGAGCGA
CAGGGCGAAGCCCTCGAGTGAGCGAGGAAGCACCAGGGAACAGCACTTAT
ATATTCTGCTTACACACGATGCCTGAAAAAACTTCCCTTGGGGTTATCCA
CTTATCCACGGGGATATTTTTATAATTATTTTTTTTATAGTTTTTAGATC
TTCTTTTTTAGAGCGCCTTGTAGGCCTTTATCCATGCTGGTTCTAGAGAA
GGTGTTGTGACAAATTGCCCTTTCAGTGTGACAAATCACCCTCAAATGAC
AGTCCTGTCTGTGACAAATTGCCCTTAACCCTGTGACAAATTGCCCTCAG
AAGAAGCTGTTTTTTCACAAAGTTATCCCTGCTTATTGACTCTTTTTTAT
TTAGTGTGACAATCTAAAAACTTGTCACACTTCACATGGATCTGTCATGG
CGGAAACAGCGGTTATCAATCACAAGAAACGTAAAAATAGCCCGCGAATC
GTCCAGTCAAACGACCTCACTGAGGCGGCATATAGTCTCTCCCGGGATCA
AAAACGTATGCTGTATCTGTTCGTTGACCAGATCAGAAAATCTGATGGCA
CCCTACAGGAACATGACGGTATCTGCGAGATCCATGTTGCTAAATATGCT
GAAATATTCGGATTGACCTCTGCGGAAGCCAGTAAGGATATACGGCAGGC
ATTGAAGAGTTTCGCGGGGAAGGAAGTGGTTTTTTATCGCCCTGAAGAGG
ATGCCGGCGATGAAAAAGGCTATGAATCTTTTCCTTGGTTTATCAAACGT
GCGCACAGTCCATCCAGAGGGCTTTACAGTGTACATATCAACCCATATCT
CATTCCCTTCTTTATCGGGTTACAGAACCGGTTTACGCAGTTTCGGCTTA
GTGAAACAAAAGAAATCACCAATCCGTATGCCATGCGTTTATACGAATCC
CTGTGTCAGTATCGTAAGCCGGATGGCTCAGGCATCGTGTCTCTGAAAAT
CGACTGGATCATAGAGCGTTACCAGCTGCCTCAAAGTTACCAGCGTATGC
CTGACTTCCGCCGCCGCTTCCTGCAGGTCTGTGTTAATGAGATCAACAGC
AGAACTCCAATGCGCCTCTCATACATTGAGAAAAAGAAAGGCCGCCAGAC
GACTCATATCGTATTTTCCTTCCGCGATATCACTTCCATGACGACAGGAT
AGTCTGAGGGTTATCTGTCACAGATTTGAGGGTGGTTCGTCACATTTGTT
CTGACCTACTGAGGGTAATTTGTCACAGTTTTGCTGTTTCCTTCAGCCTG
CATGGATTTTCTCATACTTTTTGAACTGTAATTTTTAAGGAAGCCAAATT
TGAGGGCAGTTTGTCACAGTTGATTTCCTTCTCTTTCCCTTCGTCATGTG
ACCTGATATCGGGGGTTAGTTCGTCATCATTGATGAGGGTTGATTATCAC
AGTTTATTACTCTGAATTGGCTATCCGCGTGTGTACCTCTACCTGGAGTT
TTTCCCACGGTGGATATTTCTTCTTGCGCTGAGCGTAAGAGCTATCTGAC
AGAACAGTTCTTCTTTGCTTCCTCGCCAGTTCGCTCGCTATGCTCGGTTA
CACGGCTGCGGCGAGCGCTAGTGATAATAAGTGACTGAGGTATGTGGTGT
TCTTATCTCCTTTTGTAGTGTTGCTCTTATTTTAAACAACTTTGCGGTTT
TTTGATGACTTTGCGATTTTGTTGTTGCTTTGCAGTAAATTGCAAGATTT
AATAAAAAAACGCAAAGCAATGATTAAAGGATGTTCAGAATGAAACTCAT
GGAAACACTTAACCAGTGCATAAACGCTGGTCATGAAATGACGAAGGCTA
TCGCCATTGCACAGTTTAATGATGACAGCCCGGAAGCGAGGAAAATAACC
CGGCGCTGGAGAATAGGTGAAGCAGCGGATTTAGTTGGGGTTTCTTCTCA
GGCTATCAGAGATGCCGAGAAAGCAGGGCGACTACCGCACCCGGATATGG
AAATTCGAGGACGGGTTGAGCAACGTGTTGGTTATACAATTGAACAAATT
AATCATATGCGTGATGTGTTTGGTACGCGATTGCGACGTGCTGAGGACGT
ATTTCCACCGGTGATCGGGGTTGCTGCCCATAAAGGTGGCGTTTACAAAA
CCTCAGTTTCTGTTCATCTTGCTCAGGATCTGGCTCTGAAGGGGCTACGT
GTTTTGCTCGTGGAAGGTAACGACCCCCAGGGAACAGCCTCAATGTATCA
CGGATGGGTACCAGATCTTCATATTCATGCAGAAGATACTCTCCTGCCTT
 
TCTATCTTGGGGAAAGGACGATGTCACTTATGCAATAAAGCCCACTTGC
TGGCCGGGGCTTGACATTATTCCTTCCTGTCTGGCTCTGCACCGTATTGA
AACTGAGTTAATGGGCAAATTTGATGAAGGTAAACTGCCCACCGATCCAC
ACCTGATGCTCCGACTGGCCATTGAAACTGTTGCTCATGACTATGATGTC
ATAGTTATTGACAGCGCGCCTAACCTGGGTATCGGCACGATTAATGTCGT
ATGTGCTGCTGATGTGCTGATTGTTCCCACGCCTGCTGAGTTGTTTGACT
ACACCTCCGCACTGCAGTTTTCGATATGCTTCGTGATCTGCTCAAGAAC
GTTGATCTTAAAGGGTTCGAGCCTGATGTACGTATTTTGCTTACCAAATA
CAGCAATAGTAATGGCTCTCAGTCCCCGTGGATGGAGGAGCAAATTCGGG
ATGCCTGGGGAAGCATGGTTCTAAAAAATGTTGTACGTGAAACGGATGAA
GTTGGTAAAGGTCAGATCCGGATGAGAACTGTTTTTGAACAGGCCATTGA
TCAACGCAGTTCAACTGGTGCCTGGAGAAATGCTCTTTCTATTTGGGAAC
CTGTCTGCAATGAAATTTTCGATCGTCTGATTAAACCACGCTGGAGATT
AGATAATGAAGCGTGCGCCTGTTATTCCAAAACATACGCTCAATACTCAA
CCGGTTGAAGATACTTCGTTATCGACACCAGCTGCCCCGATGGTGGATTC
GTTAATTGCGCGCGTAGGAGTAATGGCTCGCGGTAATGCCATTACTTTGC
CTGTATGTGGTCGGGATGTGAAGTTTACTCTTGAAGTGCTCCGGGGTGAT
AGTGTTGAGAAAACCTCTCGGGTATGGTCAGGTAATGAACGTGACCAGGA
GCTGCTTACTGAGGACGCACTGGATGATCTCATCCCTTCTTTTCTACTGA
CTGGTCAACAGACACCGGCGTTCGGTCGAAGAGTATCTGGTGTCATAGAA
ATTGCCGATGGGAGTCGCCGTCGTAAAGCTGCTGCACTTACCGAAAGTGA
TTATCGTGTTCTGGTTGGCGAGCTGGATGATGAGCAGATGGCTGCATTAT
CCAGATTGGGTAACGATTATCGCCCAACAAGTGCTTATGAACGTGGTCAG
CGTTATGCAAGCCGATTGCAGAATGAATTTGCTGGAAATATTTCTGCGCT
GGCTGATGCGGAAAATATTTCACGTAAGATTATTACCCGCTGTATCAACA
CCGCCAAATTGCCTAAATCAGTTGTTGCTCTTTTTTCTCACCCCGGTGAA
CTATCTGCCCGGTCAGGTGATGCACTTCAAAAAGCCTTTACAGATAAAGA
GGAATTACTTAAGCAGCAGGCATCTAACCTTCATGAGCAGAAAAAAGCTG
GGGTGATATTTGAAGCTGAAGAAGTTATCACTCTTTTAACTTCTGTGCTT
AAAACGTCATCTGCATCAAGAACTAGTTTAAGCTCACGACATCAGTTTGC
TCCTGGAGCGACAGTATTGTATAAGGGCGATAAAATGGTGCTTAACCTGG
ACAGGTCACGTGTTCCAACTGAGTGTATAGAGAAAATTGAGGCCATTCTT
AAGGAACTTGAAAAGCCAGCACCCTGATGCGACCACGTTTTAGTCTACGT
TTATCTGTCTTTACTTAATGTCCTTTGTTACAGGCCAGAAAGCATAACTG
GCCTGAATATTCTCTCTGGGCCCACTGTTCCACTTGTATCGTCGGTCTGA
TAATCAGACTGGGACCACGGTCCCACTCGTATCGTCGGTCTGATTATTAG
TCTGGGACCACGGTCCCACTCGTATCGTCGGTCTGATTATTAGTCTGGGA
CCACGGTCCCACTCGTATCGTCGGTCTGATAATCAGACTGGGACCACGGT
CCCACTCGTATCGTCGGTCTGATTATTAGTCTGGGACCATGGTCCCACTC
GTATCGTCGGTCTGATTATTAGTCTGGGACCACGGTCCCACTCGTATCGT
CGGTCTGATTATTAGTCTGGAACCACGGTCCCACTCGTATCGTCGGTCTG
ATTATTAGTCTGGGACCACGGTCCCACTCGTATCGTCGGTCTGATTATTA
GTCTGGGACCACGATCCCACTCGTGTTGTCGGTCTGATTATCGGTCTGGG
ACCACGGTCCCACTTGTATTGTCGATCAGACTATCAGCGTGAGACTACGA
TTCCATCAATGCCTGTCAAGGGCAAGTATTGACATGTCGTCGTAACCTGT
AGAACGGAGTAACCTCGGTGTGCGGTTGTATGCCTGCTGTGGATTGCTGC
TGTGTCCTGCTTATCCACAACATTTTGCGCACGGTTATGTGGACAAAATA
CCTGGTTACCCAGGCCGTGCCGGCACGTTAACCGGGCTGCATCCGATGCA
AGTGTGTCGCTGTCGACGCAAGCGGCCGCA
>pMXBG_F7E (SEQ ID NO: 46) 
CACTCTGT GACTTGGTCTCA TGTG TGAGACC GTCAGACCAAGTTTACT
CATATATACTTTAGATTGATTTAAAACTTCATTTTTAATTTAAAAGGATC
TAGGTGAAGATCCTTTTTGATAATCTCATGACCAAAATCCCTTAACGTGA
GTTTTCGTTCCACTGAGCGTCAGACCCCGTAGAAAAGATCAAAGGATCTT
CTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCT
TTTTCCGAAGGTAACTGGCTTCAGCAGAGCGCAGATACCAAATACTGTCC
TTCTAGTGTAGCCGTAGTTAGGCCACCACTTCAAGAACTCTGTAGCACCG
CCTACATACCTCGCTCTGCTAATCCTGTTACCAGTGGCTGCTGCCAGTGG
CGATAAGTCGTGTCTTACCGGGTTGGACTCAAGACGATAGTTACCGGATA
AGGCGCAGCGGTCGGGCTGAACGGGGGGTTCGTGCACACAGCCCAGCTTG
GAGCGAACGACCTACACCGAACTGAGATACCTACAGCGTGAGCTATGAGA
AAGCGCCACGCTTCCCGAAGGGAGAAAGGCGGACAGGTATCCGGTAAGCG
GCAGGGTCGGAACAGGAGAGCGCACGAGGGAGCTTCCAGGGGGAAACGCC
TGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAGCA
ACGCGGCCTTTTTACGGTTCCTGGCCTTTTGCTGGCCTTTTGCTCACATG
TTCTTTCCTGCGTTATCCCCTGATTCTGTGGATAACCGTATTACCGCCTT
TGAGTGAGCTGATACCGCTCGCCGCAGCCGAACGACCGAGCGCAGCGAGT
CAGTGAGCGAGGAAGCGGATGAGCGCCCAATACGCAAACCGCCTCTCCCC
GCGCGTTGGCCGATTCATTAATGCAGCTGGCACGACAGGTTTCCCGACTG
GAAAGCGGGCAGTGAGCGCAACGCAATTAATGTGAGTTAGCTCACTCATT
AGGCACCCCAGGCTTTACACTTTATGCTTCCGGCTCGTATGTTGTGTGGA
ATTGTGAGCGGATAACAATTTCACACAGGAAACAGCTATGACCATGATTA
CGCCAAGCTTGCATGCCTGCAGGTCGACTCTAGAGGATCCCCGGGTACCG
AGCTCGAATTCACTGGCCGTCGTTTTACAACGTCGTGACTGGGAAAACCC
TGGCGTTACCCAACTTAATCGCCTTGCAGCACATCCCCCTTTCGCCAGCT
GGCGTAATAGCGAAGAGGCCCGCACCGATCGCCCTTCCCAACAGTTGCGC
AGCCTGAATGGCGAATGGCGCCTGATGCGGTATTTTCTCCTTACGCATCT
GTGCGGTATTTCACACCGCATATGGTGCACTCTCAGTACAATCTGCTCTG
ATGCCGCATAGTTAAGCCAGCCCCGACACCCGCCAACACCCGCTGACGCG
CCCTGACGGGCTTGTCTGCTCCCGGCATCCGCTTACAGACAAGCTGTGAC
GGTCTCA CAGA TGAGACCAAGTC ACGAAGTGTGCGGCCGCCTTCAGCG
GCCGCACGAATCCATGTGGGAGTTTATTCTTGACACAGATATTTATGATA
TAATAACTGAGTAAGCTTAACATAAGGAGGAAAAACATATGTTACGCAGC
AGCAACGATGTTACGCAGCAGGGCAGTCGCCCTAAAACAAAGTTAGGTGG
CTCAAGTATGGGCATCATTCGCACATGTAGGCTCGGCCCTGACCAAGTCA
AATCCATGCGGGCTGCTCTTGATCTTTTCGGTCGTGAGTTCGGAGACGTA
GCCACCTACTCCCAACATCAGCCGGACTCCGATTACCTCGGGAACTTGCT
CCGTAGTAAGACATTCATCGCGCTTGCTGCCTTCGACCAAGAAGCGGTTG
TTGGCGCTCTCGCGGCTTACGTTCTGCCCAAGTTTGAGCAGCCGCGTAGT
GAGATCTATATCTATGATCTCGCAGTCTCCGGCGAGCACCGGAGGCAGGG
CATTGCCACCGCGCTCATCAATCTCCTCAAGCATGAGGCCAACGCGCTTG
GTGCTTATGTGATCTACGTGCAAGCAGATTACGGTGACGATCCCGCAGTG
GCTCTCTATACAAAGTTGGGCATACGGGAAGAAGTGATGCACTTTGATAT
CGACCCAAGTACCGCCACCTAAGAGCTGGAGCGGCGTAACCGTCGCACAG
GAAGGACAGAGAAAGCGCGGATCTGGGAAGTGACGGACAGAACGGTCAGG
ACCTGGATTGGGGAGGCGGTTGCCGCCGCTGCTGCTGACGGTGTGACGTT
CTCTGTTCCGGTCACACCACATACGTTCCGCCATTCCTATGCGATGCACA
TGCTGTATGCCGGTATACCGCTGAAAGTTCTGCAAAGCCTGATGGGACAT
AAGTCCATCAGTTCAACGGAAGTCTACACGAAGGTTTTTGCGCTGGATGT
GGCTGCCCGGCTCCGGGTGCAGTTTGCGATGCCGGAGTCTGATGCGGTTG
 
CGATGCTGAAACAATTATCCTGAGAATAAATGCCTTGGCCTTTATATGGA
AATGTGGAACTGAGTGGATATGCTGTTTTTGTCTGTTAAACAGAGAAGCT
GGCTGTTATCCACTGAGAAGCGAACGAAACAGTCGGGAAAATCTCCCATT
ATCGTAGAGATCCGCATTATTAATCTCAGGAGCCTGTGTAGCGTTTATAG
GAAGTAGTGTTCTGTCATGATGCCTGCAAGCGGTAACGAAAACGATTTGA
ATATGCCTTCAGGAACAATAGAAATCTTCGTGCGGTGTTACGTTGAAGTG
GAGCGGATTATGTCAGCAATGGACAGAACAACCTAATGAACACAGAACCA
TGATGTGGTCTGTCCTTTTACAGCCAGTAGTGCTCGCCGCAGTCGAGCGA
CAGGGCGAAGCCCTCGAGTGAGCGAGGAAGCACCAGGGAACAGCACTTAT
ATATTCTGCTTACACACGATGCCTGAAAAAACTTCCCTTGGGGTTATCCA
CTTATCCACGGGGATATTTTTATAATTATTTTTTTTATAGTTTTTAGATC
TTCTTTTTTAGAGCGCCTTGTAGGCCTTTATCCATGCTGGTTCTAGAGAA
GGTGTTGTGACAAATTGCCCTTTCAGTGTGACAAATCACCCTCAAATGAC
AGTCCTGTCTGTGACAAATTGCCCTTAACCCTGTGACAAATTGCCCTCAG
AAGAAGCTGTTTTTTCACAAAGTTATCCCTGCTTATTGACTCTTTTTTAT
TTAGTGTGACAATCTAAAAACTTGTCACACTTCACATGGATCTGTCATGG
CGGAAACAGCGGTTATCAATCACAAGAAACGTAAAAATAGCCCGCGAATC
GTCCAGTCAAACGACCTCACTGAGGCGGCATATAGTCTCTCCCGGGATCA
AAAACGTATGCTGTATCTGTTCGTTGACCAGATCAGAAAATCTGATGGCA
CCCTACAGGAACATGACGGTATCTGCGAGATCCATGTTGCTAAATATGCT
GAAATATTCGGATTGACCTCTGCGGAAGCCAGTAAGGATATACGGCAGGC
ATTGAAGAGTTTCGCGGGGAAGGAAGTGGTTTTTTATCGCCCTGAAGAGG
ATGCCGGCGATGAAAAAGGCTATGAATCTTTTCCTTGGTTTATCAAACGT
GCGCACAGTCCATCCAGAGGGCTTTACAGTGTACATATCAACCCATATCT
CATTCCCTTCTTTATCGGGTTACAGAACCGGTTTACGCAGTTTCGGCTTA
GTGAAACAAAAGAAATCACCAATCCGTATGCCATGCGTTTATACGAATCC
CTGTGTCAGTATCGTAAGCCGGATGGCTCAGGCATCGTGTCTCTGAAAAT
CGACTGGATCATAGAGCGTTACCAGCTGCCTCAAAGTTACCAGCGTATGC
CTGACTTCCGCCGCCGCTTCCTGCAGGTCTGTGTTAATGAGATCAACAGC
AGAACTCCAATGCGCCTCTCATACATTGAGAAAAAGAAAGGCCGCCAGAC
GACTCATATCGTATTTTCCTTCCGCGATATCACTTCCATGACGACAGGAT
AGTCTGAGGGTTATCTGTCACAGATTTGAGGGTGGTTCGTCACATTTGTT
CTGACCTACTGAGGGTAATTTGTCACAGTTTTGCTGTTTCCTTCAGCCTG
CATGGATTTTCTCATACTTTTTGAACTGTAATTTTTAAGGAAGCCAAATT
TGAGGGCAGTTTGTCACAGTTGATTTCCTTCTCTTTCCCTTCGTCATGTG
ACCTGATATCGGGGGTTAGTTCGTCATCATTGATGAGGGTTGATTATCAC
AGTTTATTACTCTGAATTGGCTATCCGCGTGTGTACCTCTACCTGGAGTT
TTTCCCACGGTGGATATTTCTTCTTGCGCTGAGCGTAAGAGCTATCTGAC
AGAACAGTTCTTCTTTGCTTCCTCGCCAGTTCGCTCGCTATGCTCGGTTA
CACGGCTGCGGCGAGCGCTAGTGATAATAAGTGACTGAGGTATGTGGTGT
TCTTATCTCCTTTTGTAGTGTTGCTCTTATTTTAAACAACTTTGCGGTTT
TTTGATGACTTTGCGATTTTGTTGTTGCTTTGCAGTAAATTGCAAGATTT
AATAAAAAAACGCAAAGCAATGATTAAAGGATGTTCAGAATGAAACTCAT
GGAAACACTTAACCAGTGCATAAACGCTGGTCATGAAATGACGAAGGCTA
TCGCCATTGCACAGTTTAATGATGACAGCCCGGAAGCGAGGAAAATAACC
CGGCGCTGGAGAATAGGTGAAGCAGCGGATTTAGTTGGGGTTTCTTCTCA
GGCTATCAGAGATGCCGAGAAAGCAGGGCGACTACCGCACCCGGATATGG
AAATTCGAGGACGGGTTGAGCAACGTGTTGGTTATACAATTGAACAAATT
AATCATATGCGTGATGTGTTTGGTACGCGATTGCGACGTGCTGAGGACGT
ATTTCCACCGGTGATCGGGGTTGCTGCCCATAAAGGTGGCGTTTACAAAA
CCTCAGTTTCTGTTCATCTTGCTCAGGATCTGGCTCTGAAGGGGCTACGT
GTTTTGCTCGTGGAAGGTAACGACCCCCAGGGAACAGCCTCAATGTATCA
CGGATGGGTACCAGATCTTCATATTCATGCAGAAGATACTCTCCTGCCTT
 
TCTATCTTGGGGAAAGGACGATGTCACTTATGCAATAAAGCCCACTTGC
TGGCCGGGGCTTGACATTATTCCTTCCTGTCTGGCTCTGCACCGTATTGA
AACTGAGTTAATGGGCAAATTTGATGAAGGTAAACTGCCCACCGATCCAC
ACCTGATGCTCCGACTGGCCATTGAAACTGTTGCTCATGACTATGATGTC
ATAGTTATTGACAGCGCGCCTAACCTGGGTATCGGCACGATTAATGTCGT
ATGTGCTGCTGATGTGCTGATTGTTCCCACGCCTGCTGAGTTGTTTGACT
ACACCTCCGCACTGCAGTTTTTCGATATGCTTCGTGATCTGCTCAAGAAC
GTTGATCTTAAAGGGTTCGAGCCTGATGTACGTATTTTGCTTACCAAATA
CAGCAATAGTAATGGCTCTCAGTCCCCGTGGATGGAGGAGCAAATTCGGG
ATGCCTGGGGAAGCATGGTTCTAAAAAATGTTGTACGTGAAACGGATGAA
GTTGGTAAAGGTCAGATCCGGATGAGAACTGTTTTTGAACAGGCCATTGA
TCAACGCAGTTCAACTGGTGCCTGGAGAAATGCTCTTTCTATTTGGGAAC
CTGTCTGCAATGAAATTTTCGATCGTCTGATTAAACCACGCTGGGAGATT
AGATAATGAAGCGTGCGCCTGTTATTCCAAAACATACGCTCAATACTCAA
CCGGTTGAAGATACTTCGTTATCGACACCAGCTGCCCCGATGGTGGATTC
GTTAATTGCGCGCGTAGGAGTAATGGCTCGCGGTAATGCCATTACTTTGC
CTGTATGTGGTCGGGATGTGAAGTTTACTCTTGAAGTGCTCCGGGGTGAT
AGTGTTGAGAAAACCTCTCGGGTATGGTCAGGTAATGAACGTGACCAGGA
GCTGCTTACTGAGGACGCACTGGATGATCTCATCCCTTCTTTTCTACTGA
CTGGTCAACAGACACCGGCGTTCGGTCGAAGAGTATCTGGTGTCATAGAA
ATTGCCGATGGGAGTCGCCGTCGTAAAGCTGCTGCACTTACCGAAAGTGA
TTATCGTGTTCTGGTTGGCGAGCTGGATGATGAGCAGATGGCTGCATTAT
CCAGATTGGGTAACGATTATCGCCCAACAAGTGCTTATGAACGTGGTCAG
CGTTATGCAAGCCGATTGCAGAATGAATTTGCTGGAAATATTTCTGCGCT
GGCTGATGCGGAAAATATTTCACGTAAGATTATTACCCGCTGTATCAACA
CCGCCAAATTGCCTAAATCAGTTGTTGCTCTTTTTTCTCACCCCGGTGAA
CTATCTGCCCGGTCAGGTGATGCACTTCAAAAAGCCTTTACAGATAAAGA
GGAATTACTTAAGCAGCAGGCATCTAACCTTCATGAGCAGAAAAAAGCTG
GGGTGATATTTGAAGCTGAAGAAGTTATCACTCTTTTAACTTCTGTGCTT
AAAACGTCATCTGCATCAAGAACTAGTTTAAGCTCACGACATCAGTTTGC
TCCTGGAGCGACAGTATTGTATAAGGGCGATAAAATGGTGCTTAACCTGG
ACAGGTCACGTGTTCCAACTGAGTGTATAGAGAAAATTGAGGCCATTCTT
AAGGAACTTGAAAAGCCAGCACCCTGATGCGACCACGTTTTAGTCTACGT
TTATCTGTCTTTACTTAATGTCCTTTGTTACAGGCCAGAAAGCATAACTG
GCCTGAATATTCTCTCTGGGCCCACTGTTCCACTTGTATCGTCGGTCTGA
TAATCAGACTGGGACCACGGTCCCACTCGTATCGTCGGTCTGATTATTAG
TCTGGGACCACGGTCCCACTCGTATCGTCGGTCTGATTATTAGTCTGGGA
CCACGGTCCCACTCGTATCGTCGGTCTGATAATCAGACTGGGACCACGGT
CCCACTCGTATCGTCGGTCTGATTATTAGTCTGGGACCATGGTCCCACTC
GTATCGTCGGTCTGATTATTAGTCTGGGACCACGGTCCCACTCGTATCGT
CGGTCTGATTATTAGTCTGGAACCACGGTCCCACTCGTATCGTCGGTCTG
ATTATTAGTCTGGGACCACGGTCCCACTCGTATCGTCGGTCTGATTATTA
GTCTGGGACCACGATCCCACTCGTGTTGTCGGTCTGATTATCGGTCTGGG
ACCACGGTCCCACTTGTATTGTCGATCAGACTATCAGCGTGAGACTACGA
TTCCATCAATGCCTGTCAAGGGCAAGTATTGACATGTCGTCGTAACCTGT
AGAACGGAGTAACCTCGGTGTGCGGTTGTATGCCTGCTGTGGATTGCTGC
TGTGTCCTGCTTATCCACAACATTTTGCGCACGGTTATGTGGACAAAATA
CCTGGTTACCCAGGCCGTGCCGGCACGTTAACCGGGCTGCATCCGATGCA
AGTGTGTCGCTGTCGACGCAAGCGGCCGCA
>pMXBK A7E (SEQ ID NO: 47)

CACTCTGT GACTTGGTCTCA TGCC TGAGACC GTCAGACCAAGTTTACT
CATATATACTTTAGATTGATTTAAAACTTCATTTTTAATTTAAAAGGATC
TAGGTGAAGATCCTTTTTGATAATCTCATGACCAAAATCCCTTAACGTGA
GTTTTCGTTCCACTGAGCGTCAGACCCCGTAGAAAAGATCAAAGGATCTT
CTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCT
TTTTCCGAAGGTAACTGGCTTCAGCAGAGCGCAGATACCAAATACTGTCC
TTCTAGTGTAGCCGTAGTTAGGCCACCACTTCAAGAACTCTGTAGCACCG
CCTACATACCTCGCTCTGCTAATCCTGTTACCAGTGGCTGCTGCCAGTGG
CGATAAGTCGTGTCTTACCGGGTTGGACTCAAGACGATAGTTACCGGATA
AGGCGCAGCGGTCGGGCTGAACGGGGGGTTCGTGCACACAGCCCAGCTTG
GAGCGAACGACCTACACCGAACTGAGATACCTACAGCGTGAGCTATGAGA
AAGCGCCACGCTTCCCGAAGGGAGAAAGGCGGACAGGTATCCGGTAAGCG
GCAGGGTCGGAACAGGAGAGCGCACGAGGGAGCTTCCAGGGGGAAACGCC
TGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAGCA
ACGCGGCCTTTTTACGGTTCCTGGCCTTTTGCTGGCCTTTTGCTCACATG
TTCTTTCCTGCGTTATCCCCTGATTCTGTGGATAACCGTATTACCGCCTT
TGAGTGAGCTGATACCGCTCGCCGCAGCCGAACGACCGAGCGCAGCGAGT
CAGTGAGCGAGGAAGCGGATGAGCGCCCAATACGCAAACCGCCTCTCCCC
GCGCGTTGGCCGATTCATTAATGCAGCTGGCACGACAGGTTTCCCGACTG
GAAAGCGGGCAGTGAGCGCAACGCAATTAATGTGAGTTAGCTCACTCATT
AGGCACCCCAGGCTTTACACTTTATGCTTCCGGCTCGTATGTTGTGTGGA
ATTGTGAGCGGATAACAATTTCACACAGGAAACAGCTATGACCATGATTA
CGCCAAGCTTGCATGCCTGCAGGTCGACTCTAGAGGATCCCCGGGTACCG
AGCTCGAATTCACTGGCCGTCGTTTTACAACGTCGTGACTGGGAAAACCC
TGGCGTTACCCAACTTAATCGCCTTGCAGCACATCCCCCTTTCGCCAGCT
GGCGTAATAGCGAAGAGGCCCGCACCGATCGCCCTTCCCAACAGTTGCGC
AGCCTGAATGGCGAATGGCGCCTGATGCGGTATTTTCTCCTTACGCATCT
GTGCGGTATTTCACACCGCATATGGTGCACTCTCAGTACAATCTGCTCTG
ATGCCGCATAGTTAAGCCAGCCCCGACACCCGCCAACACCCGCTGACGCG
CCCTGACGGGCTTGTCTGCTCCCGGCATCCGCTTACAGACAAGCTGTGAC
GGTCTCA CAGA TGAGACCAAGTC ACGAAGTGTGCGGCCGCCTTCGTGC
GCGGAACCCCTATTTGTTTATTTTTCTAAATACATTCAAATATGTATCCG
CTCATGAGACAATAACCCTGATAAATGCTTCAATAATATTGAAAAAGGAA
GAGTATGGCTAAAATGAGAATATCACCGGAATTGAAAAAACTGATCGAAA
AATACCGCTGCGTAAAAGATACGGAAGGAATGTCTCCTGCTAAGGTATAT
AAGCTGGTGGGAGAAAATGAAAACCTATATTTAAAAATGACGGACAGCCG
GTATAAAGGGACCACCTATGATGTGGAACGGGAAAAGGACATGATGCTAT
GGCTGGAAGGAAAGCTGCCTGTTCCAAAGGTCCTGCACTTTGAACGGCAT
GATGGCTGGAGCAATCTGCTCATGAGTGAGGCCGATGGCGTCCTTTGCTC
GGAAGAGTATGAAGATGAACAAAGCCCTGAAAAGATTATCGAGCTGTATG
CGGAGTGCATCAGGCTCTTTCACTCCATCGACATATCGGATTGTCCCTAT
ACGAATAGCTTAGACAGCCGCTTAGCCGAATTGGATTACTTACTGAATAA
CGATCTGGCCGATGTGGATTGCGAAAACTGGGAAGAGGACACTCCATTTA
AAGATCCGCGCGAGCTGTATGATTTTTTAAAGACGGAAAAGCCCGAAGAG
GAACTTGTCTTTTCCCACGGCGACCTGGGAGACAGCAACATCTTTGTGAA
AGATGGCAAAGTAAGTGGCTTTATTGATCTTGGGAGAAGCGGCAGGGCGG
ACAAGTGGTATGACATTGCCTTCTGCGTCCGGTCGCTCAGGGAGGATATC
GGGGAAGAACAGTATGTCGAGCTATTTTTTGACTTACTGGGGATCAAGCC
TGATTGGGAGAAAATAAAATATTATATTTTACTGGATGAATTGTTTTAGG
AGCTGGAGCGGCGTAACCGTCGCACAGGAAGGACAGAGAAAGCGCGGATC

TGGGAAGTGACGGACAGAACGGTCAGGACCTGGATTGGGGAGGCGGTTGC
CGCCGCTGCTGCTGACGGTGTGACGTTCTCTGTTCCGGTCACACCACATA
CGTTCCGCCATTCCTATGCGATGCACATGCTGTATGCCGGTATACCGCTG
AAAGTTCTGCAAAGCCTGATGGGACATAAGTCCATCAGTTCAACGGAAGT
CTACACGAAGGTTTTTGCGCTGGATGTGGCTGCCCGGCTCCGGGTGCAGT
TTGCGATGCCGGAGTCTGATGCGGTTGCGATGCTGAAACAATTATCCTGA
GAATAAATGCCTTGGCCTTTATATGGAAATGTGGAACTGAGTGGATATGC
TGTTTTTGTCTGTTAAACAGAGAAGCTGGCTGTTATCCACTGAGAAGCGA
ACGAAACAGTCGGGAAAATCTCCCATTATCGTAGAGATCCGCATTATTAA
TCTCAGGAGCCTGTGTAGCGTTTATAGGAAGTAGTGTTCTGTCATGATGC
CTGCAAGCGGTAACGAAAACGATTTGAATATGCCTTCAGGAACAATAGAA
ATCTTCGTGCGGTGTTACGTTGAAGTGGAGCGGATTATGTCAGCAATGGA
CAGAACAACCTAATGAACACAGAACCATGATGTGGTCTGTCCTTTTACAG
CCAGTAGTGCTCGCCGCAGTCGAGCGACAGGGCGAAGCCCTCGAGTGAGC
GAGGAAGCACCAGGGAACAGCACTTATATATTCTGCTTACACACGATGCC
TGAAAAAACTTCCCTTGGGGTTATCCACTTATCCACGGGGATATTTTTAT
AATTATTTTTTTTATAGTTTTTAGATCTTCTTTTTTAGAGCGCCTTGTAG
GCCTTTATCCATGCTGGTTCTAGAGAAGGTGTTGTGACAAATTGCCCTTT
CAGTGTGACAAATCACCCTCAAATGACAGTCCTGTCTGTGACAAATTGCC
CTTAACCCTGTGACAAATTGCCCTCAGAAGAAGCTGTTTTTTCACAAAGT
TATCCCTGCTTATTGACTCTTTTTTATTTAGTGTGACAATCTAAAAACTT
GTCACACTTCACATGGATCTGTCATGGCGGAAACAGCGGTTATCAATCAC
AAGAAACGTAAAAATAGCCCGCGAATCGTCCAGTCAAACGACCTCACTGA
GGCGGCATATAGTCTCTCCCGGGATCAAAAACGTATGCTGTATCTGTTCG
TTGACCAGATCAGAAAATCTGATGGCACCCTACAGGAACATGACGGTATC
TGCGAGATCCATGTTGCTAAATATGCTGAAATATTCGGATTGACCTCTGC
GGAAGCCAGTAAGGATATACGGCAGGCATTGAAGAGTTTCGCGGGGAAGG
AAGTGGTTTTTTATCGCCCTGAAGAGGATGCCGGCGATGAAAAAGGCTAT
GAATCTTTTCCTTGGTTTATCAAACGTGCGCACAGTCCATCCAGAGGGCT
TTACAGTGTACATATCAACCCATATCTCATTCCCTTCTTTATCGGGTTAC
AGAACCGGTTTACGCAGTTTCGGCTTAGTGAAACAAAAGAAATCACCAAT
CCGTATGCCATGCGTTTATACGAATCCCTGTGTCAGTATCGTAAGCCGGA
TGGCTCAGGCATCGTGTCTCTGAAAATCGACTGGATCATAGAGCGTTACC
AGCTGCCTCAAAGTTACCAGCGTATGCCTGACTTCCGCCGCCGCTTCCTG
CAGGTCTGTGTTAATGAGATCAACAGCAGAACTCCAATGCGCCTCTCATA
CATTGAGAAAAAGAAAGGCCGCCAGACGACTCATATCGTATTTTCCTTCC
GCGATATCACTTCCATGACGACAGGATAGTCTGAGGGTTATCTGTCACAG
ATTTGAGGGTGGTTCGTCACATTTGTTCTGACCTACTGAGGGTAATTTGT
CACAGTTTTGCTGTTTCCTTCAGCCTGCATGGATTTTCTCATACTTTTTG
AACTGTAATTTTTAAGGAAGCCAAATTTGAGGGCAGTTTGTCACAGTTGA
TTTCCTTCTCTTTCCCTTCGTCATGTGACCTGATATCGGGGGTTAGTTCG
TCATCATTGATGAGGGTTGATTATCACAGTTTATTACTCTGAATTGGCTA
TCCGCGTGTGTACCTCTACCTGGAGTTTTTCCCACGGTGGATATTTCTTC
TTGCGCTGAGCGTAAGAGCTATCTGACAGAACAGTTCTTCTTTGCTTCCT
CGCCAGTTCGCTCGCTATGCTCGGTTACACGGCTGCGGCGAGCGCTAGTG
ATAATAAGTGACTGAGGTATGTGGTGTTCTTATCTCCTTTTGTAGTGTTG
CTCTTATTTTAAACAACTTTGCGGTTTTTTGATGACTTTGCGATTTTGTT
GTTGCTTTGCAGTAAATTGCAAGATTTAATAAAAAAACGCAAAGCAATGA
TTAAAGGATGTTCAGAATGAAACTCATGGAAACACTTAACCAGTGCATAA
ACGCTGGTCATGAAATGACGAAGGCTATCGCCATTGCACAGTTTAATGAT
GACAGCCCGGAAGCGAGGAAAATAACCCGGCGCTGGAGAATAGGTGAAGC
AGCGGATTTAGTTGGGGTTTCTTCTCAGGCTATCAGAGATGCCGAGAAAG
CAGGGCGACTACCGCACCCGGATATGGAAATTCGAGGACGGGTTGAGCAA

CGTGTTGGTTATACAATTGAACAAATTAATCATATGCGTGATGTGTTTGG
TACGCGATTGCGACGTGCTGAGGACGTATTTCCACCGGTGATCGGGGTTG
CTGCCCATAAAGGTGGCGTTTACAAAACCTCAGTTTCTGTTCATCTTGCT
CAGGATCTGGCTCTGAAGGGGCTACGTGTTTTGCTCGTGGAAGGTAACGA
CCCCCAGGGAACAGCCTCAATGTATCACGGATGGGTACCAGATCTTCATA
TTCATGCAGAAGATACTCTCCTGCCTTTCTATCTTGGGGAAAAGGACGAT
GTCACTTATGCAATAAAGCCCACTTGCTGGCCGGGGCTTGACATTATTCC
TTCCTGTCTGGCTCTGCACCGTATTGAAACTGAGTTAATGGGCAAATTTG
ATGAAGGTAAACTGCCCACCGATCCACACCTGATGCTCCGACTGGCCATT
GAAACTGTTGCTCATGACTATGATGTCATAGTTATTGACAGCGCGCCTAA
CCTGGGTATCGGCACGATTAATGTCGTATGTGCTGCTGATGTGCTGATTG
TTCCCACGCCTGCTGAGTTGTTTGACTACACCTCCGCACTGCAGTTTTTC
GATATGCTTCGTGATCTGCTCAAGAACGTTGATCTTAAAGGGTTCGAGCC
TGATGTACGTATTTTGCTTACCAAATACAGCAATAGTAATGGCTCTCAGT
CCCCGTGGATGGAGGAGCAAATTCGGGATGCCTGGGGAAGCATGGTTCTA
AAAAATGTTGTACGTGAAACGGATGAAGTTGGTAAAGGTCAGATCCGGAT
GAGAACTGTTTTTGAACAGGCCATTGATCAACGCAGTTCAACTGGTGCCT
GGAGAAATGCTCTTTCTATTTGGGAACCTGTCTGCAATGAAATTTTCGAT
CGTCTGATTAAACCACGCTGGGAGATTAGATAATGAAGCGTGCGCCTGTT
ATTCCAAAACATACGCTCAATACTCAACCGGTTGAAGATACTTCGTTATC
GACACCAGCTGCCCCGATGGTGGATTCGTTAATTGCGCGCGTAGGAGTAA
TGGCTCGCGGTAATGCCATTACTTTGCCTGTATGTGGTCGGGATGTGAAG
TTTACTCTTGAAGTGCTCCGGGGTGATAGTGTTGAGAAAACCTCTCGGGT
ATGGTCAGGTAATGAACGTGACCAGGAGCTGCTTACTGAGGACGCACTGG
ATGATCTCATCCCTTCTTTTCTACTGACTGGTCAACAGACACCGGCGTTC
GGTCGAAGAGTATCTGGTGTCATAGAAATTGCCGATGGGAGTCGCCGTCG
TAAAGCTGCTGCACTTACCGAAAGTGATTATCGTGTTCTGGTTGGCGAGC
TGGATGATGAGCAGATGGCTGCATTATCCAGATTGGGTAACGATTATCGC
CCAACAAGTGCTTATGAACGTGGTCAGCGTTATGCAAGCCGATTGCAGAA
TGAATTTGCTGGAAATATTTCTGCGCTGGCTGATGCGGAAAATATTTCAC
GTAAGATTATTACCCGCTGTATCAACACCGCCAAATTGCCTAAATCAGTT
GTTGCTCTTTTTTCTCACCCCGGTGAACTATCTGCCCGGTCAGGTGATGC
ACTTCAAAAAGCCTTTACAGATAAAGAGGAATTACTTAAGCAGCAGGCAT
CTAACCTTCATGAGCAGAAAAAAGCTGGGGTGATATTTGAAGCTGAAGAA
GTTATCACTCTTTTAACTTCTGTGCTTAAAACGTCATCTGCATCAAGAAC
TAGTTTAAGCTCACGACATCAGTTTGCTCCTGGAGCGACAGTATTGTATA
AGGGCGATAAAATGGTGCTTAACCTGGACAGGTCACGTGTTCCAACTGAG
TGTATAGAGAAAATTGAGGCCATTCTTAAGGAACTTGAAAAGCCAGCACC
CTGATGCGACCACGTTTTAGTCTACGTTTATCTGTCTTTACTTAATGTCC
TTTGTTACAGGCCAGAAAGCATAACTGGCCTGAATATTCTCTCTGGGCCC
ACTGTTCCACTTGTATCGTCGGTCTGATAATCAGACTGGGACCACGGTCC
CACTCGTATCGTCGGTCTGATTATTAGTCTGGGACCACGGTCCCACTCGT
ATCGTCGGTCTGATTATTAGTCTGGGACCACGGTCCCACTCGTATCGTCG
GTCTGATAATCAGACTGGGACCACGGTCCCACTCGTATCGTCGGTCTGAT
TATTAGTCTGGGACCATGGTCCCACTCGTATCGTCGGTCTGATTATTAGT
CTGGGACCACGGTCCCACTCGTATCGTCGGTCTGATTATTAGTCTGGAAC
CACGGTCCCACTCGTATCGTCGGTCTGATTATTAGTCTGGGACCACGGTC
CCACTCGTATCGTCGGTCTGATTATTAGTCTGGGACCACGATCCCACTCG
TGTTGTCGGTCTGATTATCGGTCTGGGACCACGGTCCCACTTGTATTGTC
GATCAGACTATCAGCGTGAGACTACGATTCCATCAATGCCTGTCAAGGGC
AAGTATTGACATGTCGTCGTAACCTGTAGAACGGAGTAACCTCGGTGTGC
GGTTGTATGCCTGCTGTGGATTGCTGCTGTGTCCTGCTTATCCACAACAT
TTTGCGCACGGTTATGTGGACAAAATACCTGGTTACCCAGGCCGTGCCGG

CACGTTAACCGGGCTGCATCCGATGCAAGTGTGTCGCTGTCGACGCAAGC
GGCCGCA

## Claims

1. A nucleic acid for use in DNA assembly, wherein the nucleic acid comprises at least one methylation-protectable restriction element, the methylation-protectable restriction element comprising:
- a type IIS restriction enzyme recognition sequence; and
- a DNA methylase recognition sequence,
wherein the type IIS restriction enzyme recognition sequence and the DNA methylase recognition sequence overlap such that the base methylatable by the DNA methylase lies within the type IIS restriction enzyme recognition sequence,
wherein the DNA methylase recognition sequence is not identical to or enclosed by the type IIS restriction enzyme recognition sequence, and
wherein the DNA methylase recognition sequence does not overlap with the sequence that would form the overhang end sequence generated by the type IIS restriction enzyme.
wherein the nucleic acid further comprises an opposing non-protectable type IIS restriction enzyme recognition sequence provided on the opposing side of the cut site of the methylation-protectable restriction element thereby forming a truncating composite element,
wherein the opposing type IIS restriction enzyme recognition sequence of the truncating composite element is arranged to direct the type IIS restriction enzyme to cut the nucleic acid at a site that is within the recognition sequence of the type IIS restriction enzyme recognition sequence of the methylation-protectable restriction element, and/or within the nucleotides between the cut site and the recognition sequence of the type IIS restriction enzyme recognition sequence of the methylation-protectable restriction element.

2. The nucleic acid according to claim 1, wherein the nucleic acid comprises first and second methylation-protectable restriction elements,
and wherein the nucleic acid further comprises an opposing type IIS restriction enzyme recognition sequences provided on the opposing side of the cut site of each of the first and second methylation-protectable restriction elements to form:
A i) a maintaining composite element or an insertional composite element, and ii) a truncating composite element; or
B) two truncating composite elements,
wherein the maintaining composite element is an arrangement wherein the opposing type IIS restriction enzyme recognition sequence is arranged to direct the type IIS restriction enzyme to cut the nucleic acid at the same site as the type IIS restriction enzyme recognition sequence of the opposing methylation-protectable restriction element, such that the same overhang is produced,
wherein the insertional composite element is an arrangement comprising a functional sequence insert provided between the methylation-protectable restriction element and the opposing type IIS restriction enzyme recognition sequence, and
wherein the tuncating composite element is an arrangement wherein the opposing type IIS restriction enzyme recognition sequence of the truncating composite element is arranged to direct the type IIS restriction enzyme to cut the nucleic acid at a site that is within the recognition sequence of the type IIS restriction enzyme recognition sequence of the methylation-protectable restriction element, and/or within the nucleotides between the cut site and the recognition sequence of the type IIS restriction enzyme recognition sequence of the methylation-protectable restriction element.

3. The nucleic acid according to claim 2, wherein the nucleic acid comprises nucleic acid sequence between the cut sites of the two methylation-protectable restriction elements, which is a discard sequence; or
wherein the nucleic acid is linearized vector having a methylation-protectable restriction element at each end.

4. The nucleic acid according to claim 3, wherein the discard sequence comprises a selectable marker, reporter gene, or label.

5. The nucleic acid according to any preceding claims, wherein the restriction enzyme recognition sequence of the methylation-protectable restriction element is recognised by the same restriction enzyme species as the opposing non-protectable restriction enzyme recognition sequence; and/or
wherein the DNA methylase recognition sequences of each methylation-protectable restriction element is recognised by the same methylase.

6. The nucleic acid according to any preceding claim, wherein the methylation-protectable restriction element comprises or consists of the sequence GACNNGGTCTCNNNNN (BsaI/M.Osp807II - SEQ ID NO: 1) or GAAGACGCNNNNNN (BpiI/M2.NmeMC58II - SEQ ID NO: 2) or GAAGCTCTTCNNNN (LguI/M.XmnI - SEQ ID NO: 3).

7. A method of scarless DNA assembly of DNA fragments comprising the steps of:
(A) providing a first linearised methylated nucleic acid by restriction enzyme cutting of a first nucleic acid, wherein the first nucleic acid comprises first and second methylation-protectable restriction elements with a discard sequence therebetween, or providing a previously restriction enzyme cut version thereof that has the discard sequence excised,
wherein the first and second methylation-protectable restriction elements of the first nucleic acid each comprise:
- a type IIS restriction enzyme recognition sequence; and
- a DNA methylase recognition sequence,
wherein the type IIS restriction enzyme recognition sequence and the DNA methylase recognition sequence overlap such that the base methylatable by the DNA methylase lies within the type IIS restriction enzyme recognition sequence,
wherein the DNA methylase recognition sequence is not identical to or enclosed by the type IIS restriction enzyme recognition sequence, and
wherein the DNA methylase recognition sequence does not overlap with the sequence that would form the overhang end sequence generated by the type IIS restriction enzyme, and
wherein the first and second methylation-protectable restriction elements are methylated with the DNA methylase that recognises the DNA methylase recognition sequence; and wherein the first nucleic acid further comprises opposing non-protectable type IIS restriction enzyme recognition sequences provided on the opposing side of the cut site of each of the first and second methylation-protectable restriction elements to provide i) a maintaining composite element and ii) a truncating composite element,
wherein the maintaining composite element is an arrangement wherein the opposing type IIS restriction enzyme recognition sequence is arranged to direct the type IIS restriction enzyme to cut the nucleic acid at the same site as the type IIS restriction enzyme recognition sequence of the opposing methylation-protectable restriction element, such that the same overhang is produced, and
wherein the tuncating composite element is an arrangement wherein the opposing type IIS restriction enzyme recognition sequence of the truncating composite element is arranged to direct the type IIS restriction enzyme to cut the nucleic acid at a site that is within the recognition sequence of the type IIS restriction enzyme recognition sequence of the methylation-protectable restriction element, or within the nucleotides between the cut site and the recognition sequence of the type IIS restriction enzyme recognition sequence of the methylation-protectable restriction element;
wherein the first nucleic acid is restriction enzyme cut by a type IIS restriction enzyme that recognizes the opposing type IIS restriction enzyme recognition sequences thereby forming the first linearised methylated nucleic acid, and
wherein the maintaining and truncating composite elements are methylated with the DNA methylase; providing a first DNA fragment for assembly having overhang ends that are adapted to ligate to the overhang ends of the first linearised methylated nucleic acid;
ligating the first DNA fragment for assembly and first linearised methylated nucleic acid with a ligase to form a first methylated intermediate vector;
transforming the first methylated intermediate vector into a bacterial strain that does not express the DNA methylase(s) that recognises either of the DNA methylase recognition sequence(s) of the maintaining and truncating composite elements, thereby forming a first intermediate vector that is not methylated;
isolating the first intermediate vector;
(B) providing a second linearised methylated nucleic acid by restriction enzyme cutting of a second nucleic acid,
wherein the second nucleic acid comprises first and second methylation-protectable restriction elements with a discard sequence therebetween, or providing a previously restriction enzyme cut version thereof that has the discard sequence excised,
wherein the first and second methylation-protectable restriction elements of the second nucleic acid each comprise:
- a type IIS restriction enzyme recognition sequence; and
- a DNA methylase recognition sequence,
wherein the type IIS restriction enzyme recognition sequence and the DNA methylase recognition sequence overlap such that the base methylatable by the DNA methylase lies within the type IIS restriction enzyme recognition sequence,
wherein the DNA methylase recognition sequence is not identical to or enclosed by the type IIS restriction enzyme recognition sequence, and
wherein the DNA methylase recognition sequence does not overlap with the sequence that would form the overhang end sequence generated by the type IIS restriction enzyme, and
wherein the first and second methylation-protectable restriction elements are methylated with the DNA methylase that recognises the DNA methylase recognition sequence; and
wherein the second nucleic acid further comprises opposing non-protectable type IIS restriction enzyme recognition sequences provided on the opposing side of the cut site of each of the first and second methylation-protectable restriction elements to provide i) a maintaining composite element and ii) a truncating composite element,
wherein the maintaining composite element is an arrangement wherein the opposing type IIS restriction enzyme recognition sequence is arranged to direct the type IIS restriction enzyme to cut the nucleic acid at the same site as the type IIS restriction enzyme recognition sequence of the opposing methylation-protectable restriction element, such that the same overhang is produced, and
wherein the tuncating composite element is an arrangement wherein the opposing type IIS restriction enzyme recognition sequence of the truncating composite element is arranged to direct the type IIS restriction enzyme to cut the nucleic acid at a site that is within the recognition sequence of the type IIS restriction enzyme recognition sequence of the methylation-protectable restriction element, and/or within the nucleotides between the cut site and the recognition sequence of the type IIS restriction enzyme recognition sequence of the methylation-protectable restriction element;
wherein the second nucleic acid is restriction enzyme cut by a type IIS restriction enzyme that recognizes the opposing type IIS restriction enzyme recognition sequences thereby forming the second linearised methylated nucleic acid; and wherein the maintaining and truncating composite elements are methylated with the DNA methylase, and wherein the overhang provided by the maintaining composite element of the first linearised methylated nucleic acid is different in sequence to the overhang provided by the methylation-protectable restriction element of the second linearised methylated nucleic acid;
providing a second DNA fragment for assembly having overhang ends that are adapted to ligate to the overhang ends of the second linearised methylated nucleic acid;
ligating the second DNA fragment for assembly and second linearised methylated nucleic acid with a ligase to form a second methylated intermediate vector;
transforming the second methylated intermediate vector into a bacterial strain that does not express the DNA methylase(s) that recognises either of the DNA methylase recognition sequence(s) of the maintaining and truncating composite elements, thereby forming a second intermediate vector that is not methylated;
isolating the second intermediate vector;
(C) cutting the first intermediate vector with a type IIS restriction enzyme that recognises the type IIS restriction enzyme recognition sequence of the maintaining composite element and a type IIS restriction enzyme that recognises the type IIS restriction enzyme recognition sequence of the truncating composite element, thereby forming a first adapted DNA fragment insert that comprises a maintained-overhang sequence that is determined by the maintaining composite element and an opposing native-overhang sequence that is determined by the native sequence of the first DNA fragment for assembly;
(D) cutting the second intermediate vector with a type IIS restriction enzyme that recognises the type IIS restriction enzyme recognition sequence of the maintaining composite element and a type IIS restriction enzyme that recognises the type IIS restriction enzyme recognition sequence of the truncating composite element, thereby forming a second adapted DNA fragment insert that comprises a maintained-overhang sequence that is determined by the maintaining composite element and an opposing native-overhang sequence that is determined by the native sequence of the second DNA fragment for assembly;
wherein
(i) the first and second adapted DNA fragments are end fragments wherein their native-overhang sequences are complementary, such that they are arranged to ligate together; or
(ii) one or more middle DNA fragments for assembly are provided wherein the first and second adapted DNA fragments are respective end fragments in the assembly, and the one or more middle DNA fragments are arranged to be ligated between the first and second adapted DNA fragments via complementary native-overhang sequences;
further comprising the step of ligating together, with a ligase, the first and second adapted DNA fragments, or the first and second adapted DNA fragments and one or more middle DNA fragments, to form an assembled DNA fragment having maintained-overhangs at each end.

8. The method according to claim 7, wherein the middle DNA fragment comprises a first native-overhang sequence that is complementary to the native-overhang of the first adapted DNA fragment and a second native-overhang sequence that is complementary to the native-overhang of the second adapted DNA fragment.

9. The method according to claim 7, wherein the method comprises two or more middle DNA fragments for assembly, the middle DNA fragments comprise native-overhang sequences that are complementary to the native-overhang of a neighbouring middle DNA fragment, such that they can be ligated together in a pre-determined order; and wherein the first and last middle DNA fragments in the sequence are arranged to ligate to the respective first adapted DNA fragment and second adapted DNA fragment via complementary native-overhang sequences.

10. The method according to any of claims 7-9, wherein a middle DNA fragment for assembly is provided by
providing a further linearised methylated nucleic acid by restriction enzyme cutting of a nucleic acid, wherein the nucleic acid comprises first and second methylation-protectable restriction elements with a discard sequence therebetween, or providing a previously restriction enzyme cut version thereof that has the discard sequence excised,
wherein the first and second methylation-protectable restriction elements of the nucleci acid each comprise:
- a type IIS restriction enzyme recognition sequence; and
- a DNA methylase recognition sequence,
wherein the type IIS restriction enzyme recognition sequence and the DNA methylase recognition sequence overlap such that the base methylatable by the DNA methylase lies within the type IIS restriction enzyme recognition sequence,
wherein the DNA methylase recognition sequence is not identical to or enclosed by the type IIS restriction enzyme recognition sequence, and
wherein the DNA methylase recognition sequence does not overlap with the sequence that would form the overhang end sequence generated by the type IIS restriction enzyme, and
wherein the first and second methylation-protectable restriction elements are methylated with the DNA methylase that recognises the DNA methylase recognition sequence; and
wherein the nucleic acid further comprises opposing non-protectable type IIS restriction enzyme recognition sequences provided on the opposing side of the cut site of each of the first and second methylation-protectable restriction elements to provide two truncating composite elements,
wherein the tuncating composite element is an arrangement wherein the opposing type IIS restriction enzyme recognition sequence of the truncating composite element is arranged to direct the type IIS restriction enzyme to cut the nucleic acid at a site that is within the recognition sequence of the type IIS restriction enzyme recognition sequence of the methylation-protectable restriction element, and/or within the nucleotides between the cut site and the recognition sequence of the type IIS restriction enzyme recognition sequence of the methylation-protectable restriction element;
wherein the nucleic acid is restriction enzyme cut by a type IIS restriction enzyme that recognizes the opposing type IIS restriction enzyme recognition sequences thereby forming the further linearised methylated nucleic acid;
providing an adapted middle DNA fragment for assembly having overhang ends that are adapted to ligate to the overhang ends of the further linearised methylated nucleic acid;
ligating the adapted middle DNA fragment for assembly and further linearised methylated nucleic acid with a ligase to form a methylated intermediate vector;
transforming the methylated intermediate vector into a bacterial strain that does not express the DNA methylase(s) that recognises either of the DNA methylase recognition sequence(s) of the methylation-protectable restriction elements, thereby forming an intermediate vector that is not methylated;
isolating the intermediate vector;
cutting the intermediate vector with restriction enzymes that recognise the type IIS restriction enzyme recognition sequence of the methylation-protectable restriction elements, thereby forming a middle DNA fragment insert that comprises native-overhang sequences at each end that are determined by the native sequence of the middle DNA fragment for assembly.

11. The method according to any of claims 7-10, further comprise the step of providing a linearised destination vector for insertion of the assembled DNA fragments, optionally wherein the linearised destination vector is provided by cutting a circular destination vector with the same type IIS restriction enzyme(s) that recognise the type IIS restriction enzyme recognition sequences of the maintaining and/or truncating composite element.

12. A composition comprising the nucleic acid according to any of claims 1-6.

13. A kit comprising one or more nucleic acids according to any of claims 1-6, and a restriction enzyme and/or a ligase.

14. A host cell comprising the nucleic acid according to any of claims 1-6.

15. Use of the nucleic acid according to any of claims 1-6 for assembling DNA fragments of interest, optionally wherein the use is *in vitro.*

## Patentansprüche

1. Nukleinsäure zur Verwendung bei der DNA-Anordnung, wobei die Nukleinsäure mindestens ein durch Methylierung schützbares Restriktionselement umfasst, wobei das durch Methylierung schützbare Restriktionselement Folgendes umfasst:
- eine Typ-IIS-Restriktionsenzym-Erkennungssequenz; und
- eine DNA-Methylase-Erkennungssequenz,
wobei die Typ-IIS-Restriktionsenzym-Erkennungssequenz und die DNA-Methylase-Erkennungssequenz überlappen, sodass die durch die DNA-Methylase methylierbare Base innerhalb der Typ-IIS-Restriktionsenzym-Erkennungssequenz liegt,
wobei die DNA-Methylase-Erkennungssequenz nicht identisch mit der Typ-IIS-Restriktionsenzym-Erkennungssequenz ist oder durch diese eingeschlossen ist und
wobei die DNA-Methylase-Erkennungssequenz nicht mit der Sequenz überlappt, die die durch das Typ-IIS-Restriktionsenzym erzeugte Überhang-Endsequenz bilden würde, wobei die Nukleinsäure ferner eine gegenüberliegende nicht schützbare Typ-IIS-Restriktionsenzym-Erkennungssequenz umfasst, die auf der gegenüberliegenden Seite der Schnittstelle des durch Methylierung schützbaren Restriktionselements bereitgestellt ist, wodurch ein trunkierendes zusammengesetztes Element gebildet wird, wobei die gegenüberliegende Typ-IIS-Restriktionsenzym-Erkennungssequenz des trunkierenden zusammengesetzten Elements ausgestaltet ist, um das Typ-IIS-Restriktionsenzym anzuweisen, die Nukleinsäure an einer Stelle zu schneiden, die sich innerhalb der Erkennungssequenz der Typ-IIS-Restriktionsenzym-Erkennungssequenz des durch Methylierung schützbaren Restriktionselements und/oder innerhalb der Nukleotide zwischen der Schnittstelle und der Erkennungssequenz der Typ-IIS-Restriktionsenzym-Erkennungssequenz des durch Methylierung schützbaren Restriktionselements befindet.

2. Nukleinsäure nach Anspruch 1, wobei die Nukleinsäure ein erstes und ein zweites durch Methylierung schützbares Restriktionselement umfasst
und wobei die Nukleinsäure ferner eine gegenüberliegende Typ-IIS-Restriktionsenzym-Erkennungssequenz umfasst, die auf der gegenüberliegenden Seite der Schnittstelle von jedem von dem ersten und dem zweiten durch Methylierung schützbaren Restriktionselement bereitgestellt ist, um Folgendes zu bilden:
A i) ein erhaltendes zusammengesetztes Element oder ein insertionales zusammengesetztes Element und ii) ein trunkierendes zusammengesetztes Element; oder
B) zwei trunkierende zusammengesetzte Elemente,
wobei das erhaltende zusammengesetzte Element eine Ausgestaltung ist, bei der die gegenüberliegende Typ-IIS-Restriktionsenzym-Erkennungssequenz so ausgestaltet ist, dass sie das Typ-IIS-Restriktionsenzym anweist, die Nukleinsäure an der gleichen Stelle wie die Typ-IIS-Restriktionsenzym-Erkennungssequenz des gegenüberliegenden durch Methylierung schützbaren Restriktionselements zu schneiden, sodass der gleiche Überhang erzeugt wird,
wobei das insertionale zusammengesetzte Element eine Ausgestaltung ist, die eine funktionelle Sequenzinsertion umfasst, die zwischen dem durch Methylierung schützbaren Restriktionselement und der gegenüberliegenden Typ-IIS-Restriktionsenzym-Erkennungssequenz bereitgestellt ist, und
wobei das trunkierende zusammengesetzte Element eine Ausgestaltung ist, bei der die gegenüberliegende Typ-IIS-Restriktionsenzym-Erkennungssequenz des trunkierenden zusammengesetzten Elements ausgestaltet ist, um das Typ-IIS-Restriktionsenzym anzuweisen, die Nukleinsäure an einer Stelle zu schneiden, die sich innerhalb der Erkennungssequenz der Typ-IIS-Restriktionsenzym-Erkennungssequenz des durch Methylierung schützbaren Restriktionselements und/oder innerhalb der Nukleotide zwischen der Schnittstelle und der Erkennungssequenz der Typ-IIS-Restriktionsenzym-Erkennungssequenz des durch Methylierung schützbaren Restriktionselements befindet.

3. Nukleinsäure nach Anspruch 2, wobei die Nukleinsäure eine Nukleinsäuresequenz zwischen den Schnittstellen der beiden durch Methylierung schützbaren Restriktionselemente umfasst, die eine Verwerf-Sequenz ist; oder
wobei die Nukleinsäure ein linearisierter Vektor ist, der an jedem Ende ein durch Methylierung schützbares Restriktionselement aufweist.

4. Nukleinsäure nach Anspruch 3, wobei die Verwerf-Sequenz einen selektierbaren Marker, ein Reportergen oder eine Markierung umfasst.

5. Nukleinsäure nach einem der vorhergehenden Ansprüche, wobei die Restriktionsenzym-Erkennungssequenz des durch Methylierung schützbaren Restriktionselements durch dieselbe Restriktionsenzymspezies wie die gegenüberliegende nicht schützbare Restriktionsenzym-Erkennungssequenz erkannt wird; und/oder
wobei die DNA-Methylase-Erkennungssequenzen jedes durch Methylierung schützbaren Restriktionselements durch dieselbe Methylase erkannt werden.

6. Nukleinsäure nach einem der vorhergehenden Ansprüche, wobei das durch Methylierung schützbare Restriktionselement die Sequenz GACNNGGTCTCNNNNN (BsaI/M.Osp807II - SEQ ID NO: 1) oder GAAGACGCNNNNNN (BpiI/M2.NmeMC58II - SEQ ID NO: 2) oder GAAGCTCTTCNNNN (LguI/M.XmnI - SEQ ID NO: 3) umfasst oder daraus besteht.

7. Verfahren zur narbenlosen DNA-Anordnung von DNA-Fragmenten, die folgenden Schritte umfassend:
(A) Bereitstellen einer ersten linearisierten methylierten Nukleinsäure durch Restriktionsenzymschneiden einer ersten Nukleinsäure, wobei die erste Nukleinsäure ein erstes und ein zweites durch Methylierung schützbares Restriktionselement mit einer Verwerf-Sequenz dazwischen umfasst, oder Bereitstellen einer zuvor Restriktionsenzym-geschnittenen Version davon, bei der die Verwerf-Sequenz herausgeschnitten ist,
wobei das erste und das zweite durch Methylierung schützbare Restriktionselement der ersten Nukleinsäure jeweils Folgendes umfassen:
- eine Typ-IIS-Restriktionsenzym-Erkennungssequenz; und
- eine DNA-Methylase-Erkennungssequenz,
wobei die Typ-IIS-Restriktionsenzym-Erkennungssequenz und die DNA-Methylase-Erkennungssequenz überlappen, sodass die durch die DNA-Methylase methylierbare Base innerhalb der Typ-IIS-Restriktionsenzym-Erkennungssequenz liegt,
wobei die DNA-Methylase-Erkennungssequenz nicht identisch mit der Typ-IIS-Restriktionsenzym-Erkennungssequenz ist oder durch diese eingeschlossen ist und
wobei die DNA-Methylase-Erkennungssequenz nicht mit der Sequenz überlappt, die die durch das Typ-IIS-Restriktionsenzym erzeugte Überhang-Endsequenz bilden würde, und
wobei das erste und das zweite durch Methylierung schützbare Restriktionselement mit der DNA-Methylase methyliert sind, die die DNA-Methylase-Erkennungssequenz erkennt; und
wobei die erste Nukleinsäure ferner gegenüberliegende nicht schützbare Typ-IIS-Restriktionsenzym-Erkennungssequenzen umfasst, die auf der gegenüberliegenden Seite der Schnittstelle von jedem von dem ersten und dem zweiten durch Methylierung schützbaren Restriktionselement bereitgestellt sind, um i) ein erhaltendes zusammengesetztes Element und ii) ein trunkierendes zusammengesetztes Element bereitzustellen,
wobei das erhaltende zusammengesetzte Element eine Ausgestaltung ist, bei der die gegenüberliegende Typ-IIS-Restriktionsenzym-Erkennungssequenz so ausgestaltet ist, dass sie das Typ-IIS-Restriktionsenzym anweist, die Nukleinsäure an der gleichen Stelle wie die Typ-IIS-Restriktionsenzym-Erkennungssequenz des gegenüberliegenden durch Methylierung schützbaren Restriktionselements zu schneiden, sodass der gleiche Überhang erzeugt wird, und wobei das trunkierende zusammengesetzte Element eine Ausgestaltung ist, bei der die gegenüberliegende Typ-IIS-Restriktionsenzym-Erkennungssequenz des trunkierenden zusammengesetzten Elements ausgestaltet ist, um das Typ-IIS-Restriktionsenzym anzuweisen, die Nukleinsäure an einer Stelle zu schneiden, die sich innerhalb der Erkennungssequenz der Typ-IIS-Restriktionsenzym-Erkennungssequenz des durch Methylierung schützbaren Restriktionselements oder innerhalb der Nukleotide zwischen der Schnittstelle und der Erkennungssequenz der Typ-IIS-Restriktionsenzym-Erkennungssequenz des durch Methylierung schützbaren Restriktionselements befindet;
wobei die erste Nukleinsäure ein Restriktionsenzym ist, das durch ein Typ-IIS-Restriktionsenzym geschnitten wird, das die gegenüberliegenden Typ-IIS-Restriktionsenzym-Erkennungssequenzen erkennt, wodurch die erste linearisierte methylierte Nukleinsäure gebildet wird, und
wobei das erhaltende und das trunkierende zusammengesetzte Element mit der DNA-Methylase methyliert sind;
Bereitstellen eines ersten DNA-Fragments zur Anordnung mit Überhang-Enden, die angepasst sind, um an die Überhang-Enden der ersten linearisierten methylierten Nukleinsäure zu ligieren;
Ligieren des ersten DNA-Fragments zur Anordnung und der ersten linearisierten methylierten Nukleinsäure mit einer Ligase, um einen ersten methylierten Zwischenvektor zu bilden;
Transformieren des ersten methylierten Zwischenvektors in einen Bakterienstamm, der die DNA-Methylase(n) nicht exprimiert, die eine der DNA-Methylase-Erkennungssequenz(en) des erhaltenden und des trunkierenden zusammengesetzten Elements erkennt, wodurch ein erster Zwischenvektor gebildet wird, der nicht methyliert ist;
Isolieren des ersten Zwischenvektors;
(B) Bereitstellen einer zweiten linearisierten methylierten Nukleinsäure durch Restriktionsenzymschneiden einer zweiten Nukleinsäure,
wobei die zweite Nukleinsäure ein erstes und ein zweites durch Methylierung schützbares Restriktionselement mit einer Verwerf-Sequenz dazwischen umfasst, oder Bereitstellen einer zuvor Restriktionsenzym-geschnittenen Version davon, bei der die Verwerf-Sequenz herausgeschnitten ist,
wobei das erste und das zweite durch Methylierung schützbare Restriktionselement der zweiten Nukleinsäure jeweils Folgendes umfassen:
- eine Typ-IIS-Restriktionsenzym-Erkennungssequenz; und
- eine DNA-Methylase-Erkennungssequenz,
wobei die Typ-IIS-Restriktionsenzym-Erkennungssequenz und die DNA-Methylase-Erkennungssequenz überlappen, sodass die durch die DNA-Methylase methylierbare Base innerhalb der Typ-IIS-Restriktionsenzym-Erkennungssequenz liegt,
wobei die DNA-Methylase-Erkennungssequenz nicht identisch mit der Typ-IIS-Restriktionsenzym-Erkennungssequenz ist oder durch diese eingeschlossen ist und
wobei die DNA-Methylase-Erkennungssequenz nicht mit der Sequenz überlappt, die die durch das Typ-IIS-Restriktionsenzym erzeugte Überhang-Endsequenz bilden würde, und
wobei das erste und das zweite durch Methylierung schützbare Restriktionselement mit der DNA-Methylase methyliert sind, die die DNA-Methylase-Erkennungssequenz erkennt; und
wobei die zweite Nukleinsäure ferner gegenüberliegende nicht schützbare Typ-IIS-Restriktionsenzym-Erkennungssequenzen umfasst, die auf der gegenüberliegenden Seite der Schnittstelle von jedem von dem ersten und dem zweiten durch Methylierung schützbaren Restriktionselement bereitgestellt sind, um i) ein erhaltendes zusammengesetztes Element und ii) ein trunkierendes zusammengesetztes Element bereitzustellen,
wobei das erhaltende zusammengesetzte Element eine Ausgestaltung ist, bei der die gegenüberliegende Typ-IIS-Restriktionsenzym-Erkennungssequenz so ausgestaltet ist, dass sie das Typ-IIS-Restriktionsenzym anweist, die Nukleinsäure an der gleichen Stelle wie die Typ-IIS-Restriktionsenzym-Erkennungssequenz des gegenüberliegenden durch Methylierung schützbaren Restriktionselements zu schneiden, sodass der gleiche Überhang erzeugt wird, und wobei das trunkierende zusammengesetzte Element eine Ausgestaltung ist, bei der die gegenüberliegende Typ-IIS-Restriktionsenzym-Erkennungssequenz des trunkierenden zusammengesetzten Elements ausgestaltet ist, um das Typ-IIS-Restriktionsenzym anzuweisen, die Nukleinsäure an einer Stelle zu schneiden, die sich innerhalb der Erkennungssequenz der Typ-IIS-Restriktionsenzym-Erkennungssequenz des durch Methylierung schützbaren Restriktionselements und/oder innerhalb der Nukleotide zwischen der Schnittstelle und der Erkennungssequenz der Typ-IIS-Restriktionsenzym-Erkennungssequenz des durch Methylierung schützbaren Restriktionselements befindet;
wobei die zweite Nukleinsäure ein Restriktionsenzym ist, das durch ein Typ-IIS-Restriktionsenzym geschnitten wird, das die gegenüberliegenden Typ-IIS-Restriktionsenzym-Erkennungssequenzen erkennt, wodurch die zweite linearisierte methylierte Nukleinsäure gebildet wird; und wobei das erhaltende und das trunkierende zusammengesetzte Element mit der DNA-Methylase methyliert sind und wobei der Überhang, der durch das erhaltende zusammengesetzte Element der ersten linearisierten methylierten Nukleinsäure bereitgestellt wird, sich in der Sequenz von dem Überhang unterscheidet, der durch das durch Methylierung schützbare Restriktionselement der zweiten linearisierten methylierten Nukleinsäure bereitgestellt wird;
Bereitstellen eines zweiten DNA-Fragments zur Anordnung mit Überhang-Enden, die angepasst sind, um an die Überhang-Enden der zweiten linearisierten methylierten Nukleinsäure zu ligieren;
Ligieren des zweiten DNA-Fragments zur Anordnung und der zweiten linearisierten methylierten Nukleinsäure mit einer Ligase, um einen zweiten methylierten Zwischenvektor zu bilden;
Transformieren des zweiten methylierten Zwischenvektors in einen Bakterienstamm, der die DNA-Methylase(n) nicht exprimiert, die eine der DNA-Methylase-Erkennungssequenz(en) des erhaltenden und des trunkierenden zusammengesetzten Elements erkennt, wodurch ein zweiter Zwischenvektor gebildet wird, der nicht methyliert ist;
Isolieren des zweiten Zwischenvektors;
(C) Schneiden des ersten Zwischenvektors mit einem Typ-IIS-Restriktionsenzym, das die Typ-IIS-Restriktionsenzym-Erkennungssequenz des erhaltenden zusammengesetzten Elements erkennt, und einem Typ-IIS-Restriktionsenzym, das die Typ-IIS-Restriktionsenzym-Erkennungssequenz des trunkierenden zusammengesetzten Elements erkennt, wodurch eine erste angepasste DNA-Fragment-Insertion gebildet wird, die eine erhaltene Überhangsequenz, die durch das erhaltende zusammengesetzte Element bestimmt ist, und eine gegenüberliegende native Überhangsequenz, die durch die native Sequenz des ersten DNA-Fragments zur Anordnung bestimmt ist, umfasst;
(D) Schneiden des zweiten Zwischenvektors mit einem Typ-IIS-Restriktionsenzym, das die Typ-IIS-Restriktionsenzym-Erkennungssequenz des erhaltenden zusammengesetzten Elements erkennt, und einem Typ-IIS-Restriktionsenzym, das die Typ-IIS-Restriktionsenzym-Erkennungssequenz des trunkierenden zusammengesetzten Elements erkennt, wodurch eine zweite angepasste DNA-Fragment-Insertion gebildet wird, die eine erhaltene Überhangsequenz, die durch das erhaltende zusammengesetzte Element bestimmt ist, und eine gegenüberliegende native Überhangsequenz, die durch die native Sequenz des zweiten DNA-Fragments zur Anordnung bestimmt ist, umfasst;
wobei
(i) das erste und das zweite angepasste DNA-Fragment Endfragmente sind, wobei ihre nativen Überhangsequenzen komplementär sind, sodass sie derart ausgestaltet sind, dass sie miteinander ligieren; oder
(ii) ein oder mehrere mittlere DNA-Fragmente zur Anordnung bereitgestellt werden, wobei das erste und das zweite angepasste DNA-Fragment jeweilige Endfragmente in der Anordnung sind und das eine oder die mehreren mittleren DNA-Fragmente derart ausgestaltet sind, dass sie zwischen dem ersten und dem zweiten angepassten DNA-Fragment über komplementäre native Überhangsequenzen ligiert werden;
ferner umfassend den Schritt des Ligierens des ersten und zweiten angepassten DNA-Fragments oder des ersten und zweiten angepassten DNA-Fragments und eines oder mehrerer mittlerer DNA-Fragmente miteinander mit einer Ligase, um ein zusammengesetztes DNA-Fragment zu bilden, das an jedem Ende erhaltene Überhänge aufweist.

8. Verfahren nach Anspruch 7, wobei das mittlere DNA-Fragment eine erste native Überhangsequenz, die komplementär zu dem nativen Überhang des ersten angepassten DNA-Fragments ist, und eine zweite native Überhangsequenz, die komplementär zu dem nativen Überhang des zweiten angepassten DNA-Fragments ist, umfasst.

9. Verfahren nach Anspruch 7, wobei das Verfahren zwei oder mehr mittlere DNA-Fragmente zur Anordnung umfasst, wobei die mittleren DNA-Fragmente native Überhangsequenzen umfassen, die komplementär zu dem nativen Überhang eines benachbarten mittleren DNA-Fragments sind, sodass sie in einer vorbestimmten Reihenfolge miteinander ligiert werden können; und wobei das erste und das letzte mittlere DNA-Fragment in der Sequenz ausgestaltet sind, um über komplementäre native Überhangsequenzen mit dem jeweiligen ersten angepassten DNA-Fragment und dem zweiten angepassten DNA-Fragment zu ligieren.

10. Verfahren nach einem der Ansprüche 7-9, wobei ein mittleres DNA-Fragment zur Anordnung bereitgestellt wird durch
Bereitstellen einer weiteren linearisierten methylierten Nukleinsäure durch Restriktionsenzymschneiden einer Nukleinsäure, wobei die Nukleinsäure ein erstes und ein zweites durch Methylierung schützbares Restriktionselement mit einer Verwerf-Sequenz dazwischen umfasst, oder Bereitstellen einer zuvor Restriktionsenzym-geschnittenen Version davon, bei der die Verwerf-Sequenz herausgeschnitten ist,
wobei das erste und das zweite durch Methylierung schützbare Restriktionselement der Nukleinsäure jeweils Folgendes umfassen:
- eine Typ-IIS-Restriktionsenzym-Erkennungssequenz; und
- eine DNA-Methylase-Erkennungssequenz,
wobei die Typ-IIS-Restriktionsenzym-Erkennungssequenz und die DNA-Methylase-Erkennungssequenz überlappen, sodass die durch die DNA-Methylase methylierbare Base innerhalb der Typ-IIS-Restriktionsenzym-Erkennungssequenz liegt,
wobei die DNA-Methylase-Erkennungssequenz nicht identisch mit der Typ-IIS-Restriktionsenzym-Erkennungssequenz ist oder durch diese eingeschlossen ist und
wobei die DNA-Methylase-Erkennungssequenz nicht mit der Sequenz überlappt, die die durch das Typ-IIS-Restriktionsenzym erzeugte Überhang-Endsequenz bilden würde, und
wobei das erste und das zweite durch Methylierung schützbare Restriktionselement mit der DNA-Methylase methyliert sind, die die DNA-Methylase-Erkennungssequenz erkennt; und
wobei die Nukleinsäure ferner gegenüberliegende nicht schützbare Typ-IIS-Restriktionsenzym-Erkennungssequenzen umfasst, die auf der gegenüberliegenden Seite der Schnittstelle von jedem von dem ersten und dem zweiten durch Methylierung schützbaren Restriktionselement bereitgestellt sind, um zwei trunkierende zusammengesetzte Elemente bereitzustellen,
wobei das trunkierende zusammengesetzte Element eine Ausgestaltung ist, bei der die gegenüberliegende Typ-IIS-Restriktionsenzym-Erkennungssequenz des trunkierenden zusammengesetzten Elements ausgestaltet ist, um das Typ-IIS-Restriktionsenzym anzuweisen, die Nukleinsäure an einer Stelle zu schneiden, die sich innerhalb der Erkennungssequenz der Typ-IIS-Restriktionsenzym-Erkennungssequenz des durch Methylierung schützbaren Restriktionselements und/oder innerhalb der Nukleotide zwischen der Schnittstelle und der Erkennungssequenz der Typ-IIS-Restriktionsenzym-Erkennungssequenz des durch Methylierung schützbaren Restriktionselements befindet; wobei die Nukleinsäure ein Restriktionsenzym ist, das durch ein Typ-IIS-Restriktionsenzym geschnitten wird, das die gegenüberliegenden Typ-IIS-Restriktionsenzym-Erkennungssequenzen erkennt, wodurch die weitere linearisierte methylierte Nukleinsäure gebildet wird; Bereitstellen eines angepassten mittleren DNA-Fragments zur Anordnung mit Überhang-Enden, die angepasst sind, um an die Überhang-Enden der weiteren linearisierten methylierten Nukleinsäure zu ligieren;
Ligieren des angepassten mittleren DNA-Fragments zur Anordnung und der weiteren linearisierten methylierten Nukleinsäure mit einer Ligase, um einen methylierten Zwischenvektor zu bilden;
Transformieren des methylierten Zwischenvektors in einen Bakterienstamm, der die DNA-Methylase(n) nicht exprimiert, die eine der DNA-Methylase-Erkennungssequenz(en) der durch Methylierung schützbaren Restriktionselemente erkennt, wodurch ein Zwischenvektor gebildet wird, der nicht methyliert ist;
Isolieren des Zwischenvektors;
Schneiden des Zwischenvektors mit Restriktionsenzymen, die die Typ-IIS-Restriktionsenzym-Erkennungssequenz der durch Methylierung schützbaren Restriktionselemente erkennen, wodurch eine mittlere DNA-Fragment-Insertion gebildet wird, die an jedem Ende native Überhangsequenzen umfasst, die durch die native Sequenz des mittleren DNA-Fragments für die Anordnung bestimmt sind.

11. Verfahren nach einem der Ansprüche 7-10, ferner den Schritt des Bereitstellens eines linearisierten Zielvektors zur Insertion der angeordneten DNA-Fragmente umfassend, optional wobei der linearisierte Zielvektor durch Schneiden eines zirkulären Zielvektors mit dem/den gleichen Typ-IIS-Restriktionsenzym(en) bereitgestellt wird, die die Typ-IIS-Restriktionsenzym-Erkennungssequenzen des erhaltenden und/oder des trunkierenden zusammengesetzten Elements erkennen.

12. Zusammensetzung, umfassend die Nukleinsäure nach einem der Ansprüche 1-6.

13. Kit, umfassend eine oder mehrere Nukleinsäuren nach einem der Ansprüche 1-6 und ein Restriktionsenzym und/oder eine Ligase.

14. Wirtszelle, umfassend die Nukleinsäure nach einem der Ansprüche 1-6.

15. Verwendung der Nukleinsäure nach einem der Ansprüche 1-6 zum Anordnen von DNA-Fragmenten von Interesse, optional wobei die Verwendung *in vitro* erfolgt.

## Revendications

1. Acide nucléique destiné à être utilisé dans l'assemblage d'ADN, dans lequel l'acide nucléique comprend au moins un élément de restriction pouvant être protégé par méthylation, l'élément de restriction pouvant être protégé par méthylation comprenant :
- une séquence de reconnaissance d'enzyme de restriction de type IIS ; et
- une séquence de reconnaissance de méthylase d'ADN, dans lequel la séquence de reconnaissance d'enzyme de restriction de type IIS et la séquence de reconnaissance de méthylase d'ADN se chevauchent de sorte que la base pouvant être méthylée par méthylase d'ADN se situe dans la séquence de reconnaissance d'enzyme de restriction de type IIS,
dans lequel la séquence de reconnaissance de méthylase d'ADN n'est pas identique à la séquence de reconnaissance d'enzyme de restriction de type IIS ou contenue dans celle-ci, et dans lequel la séquence de reconnaissance de méthylase d'ADN ne chevauche pas la séquence qui formerait la séquence terminale en surplomb générée par l'enzyme de restriction de type IIS.
dans lequel l'acide nucléique comprend en outre une séquence de reconnaissance d'enzyme de restriction de type IIS ne pouvant pas être protégée opposée prévue sur le côté opposé du site coupé de l'élément de restriction pouvant être protégé par méthylation, formant ainsi un élément composite de troncature,
dans lequel la séquence de reconnaissance d'enzyme de restriction de type IIS opposée de l'élément composite de troncature est agencée pour diriger l'enzyme de restriction de type IIS afin de couper l'acide nucléique au niveau d'un site qui se trouve dans la séquence de reconnaissance de la séquence de reconnaissance d'enzyme de restriction de type IIS de l'élément de restriction pouvant être protégé par méthylation, et/ou dans les nucléotides entre le site coupé et la séquence de reconnaissance de la séquence de reconnaissance d'enzyme de restriction de type IIS de l'élément de restriction pouvant être protégé par méthylation.

2. Acide nucléique selon la revendication 1, dans lequel l'acide nucléique comprend des premier et second éléments de restriction pouvant être protégés par méthylation,
et dans lequel l'acide nucléique comprend en outre des séquences de reconnaissance d'enzyme de restriction de type IIS opposées prévues sur le côté opposé du site coupé de chacun des premier et second éléments de restriction pouvant être protégés par méthylation pour former :
A i) un élément composite de maintien ou un élément composite d'insertion, et ii) un élément composite de troncature ; ou
B) deux éléments composites de troncature,
dans lequel l'élément composite de maintien est un agencement dans lequel la séquence de reconnaissance d'enzyme de restriction de type IIS opposée est agencée pour diriger l'enzyme de restriction de type IIS afin de couper l'acide nucléique au niveau du même site que la séquence de reconnaissance d'enzyme de restriction de type IIS de l'élément de restriction pouvant être protégé par méthylation, de sorte que le même surplomb est produit,
dans lequel l'élément composite d'insertion est un agencement comprenant un insert de séquence fonctionnelle prévu entre l'élément de restriction pouvant être protégé par méthylation et la séquence de reconnaissance d'enzyme de restriction de type IIS opposée, et
dans lequel l'élément composite de troncature est un agencement dans lequel la séquence de reconnaissance d'enzyme de restriction de type IIS opposée de l'élément composite de troncature est agencée pour diriger l'enzyme de restriction de type IIS afin de couper l'acide nucléique au niveau d'un site qui se trouve dans la séquence de reconnaissance de la séquence de reconnaissance d'enzyme de restriction de type IIS de l'élément de restriction pouvant être protégé par méthylation, et/ou dans les nucléotides entre le site coupé et la séquence de reconnaissance de la séquence de reconnaissance d'enzyme de restriction de type IIS de l'élément de restriction pouvant être protégé par méthylation.

3. Acide nucléique selon la revendication 2, dans lequel l'acide nucléique comprend une séquence d'acide nucléique entre les sites coupés des deux éléments de restriction pouvant être protégés par méthylation, qui est une séquence de rejet ; ou
dans lequel l'acide nucléique est un vecteur linéarisé présentant un élément de restriction pouvant être protégé par méthylation à chaque extrémité.

4. Acide nucléique selon la revendication 3, dans lequel la séquence de rejet comprend un marqueur sélectionnable, un gène rapporteur ou un marqueur.

5. Acide nucléique selon une quelconque revendication précédente, dans lequel la séquence de reconnaissance d'enzyme de restriction de l'élément de restriction pouvant être protégé par méthylation est reconnue par la même espèce d'enzyme de restriction que la séquence de reconnaissance d'enzyme de restriction ne pouvant pas être protégée opposée ; et/ou
dans lequel les séquences de reconnaissance de méthylase d'ADN de chaque élément de restriction pouvant être protégé par méthylation sont reconnues par la même méthylase.

6. Acide nucléique selon une quelconque revendication précédente, dans lequel l'élément de restriction pouvant être protégé par méthylation comprend ou consiste en la séquence GACNNGGTCTCNNNNN (BsaI/M.Osp807II - SEQ ID NO : 1) ou GAAGACGCNNNNNN (BpiI/M2.NmeMC58II - SEQ ID NO : 2) ou GAAGCTCTTCNNNN (LguI/M.XmnI - SEQ ID NO : 3).

7. Procédé d'assemblage d'ADN sans cicatrice de fragments d'ADN comprenant les étapes de :
(A) fourniture d'un premier acide nucléique méthylé linéarisé par coupure par une enzyme de restriction d'un premier acide nucléique, dans lequel le premier acide nucléique comprend des premier et second éléments de restriction pouvant être protégés par méthylation avec une séquence de rejet entre eux, ou fourniture d'une version précédemment coupée par une enzyme de restriction de celui-ci où la séquence de rejet est excisée,
dans lequel les premier et second éléments de restriction pouvant être protégés par méthylation du premier acide nucléique comprennent chacun :
- une séquence de reconnaissance d'enzyme de restriction de type IIS ; et
- une séquence de reconnaissance de méthylase d'ADN, dans lequel la séquence de reconnaissance d'enzyme de restriction de type IIS et la séquence de reconnaissance de méthylase d'ADN se chevauchent de sorte que la base pouvant être méthylée par méthylase d'ADN se situe dans la séquence de reconnaissance d'enzyme de restriction de type IIS,
dans lequel la séquence de reconnaissance de méthylase d'ADN n'est pas identique à la séquence de reconnaissance d'enzyme de restriction de type IIS ou contenue dans celle-ci, et dans lequel la séquence de reconnaissance de méthylase d'ADN ne chevauche pas la séquence qui formerait la séquence terminale en surplomb générée par l'enzyme de restriction de type IIS, et
dans lequel les premier et second éléments de restriction pouvant être protégés par méthylation sont méthylés avec la méthylase d'ADN qui reconnaît la séquence de reconnaissance de méthylase d'ADN ; et
dans lequel le premier acide nucléique comprend en outre des séquences de reconnaissance d'enzyme de restriction de type IIS ne pouvant pas être protégées opposées prévues sur le côté opposé du site coupé de chacun des premier et second éléments de restriction pouvant être protégés par méthylation pour fournir i) un élément composite de maintien et ii) un élément composite de troncature,
dans lequel l'élément composite de maintien est un agencement dans lequel la séquence de reconnaissance d'enzyme de restriction de type IIS opposée est agencée pour diriger l'enzyme de restriction de type IIS afin de couper l'acide nucléique au niveau du même site que la séquence de reconnaissance d'enzyme de restriction de type IIS de l'élément de restriction pouvant être protégé par méthylation, de sorte que le même surplomb est produit, et dans lequel l'élément composite de troncature est un agencement dans lequel la séquence de reconnaissance d'enzyme de restriction de type IIS opposée de l'élément composite de troncature est agencée pour diriger l'enzyme de restriction de type IIS afin de couper l'acide nucléique au niveau d'un site qui se trouve dans la séquence de reconnaissance de la séquence de reconnaissance d'enzyme de restriction de type IIS de l'élément de restriction pouvant être protégé par méthylation, ou dans les nucléotides entre le site coupé et la séquence de reconnaissance de la séquence de reconnaissance d'enzyme de restriction de type IIS de l'élément de restriction pouvant être protégé par méthylation ;
dans lequel le premier acide nucléique est coupé par une enzyme de restriction au moyen d'une enzyme de restriction de type IIS qui reconnaît les séquences de reconnaissance d'enzyme de restriction de type IIS opposées formant ainsi le premier acide nucléique méthylé linéarisé, et
dans lequel les éléments composites de maintien et de troncature sont méthylés avec la méthylase d'ADN ; fourniture d'un premier fragment d'ADN pour assemblage présentant des extrémités en surplomb qui sont adaptées pour se lier aux extrémités en surplomb du premier acide nucléique méthylé linéarisé ;
ligature du premier fragment d'ADN pour assemblage et le premier acide nucléique méthylé linéarisé avec une ligase pour former un premier vecteur intermédiaire méthylé ; transformation du premier vecteur intermédiaire méthylé en une souche bactérienne qui n'exprime pas la ou les méthylases d'ADN qui reconnaissent l'une ou l'autre des séquences de reconnaissance de méthylase d'ADN des éléments composites de maintien et de troncature, formant ainsi un premier vecteur intermédiaire qui n'est pas méthylé ;
isolement du premier vecteur intermédiaire ;
(B) fourniture d'un second acide nucléique méthylé linéarisé par coupure par une enzyme de restriction d'un second acide nucléique,
dans lequel le second acide nucléique comprend des premier et seconds éléments de restriction pouvant être protégés par méthylation avec une séquence de rejet entre eux, ou fourniture d'une version précédemment coupée par une enzyme de restriction de celui-ci où la séquence de rejet est excisée,
dans lequel les premier et second éléments de restriction pouvant être protégés par méthylation du second acide nucléique comprennent chacun :
- une séquence de reconnaissance d'enzyme de restriction de type IIS ; et
- une séquence de reconnaissance de méthylase d'ADN, dans lequel la séquence de reconnaissance d'enzyme de restriction de type IIS et la séquence de reconnaissance de méthylase d'ADN se chevauchent de sorte que la base pouvant être méthylée par méthylase d'ADN se situe dans la séquence de reconnaissance d'enzyme de restriction de type IIS,
dans lequel la séquence de reconnaissance de méthylase d'ADN n'est pas identique à la séquence de reconnaissance d'enzyme de restriction de type IIS ou contenue dans celle-ci, et dans lequel la séquence de reconnaissance de méthylase d'ADN ne chevauche pas la séquence qui formerait la séquence terminale en surplomb générée par l'enzyme de restriction de type IIS, et
dans lequel les premier et second éléments de restriction pouvant être protégés par méthylation sont méthylés avec la méthylase d'ADN qui reconnaît la séquence de reconnaissance de méthylase d'ADN ; et
dans lequel le second acide nucléique comprend en outre des séquences de reconnaissance d'enzyme de restriction de type IIS ne pouvant pas être protégées opposées prévues sur le côté opposé du site coupé de chacun des premier et second éléments de restriction pouvant être protégés par méthylation pour fournir i) un élément composite de maintien et ii) un élément composite de troncature,
dans lequel l'élément composite de maintien est un agencement dans lequel la séquence de reconnaissance d'enzyme de restriction de type IIS opposée est agencée pour diriger l'enzyme de restriction de type IIS afin de couper l'acide nucléique au niveau du même site que la séquence de reconnaissance d'enzyme de restriction de type IIS de l'élément de restriction pouvant être protégé par méthylation, de sorte que le même surplomb est produit, et
dans lequel l'élément composite de troncature est un agencement dans lequel la séquence de reconnaissance d'enzyme de restriction de type IIS opposée de l'élément composite de troncature est agencée pour diriger l'enzyme de restriction de type IIS afin de couper l'acide nucléique au niveau d'un site qui se trouve dans la séquence de reconnaissance de la séquence de reconnaissance d'enzyme de restriction de type IIS de l'élément de restriction pouvant être protégé par méthylation, ou dans les nucléotides entre le site coupé et la séquence de reconnaissance de la séquence de reconnaissance d'enzyme de restriction de type IIS de l'élément de restriction pouvant être protégé par méthylation ;
dans lequel le second acide nucléique est coupé par une enzyme de restriction au moyen d'une enzyme de restriction de type IIS qui reconnaît les séquences de reconnaissance d'enzyme de restriction de type IIS opposées formant ainsi le second acide nucléique méthylé linéarisé ; et dans lequel les éléments composites de maintien et de troncature sont méthylés avec la méthylase d'ADN, et dans lequel le surplomb fourni par l'élément composite de maintien du premier acide nucléique méthylé linéarisé est différent en séquence du surplomb fourni par l'élément de restriction pouvant être protégé par méthylation du second acide nucléique méthylé linéarisé ;
fourniture d'un second fragment d'ADN pour assemblage présentant des extrémités en surplomb qui sont adaptées pour se lier aux extrémités en surplomb du second acide nucléique méthylé linéarisé ;
ligature du second fragment d'ADN pour assemblage et du second acide nucléique méthylé linéarisé avec une ligase pour former un second vecteur intermédiaire méthylé ; transformation du second vecteur intermédiaire méthylé en une souche bactérienne qui n'exprime pas la ou les méthylases d'ADN qui reconnaissent l'une ou l'autre des séquences de reconnaissance de méthylase d'ADN des éléments composites de maintien et de troncature, formant ainsi un second vecteur intermédiaire qui n'est pas méthylé ;
isolement du second vecteur intermédiaire ;
(C) coupure du premier vecteur intermédiaire avec une enzyme de restriction de type IIS qui reconnaît la séquence de reconnaissance d'enzyme de restriction de type IIS de l'élément composite de maintien et une enzyme de restriction de type IIS qui reconnaît la séquence de reconnaissance d'enzyme de restriction de type IIS de l'élément composite de troncature, formant ainsi un premier insert de fragment d'ADN adapté qui comprend une séquence en surplomb maintenue qui est déterminée par l'élément composite de maintien et une séquence en surplomb native opposée qui est déterminée par la séquence native du premier fragment d'ADN pour assemblage ;
(D) coupure du second vecteur intermédiaire avec une enzyme de restriction de type IIS qui reconnaît la séquence de reconnaissance d'enzyme de restriction de type IIS de l'élément composite de maintien et une enzyme de restriction de type IIS qui reconnaît la séquence de reconnaissance d'enzyme de restriction de type IIS de l'élément composite de troncature, formant ainsi un second insert de fragment d'ADN adapté qui comprend une séquence en surplomb maintenue qui est déterminée par l'élément composite de maintien et une séquence en surplomb native opposée qui est déterminée par la séquence native du second fragment d'ADN pour assemblage ;
dans lequel
(i) les premier et second fragments d'ADN adaptés sont des fragments terminaux dans lequel leurs séquences natives en surplomb sont complémentaires, de sorte qu'elles sont agencées pour se ligaturer ensemble ; ou
(ii) un ou plusieurs fragments d'ADN médians pour assemblage sont fournis dans lequel les premier et second fragments d'ADN adaptés sont des fragments terminaux respectifs dans l'assemblage, et les un ou plusieurs fragments d'ADN médians sont agencés pour être ligaturés entre les premier et second fragments d'ADN adaptés via des séquences natives en surplomb complémentaires ;
comprenant en outre l'étape de ligature ensemble, avec une ligase, des premier et second fragments d'ADN adaptés, ou des premier et second fragments d'ADN adaptés et d'un ou plusieurs fragments d'ADN médians, pour former un fragment d'ADN assemblé présentant des surplombs maintenus à chaque extrémité.

8. Procédé selon la revendication 7, dans lequel le fragment d'ADN médian comprend une première séquence en surplomb native qui est complémentaire du surplomb natif du premier fragment d'ADN adapté et une seconde séquence en surplomb native qui est complémentaire du surplomb natif du second fragment d'ADN adapté.

9. Procédé selon la revendication 7, dans lequel le procédé comprend deux fragments d'ADN médians ou plus pour assemblage, les fragments d'ADN médians comprennent des séquences en surplomb natives qui sont complémentaires du surplomb natif d'un fragment d'ADN médian voisin, de sorte qu'elles peuvent être liées ensemble dans un ordre prédéterminé ; et dans lequel les premier et dernier fragments d'ADN médians dans la séquence sont agencés pour se ligaturer au premier fragment d'ADN adapté et au second fragment d'ADN adapté respectifs via des séquences en surplomb natives complémentaires.

10. Procédé selon l'une quelconque des revendications 7 à 9, dans lequel un fragment d'ADN médian pour assemblage est fourni par
fourniture d'un acide nucléique méthylé linéarisé supplémentaire par coupure par une enzyme de restriction d'un acide nucléique, dans lequel l'acide nucléique comprend des premier et second éléments de restriction pouvant être protégés par méthylation avec une séquence de rejet entre eux, ou par fourniture d'une version précédemment coupée par une enzyme de restriction de celui-ci où la séquence de rejet est excisée,
dans lequel les premier et second éléments de restriction pouvant être protégés par méthylation de l'acide nucléique comprennent chacun :
- une séquence de reconnaissance d'enzyme de restriction de type IIS ; et
- une séquence de reconnaissance de méthylase d'ADN, dans lequel la séquence de reconnaissance d'enzyme de restriction de type IIS et la séquence de reconnaissance de méthylase d'ADN se chevauchent de sorte que la base pouvant être méthylée par méthylase d'ADN se situe dans la séquence de reconnaissance d'enzyme de restriction de type IIS,
dans lequel la séquence de reconnaissance de méthylase d'ADN n'est pas identique à la séquence de reconnaissance d'enzyme de restriction de type IIS ou contenue dans celle-ci, et dans lequel la séquence de reconnaissance de méthylase d'ADN ne chevauche pas la séquence qui formerait la séquence terminale en surplomb générée par l'enzyme de restriction de type IIS, et
dans lequel les premier et second éléments de restriction pouvant être protégés par méthylation sont méthylés avec la méthylase d'ADN qui reconnaît la séquence de reconnaissance de méthylase d'ADN ; et
dans lequel l'acide nucléique comprend en outre des séquences de reconnaissance d'enzyme de restriction de type IIS ne pouvant pas être protégées opposées prévues sur le côté opposé du site coupé de chacun des premier et second éléments de restriction pouvant être protégés par méthylation pour fournir deux éléments composites de troncature,
dans lequel l'élément composite de troncature est un agencement dans lequel la séquence de reconnaissance d'enzyme de restriction de type IIS opposée de l'élément composite de troncature est agencée pour diriger l'enzyme de restriction de type IIS afin de couper l'acide nucléique au niveau d'un site qui se trouve dans la séquence de reconnaissance de la séquence de reconnaissance d'enzyme de restriction de type IIS de l'élément de restriction pouvant être protégé par méthylation, ou dans les nucléotides entre le site coupé et la séquence de reconnaissance de la séquence de reconnaissance d'enzyme de restriction de type IIS de l'élément de restriction pouvant être protégé par méthylation ;
dans lequel l'acide nucléique est coupé par enzyme de restriction au moyen d'une enzyme de restriction de type IIS qui reconnaît les séquences de reconnaissance d'enzyme de restriction de type IIS opposées formant ainsi l'acide nucléique méthylé linéarisé supplémentaire,
fourniture d'un fragment d'ADN médian pour assemblage présentant des extrémités en surplomb qui sont adaptées pour se lier aux extrémités en surplomb de l'acide nucléique méthylé linéarisé supplémentaire ;
ligature du fragment d'ADN médian pour assemblage et de l'acide nucléique méthylé linéarisé supplémentaire avec une ligase pour former un vecteur intermédiaire méthylé ; transformation du vecteur intermédiaire méthylé en une souche bactérienne qui n'exprime pas la ou les méthylases d'ADN qui reconnaissent l'une ou l'autre des séquences de reconnaissance de méthylase d'ADN des éléments de restriction pouvant être protégés par méthylation, formant ainsi un vecteur intermédiaire qui n'est pas méthylé ; isolement du vecteur intermédiaire ;
coupure du vecteur intermédiaire avec des enzymes de restriction qui reconnaissent la séquence de reconnaissance d'enzyme de restriction de type IIS des éléments de restriction pouvant être protégés par méthylation, formant ainsi un insert de fragment d'ADN médian adapté qui comprend des séquences en surplomb natives à chaque extrémité qui sont déterminées par les séquences natives du fragment d'ADN médian pour assemblage.

11. Procédé selon l'une quelconque des revendications 7 à 10, comprenant en outre l'étape de fourniture d'un vecteur de destination linéarisé pour l'insertion des fragments d'ADN assemblés, éventuellement dans lequel le vecteur de destination linéarisé est fourni par coupure d'un vecteur de destination circulaire avec la ou les mêmes enzyme de restriction de type IIS qui reconnaissent les séquences de reconnaissance d'enzyme de restriction de type IIS de l'élément composite de maintien et/ou de troncature.

12. Composition comprenant l'acide nucléique selon l'une quelconque des revendications 1 à 6.

13. Kit comprenant un ou plusieurs acides nucléiques selon l'une quelconque des revendications 1 à 6, et une enzyme de restriction et/ou une ligase.

14. Cellule hôte comprenant l'acide nucléique selon l'une quelconque des revendications 1 à 6.

15. Utilisation de l'acide nucléique selon l'une quelconque des revendications 1 à 6 pour assembler des fragments d'ADN d'intérêt, éventuellement dans laquelle l'utilisation est *in vi tro.*
